(19) Europäisches Patentamt
European Patent Office
Office européen des brevets

(11) EP 2 530 078 A1

(12) **EUROPEAN PATENT APPLICATION**
published in accordance with Art. 153(4) EPC

(43) Date of publication:
**05.12.2012 Bulletin 2012/49**

(21) Application number: **11737065.0**

(22) Date of filing: **26.01.2011**

(51) Int Cl.:
*C07D 277/20* (2006.01) *A61K 31/426* (2006.01)
*A61K 31/427* (2006.01) *A61K 31/428* (2006.01)
*A61K 31/433* (2006.01) *A61K 31/4439* (2006.01)
*A61K 31/454* (2006.01) *A61K 31/4709* (2006.01)
*A61K 31/496* (2006.01) *A61K 31/497* (2006.01)
*A61K 31/501* (2006.01) *A61K 31/502* (2006.01)
*A61K 31/506* (2006.01) *A61K 31/519* (2006.01)
*A61K 31/5377* (2006.01) *A61K 31/541* (2006.01)
*A61K 31/5415* (2006.01) *A61P 25/18* (2006.01)
*C07D 277/44* (2006.01) *C07D 277/68* (2006.01)

(86) International application number:
**PCT/JP2011/051530**

(87) International publication number:
**WO 2011/093352 (04.08.2011 Gazette 2011/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.01.2010 JP 2010015648**

(71) Applicant: **Takeda Pharmaceutical Company Limited**
**Osaka-shi, Osaka 541-0045 (JP)**

(72) Inventors:
- **IMAEDA, Yasuhiro**
**Osaka-shi**
**Osaka 532-8686 (JP)**
- **WAKABAYASHI, Takeshi**
**Osaka-shi**
**Osaka 532-8686 (JP)**
- **KIMURA, Eiji**
**Osaka-shi**
**Osaka 532-8686 (JP)**
- **TOKUMARU, Kazuyuki**
**Osaka-shi**
**Osaka 532-8686 (JP)**

(74) Representative: **Held, Stephan et al**
**Meissner, Bolte & Partner GbR**
**Chemistry**
**Widenmayerstraße 47**
**80538 München (DE)**

(54) **THIAZOLE DERIVATIVE**

(57) Provided is a compound having an agonist action on GPR52, which is useful as a prophylactic or therapeutic drug for mental diseases such as schizophrenia and the like, and the like.

A compound represented by the formula (I):

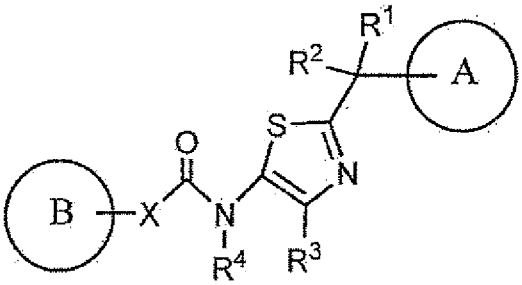

(I)

wherein each symbol is as defined in the specification, or a salt thereof.

EP 2 530 078 A1

**Description**

Technical Field

The present invention relates to a novel amide compound and a production method of the same, and a medicament containing the same. More specifically, the present invention relates to a compound having an agonistic action on GPR52, which is effective as a medicament for the prophylaxis or treatment of mental disorders such as schizophrenia and the like.

(Background of the Invention)

Schizophrenia is a disease that occurs in people from adolescence to adulthood and shows characteristic thinking disturbances, disturbances of ego, and behavioral abnormalities associated therewith. The incidence of the disease is allegedly about 1% of the entire population. Most of them are chronic, so that the initiative or interpersonal contact of patients, and the like may be decreased, thereby seriously interfering with the social lives of the patients. The core symptoms of schizophrenia are broadly classified into (1) positive symptoms such as delusions and hallucination, (2) negative symptoms such as hypesthesia, social withdrawal, diminished motivation, and loss of concentration, and (3) cognitive dysfunction. In these core symptoms, the expression of positive symptoms is intimately involved in over activity of the dopamine nervous system in the mesolimbic system. The expression of the negative symptoms and impaired cognitive function are intimately involved in deterioration of the function of the nervous system such as the glutamic acid nervous system in the cortex of frontal lobe.
In addition, a typical antipsychotic agent having an antagonistic action on a dopamine D2 receptor, such as chlorpromazine, has favorable effects on the positive symptoms. On the other hand, drugs effective to multiple receptors, such as clozapine and olanzapine have certain effects on negative symptoms and cognitive dysfunction. However, it is known that many patients have poor response on these drugs. Also, the typical antipsychotic agent has controversial side effects such as the occurrence of extrapyramidal syndromes, for example, akathisia, dystonia and Parkinson-like movement disorders, and the occurrence of hyperprolactinemia. Furthermore, clozapine may cause agranulocytosis as a grave side effect. An atypical antipsychotic agent such as olanzapine may cause side effects, such as weight gain, lipidosis, excessive sedative effect, and prolonged cardiac QT interval.
Human GPR52 (non-patent reference 1) has been known as one of GPCRs. In recent years, because of an increase in cellular cAMP level in nerve cells expressing GPR52 or the like, any of agonists and ligands against GPR52 has been considered to have an effect of improving the positive symptoms of schizophrenia by suppressing the hyperactivation of dopamine pathway in the mesolimbic region, one of the causes of the positive symptoms of schizophrenia. In addition, it has been also found that the agonists and ligands against GPR52 can improve the negative symptoms and cognitive dysfunction of schizophrenia by an improvement in decreased function of NMDA receptors in the cerebral cortex, which has been considered as one of the causes of the negative symptoms and cognitive dysfunction of schizophrenia (patent reference 1).
Furthermore, an action to increase an intracellular cAMP concentration in the aforementioned nerve cells may be involved in the adjustment of the function of not only the dopamine system but also various neurotransmitters such as norepinephrine, serotonin, histamine, acetylcholine and the like, and of the survival, differentiation and plastic change of nerves. Thus, an agonist and a ligand of GPR52 and the like can be expected to show an action useful for mental diseases, neurodegenerative diseases and various systemic diseases caused by disturbance of the nerve system.
Therefore, it has been demanded to develop a compound having an agonistic action on GPR52 and useful as a medicament for the prophylaxis or treatment of various diseases such as mental diseases (schizophrenia etc.).

As compounds having an agonist action on GPR52, for example, patent document 2 discloses a compound represented by the following formula.

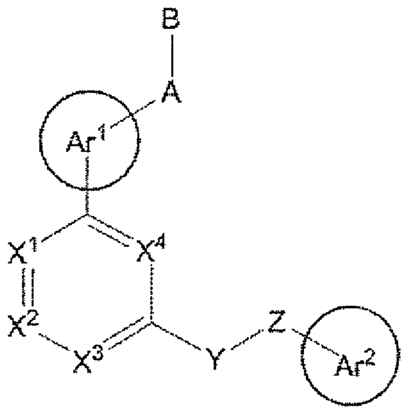

wherein A is $-(CH_2)n-CO-NR^a-$ (n is an integer of 0 - 3) or $-NR^a-CO-$; B is a hydrogen atom, a halogen atom, a cyano group, a hydroxy group, $-O-R^b$ or the like; $X^1$, $X^2$, $X^3$ and $X^4$ are the same or different and each is $-CR^x=$ or -N=; Y is -O-, -S-, -S(O)-, - $S(O)_2$- or $-NR^y-$; Z is a bond, methylene or ethylene; $Ar^1$ is a 5-to 10-membered aromatic ring optionally substituted by one or more substituents selected from a halogen atom, an optionally halogenated $C_{1-6}$ alkyl group and an optionally halogenated $C_{1-6}$ alkoxy group (excluding thiazole); $Ar^2$ is a 5- or 6-membered aromatic ring which is optionally substituted by one or more substituents selected from a halogen atom, an optionally halogenated $C_{1-6}$ alkyl group and an optionally halogenated $C_{1-6}$ alkoxy group, and optionally condensed with an optionally substituted 5- or 6-membered ring; and $R^a$, $R^b$, $R^x$ and $R^y$ are the same or different and each is a hydrogen atom, a halogen atom, an optionally halogenated $C_{1-6}$ alkyl group or an optionally halogenated $C_{1-6}$ alkoxy group.

In addition, as a compound having an agonist action on GPR52, for example, patent document 3 discloses a compound represented by the following formula.

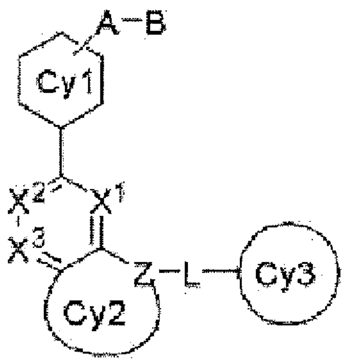

wherein A is -CONH- or the like; B is a hydrogen atom or a substituent; ring Cy1 is a benzene ring or the like; $X^1$, $X^2$ and $X^3$ are each independently -CH=, -N= or the like; ring Cy2 is carbocycle or the like; Z is a carbon atom or a nitrogen atom; L is a bond or the like; n is 1 or 2; $R^b$ is a hydrogen atom or a substituent; and ring Cy3 is a benzene ring or the like.

In addition, as a compound having an agonist action on GPR52, for example, patent document 4 discloses a compound represented by the following formula.

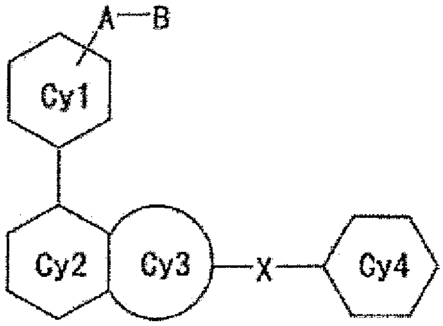

(Ia)

wherein A is $-CONR^2-$ or $-NR^2CO-$; $R^2$ is a hydrogen atom or the like; B is a hydrogen atom or the like; ring Cy1 is a 6-membered aromatic ring optionally having one or more substituents besides a group represented by -A-B; ring Cy2 is a 6-membered ring optionally substituted by a halogen atom and the like; ring Cy3 is a 5- or 6-membered ring optionally having one or more substituents; X is $C_{1-2}$ alkylene or the like; m is an integer of 0 - 2; and ring Cy4 is a 6-membered aromatic ring optionally having one or more substituents.

As a thiazole compound, non-patent document 2 discloses the following compound.

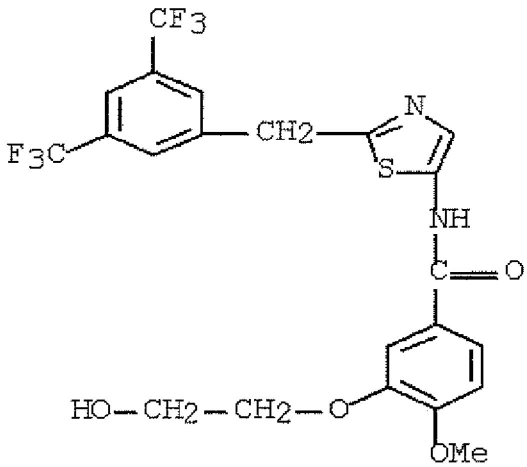

In addition, patent document 5 discloses a thiazole derivative included in the following formula.

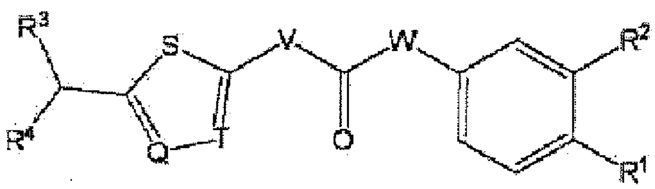

(I)

wherein $R^1$ is a hydroxy group, a $C_{1-6}$ alkoxy group, a $C_{2-6}$ hydroxyalkoxy group or the like; $R^2$ is a hydroxy group, a $C_{1-6}$ alkoxy group, a $C_{2-6}$ hydroxyalkoxy group or the like; $R^3$ is a hydrogen atom or the like; $R^4$ is a $C_{6-10}$ aryl group optionally substituted by 1 to 5 independent groups selected from substituent group A or the like; V is -NH- or a single bond; W is -NH- or a single bond; T is $=C(R^5)-$ or a nitrogen atom; and Q is $=C(R^6)-$ or a nitrogen atom.

Furthermore, patent document 6 discloses a thiazole derivative included in the following formula.

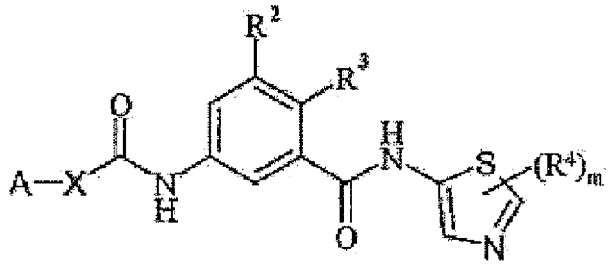

wherein A is aryl optionally substituted by 1 to 4 substituents selected from halo, nitro and the like or the like; X is absent, or O or $NR_a$ ($R_a$ is a hydrogen atom or $C_{1-6}$ alkyl); $R^2$ and $R^3$ are each independently a hydrogen atom, halo, nitro or the like; $R^4$ is $C_{1-6}$ alkyl optionally substituted by one or more $R^{16}$ or the like; $R^{16}$ is carbocycle $-R^{22}-$ optionally substituted by one or more $R^{24}$ or the like; $R^{22}$ is a single bond or the like; $R^{24}$ is halo, nitro or the like; and m is 0 - 2.

Moreover, patent document 7 discloses a thiazole derivative included in the following formula.

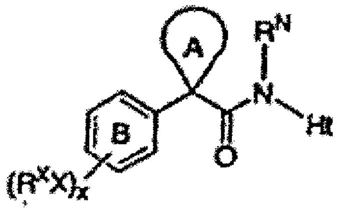

wherein Ht is an optionally substituted 5-membered aromatic heterocycle or the like: $R^N$ is H or R; R is hydrogen or $C_{1-6}$ fatty series; ring A is an optionally substituted 3- to 7-membered monocycle; ring B is a 5- or 6-membered aromatic or nonaromatic carbocycle or heterocyclic ring; x is 0-5; or the like.

Furthermore, patent document 8 discloses a thiazole derivative included in the following formula.

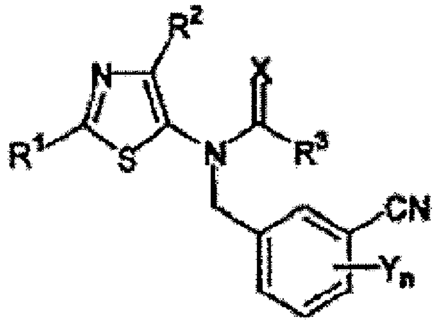

(I)

wherein $R^1$ is a $C_{1-6}$ alkyl group optionally substituted by an optionally substituted $C_{3-7}$ cycloalkyl group and the like, or the like; $R^2$ is a hydrogen atom or an optionally substituted $C_{1-6}$ alkyl group; $R^3$ is a halogen atom, an optionally substituted $C_{1-6}$ alkyl group, a $C_{3-7}$ cycloalkyl group, an optionally substituted phenyl group or the like; X is an oxygen atom or a sulfur atom; Y is a $C_{1-6}$ alkyl group or the like; and n is an integer of 0 - 4.

Moreover, patent document 9 discloses the following thiazole derivative.

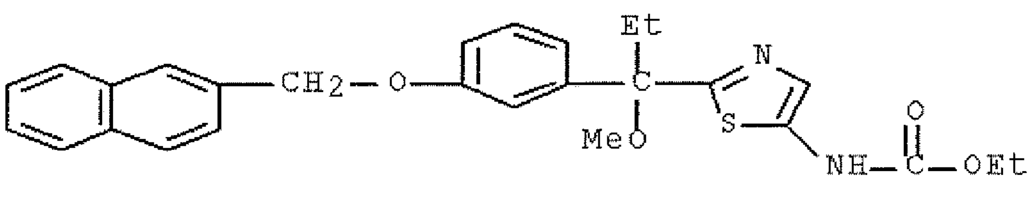

Furthermore, as a thiazole derivative, the following compound is known.

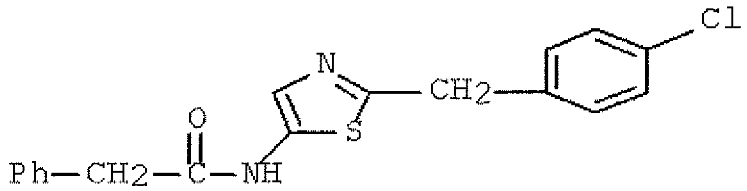

[Document List]

[patent documents]

patent document 1: WO2006/098520
patent document 2: WO2009/107391
patent document 3: WO2009/157196
patent document 4: WO2010/018874
patent document 5: WO2008/139845
patent document 6: WO2007/071955
patent document 7: WO2005/075435
patent document 8: JP-A-2003-212864
patent document 9: EP351194
[non-patent documents]

non-patent document 1: Molecular Brain Research, vol.64, p.193-198, 1999
non-patent document 2: Bioorg. Med. Chem. Lett., vol.19, issue 15, p.4159-4166, 2009

[SUMMARY OF THE INVENTION]

Problems to be solved by the Invention

An object of the present invention is to provide a compound having an agonistic action on GPR52 and useful as a medicament for the prophylaxis or treatment of mental diseases such as schizophrenia.

Means of Solving the Problems

The present inventors have found that compounds represented by the following formula (I) or salts thereof have an agonistic action on GPR52 and finally completed the present invention by further investigations.
Accordingly, the present invention relates to

[1] a compound represented by the formula:

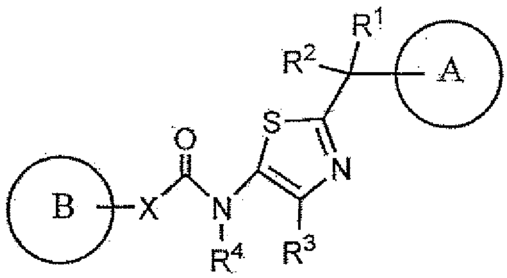

(I)

wherein

$R^1$ and $R^2$ are the same or different and each is a hydrogen atom, a hydroxy group, a $C_{1-6}$ alkyl group optionally having substituent(s), a $C_{3-6}$ cycloalkyl group optionally having substituent(s) or a $C_{1-6}$ alkoxy group optionally having substituent(s), or

$R^1$ and $R^2$ may form, together with the adjacent carbon atom, a 3- to 6-membered saturated hydrocarbon ring;

$R^3$ is a hydrogen atom, a halogen atom, a $C_{1-6}$ alkyl group optionally having substituent(s), a $C_{2-6}$ alkenyl group optionally having substituent(s), a $C_{2-6}$ alkynyl group optionally having substituent(s), a $C_{3-6}$ cycloalkyl group optionally having substituent(s) or an acyl group;

$R^4$ is a hydrogen atom, a $C_{1-6}$ alkyl group optionally having substituent(s) or a $C_{3-10}$ cycloalkyl group optionally having substituent(s) ;

ring A is a 3- to 10-membered hydrocarbon ring optionally having substituent(s);

ring B is a 3- to 10-membered hydrocarbon ring optionally having substituent(s) or a 3- to 11-membered heterocycle optionally having substituent(s); and

X is a single bond, a $C_{1-6}$ alkylene group optionally having substituent(s) or a $C_{2-6}$ alkenylene group, [excluding N-{2-[3,5-bis(trifluoromethyl)benzyl]-1,3-thiazol-5-yl}-3-(2-hydroxyethoxy)-4-methoxybenzamide,
N-{2-[3,5-bis(trifluoromethyl)benzyl]-1,3-thiazol-5-yl}-4-methoxy-3-[2-(tetrahydro-2H-pyran-2-yloxy)ethoxy]benzamide, 1-(1,3-benzodioxol-5-yl)-N-[2-(2-chlorobenzyl)-1,3-thiazol-5-yl]cyclopropanecarboxamide,
N-[2-(2-chlorobenzyl)-1,3-thiazol-5-yl]-1-(4-methoxyphenyl)cyclopropanecarboxamide,
N-(3-cyanobenzyl)-N-[2-(cyclopentylmethyl)-4-methyl-1,3-thiazol-5-yl]cyclopropanecarboxamide,
N-(3-cyanobenzyl)-N-[2-(cyclopentylmethyl)-4-methyl-1,3-thiazol-5-yl]azetidine-1-carboxamide,
N-(3-cyanobenzyl)-N-[2-(cyclopentylmethyl)-4-methyl-1,3-thiazol-5-yl]pyrrolidine-1-carboxamide,
methyl 1-{(3-cyanobenzyl)[2-(cyclopentylmethyl)-4-methyl-1,3-thiazol-5-yl]carbamoyl}pyrrolidine-3-carboxylate,
N-(3-cyanobenzyl)-N-[2-(cyclopentylmethyl)-4-methyl-1,3-thiazol-5-yl]morpholine-4-carboxamide, and
N-[2-(4-chlorobenzyl)-1,3-thiazol-5-yl]-2-phenylacetamide]
or a salt thereof;

[2] the compound of the above-mentioned [1], wherein $R^4$ is

(1) a hydrogen atom,
(2) a $C_{3-10}$ cycloalkyl group optionally having substituent(s),
(3) a methyl group optionally having substituent(s) selected from a halogen atom, a cyano group, a hydroxy group, a $C_{1-6}$ alkoxy group optionally having substituent(s), a $C_{6-10}$ aryloxy group optionally having substituent (s), a sulfanyl group optionally having substituent(s), an amino group optionally having substituent(s), an acyl group, a $C_{3-10}$ cycloalkyl group optionally having substituent(s) and a 3- to 11-membered heterocyclic group optionally having substituent(s), or
(4) a $C_{2-6}$ alkyl group optionally having substituent(s),
ring B is

(1) benzene having at least one substituent selected from a halogen atom, a cyano group, a $C_{1-6}$ alkyl group optionally having substituent(s), a $C_{2-6}$ alkenyl group optionally having substituent(s), a $C_{2-6}$ alkynyl group optionally having substituent(s), a sulfanyl group optionally having substituent(s), an amino group optionally having substituent(s), an acyl group, a 3- to 10-membered hydrocarbon ring group optionally having substituent(s) and a 3- to 11-membered heterocyclic group optionally having substituent(s),
(2) a 3- to 10-membered saturated hydrocarbon ring optionally having substituent(s) selected from a halogen atom, a cyano group, a hydroxy group, a $C_{1-6}$ alkyl group optionally having substituent(s), a $C_{2-6}$ alkenyl group optionally having substituent(s), a $C_{2-6}$ alkynyl group optionally having substituent(s), a $C_{1-6}$ alkoxy group optionally having substituent(s), a $C_{6-10}$ aryloxy group optionally having substituent(s), a sulfanyl group optionally having substituent(s), an amino group optionally having substituent(s), an acyl group, a $C_{3-10}$ cycloalkyl group optionally having substituent(s) and a 3- to 11-membered nitrogen-containing heterocyclic group optionally having substituent(s), or
(3) a 3- to 11-membered heterocycle optionally having substituent(s),

or a salt thereof;

[3] the compound of the above-mentioned [1], wherein $R^3$ is

(1) a hydrogen atom,
(2) a $C_{1-6}$ alkyl group optionally substituted by 1 to 3 substituents selected from a halogen atom, a hydroxy group and a $C_{1-6}$ alkoxy group,
(3) a $C_{2-6}$ alkenyl group,

(4) a $C_{1-6}$ alkyl-carbonyl group, or
(5) a $C_{1-6}$ alkoxy-carbonyl group,
or a salt thereof;

[4] the compound of the above-mentioned [1], wherein $R^4$ is a hydrogen atom or a $C_{1-6}$ alkyl group, or a salt thereof;
[5] the compound of the above-mentioned [1], wherein ring A is a 6- to 10-membered aromatic hydrocarbon ring optionally substituted by 1 to 3 substituents selected from (i) a halogen atom, (ii) a $C_{1-6}$ alkyl group optionally substituted by 1 to 3 halogen atoms, (iii) a nitro group, (iv) a $C_{1-6}$ alkoxy group, (v) a hydroxy group, (vi) a $C_{3-10}$ cycloalkyl group and (vii) a $C_{1-6}$ alkylsulfonyl group, or a salt thereof;
[6] the compound of the above-mentioned [1], wherein ring B is (1) a 3- to 10-membered hydrocarbon ring optionally substituted by 1 to 3 substituents selected from

(a) a nonaromatic heterocyclic group optionally substituted by 1 to 3 oxo groups,
(b) an amino group optionally mono- or di-substituted by substituent(s) selected from a $C_{1-6}$ alkyl group and a $C_{1-6}$ alkyl-carbonyl group,
(c) a $C_{1-6}$ alkoxy group,
(d) a $C_{1-6}$ alkylsulfanyl group,
(e) a cyano group,
(f) a halogen atom,
(g) an acyl group and
(h) an oxo group, or

(2) a 3- to 11-membered heterocycle optionally having 1 to 3 substituents selected from

(a) a $C_{3-10}$ cycloalkyl group,
(b) a $C_{6-14}$ aryl group optionally substituted by 1 to 3 substituents selected from

(i) a $C_{1-6}$ alkoxy group,
(ii) a halogen atom and
(iii) an amino group optionally mono- or di-substituted by a $C_{1-6}$ alkyl group,

(c) an aromatic heterocyclic group,
(d) a nonaromatic heterocyclic group,
(e) an amino group optionally mono- or di-substituted by a $C_{1-6}$ alkyl-carbonyl group,
(f) a $C_{1-6}$ alkoxy group,
(g) a $C_{6-10}$ aryloxy group optionally substituted by 1 to 3 halogen atoms,
(h) a $C_{1-6}$ alkylsulfanyl group,
(i) a cyano group,
(j) a nitro group,
(k) a halogen atom,
(l) an acyl group,
(m) a $C_{1-6}$ alkyl group optionally substituted by 1 to 3 substituents selected from

(i) a halogen atom,
(ii) a hydroxy group,
(iii) an amino group optionally mono- or di-substituted by a $C_{1-6}$ alkyl group,
(iv) a $C_{6-10}$ aryloxy group optionally substituted by a $C_{1-6}$ alkoxy group,
(v) a carbamoyl group,
(vi) an aromatic heterocyclic group optionally substituted by 1 to 3 $C_{1-6}$ alkyl groups,
(vii) a nonaromatic heterocyclic group optionally substituted by 1 to 3 oxos
(viii) a $C_{1-6}$ alkoxy group,
(ix) a cyano group and
(x) a $C_{1-6}$ alkylsulfonyl group,

(n) a $C_{7-13}$ aralkyl group optionally substituted by 1 to 3 substituents selected from a $C_{1-6}$ alkyl group optionally substituted by 1 to 3 halogen atoms, and
(o) an oxo group,
or a salt thereof;

[7] the compound of the above-mentioned [1], wherein X is a single bond, a $C_{1-6}$ alkylene group or a $C_{2-6}$ alkenylene group, or a salt thereof;
[8] the compound of the above-mentioned [1], wherein $R^1$ and $R^2$ are the same or different and each is a hydrogen atom, a hydroxy group or a $C_{1-6}$ alkyl group, or $R^1$ and $R^2$ form, together with the adjacent carbon atom, a 3- to 6-membered saturated hydrocarbon ring,
or a salt thereof;
[9] 1-methyl-N-{4-methyl-2-[3-(trifluoromethyl)benzyl]-1,3-thiazol-5-yl}-1H-pyrazole-5-carboxamide or a salt thereof (Example 3);
[10] N-[2-(3-chloro-5-fluorobenzyl)-4-methyl-1,3-thiazol-5-yl]-1-methyl-1H-pyrazole-5-carboxamide or a salt thereof (Example 145);
[11] N-[2-(3-chloro-5-fluorobenzyl)-4-methyl-1,3-thiazol-5-yl]-1,3-dimethyl-1H-pyrazole-4-carboxamide or a salt thereof (Example 146);
[12] N-[2-(3-chloro-5-fluorobenzyl)-4-methyl-1,3-thiazol-5-yl]-1-ethyl-1H-pyrazole-5-carboxamide or a salt thereof (Example 148);
[13] N-[2-(3-chloro-5-fluorobenzyl)-4-methyl-1,3-thiazol-5-yl]-1-propyl-1H-pyrazole-5-carboxamide or a salt thereof (Example 153);
[14] a medicament comprising the compound of the above-mentioned [1] or a salt thereof;
[15] the medicament of the above-mentioned [14], which is a GPR52 activating agent;
[16] the medicament of the above-mentioned [14], which is an agent for the prophylaxis or treatment of schizophrenia;
[17] a method for activation of GPR52 in a mammal, which comprises administering an effective amount of the compound of the above-mentioned [1] or a salt thereof to the mammal;
[18] a method for the prophylaxis or treatment of schizophrenia in a mammal, which comprises administering an effective amount of the compound of the above-mentioned [1] or a salt thereof to the mammal;
[19] use of the compound of the above-mentioned [1] or a salt thereof for the production of a GPR52 activating agent;
[20] use of the compound of the above-mentioned [1] or a salt thereof for the production of an agent for the prophylaxis or treatment of schizophrenia;
[21] the compound of the above-mentioned [1] or a salt thereof for use in a method of activating GPR52;
[22] the compound of the above-mentioned [1] or a salt thereof for use in the prophylaxis or treatment of schizophrenia;
and the like.

Effect of the Invention

The compound of the present invention has an agonistic action on GPR52 and is advantageously used as a drug for the prophylaxis or treatment of mental diseases such as schizophrenia.

(Detailed Description of the Invention)

The present invention is explained in detail in the following.
Unless particularly limited, examples of the "halogen atom" in the present specification include a fluorine atom, a chlorine atom, a bromine atom and an iodine atom.
Unless particularly limited, examples of the "$C_{1-10}$ alkyl group" in the present specification include methyl, ethyl, propyl, isopropyl, butyl, isobutyl, sec-butyl, tert-butyl, pentyl, isopentyl, neopentyl, 1-ethylpropyl, 1,1-dimethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.
Unless particularly limited, examples of the "$C_{1-6}$ alkyl group" and "$C_{1-6}$ alkyl" used as the substituents in the present specification include those having a carbon number of 1 - 6. Of these, $C_{1-4}$ alkyl (group) is preferable.
Unless particularly limited, examples of the "$C_{2-6}$ alkyl group" in the present specification include those having a carbon number of 2 - 6.
Unless particularly limited, examples of the "$C_{1-6}$ alkoxy group" and "$C_{1-6}$ alkoxy" used as the substituent in the present specification include methoxy, ethoxy, propoxy, isopropoxy, butoxy, isobutoxy, sec-butoxy, tert-butoxy, pentyloxy, hexyloxy and the like. Of these, "$C_{1-4}$ alkoxy (group)" is preferable.
Unless particularly limited, examples of the "$C_{2-10}$ alkenyl group" in the present specification include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.
Unless particularly limited, examples of the "$C_{2-6}$ alkenyl group" and "$C_{2-6}$ alkynyl" used as the substituents in the present specification include those having a carbon number of 2 - 6. Of these, $C_{2-4}$ alkenyl (group) is preferable.
Unless particularly limited, examples of the "$C_{2-10}$ alkynyl group" in the present specification include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like. Unless particularly limited, examples of the

"$C_{2-6}$ alkynyl group" and "$C_{2-6}$ alkynyl" used as the substituents in the present specification include those having a carbon number of 2 - 6. Of these, $C_{2-4}$ alkynyl (group) is preferable.

Unless particularly limited, examples of the "$C_{3-10}$ cycloalkyl group" in the present specification include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1.]decyl, adamantyl and the lake.

Unless particularly limited, examples of the "$C_{3-6}$ cycloalkyl group" and "$C_{3-6}$ cycloalkyl" used as the substituent in the present specification include cyclopropyl, cyclobutyl, cyclopentyl, cyclohexyl and the like.

Unless particularly limited, examples of the "$C_{3-10}$ cycloalkenyl group" in the present specification include 2-cyclopenten-1-yl, 3-cyclopenten-1-yl, 2-cyclohexen-1-yl, 3-cyclohexen-1-yl and the like.

Unless particularly limited, examples of the "$C_{4-10}$ cycloalkadienyl group" in the present specification include 2,4-cyclopentadien-1-yl, 2,4-cyclohexadien-1-yl, 2,5-cyclohexadien-1-yl and the like.

The above-mentioned $C_{3-10}$ cycloalkyl group, $C_{3-10}$ cycloalkenyl group and $C_{4-10}$ cycloalkadienyl group may be each fused with a benzene ring. Examples of such fused ring group include indanyl, dihydronaphthyl, tetrahydronaphthyl, fluorenyl and the like.

Unless particularly limited, examples of the "$C_{6-14}$ aryl group" and "$C_{6-14}$ aryl" used as the substituent in the present specification include phenyl, naphthyl, anthryl, phenanthryl, acenaphthyl, biphenylyl and the like.

Examples of the "$C_{6-10}$ aryl group" and "$C_{6-10}$ aryl" used as the substituent in the present specification include phenyl, naphthyl (1-naphthyl, 2-naphthyl) and the like.

Unless particularly limited, examples of the "$C_{6-14}$ aryloxy group" in the present specification include phenyloxy, naphthyloxy, anthryloxy, phenanthryloxy, acenaphthyloxy, biphenylyloxy and the like.

Unless particularly limited, examples of the "$C_{6-10}$ aryloxy group" in the present specification include phenyloxy, naphthyloxy (1-naphthyloxy, 2-naphthyloxy) and the like.

Unless particularly limited, examples of the "$C_{7-20}$ aralkyl group" and "$C_{7-20}$ aralkyl" used as the substituents in the present specification include a $C_{1-6}$ alkyl group substituted by 1 to 3 $C_{6-10}$ aryl groups. More specifically, for example, benzyl, phenethyl, naphthylmethyl, biphenylylmethyl, trityl and the like can be mentioned.

Examples of the "$C_{7-13}$ aralkyl group" and "$C_{7-13}$ aralkyl" used as the substituent in the present specification include benzyl, phenethyl, naphthylmethyl, biphenylylmethyl and the like.

Unless particularly limited, examples of the "$C_{1-6}$ alkoxy-carbonyl group" in the present specification include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl and the like.

Unless particularly limited, examples of the "$C_{1-6}$ alkyl-carbonyl group" in the present specification include acetyl, propanoyl, butanoyl, 2-methylpropanoyl, pentanoyl, 3-methylbutanoyl, 2-methylbutanoyl, 2,2-dimethylpropanoyl, hexanoyl, heptanoyl and the like.

Unless particularly limited, examples of the "aromatic heterocyclic group and "aromatic heterocyclyl-" used as the substituent in the present specification include a 4- to 7-membered (preferably 5- or 6-membered) monocyclic aromatic heterocyclic group containing, as a ring-constituting atom besides carbon atoms, 1 to 4 hetero atoms selected from an oxygen atom, a sulfur atom (the sulfur atom is optionally oxidized) and a nitrogen atom, and a fused aromatic heterocyclic group. Examples of the fused aromatic heterocyclic group include a group derived from a fused ring wherein a ring corresponding to the 4- to 7-membered monocyclic aromatic heterocyclic group and 1 or 2 rings selected from a 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene) and a benzene ring are fused, and the like.

Preferable examples of the aromatic heterocyclic group include monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,5-oxadiazol-3-yl, 1,3,4-oxadiazol-2-yl), thiadiazolyl (e.g., 1,2,3-thiadiazol-4-yl, 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-3-yl, 1,2,4-triazin-5-yl, 1,2,4-triazin-6-yl) and the like; fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl, 4-benzofuranyl, 5-benzofuranyl, 6-benzofuranyl, 7-benzofuranyl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl, 6-benzothiazolyl), benzimidazolyl (e.g., benzimidazol-1-yl, benzimidazol-2-yl, benzimidazol-5-yl), benzotriazolyl (e.g., 1H-1,2,3-benzotriazol-1-yl, 1H-1,2,3-benzotriazol-5-yl), indolyl (e.g., indol-1-yl, indol-2-yl, indol-3-yl, indol-5-yl), indazolyl (e.g., 2H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), thienopyrazolyl (e.g., 1H-thieno[2,3-c]pyrazol-5-yl), pyrazolotriazinyl (e.g., pyrazolo[5,1-c][1,2,4]triazin-3-yl), triazolopyrimidinyl (e.g., [1,2,4]triazolo[1,5-a]pyrimidin-2-yl), phthalazinyl and the like; and the like.

Unless particularly limited, examples of the "non-aromatic heterocyclic groups and "non-aromatic heterocyclyl-" used as the substituent in the present specification include a 4- to 7-membered (preferably 5- or 6-membered) monocyclic non-aromatic heterocyclic group containing, as a ring-constituting atom besides carbon atoms, 1 to 4 hetero atoms selected from an oxygen atom, a sulfur atom (the sulfur atom is optionally oxidized) and a nitrogen atom, and a fused non-aromatic heterocyclic group. Examples of the fused non-aromatic heterocyclic group include a group derived from a fused ring wherein a ring corresponding to the 4- to 7-membered monocyclic non-aromatic heterocyclic group and 1 or 2 rings selected from a 5- or 6-membered aromatic or non-aromatic heterocycle containing 1 or 2 nitrogen atoms (e.g., pyrrole, imidazole, pyrazole, pyrazine, pyridine, pyrimidine), a 5-membered aromatic or non-aromatic heterocycle containing one sulfur atom (e.g., thiophene) and a benzene ring are fused, and the like.

Preferable examples of the nonaromatic heterocyclic group include monocyclic nonaromatic heterocyclic groups such as pyrrolidinyl (e.g., 1-pyrrolidinyl, 2-pyrrolidinyl, 3-pyrrolidinyl), piperidinyl (e.g., piperidino, 2-piperidinyl, 3-piperidinyl, 4-piperidinyl), homopiperidinyl (e.g., homopiperidino, 2-homopiperidinyl, 3-homopiperidinyl, 4-homopiperidinyl), tetrahydropyridyl (e.g., 1,2,3,6-tetrahydropyridin-1-yl), dihydropyridyl (e.g., 2,3-dihydropyridin-4-yl), morpholinyl (e.g., morpholino, 2-morpholinyl), thiomorpholinyl (e.g., thiomorpholino), 1,1-dioxidothiomorpholinyl (e.g., 1,1-dioxidothiomorpholino), piperazinyl (e.g., 1-piperazinyl, 2-piperazinyl), hexamethyleniminyl (e.g., 1-hexamethyleniminyl), oxazolidinyl (e.g., 2-oxazolidinyl), thiazolidinyl (e.g., 3-thiazolidinyl, 2-thiazolidinyl), isothiazolidinyl (e.g., 5-isothiazolidinyl), 1,1-dioxidoisothiazolidinyl (e.g., 1,1-dioxidoisothiazolidin-5-yl), imidazolidinyl (e.g., 2-imidazolidinyl, 3-imidazolidinyl), oxazolinyl (e.g., 2-oxazolinyl), thiazolinyl (e.g., 2-thiazolinyl), imidazolinyl (e.g., 2-imidazolinyl, 3-imidazolinyl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), pyranyl (e.g., 2-pyranyl, 4-pyranyl), tetrahydropyranyl (e.g., 2-tetrahydropyranyl, 3-tetrahydropyranyl, 4-tetrahydropyranyl), thiopyranyl (e.g., 4-thiopyranyl), tetrahydrothiopyranyl (e.g., 2-tetrahydrothiopyranyl, 3-tetrahydrothiopyranyl, 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl, tetrahydrofuran-2-yl), tetrahydrothienyl, 1,1-dioxidotetrahydrothienyl, pyrazolidinyl (e.g., 1-pyrazolidinyl, 3-pyrazolidinyl), pyrazolinyl (e.g., 1-pyrazolinyl), tetrahydropyrimidinyl (e.g., 1-tetrahydropyrimidinyl), dihydrotriazolyl (e.g., 2,3-dihydro-1H-1,2,3-triazol-1-yl), tetrahydrotriazolyl (e.g., 2,3,4,5-tetrahydro-1H-1,2,3-triazol-1-yl), dihydrooxadiazolyl (e.g., 4,5-dihydro-1,2,4-oxadiazol-3-yl), thiazinyl (e.g., 1,4-thiazin-2-yl), 1,1-dioxidothiazinanyl (e.g., 1,1-dioxido-1,2-thiazinan-2-yl), dihydropyridazinyl (e.g., 1,6-dihydropyridazin-3-yl), tetrahydropyridazinyl (e.g., 1,4,5,6-tetrahydropyridazin-3-yl), dihydrothioxazinyl (e.g., 2,3-dihydro-1,4-thioxazin-3-yl), dihydrothiazinyl (e.g., 3,4-dihydro-2H-1,4-thiazin-5-yl) and the like;

fused nonaromatic heterocyclic groups such as dihydroindolyl (e.g., 2,3-dihydro-1H-indol-1-yl), dihydroisoindolyl (e.g., 2,3-dihydro-1H-isoindol-1-yl, 1,3-dihydro-2H-isoindol-2-yl), dihydrobenzofuranyl (e.g., 2,3-dihydro-1-benzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepin-7-yl), tetrahydrobenzofuranyl (e.g., 4,5,6,7-tetrahydro-1-benzofuran-3-yl), chromenyl (e.g., 4H-chromen-2-yl, 2H-chromen-3-yl, 2H-chromen-7-yl), dihydroquinolinyl (e.g., 1,2-dihydroquinolin-4-yl, 3,4-dihydroquinolin-1(2H)-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl, 1,2,3,4-tetrahydroisoquinolin-2-yl), dihydrophthalazinyl (e.g., 3,4-dihydrophthalazin-1-yl, 1,4-dihydrophthalazin-4-yl), tetrahydrobenzoazepinyl (e.g., 2,3,4,5-tetrahydro-1H-benzo[c]azepin-1-yl), benzodioxolyl (e.g., 1,3-benzodioxol-5-yl), benzothiazinyl (e.g., 3,4-dihydro-2H-1,4-benzothiazin-2-yl), 2,2-dioxido-2-thia-3-azabicyclo[3.1.0]hexynyl (e.g., 2,2-dioxido-2-thia-3-azabicyclo[3.1.0]hexan-1-yl), 2,2-dioxido-2-thia-3-azabicyclo[4.1.0]heptanyl (e.g., 2,2-dioxido-2-thia-3-azabicyclo[4.1.0]heptan-1-yl), 1,3-diazaspiro[4.4]non-3-yl and the like;

and the like.

Unless particularly limited, examples of the "heterocyclic group" and the "heterocyclyl-" of the substituents in the present specification include the aforementioned aromatic heterocyclic group and nonaromatic heterocyclic group.

Unless particularly limited, examples of the "3- to 11-membered heterocyclic group" in the present specification include the aforementioned heterocyclic groups which are 3- to 11-membered.

Unless particularly limited, examples of the "3- to 11-membered nitrogen-containing heterocyclic group" in the present specification include the aforementioned 3- to 11-membered heterocyclic groups which contain at least one nitrogen atom as a ring-constituting atom.

Unless particularly limited, examples of the "3- to 11-membered heterocycle" in the present specification include a 3- to 11-membered aromatic heterocycle and a non-aromatic heterocycle.

Here, examples of the 3- to 11-membered aromatic heterocycle include those exemplified as the above-mentioned "aromatic heterocyclic group" and the "aromatic heterocyclyl-" of the substituents, which constitute a 3- to 11-membered aromatic heterocyclic group.

Preferable examples of the aromatic heterocycle include monocyclic aromatic heterocycles such as furan, thiophene, pyridine, pyrimidine, pyridazine, pyrazine, pyrrole, imidazole, pyrazole, thiazole, isothiazole, oxazole, isoxazole, oxadiazole (e.g., 1,2,5-oxadiazole, 1,3,4-oxadiazole), thiadiazole (e.g., 1,2,3-thiadiazole, 1,3,4-thiadiazole), triazole (e.g., 1,2,4-triazole, 1,2,3-triazole), tetrazole, triazine (e.g., 1,2,4-triazine) and the like;
fused aromatic heterocycles such as quinoline, isoquinoline, quinazoline, quinoxaline, benzofuran, benzothiophene, benzoxazole, benzisoxazole, benzothiazole, benzimidazole, benzotriazole (e.g., 1H-1,2,3-benzotriazole), indole, indazole, pyrrolopyrazine (e.g., 1H-pyrrolo[2,3-b]pyrazine, imidazopyridine (e.g., 1H-imidazo[4,5-b]pyridine, 1H-imidazo[4,5-c]pyridine, 2H-imidazo[1,2-a]pyridine), imidazopyrazine (e.g., 1H-imidazo[4,5-b]pyrazine), pyrazolopyridine (e.g., 1H-pyrazolo[4,3-c]pyridine), thienopyrazole (e.g., 1H-thieno[2,3-c]pyrazole), pyrazolotriazine (e.g., pyrazolo[5,1-c][1,2,4]triazine), triazolopyrimidine (e.g., [1,2,4]triazolo[1,5-a]pyrimidine), phthalazine and the like;
and the like.

Examples of the 3- to 11-membered non-aromatic heterocycle include rings constituting the 3- to 11-membered non-aromatic heterocyclic groups, from among those exemplified as the above-mentioned "non-aromatic heterocyclic group" and "non-aromatic heterocyclyl-" used as the substituent.
Preferable examples of such non-aromatic heterocycle include
monocyclic nonaromatic heterocyclic groups such as pyrrolidine, piperidine, homopiperidine, tetrahydropyridine (e.g., 1,2,3,6-tetrahydropyridine), dihydropyridine (e.g., 1,2-dihydropyridine, 2,3-dihydropyridine), morpholine, thiomorpholine, 1,1-dioxidothiomorpholine, piperazine, hexamethylenimine, oxazolidine, thiazolidine, isothiazolidine, 1,1-dioxidoisothiazolidine, imidazolidine, oxazoline, thiazoline, imidazoline, dioxole (e.g., 1,3-dioxole),
dioxolane (e.g., 1,3-dioxolane), dihydrooxadiazole (e.g., 4,5-dihydro-1,2,4-oxadiazole), pyran, tetrahydropyran, thiopyran, tetrahydrothiopyran, 1-oxidotetrahydrothiopyran, 1,1-dioxidotetrahydrothiopyran, tetrahydrofuran,
tetrahydrothiophene, 1,1-dioxidotetrahydrothiophene, pyrazolidine, pyrazoline, tetrahydropyrimidine, dihydrotriazole (e.g., 2,3-dihydro-1H-1,2,3-triazole), tetrahydrotriazole (e.g., 2,3,4,5-tetrahydro-1H-1,2,3-triazole), dihydrooxadiazole (e.g., 4,5-dihydro-1,2,4-oxadiazole), thiazine (e.g., 1,4-thiazine), 1,1-dioxidothiazinane (e.g., 1,1-dioxido-1,2-thiazinane), dihydropyridazine (e.g., 1,6-dihydropyridazine), tetrahydropyridazine (e.g., 1,4,5,6-tetrahydropyridazine), dihydrothioxazine (e.g., 2,3-dihydro-1,4-thioxazine), dihydrothiazine (e.g., 3,4-dihydro-2H-1,4-thiazine) and the like;
non-aromatic fused heterocycles such as dihydroindole (e.g., 2,3-dihydro-1H-indole), dihydroisoindole (e.g., 2,3-dihydro-1H-isoindole, 1,3-dihydro-2H-isoindole), dihydrobenzofuran (e.g., 2,3-dihydro-1-benzofuran), dihydrobenzodioxine (e.g., 2,3-dihydro-1,4-benzodioxine), dihydrobenzodioxepine (e.g., 3,4-dihydro-2H-1,5-benzodioxepine), tetrahydrobenzofuran (e.g., 4,5,6,7-tetrahydro-1-benzofuran), chromene (e.g., 4H-chromene, 2H-chromene), dihydroquinoline (e.g., 1,2-dihydroquinoline), tetrahydroquinoline (e.g., 1,2,3,4-tetrahydroquinoline), dihydroisoquinoline (e.g., 1,2-dihydroisoquinoline), tetrahydroisoquinoline (e.g., 1,2,3,4-tetrahydroisoquinoline), dihydrophthalazine (e.g., 3,4-dihydrophthalazine, 1,4-dihydrophthalazine), tetrahydrobenzoazepine (e.g., 2,3,4,5-tetrahydro-1H-benzo[c]azepine), benzodioxole (e.g., 1,3-benzodioxole), benzothiazine (e.g., 3,4-dihydro-2H-1,4-benzothiazine), 2,2-dioxido-2-thia-3-azabicyclo[3.1.0]hexane, 1,3-diazaspiro[4.4]nonane and the like;
and the like.

Unless particularly limited, examples of the "3- to 10-membered saturated hydrocarbon ring" in the present specification include cyclopropane, cyclobutane, cyclopentane, cyclohexane, cycloheptane, cyclooctane, bicyclo[2.2.1]heptane, bicyclo[2.2.2]octane, bicyclo[3.2.1]octane, bicyclo[3.2.2]nonane, bicyclo[3.3.1]nonane, bicyclo[4.2.1]nonane, bicyclo[4.3.1]decane, adamantane and the like.
Unless particularly limited, examples of the "3- to 6-membered saturated hydrocarbon ring" in the present specification include cyclopropane, cyclobutane, cyclopentane, cyclohexane and the like.

Unless particularly limited, examples of the "3- to 10-membered hydrocarbon ring" in the present specification include the above-mentioned "3- to 10-membered saturated hydrocarbon ring" and a 3- to 10-membered unsaturated hydrocarbon ring.
Here, examples of the 3- to 10-membered unsaturated hydrocarbon ring include a 6- to 10-membered aromatic hydrocarbon ring, $C_{3-10}$ cycloalkene, $C_{3-10}$ cycloalkyne and the like.
Examples of the 6- to 10-membered aromatic hydrocarbon ring include benzene, naphthalene and the like.
Examples of the $C_{3-10}$ cycloalkene include cyclopropene, cyclobutene, cyclopentene, cyclohexene, cycloheptene, cyclooctene, cyclodecene, 1,3-cyclopentadiene, 1,3-cyclohexadiene, 1,4-cyclohexadiene and the like.
Examples of the $C_{3-10}$ cycloalkyne include cyclopropyne, cyclobutyne, cyclopentyne, cyclohexyne, cycloheptyne, cyclooctyne, cyclodecyne and the like.
The above-mentioned $C_{3-10}$ cycloalkene and $C_{3-10}$ cycloalkyne may be each fused with a benzene ring. Examples of such fused ring include indane, dihydronaphthalene, tetrahydronaphthalene and the like.

Unless particularly limited, examples of the "3- to 10-membered hydrocarbon ring group" in the present specification include the above-mentioned "$C_{6-10}$ aryl group", "$C_{3-10}$ cycloalkyl group", "$C_{3-10}$ cycloalkenyl group", "$C_{4-10}$ cycloalkadienyl group" and the like.

The definition of each symbol in the formula (I) is explained in detail in the following.

The "$C_{1-6}$ alkyl group" of the "$C_{1-6}$ alkyl group optionally having substituent(s)" for $R^1$, $R^2$, $R^3$ or $R^4$ may have 1 to 3 (preferably 1 or 2) substituents at substitutable position(s).
Examples of such substituent include

(1) a $C_{3-10}$ cycloalkyl group (e.g., cyclopropyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group, and
(d) a halogen atom;

(2) a $C_{6-14}$ aryl group (e.g., phenyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group,
(d) a halogen atom, and
(e) an amino group optionally mono- or di-substituted by a $C_{1-6}$ alkyl group (e.g., dimethylamino);

(3) an aromatic heterocyclic group (e.g., thienyl, furyl, pyridyl, oxazolyl, thiazolyl, tetrazolyl, oxadiazolyl, pyrazinyl, quinolyl, indolyl, pyrimidinyl, imidazolyl, pyrazolyl, thiadiazolyl, isoxazolyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group and
(d) a halogen atom;

(4) a nonaromatic heterocyclic group (e.g., tetrahydrofuryl, morpholinyl, thiomorpholinyl, piperidinyl (including piperidino), pyrrolidinyl, piperazinyl, dioxolyl, dioxolanyl, 1,3-dihydro-2-benzofuranyl, thiazolidinyl, 1,1-dioxido-1,2-thiazinan-2-yl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group,
(d) a halogen atom and
(e) an oxo group;

(5) an amino group optionally mono- or di-substituted by substituent(s) selected from

(a) a $C_{1-6}$ alkyl group optionally substituted by 1 to 3 substituents selected from

(i) a $C_{1-6}$ alkoxy group,
(ii) a $C_{6-14}$ aryloxy group (e.g., phenoxy),
(iii) a carboxyl group,
(iv) a $C_{1-6}$ alkoxy-carbonyl group,
(v) a $C_{1-6}$ alkyl-carbonyl group (e.g., acetyl) and
(vi) a hydroxy group,

(b) a $C_{7-13}$ aralkyloxy group (e.g., benzyloxy),
(c) a $C_{1-6}$ alkyl-carbonyl group (e.g., acetyl),
(d) a $C_{3-10}$ cycloalkyl-carbonyl group (e.g., cyclopropylcarbonyl, cyclopentylcarbonyl, cyclohexylcarbonyl),
(e) a $C_{1-6}$ alkoxy-carbonyl group,
(f) a $C_{6-14}$ aryl-carbonyl group (e.g., benzoyl) optionally substituted by 1 to 3 substituents selected from

(i) a $C_{1-6}$ alkyl group optionally substituted by 1 to 3 halogen atoms, and
(ii) a halogen atom,

(g) a $C_{7-13}$ aralkyl-carbonyl group (e.g., benzylcarbonyl, phenethylcarbonyl),
(h) an aromatic heterocyclyl-carbonyl group optionally substituted by 1 to 3 $C_{1-6}$ alkyl groups (e.g., thienylcarbonyl, furylcarbonyl, pyridylcarbonyl, oxazolylcarbonyl, thiazolylcarbonyl, tetrazolylcarbonyl, oxadiazolylcarbonyl, pyrazinylcarbonyl, quinolylcarbonyl, indolylcarbonyl, imidazolylcarbonyl, pyrazolylcarbonyl, thiadiazolylcarbonyl, isoxazolylcarbonyl),
(i) an aromatic heterocyclyl-carbamoyl group (e.g., thienylcarbamoyl, furylcarbamoyl, pyridylcarbamoyl, oxazolylcarbamoyl, thiazolylcarbamoyl, tetrazolylcarbamoyl, oxadiazolylcarbamoyl, pyrazinylcarbamoyl, quinolylcarbamoyl, indolylcarbamoyl, imidazolylcarbamoyl, pyrazolylcarbamoyl, thiadiazolylcarbamoyl, isoxazolylcarbamoyl),
(j) a $C_{1-6}$ alkyl-carbamoyl group (e.g., methylcarbamoyl, ethylcarbamoyl),
(k) a $C_{6-14}$ aryl-carbamoyl group (e.g., phenylcarbamoyl, 1-naphthylcarbamoyl, 2-naphthylcarbamoyl),
(1) a $C_{7-13}$ aralkyl-carbamoyl group (e.g., benzylcarbamoyl),
(m) a $C_{1-6}$ alkyl-sulfonyl group (e.g., methylsulfonyl, ethylsulfonyl, isopropylsulfonyl),
(n) a $C_{6-14}$ aryl-sulfonyl group (e.g., benzenesulfonyl, toluenesulfonyl, 1-naphthalenesulfonyl, 2-naphthalenesulfonyl), and
(o) a $C_{7-13}$ aralkyl-sulfonyl group (e.g., benzylsulfonyl);

(6) an amidino group;
(7) a hydroxy group;
(8) a $C_{1-6}$ alkoxy group optionally substituted by 1 to 3 substituents selected from

(a) a halogen atom,
(b) a carboxyl group,
(c) a $C_{1-6}$ alkoxy group and
(d) a $C_{1-6}$ alkoxy-carbonyl group;

(9) a $C_{2-6}$ alkenyloxy group (e.g., ethenyloxy) optionally substituted by 1 to 3 halogen atoms;
(10) a $C_{3-10}$ cycloalkyloxy group (e.g., cyclohexyloxy);
(11) a $C_{7-13}$ aralkyloxy group (e.g., benzyloxy);
(12) a $C_{6-10}$ aryloxy group (e.g., phenoxy, naphthyloxy) optionally substituted by 1 to 3 halogen atoms;
(13) a $C_{1-6}$ alkyl-carbonyloxy group (e.g., acetyloxy, tert-butylcarbonyloxy);
(14) a sulfanyl group;
(15) a $C_{1-6}$ alkylsulfanyl group (e.g., methylsulfanyl, ethylsulfanyl) optionally substituted by 1 to 3 halogen atoms;
(16) a $C_{7-13}$ aralkylsulfanyl group (e.g., benzylsulfanyl);
(17) a $C_{6-14}$ arylsulfanyl group (e.g., phenylsulfanyl, naphthylsulfanyl);
(18) a sulfo group;
(19) a cyano group;
(20) an azido group;
(21) a nitro group;
(22) a nitroso group;
(23) a halogen atom;
(24) a $C_{3-10}$ cycloalkyl-$C_{1-6}$ alkyloxy group (e.g., cyclopropylmethyloxy);
(25) a $C_{1-3}$ alkylenedioxy group (e.g., methylenedioxy, ethylenedioxy);
(26) a mono- or di-$C_{1-6}$ alkylphosphoryl group (e.g., diethylphosphoryl);
(27) an acyl group;
and the like. When the number of the substituents is not less than 2, the respective substituents may be the same or different.

Here, examples of the "acyl group" include groups represented by the formulas: $-COR^A$, $-CO-OR^A$, $-SO_2R^A$, $-SOR^A$, $-CO-NR^{A}{}_{,}R^{B'}$, $-SO_2-NR^{A'}R^{B'}$, $-SO_2-NR^{A'}(COR^{B'})$, $-CS-NR^{A'}R^{B'}$ [$R^A$ is a hydrogen atom, a hydrocarbon group optionally having substituent(s), or a heterocyclic group optionally having substituent(s); $R^{A'}$ is a hydrogen atom, a hydroxy group, a hydrocarbon group optionally having substituent(s), or a heterocyclic group optionally having substituent (s); $R^{B'}$ is a hydrogen atom, a hydrocarbon group optionally having substituent(s), or a heterocyclic group optionally having substituent(s); or $R^{A}{}_{,}$ and $R^{B'}$ form, together with the adjacent nitrogen atom, a nitrogen-containing heterocycle optionally having substituent(s)] and the like.
Examples of the "hydrocarbon group" of the "hydrocarbon group optionally having substituent(s)" for $R^{A'}$, $R^{A'}$ or $R^{B'}$ include a $C_{1-10}$ alkyl group, a $C_{2-10}$ alkenyl group, a $C_{2-10}$ alkynyl group, a $C_{3-10}$ cycloalkyl group, a $C_{3-10}$ cycloalkenyl group, a $C_4$-$_{10}$ cycloalkadienyl group, a $C_{6-14}$ aryl group, a $C_{7-13}$ aralkyl group, a $C_{8-13}$ arylalkenyl group, a $C_{3-10}$ cycloalkyl-

$C_{1-6}$ alkyl group and the like.

Here, examples of the $C_{8-13}$ arylalkenyl group include styryl and the like.

Here, examples of the $C_{3-10}$ cycloalkyl-$C_{1-6}$ alkyl group include cyclohexylmethyl and the like.

The $C_{1-10}$ alkyl group, $C_{2-10}$ alkenyl group and $C_{2-10}$ alkynyl group exemplified as the aforementioned "hydrocarbon group" may have 1 to 3 substituents at substitutable position(s).

Examples of such substituent include

(1) a $C_{3-10}$ cycloalkyl group optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group and
(d) a halogen atom;

(2) a $C_{6-14}$ aryl group (e.g., phenyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group and
(d) a halogen atom;

(3) an aromatic heterocyclic group (e.g., thienyl, furyl, pyridyl, oxazolyl, thiazolyl, tetrazolyl, oxadiazolyl, pyrazinyl, quinolyl, indolyl, pyrimidinyl, imidazolyl, pyrazolyl, thiadiazolyl, isoxazolyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group and
(d) a halogen atom;

(4) a nonaromatic heterocyclic group (e.g., tetrahydrofuryl, morpholinyl, thiomorpholinyl, piperidinyl, pyrrolidinyl, piperazinyl, dioxolyl, dioxolanyl, 1,3-dihydro-2-benzofuranyl, thiazolidinyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group,
(d) a halogen atom and
(e) an oxo group;

(5) an amino group optionally mono- or di-substituted by substituent(s) selected from

(a) a $C_{1-6}$ alkyl group optionally substituted by 1 to 3 substituents selected from

(i) a $C_{1-6}$ alkoxy group,
(ii) a $C_{6-14}$ aryloxy group (e.g., phenoxy),
(iii) a carboxyl group,
(iv) a $C_{1-6}$ alkoxy-carbonyl group,
(v) a $C_{1-6}$ alkyl-carbonyl group (e.g., acetyl) and
(vi) a hydroxy group,

(b) a $C_{7-13}$ aralkyloxy group (e.g., benzyloxy),
(c) a $C_{1-6}$ alkyl-carbonyl group (e.g., acetyl),
(d) a $C_{3-10}$ cycloalkyl-carbonyl group (e.g., cyclopropylcarbonyl, cyclopentylcarbonyl, cyclohexylcarbonyl),
(e) a $C_{1-6}$ alkoxy-carbonyl group,
(f) a $C_{6-14}$ aryl-carbonyl group (e.g., benzoyl) optionally substituted by 1 to 3 substituents selected from (i) a $C_{1-6}$ alkyl group optionally substituted by 1 to 3 halogen atoms and

(ii) a halogen atom,

(g) a $C_{7-13}$ aralkyl-carbonyl group (e.g., benzylcarbonyl, phenethylcarbonyl),
(h) an aromatic heterocyclyl-carbonyl group (e.g., thienylcarbonyl, furylcarbonyl, pyridylcarbonyl, oxazolylcarbonyl, thiazolylcarbonyl, tetrazolylcarbonyl, oxadiazolylcarbonyl, pyrazinylcarbonyl, quinolylcarbonyl, indolylcarbonyl, imidazolylcarbonyl, pyrazolylcarbonyl, thiadiazolylcarbonyl, isoxazolylcarbonyl) optionally substituted by 1 to 3 $C_{1-6}$ alkyl groups,
(i) an aromatic heterocyclyl-carbamoyl group (e.g., thienylcarbamoyl, furylcarbamoyl, pyridylcarbamoyl, oxazolylcarbamoyl, thiazolylcarbamoyl, tetrazolylcarbamoyl, oxadiazolylcarbamoyl, pyrazinylcarbamoyl, quinolylcarbamoyl, indolylcarbamoyl, imidazolylcarbamoyl, pyrazolylcarbamoyl, thiadiazolylcarbamoyl, isoxazolylcarbamoyl),
(j) a $C_{1-6}$ alkyl-carbamoyl group (e.g., methylcarbamoyl, ethylcarbamoyl),
(k) a $C_{6-14}$ aryl-carbamoyl group (e.g., phenylcarbamoyl, 1-naphthylcarbamoyl, 2-naphthylcarbamoyl),
(1) a $C_{7-13}$ aralkyl-carbamoyl group (e.g., benzylcarbamoyl),
(m) a $C_{1-6}$ alkyl-sulfonyl group (e.g., methylsulfonyl, ethylsulfonyl, isopropylsulfonyl),
(n) a $C_{6-14}$ aryl-sulfonyl group (e.g., benzenesulfonyl, toluenesulfonyl, 1-naphthalenesulfonyl, 2-naphthalenesulfonyl), and
(o) a $C_{7-13}$ aralkyl-sulfonyl group (e.g., benzylsulfonyl);

(6) an amidino group;
(7) a hydroxy group;
(8) a $C_{1-6}$ alkoxy group optionally substituted by 1 to 3 substituents selected from

(a) a halogen atom,
(b) a carboxyl group,
(c) a $C_{1-6}$ alkoxy group and
(d) a $C_{1-6}$ alkoxy-carbonyl group;

(9) a $C_{2-6}$ alkenyloxy group (e.g., ethenyloxy) optionally substituted by 1 to 3 halogen atoms;
(10) a $C_{3-10}$ cycloalkyloxy group (e.g., cyclohexyloxy);
(11) a $C_{7-13}$ aralkyloxy group (e.g., benzyloxy);
(12) a $C_{6-10}$ aryloxy group (e.g., phenoxy, naphthyloxy) optionally substituted by 1 to 3 halogen atoms;
(13) a $C_{1-6}$ alkyl-carbonyloxy group (e.g., acetyloxy, tert-butylcarbonyloxy);
(14) a sulfanyl group;
(15) a $C_{1-6}$ alkylsulfanyl group (e.g., methylsulfanyl, ethylsulfanyl) optionally substituted by 1 to 3 halogen atoms;
(16) a $C_{7-13}$ aralkylsulfanyl group (e.g., benzylsulfanyl);
(17) a $C_{6-14}$ arylsulfanyl group (e.g., phenylsulfanyl, naphthylsulfanyl);
(18) a sulfo group;
(19) a cyano group;
(20) an azido group;
(21) a nitro group;
(22) a nitroso group;
(23) a halogen atom;
(24) a $C_{3-10}$ cycloalkyl-$C_{1-6}$ alkyloxy group (e.g., cyclopropylmethyloxy);
(25) a $C_{1-3}$ alkylenedioxy group (e.g., methylenedioxy, ethylenedioxy);
(26) a mono- or di-$C_{1-6}$ alkylphosphoryl group (e.g., diethylphosphoryl);
(27) a $C_{1-6}$ alkyl-carbonyl group (e.g., acetyl) optionally substituted by 1 to 3 halogen atoms;
(28) a $C_{1-6}$ alkoxy-carbonyl group (e.g., methoxycarbonyl, tert-butoxycarbonyl) optionally substituted by 1 to 3 halogen atoms;
(29) a $C_{1-6}$ alkylsulfonyl group (e.g., methylsulfonyl, ethylsulfonyl) optionally substituted by 1 to 3 halogen atoms;
(30) a carbamoyl group optionally mono- or di-substituted by substituent(s) selected from

(a) a $C_{1-6}$ alkyl group optionally substituted by 1 to 3 substituents selected from

(i) a halogen atom,
(ii) a cyano group,
(iii) a hydroxy group and
(iv) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl),

(b) a $C_{6-14}$ aryl group (e.g., phenyl),
(c) a $C_{7-13}$ aralkyl group (e.g., benzyl) and
(d) an aromatic heterocyclyl-$C_{1-6}$ alkyl group (e.g., furfuryl);

(31) a thiocarbamoyl group optionally mono- or di-substituted by a $C_{1-6}$ alkyl group optionally substituted by 1 to 3 halogen atoms;
(32) a sulfamoyl group optionally mono- or di-substituted by a $C_{1-6}$ alkyl group optionally substituted by 1 to 3 substituents selected from a halogen atom and a nonaromatic heterocyclic group (e.g., tetrahydrofuryl);
(33) a carboxyl group;
(34) a $C_{1-6}$ alkylsulfinyl group (e.g., methylsulfinyl);
(35) a $C_{6-14}$ arylsulfonyl group (e.g., phenylsulfonyl) optionally substituted by 1 to 3 substituents selected from

(a) a halogen atom and
(b) a cyano group;

(36) an aromatic heterocyclyl-sulfonyl group (e.g., isoxazolylsulfonyl) optionally substituted by 1 to 3 $C_{1-6}$ alkyl groups;
(37) a non-aromatic heterocyclyl-sulfonyl group (e.g., piperidinylsulfonyl (including piperidinosulfonyl));
(38) a non-aromatic heterocyclyl-carbonyl group (e.g., tetrahydrofurylcarbonyl, morpholinylcarbonyl, thiomorpholinylcarbonyl, piperidinylcarbonyl, pyrrolidinylcarbonyl, piperazinylcarbonyl, dioxolylcarbonyl, dioxolanylcarbonyl, 1,3-dihydro-2-benzofuranylcarbonyl, thiazolidinylcarbonyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group and
(d) a halogen atom;

and the like. When the number of the substituents is not less than 2, the respective substituents may be the same or different.

The $C_{6-14}$ aryl group, $C_{7-13}$ aralkyl group and $C_{8-13}$ arylalkenyl group, exemplified as the aforementioned "hydrocarbon group", optionally have 1 to 3 substituents at substitutable position(s).

Examples of such substituent include

(1) groups exemplified as the substituent that the aforementioned $C_{1-10}$ alkyl group and the like optionally have;
(2) a $C_{1-6}$ alkyl group optionally substituted by 1 to 3 substituents selected from

(a) a halogen atom,
(b) a carboxyl group,
(c) a hydroxy group optionally substituted by a nonaromatic heterocyclic group (e.g., piperidino, tetrahydropyranyl),
(d) an amino group optionally mono- or di-substituted by a $C_{1-6}$ alkyl group,
(e) a $C_{6-10}$ aryloxy group (e.g., phenoxy) optionally substituted by a $C_{1-6}$ alkoxy group,
(f) a $C_{1-6}$ alkoxy-carbonyl group,
(g) a $C_{1-6}$ alkyl-carbonyloxy group (e.g., acetyloxy, tert-butylcarbonyloxy),
(h) a carbamoyl group,
(i) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl) optionally substituted by 1 - 3 $C_{1-6}$ alkyl groups optionally substituted by 1 to 3 hydroxy groups (e.g., hydroxymethyl), and
(j) a nonaromatic heterocyclic group (e.g., piperidino, tetrahydropyranyl);

(3) a $C_{2-6}$ alkenyl group (e.g., ethenyl, 1-propenyl) optionally substituted by 1 to 3 substituents selected from

(a) a halogen atom,
(b) a carboxyl group,
(c) a $C_{1-6}$ alkoxy-carbonyl group, and

(d) a carbamoyl group;

(4) a $C_{2-6}$ alkenyl group (e.g., ethynyl, 1-propynyl) optionally substituted by 1 to 3 substituents selected from

(a) a halogen atom,
(b) a carboxyl group,
(c) a $C_{1-6}$ alkoxy-carbonyl group, and
(d) a carbamoyl group;

(5) a $C_{7-13}$ aralkyl group (e.g., benzyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group, and
(d) a halogen atom;

and the like can be mentioned. When the number of the substituents is not less than 2, the respective substituents may be the same or different.

In addition, the $C_{3-10}$ cycloalkyl group, $C_{3-10}$ cycloalkenyl group, $C_{4-10}$ cycloalkadienyl group and $C_{3-10}$ cycloalkyl-$C_{1-6}$ alkyl group, exemplified as the aforementioned "hydrocarbon group", optionally have 1 to 3 substituents at substitutable position(s).
Examples of such substituent include

(1) groups exemplified as the substituent that the aforementioned $C_{6-14}$ aryl group and the like optionally have;
(2) an oxo group;
and the like. When the number of the substituents is not less than 2, the respective substituents may be the same or different.

Examples of the "heterocyclic group" of the "heterocyclic group optionally having substituent(s)" for $R^A$, $R^{A'}$ or $R^{B'}$ include an aromatic heterocyclic group and a nonaromatic heterocyclic group.
The "aromatic heterocyclic group" and "nonaromatic heterocyclic group" optionally have 1 to 3 substituents at substitutable position(s).
Here, examples of the substituent of the aromatic heterocyclic group include those exemplified as the substituents that the $C_{6-14}$ aryl exemplified as the "hydrocarbon group" of the aforementioned "hydrocarbon group optionally having substituent(s)" and the like optionally has. When the number of the substituents is not less than 2, the respective substituents may be the same or different.
In addition, examples of the substituent of the nonaromatic heterocyclic group include those exemplified as the substituent that the $C_{3-10}$ cycloalkyl group exemplified as the "hydrocarbon group" of the aforementioned "hydrocarbon group optionally having substituent(s)" and the like optionally has. When the number of the substituents is not less than 2, the respective substituents may be the same or different.

Examples of the "nitrogen-containing heterocycle" of the "nitrogen-containing heterocycle optionally having substituent(s)" formed by $R^{A'}$ and $R^{B'}$ together with the adjacent nitrogen atom include a 5- to 7-membered nitrogen-containing heterocycle containing, as a ring constituting atom besides carbon atom, at least one nitrogen atom and further optionally containing 1 or 2 hetero atoms selected from an oxygen atom, a sulfur atom (said sulfur atom is optionally oxidized) and a nitrogen atom. Preferable examples of the nitrogen-containing heterocycle include pyrrolidine, imidazolidine, pyrazolidine, piperidine, piperazine, morpholine, thiomorpholine, oxopiperazine and the like.
The nitrogen-containing heterocycle optionally has 1 to 3 (preferably 1 or 2) substituents at substitutable position(s). Examples of such substituent include those exemplified as the substituent that the $C_{3-10}$ cycloalkyl group exemplified as the "hydrocarbon group" of the aforementioned "hydrocarbon group optionally having substituent(s)" and the like optionally has. When the number of the substituents is not less than 2, the respective substituents may be the same or different.

Specific examples of the aforementioned "acyl group" include

(1) a formyl group,
(2) a $C_{1-6}$ alkyl-carbonyl group optionally having substituent(s),
(3) a $C_{2-6}$ alkenyl-carbonyl group optionally having substituent(s),
(4) a $C_{2-6}$ alkynyl-carbonyl group optionally having substituent(s),
(5) a $C_{3-6}$ cycloalkyl-carbonyl group optionally having substituent(s),

(6) a $C_{3-6}$ cycloalkenyl-carbonyl group optionally having substituent(s),
(7) a $C_{6-10}$ aryl-carbonyl group optionally having substituent(s),
(8) a heterocyclyl-carbonyl group optionally having substituent(s),
(9) a carboxyl group,
(10) a $C_{1-6}$ alkoxy-carbonyl group optionally having substituent(s),
(11) a $C_{2-6}$ alkenyloxy-carbonyl group optionally having substituent(s),
(12) a $C_{2-6}$ alkynyloxy-carbonyl group optionally having substituent(s),
(13) a $C_{3-6}$ cycloalkyloxy-carbonyl group optionally having substituent(s),
(14) a $C_{3-6}$ cycloalkenyloxy-carbonyl group optionally having substituent(s),
(15) a $C_{6-10}$ aryloxy-carbonyl group optionally having substituent(s),
(16) a heterocyclyl-oxycarbonyl group optionally having substituent(s),
(17) a $C_{1-6}$ alkylsulfinyl group optionally having substituent(s),
(18) a $C_{1-6}$ alkylsulfonyl group optionally having substituent(s),
(19) a $C_{6-14}$ arylsulfonyl group optionally having substituent(s),
(20) a heterocyclyl-sulfonyl group optionally having substituent(s),
(21) a carbamoyl group optionally having substituent(s),
(22) a thiocarbamoyl group optionally having substituent(s),
(23) a sulfamoyl group optionally having substituent(s), and the like.

Here, examples of the "$C_{2-6}$ alkenyl-carbonyl group" of the "$C_{2-6}$ alkenyl-carbonyl group optionally having substituent(s)" include ethenylcarbonyl, 1-propenylcarbonyl, 2-propenylcarbonyl, 2-methyl-1-propenylcarbonyl, 1-butenylcarbonyl, 2-butenylcarbonyl, 3-butenylcarbonyl, 3-methyl-2-butenylcarbonyl, 1-pentenylcarbonyl, 2-pentenylcarbonyl, 3-pentenylcarbonyl, 4-pentenylcarbonyl, 4-methyl-3-pentenylcarbonyl, 1-hexenylcarbonyl, 2-hexenylcarbonyl, 3-hexenylcarbonyl, 4-hexenylcarbonyl, 5-hexenylcarbonyl and the like.
Examples of the "$C_{2-6}$ alkynyl-carbonyl group" of the "$C_{2-6}$ alkynyl-carbonyl group optionally having substituent(s)" include ethynylcarbonyl, 1-propynylcarbonyl, 2-propynylcarbonyl, 1-butynylcarbonyl, 2-butynylcarbonyl, 3-butynylcarbonyl, 1-pentynylcarbonyl, 2-pentynylcarbonyl, 3-pentynylcarbonyl, 4-pentynylcarbonyl, 1-hexynylcarbonyl, 2-hexynylcarbonyl, 3-hexynylcarbonyl, 4-hexynylcarbonyl, 5-hexynylcarbonyl and the like.
Examples of the "$C_{3-6}$ cycloalkyl-carbonyl group" of the "$C_{3-6}$ cycloalkyl-carbonyl group optionally having substituent(s)" include cyclopropylcarbonyl, cyclobutylcarbonyl, cyclopentylcarbonyl, cyclohexylcarbonyl and the like.
Examples of the "$C_{3-6}$ cycloalkenyl-carbonyl group" of the "$C_{3-6}$ cycloalkenyl-carbonyl group optionally having substituent (s)" include 2-cyclopropen-1-ylcarbonyl, 2-cyclobuten-1-ylcarbonyl, 2-cyclopenten-1-ylcarbonyl, 3-cyclopenten-1-ylcarbonyl, 2-cyclohexen-1-ylcarbonyl, 3-cyclohexen-1-ylcarbonyl and the like.
Examples of the "$C_{6-10}$ aryl-carbonyl group" of the "$C_{6-10}$ aryl-carbonyl group optionally having substituent(s)" include benzoyl, 1-naphthoyl, 2-naphthoyl and the like.
Examples of the "heterocyclyl-carbonyl group" of the "heterocyclyl-carbonyl group optionally having substituent(s)" include (1) a 5- or 6-membered monocyclic aromatic heterocycle (e.g., furan, thiophene, pyrrole, oxazole, isoxazole, thiazole, isothiazole, imidazole, pyridine, pyrazole etc.)-carbonyl, (2) a 8- to 12-membered fused aromatic heterocycle (e.g., benzofuran, isobenzofuran, benzothiophene, isobenzothiophene, indole, isoindole, 1H-indazole, benzimidazole, benzoxazole etc.)-carbonyl, (3) a 3- to 6-membered non-aromatic heterocycle (e.g., oxirane, azetidine, oxetane, pyrrolidine, tetrahydrofuran, thiolane, piperidine etc.)-carbonyl and the like.
Examples of the "$C_{2-6}$ alkenyloxy-carbonyl group" of the "$C_{2-6}$ alkenyloxy-carbonyl group optionally having substituent(s)" include ethenyloxycarbonyl, 1-propenyloxycarbonyl, 2-propenyloxycarbonyl, 1-butenyloxycarbonyl, 2-butenyloxycarbonyl, 3-butenyloxycarbonyl, 3-methyl-2-butenyloxycarbonyl, 1-pentenyloxycarbonyl, 2-pentenyloxycarbonyl, 3-pentenyloxycarbonyl, 4-pentenyloxycarbonyl, 1-hexenyloxycarbonyl, 2-hexenyloxycarbonyl, 3-hexenyloxycarbonyl, 4-hexenyloxycarbonyl, 5-hexenyloxycarbonyl and the like.
Examples of the "$C_{2-6}$ alkynyloxy-carbonyl group" of the "$C_{2-6}$ alkynyloxy-carbonyl group optionally having substituent (s)" include ethynyloxycarbonyl, 1-propynyloxycarbonyl, 2-propynyloxycarbonyl, 1-butynyloxycarbonyl, 2-butynyloxycarbonyl, 3-butynyloxycarbonyl, 1-pentynyloxycarbonyl, 2-pentynyloxycarbonyl, 3-pentynyloxycarbonyl, 4-pentynyloxycarbonyl, 1-hexynyloxycarbonyl, 2-hexynyloxycarbonyl, 3-hexynyloxycarbonyl, 4-hexynyloxycarbonyl, 5-hexynyloxycarbonyl and the like.
Examples of the "$C_{3-6}$ cycloalkyloxy-carbonyl group" of the "$C_{3-6}$ cycloalkyloxy-carbonyl group optionally having substituent(s)" include cyclopropyloxycarbonyl, cyclobutyloxycarbonyl, cyclopentyloxycarbonyl, cyclohexyloxycarbonyl and the like.
Examples of the "$C_{3-6}$ cycloalkenyloxy-carbonyl group" of the "$C_{3-6}$ cycloalkenyloxy-carbonyl group optionally having substituent(s)" include 2-cyclopropen-1-yloxycarbonyl, 2-cyclobuten-1-yloxycarbonyl, 2-cyclopenten-1-yloxycarbonyl, 3-cyclopenten-1-yloxycarbonyl, 2-cyclohexen-1-yloxycarbonyl, 3-cyclohexen-1-yloxycarbonyl and the like.
Examples of the "$C_{6-10}$ aryloxy-carbonyl group" of the "$C_{6-10}$ aryloxy-carbonyl group optionally having substituent(s)" include phenoxycarbonyl, 1-naphthyloxycarbonyl, 2-naphthyloxycarbonyl and the like.
Examples of the "heterocyclyl-oxycarbonyl group" of the "heterocyclyl-oxycarbonyl group optionally having substituent (s)" include (1) a 5- or 6-membered monocyclic aromatic heterocycle (e.g., furan, thiophene, pyrrole, oxazole, isoxazole, thiazole, isothiazole, imidazole, pyridine, pyrazole etc.)-oxycarbonyl, (2) a 8- to 12-membered fused aromatic heterocycle (e.g., benzofuran, isobenzofuran, benzothiophene, isobenzothiophene, indole, isoindole, 1H-indazole, benzimidazole, benzoxazole etc.)-oxycarbonyl, (3) a 3- to 6-membered non-aromatic heterocycle (e.g., oxirane, azetidine, oxetane, pyrrolidine, tetrahydrofuran, thiolane, piperidine etc.)-oxycarbonyl and the like.

Examples of the "$C_{1-6}$ alkylsulfinyl group" of the "$C_{1-6}$ alkylsulfinyl group optionally having substituent(s)" include methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, isopentylsulfinyl, neopentylsulfinyl, 1-ethylpropylsulfinyl, 1,1-dimethylpropylsulfinyl, hexylsulfinyl, isohexylsulfinyl, 1,1-dimethylbutylsulfinyl, 2,2-dimethylbutylsulfinyl, 3,3-dimethylbutylsulfinyl, 2-ethylbutylsulfinyl and the like.
Examples of the "$C_{1-6}$ alkylsulfonyl group" of the "$C_{1-6}$ alkylsulfonyl group optionally having substituent(s)" include methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, 1-ethylpropylsulfonyl, 1,1-dimethylpropylsulfonyl, hexylsulfonyl, isohexylsulfonyl, 1,1-dimethylbutylsulfonyl, 2,2-dimethylbutylsulfonyl, 3,3-dimethylbutylsulfonyl, 2-ethylbutylsulfonyl and the like.

Examples of the "$C_{6-14}$ arylsulfonyl group" of the "$C_{6-14}$ arylsulfonyl group optionally having substituent(s)" include phenylsulfonyl, 1-naphthylsulfonyl, 2-naphthylsulfonyl and the like.

Examples of the "heterocyclyl-sulfonyl group" of the "heterocyclyl-sulfonyl group optionally having substituent (s)" include (1) a 5- or 6-membered monocyclic aromatic heterocycle (e.g., furan, thiophene, pyrrole, oxazole, isoxazole, thiazole, isothiazole, imidazole, pyridine, pyrazole etc.)-sulfonyl, (2) a 8- to 12-membered fused aromatic heterocycle (e.g., benzofuran, isobenzofuran, benzothiophene, isobenzothiophene, indole, isoindole, 1H-indazole, benzimidazole, benzoxazole etc.)-sulfonyl, (3) a 3- to 6-membered non-aromatic heterocycle (e.g., oxirane, azetidine, oxetane, pyrrolidine, tetrahydrofuran, thiolane, piperidine etc.)-sulfonyl and the like.

These $C_{1-6}$ alkyl-carbonyl group, $C_{2-6}$ alkenyl-carbonyl group, $C_{2-6}$ alkynyl-carbonyl group, $C_{3-6}$ cycloalkyl-carbonyl group, $C_{3-6}$ cycloalkenyl-carbonyl group, $C_{6-10}$ aryl-carbonyl group, heterocyclyl-carbonyl group, $C_{1-6}$ alkoxy-carbonyl group, $C_{2-6}$ alkenyloxy-carbonyl group, $C_{2-6}$ alkynyloxy-carbonyl group, $C_{3-6}$ cycloalkyloxy-carbonyl group, $C_{3-6}$ cycloalkenyloxy-carbonyl group, $C_{6-10}$ aryloxy-carbonyl group, heterocyclyl-oxycarbonyl group, $C_{1-6}$ alkylsulfinyl group, $C_{1-6}$ alkylsulfonyl group, $C_{6-14}$ arylsulfonyl group and heterocyclyl-sulfonyl group each optionally have 1 to 3 substituents at substitutable position(s). When the number of the substituents is not less than 2, the respective substituents may be the same or different.
Here, examples of the substituent of the $C_{1-6}$ alkyl-carbonyl group, $C_{2-6}$ alkenyl-carbonyl group, $C_{2-6}$ alkynyl-carbonyl group, $C_{1-6}$ alkoxy-carbonyl group, $C_{2-6}$ alkenyloxy-carbonyl group, $C_{2-6}$ alkynyloxy-carbonyl group, $C_{1-6}$ alkylsulfinyl group and $C_{1-6}$ alkylsulfonyl group include those exemplified as the substituent that the $C_{1-10}$ alkyl group and the like exemplified as the "hydrocarbon group" of the aforementioned "hydrocarbon group optionally having substituent(s)" for $R^A$ and the like optionally has. When the number of the substituents is not less than 2, the respective substituents may be the same or different.
In addition, examples of the substituent of the $C_{3-6}$ cycloalkyl-carbonyl group, $C_{3-6}$ cycloalkenyl-carbonyl group, heterocyclyl-carbonyl group, $C_{3-6}$ cycloalkyloxy-carbonyl group, $C_{3-6}$ cycloalkenyloxy-carbonyl group, heterocyclyl-oxycarbonyl group and heterocyclyl-sulfonyl group include those exemplified as the substituent that the $C_{3-10}$ cycloalkyl group and the like exemplified as the "hydrocarbon group" of the aforementioned "hydrocarbon group optionally having substituent(s)" for $R^A$ and the like optionally have (excluding an oxo group when the heterocycle of the heterocyclyl-carbonyl group, heterocyclyl-oxycarbonyl group and heterocyclyl-sulfonyl group are aromatic heterocycles). When the number of the substituents is not less than 2, the respective substituents may be the same or different.
In addition, examples of the substituent of the $C_{6-10}$ aryl-carbonyl group, $C_{6-10}$ aryloxy-carbonyl group and $C_{6-14}$ arylsulfonyl group include those exemplified as the substituent that the $C_{6-14}$ aryl and the like exemplified as the "hydrocarbon group" of the aforementioned "hydrocarbon group optionally having substituent(s)" for $R^A$ and the like optionally has. When the number of the substituents is not less than 2, the respective substituents may be the same or different.

Examples of the "carbamoyl group optionally having substituent(s)", "thiocarbamoyl group optionally having substituent(s)" and "sulfamoyl group optionally having substituent(s)" include a carbamoyl group, a thiocarbamoyl group and a sulfamoyl group, each optionally mono- or di-substituted by substituent(s) selected from, for example, a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group, a heterocyclic group and the like, each of which optionally has substituent(s).
Here, as the $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group and $C_{8-13}$ arylalkenyl group, those exemplified as the "hydrocarbon group" of the aforementioned "hydrocarbon group optionally having substituent(s)" for $R^A$ and the like can be mentioned.
As the heterocyclic group, an "aromatic heterocyclic group" and a "nonaromatic heterocyclic group" can be mentioned.

These $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group, aromatic heterocyclic group and nonaromatic heterocyclic group each optionally have 1 to 3 substituents at substitutable position(s). When the number of the substituents is not less than 2, the respective substituents may be the same or different.
Here, examples of the substituent of the $C_{1-10}$ alkyl group and $C_{2-10}$ alkenyl group include those exemplified as the substituent that the $C_{1-10}$ alkyl group and the like exemplified as the "hydrocarbon group" of the aforementioned "hydrocarbon group optionally having substituent(s)" for $R^A$ and the like optionally have.
In addition, examples of the substituent of the $C_{3-10}$ cycloalkyl group, $C_{3-10}$ cycloalkenyl group and nonaromatic heterocyclic group include those exemplified as the substituent that the $C_{3-10}$ cycloalkyl group and the like exemplified as the "hydrocarbon group" of the aforementioned "hydrocarbon group optionally having substituent(s)" for $R^A$ and the like optionally have.
In addition, examples of the substituent of the $C_{6-14}$ aryl group, $C_{7-13}$ aralkyl group, $C_{8-13}$ arylalkenyl group and aromatic heterocyclic group include those exemplified as the substituent that the $C_{6-14}$ aryl group and the like exemplified as the "hydrocarbon group" of the aforementioned "hydrocarbon group optionally having substituent(s)" for $R^A$ and the like optionally have.

The aforementioned "acyl group" is preferably

(1) a $C_{1-6}$ alkyl-carbonyl group (e.g., acetyl) optionally substituted by 1 to 3 halogen atoms;
(2) a $C_{1-6}$ alkoxy-carbonyl group (e.g., methoxycarbonyl, ethoxycarbonyl, tert-butoxycarbonyl) optionally substituted by 1 to 3 halogen atoms;
(3) a $C_{1-6}$ alkylsulfonyl group (e.g., methylsulfonyl, ethylsulfonyl) optionally substituted by 1 to 3 halogen atoms;
(4) a carbamoyl group optionally mono- or di-substituted by substituent(s) selected from

(a) a $C_{1-6}$ alkyl group optionally substituted by 1 to 3 substituents selected from

(i) a halogen atom,
(ii) a cyano group,
(iii) a hydroxy group and
(iv) an amino group optionally mono- or di-substituted by a $C_{1-6}$ alkyl group,
(v) a $C_{1-6}$ alkoxy group, and
(vi) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl),

(b) a $C_{6-14}$ aryl group (e.g., phenyl),
(c) a $C_{7-13}$ aralkyl group (e.g., benzyl) and
(d) an aromatic heterocyclyl-$C_{1-6}$ alkyl group (e.g., furfuryl);

(5) a thiocarbamoyl group optionally mono- or di-substituted by a $C_{1-6}$ alkyl group optionally substituted by 1 to 3 halogen atoms;
(6) a sulfamoyl group optionally mono- or di-substituted by a $C_{1-6}$ alkyl group optionally substituted by 1 to 3 substituents selected from a halogen atom and a nonaromatic heterocyclic group (e.g., tetrahydrofuryl);
(7) a carboxyl group;
(8) a $C_{1-6}$ alkylsulfinyl group (e.g., methylsulfinyl);
(9) a $C_{6-14}$ arylsulfonyl group (e.g., phenylsulfonyl) optionally substituted by 1 to 3 substituents selected from

(a) a halogen atom and
(b) a cyano group;

(10) an aromatic heterocyclyl-sulfonyl group (e.g., isoxazolylsulfonyl) optionally substituted by 1 to 3 $C_{1-6}$ alkyl groups;
(11) a non-aromatic heterocyclyl-sulfonyl group (e.g., piperidinylsulfonyl (including piperidinosulfonyl));
(12) a non-aromatic heterocyclyl-carbonyl group (e.g., tetrahydrofurylcarbonyl, morpholinylcarbonyl, thiomorpholinylcarbonyl, piperidinylcarbonyl, pyrrolidinylcarbonyl, piperazinylcarbonyl, dioxolylcarbonyl, dioxolanylcarbonyl, 1,3-dihydro-2-benzofuranylcarbonyl, thiazolidinylcarbonyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group and
(d) a halogen atom;

and the like.

The "$C_{3-6}$ cycloalkyl group" of the "$C_{3-6}$ cycloalkyl group optionally having substituent(s)" for $R^1$, $R^2$ or $R^3$ optionally has 1 to 3 (preferably 1 or 2, more preferably 1) substituents at substitutable position(s). Examples of such substituent include

(1) groups exemplified as the substituent that the "$C_{1-6}$ alkyl group" of the aforementioned "$C_{1-6}$ alkyl group optionally having substituent(s)" for $R^1$ and the like optionally have;
(2) a $C_{1-6}$ alkyl group optionally substituted by 1 to 3 substituents selected from

(a) a halogen atom,
(b) a carboxyl group,
(c) a hydroxy group optionally substituted by a nonaromatic heterocyclic group (e.g., piperidino, tetrahydropyranyl),
(d) an amino group optionally mono- or di-substituted by a $C_{1-6}$ alkyl group,
(e) a $C_{6-10}$ aryloxy group (e.g., phenoxy) optionally substituted by a $C_{1-6}$ alkoxy group,
(f) a $C_{1-6}$ alkoxy-carbonyl group,
(g) a $C_{1-6}$ alkyl-carbonyloxy group (e.g., acetyloxy, tert-butylcarbonyloxy),
(h) a carbamoyl group,
(i) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl) optionally substituted by 1 to 3 $C_{1-6}$ alkyl groups,
(j) a cyano group,
(k) a $C_{1-6}$ alkoxy group,
(l) a $C_{1-6}$ alkylsulfonyl group (e.g., methylsulfonyl), and
(m) a nonaromatic heterocyclic group (e.g., morpholino, pyrrolidinyl, piperidino, tetrahydropyranyl) optionally substituted by 1 to 3 oxo groups;

(3) a $C_{2-6}$ alkenyl group (e.g., ethenyl, 1-propenyl) optionally substituted by 1 to 3 substituents selected from

(a) a halogen atom,
(b) a carboxyl group,
(c) a $C_{1-6}$ alkoxy-carbonyl group, and
(d) a carbamoyl group;

(4) a $C_{2-6}$ alkenyl group (e.g., ethynyl, 1-propynyl) optionally substituted by 1 to 3 substituents selected from

(a) a halogen atom,
(b) a carboxyl group,
(c) a $C_{1-6}$ alkoxy-carbonyl group, and
(d) a carbamoyl group;

(5) a $C_{7-13}$ aralkyl group (e.g., benzyl) optionally substituted by 1 to 3 substituents selected from

(a) a $C_{1-6}$ alkyl group optionally substituted by 1 to 3 halogen atoms,
(b) a hydroxy group,
(c) a $C_{1-6}$ alkoxy group, and
(d) a halogen atom;

(6) an oxo group; and the like can be mentioned. When the number of the substituents is not less than 2, the respective substituents may be the same or different.

The "$C_{1-6}$ alkoxy group" of the "$C_{1-6}$ alkoxy group optionally having substituent(s)" for $R^1$ or $R^2$ optionally has 1 to 3 (preferably 1 or 2) substituents at substitutable position(s).
Examples of such substituent include groups exemplified as the substituent that the "$C_{1-6}$ alkyl group" of the aforementioned "$C_{1-6}$ alkyl group optionally having substituent(s)" for $R^1$ and the like optionally has. When the number of the substituents is not less than 2, the respective substituents may be the same or different.
Examples of the "3- to 6-membered saturated hydrocarbon ring" formed by $R^1$ and $R^2$ together with the adjacent carbon atom include cyclopropane, cyclobutane, cyclopentane, cyclohexane and the like. The "3- to 6-membered saturated hydrocarbon ring" optionally has 1 - 3 (preferably 1 or 2) substituents at substitutable position(s).
Examples of such substituent include groups exemplified as the substituent that the "$C_{3-6}$ cycloalkyl group" of the aforementioned "$C_{3-6}$ cycloalkyl group optionally having substituent(s)" for $R^1$ and the like optionally has. When the number of the substituents is not less than 2, the respective substituents may be the same or different.

The "$C_{2-6}$ alkenyl group" and "$C_{2-6}$ alkynyl group" of the "$C_{2-6}$ alkenyl group optionally having substituent(s)" and "$C_{2-6}$ alkynyl group optionally having substituent(s)" for $R^3$ optionally have 1 to 3 (preferably 1 or 2, more preferably 1) substituents at substitutable position(s).
Examples of such substituent include those similar to the substituents that the "$C_{1-6}$ alkyl group" of the aforementioned "$C_{1-6}$ alkyl group optionally having substituent(s)" for $R^1$ and the like optionally has. When the number of the substituents is not less than 2, the respective substituents may be the same or different.
Examples of the "acyl group" for $R^3$ include those similar to the "acyl group" exemplified as the substituent that the "$C_{1-6}$ alkyl group" of the aforementioned "$C_{1-6}$ alkyl group optionally having substituent(s)" for $R^1$ and the like optionally have.

Examples of the "3- to 10-membered hydrocarbon ring group" of the "3- to 10-membered hydrocarbon ring group optionally having substituent(s)" for $R^4$ include a $C_{6-10}$ aryl group, a $C_{3-10}$ cycloalkyl group, a $C_{3-10}$ cycloalkenyl group, a $C_{4-10}$ cycloalkadienyl group and the like.
These $C_{6-10}$ aryl group, $C_{3-10}$ cycloalkyl group, $C_{3-10}$ cycloalkenyl group and $C_{4-10}$ cycloalkadienyl group optionally have 1 to 3 (preferably 1 or 2) substituents at substitutable position(s). When the number of the substituents is not less than 2, the respective substituents may be the same or different.
Here, examples of the substituent of the $C_{3-10}$ cycloalkyl group, $C_{3-10}$ cycloalkenyl group and $C_{4-10}$ cycloalkadienyl group include those exemplified as the substituent that the "$C_{3-6}$ cycloalkyl group" of the aforementioned "$C_{3-3}$ cycloalkyl group optionally having substituent(s)" for $R^1$ and the like optionally have.
In addition, examples of the substituent of the $C_{6-10}$ aryl group include

(1) groups exemplified as the substituent that the "$C_{1-6}$ alkyl group" of the aforementioned "$C_{1-6}$ alkyl group optionally having substituent(s)" for $R^1$ and the like optionally have;
(2) a $C_{1-6}$ alkyl group optionally substituted by 1 to 3 substituents selected from

(a) a halogen atom,
(b) a carboxyl group,
(c) a hydroxy group optionally substituted by a nonaromatic heterocyclic group (e.g., piperidino, tetrahydropyranyl),
(d) an amino group optionally mono- or di-substituted by a $C_{1-6}$ alkyl group,
(e) a $C_{6-10}$ aryloxy group (e.g., phenoxy) optionally substituted by a $C_{1-6}$ alkoxy group,
(f) a $C_{1-6}$ alkoxy-carbonyl group,
(g) a $C_{1-6}$ alkyl-carbonyloxy group (e.g., acetyloxy, tert-butylcarbonyloxy),
(h) a carbamoyl group,
(i) an aromatic heterocyclic group (e.g., thienyl, furyl, pyridyl, oxazolyl, thiazolyl, tetrazolyl, oxadiazolyl, pyrazinyl, quinolyl, indolyl, imidazolyl, pyrazolyl, thiadiazolyl, isoxazolyl) optionally substituted by 1 - 3 $C_{1-6}$ alkyl groups optionally substituted by 1 to 3 hydroxy groups (e.g., hydroxymethyl), and
(j) a nonaromatic heterocyclic group (e.g., piperidino, tetrahydropyranyl);

(3) a $C_{2-6}$ alkenyl group (e.g., ethenyl, 1-propenyl) optionally substituted by 1 to 3 substituents selected from

(a) a halogen atom,
(b) a carboxyl group,
(c) a $C_{1-6}$ alkoxy-carbonyl group, and
(d) a carbamoyl group;

(4) a $C_{2-6}$ alkynyl group (e.g., ethynyl, 1-propynyl) optionally substituted by 1 to 3 substituents selected from

(a) a halogen atom,
(b) a carboxyl group,
(c) a $C_{1-6}$ alkoxy-carbonyl group, and
(d) a carbamoyl group;

(5) a $C_{7-13}$ aralkyl group (e.g., benzyl) optionally substituted by 1 to 3 substituents selected from

(a) a $C_{1-6}$ alkyl group optionally-substituted by 1 to 3 halogen atoms,

(b) a hydroxy group,
(c) a $C_{1-6}$ alkoxy group, and
(d) a halogen atom;

and the like can be mentioned.

Examples of the "3- to 10-membered hydrocarbon ring" of the "3- to 10-membered hydrocarbon ring optionally having substituent(s)" for ring A or ring B include a 3- to 10-membered saturated hydrocarbon ring, a 6- to 10-membered aromatic heterocycle, $C_{3-10}$ cycloalkene, $C_{3-10}$ cycloalkyne and the like.

These 3- to 10-membered saturated hydrocarbon ring, 6- to 10-membered aromatic hydrocarbon ring, $C_{3-10}$ cycloalkene and $C_{3-10}$ cycloalkyne optionally have 1 to 3 (preferably 1 or 2) substituents at substitutable position(s). When the number of the substituents is not less than 2, the respective substituents may be the same or different.

Here, examples of the substituent of the 3 - 10-membered saturated hydrocarbon ring, $C_{3-10}$ cycloalkene and $C_{3-10}$ cycloalkyne include those exemplified as the substituent that the "$C_{3-6}$ cycloalkyl group" of the aforementioned "$C_{3-6}$ cycloalkyl group optionally having substituent(s)" for $R^1$ and the like optionally have.

In addition, examples of the substituent of the 6- to 10-membered aromatic hydrocarbon ring include those exemplified as the substituent that the $C_{6-10}$ aryl group exemplified as the "3- to 10-membered hydrocarbon ring group" of the aforementioned "3- to 10-membered hydrocarbon ring group optionally having substituent(s)" for $R^4$ optionally have.

Examples of the "3- to 11-membered heterocycle" of the "3- to 11-membered heterocycle optionally having substituent(s)" for ring B include 3- to 11-membered aromatic heterocycle and non-aromatic heterocycle.

These aromatic heterocycle and non-aromatic heterocycle optionally have 1 to 3 (preferably 1 or 2) substituents at substitutable position(s). When the number of the substituents is not less than 2, the respective substituents may be the same or different.

Here, examples of the substituent of the 3- to 11-membered non-aromatic heterocycle include those exemplified as the substituent that the "$C_{3-6}$ cycloalkyl group" of the aforementioned "$C_{3-6}$ cycloalkyl group optionally having substituent (s)" for $R^1$ and the like optionally have.

In addition, examples of the substituent of the 3- to 11-membered aromatic heterocycle include those exemplified as the substituent that the $C_{6-10}$ aryl group exemplified as the "3- to 10-membered hydrocarbon ring group" of the aforementioned "3-to 10-membered hydrocarbon ring group optionally having substituent(s)" for $R^4$ optionally have.

Examples of the "$C_{1-6}$ alkylene group" of the "$C_{1-6}$ alkylene group optionally having substituent(s)" for X include methylene, $-(CH_2)_2-$, $-(CH_2)_3-$, $-(CH_2)_4-$, $-(CH_2)_5-$, $-(CH_2)_6-$, $-CH(CH_3)-$, $-CH(C_2H_5)-$, $-CH(C_3H_7)-$, $-CH(i-C_3H_7)-$, $-CH(CH_3)-CH_2-$, $-CH_2CH(CH_3)-$, $-CH(CH_3)(CH_2)_2-$, $-(CH_2)_2CH(CH_3)-$, $-CH_2-CH(CH_3)-CH_2-$, $-C(CH_3)_2-$, $-C(CH_3)(C_2H_5)-$, $-CH(CH_3)-CH(CH_3)-$, $-CH_2-C(CH_3)_2-$ and the like.

The "$C_{1-6}$ alkylene group" optionally has 1 to 3 (preferably 1 or 2) substituents at substitutable position(s).

Examples of such substituents include those similar to the substituent that the "$C_{1-6}$ alkyl group" of the aforementioned "$C_{1-6}$ alkyl group optionally having substituent(s)" for $R^1$ and the like optionally has. When the number of the substituents is not less than 2, the respective substituents may be the same or different.

Examples of the "$C_{2-6}$ alkenylene group" of the "$C_{2-6}$ alkenylene group optionally having substituent(s)" for X include vinylene, $-CH=CH-CH_2-$, $-CH_2-CH=CH-$, $-C(CH3)_2-CH=CH-$, $-CH_2-CH=CH-CH_2-$, $-CH_2-CH_2-CH=CH-$, $-CH=CH-CH=CH-$, $-C(CH_3)=CH-$, $-CH=C(CH_3)-$, $-CH=C(C_2H_5)-$, $-CH=CH-(CH_2)_3-$, $-CH_2-CH=CH-(CH_2)_2-$, $-(CH_2)_2-CH=CH-CH_2-$, $-(CH_2)_3-CH=CH-$ and the like.

The "$C_{2-6}$ alkenylene group" optionally has 1 to 3 (preferably 1 or 2) substituents at substitutable position(s).

Examples of such substituent include those similar to the substituent that the "$C_{1-6}$ alkyl group" of the aforementioned "$C_{1-6}$ alkyl group optionally having substituent(s)" for $R^1$ and the like optionally has. When the number of the substituents is not less than 2, the respective substituents may be the same or different.

$R^1$ and $R^2$ are the same or different and each is a hydrogen atom, a hydroxy group, a $C_{1-6}$ alkyl group optionally having substituent(s), a $C_{3-6}$ cycloalkyl group optionally having substituent(s), or a $C_{1-6}$ alkoxy group optionally having substituent(s), or may form, together with the adjacent carbon atom, a 3- to 6-membered saturated hydrocarbon ring.

Preferably, $R^1$ and $R^2$ are the same or different and each is a hydrogen atom, a hydroxy group or a $C_{1-6}$ alkyl group optionally having substituent(s), or $R^1$ and $R^2$ may form, together with the adjacent carbon atom, a 3- to 6-membered saturated hydrocarbon ring.

More preferably, $R^1$ and $R^2$ are the same or different and each is a hydrogen atom, a hydroxy group or a $C_{1-6}$ alkyl group (preferably, methyl), or $R^1$ and $R^2$ may form, together with the adjacent carbon atom, a 3- to 6-membered saturated hydrocarbon ring (preferably, cyclopropane).

$R^3$ is a hydrogen atom, a halogen atom, a $C_{1-6}$ alkyl group optionally having substituent(s), a $C_{2-6}$ alkenyl group optionally having substituent(s), a $C_{2-6}$ alkynyl group optionally having substituent(s), a $C_{3-6}$ cycloalkyl group optionally having substituent(s) or an acyl group.

The "$C_{1-6}$ alkyl group optionally having substituent(s)" for $R^3$ is preferably a $C_{1-6}$ alkyl group (preferably, methyl, ethyl, propyl, isopropyl, butyl) optionally substituted by 1 to 3 substituents selected from a halogen atom, a hydroxy group and

a $C_{1-6}$ alkoxy group (preferably, methoxy).
The "$C_{2-6}$ alkenyl group optionally having substituent(s)" for $R^3$ is preferably a $C_{2-6}$ alkenyl group (preferably, ethenyl).
The "acyl group" for $R^3$ is preferably a $C_{1-6}$ alkyl-carbonyl group (preferably, acetyl) optionally having substituent(s), a $C_{1-6}$ alkoxy-carbonyl group (preferably, ethoxycarbonyl) optionally having substituent(s), more preferably, a $C_{1-6}$ alkyl-carbonyl group (preferably, acetyl) or a $C_{1-6}$ alkoxy-carbonyl group (preferably, ethoxycarbonyl).

$R^3$ is preferably a hydrogen atom, a $C_{1-6}$ alkyl group optionally having substituent(s), a $C_{2-6}$ alkenyl group optionally having substituent(s), an acyl group (preferably, a $C_{1-6}$ alkyl-carbonyl group optionally having substituent(s), or a $C_{1-6}$ alkoxy-carbonyl group optionally having substituent(s)), more preferably,

(1) a hydrogen atom,
(2) a $C_{1-6}$ alkyl group (preferably, methyl, ethyl, propyl, isopropyl, butyl) optionally substituted by 1 to 3 substituents selected from a halogen atom, a hydroxy group and a $C_{1-6}$ alkoxy group (preferably, methoxy),
(3) a $C_{2-6}$ alkenyl group (preferably, ethenyl),
(4) a $C_{1-6}$ alkyl-carbonyl group (preferably, acetyl), or
(5) a $C_{1-6}$ alkoxy-carbonyl group (preferably, ethoxycarbonyl).

$R^4$ is a hydrogen atom, a $C_{1-6}$ alkyl group optionally having substituent(s) or a $C_{3-10}$ cycloalkyl group optionally having substituent(s). The "$C_{1-6}$ alkyl group optionally having substituent(s)" for $R^4$ is preferably

(1) a methyl group optionally having substituent(s) selected from a halogen atom, a cyano group, a hydroxy group, a $C_{1-6}$ alkoxy group optionally having substituent(s), a $C_{6-10}$ aryloxy group optionally having substituent(s), a sulfanyl group optionally having substituent(s), an amino group optionally having substituent(s), an acyl group, a $C_{3-10}$ cycloalkyl group optionally having substituent(s) and a 3- to 11-membered heterocyclic group optionally having substituent(s), or
(2) a $C_{2-6}$ alkyl group optionally having substituent(s), more preferably methyl.

Here, examples of the "$C_{1-6}$ alkoxy group optionally having substituent(s)" as the substituent that the aforementioned "methyl group" optionally has include those similar to the aforementioned "$C_{1-6}$ alkoxy group optionally having substituent(s)" for $R^1$ and the like.

In addition, the "$C_{6-10}$ aryloxy group optionally having substituent(s)" as the substituent that the aforementioned methyl group optionally has, optionally has 1 to 3 (preferably 1 or 2) substituents at substitutable position(s).

Examples of such substituent include those exemplified as the substituent that the $C_{6-10}$ aryl group exemplified as the "3-to 10-membered hydrocarbon ring group" of the aforementioned "3- to 10-membered hydrocarbon ring group optionally having substituent(s)" for $R^4$ optionally has.

In addition, examples of the "sulfanyl group optionally having substituent(s)" as the substituent that the aforementioned methyl group optionally has include a sulfanyl group optionally substituted by substituent(s) selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group, a heterocyclic group, and the like, each of which optionally has substituent(s).
Here, examples of the "$C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group, heterocyclic group, and the like, each of which optionally has substituent(s)" include those similar to the "$C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group, heterocyclic group, each of which optionally has substituent(s), and the like" exemplified as the substituent that the carbamoyl group optionally having substituent(s)" and the like exemplified as specific examples of the aforementioned "acyl group" for $R^3$ optionally has.
In addition, examples of the "amino group optionally having substituent(s)" as the substituent that the aforementioned methyl group optionally has include an amino group optionally mono- or di-substituted by substituent(s) selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group, a heterocyclic group and the like, each of which optionally has substituent(s).
Here, examples of the "$C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group, heterocyclic group, and the like, each of which optionally has substituent(s)" include those similar to the "$C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group, heterocyclic group, each of which optionally has substituent(s), and the like" exemplified as the substituent that the "carbamoyl group optionally having substituent(s)" and the like as specific examples of the aforementioned "acyl group" for $R^3$ optionally has.

In addition, examples of the "acyl group" as the substituent that the aforementioned methyl group optionally has include those similar to the aforementioned "acyl group" for $R^3$.
In addition, the "$C_{3-10}$ cycloalkyl group" of the "$C_{3-10}$ cycloalkyl group optionally having substituent(s)" as the substituent that the aforementioned methyl group optionally has optionally has 1 to 3 (preferably 1 or 2) substituents at substitutable

position(s).
Examples of such substituents include those exemplified as the substituent that the "$C_{3-6}$ cycloalkyl group" of the aforementioned "$C_{3-6}$ cycloalkyl group optionally having substituent(s)" for $R^1$ and the like optionally has.
In addition, examples of the"3- to 11-membered heterocyclic group optionally having substituent(s)" as the substituent that the aforementioned methyl group optionally has include a 3- to 11-membered aromatic heterocyclic group and a nonaromatic heterocyclic group.
These aromatic heterocyclic group and nonaromatic heterocyclic group optionally have 1 to 3 (preferably 1 or 2) substituents at substitutable position(s). When the number of the substituents is not less than 2, the respective substituents may be the same or different.
Here, examples of the substituent of the 3- to 11-membered nonaromatic heterocyclic group include those exemplified as the substituent that the "$C_{3-6}$ cycloalkyl group" of the aforementioned "$C_{3-6}$ cycloalkyl group optionally having substituent(s)" for $R^1$ and the like optionally has.
In addition, examples of the substituent of the 3- to 11-membered aromatic heterocyclic group include those exemplified as the substituent that the $C_{6-10}$ aryl group exemplified as the "3- to 10-membered hydrocarbon ring group" of the aforementioned "3- to 10-membered hydrocarbon ring group optionally having substituent(s)" for the $R^4$ optionally has.
Here, the "$C_{2-6}$ alkyl group" of the aforementioned "$C_{2-6}$ alkyl group optionally having substituent(s)" optionally has 1 to 3 (preferably 1 or 2) substituents at substitutable position(s).
Examples of such substituent include those exemplified as the substituent that the "$C_{1-6}$ alkyl group" of the aforementioned "$C_{1-6}$ alkyl group optionally having substituent(s)" for $R^1$ and the like optionally has.
$R^4$ is preferably

(1) a hydrogen atom,
(2) a $C_{3-10}$ cycloalkyl group optionally having substituent(s),
(3) a methyl group optionally having substituent(s) selected from a halogen atom, a cyano group, a hydroxy group, a $C_{1-6}$ alkoxy group optionally having substituent(s), a $C_{6-10}$ aryloxy group optionally having substituent(s), a sulfanyl group optionally having substituent(s), an amino group optionally having substituent(s), an acyl group, a $C_{3-10}$ cycloalkyl group optionally having substituent(s) and a 3- to 11-membered heterocyclic group optionally having substituent(s), or
(4) a $C_{2-6}$ alkyl group optionally having substituent(s).

In another embodiment, $R^4$ is preferably a hydrogen atom or a $C_{1-6}$ alkyl group (preferably, methyl).
Ring A is a 3- to 10-membered hydrocarbon ring optionally having substituent(s).
Ring A is preferably a 3- to 10-membered unsaturated hydrocarbon ring optionally having substituent(s), more preferably a 6- to 10-membered aromatic hydrocarbon ring (preferably, benzene) optionally having substituent(s).
Ring A is more preferably a 6- to 10-membered aromatic hydrocarbon ring (preferably, benzene, naphthalene) optionally substituted by 1 - 3 (preferably 1 or 2) substituents selected from (i) a halogen atom (preferably, a fluorine atom, a chlorine atom, a bromine atom), (ii) a $C_{1-6}$ alkyl group (preferably, methyl) optionally substituted by 1 to 3 halogen atoms (preferably, fluorine atom), (iii) a nitro group, (iv) a $C_{1-6}$ alkoxy group (preferably, methoxy), (v) a hydroxy group, (vi) a $C_{3-10}$ cycloalkyl group (preferably, cyclopropyl) and (vii) $C_{1-6}$ alkylsulfonyl(preferably, methylsulfonyl).
Ring B is a 3- to 10-membered hydrocarbon ring optionally having substituent(s) or a 3- to 11-membered heterocycle optionally having substituent(s).
As ring B, a 6- to 10-membered aromatic hydrocarbon ring (preferably, benzene) optionally having substituent(s), a 3- to 10-membered saturated hydrocarbon ring (preferably, cyclopropane, cyclohexane) optionally having substituent(s), or a 3- to 11-membered heterocycle optionally having substituent(s) is preferable.
Ring B is more preferably

(1) benzene having at least one substituent selected from a halogen atom, a cyano group, a $C_{1-6}$ alkyl group optionally having substituent(s), a $C_{2-6}$ alkenyl group optionally having substituent(s), a $C_{2-6}$ alkynyl group optionally having substituent(s), a sulfanyl group optionally having substituent(s), an amino group optionally having substituent(s), an acyl group, a 3- to 10-membered hydrocarbon ring group optionally having substituent(s) and a 3- to 11-membered heterocyclic group optionally having substituent(s),
(2) a 3- to 10-membered saturated hydrocarbon ring optionally having substituent(s) selected from a halogen atom, a cyano group, a hydroxy group, a $C_{1-6}$ alkyl group optionally having substituent(s), a $C_{2-6}$ alkenyl group optionally having substituent(s), a $C_{2-6}$ alkynyl group optionally having substituent(s), a $C_{1-6}$ alkoxy group optionally having substituent(s), a $C_{6-10}$ aryloxy group optionally having substituent(s), a sulfanyl group optionally having substituent (s), an amino group optionally having substituent (s), an acyl group, a $C_{3-10}$ cycloalkyl group optionally having substituent(s) and a 3- to 11-membered nitrogen-containing heterocyclic group optionally having substituent(s), or
(3) a 3- to 11-membered heterocycle optionally having substituent(s),

or a salt thereof.

Here, examples of the "$C_{1-6}$ alkyl group optionally having substituent(s)" as the substituent that the aforementioned "benzene" or "3- to 10-membered saturated hydrocarbon ring" optionally has include those similar to the aforementioned "$C_{1-6}$ alkyl group optionally having substituent(s)" for $R^1$ and the like.

In addition, the "$C_{2-6}$ alkenyl group optionally having substituent(s)" and "$C_{2-6}$ alkynyl group optionally having substituent (s)" as the substituent that the aforementioned "benzene" or "3- to 10-membered saturated hydrocarbon ring" optionally has include those similar to each of the aforementioned "$C_{2-6}$ alkenyl group optionally having substituent(s)" and "$C_{2-6}$ alkynyl group optionally having substituent(s)" for $R^3$.

In addition, examples of the "sulfanyl group optionally having substituent(s)", "amino group optionally having substituent(s)", "acyl group" and "3- to 11-membered heterocyclic group optionally having substituent(s)" as the substituent that the aforementioned benzene optionally has include those similar to each of the "sulfanyl group optionally having substituent(s)", "amino group optionally having substituent(s)", "acyl group" and "3- to 11-membered heterocyclic group optionally having substituent(s)" as the substituent that the methyl group exemplified as preferable embodiment of the aforementioned "$C_{1-6}$ alkyl group optionally having substituent(s)" for $R^4$ optionally has.

In addition, examples of the "3- to 10-membered hydrocarbon ring group optionally having substituent(s)" as the substituent that the aforementioned benzene optionally has include those similar to the "3- to 10-membered hydrocarbon ring group optionally having substituent(s)" for $R^4$.

In addition, examples of the "$C_{1-6}$ alkoxy group optionally having substituent(s)", "$C_{6-10}$ aryloxy group optionally having substituent(s)", "sulfanyl group optionally having substituent(s)", "amino group optionally having substituent(s)", "acyl group" and "$C_{3-10}$ cycloalkyl group optionally having substituent(s)" as the substituent that the aforementioned 3- to 10-membered saturated hydrocarbon ring optionally has include those similar to each of the "$C_{1-6}$ alkoxy group optionally having substituent(s)", "$C_{6-10}$ aryloxy group optionally having substituent(s)", "sulfanyl group optionally having substituent(s)", "amino group optionally having substituent(s)", "acyl group" and "$C_{3-10}$ cycloalkyl group optionally having substituent(s)" as the substituent that the methyl group exemplified as preferable embodiment of the aforementioned "$C_{1-6}$ alkyl group optionally having substituent(s)" for $R^4$ optionally has.

In addition, examples of the "3- to 11-membered nitrogen-containing heterocyclic group" of the "3- to 11-membered nitrogen-containing heterocyclic group optionally having substituent(s)" as the substituent that the aforementioned 3-to 10-membered saturated hydrocarbon ring optionally has include a 3- to 11-membered nitrogen-containing aromatic heterocyclic group and a nitrogen-containing nonaromatic heterocyclic group.

These 3- to 11-membered nitrogen-containing aromatic heterocyclic group and nitrogen-containing nonaromatic heterocyclic group may have 1 to 3 (preferably 1 or 2) substituents at substitutable position(s). When the number of the substituents is not less than 2, the respective substituents may be the same or different.

Here, examples of the substituent of the 3- to 11-membered nitrogen-containing nonaromatic heterocyclic group include those exemplified as the substituent that the "$C_{3-6}$ cycloalkyl group" of the aforementioned "$C_{3-6}$ cycloalkyl group optionally having substituent(s)" for $R^1$ and the like optionally has.

In addition, examples of the substituent of the 3- to 11-membered nitrogen-containing aromatic heterocyclic group include those exemplified as the substituent that the "3- to 10-membered hydrocarbon ring group" of the aforementioned "3-to 10-membered hydrocarbon ring group optionally having substituent(s)" for $R^4$ optionally has.

Ring B is more preferably

(1) benzene having at least one substituent selected from a halogen atom, a sulfanyl group optionally having substituent(s), an amino group optionally having substituent(s), an acyl group (preferably, a sulfamoyl group optionally having substituent(s), a $C_{1-6}$ alkylsulfinyl group optionally having substituent(s), a $C_{1-6}$ alkylsulfonyl group optionally having substituent(s)), and a 3- to 11-membered heterocyclic group optionally having substituent(s) (preferably, a 3- to 11-membered nonaromatic heterocyclic group optionally having substituent(s)),

(2) a 3- to 10-membered saturated hydrocarbon ring (preferably, cyclopropane, cyclopentane) optionally having 1 to 3 substituents selected from a cyano group and an acyl group (preferably, a $C_{6-14}$ arylsulfonyl group optionally having substituent(s), a $C_{1-6}$ alkylsulfonyl group optionally having substituent(s), a $C_{1-6}$ alkyl-carbonyl group optionally having substituent(s), a $C_{1-6}$ alkoxy-carbonyl group optionally having substituent(s), a carboxyl group, a carbamoyl group optionally having substituent(s)),

(3) a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole, 1,2,5-oxadiazole, benzimidazole, benzotriazole, benzofuran, tetrazole, 1H-thieno[2,3-c]pyrazole, [1,2,4]triazolo[1,5-a]pyrimidine) optionally substituted by 1 to 3 substituents selected from a halogen atom, a $C_{1-6}$ alkyl group optionally having substituent(s), a hydroxy group, a $C_{1-6}$ alkoxy group optionally having substituent(s), a $C_{6-10}$ aryl group optionally having substituent(s), a $C_{3-10}$ cycloalkyl group optionally having substituent(s), a 3- to 11-membered heterocyclic group optionally having substituent(s), an amino group optionally having substituent(s), a $C_{1-6}$ alkylsulfanyl group optionally having substituent(s), a $C_{6-10}$ aryloxy group optionally having substituent(s), a nitro group,

and an acyl group (preferably, a sulfamoyl group optionally having substituent(s), a $C_{1-6}$ alkylsulfonyl group optionally having substituent(s), a $C_{1-6}$ alkylsulfinyl group optionally having substituent(s), a $C_{1-6}$ alkoxy-carbonyl group optionally having substituent(s), a carbamoyl group optionally having substituent(s), a non-aromatic heterocyclyl-sulfonyl group optionally having substituent(s)),
(4) a 3- to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxy-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydro-1-benzofuran, 1,4,5,6-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane), optionally having 1 to 3 substituents selected from a $C_{1-6}$ alkyl group optionally having substituent(s), a hydroxy group, an amino group optionally having substituent(s), a $C_{1-6}$ alkoxy group optionally having substituent(s), a $C_{6-10}$ aryl group optionally having substituent(s), a $C_{7-13}$ aralkyl group optionally having substituent(s), an acyl group (preferably, a $C_{1-6}$ alkyl-carbonyl group optionally having substituent(s), a $C_{1-6}$ alkoxy-carbonyl group optionally having substituent(s), a $C_{1-6}$ alkylsulfonyl group optionally having substituent(s), a $C_{6-14}$ arylsulfonyl group optionally having substituent(s), a heterocyclyl-sulfonyl group optionally having substituent(s) (preferably,
an aromatic heterocyclyl-sulfonyl group optionally having substituent(s)), a carbamoyl group optionally having substituent(s)), and an oxo group,
particularly preferably

(1) benzene having at least one substituent selected from

(a) a halogen atom,
(b) an amino group optionally mono- or di-substituted by substituent(s) selected from

(i) a $C_{1-6}$ alkyl group (preferably, methyl), and
(ii) $C_{1-6}$ alkyl-carbonyl (preferably, acetyl),

(c) a sulfamoyl group optionally mono- or di-substituted by a $C_{1-6}$ alkyl group (preferably, methyl) optionally substituted by a nonaromatic heterocyclic group (preferably, tetrahydrofuryl),
(d) a $C_{1-6}$ alkylsulfanyl group (preferably, methylsulfanyl),
(e) a $C_{1-6}$ alkylsulfonyl group (preferably, methylsulfonyl),
(f) a $C_{1-6}$ alkylsulfinyl group (preferably, methylsulfinyl), and
(g) a nonaromatic heterocyclic group (preferably, pyrrolidinyl, 1,1-dioxido-1,2-thiazinan-2-yl) optionally substituted by 1 to 3 oxo groups,

(2) a 3- to 10-membered saturated hydrocarbon ring (preferably, cyclopropane, cyclopentane) optionally substituted by 1 to 3 substituents selected from

(a) a cyano group,
(b) $C_{6-14}$ arylsulfonyl (preferably, phenylsulfonyl) optionally substituted by 1 to 3 halogen atoms,
(c) a $C_{1-6}$ alkylsulfonyl group (preferably, methylsulfonyl),
(d) a $C_{1-6}$ alkyl-carbonyl group (preferably, acetyl),
(e) a $C_{1-6}$ alkoxy-carbonyl group (preferably, methoxycarbonyl, ethoxycarbonyl),
(f) a carboxyl group, and
(g) a carbamoyl group optionally mono- or di-substituted by a $C_{1-6}$ alkyl group (preferably, ethyl) optionally substituted by a $C_{1-6}$ alkoxy group (preferably, methoxy),

(3) a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole, 1,2,5-oxadiazole, benzimidazole, benzotriazole, benzofuran, tetrazole, 1H-thieno[2,3-c]pyrazole, [1,2,4]triazolo[1,5-a]pyrimidine) optionally substituted by 1 to 3 substituents selected from

(a) a $C_{1-6}$ alkyl group (preferably, methyl, ethyl, propyl, isopropyl, isobutyl, tert-butyl) optionally substituted by 1 to 3 substituents selected from

(i) a halogen atom,

(ii) a $C_{6-10}$ aryloxy group (preferably, phenoxy) optionally substituted by a $C_{1-6}$ alkoxy group (preferably, methoxy),
(iii) a nonaromatic heterocyclic group (preferably, morpholino, pyrrolidinyl) optionally substituted by 1 to 3 oxo groups,
(iv) an aromatic heterocyclic group (preferably, pyrazolyl, imidazolyl) optionally substituted by 1 to 3 $C_{1-6}$ alkyl groups (preferably, methyl),
(v) a hydroxy group,
(vi) an amino group optionally mono- or di-substituted by a $C_{1-6}$ alkyl group (preferably, methyl),
(vii) a $C_{1-6}$ alkoxy group (preferably, methoxy),
(viii) a cyano group,
(ix) a carbamoyl group, and
(x) a $C_{1-6}$ alkylsulfonyl group (preferably, methylsulfonyl),

(b) a $C_{6-14}$ aryl group (preferably, phenyl) optionally substituted by 1 to 3 halogen atoms,
(c) an amino group optionally mono- or di-substituted by a $C_{1-6}$ alkyl-carbonyl group (preferably, acetyl),
(d) a sulfamoyl group optionally mono- or di-substituted by a $C_{1-6}$ alkyl group (preferably, methyl),
(e) a $C_{1-6}$ alkylsulfanyl group (preferably, methylsulfanyl),
(f) a $C_{1-6}$ alkylsulfonyl group (preferably, methylsulfonyl),
(g) a $C_{1-6}$ alkylsulfinyl group (preferably, methylsulfinyl),
(h) a halogen atom,
(i) a $C_{3-10}$ cycloalkyl group (preferably, cyclopropyl),
(j) an aromatic heterocyclic group (preferably, furyl, thienyl, pyrazolyl),
(k) a nonaromatic heterocyclic group (preferably, piperidino),
(l) a non-aromatic heterocyclyl-sulfonyl group (preferably, piperidinosulfonyl),
(m) a $C_{6-10}$ aryloxy group (preferably, phenoxy) optionally substituted by 1 to 3 halogen atoms,
(n) a nitro group,
(o) a $C_{1-6}$ alkoxy-carbonyl group (preferably, ethoxycarbonyl),
(p) a $C_{1-6}$ alkoxy group (preferably, methoxy),
(q) a carbamoyl group optionally mono- or di-substituted by a $C_{1-6}$ alkyl group (preferably, ethyl) optionally substituted by substituents selected from

(i) a hydroxy group, and
(ii) an amino group optionally mono- or di-substituted by a $C_{1-6}$ alkyl group (preferably, methyl), (4) a 3- to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxy-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydro-1-benzofuran, 1,4,5,6-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane) optionally substituted by 1 to 3 substituents selected from

(a) a $C_{1-6}$ alkyl group (preferably, methyl, ethyl, isopropyl) optionally substituted by an aromatic heterocyclic group (preferably, thienyl),
(b) a $C_{1-6}$ alkoxy group (preferably, methoxy),
(c) a $C_{1-6}$ alkoxy-carbonyl group (preferably, methoxycarbonyl, tert-butoxycarbonyl),
(d) a $C_{6-14}$ aryl group (preferably, phenyl) optionally substituted by substituents selected from

(i) a $C_{1-6}$ alkoxy group (preferably, methoxy), and
(ii) an amino group optionally mono- or di-substituted by a $C_{1-6}$ alkyl group (preferably, methyl),

(e) a $C_{7-13}$ aralkyl group (preferably, benzyl) optionally substituted by 1 to 3 substituents selected from a $C_{1-6}$ alkyl group (preferably, methyl) optionally substituted by 1 to 3 halogen atoms,
(f) a $C_{6-14}$ arylsulfonyl group (preferably, phenylsulfonyl) optionally substituted by 1 to 3 halogen atoms,
(g) a $C_{1-6}$ alkyl-carbonyl group (preferably, acetyl),
(h) a $C_{1-6}$ alkylsulfonyl group (preferably, methylsulfonyl ethylsulfonyl) optionally substituted by 1 to 3 halogen atoms),
(i) a carbamoyl group optionally mono- or di-substituted by a $C_{1-6}$ alkyl group (preferably, ethyl),

(j) an oxo group, and
(k) an aromatic heterocyclyl-sulfonyl group (preferably, isooxazolylsulfonyl) optionally substituted by 1 to 3 $C_{1-6}$ alkyl groups (preferably, methyl).

In other embodiment, ring B is preferably

(1) a 3- to 10-membered hydrocarbon ring (preferably, a 3- to 10-membered unsaturated hydrocarbon ring [preferably, benzene, indane (preferably, 2,3-dihydro-1H-inden)], a 3- to 10-membered saturated hydrocarbon ring (preferably, cyclopropane, cyclopentane)] optionally having 1 to 3 substituents selected from

(a) a nonaromatic heterocyclic group (preferably, pyrrolidinyl, 1,1-dioxido-1,2-thiazinan-2-yl) optionally substituted by 1 to 3 oxo groups,
(b) an amino group optionally mono- or di-substituted by substituent(s) selected from a $C_{1-6}$ alkyl group (preferably, methyl) and a $C_{1-6}$ alkyl-carbonyl group (preferably, acetyl),
(c) a $C_{1-6}$ alkoxy group,
(d) a $C_{1-6}$ alkylsulfanyl group (preferably, methylsulfanyl),
(e) a cyano group,
(f) a halogen atom,
(g) an acyl group {preferably, sulfamoyl optionally having substituent(s) [preferably, a sulfamoyl group optionally mono- or di-substituted by a $C_{1-6}$ alkyl group (preferably, methyl) optionally substituted by a nonaromatic heterocyclic group (preferably, tetrahydrofuryl)], a $C_{1-6}$ alkylsulfonyl group (preferably, methylsulfonyl), a $C_{1-6}$ alkylsulfinyl group (preferably, methylsulfinyl), a $C_{6-14}$ arylsulfonyl group optionally having substituent(s) [preferably, a $C_{6-14}$ arylsulfonyl group (preferably, phenylsulfonyl) optionally substituted by 1 to 3 halogen atoms], a $C_{1-6}$ alkyl-carbonyl group (preferably, acetyl), a $C_{1-6}$ alkoxy-carbonyl group (preferably, methoxycarbonyl, ethoxycarbonyl), a carboxyl group, a carbamoyl group optionally having substituent(s) [preferably, a carbamoyl group optionally mono- or di-substituted by a $C_{1-6}$ alkyl group (preferably, ethyl) optionally substituted by a $C_{1-6}$ alkoxy group (preferably, methoxy)]}, and
(h) an oxo group, or

(2) a 3- to 11-membered heterocycle [preferably, a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole, 1,2,5-oxadiazole, benzimidazole, benzotriazole, benzofuran, tetrazole, 1H-thieno[2,3-c]pyrazole, [1,2,4]triazolo[1,5-a]pyrimidine); a 3-to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxy-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydro-1-benzofuran, 1,4,5,6-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane)] optionally having 1 to 3 substituents selected from

(a) a $C_{3-10}$ cycloalkyl group (preferably, cyclopropyl) ,
(b) a $C_{6-14}$ aryl group (preferably, phenyl) optionally substituted by 1 to 3 substituents selected from

(i) a $C_{1-6}$ alkoxy group (preferably, methoxy),
(ii) a halogen atom, and
(iii) an amino group optionally mono- or di-substituted by a $C_{1-6}$ alkyl group (preferably, methyl),

(c) an aromatic heterocyclic group (preferably, furyl, thienyl, pyrazolyl),
(d) a nonaromatic heterocyclic group (preferably, piperidino),
(e) an amino group optionally mono- or di-substituted by a $C_{1-6}$ alkyl-carbonyl group (preferably, acetyl),
(f) a $C_{1-6}$ alkoxy group (preferably, methoxy),
(g) a $C_{6-10}$ aryloxy group (preferably, phenoxy) optionally substituted by 1 to 3 halogen atoms,
(h) a $C_{1-6}$ alkylsulfanyl group (preferably, methylsulfanyl),
(i) a cyano group,
(j) a nitro group,
(k) a halogen atom,
(l) an acyl group {preferably, sulfamoyl optionally having substituent(s) [preferably, a sulfamoyl group optionally

mono- or di-substituted by a $C_{1-6}$ alkyl group (preferably, methyl)], a $C_{1-6}$ alkylsulfonyl group optionally having substituent(s) [preferably, a $C_{1-6}$ alkylsulfonyl group (preferably, methylsulfonyl, ethylsulfonyl) optionally substituted by 1 to 3 halogen atoms], a $C_{1-6}$ alkylsulfinyl group (preferably, methylsulfinyl), a $C_{6-14}$ arylsulfonyl group optionally having substituent(s) [preferably, a $C_{6-14}$ arylsulfonyl group (preferably, phenylsulfonyl) optionally substituted by 1 to 3 halogen atoms], a non-aromatic heterocyclyl-sulfonyl group (preferably, piperidinosulfonyl), an aromatic heterocyclyl-sulfonyl group optionally having substituent(s) [preferably, an aromatic heterocyclyl-sulfonyl group (preferably, isooxazolylsulfonyl) optionally substituted by 1 to 3 $C_{1-6}$ alkyl groups (preferably, methyl)], a $C_{1-6}$ alkoxy-carbonyl group (preferably, methoxycarbonyl, ethoxycarbonyl), a $C_{1-6}$ alkyl-carbonyl group (preferably, acetyl), a carbamoyl group optionally having substituent(s) [preferably, a carbamoyl group optionally mono- or di-substituted by a $C_{1-6}$ alkyl group (preferably, ethyl) optionally substituted by substituents selected from

(i) an amino group optionally mono- or di-substituted by a $C_{1-6}$ alkyl group (preferably, methyl), and
(ii) a hydroxy group]},

(m) a $C_{1-6}$ alkyl group (preferably, methyl, ethyl, propyl, isopropyl, isobutyl, tert-butyl) optionally substituted by 1 to 3 substituents selected from

(i) a halogen atom,
(ii) a hydroxy group,
(iii) an amino group optionally mono- or di-substituted by a $C_{1-6}$ alkyl group (preferably, methyl),
(iv) a $C_{6-10}$ aryloxy group (preferably, phenoxy) optionally substituted by a $C_{1-6}$ alkoxy group (preferably, methoxy),
(v) a carbamoyl group,
(vi) an aromatic heterocyclic group (preferably, thienyl, pyrazolyl, imidazolyl) optionally substituted by 1 to 3 $C_{1-6}$ alkyl groups (preferably, methyl),
(vii) a nonaromatic heterocyclic group (preferably, morpholino, pyrrolidinyl) optionally substituted by 1 to 3 oxos,
(viii) a $C_{1-6}$ alkoxy group (preferably, methoxy),
(ix) a cyano group, and
(x) a $C_{1-6}$ alkylsulfonyl group (preferably, methylsulfonyl),

(n) a $C_{7-13}$ aralkyl group (preferably, benzyl) optionally substituted by 1 to 3 substituents selected from a $C_{1-6}$ alkyl group (preferably, methyl) optionally substituted by 1 to 3 halogen atoms, and
(o) an oxo group.

In another embodiment, ring B is preferably

(1) a 3- to 10-membered unsaturated hydrocarbon ring (preferably, benzene, indane) optionally having 1 to 3 substituents selected from a halogen atom, an amino group optionally having substituent(s), a $C_{1-6}$ alkylsulfanyl group optionally having substituent(s), an acyl group (preferably, a sulfamoyl group optionally having substituent(s), a $C_{1-6}$ alkylsulfonyl group optionally having substituent(s), a $C_{1-6}$ alkylsulfinyl group optionally having substituent(s)), and a 3- to 11-membered heterocyclic group optionally having substituent(s) (preferably, a 3- to 11-membered nonaromatic heterocyclic group optionally having substituent(s)),
(2) a 3- to 10-membered saturated hydrocarbon ring (preferably, cyclopropane, cyclopentane) optionally having 1 to 3 substituents selected from a cyano group, and an acyl group (preferably, a $C_{6-14}$ arylsulfonyl group optionally having substituent(s), a $C_{1-6}$ alkylsulfonyl group optionally having substituent(s), a $C_{1-6}$ alkyl-carbonyl group optionally having substituent(s), a $C_{1-6}$ alkoxy-carbonyl group optionally having substituent(s), a carboxyl group, a carbamoyl group optionally having substituent(s)),
(3) a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole, 1,2,5-oxadiazole, benzimidazole, benzotriazole, benzofuran, tetrazole, 1H-thieno[2,3-c]pyrazole, [1,2,4]triazolo[1,5-a]pyrimidine) optionally substituted by 1 to 3 substituents selected from a halogen atom, a $C_{1-6}$ alkyl group optionally having substituent(s), a hydroxy group, a $C_{1-6}$ alkoxy group optionally having substituent(s), a $C_{6-10}$ aryl group optionally having substituent(s), a $C_{3-10}$ cycloalkyl group optionally having substituent(s), a 3- to 11-membered heterocyclic group optionally having substituent(s), an amino group optionally having substituent(s), a $C_{1-6}$ alkylsulfanyl group optionally having substituent(s), a $C_{6-10}$ aryloxy group optionally having substituent(s), a nitro group, and an acyl group (preferably, a sulfamoyl group optionally having substituent(s), a $C_{1-6}$ alkylsulfonyl group optionally

having substituent(s), a $C_{1-6}$ alkylsulfinyl group optionally having substituent(s), a $C_{1-6}$ alkoxy-carbonyl group optionally having substituent(s), a carbamoyl group optionally having substituent(s), a non-aromatic heterocyclyl-sulfonyl group optionally having substituent(s)),
(4) a 3- to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxy-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydra-1-benzofuran, 1,4,5,6-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane) optionally having 1 to 3 substituents selected from a $C_{1-6}$ alkyl group optionally having substituent(s), an amino group optionally having substituent(s), a $C_{1-6}$ alkoxy group optionally having substituent(s), a $C_{6-10}$ aryl group optionally having substituent(s), a $C_{7-13}$ aralkyl group optionally having substituent(s), an acyl group (preferably, a $C_{1-6}$ alkylcarbonyl group optionally having substituent(s), a $C_{1-6}$ alkoxy-carbonyl group optionally having substituent(s), a $C_{1-6}$ alkylsulfonyl group optionally having substituent(s), a $C_{6-14}$ arylsulfonyl group optionally having substituent(s), a heterocyclyl-sulfonyl group optionally having substituent(s) (preferably, an aromatic heterocyclyl-sulfonyl group optionally having substituent(s)), a carbamoyl group optionally having substituent(s)), and an oxo group,

more preferably

(1) a 3- to 10-membered unsaturated hydrocarbon ring (preferably, benzene, indane (preferably, 2,3-dihydro-1H-inden)) optionally having 1 to 3 substituents selected from

(a) a halogen atom,
(b) an amino group optionally mono- or di-substituted by substituent(s) selected from

(i) a $C_{1-6}$ alkyl group (preferably, methyl), and
(ii) $C_{1-6}$ alkyl-carbonyl (preferably, acetyl),

(c) a sulfamoyl group optionally mono- or di-substituted by a $C_{1-6}$ alkyl group (preferably, methyl) optionally substituted by a nonaromatic heterocyclic group (preferably, tetrahydrofuryl),
(d) a $C_{1-6}$ alkylsulfanyl group (preferably, methylsulfanyl),
(e) a $C_{1-6}$ alkylsulfonyl group (preferably, methylsulfonyl),
(f) a $C_{1-6}$ alkylsulfinyl group (preferably, methylsulfinyl), and
(g) a nonaromatic heterocyclic group (preferably, pyrrolidinyl, 1,1-dioxido-1,2-thiazinan-2-yl) optionally substituted by 1 to 3 oxo groups,

(2) a 3- to 10-membered saturated hydrocarbon ring (preferably, cyclopropane, cyclopentane) optionally substituted by 1 to 3 substituents selected from

(a) a cyano group,
(b) $C_{6-14}$ arylsulfonyl (preferably, phenylsulfonyl) optionally substituted by 1 to 3 halogen atoms,
(c) a $C_{1-6}$ alkylsulfonyl group (preferably, methylsulfonyl),
(d) a $C_{1-6}$ alkyl-carbonyl group (preferably, acetyl),
(e) a $C_{1-6}$ alkoxy-carbonyl group (preferably, methoxycarbonyl, ethoxycarbonyl),
(f) a carboxyl group, and
(g) a carbamoyl group optionally mono- or di-substituted by a $C_{1-6}$ alkyl group (preferably, ethyl) optionally substituted by a $C_{1-6}$ alkoxy group (preferably, methoxy),

(3) a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole, 1,2,5-oxadiazole, benzimidazole, benzotriazole, benzofuran, tetrazole, 1H-thieno[2,3-c]pyrazole, [1,2,4]triazolo[1,5-a]pyrimidine) optionally substituted by 1 to 3 substituents selected from

(a) a $C_{1-6}$ alkyl group (preferably, methyl, ethyl, propyl, isopropyl, isobutyl, tert-butyl) optionally substituted by 1 to 3 substituents selected from

(i) a halogen atom,

(ii) a $C_{6-10}$ aryloxy group (preferably, phenoxy) optionally substituted by a $C_{1-6}$ alkoxy group (preferably, methoxy),
(iii) a nonaromatic heterocyclic group (preferably, morpholino, pyrrolidinyl) optionally substituted by 1 to 3 oxo groups,
(iv) an aromatic heterocyclic group (preferably, pyrazolyl, imidazolyl) optionally substituted by 1 to 3 $C_{1-6}$ alkyl groups (preferably, methyl),
(v) a hydroxy group,
(vi) an amino group optionally mono- or di-substituted by a $C_{1-6}$ alkyl group (preferably, methyl),
(vii) a $C_{1-6}$ alkoxy group (preferably, methoxy),
(viii) a cyano group,
(ix) a carbamoyl group, and
(x) a $C_{1-6}$ alkylsulfonyl group (preferably, methylsulfonyl),

(b) a $C_{6-14}$ aryl group (preferably, phenyl) optionally substituted by 1 to 3 halogen atoms,
(c) an amino group optionally mono- or di-substituted by a $C_{1-6}$ alkyl-carbonyl group (preferably, acetyl),
(d) a sulfamoyl group optionally mono- or di-substituted by a $C_{1-6}$ alkyl group (preferably, methyl),
(e) a $C_{1-6}$ alkylsulfanyl group (preferably, methylsulfanyl),
(f) a $C_{1-6}$ alkylsulfonyl group (preferably, methylsulfonyl),
(g) a $C_{1-6}$ alkylsulfinyl group (preferably, methylsulfinyl),
(h) a halogen atom,
(i) a $C_{3-10}$ cycloalkyl group (preferably, cyclopropyl),
(j) an aromatic heterocyclic group (preferably, furyl, thienyl, pyrazolyl),
(k) a nonaromatic heterocyclic group (preferably, piperidino),
(l) a non-aromatic heterocyclyl-sulfonyl group (preferably, piperidinosulfonyl),
(m) a $C_{6-10}$ aryloxy group (preferably, phenoxy) optionally substituted by 1 to 3 halogen atoms,
(n) a nitro group,
(o) a $C_{1-6}$ alkoxy-carbonyl group (preferably, ethoxycarbonyl),
(p) a $C_{1-6}$ alkoxy group (preferably, methoxy),
(q) a carbamoyl group optionally mono- or di-substituted by a $C_{1-6}$ alkyl group (preferably, ethyl) optionally substituted by substituents selected from

(i) a hydroxy group, and
(ii) an amino group optionally mono- or di-substituted by a $C_{1-6}$ alkyl group (preferably, methyl),

(4) a 3- to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxido-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydro-1-benzofuran, 1,4,5,6-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane) optionally substituted by 1 to 3 substituents selected from

(a) a $C_{1-6}$ alkyl group (preferably, methyl, ethyl, isopropyl) optionally substituted by an aromatic heterocyclic group (preferably, thienyl),
(b) a $C_{1-6}$ alkoxy group (preferably, methoxy),
(c) a $C_{1-6}$ alkoxy-carbonyl group (preferably, methoxycarbonyl, tert-butoxycarbonyl),
(d) a $C_{6-14}$ aryl group (preferably, phenyl) optionally substituted by substituents selected from

(i) a $C_{1-6}$ alkoxy group (preferably, methoxy), and
(ii) an amino group optionally mono- or di-substituted by a $C_{1-6}$ alkyl group (preferably, methyl),

(e) a $C_{7-13}$ aralkyl group (preferably, benzyl) optionally substituted by 1 to 3 substituents selected from a $C_{1-6}$ alkyl group (preferably, methyl) optionally substituted by 1 to 3 halogen atoms,
(f) a $C_{6-14}$ arylsulfonyl group (preferably, phenylsulfonyl) optionally substituted by 1 to 3 halogen atoms,
(g) a $C_{1-6}$ alkyl-carbonyl group (preferably, acetyl),
(h) a $C_{1-6}$ alkylsulfonyl group (preferably, methylsulfonyl, ethylsulfonyl) optionally substituted by 1 to 3 halogen atoms,

(i) a carbamoyl group optionally mono- or di-substituted by a $C_{1-6}$ alkyl group (preferably, ethyl),
(j) an oxo group, and
(k) an aromatic heterocyclyl-sulfonyl group (preferably, isooxazolylsulfonyl) optionally substituted by 1 to 3 $C_{1-6}$ alkyl groups (preferably, methyl).

X is a single bond, a $C_{1-6}$ alkylene group optionally having substituent(s) or a $C_{2-6}$ alkenylene group.
X is preferably a single bond, a $C_{1-6}$ alkylene group or a $C_{2-6}$ alkenylene group, more preferably, a single bond, vinylene or $C_{1-6}$ alkylene (preferably, methylene, $-(CH_2)_2-$, $-CH(i-C_3H_7)-$).
Preferred as a compound represented by the formula (I) is a compound wherein
$R^1$ and $R^2$ are the same or different and each is a hydrogen atom, a hydroxy group or a $C_{1-6}$ alkyl group optionally having substituent(s) [preferably, a $C_{1-6}$ alkyl group (preferably, methyl)], or $R^1$ and $R^2$ may form, together with the adjacent carbon atom, a 3- to 6-membered saturated hydrocarbon ring (preferably, cyclopropane);
$R^3$ is a hydrogen atom, a $C_{1-6}$ alkyl group optionally having substituent(s) [preferably, a $C_{1-6}$ alkyl group (preferably, methyl, ethyl, propyl, isopropyl, butyl) optionally substituted by 1 to 3 substituents selected from a halogen atom, a hydroxy group and a $C_{1-6}$ alkoxy group (preferably, methoxy)], a $C_{2-6}$ alkenyl group optionally having substituent(s) [preferably, a $C_{2-6}$ alkenyl group (preferably, ethenyl)], or an acyl group {preferably, a $C_{1-6}$ alkyl-carbonyl group optionally having substituent(s) [preferably, a $C_{1-6}$ alkyl-carbonyl group (preferably, acetyl)], a $C_{1-6}$ alkoxy-carbonyl group optionally having substituent(s) [preferably, a $C_{1-6}$ alkoxy-carbonyl group (preferably, ethoxycarbonyl)]};
$R^4$ is

(1) a hydrogen atom,
(2) a $C_{3-10}$ cycloalkyl group optionally having substituent(s),
(3) a methyl group optionally having substituent(s) selected from a halogen atom, a cyano group, a hydroxy group, a $C_{1-6}$ alkoxy group optionally having substituent(s), a $C_{6-10}$ aryloxy group optionally having substituent(s), a sulfanyl group optionally having substituent(s), an amino group optionally having substituent(s), an acyl group, a $C_{3-10}$ cycloalkyl group optionally having substituent(s), and a 3- to 11-membered heterocyclic group optionally having substituent(s), or
(4) a $C_{2-6}$ alkyl group optionally having substituent(s);
ring A is a 3- to 10-membered unsaturated hydrocarbon ring optionally having substituent(s) {preferably, a 6- to 10-membered aromatic hydrocarbon ring (preferably, benzene, naphthalene) optionally substituted by 1 - 3 (preferably 1 or 2) substituents selected from (i) a halogen atom (preferably, a fluorine atom, a chlorine atom, a bromine atom), (ii) a $C_{1-6}$ alkyl group (preferably, methyl) optionally substituted by 1 to 3 halogen atoms (preferably, fluorine atom), (iii) a nitro group, (iv) a $C_{1-6}$ alkoxy group (preferably, methoxy), (v) a hydroxy group, (vi) a $C_{3-10}$ cycloalkyl group (preferably, cyclopropyl) and (vii) $C_{1-6}$ alkylsulfonyl(preferably, methylsulfonyl)};

ring B is

(1) benzene having at least one substituent selected from a halogen atom, a cyano group, a $C_{1-6}$ alkyl group optionally having substituent(s), a $C_{2-6}$ alkenyl group optionally having substituent(s), a $C_{2-6}$ alkynyl group optionally having substituent(s), a sulfanyl group optionally having substituent(s), an amino group optionally having substituent(s), an acyl group, a 3- to 10--membered hydrocarbon ring group optionally having substituent(s) and a 3- to 11-membered heterocyclic group optionally having substituent(s),
(2) a 3- to 10-membered saturated hydrocarbon ring optionally having substituent(s) selected from a halogen atom, a cyano group, a hydroxy group, a $C_{1-6}$ alkyl group optionally having substituent(s), a $C_{2-6}$ alkenyl group optionally having substituent(s), a $C_{2-6}$ alkynyl group optionally having substituent(s), a $C_{1-6}$ alkoxy group optionally having substituent(s), a $C_{6-10}$ aryloxy group optionally having substituent(s), a sulfanyl group optionally having substituent (s), an amino group optionally having substituent(s), an acyl group, a $C_{3-10}$ cycloalkyl group optionally having substituent(s) and a 3- to 11-membered nitrogen-containing heterocyclic group optionally having substituent(s), or
(3) a 3- to 11-membered heterocycle optionally having substituent(s),

{preferably,

(1) benzene having at least one substituent selected from a halogen atom, a sulfanyl group optionally having substituent(s), an amino group optionally having substituent(s), an acyl group (preferably, a sulfamoyl group optionally having substituent(s), a $C_{1-6}$ alkylsulfinyl group optionally having substituent(s), a $C_{1-6}$ alkylsulfonyl group optionally having substituent(s)), and a 3- to 11-membered heterocyclic group optionally having substituent(s) (preferably, a 3- to 11-membered nonaromatic heterocyclic group optionally having substituent(s)),
(2) a 3- to 10-membered saturated hydrocarbon ring (preferably, cyclopropane, cyclopentane) optionally having 1

to 3 substituents selected from a cyano group, and an acyl group (preferably, a $C_{6-14}$ arylsulfonyl group optionally having substituent(s), a $C_{1-6}$ alkylsulfonyl group optionally having substituent(s), a $C_{1-6}$ alkyl-carbonyl group optionally having substituent(s), a $C_{1-6}$ alkoxy-carbonyl group optionally having substituent(s), a carboxyl group, a carbamoyl group optionally having substituent(s)),

(3) a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole, 1,2,5-oxadiazole, benzimidazole, benzotriazole, benzofuran, tetrazole, 1H-thieno[2,3-c]pyrazole, [1,2,4]triazolo[1,5-a]pyrimidine) optionally substituted by 1 to 3 substituents selected from a halogen atom, a $C_{1-6}$ alkyl group optionally having substituent(s), a hydroxy group, a $C_{1-6}$ alkoxy group optionally having substituent(s), a $C_{6-10}$ aryl group optionally having substituent(s), a $C_{3-10}$ cycloalkyl group optionally having substituent(s), a 3- to 11-membered heterocyclic group optionally having substituent(s), an amino group optionally having substituent(s), a $C_{1-6}$ alkylsulfanyl group optionally having substituent(s), a $C_{6-10}$ aryloxy group optionally having substituent(s), a nitro group, and an acyl group (preferably, a sulfamoyl group optionally having substituent(s), a $C_{1-6}$ alkylsulfonyl group optionally having substituent(s), a $C_{1-6}$ alkylsulfinyl group optionally having substituent(s), a $C_{1-6}$ alkoxy-carbonyl group optionally having substituent(s), a carbamoyl group optionally having substituent(s), a non-aromatic heterocyclyl-sulfonyl group optionally having substituent(s)),

(4) a 3- to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxy-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydro-1-benzofuran, 1,4,3,E-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane) optionally having 1 to 3 substituents selected from a $C_{1-6}$ alkyl group optionally having substituent(s), a hydroxy group, an amino group optionally having substituent(s), a $C_{1-6}$ alkoxy group optionally having substituent(s), a $C_{6-10}$ aryl group optionally having substituent(s), a $C_{7-13}$ aralkyl group optionally having substituent(s), an acyl group (preferably, a $C_{1-6}$ alkyl-carbonyl group optionally having substituent(s), a $C_{1-6}$ alkoxy-carbonyl group optionally having substituent(s), a $C_{1-6}$ alkylsulfonyl group optionally having substituent(s), a $C_{6-14}$arylsulfonyl group optionally having substituent(s), a heterocyclyl-sulfonyl group optionally having substituent(s) (preferably, an aromatic heterocyclyl-sulfonyl group optionally having substituent(s)), a carbamoyl group optionally having substituent(s)), and an oxo group,

more preferably

(1) benzene having at least one substituent selected from

(a) a halogen atom,
(b) an amino group optionally mono- or di-substituted by substituent(s) selected from

(i) a $C_{1-6}$ alkyl group (preferably, methyl), and
(ii) $C_{1-6}$ alkyl-carbonyl (preferably, acetyl),

(c) a sulfamoyl group optionally mono- or di-substituted by a $C_{1-6}$ alkyl group (preferably, methyl) optionally substituted by a nonaromatic heterocyclic group (preferably, tetrahydrofuryl),
(d) a $C_{1-6}$ alkylsulfanyl group (preferably, methylsulfanyl),
(e) a $C_{1-6}$ alkylsulfonyl group (preferably, methylsulfonyl),
(f) a $C_{1-6}$ alkylsulfinyl group (preferably, methylsulfinyl), and
(g) a nonaromatic heterocyclic group (preferably, pyrrolidinyl, 1,1-dioxido-1,2-thiazinan-2-yl) optionally substituted by 1 to 3 oxo groups,

(2) a 3- to 10-membered saturated hydrocarbon ring (preferably, cyclopropane, cyclopentane) optionally substituted by 1 to 3 substituents selected from

(a) a cyano group,
(b) $C_{6-14}$ arylsulfonyl (preferably, phenylsulfonyl) optionally substituted by 1 to 3 halogen atoms,
(c) a $C_{1-6}$ alkylsulfonyl group (preferably, methylsulfonyl),
(d) a $C_{1-6}$ alkyl-carbonyl group (preferably, acetyl),
(e) a $C_{1-6}$ alkoxy-carbonyl group (preferably, methoxycarbonyl, ethoxycarbonyl),

(f) a carboxyl group, and

(g) a carbamoyl group optionally mono- or di-substituted by a $C_{1-6}$ alkyl group (preferably, ethyl) optionally substituted by a $C_{1-6}$ alkoxy group (preferably, methoxy),

(3) a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole, 1,2,5-oxadiazole, benzimidazole, benzotriazole, benzofuran, tetrazole, 1H-thieno[2,3-c]pyrazole, [1,2,4]triazolo[1,5-a]pyrimidine) optionally substituted by 1 to 3 substituents selected from

(a) a $C_{1-6}$ alkyl group (preferably, methyl, ethyl, propyl, isopropyl, isobutyl, tert-butyl) optionally substituted by 1 to 3 substituents selected from

(i) a halogen atom,

(ii) a $C_{6-10}$ aryloxy group (preferably, phenoxy) optionally substituted by a $C_{1-6}$ alkoxy group (preferably, methoxy),

(iii) a nonaromatic heterocyclic group (preferably, morpholino, pyrrolidinyl) optionally substituted by 1 to 3 oxo groups,

(iv) an aromatic heterocyclic group (preferably, pyrazolyl, imidazolyl) optionally substituted by 1 to 3 $C_{1-6}$ alkyl groups (preferably, methyl),

(v) a hydroxy group,

(vi) an amino group optionally mono- or di-substituted by a $C_{1-6}$ alkyl group (preferably, methyl),

(vii) a $C_{1-6}$ alkoxy group (preferably, methoxy),

(viii) a cyano group,

(ix) a carbamoyl group, and

(x) a $C_{1-6}$ alkylsulfonyl group (preferably, methylsulfonyl),

(b) a $C_{6-14}$ aryl group (preferably, phenyl) optionally substituted by 1 to 3 halogen atoms,

(c) an amino group optionally mono- or di-substituted by a $C_{1-6}$ alkyl-carbonyl group (preferably, acetyl),

(d) a sulfamoyl group optionally mono- or di-substituted by a $C_{1-6}$ alkyl group (preferably, methyl),

(e) a $C_{1-6}$ alkylsulfanyl group (preferably, methylsulfanyl),

(f) a $C_{1-6}$ alkylsulfonyl group (preferably, methylsulfonyl),

(g) a $C_{1-6}$ alkylsulfinyl group (preferably, methylsulfinyl),

(h) a halogen atom,

(i) a $C_{3-10}$ cycloalkyl group (preferably, cyclopropyl),

(j) an aromatic heterocyclic group (preferably, furyl, thienyl, pyrazolyl),

(k) a nonaromatic heterocyclic group (preferably, piperidino),

(1) a non-aromatic heterocyclyl-sulfonyl group (preferably, piperidinosulfonyl),

(m) a $C_{6-10}$ aryloxy group (preferably, phenoxy) optionally substituted by 1 to 3 halogen atoms,

(n) a nitro group,

(o) a $C_{1-6}$ alkoxy-carbonyl group (preferably, ethoxycarbonyl),

(p) a $C_{1-6}$ alkoxy group (preferably, methoxy),

(q) a carbamoyl group optionally mono- or di-substituted by a $C_{1-6}$ alkyl group (preferably, ethyl) optionally substituted by substituents selected from

(i) a hydroxy group, and

(ii) an amino group optionally mono- or di-substituted by a $C_{1-6}$ alkyl group (preferably, methyl),

(4) a 3- to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxy-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydro-1-benzofuran, 1,4,5,6-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane) optionally substituted by 1 to 3 substituents selected from

(a) a $C_{1-6}$ alkyl group (preferably, methyl, ethyl, isopropyl) optionally substituted by an aromatic heterocyclic group (preferably, thienyl),

(b) a $C_{1-6}$ alkoxy group (preferably, methoxy),
(c) a $C_{1-6}$ alkoxy-carbonyl group (preferably, methoxycarbonyl, tert-butoxycarbonyl),
(d) a $C_{6-14}$ aryl group (preferably, phenyl) optionally substituted by substituents selected from

(i) a $C_{1-6}$ alkoxy group (preferably, methoxy), and
(ii) an amino group optionally mono- or di-substituted by a $C_{1-6}$ alkyl group (preferably, methyl),

(e) a $C_{7-13}$ aralkyl group (preferably, benzyl) optionally substituted by 1 to 3 substituents selected from a $C_{1-6}$ alkyl group (preferably, methyl) optionally substituted by 1 to 3 halogen atoms,
(f) a $C_{6-14}$ arylsulfonyl group (preferably, phenylsulfonyl) optionally substituted by 1 to 3 halogen atoms,
(g) a $C_{1-6}$ alkyl-carbonyl group (preferably, acetyl),
(h) a $C_{1-6}$ alkylsulfonyl group (preferably, methylsulfonyl, ethylsulfonyl) optionally substituted by 1 to 3 halogen atoms),
(i) a carbamoyl group optionally mono- or di-substituted by a $C_{1-6}$ alkyl group (preferably, ethyl),
(j) an oxo group, and
(k) an aromatic heterocyclyl-sulfonyl group (preferably, isooxazolylsulfonyl) optionally substituted by 1 to 3 $C_{1-6}$ alkyl groups (preferably, methyl); and
X is a single bond, a $C_{1-6}$ alkylene group (preferably, methylene, $-(CH_2)_2-$, $-CH(i\text{-}C_3H_7)-$), or a $C_{2-6}$ alkenylene group (preferably, vinylene).

In another embodiment, preferred as a compound represented by the formula (I) is a compound wherein $R^1$ and $R^2$ are the same or different and each is a hydrogen atom, a hydroxy group or a $C_{1-6}$ alkyl group optionally having substituent(s) [preferably, a $C_{1-6}$ alkyl group (preferably, methyl)], or $R^1$ and $R^2$ may form, together with the adjacent carbon atom, a 3- to 6-membered saturated hydrocarbon ring (preferably, cyclopropane);
$R^3$ is a hydrogen atom, a $C_{1-6}$ alkyl group optionally having substituent(s) [preferably, a $C_{1-6}$ alkyl group (preferably, methyl, ethyl, propyl, isopropyl, butyl) optionally substituted by 1 to 3 substituents selected from a halogen atom, a hydroxy group and a $C_{1-6}$ alkoxy group (preferably, methoxy)], a $C_{2-6}$ alkenyl group optionally having substituent(s) [preferably, a $C_{2-6}$ alkenyl group (preferably, ethenyl)], or an acyl group {preferably, a $C_{1-6}$ alkyl-carbonyl group optionally having substituent(s) [preferably, a $C_{1-6}$ alkyl-carbonyl group (preferably, acetyl)], a $C_{1-6}$ alkoxy-carbonyl group optionally having substituent(s) [preferably, a $C_{1-6}$ alkoxy-carbonyl group (preferably, ethoxycarbonyl)]};
$R^4$ is

(1) a hydrogen atom,
(2) a $C_{3-10}$ cycloalkyl group optionally having substituent(s),
(3) a methyl group optionally having substituent(s) selected from a halogen atom, a cyano group, a hydroxy group, a $C_{1-6}$ alkoxy group optionally having substituent(s), a $C_{6-10}$ aryloxy group optionally having substituent(s), a sulfanyl group optionally having substituent(s), an amino group optionally having substituent(s), an acyl group, a $C_{3-10}$ cycloalkyl group optionally having substituent(s), and a 3- to 11-membered heterocyclic group optionally having substituent(s), or
(4) a $C_{2-6}$ alkyl group optionally having substituent(s);

ring A is a 3- to 10-membered unsaturated hydrocarbon ring optionally having substituent(s) {preferably, a 6- to 10-membered aromatic hydrocarbon ring optionally having substituent(s) [preferably, a 6- to 10-membered aromatic hydrocarbon ring (preferably, benzene, naphthalene) optionally substituted by 1 - 3 (preferably 1 or 2) substituents selected from (i) a halogen atom (preferably, a fluorine atom, a chlorine atom, a bromine atom), (ii) a $C_{1-6}$ alkyl group (preferably, methyl) optionally substituted by 1 to 3 halogen atoms (preferably, fluorine atom), (iii) a nitro group, (iv) a $C_{1-6}$ alkoxy group (preferably, methoxy), (v) a hydroxy group, (vi) a $C_{3-10}$ cycloalkyl group (preferably, cyclopropyl) and (vii) $C_{1-6}$ alkylsulfonyl(preferably, methylsulfonyl)};
ring B is

(1) a 3- to 10-membered hydrocarbon ring {preferably, a 3- to 10-membered unsaturated hydrocarbon ring [preferably, benzene, indane (preferably, 2,3-dihydro-1H-indene)], a 3- to 10-membered saturated hydrocarbon ring (preferably, cyclopropane, cyclopentane)] optionally having 1 to 3 substituents selected from

(a) a nonaromatic heterocyclic group (preferably, pyrrolidinyl, 1,1-dioxido-1,2-thiazinan-2-yl) optionally substituted by 1 to 3 oxo groups,
(b) an amino group optionally mono- or di-substituted by substituent(s) selected from a $C_{1-6}$ alkyl group (preferably, methyl) and a $C_{1-6}$ alkyl-carbonyl group (preferably, acetyl),

(c) a $C_{1-6}$ alkoxy group,
(d) a $C_{1-6}$ alkylsulfanyl group (preferably, methylsulfanyl),
(e) a cyano group,
(f) a halogen atom,
(g) an acyl group {preferably, sulfamoyl optionally having substituent(s) [preferably, a sulfamoyl group optionally mono- or di-substituted by a $C_{1-6}$ alkyl group (preferably, methyl) optionally substituted by a nonaromatic heterocyclic group (preferably, tetrahydrofuryl)], a $C_{1-6}$ alkylsulfonyl group (preferably, methylsulfonyl), a $C_{1-6}$ alkylsulfinyl group (preferably, methylsulfinyl), a $C_{6-14}$ arylsulfonyl group optionally having substituent(s) [preferably, a $C_{6-14}$ arylsulfonyl group (preferably, phenylsulfonyl) optionally substituted by 1 to 3 halogen atoms], a $C_{1-6}$ alkyl-carbonyl group (preferably, acetyl), a $C_{1-6}$ alkoxy-carbonyl group (preferably, methoxycarbonyl, ethoxycarbonyl), a carboxyl group, a carbamoyl group optionally having substituent(s) [preferably, a carbamoyl group optionally mono- or di-substituted by a $C_{1-6}$ alkyl group (preferably, ethyl) optionally substituted by a $C_{1-6}$ alkoxy group (preferably, methoxy)]}, and
(h) an oxo group, or

(2) a 3- to 11-membered heterocycle [preferably, a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole, 1,2,5-oxadiazole, benzimidazole, benzotriazole, benzofuran, tetrazole, 1H-thieno[2,3-c]pyrazole, [1,2,4]triazolo[1,5-a]pyrimidine); a 3-to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxy-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydro-1-benzofuran, 1,4,5,6-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane)] optionally having 1 to 3 substituents selected from

(a) a $C_{3-10}$ cycloalkyl group (preferably, cyclopropyl),
(b) a $C_{6-14}$ aryl group (preferably, phenyl) optionally substituted by 1 to 3 substituents selected from

(i) a $C_{1-6}$ alkoxy group (preferably, methoxy),
(ii) a halogen atom, and
(iii) an amino group optionally mono- or di-substituted by a $C_{1-6}$ alkyl group (preferably, methyl),

(c) an aromatic heterocyclic group (preferably, furyl, thienyl, pyrazolyl),
(d) a nonaromatic heterocyclic group (preferably, piperidino),
(e) an amino group optionally mono- or di-substituted by a $C_{1-6}$ alkyl-carbonyl group (preferably, acetyl),
(f) a $C_{1-6}$ alkoxy group (preferably, methoxy),
(g) a $C_{6-10}$ aryloxy group (preferably, phenoxy) optionally substituted by 1 to 3 halogen atoms,
(h) a $C_{1-6}$ alkylsulfanyl group (preferably, methylsulfanyl),
(i) a cyano group,
(j) a nitro group,
(k) a halogen atom,
(l) an acyl group {preferably, sulfamoyl optionally having substituent(s) [preferably, a sulfamoyl group optionally mono- or di-substituted by a $C_{1-6}$ alkyl group (preferably, methyl)], a $C_{1-6}$ alkylsulfonyl group optionally having substituent(s) [preferably, a $C_{1-6}$ alkylsulfonyl group (preferably, methylsulfonyl, ethylsulfonyl) optionally substituted by 1 to 3 halogen atoms], a $C_{1-6}$ alkylsulfinyl group (preferably, methylsulfinyl), a $C_{6-14}$ arylsulfonyl group optionally having substituent(s) [preferably, a $C_{6-14}$ arylsulfonyl group (preferably, phenylsulfonyl) optionally substituted by 1 to 3 halogen atoms], a non-aromatic heterocyclyl-sulfonyl group (preferably, piperidinosulfonyl), an aromatic heterocyclyl-sulfonyl group optionally having substituent(s) [preferably, an aromatic heterocyclyl-sulfonyl group (preferably, isooxazolylsulfonyl) optionally substituted by 1 to 3 $C_{1-6}$ alkyl groups (preferably, methyl)], a $C_{1-6}$ alkoxy-carbonyl group (preferably, methoxycarbonyl, ethoxycarbonyl),
a $C_{1-6}$ alkyl-carbonyl group (preferably, acetyl), a carbamoyl group optionally having substituent(s) [preferably, a carbamoyl group optionally mono- or di-substituted by a $C_{1-6}$ alkyl group (preferably, ethyl) optionally substituted by substituents selected from

(i) an amino group optionally mono- or di-substituted by a $C_{1-6}$ alkyl group (preferably, methyl), and
(ii) a hydroxy group]},

(m) a $C_{1-6}$ alkyl group (preferably, methyl, ethyl, propyl, isopropyl, isobutyl, tert-butyl) optionally substituted by 1 to 3 substituents selected from

(i) a halogen atom,
(ii) a hydroxy group,
(iii) an amino group optionally mono- or di-substituted by a $C_{1-6}$ alkyl group (preferably, methyl),
(iv) a $C_{6-10}$ aryloxy group (preferably, phenoxy) optionally substituted by a $C_{1-6}$ alkoxy group (preferably, methoxy),
(v) a carbamoyl group,
(vi) an aromatic heterocyclic group (preferably, thienyl, pyrazolyl, imidazolyl) optionally substituted by 1 to 3 $C_{1-6}$ alkyl groups (preferably, methyl),
(vii) a nonaromatic heterocyclic group (preferably, morpholino, pyrrolidinyl) optionally substituted by 1 to 3 oxos,
(viii) a $C_{1-6}$ alkoxy group (preferably, methoxy),
(ix) a cyano group, and
(x) a $C_{1-6}$ alkylsulfonyl group (preferably, methylsulfonyl),

(n) a $C_{7-13}$ aralkyl group (preferably, benzyl) optionally substituted by 1 to 3 substituents selected from a $C_{1-6}$ alkyl group (preferably, methyl) optionally substituted by 1 to 3 halogen atoms, and
(o) an oxo group; and

X is a single bond, a $C_{1-6}$ alkylene group (preferably, methylene, $-(CH_2)_2-$, $-CH(i-C_3H_7)-$), or a $C_{2-6}$ alkenylene group (preferably, vinylene).

In addition, in another embodiment, preferred as a compound represented by the formula (I) is a compound wherein
$R^1$ and $R^2$ are the same or different and each is a hydrogen atom, a hydroxy group or a $C_{1-6}$ alkyl group optionally having substituent(s) [preferably, a $C_{1-6}$ alkyl group (preferably, methyl)], or $R^1$ and $R^2$ may form, together with the adjacent carbon atom, a 3- to 6-membered saturated hydrocarbon ring (preferably, cyclopropane);
$R^3$ is a hydrogen atom, a $C_{1-6}$ alkyl group optionally having substituent(s) [preferably, a $C_{1-6}$ alkyl group (preferably, methyl, ethyl, propyl, isopropyl, butyl) optionally substituted by 1 to 3 substituents selected from a halogen atom, a hydroxy group and a $C_{1-6}$ alkoxy group (preferably, methoxy)], a $C_{2-6}$ alkenyl group optionally having substituent(s) [preferably, a $C_{2-6}$ alkenyl group (preferably, ethenyl)], or an acyl group {preferably, a $C_{1-6}$ alkyl-carbonyl group optionally having substituent(s) [preferably, a $C_{1-6}$ alkyl-carbonyl group (preferably, acetyl)], a $C_{1-6}$ alkoxy-carbonyl group optionally having substituent(s) [preferably, a $C_{1-6}$ alkoxy-carbonyl group (preferably, ethoxycarbonyl)]};
$R^4$ is a hydrogen atom or a $C_{1-6}$ alkyl group (preferably, methyl);
ring A is a 3- to 10-membered unsaturated hydrocarbon ring optionally having substituent(s) {preferably, a 6- to 10-membered aromatic hydrocarbon ring optionally having substituent(s) [preferably, a 6- to 10-membered aromatic hydrocarbon ring (preferably, benzene, naphthalene) optionally substituted by 1 - 3 (preferably 1 or 2) substituents selected from (i) a halogen atom (preferably, a fluorine atom, a chlorine atom, a bromine atom), (ii) a $C_{1-6}$ alkyl group (preferably, methyl) optionally substituted by 1 to 3 halogen atoms (preferably, fluorine atom), (iii) a nitro group, (iv) a $C_{1-6}$ alkoxy group (preferably, methoxy), (v) a hydroxy group, (vi) a $C_{3-10}$ cycloalkyl group (preferably, cyclopropyl) and (vii) $C_{1-6}$ alkylsulfonyl (preferably, methylsulfonyl)};
ring B is

(1) a 3- to 10-membered unsaturated hydrocarbon ring (preferably, benzene, indane) optionally having 1 to 3 substituents selected from a halogen atom, an amino group optionally having substituent(s), a $C_{1-6}$ alkylsulfanyl group optionally having substituent(s), an acyl group (preferably, a sulfamoyl group optionally having substituent(s), a $C_{1-6}$ alkylsulfonyl group optionally having substituent(s), a $C_{1-6}$ alkylsulfinyl group optionally having substituent(s)), and a 3- to 11-membered heterocyclic group optionally having substituent(s) (preferably, a 3- to 11-membered nonaromatic heterocyclic group optionally having substituent(s)),
(2) a 3- to 10-membered saturated hydrocarbon ring (preferably, cyclopropane, cyclopentane) optionally having 1 to 3 substituents selected from a cyano group, and an acyl group (preferably, a $C_{6-14}$ arylsulfonyl group optionally having substituent(s), a $C_{1-6}$ alkylsulfonyl group optionally having substituent(s), a $C_{1-6}$ alkyl-carbonyl group optionally having substituent(s), a $C_{1-6}$ alkoxy-carbonyl group optionally having substituent(s), a carboxyl group, a carbamoyl group optionally having substituent(s)),
(3) a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole,

1,2,5-oxadiazole, benzimidazole, benzotriazole, benzofuran, tetrazole, 1H-thieno[2,3-c]pyrazole, [1,2,4]triazolo[1,5-alpyrimidine) optionally substituted by 1 to 3 substituents selected from a halogen atom, a $C_{1-6}$ alkyl group optionally having substituent(s), a hydroxy group, a $C_{1-6}$ alkoxy group optionally having substituent(s), a $C_{6-10}$ aryl group optionally having substituent(s), a $C_{3-10}$ cycloalkyl group optionally having substituent(s), a 3- to 11-membered heterocyclic group optionally having substituent(s), an amino group optionally having substituent(s), a $C_{1-6}$ alkylsulfanyl group optionally having substituent(s), a $C_{6-10}$ aryloxy group optionally having substituent(s), a nitro group, and an acyl group (preferably, a sulfamoyl group optionally having substituent(s), a $C_{1-6}$ alkylsulfonyl group optionally having substituent(s), a $C_{1-6}$ alkylsulfinyl group optionally having substituent(s), a $C_{1-6}$ alkoxy-carbonyl group optionally having substituent(s), a carbamoyl group optionally having substituent(s), a non-aromatic heterocyclyl-sulfonyl group optionally having substituent(s)), or

(4) a 3- to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxy-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydro-1-benzofuran, 1,4,5,6-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane) optionally having 1 to 3 substituents selected from a $C_{1-6}$ alkyl group optionally having substituent(s), an amino group optionally having substituent(s), a $C_{1-6}$ alkoxy group optionally having substituent(s), a $C_{6-10}$ aryl group optionally having substituent(s), a $C_{7-13}$ aralkyl group optionally having substituent(s), an acyl group (preferably, a $C_{1-6}$ alkylcarbonyl group optionally having substituent(s), a $C_{1-6}$ alkoxy-carbonyl group optionally having substituent(s), a $C_{1-6}$ alkylsulfonyl group optionally having substituent(s), a $C_{6-14}$ arylsulfonyl group optionally having substituent(s), a heterocyclyl-sulfonyl group optionally having substituent(s) (preferably, an aromatic heterocyclyl-sulfonyl group optionally having substituent(s)), a carbamoyl group optionally having substituent(s)), and an oxo group,
{preferably

(1) a 3- to 10-membered unsaturated hydrocarbon ring (preferably, benzene, indane (preferably, 2,3-dihydro-1H-indene)) optionally having 1 to 3 substituents selected from

(a) a halogen atom,
(b) an amino group optionally mono- or di-substituted by substituent(s) selected from

(i) a $C_{1-6}$ alkyl group (preferably, methyl), and
(ii) $C_{1-6}$ alkyl-carbonyl (preferably, acetyl),

(c) a sulfamoyl group optionally mono- or di-substituted by a $C_{1-6}$ alkyl group (preferably, methyl) optionally substituted by a nonaromatic heterocyclic group (preferably, tetrahydrofuryl),
(d) a $C_{1-6}$ alkylsulfanyl group (preferably, methylsulfanyl),
(e) a $C_{1-6}$alkylsulfonyl group (preferably, methylsulfonyl),
(f) a $C_{1-6}$ alkylsulfinyl group (preferably, methylsulfinyl), and
(g) a nonaromatic heterocyclic group (preferably, pyrrolidinyl, 1,1-dioxido-1,2-thiazinan-2-yl) optionally substituted by 1 to 3 oxo groups,

(2) a 3- to 10-membered saturated hydrocarbon ring (preferably, cyclopropane, cyclopentane) optionally substituted by 1 to 3 substituents selected from

(a) a cyano group,
(b) $C_{6-14}$ arylsulfonyl (preferably, phenylsulfonyl) optionally substituted by 1 to 3 halogen atoms,
(c) a $C_{1-6}$ alkylsulfonyl group (preferably, methylsulfonyl),
(d) a $C_{1-6}$ alkyl-carbonyl group (preferably, acetyl),
(e) a $C_{1-6}$ alkoxy-carbonyl group (preferably, methoxycarbonyl, ethoxycarbonyl),
(f) a carboxyl group, and
(g) a carbamoyl group optionally mono- or di-substituted by a $C_{1-6}$ alkyl group (preferably, ethyl) optionally substituted by a $C_{1-6}$ alkoxy group (preferably, methoxy),

(3) a 3- to 11-membered aromatic heterocycle (preferably, pyrazole, oxazole, isoxazole, furan, thiophene, pyridine, pyrimidine, pyridazine, pyrazine, imidazole, thiazole, indazole, pyrrole, benzothiazole, indole, 1,2,3-thiadiazole, 1,2,5-oxadiazole, benzimidazole, benzotriazole, benzofuran, tetrazole, 1H-thieno[2,3-c]pyrazole,

[1,2,4]triazolo[1,5-a]pyrimidine) optionally substituted by 1 to 3 substituents selected from

(a) a $C_{1-6}$ alkyl group (preferably, methyl, ethyl, propyl, isopropyl, isobutyl, tert-butyl) optionally substituted by 1 to 3 substituents selected from

(i) a halogen atom,
(ii) a $C_{6-10}$ aryloxy group (preferably, phenoxy) optionally substituted by a $C_{1-6}$ alkoxy group (preferably, methoxy),
(iii) a nonaromatic heterocyclic group (preferably, morpholino, pyrrolidinyl) optionally substituted by 1 to 3 oxo groups,
(iv) an aromatic heterocyclic group (preferably, pyrazolyl, imidazolyl) optionally substituted by 1 to 3 $C_{1-6}$ alkyl groups (preferably, methyl),
(v) a hydroxy group,
(vi) an amino group optionally mono- or di-substituted by a $C_{1-6}$ alkyl group (preferably, methyl),
(vii) a $C_{1-6}$ alkoxy group (preferably, methoxy),
(viii) a cyano group,
(ix) a carbamoyl group, and
(x) a $C_{1-6}$ alkylsulfonyl group (preferably, methylsulfonyl),

(b) a $C_{6-14}$ aryl group (preferably, phenyl) optionally substituted by 1 to 3 halogen atoms,
(c) an amino group optionally mono- or di-substituted by a $C_{1-6}$ alkyl-carbonyl group (preferably, acetyl),
(d) a sulfamoyl group optionally mono- or di-substituted by a $C_{1-6}$ alkyl group (preferably, methyl),
(e) a $C_{1-6}$ alkylsulfanyl group (preferably, methylsulfanyl),
(f) a $C_{1-6}$ alkylsulfonyl group (preferably, methylsulfonyl),
(g) a $C_{1-6}$ alkylsulfinyl group (preferably, methylsulfinyl),
(h) a halogen atom,
(i) a $C_{3-10}$ cycloalkyl group (preferably, cyclopropyl)),
(j) an aromatic heterocyclic group (preferably, furyl, thienyl, pyrazolyl),
(k) a nonaromatic heterocyclic group (preferably, piperidino),
(l) a non-aromatic heterocyclyl-sulfonyl group (preferably, piperidinosulfonyl),
(m) a $C_{6-10}$ aryloxy group (preferably, phenoxy) optionally substituted by 1 to 3 halogen atoms,
(n) a nitro group,
(o) a $C_{1-6}$ alkoxy-carbonyl group (preferably, ethoxycarbonyl),
(p) a $C_{1-6}$ alkoxy group (preferably, methoxy),
(q) a carbamoyl group optionally mono- or di-substituted by a $C_{1-6}$ alkyl group (preferably, ethyl) optionally substituted by substituents selected from

(i) a hydroxy group, and
(ii) an amino group optionally mono- or di-substituted by a $C_{1-6}$ alkyl group (preferably, methyl), or

(4) a 3- to 11-membered non-aromatic heterocycle (preferably, pyrrolidine, piperidine, tetrahydrofuran, tetrahydrothiopyran, 1,1-dioxidotetrahydrothiopyran, 1,1-dioxidotetrahydrothiophene, 1,1-dioxidoisothiazolidine, imidazolidine, morpholine, thiazolidine, piperazine, 1,1-dioxidothiomorpholine, 1,1-dioxido-1,2-thiazinane, dihydropyridine (1,2-dihydropyridine, 2,3-dihydropyridine), 1,6-dihydropyridazine, 4H-chromene, 1,3-benzodioxole, 1,2,3,4-tetrahydroquinoline, 2,3-dihydro-1-benzofuran, 1,4,5,6-tetrahydropyridazine, 3,4-dihydro-2H-1,4-thiazine, 3,4-dihydro-2H-1,4-benzothiazine, 3,4-dihydro-2H-1,5-benzodioxepine, 3,4-dihydrophthalazine, 2,3-dihydro-1H-isoindole, 1,3-diazaspiro[4.4]nonane, 2,2-dioxido-2-thia-3-azabicyclo[3.1.0]hexane, 2,2-dioxido-2-thia-3-azabicyclo[4.1.0]heptane) optionally substituted by 1 to 3 substituents selected from

(a) a $C_{1-6}$ alkyl group (preferably, methyl, ethyl, isopropyl) optionally substituted by an aromatic heterocyclic group (preferably, thienyl),
(b) a $C_{1-6}$ alkoxy group (preferably, methoxy),
(c) a $C_{1-6}$ alkoxy-carbonyl group (preferably, methoxycarbonyl, tert-butoxycarbonyl),
(d) a $C_{6-14}$ aryl group (preferably, phenyl) optionally substituted by substituents selected from

(i) a $C_{1-6}$ alkoxy group (preferably, methoxy), and
(ii) an amino group optionally mono- or di-substituted by a $C_{1-6}$ alkyl group (preferably, methyl),

(e) a $C_{7-13}$ aralkyl group (preferably, benzyl) optionally substituted by 1 to 3 substituents selected from a $C_{1-6}$ alkyl group (preferably, methyl) optionally substituted by 1 to 3 halogen atoms,
(f) a $C_{6-14}$ arylsulfonyl group (preferably, phenylsulfonyl) optionally substituted by 1 to 3 halogen atoms,
(g) a $C_{1-6}$ alkyl-carbonyl group (preferably, acetyl),
(h) a $C_{1-6}$ alkylsulfonyl group (preferably, methylsulfonyl, ethylsulfonyl) optionally substituted by 1 to 3 halogen atoms,
(i) a carbamoyl group optionally mono- or di-substituted by a $C_{1-6}$ alkyl group (preferably, ethyl),
(j) an oxo group, and
(k) an aromatic heterocyclyl-sulfonyl group (preferably, isooxazolylsulfonyl) optionally substituted by 1 to 3 $C_{1-6}$ alkyl groups (preferably, methyl); and
X is a single bond, a $C_{1-6}$ alkylene group (preferably, methylene, $-(CH_2)_2-$, $-CH(i\text{-}C_3H_7)-$), or a $C_{2-6}$ alkenylene group (preferably, vinylene).

When compound (I) is a salt, examples thereof include metal salts, ammonium salt, salts with organic base, salts with inorganic acid, salts with organic acid, salts with basic or acidic amino acid, and the like. Preferable examples of the metal salt include alkaline metal salts such as sodium salt, potassium salt and the like; alkaline earth metal salts such as calcium salt, magnesium salt, barium salt and the like; aluminum salt and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of the salt with inorganic acid include salts with hydrochloric acid, hydrobromic acid, nitric acid, sulfuric acid, phosphoric acid and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salt with basic amino acid include salts with arginine, lysine, ornithine and the like. Preferable examples of the salt with acidic amino acid include salts with aspartic acid, glutamic acid and the like. Among them, pharmaceutically acceptable salts are preferable. For example, if the compound has an acidic functional group therein, examples of the salt include inorganic salts such as alkaline metal salts (e.g., sodium salt, potassium salt etc.), alkaline earth metal salt (e.g., calcium salt, magnesium salt, barium salt etc.); ammonium salt and the like. If the compound has a basic functional group therein, examples of the salt thereof include salts with inorganic acid such as hydrochloric acid, hydrobromic acid, nitric acid, sulfuric acid, phosphoric acid and the like, and salts with organic acid such as acetic acid, phthalic acid, fumaric acid, oxalic acid, tartaric aid, maleic acid, citric aid, succinic acid, methanesulfonic acid, p-toluenesulfonic acid and the like.
When compound (I) has an isomer such as tautomer, optical isomer, stereoisomer, positional isomer, rotational isomer and the like, any isomer and a mixture thereof are encompassed in the present invention. In addition, when compound (I) has an optical isomer, an optical isomer resolved from a racemate is also encompassed in compound (I).
Compound (I) may be a crystal, and both a single crystal and crystal mixtures are encompassed in compound (I). Compound (I) may be a pharmaceutically acceptable cocrystal or cocrystal salt. Here, the cocrystal or cocrystal salt means a crystalline substance consisting of two or more particular substances which are solids at room temperature, each having different physical properties (e.g., structure, melting point, heat of melting, hygroscopicity, solubility, stability etc.). The cocrystal and cocrystal salt can be produced by cocrystallization method known per se.
Compound (I) may be a solvate (e.g., hydrate etc.) or a non-solvate, and both are encompassed in compound (I).
A compound labeled or substituted with an isotope (e.g., $^{2}H$, $^{3}H$, $^{11}C$, $^{14}C$, $^{18}F$, $^{35}S$, $^{125}I$ etc. ) is also encompassed in compound (I).
A prodrug of compound (I) means a compound converted to compound (I) by a reaction due to an enzyme, a gastric acid, etc. under the physiological condition in the living body, that is, a compound converted to compound (I) by oxidation, reduction, hydrolysis, etc. due to an enzyme, a compound converted to compound (I) by hydrolysis etc. due to gastric acid, and the like.
Examples of the prodrug of compound (I) include a compound obtained by subjecting amino group in compound (I) to an acylation, alkylation or phosphorylation (e.g., a compound obtained by subjecting amino group in compound (I) to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation); a compound obtained by subjecting hydroxy group in compound (I) to acylation, alkylation, phosphorylation or boration (e.g., a compound obtained by subjecting hydroxy group in the compound (I) to acetylation, palmitoylation, propanoylation, pivaloylation, succinylation, fumarylation, alanylation or dimethylaminoacethylation); a compound obtained by subjecting carboxy group in compound (I) to esterification or amidation (e.g., a compound obtained by subjecting carboxy group in compound (I) to ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation) and the like. Any one of these compounds can be produced from compound (I) by a method known per se. A prodrug of compound (I) may also be a compound converted

into compound (I) under physiological conditions, such as those described in IYAKUHIN no KAIHATSU (Development of Pharmaceuticals), Vol. 7, Design of Molecules, p.163-198, Published by HIROKAWA SHOTEN (1990).

The compound of the present invention has an excellent GPR52 agonist activity and is useful, for example, as an agent for the prophylaxis or treatment of diseases or symptoms in described in the following (1) to (10).

(1) mental diseases [e.g., depression, major depression, bipolar depression, dysthymic disorder, emotional disorder (seasonal emotional disorder and the like), recurrent depression, postpartum depression, stress disorder, symptom of depression, mania, anxiety, generalized anxiety disorder, anxiety syndrome, panic disorder, phobia, social phobia, social anxiety disorder, obsessive disorder, post-traumatic stress syndrome, post-traumatic stress disorder, Tourette syndrome, autism, adjustment disorder, bipolar disorder, neurosis, schizophrenia, neurosis, chronic fatigue syndrome, anxiety neurosis, compulsive neurosis, phobic disorder, epilepsy, anxiety, anxious mental state, dysthymia, cyclothymia, nervous erethism, faint, addiction, low sex drive, attention deficit hyperactivity disorder (ADHD), psychotic major depression, intractable major depression, treatment-resistant depression],
(2) neurodegenerative diseases [e.g., Alzheimer's disease, alzheimer-type senile dementia, Parkinson's disease, Huntington chorea, spinocerebellar degeneration, multi-infarct dementia, frontotemporal dementia, Parkinson's type frontotemporal dementia, progressive supranuclear palsy, Pick's syndrome, Niemann-Pick's syndrome, corticobasal degeneration, Down's disease, vascular dementia, postencephalitic parkinsonism, Lewy body dementia, HIV dementia, amyotrophic lateral sclerosis (ALS), motor neurogenesis disease (MND), Creutzfeldt-Jakob disease or prion disease, cerebral palsy, progressive supranuclear palsy, multiple sclerosis],
(3) age-related cognition memory disorders [e.g., age-related memory disorders, senile dementia],
(4) sleep disorders [e.g., intrinsic sleep disorder (e.g., psychophysiological insomnia and the like), extrinsic sleep disorder, circadian rhythm disorder (e.g., time zone change syndrome (jet lag), shift work sleep disorder, irregular sleep-wake pattern, delayed sleep phase syndrome, advanced sleep phase syndrome, non-24-hour sleep-wake and the like), parasomnia, sleep disorder with internal or psychiatric disorder (e.g., chronic obstructive pulmonary diseases, Alzheimer's disease, Parkinson's disease, cerebrovascular dementia, schizophrenia, depression, anxiety neurosis), stress-related insomnia, insomnia, insomnia-related neurosis, sleep apnea syndrome],
(5) respiratory depression caused by anesthetics (e.g., morphine), traumatic disease or neurodegenerative disease and the like,
(6) traumatic brain injury, cerebral apoplexy, and complications, which are depression, dysuria, pain and the like, with them,
(7) neurotic anorexia, eating disorder, anorexia nervosa, hyperorexia, other eating disorder, alcohol dependence, alcohol abuse, alcoholic amnesia, alcohol paranoia, alcohol preference, alcohol withdrawal, alcoholic mental disease, alcohol addiction, alcoholic jealousy, alcoholic mania, alcohol-dependent mental disorder, alcoholic insanity, nicotine addiction, pharmacophilia, pharmacophobia, pharmacomania, drug withdrawal, Meniere's disease, autonomic ataxia, as other neurological diseases
(8) neuropathic pain, pain after operation, carcinomatous pain, inflammatory pain, migraine, stress headache, catatonic headache, dysesthesia (e.g., hypesthesia etc.),
(9) diabetic neuropathy, obesity, diabetes, muscular spasm, alopecia, glaucoma, hypertension, cardiac disease, tachycardia, cardiac failure, hyperventilation, bronchial asthma, apnea, inflammatory disease, allergic disease, impotence, infertility, immunodeficiency syndrome, acromegaly, incontinence, metabolic syndrome, osteoporosis, gastrointestinal disorder, peptic ulcer, irritable bowel syndrome, inflammatory bowel disease, ulcerative colitis, Crohn's disease, vomiting, diarrhea, constipation and postoperative ileus, caused by stress and the like,
(10) others, climacteric disorder, sudden infant death syndrome, brain tumor and the like malignant tumor, immune deficiency syndrome caused by HIV infection, cerebrospinal meningitis.

The compound of the present invention is particularly useful as a prophylactic or therapeutic drug for symptoms of mental disorders (e.g., schizophrenia, depression, anxiety, bipolar disorders or PTSD, anxiety neuroses, obsessive-compulsive neuroses etc.), neurodegenerative diseases (e.g., Alzheimer's disease, mild cognitive impairment (MCI), Parkinson's disease, amyotrophic lateral sclerosis (ALS), Huntington's disease, spinocerebellar degeneration, multiple sclerosis (MS), Pick disease etc.), among them, schizophrenia, including (1) positive symptoms such as delusion and hallucination, (2) negative symptoms such as hypesthesia, social withdrawal, and diminished motivation/loss of concentration, and (3) cognitive dysfunction.

The compound of the present invention is superior in metabolic stability, so that the compound of this invention can be expected to have an excellent therapeutic effect on the above-mentioned diseases even in a low dose.

In addition, the compound of the present invention has low toxicity (which is a medicament superior to others in, for example, acute toxicity, chronic toxicity, genetic toxicity, reproductive toxicity, cardiotoxicity, drug interactions, carcinogenicity etc.). The compound of the present invention can be directly used as a medicament or a pharmaceutical preparation mixed with a pharmaceutically acceptable carrier or the like to be orally or parenterally administered to

mammals (e.g., humans, monkeys, cows, horses, pigs, mice, rats, hamsters, rabbits, cats, dogs, sheep, goats etc.) in safety.

The medicament containing the compound of the present invention can be used in the compound of the present invention as the sole regimen or as a pharmaceutical composition mixed with the compound of the present invention and a pharmacologically acceptable carrier according to method known per se as a production method for pharmaceutical preparation (e.g., the method described in the Japanese Pharmacopoeia etc.). The medicament containing the compound of the present invention can be safely administered orally or parenterally (e.g., intravenous, intramuscular, subcutaneous, intraorgan, intranasal, intradermal, instillation, intracerebral, intrarectal, vaginal, intraperitoneal, interior of a tumor, proximal of a tumor, lesion and the like) in the form of a tablet (inclusive of a sugar-coated tablet, a film-coated tablet, a sublingual tablet, an orally disintegrating tablet, a buccal tablet and the like), a pill, powders, granules, a capsule (inclusive of a soft capsule, a microcapsule), a troche, syrups, a liquid drug, emulsions, a suspension drug, controlled-release preparation (e.g., immediate-release preparation, sustained-release preparation, sustained-release microcapsule), aerosols, films (e.g., orally disintegrable films, oral mucosal adhesive film), an injection drug (e.g., subcutaneous injection, intravenous injection, intramuscular injection, intraperitoneal injection), drip infusion, transdermal absorption type preparation, ointment, lotion, adhesive preparation, suppository (e.g., rectal suppository, vaginal suppository), pellet, nasal preparations, pulmonary preparation (inhalant), eye drop and the like.

Here, examples of the pharmacologically acceptable carrier include various organic or inorganic carrier substances conventionally used as preparation materials, which are added as excipient, lubricant, binder or disintegrant for solid preparations; as solvent, solubilizing agent, suspending agent, isotonicity agent, buffer or soothing agent for liquid preparation, and the like. Where necessary, preparation additives such as preservative, antioxidant, colorant, sweetener and the like can also be used.

Preferable examples of the excipient include lactose, sucrose, D-mannitol, D-sorbitol, starch, pregelatinized starch, dextrin, crystalline cellulose, low-substituted hydroxypropyl cellulose, sodium carboxymethyl cellulose, gum arabic, pullulan, light anhydrous silicic acid, synthetic aluminum silicate, magnesium aluminometasilicate and the like.
Preferable examples of the lubricant include magnesium stearate, calcium stearate, talc, colloidal silica and the like.

Preferable examples of the binder include pregelatinized starch, saccharose, gelatin, gum arabic, methylcellulose, carboxymethyl cellulose, sodium carboxymethyl cellulose, crystalline cellulose, sucrose, D-mannitol, trehalose, dextrin, pullulan, hydroxypropyl cellulose, hydroxypropyl methylcellulose, polyvinyl pyrrolidone and the like.

Preferable examples of the disintegrant include lactose, sucrose, starch, carboxymethyl cellulose, carboxymethyl cellulose calcium, croscarmellose sodium, sodium carboxymethyl starch, light anhydrous silicic acid, low-substituted hydroxypropyl cellulose and the like.

Preferable examples of the solvent include water for injection, physiological saline, Ringer's solution, alcohol, propylene glycol, polyethylene glycol, sesame oil, corn oil, olive oil, cottonseed oil and the like.

Preferable examples of the solubilizing agents include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferable examples of the suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinyl pyrrolidone, sodium carboxymethyl cellulose, methylcellulose, hydroxymethyl cellulose, hydroxyethyl cellulose, hydroxypropyl cellulose and the like; polysorbates, polyoxyethylene hydrogenated castor oil and the like.

Preferable examples of an isotonicity agent include sodium chloride, glycerin, D-mannitol, D-sorbitol, glucose and the like.
Preferable examples of the buffer include buffers such as phosphate, acetate, carbonate, citrate and the like.
Preferable examples of the soothing agent include benzyl alcohol and the like.
Preferable examples of the preservative include p-oxybenzoic acid esters, chlorobutanol, benzyl alcohol, phenethyl alcohol, dehydroacetic acid, sorbic acid and the like.
Preferable examples of the antioxidant include sulfite, ascorbate and the like.

Preferable examples of the colorant include water-soluble edible tar pigments (e.g., food colors such as Food Color Red Nos. 2 and 3, Food Color Yellow Nos. 4 and 5 and Food Color Blue Nos. 1 and 2), water-insoluble lake pigments (e.g., aluminum salt of the aforementioned water-soluble edible tar pigment), natural pigments (e.g., β-carotene, chlorophil and colcothar) and the like.
Preferable examples of the sweetening agent include saccharin sodium, dipotassium glycyrrhizinate, aspartame, stevia and the like.

While the content of the compound of the present invention in the pharmaceutical composition varies depending on the dosage form, the dose of the compound of the present invention and the like, for example, it is about 0.01 to 100% by weight, preferably 0.1 to 95% by weight with respect to the total amount of the composition.

While the dose of the compound of the present invention varies depending on the subject of administration,

administration route, target disease, symptom and the like, for example, for oral administration to a schizophrenia patient (adult, about 60 kg in weight), it is generally about 0.1 to about 20 mg/kg body weight, preferably about 0.2 to about 10 mg/kg body weight, more preferably about 0.5 to about 10 mg/kg body weight for one dose, which is desirably administered once to several times (e.g., three times) a day.

The compound of the present invention may be used in combination with other active ingredients. Examples of such active ingredients (hereinafter to be referred as concomitant drug) include:

(1) atypical antipsychotic agents (such as clozapine, olanzapine, risperidone, aripiprazole, iloperidone, asenapine, ziprasidone, quetiapine, zotepine, paliperidone, lurasidone);
(2) typical antipsychotic agents (such as haloperidol and chlorpromazine);
(3) selective serotonin reuptake inhibitors (such as paroxetine, sertraline, fluvoxamine, and fluoxetine), selective serotonin-noradrenaline reuptake inhibitors (such as milnacipran and venlafaxine),
(4) selective noradrenaline-dopamine reuptake inhibitors (e.g., bupropion);
(5) tetracyclic antidepressants (such as amoxapine and clomipramine);
(6) tricyclic antidepressants (such as imipramine and amitriptyline);
(7) other antidepressant agents (such as NS-2359, Lu AA21004, and DOV21947);
(8) nicotinic acetylcholine receptor modulators (e.g., varenicline, SSR-180711, PNU-120596 etc.),
(9) muscarinic acetylcholine receptor modulators (e.g., sabcomeline etc.),
(10) ionotropic glutamic acid receptor modulators (e.g., D-serine, LY-451395 etc.),
(11) metabolic glutamic acid receptor modulators (e.g., CDPPB, MPEP, LY-2140023 etc.),
(12) GABA receptor modulators (e.g., valproic acid, MK-0777 etc.),
(13) histamine receptor modulators (e.g., GSK-239512 etc.),
(14) dopamine receptor modulators (e.g., cariprazine etc.),
(15) serotonin receptor modulators (e.g., pimavanserin, GSK-742457 etc.),
(16) noradrenaline receptor antagonist (e.g., idazoxan etc.),
(17) CRF receptor modulators (e.g., pexacerfont etc.),
(18) substance P receptor modulators (e.g., aprepitant, orvepitant etc.),
(19) orexin receptor modulators (e.g., almorexant, MK-4305 etc.),
(20) phosphodiesterase inhibitor (e.g., MP-10, WEB-3, rolipram etc.),
(21) glycine transporter 1 inhibitor (e.g., RG1678, ALX5407, SSR504734 etc.),
(22) anxiolytics [benzodiazepine-based agents (such as diazepam and etizolam) and serotonin 5-$HT_{1A}$ agonists (such as tandospirone)];
(23) sleep inducing agents [benzodiazepine-based agents (e.g.., estazolam and triazolam);
(24) non-benzodiazepine-based agents (such as zolpidem),
(25) melatonin receptor agonists (such as ramelteon)];
(26) β-amyloid vaccines, β-amyloid antibody;
(27) β-amyloid degrading enzyme etc.,
(28) brain function activators (such as aniracetam and nicergoline);
(29) anti-Parkinson agents [such as dopamine receptor agonists (including L-DOPA, bromocriptine, pergolide, talipexole, pramipexole, cabergoline, and amantadine), mono-amine oxidase (MAO) inhibitors (such as deprenyl, selegiline, remacemide, and riluzole), anticholinergic agents (such as trihexyphenidyl and biperiden); and COMT inhibitors (such as entacapone)];
(30) therapeutic agents for amyotrophic lateral sclerosis (including neurotrophic factors and riluzole);
(31) antihyperlipidemic drugs such as cholesterol-lowering drugs [statins (such as pravastatin sodium, atorvastatin, simvastatin, and rosuvastatin), fibrates (such as clofibrate), and squalene synthase inhibitors];
(32) therapeutic agents for abnormal behaviors/wandering symptoms associated with dementia (such as sedatives and anxiolytics);
(33) apoptosis inhibitors (such as CPI-1189, IDN-6556, and CEP-1347);
(34) neuronal differentiation/regeneration accelerators (such as leteprinim and xaliproden (SR-57746-A), and SB-216763);
(35) antihypertensive agents;
(36) therapeutic agents for diabetes mellitus;
(37) nonsteroidal anti-inflammatory agents (such as meloxicam, tenoxicam, indomethacin, ibuprofen, celecoxib, rofecoxib, aspirin, and indomethacin);
(38) disease-modifying anti-rheumatic drugs (DMARDs);
(39) anticytokine agents (such as TNF inhibitors and MAP kinase inhibitors);
(40) steroid agents (such as dexamethasone, hexestrol, and cortisone acetate); sex hormones or the derivatives thereof (such as progesterone, estradiol, and estradiol benzoate);

(41) parathyroid hormone (PTH);
(42) calcium receptor blockers
(43) antiepileptic drugs (e.g., phenytoin, pregabalin, carbamazepine etc.),

can be mentioned.
As concomitant drug, various drugs acting on the ventral nervous system, or a therapeutic drug for easily-associated diseases with schizophrenia (therapeutic drug for diabetes etc.) is preferable.
The compound of the present invention can be used in combination with a concomitant drug which particularly does not act for GPR52.

The dosage forms of the compound of the present invention and the concomitant drugs thereof are not specifically limited. Any dosage form may be employed as long as the compound of the present invention is combined with any of the concomitant drugs at the time of administration. Exemplary dosage forms include:

(1) administration of a single medicament prepared by simultaneously formulating the compound of the present invention and the concomitant drug;
(2) administration of two different medicaments by the same administering route at same time, which are independently formulated from the compound of the present invention and the concomitant drug;
(3) administration of two different medicaments by the same administering route at different times, which are independently formulated from the compound of the present invention and the concomitant drug;
(4) administration of two different medicaments by different administering routes at same time, which are independently formulated from the compound of the present invention and the concomitant drug;
(5) administration of two different medicaments by different administering routes at different times, which are independently formulated from the compound of the present invention and the concomitant drug (e.g., the compound of the present invention and the concomitant drug are administered in this order and vice versa); and the like. Hereinafter, these dosage forms are collectively referred to as a combination agent of the present invention.

When the combination agent of the present invention is administered, both the concomitant drug and the compound of the present invention may be simultaneously administered. Alternatively, after the administration of a concomitant drug, the compound of the present invention may be administered. Alternatively, the concomitant drug may be administered after the administration of the compound of the present invention. In the case of administration at different times, the time difference may vary among active ingredients, dosage forms and medication methods. For instance, there is a method in which, when the concomitant drug is administered first, the compound of the present invention is administered after one minute or more but not more than three days, preferably 10 minutes to one day, more preferably 15 minutes to one hour from the administration of the concomitant drug. For instance, there is another method in which, when the concomitant drug is administered after one minute or more but not more than one day, preferably 10 minutes to 6 hours, more preferably 15 minutes to one hour from the administration of the compound of the present invention.

The concomitant drug may be contained in any amount as long as a side effect does not pose a problem. The daily dose of the concomitant drug may vary depending on the target of administration, route of administration, target diseases, symptoms, and so on. For example, when orally administering to a schizophrenia patient (adult, about 60 kg in weight), it is desirable to administer the concomitant drug in general at a unit dose of about 0.1 to about 20 mg/kg weight, preferably about 0.2 to about 10 mg/kg weight, more preferably about 0.5 to about 10 mg/kg weight. The unit dose of the concomitant drug may be preferably administered one to several times (e.g., three times) a day.
When the compound of the present invention is administered in combination with the concomitant drug, the amounts of the respective agents may be reduced within their safe ranges in consideration of their opposing effects.

The concomitant drug of the present invention shows low toxicity and, for example, the compound of the present invention or(and) the above-mentioned concomitant drug is(are) mixed with a pharmacologically acceptable carrier according to a known method and obtained as, for example, tablet (including sugar-coated tablet, film-coated tablet, sublingual tablet, orally disintegrating tablet, buccal tablet and the like), pill, powder, granule, capsule (including soft capsule, microcapsule), troche, syrup, liquid, emulsion, suspension, controlled-release preparation (e.g., immediate-release preparation, sustained-release preparation, sustained-release microcapsule), aerosol, films (e.g., orally disintegrable films, oral cavity mucosa patch film), injection (e.g., subcutaneous injection, intravenous injection, intramuscular injection, intraperitoneal injection), drip infusion, transdermal absorption type preparation, ointment, lotion, adhesive preparation, suppository (e.g., rectal suppository, vaginal suppository), pellet, nasal preparations, pulmonary preparation (inhalant), eye drop and the like. It can be safely administered orally or parenterally (e.g., intravenous, intramuscular, subcutaneous, intraorgan, intranasal, intradermal, instillation, intracerebral, rectal, vaginal, intraperitoneal, intratumor, tumor proximal administration, administration to a lesion and the like).

The pharmaceutically acceptable carrier to be used in the production of the combination agent of the present

invention may be any of those used for the pharmaceutical composition of the present invention.

A blending of the compound of the present invention to the concomitant drug in the combination agent of the present invention can be appropriately determined depending on the target of administration, the route of administration, diseases and the like.

Two or more of the concomitant drugs as described above may be combined together at an appropriate ratio.

The dosage of the concomitant drug can be appropriately determined on the basis of a clinically used dosage. A blending ratio of the compound of the present invention to the concomitant drug can be appropriately determined depending on the target of administration, the route of administration, target diseases, symptoms, combination and the like. For example, if the target of administration is a human, 0.01 to 100 parts by weight of the concomitant drug may be used for one part by weight of the compound of the present invention.

For instance, the content of the compound of the present invention in the combination agent of the present invention varies among the dosage forms. In general, however, the content of the compound of the present invention is in the range of about 0.01 to 99.9% by weight, preferably about 0.1 to 50% by weight, more preferably about 0.5 to 20% by weight with respect to the whole amount of the medicament.

The content of the concomitant drug in the combination agent of the present invention varies among the dosage forms. In general, however, the content of the concomitant drug is in the range of about 0.01 to 99.9% by weight, preferably about 0.1 to about 50% by weight, more preferably about 0.5 to about 20% by weight with respect to the whole amount of the medicament.

The content of any additive such as a carrier in the combination agent of the present invention varies among the dosage forms. In general, however, the content of the additive is in the range of about 1 to 99.99% by weight, preferably about 10 to about 90% by weight with respect to the whole amount of the medicament.

The contents of the compound of the present invention and the concomitant drug may be equal to those described above even if they are independently formulated.

As described above, the dosage varies under various conditions, so that the contents of the compound of the present invention and the concomitant drug may be less than the above dosages or may be higher than the above dosages in some cases.

[Production method]

The production methods of the compound of the present invention are explained below.

Compound (I) and compounds (Ia) - (Io) encompassed in compound (I) can be obtained by, for example, the methods in the following reaction schemes or a method analogous thereto and the like. Each compound of the formula also includes salts, and examples of such salt include those similar to the salts of the aforementioned compound (I) and the like. The compound obtained in each step can be used directly in the form of a reaction mixture or as a crude product for the next reaction. It can also be isolated from the reaction mixture according to a conventional method, and can be easily purified by a means known per se, for example, separation means such as extraction, concentration, neutralization, filtration, distillation, recrystallization, chromatography and the like. Alternatively, when the compound in the formula is commercially available, the commercially available product can also be used as it is.

Compound (I) can be produced, for example, by the following production method 1 or a method analogous thereto. Production method 1

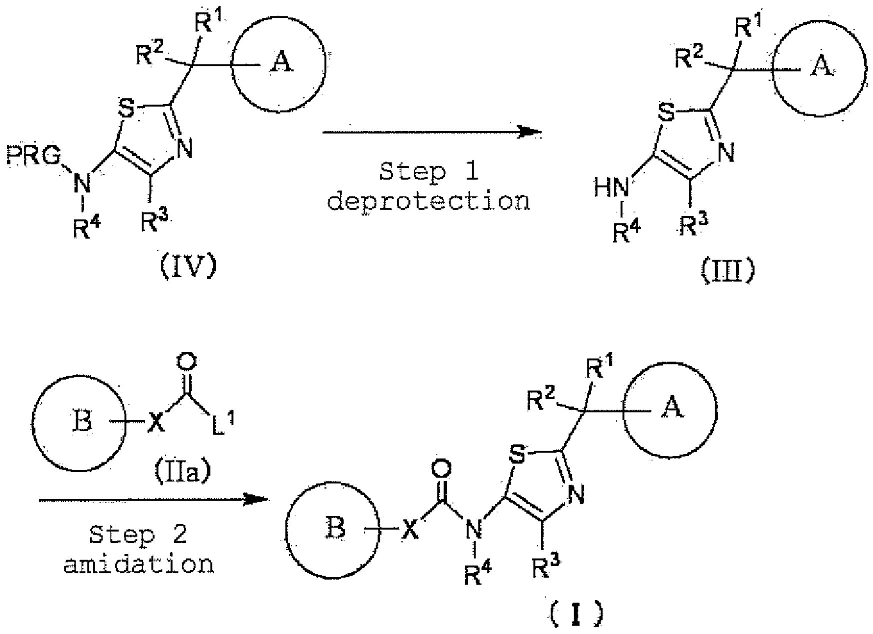


wherein PRG is an amino protecting group, $L^1$ is a leaving group, and other symbols are as defined above.

Examples of the "amino protecting group" for PRG include the protecting groups described in Greene's protective groups in organic synthesis 4th edition. Examples of the amino protecting group include acyl groups such as formyl, $C_{1-6}$ alkyl-carbonyl optionally having substituent(s) (e.g., acetyl, propanoyl, trifluoroacetyl, chloroacetyl and the like), benzoyl, $C_{1-6}$ alkoxy-carbonyl (e.g., methoxycarbonyl, ethoxycarbonyl, tert-butoxycarbonyl and the like), $C_{6-10}$ aryloxy-carbonyl (e.g., phenoxycarbonyl and the like), $C_{7-15}$ aralkyloxy-carbonyl (e.g., benzyloxycarbonyl, 9-fluorenylmethyloxycarbonyl and the like) and the like, hydrocarbon groups such as methyl, benzyl, trityl and the like, and the like. Of these, carbamate type protecting groups such as tert-butoxycarbonyl (Boc) group, benzyloxycarbonyl (Cbz) group, 9-fluorenylmethyloxycarbonyl (Fmoc) group and the like are preferable.

Examples of the "leaving group" represented by $L^1$ include a hydroxy group, a halogen atom (e.g. a fluorine atom, a chlorine atom, a bromine atom, an iodine atom), a $C_{1-6}$ alkoxy group (e.g., methoxy, ethoxy, propoxy, isopropoxy, butoxy, isobutoxy, sec-butoxy, pentyloxy, or hexyloxy) which may be halogenated, a $C_{1-5}$ alkylsulfonyloxy group (e.g., methanesulfonyloxy, ethanesulfonyloxy, or trichloromethanesulfonyloxy) which may be halogenated, a $C_{6-14}$ arylsulfonyloxy group which may be substituted, a phenoxy group which may be substituted, or a benzothiazol-2-ylthio group which may be substituted.

Here, Examples of the "optionally substituted $C_{6-14}$ arylsulfonyloxy group" include $C_{6-14}$ arylsulfonyloxy group (e.g., phenylsulfonyloxy, naphthylsulfonyloxy etc.) optionally having 1 - 3 substituents selected from $C_{1-6}$ alkyl group (e.g., methyl, ethyl, propyl, isopropyl, butyl, isobutyl, sec-butyl, tert-butyl, pentyl, hexyl etc.), $C_{1-6}$ alkoxy group (e.g., methoxy, ethoxy, propoxy, isopropoxy, butoxy, isobutoxy, sec-butoxy, pentyloxy, hexyloxy etc.) and nitro group and the like. Specific examples include phenylsulfonyloxy, m-nitrophenylsulfonyloxy, p-toluenesulfonyloxy and the like.

Examples of the "optionally substituted phenoxy group" include phenoxy group optionally having 1 - 3 substituents selected from $C_{1-6}$ alkyl group (e.g., methyl, ethyl, propyl, isopropyl, butyl, isobutyl, sec-butyl, tert-butyl, pentyl, hexyl etc.), $C_{1-6}$ alkoxy group (e.g., methoxy, ethoxy, propoxy, isopropoxy, butoxy, isobutoxy, sec-butoxy, pentyloxy, hexyloxy etc.) and nitro group and the like. Specific examples include phenoxy, 4-nitrophenoxy and the like.

Examples of the "optionally substituted benzothiazol-2-ylthio group" include benzothiazol-2-ylthio group optionally having 1 - 3 substituents selected from $C_{1-6}$ alkyl group (e.g., methyl, ethyl, propyl, isopropyl, butyl, isobutyl, sec-butyl, tert-butyl, pentyl, hexyl etc.), $C_{1-6}$ alkoxy group (e.g., methoxy, ethoxy, propoxy, isopropoxy, butoxy, isobutoxy, sec-butoxy, pentyloxy, hexyloxy etc.) and nitro group and the like, with preference given to benzothiazol-2-ylthio and the like.

[step 1]

Compound (III) can be produced by the deprotection of compound (IV). For the deprotection, the conditions described in the above-mentioned Greene's protective groups in organic synthesis 4th edition and the like are used. For example, a method using acid, base, reduction, ultraviolet rays, palladium acetate and the like, and the like are used. The reaction is generally performed in a "solvent used for organic synthesis", and a solvent that does not inhibit the reaction is appropriately selected.

As the "solvent used for organic synthesis", alcohols (e.g., methanol, ethanol, propanol, isopropanol, butanol, tert-butanol etc.), ethers (e.g., dioxane, tetrahydrofuran, diethyl ether, tert-butyl methyl ether, diisopropyl ether, ethylene glycol-dimethyl ether etc.), esters (e.g., ethyl formate, ethyl acetate, n-butyl acetate etc.), carboxylic acids (e.g., formic acid, acetic acid, propionic acid etc.), halogenated hydrocarbons (e.g., dichloromethane, chloroform, carbon tetrachloride, trichloroethylene, 1,2-dichloroethane, chlorobenzene etc.), hydrocarbons (e.g., n-hexane, benzene, toluene etc.), amides (e.g., formamide, N,N-dimethylformamide, N,N-dimethylacetamide etc.), ketones (e.g., acetone, methyl ethyl ketone, methyl isobutyl ketone etc.), nitriles (e.g., acetonitrile, propionitrile etc.), sulfoxides (e.g., dimethyl sulfoxide etc.), nitrogen-containing aromatic hydrocarbons (e.g., pyridine, lutidine, quinoline etc.) etc., and sulfolane, hexamethylphosphoramide, water and the like are used singly or as a mixed solvent.
The reaction temperature is generally about -50°C - about 150°C, preferably about -20°C - about 100°C. While the reaction time varies depending on the kind of compound (IV), solvent, base and the like, reaction temperature and the like, it is generally about 1 min - about 100 hr, preferably about 5 min - about 48 hr.
When the amino protecting group is a Boc group, deprotection by a treatment with an acid such as hydrochloric acid, trifluoroacetic acid and the like is preferable. The acid can be used in a molar equivalent to a large excess relative to compound (IV). The solvent may be appropriately selected from the aforementioned "solvent used for organic synthesis" and used. Of these, alcohols such as ethanol and the like, or water is preferable. Where necessary, an acid itself may be used as a solvent.
When the amino protecting group is a Cbz group, for example, deprotection can be performed by catalytic hydrogenation using a metal catalyst. As the catalyst used for catalytic hydrogenation, metals such as palladium, platinum, nickel, rhodium and the like, or oxide thereof, a salt thereof, a complex and the like can be mentioned. These catalysts can also be used by being carried on various carriers such as carbon and the like. In addition, the catalytic hydrogenation can be performed under normal pressure or under pressurization. The solvent can be appropriately selected from the aforementioned "solvent used for organic synthesis" and used. Of these, alcohols such as ethanol and the like, ethers such as tetrahydrofuran and the like or water is preferable.

[step 2]

Compound (I) can be produced by reacting compound (III) with compound (IIa) in the presence of a base or an acid when desired.

Compound (IIa) can be obtained as a commercially available product, or can also be produced according to a method known per se or a method analogous thereto.
The amount of the compound (IIa) used is about 1 to 10 mol, preferably about 1 to 2 mol, per mol of the compound (III).
Examples of the "base" include basic salts such as sodium carbonate, potassium carbonate, cesium carbonate and sodium hydrogen carbonate; aromatic amines such as pyridine and lutidine; tertiary amines such as triethylamine, tripropylamine, tributylamine, N,N-diisopropylethylamine, cyclohexyldimethylamine, 4-dimethylaminopyridine, N-methylpiperidine, N-methylpyrrolidine, N-methylmorpholine and the like; amidines such as DBU (1,8-diazabicyclo[5.4.0]undec-7-ene), DBN (1,5-diazabicyclo[4.3.0]non-5-ene) and the like; alkali metal hydrides such as sodium hydride, potassium hydride and the like; metal amides such as sodium amide, lithium diisopropylamide, lithium hexamethyl disilazide and the like; metal alkoxides such as sodium methoxide, sodium ethoxide, potassium tert-butoxide and the like; and the like. Of these, triethylamine, N,N-diisopropylethylamine is preferable.
The amount of the "base" used is generally about 0.1 to 10, preferably 0.5 to 3 molar equivalents per compound (III).
Examples of the "acid" include mineral acids such as hydrochloric acid, hydrobromic acid, sulfuric acid, nitric acid and the like; sulfonic acids such as methanesulfonic acid, p-toluenesulfonic acid and the like; organic acids such as acetic acid, propionic acid, trifluoroacetic acid and the like; Lewis acids such as zinc chloride, aluminum chloride and the like; and the like.
The amount of the "acid" to be used is generally about 0.1 - 10 molar equivalents, preferably 0.8 - 3 molar equivalents, relative to compound (III).
It is advantageous to carry out the present reaction in the absence of a solvent or in the presence of a solvent inactive to the reaction. Preferable examples of the "solvent inactive to the reaction" include, but not specifically limited as long as the reaction proceeds, water; ethers such as diethyl ether, tetrahydrofuran, 1,4-dioxane and 1,2-dimethoxyethane; hydrocarbons such as benzene, toluene, cyclohexane and hexane; amides such as N,N-dimethylformamide and N,N-dimethylacetamide; halogenated hydrocarbons such as dichloromethane, chloroform, carbon tetrachloride and 1,2-dichloroethane; nitriles such as acetonitrile and propionitrile; sulfoxides such as dimethyl sulfoxide; and nitrogen-containing aromatic hydrocarbons such as pyridine, lutidine and quinoline, or mixtures thereof.
The reaction temperature is generally in the range of about -40 to 150°C, preferably 0 to 100°C. The reaction time is generally in the range of 5 minutes to 24 hours, preferably 10 minutes to 5 hours.

When $L^1$ is a hydroxy group, as a different method, compound (IIa) may be reacted with compound (III) in the presence of a suitable condensing agent, or in the presence of a base when desired. This reaction can be performed,

for example, by the method described in J. Org. Chem., 55, 270 (1990), wherein compound (III) and a condensing agent are used.
Examples of the "condensing agent" include: N,N'-carbodiimides such as N,N'-dicyclohexylcarbodiimide and 1-ethyl-3-(3-dimethylaminopropyl)carbodiimide hydrochloride salt (WSC); azolides such as N,N'-carbonylimidazole; 2-halogenopyridinium salts such as 2-chloro-1-methylpyridinium iodide and 2-fluoro-1-methylpyridinium iodide; aminium condensing agents such as N-[(dimethylamino)(3H-[1,2,3]triazolo[4,5-b]pyridin-3-yloxy)methylidene]-N-methylmethanaminium hexafluorophosphate (HATU), N-[(1H-benzotriazol-1-yloxy)(dimethylamino)methylidene]-N-methylmethanaminium hexafluorophosphate (HTBU) and the like; phosphonium condensing agents such as (1H-benzotriazol-1-yloxy)(tripyrrolidin-1-yl)phosphonium hexafluorophosphate and the like; N,N,N',N'-tetramethylchloroform amidinium hexafluorophosphate (TCFH), N-ethoxycarbonyl-2-ethoxy-1,2-dihydroquinoline, 4-(4,6-dimethoxy-1,3,5-triazin-2-yl)-4-methylmorpholinium chloride n hydrate (DMTMM), diethyl cyanophosphate, phosphorus oxychloride, acetic anhydride and the like. Of these, N,N,N',N'-tetramethylchloroform amidinium hexafluorophosphate (TCFH) and N-[(dimethylamino)(3H-[1,2,3]triazolo[4,5-b]pyridin-3-yloxy)methylidene]-N-methylmethanaminium hexafluorophosphate (HATU) are preferable.
When a carbodiimide condensing agent such as carbodiimide and hydrochloride thereof and the like is used as the "condensing agent", a suitable condensation promoter (e.g., 1-hydroxy-7-azabenzotriazole, 1-hydroxybenzotriazole, N-hydroxysuccinimide, N-hydroxyphthalimide and the like) may be used as necessary.
The amount of compound (IIa) to be used is generally 1 - 10 molar equivalents, preferably 1 - 2 molar equivalents, relative to compound (III).
The amount of the "condensing agent" used is generally 1 to 10 molar equivalents, preferably 1 to 2 molar equivalents, per mol of the compound (III).
The amount of the "condensation promoter" to be used is 0.1 - 10 molar equivalents, preferably 0.3 - 3 molar equivalents, relative to compound (III).
As the solvent, those similar to the "solvent used for organic synthesis" shown in step 1 can be used. Of these, amide solvents such as N,N-dimethylformamide, N,N-dimethylacetamide and the like, and the like are preferable.
This reaction may be performed by adding a suitable base. As the "base", those equivalent to the above-mentioned "base" are used. Preferable examples of the base to be added to the amidation include triethylamine, N,N-diisopropylethylamine and the like.
The amount of the "base" to be used is generally 0.5 - 10 molar equivalents, preferably 0.5 - 2 molar equivalents, relative to compound (III).
The reaction temperature is generally about -100°C - about 150°C, preferably about 0°C - about 100°C. The reaction time is generally about 5 min - about 72 hr, preferably about 1 hr - about 24 hr.
The reaction time can be shortened using a microwave reactor or the like.
The compound (I) thus obtained may be isolated from the reaction mixture by a conventional method and can be easily purified by any of well-known separation techniques, such as concentration, vacuum concentration, solvent extraction, crystallization, recrystallization, transfer dissolution and chromatography.

The production method of compound (IV) is shown below. Compound (IV) is obtained, for example, by the following production method 2 or a method analogous thereto and the like. Production method 2

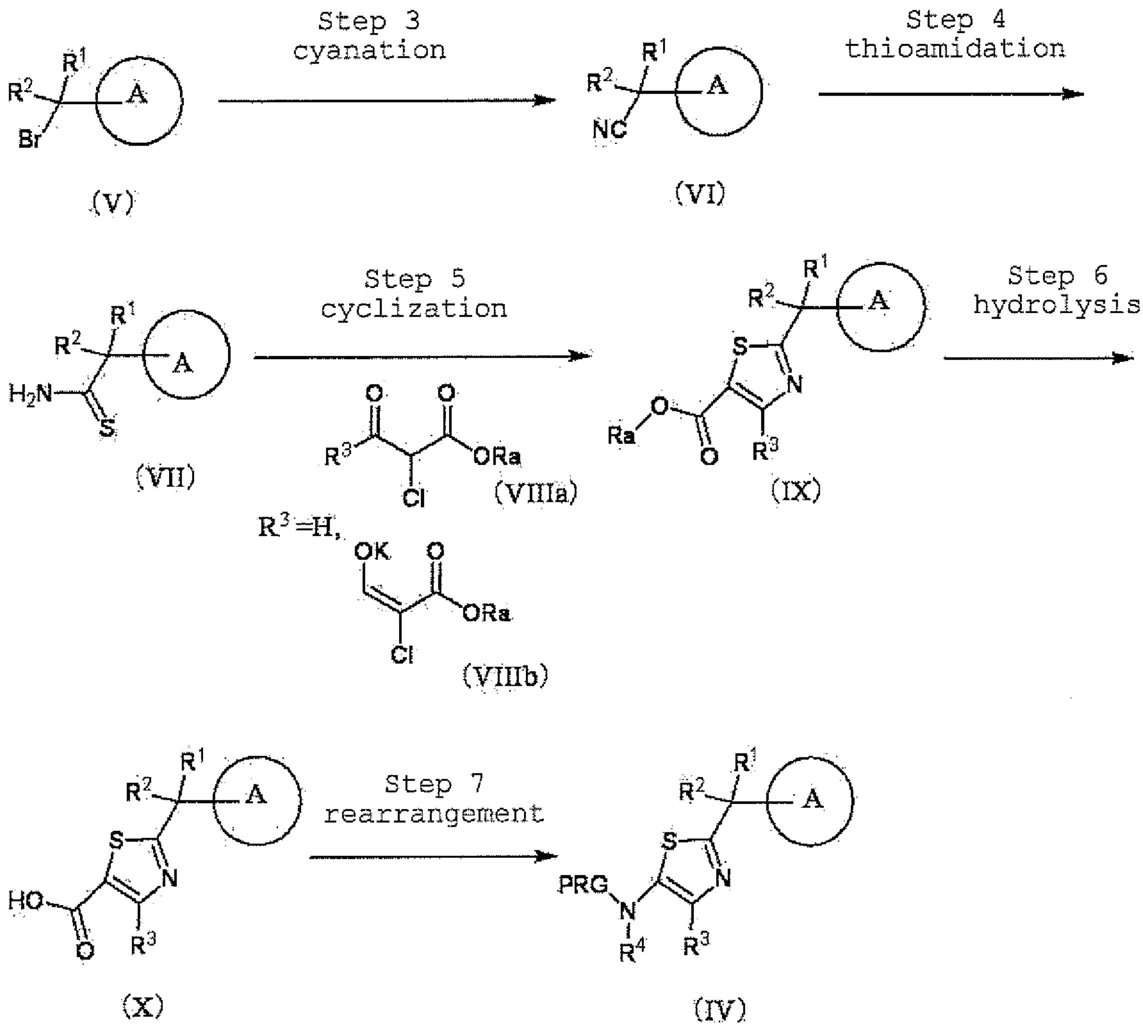


wherein Ra is a $C_{1-6}$ alkyl group, and other symbols are as defined above.

Examples of the "$C_{1-6}$ alkyl group" for Ra include methyl group, ethyl group, propyl group and the like. Of these, methyl group and ethyl group are preferable.

[step 3]

Compound (VI) can be produced by cyanation of compound (V) such as benzyl bromide and the like. The cyanation reaction can be performed, for example, by a method using sodium cyanide, potassium cyanide and the like, a method using trimethylsilyl cyanide and tetrabutylammonium fluoride (the method described in J. Org. Chem., 64, 3171-3177, (1999)), or a method using sodium cyanide and tributylbenzylammonium chloride as a phase-transfer catalyst (the method described in WO2005/51954 J. Org. Chem., 46, 4247-4252(1981)) and the like. Of these, a method using sodium cyanide and tributylbenzylammonium chloride is preferable.
For cyanation reaction, those similar to the "solvent used for organic synthesis" shown in the above-mentioned production method 1, step 1 are used. Of these, alcohols such as methanol, ethanol and the like, ethers such as tetrahydrofuran and the like, amides such as N,N-dimethylformamide and the like, acids such as acetic acid and the like, dimethyl sulfoxide and water, which are used alone or as mixed solvents thereof, are preferable.
The amount of the cyanating agent to be used is preferably 1 - 10 molar equivalents relative to compound (V).
The reaction time is generally about 0.1 - about 72 hr, preferably about 0.1 - about 5 hr. The reaction temperature is generally about -30°C - about 150°C, preferably about 0°C - about 100°C.

[step 4]

Compound (VII) can be produced by thioamidation of compound (VI) such as phenylacetonitrile and the like. As compound (VI), a commercially available product can also be used. The thioamidation reaction can be performed

according to, for example, a method using, in ethanol, hydrogen sulfide and sodium hydrosulfide (the method described in US-A-2005/176776), a method using 0,0-dimethyl dithiophosphate (the method described in US-A-2001/6248766) or a method analogous thereto. Of these, a method using O,O-diethyl dithiophosphate is preferable.
The reaction time is generally about 0.1 - about 100 hr, preferably about 0.1 - about 24 hr. The reaction temperature is generally about -30°C - about 150°C, preferably about 0°C - about 100°C.

[step 5]

Compound (IX) can be produced by a thiazole ring construction reaction of compound (VII) and compound (VIIIa) [when $R^3$ is a hydrogen atom, compound (VIIIb)] (synthesized by the method described in Chemische Berichte, 43, 3529, (1910)). The thiazole ring construction reaction can be performed according to the method described in, for example, Bioorg. Med. Chem. Lett., 15, 5211-5217, (2005). When desired, the compounds may be reacted in the presence of a base.
For the reaction, the "solvent used for organic synthesis" recited in production method 1, step 1, are used. Of these, alcohols such as methanol, ethanol and the like, carboxylic acids such as acetic acid and the like and pyridine, which are used alone or as mixed solvents thereof, are preferable.
The amount of Compound (VIIIa) or compound (VIIIb) to be used is preferably 1 - 1.5 molar equivalents relative to compound (VII).
The reaction time is generally about 0.5 - about 140 hr, preferably about 1 - about 72 hr. The reaction temperature is generally about -100°C - about 250°C, preferably about 0°C - about 150°C.

[step 6]

Compound (X) can be produced by hydrolyzing compound (IX). The hydrolysis can be performed under the alkaline conditions or acidic conditions.
Under the alkaline conditions, the reaction is performed in the presence of a base, in a solvent that does not adversely influence the reaction. As the base, sodium hydroxide, potassium hydroxide, lithium hydroxide, potassium carbonate, sodium carbonate, sodium ethoxide, potassium tert-butoxide and the like can be mentioned.
The amount of the base to be used is preferably about 1 - about 5 molar equivalents relative to compound (IX).
The solvent that does not adversely influence the reaction is selected from the "solvent used for organic synthesis" recited in production method 1, step 1, and used. For example, alcohols such as ethanol and the like, ethers such as tetrahydrofuran and the like, water and the like, which are used alone or as mixed solvents thereof, are preferable.
The reaction temperature is generally about -100°C - about 250°C, preferably about 0°C - about 150°C. The reaction time is generally about 0.1 - about 48 hr.
Under the acidic conditions, the reaction is performed in the presence of an acid, in a solvent that does not adversely influence the reaction. As the acid, mineral acids such as hydrochloric acid, hydrobromic acid, sulfuric acid, phosphoric acid, perchloric acid, nitric acid and the like are preferable.
Examples of the solvent that does not adversely influence the reaction include the "solvent used for organic synthesis" recited in production method 1, step 1. For example, alcohols such as ethanol and the like or water is preferable.
The reaction temperature is generally about -100°C - about 250°C, preferably about 0°C - about 150°C. The reaction time is generally about 0.1 - about 48 hr.

[step 7]

For example, when PRG is a carbamate protecting group, compound (IV) can be produced by Curtius rearrangement of compound (X) in the presence of the corresponding alcohol (for example, tert-butanol, benzyl alcohol etc.). This reaction can be performed according to the method described in, for example, Org. React., 3, 337, (1947) or J. Org. Chem., 52, 4875, (1987). An intermediate acylazide compound can be mostly synthesized by the following 3 methods (methods A - C).

Method A

First, compound (X) is treated with 1 molar equivalent to a large excess of a halogenating agent (for example, thionyl chloride, oxalyl chloride, phosphorus pentachloride and the like) to convert the compound to halogenated acyl. In this reaction, a basic compound such as pyridine, 4-N,N-dimethylaminopyridine, triethylamine and the like may be used in 1 - 10 molar equivalents relative to compound (X). For the reaction, a catalytic amount of N,N-dimethylformamide may be added as a reaction promoter.
Examples of the solvent used in this case include the "solvent used for organic synthesis" recited in production method

1, step 1, and, for example, ethers (e.g., tetrahydrofuran, dioxane, diethyl ether and the like), hydrocarbons (e.g., n-hexane, benzene, toluene etc.), halogenated hydrocarbon (e.g., methylene chloride, chloroform etc.), ketones (e.g., acetone, methyl ethyl ketone and the like), esters (e.g., ethyl acetate and the like), aprotic polar solvent (e.g., N,N-dimethylformamide, dimethyl sulfoxide and the like) and the like can be mentioned.
The reaction temperature is generally about -100°C - 200°C, preferably about -20°C - 100°C. The reaction time is generally about 0.1 - about 48 hr.
The obtained halogenated acyl is reacted with an alkali metal azide salt (for example, sodium azide and the like) in 1 molar equivalent to a large excess relative to compound (X) to produce acylazide. In this reaction, a basic compound such as pyridine, 4-N,N-dimethylaminopyridine, triethylamine and the like may be used in 1 - 10 molar equivalents relative to compound (X).
Examples of the solvent used in this case include ethers (e.g., tetrahydrofuran, dioxane, diethyl ether and the like), halogenated hydrocarbon (e.g., methylene chloride, chloroform etc.), ketones (e.g., acetone, methyl ethyl ketone and the like), esters (e.g., ethyl acetate and the like), aprotic polar solvent (e.g., N,N-dimethylformamide, dimethyl sulfoxide and the like) and the like.
The reaction temperature is generally about -100°C - about 200°C, preferably about -20°C - about 100°C. The reaction time is generally about 0.1 - about 48 hr.

method B

After conversion of compound (X) to halogenated acyl by the method shown in method A, the halogenated acyl is treated with hydrazine in 1 molar equivalent to a large excess relative to compound (X) to give hydrazide. In this reaction, a basic compound such as pyridine, 4-N,N-dimethylaminopyridine, triethylamine and the like may be used in 1 - 10 molar equivalents relative to compound (X).
Examples of the solvent used in this case include ethers (e.g., tetrahydrofuran, dioxane, diethyl ether and the like), halogenated hydrocarbon (e.g., methylene chloride, chloroform etc.), ketones (e.g., acetone, methyl ethyl ketone and the like), esters (e.g., ethyl acetate and the like), aprotic polar solvent (e.g., N,N-dimethylformamide, dimethyl sulfoxide and the like) and the like.
The reaction temperature is generally about -100°C - about 200°C, preferably about -20°C - about 100°C. The reaction time is generally about 0.1 - about 48 hr.
The obtained hydrazide is treated with nitrous acid (can also be developed from metal nitrite such as sodium nitrite and the like in the presence of acid) in 1 molar equivalent to a large excess relative to compound (X) to produce acylazide.
Examples of the solvent used in this case include the "solvent used for organic synthesis" recited in production method 1, step 1, and, for example, water, alcohols (e.g., methanol, ethanol and the like), ethers (e.g., tetrahydrofuran, dioxane, diethyl ether and the like), halogenated hydrocarbon (e.g., methylene chloride, chloroform etc.), ketones (e.g., acetone, methyl ethyl ketone and the like), esters (e.g., ethyl acetate and the like), aprotic polar solvent (e.g., N,N-dimethylformamide, dimethyl sulfoxide and the like) and the like can be mentioned.
The reaction temperature is generally about -100°C - about 200°C, preferably about -20°C - about 50°C. The reaction time is generally about 0.1 - about 48 hr.

method C

As described in, for example, Org. Lett., 4, 3663, (2002), compound (X) is reacted with 1 molar equivalent to a large excess of diphenylphosphoryl azide (DPPA) to produce acylazide. In this case, for example, a basic compound such as sodium hydroxide, potassium hydroxide, sodium hydride, potassium carbonate, triethylamine, N,N-diisopropylethylamine, pyridine, 4-N, N-dimethylaminopyridine (DMAP) , 1,8-diazabicyclo[5.4.0]-7-undecene, 1,4-diazabicyclo[2.2.2]octane (DABCO) and the like may be used in 1 - 10 molar equivalents relative to compound (X).
Examples of the solvent used in this case include the "solvent used for organic synthesis" recited in production method 1, step 1, and, for example, water, alcohols (e.g., methanol, ethanol and the like), ethers (e.g., tetrahydrofuran, dioxane, diethyl ether and the like), hydrocarbons (e.g., n-hexane, benzene, toluene etc.), halogenated hydrocarbon (e.g., methylene chloride, chloroform etc.), ketones (e.g., acetone, methyl ethyl ketone and the like), esters (e.g., ethyl acetate and the like), aprotic polar solvent (e.g., N,N-dimethylformamide, dimethyl sulfoxide and the like) and the like can be mentioned.
The reaction time is generally about 10 min - about 24 hr, preferably about 0.5 - about 6 hr. The reaction temperature is generally about -20°C - about 200°C, preferably about -20°C - about 100°C.

The obtained acylazide is subjected to Curtius rearrangement to induce isocyanate, and the obtained isocyanate is reacted with a desired alcohol to synthesize compound (IV) as a carbamate compound.

In addition, by performing Curtius rearrangement using acylazide in the presence of alcohol, compound (IV) can be synthesized as a desired carbamate compound. For example, when tert-butanol is co-present, a compound

wherein PRG is a Boc group can be synthesized, and when benzyl alcohol is co-present, compound (IV) wherein PRG is a Cbz group can be synthesized.
The reaction is performed by heating the obtained acylazide to 30°C - 200°C. In this case, for example, a basic compound such as triethylamine, N,N-diisopropylethylamine, pyridine, 4-N,N-dimethylaminopyridine (DMAP), 1,8-diazabicyclo [5.4.0]-7-undecene, 1,4-diazabicyclo[2.2.2]octane (DABCO) and the like may be used in 1 - 10 molar equivalents relative to compound (V). Examples of the solvent used in this case include the "solvent used for organic synthesis" recited in step 1, and, for example, water, alcohols (e.g., methanol, ethanol, tert-butanol and the like), ethers (e.g., tetrahydrofuran, dioxane, diethyl ether and the like), hydrocarbons (e.g., n-hexane, benzene, toluene etc.), halogenated hydrocarbon (e.g., methylene chloride, chloroform etc.), ketones (e.g., acetone, methyl ethyl ketone and the like), esters (e.g., ethyl acetate and the like), and aprotic polar solvents (e.g., N,N-dimethylformamide, dimethyl sulfoxide and the like) and the like can be mentioned.
The reaction time is generally about 10 min - about 24 hr, preferably about 0.5 - about 6 hr. The reaction temperature is generally about -20°C - about 200°C, preferably about 0°C - about 150°C.
The series of reaction from compound (X) to compound (IV) can also be performed in the same system without isolating each compound.
Of these three methods, method C using diphenylphosphoryl azide (DPPA) is more preferable.
When PRG is other than carbamate protecting groups, for example, the protecting group of compound (IV) having a carbamate protecting group and produced by the above-mentioned Curtius rearrangement reaction is changed to give compound (IV).

Of compounds (I), the following compounds (Ib) - (Ie) can be produced, for example, by the following production method 3 or a method analogous thereto.

Production method 3

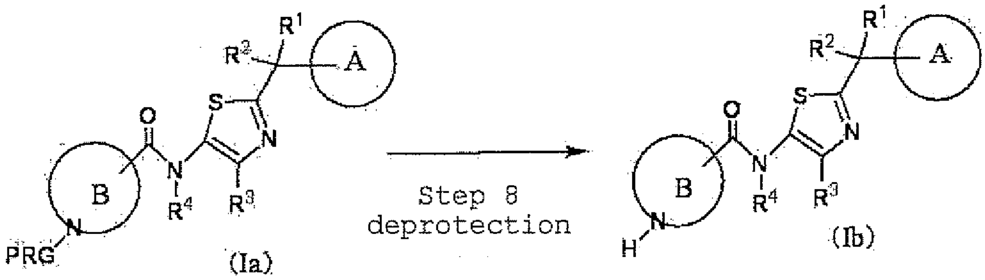


(Ia) (Ib)

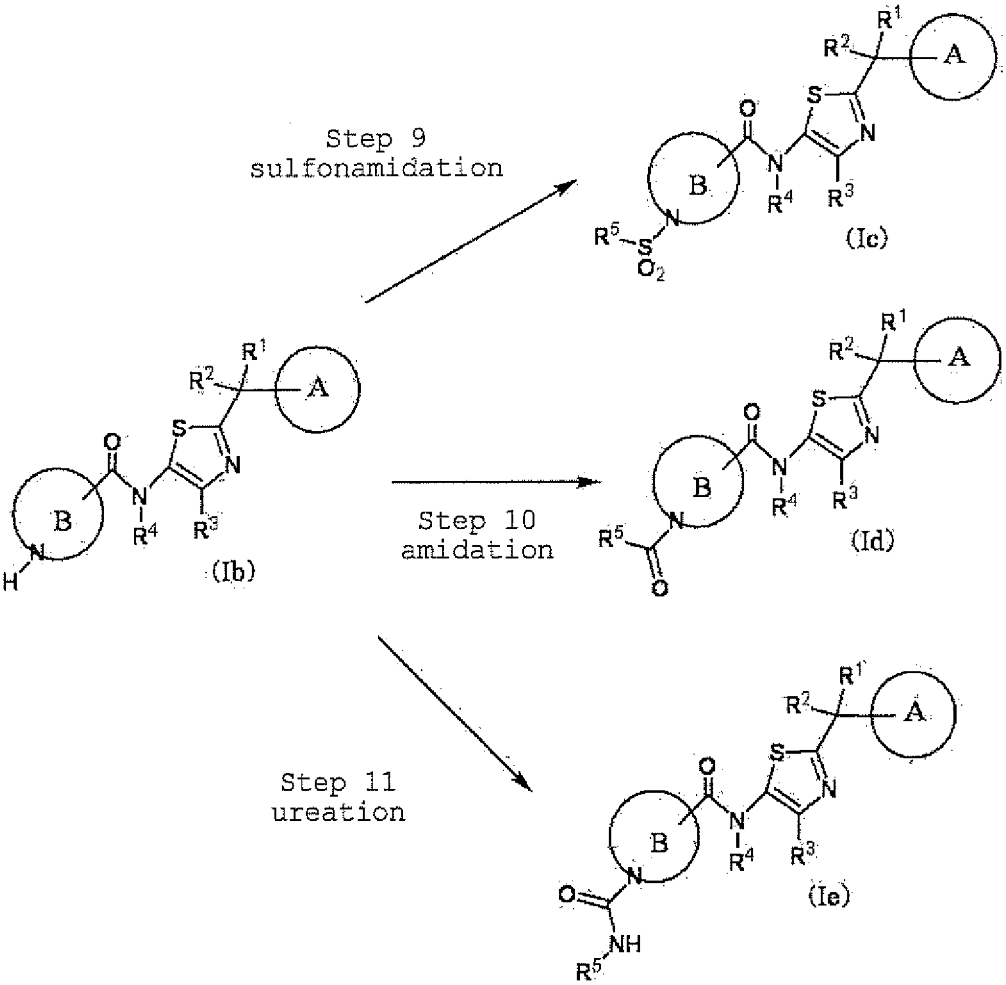


(Ib) (Ic) (Id) (Ie)

wherein $R^5$ is an optionally substituted $C_{1-6}$ alkyl group, and other symbols are as defined above.

[step 8]

Of compound (I), compound (Ib) wherein ring B is particularly 3 - 11-membered nitrogen-containing heterocycle can be produced by deprotection of compound (Ia).
As PRG in compound (Ia), a carbamate protecting group such as Boc group, Cbz group, Fmoc group and the like is preferable.
In such deprotection, a method similar to the deprotection shown in production method 1, step 1, is used. Of these, when PRG is a Boc group, deprotection by a treatment with an acid such as hydrochloric acid, trifluoroacetic acid and the like is preferable. The acid can be used in 1 molar equivalent to a large excess relative to compound (Ia) .
The solvent is appropriately selected from the "solvent used for organic synthesis" recited in production method 1, step 1, and used. Of these, alcohols such as ethanol and the like, or water is preferable. Where necessary, an acid itself may be used as a solvent.
When PRG is a Cbz group, for example, deprotection can be performed by catalytic hydrogenation using a metal catalyst.

As the catalyst used for the catalytic hydrogenation, metals such as palladium, platinum, nickel, rhodium and the like or oxides, salt, complex and the like thereof can be mentioned. These catalysts can also be used by carrying on various carriers such as carbon and the like. The catalytic hydrogenation can be performed under normal pressure or under pressurization. The solvent is appropriately selected from the "solvent used for organic synthesis" recited in production method 1, step 1, and used. Of these, alcohols such as ethanol and the like, ethers such as tetrahydrofuran and the like or water is preferable.

[step 9]

Compound (Ic) can be produced by sulfonamidation reaction of compound (Ib). The sulfonamidation reaction can be performed, for example, by reacting compound (Ib) with the corresponding sulfonyl chloride according to the method described in J. Med. Chem., 58, 3429-3434, (1993). In this case, for example, a basic compound such as sodium hydroxide, potassium hydroxide, sodium hydride, potassium carbonate, triethylamine, N,N-diisopropylethylamine, pyridine, 4-N,N-dimethylaminopyridine (DMAP), 1,8-diazabicyclo[5.4.0]-7-undecene, 1,4-diazabicyclo[2.2.2]octane (DABCO) and the like may be used in 1 - 10 molar equivalents relative to compound (Ib).
The amount of sulfonyl chloride to be used is preferably 1 - 5 molar equivalents relative to compound (Ib).
For the reaction, the "solvent used for organic synthesis" recited in production method 1, step 1, is used, and ethers such as tetrahydrofuran and the like, amides such as N,N-dimethylamide and the like, or dimethylsulfoxide is preferable.
The reaction time is generally about 0.5 - about 140 hr, preferably about 1 - about 24 hr. The reaction temperature is generally about -100°C - about 250°C, preferably about 0°C - about 100°C.

[step 10]

Compound (Id) can be produced by an amidation reaction of compound (Ib). The amidation reaction can be performed by a method similar to the amidation reaction shown in production method 1, step 2, and a reaction reagent and the like can also be combined as appropriate.

[step 11]

Compound (Ie) can be produced by a ureation reaction of compound (Ib). The ureation reaction can be performed, for example, by reacting compound (Ib) with the corresponding isocyanate according to the method described in J. Med. Chem., 33, 464-479, (1990). For the reaction, the "solvent used for organic synthesis" shown in the above-mentioned production method 1, step 1 are used. Of these, ethers such as tetrahydrofuran and the like, or toluene is preferable.
The amount of isocyanate to be used is preferably 1 - 10 molar equivalents relative to compound (Ib).
The reaction time is generally about 0.5 - about 140 hr, preferably about 1 - about 24 hr. The reaction temperature is generally about -100°C - about 250°C, preferably about 0°C - about 100°C.
Compound (If) can be produced by, for example, the following production method 4 or a method analogous thereto. Production method 4
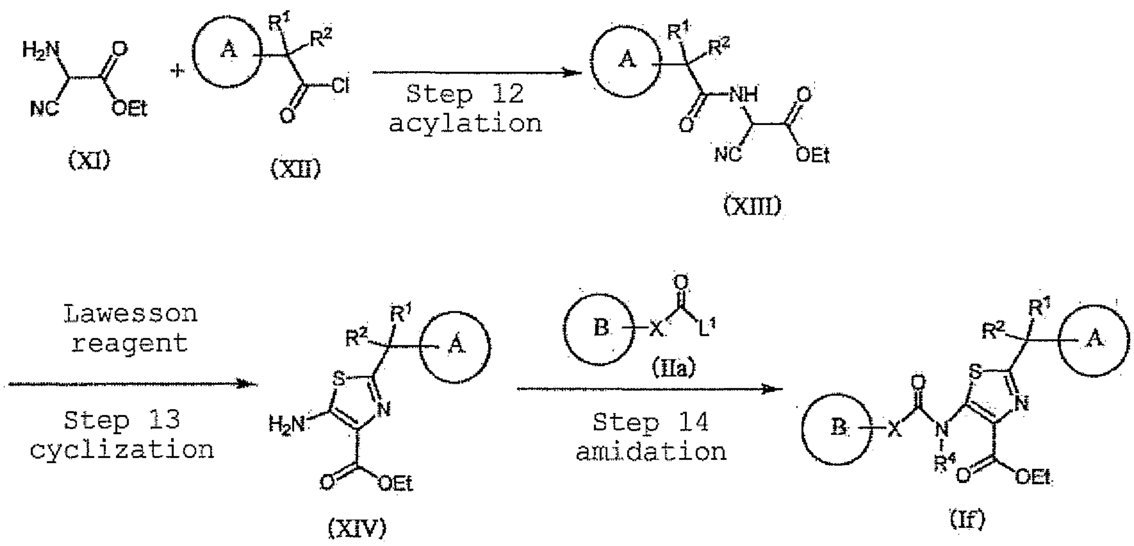


(XI) (XII) (XIII) (XIV) (If)

wherein each symbol is as defined above.

[step 12]

Compound (XIII) can be produced by an acylation reaction of compound (XI) (synthesized by the method described in J. Med. Chem., 40, 2196-2210, (1997)) with acid chloride (XII). The acid chloride (XII) is available as a commercial product, or can be produced by reacting the corresponding carboxylic acid with thionyl chloride, oxalyl chloride and the like according to the methods described in J. Med. Chem., 33, 1496-1504, (1990), J. Med. Chain., 34, 397-403, (1991) and the like.
The amidation reaction can be performed, for example, according to the method described in Chimia, 57, 248-254, (2003). For the reaction, the "solvent used for organic synthesis" shown in the above-mentioned production method 1, step 1 are used. Of these, ethers such as tetrahydrofuran and the like, and halogenated hydrocarbons such as dichloromethane, chloroform and the like are preferable.
The amount of acid chloride (XII) to be used is preferably 1 - 5 molar equivalents relative to compound (XI).
The reaction time is generally about 0.5 - about 140 hr, preferably about 1 - about 24 hr. The reaction temperature is generally about -100°C - about 250°C, preferably about 0°C - about 100°C.

[step 13]

Compound (XIV) can be produced by reacting compound (XIII) with a Lawesson reagent to construct a thiazole ring.
The reaction can be performed according to the method described in, for example, WO 2008/59368 or Nucleosides & Nucleotides, 14, 313-316, (1995). For the reaction, the "solvent used for organic synthesis" shown in the above-mentioned production method 1, step 1, are used. Of these, hydrocarbons such as benzene, toluene and the like are preferable.
The amount of the Lawesson reagent to be used is preferably 0.5 - 20 molar equivalents relative to compound (XIII).
The reaction time is generally about 0.5 - about 140 hr, preferably about 1 - about 24 hr. The reaction temperature is generally about -100°C - about 250°C, preferably about 0°C - about 120°C.

[step 14]

Compound (If) can be produced by an amidation reaction of compound (XIV) with compound (IIa). The amidation reaction can be performed by a method similar to the amidation shown in production method 1, step 2, and a reaction reagent and the like can also be combined as appropriate.
Compounds (Ig) - (Ii) can be produced by, for example, the following production method 5 or a method analogous thereto.

Production method 5

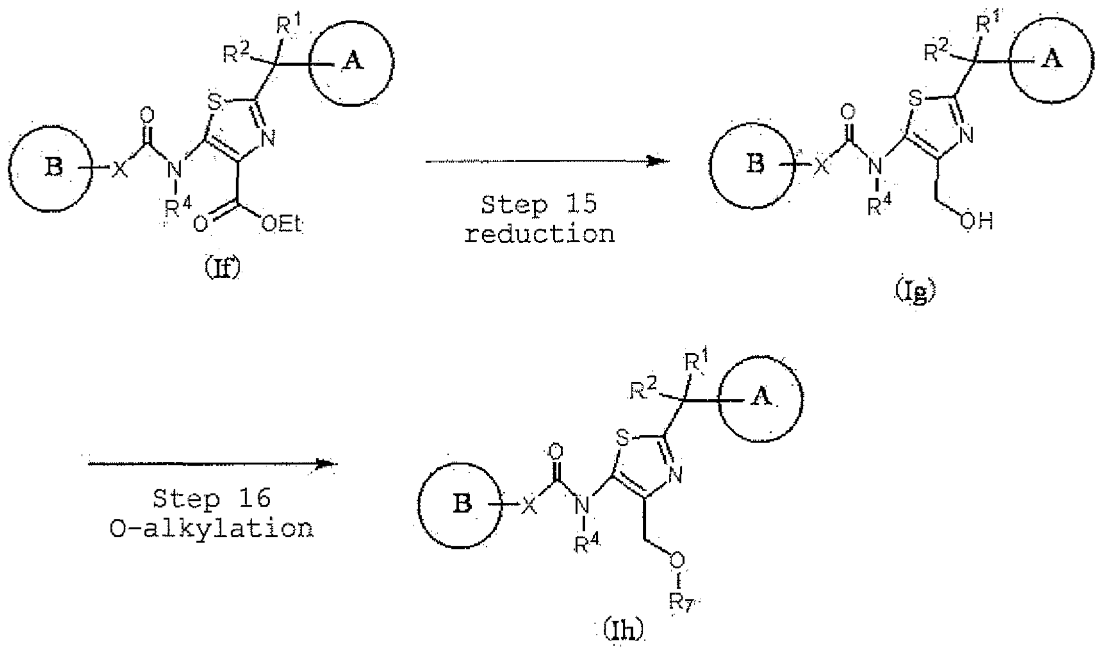


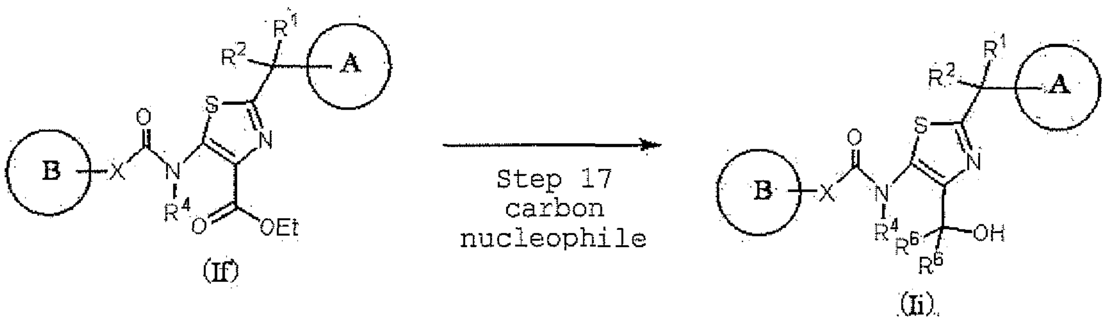


wherein $R^6$ is alkyl optionally having substituent(s) (wherein two $R^6$ each form $C_{1-6}$ alkyl optionally having substituent(s) together with the adjacent carbon atom), $R^7$ is $C_{1-6}$ alkyl optionally having substituent(s), and other symbols are as defined above.

[step 15]

Compound (Ig) can be produced by reduction of compound (If). In this reaction, a reducing agent is used in 0.1 molar equivalent to a large excess (preferably 0.3 - 10 molar equivalents) relative to compound (If). Examples of the reducing agent include lithium aluminum hydride, sodium borohydride, lithium borohydride, diisobutylaluminum hydride, calcium borohydride, and borane complex (borane-THF complex etc.). Of these, diisobutylaluminum hydride is preferable. The solvent is appropriately selected from, for example, the "solvent used for organic synthesis" recited in production method 1, step 1, and used. Of these, ether solvents such as tetrahydrofuran and the like, alcohol solvents such as methanol and ethanol and the like are preferable.
The reaction time is generally about 0.1 - about 72 hr, preferably about 0.3 - about 24 hr. The reaction temperature is generally about -80°C - about 150°C, preferably about -30°C - about 100°C.

[step 16]

Compound (Ih) can be produced by O-alkylation of compound (Ig). This reaction can be performed by treating with an acid such as sulfuric acid, hydrochloric acid and the like according to the method described in, for example, J. Med. Chem., 10, 932-936 (1967), in the presence of the corresponding alcohol ($R^7$-OH).
The amount of alcohol ($R^7$-OH) to be used is 1 molar equivalent to a large excess relative to compound (Ih).
The amount of the acid to be used is 0.01 - 10 molar equivalents relative to compound (Ih).
The reaction time is about 0.1 - about 72 hr, preferably about 0.3 - about 24 hr. The reaction temperature is generally

about -80°C - about 150°C, preferably about -30°C - about 100°C.

[step 17]

Compound (Ii) can be produced by, for example, reacting compound (If) with carbon nucleophile.
As the "carbon nucleophile", an organic lithium reagent such as alkyllithium, aryllithium and the like, an organic magnesium reagent such as alkylmagnesium bromide, arylmagnesium bromide and the like, an alkyl nitro compound such as nitromethane and the like, an enol ether compound such as alkyl enol ether, alkyl silyl enol ether and the like, an active methylene compound such as malonic acid ester, acetoacetic acid ester, 1,3-dithiane and the like, and the like can be mentioned. Of these, an organic lithium reagent (for example, methyllithium and the like) or an organic magnesium reagent (e.g., methylmagnesium bromide and the like) is preferable. The amount of "carbon nucleophile" to be used is 0.1 - 20 molar equivalents, preferably 1 - 10 molar equivalents, relative to compound (If).
This reaction is performed in the presence of a base as necessary. Examples of the "base" include those similar to the "base" in production method 1, step 2. Of these, hydride of alkali metal or alkaline earth metal such as sodium hydride and the like, amide of alkali metal or alkaline earth metal such as sodium amide and the like, and lower alkoxide of alkali metal or alkaline earth metal such as potassium tert-butoxide and the like are preferable. The amount of the "base" to be used is 0.1 - 10 molar equivalents, preferably 0.3 - 3 molar equivalents, relative to compound (If).
As the solvent, those similar to the "solvent used for organic synthesis" shown in production method 1, step 1, can be used. Of these, ether solvents such as tetrahydrofuran and the like are preferable.
The reaction temperature is generally about -80°C - about 150°C, preferably about -78°C - about 80°C. The reaction time is generally about 0.1 " about 72 hr, preferably about 0.3 - about 24 hr.
Compound (Ij) and (Ik) can be produced by, for example, the following production method 6 or a method analogous thereto.

production method 6

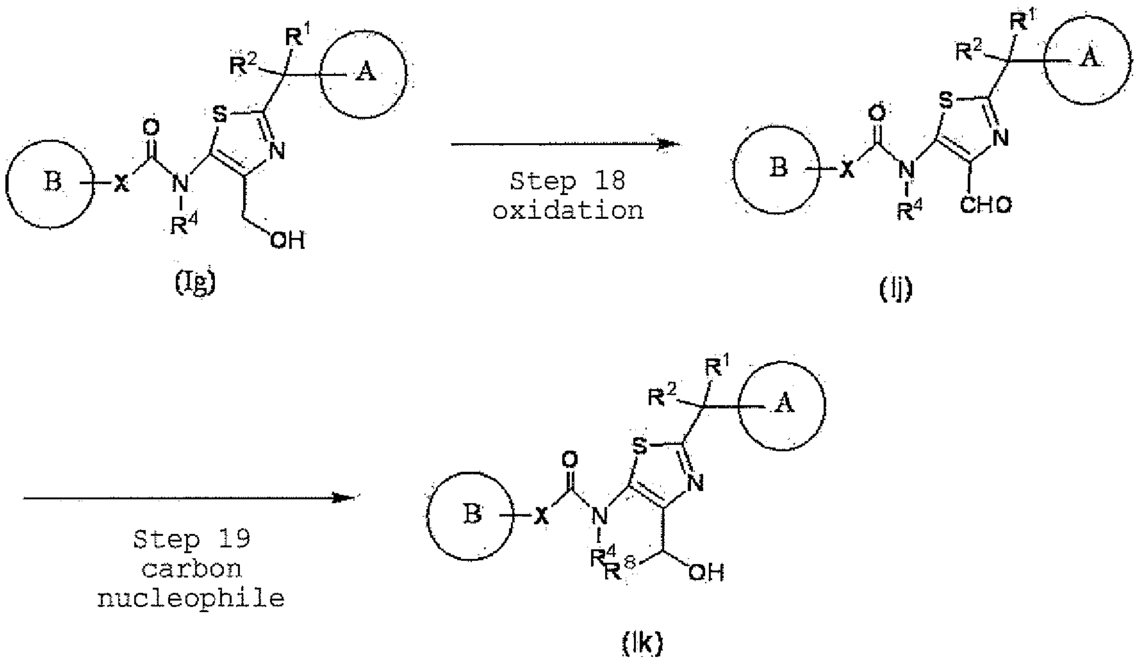


(Ig) (Ij) (Ik)

wherein $R^8$ is a $C_{1-5}$ alkyl group optionally having substituent(s), and other symbols are as defined above.

[step 18]

Compound (Ij) can be produced by, for example, oxidation reaction of compound (Ig). Examples of the oxidation reaction include a method using pyridinium chlorochromate (PCC) or pyridinium dichromate (PDC), swern oxidation using dimethyl sulfoxide as an oxidant, oxidation with a pyridine-sulfur trioxide complex, oxidation with tetrapropylammonium perruthenate (TPAP) and N-methylmorpholine N-oxide (NMO), Dess-Martin oxidation using 1,1,1-triacetoxy-1,1-dihydro-1,2-benziodoxol-3(1H)-one (Dess-Martin Periodinane) (Dess-Martin reagent), oxidation using 2,2,6,6-tetramethylpiperidin-1-oxyl (TEMPO) and sodium hypochlorite, Mukaiyama oxidation using N-tert-butylbenzenesulfinimidoyl chloride and 1,8-diazabicyclo[5.4.0]undec-7-ene (DBU) and the like. Of these, Dess-Martin oxidation is preferable. The amount of the Dess-Martin reagent to be used is 0.1 - 10 molar equivalents, preferably 1 - 3 molar equivalents, relative to compound (Ii).
As the solvent, those similar to the "solvent used for organic synthesis" shown in production method 1, step 1, can be used. Of these, halogenated hydrocarbon such as dichloromethane and the like, nitriles such as acetonitrile and the like, amides such as N,N-dimethylformamide and the like, which are used alone or as mixed solvents thereof, are preferable.
The reaction temperature is generally about -80°C - about 150°C, preferably about -30°C - about 100°C. The reaction time is generally about 0.1 - about 72 hr, preferably about 0.3 - about 24 hr.

[step 19]

Compound (Ik) can be produced by, for example, reacting compound (Ij) with carbon nucleophile. As the "carbon nucleophile", those similar to the "carbon nucleophile" in production method 5, step 17, are used. Of these, organic lithium reagent (for example, methyllithium and the like) and organic magnesium reagent (e.g., methylmagnesium bromide, ethylmagnesium bromide, n-propylmagnesium bromide and the like) are preferable. The amount of the "carbon nucleophile" to be used is 0.1 - 20 molar equivalents, preferably 1 - 10 molar equivalents, relative to compound (Ij).
This reaction is performed in the presence of a base as necessary. Examples of the "base" include those similar to the "base" in production method 1, step 2. Of these, hydride of alkali metal or alkaline earth metal such as sodium hydride and the like, amide of alkali metal or alkaline earth metal such as sodium amide and the like, and lower alkoxide of alkali metal or alkaline earth metal such as potassium tert-butoxide and the like are preferable. The amount of the "base" to be used is 0.1 - 10 molar equivalents, preferably 0.3 - 3 molar equivalents, relative to compound (Ij).
As the solvent, those similar to the "solvent used for organic synthesis" shown in production method 1, step 1, can be used. Of these, ether solvents such as tetrahydrofuran and the like are preferable.
The reaction temperature is generally about -80°C - about 150°C, preferably about -78°C - about 80°C. The reaction time is generally about 0.1 - about 72 hr, preferably about 0.3 - about 24 hr.
Compound (I1) - (In) can be produced by, for example, the following production method 7 or a method analogous thereto. Production method 7
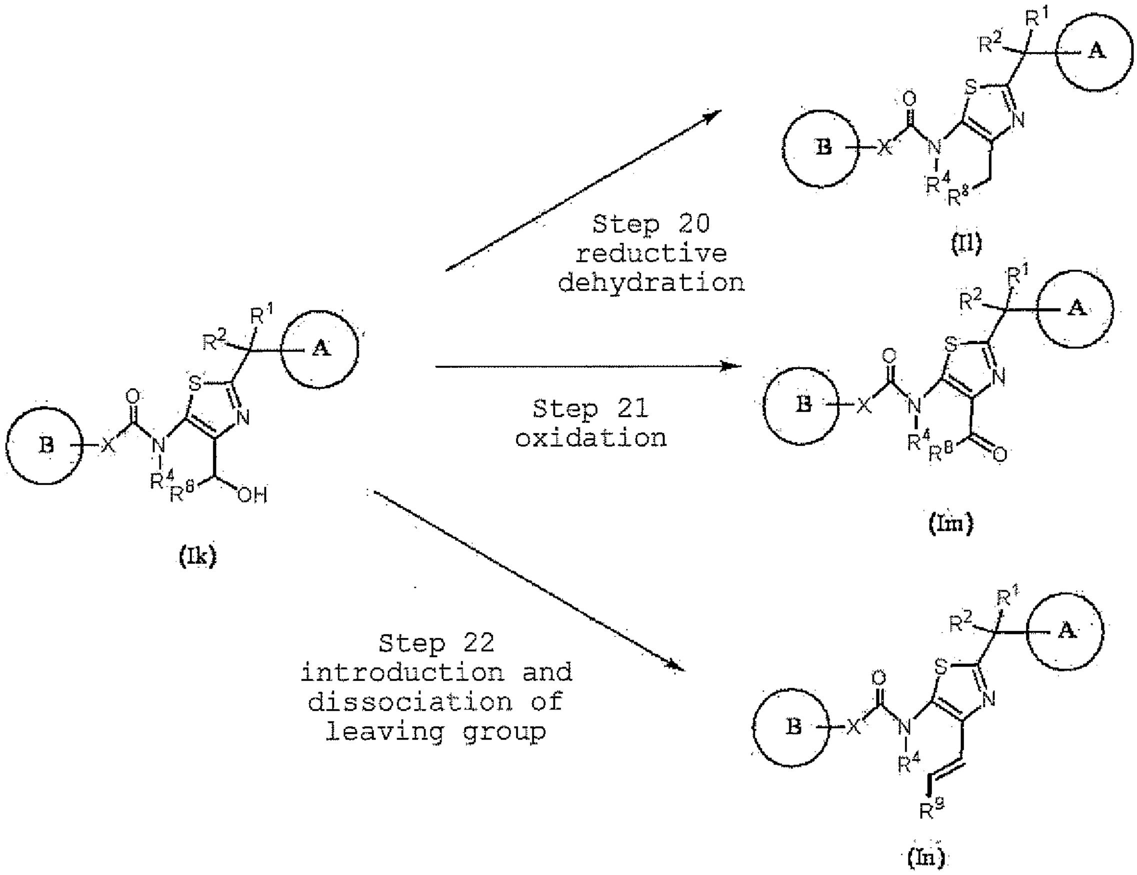


(Ik) (I1) (Im) (In)

wherein $R^9$ is a $C_{1-4}$ alkyl group optionally having substituent(s), and other symbols are as defined above.

[step 20]

Compound (I1) can be produced by, for example, subjecting compound (Ik) to a reductive dehydrating reaction. As the reductive dehydrating reaction, a catalytic reduction method known per se, a method using an organic silyl reagent (alkylsilane reagent etc.) and the like can be mentioned.
In the catalytic reduction method, compound (Ik) is reacted with a metal catalyst under a hydrogen atmosphere to give compound (I1). In the reaction, a suitable acid catalyst may be added when desired.
Examples of the "metal catalyst" include Raney-nickel, platinum oxide, metal palladium, palladium on activated carbon and the like. The amount of the "metal catalyst" to be used is generally about 1 to about 1000 wt%, preferably about 5 to about 20 wt%, relative to compound (Ik).
Examples of the "acid catalyst" include organic acids such as formic acid, acetic acid, trifluoroacetic acid, p-toluenesulfonic acid and the like; mineral acids such as sulfuric acid, hydrochloric acid, hydrobromic acid, nitric acid and the like; and the like. The amount of the "acid catalyst" to be used is about 0.1 molar equivalent to excess amount, relative to compound (Ik).
This reaction is advantageously carried out in a solvent inert to the reaction. The solvent is not particularly limited as long as the reaction proceeds, and preferable examples thereof include alcohols such as methanol, ethanol, propanol and the like; ethers such as diethyl ether, tetrahydrofuran, dioxane, 1,2-dimethoxyethane and the like; hydrocarbons such as benzene, toluene, cyclohexane, hexane and the like; amides such as N,N-dimethylformamide, N,N-da.methylacetamide and the like; organic acids such as acetic acid and the like; water; a mixed solvent thereof; and the like.
The hydrogen pressure is generally about 1 to about 100 pressure, preferably about 1 to about 5 pressure.
The reaction time is generally about 30 min to about 48 hr, preferably about 1 to 24 hr. The reaction temperature is generally about 0 to about 120°C, preferably about 20 to about 80°C.

In the method using an organic silyl reagent (an alkylsilane reagent), compound (I1) can be produced by reacting compound (Ik) with an organic silyl reagent (alkylsilane reagent etc.) and an acid.
Examples of the "alkylsilane reagent" include triethylsilane, phenyldimethylsilane and the like. The amount of the organic silyl reagent (alkylsilane reagent etc.) to be used is generally about 0.8 - about 20 molar equivalents, preferably about 1 - about 10 molar equivalents, relative to compound (Ik).

Examples of the "acid" include organic acids such as trifluoroacetic acid and the like. The amount of the acid to be used is generally about 0.1 molar equivalent to an excess amount relative to compound (Ik).
This reaction is advantageously carried out without a solvent or in a solvent inert to the reaction. The solvent is not particularly limited as long as the reaction proceeds, and preferable examples thereof include ethers such as diethyl ether, tetrahydrofuran, dioxane, 1,2-dimethoxyethane and the like; hydrocarbons such as benzene, toluene, cyclohexane, hexane and the like; organic acids such as acetic acid, trifluoroacetic acid and the like; a mixed solvent thereof; and the like.

[step 21]

Compound (Im) can be produced by, for example, by subjecting compound (Ik) to an oxidation reaction.
As such oxidation reaction, a method similar to the oxidation reaction in production method 6, step 18, can be mentioned. Of them, Dess-Martin oxidation using a Dess-Martin reagent is preferable.
The amount of the Dess-Martin reagent to be used is generally 0.1 - 10 molar equivalents, preferably 1 - 3 molar equivalents, relative to compound (Ik).
As the solvent, those similar to the "solvent used for organic synthesis" shown in production method 1, step 1, can be used. Of these, halogenated hydrocarbon such as dichloromethane and the like, nitriles such as acetonitrile and the like, amides such as N,N-dimethylformamide and the like, which are used alone or as mixed solvents thereof, are preferable.
The reaction temperature is generally about -80°C - about 150°C, preferably about -30°C - about 100°C. The reaction time is generally about 0.1 - about 72 hr, preferably about 0.3 - about 24 hr.

[step 22]

Compound (In) can be produced by introduction of a leaving group into compound (Ik) and a dissociation reaction thereof.
Examples of the "leaving group" include optionally halogenated $C_{1-5}$ alkylsulfonyloxy group (e.g., methanesulfonyloxy, ethanesulfonyloxy, trichloromethanesulfonyloxy etc.), optionally substituted $C_{6-14}$ arylsulfonyloxy group and the like. Of these, methanesulfonyloxy group and 4-methylbenzenesulfonyloxy group are preferable.
The leaving group can be introduced by, for example, reacting with the corresponding sulfonyl chloride according to the method described in J. Org. Chem., 44, 3938-3945, (1979), in the presence of a base. Examples of the "base" include basic salts such as sodium carbonate, potassium carbonate, cesium carbonate, sodium hydrogen carbonate and the like, organic bases such as aromatic amines such as pyridine, lutidine and the like, tertiary amines such as triethylamine, tripropylamine, tributylamine, N,N-diisopropylethylamine, cyclohexyldimethylamine, 4-dimethylaminopyridine, N-methylpiperidine, N-methylpyrrolidine, N-methylmorpholine and the like, amidines such as DBU (1,8-diazabicyclo[5.4.0]undec-7-ene), DBN (1,5-diazabicyclo[4.3.0]non-5-ene) and the like, basic heterocyclic compounds such as pyridine, dimethylaminopyridine, imidazole, 2,6-lutidine and the like, and the like. Of these, DBU is preferable. The amount of the base to be used is about 0.1 molar equivalents to an excess amount relative to compound (Ik).
As the solvent, those similar to the "solvent used for organic synthesis" shown in production method 1, step 1 can be used. Of these, halogenated hydrocarbon such as dichloromethane and the like, ethers such as THF and the like, hydrocarbon solvents such as toluene and the like are preferable.
The reaction temperature is generally about -80°C - about 150°C, preferably about -30°C - about 100°C. The reaction time is generally about 0.1 - about 72 hr, preferably about 0.3 - about 24 hr.
The dissociation reaction of the leaving group can be performed according to the method described in J. Med. Chem., 44, 4207-4215, (2001).
As the "base", those similar to the "base" shown for the aforementioned introduction of the leaving group can be mentioned. Of these, DBU is preferable. The amount of the base to be used is about 0.1 molar equivalent to an excess amount relative to compound (Ik).
As the solvent, those similar to the "solvent used for organic synthesis" shown in production method 1, step 1 can be used. Of these, halogenated hydrocarbon such as dichloromethane and the like, ethers such as THF and the like, hydrocarbon solvents such as toluene and the like are preferable.
The reaction temperature is generally about -80°C - about 150°C, preferably about -30°C - about 100°C. The reaction time is generally about 0.1 - about 72 hr, preferably about 0.3 - about 24 hr.
Compound (Ip) can be produced by, for example, the following production method 8 or a method analogous thereto. Production method 8
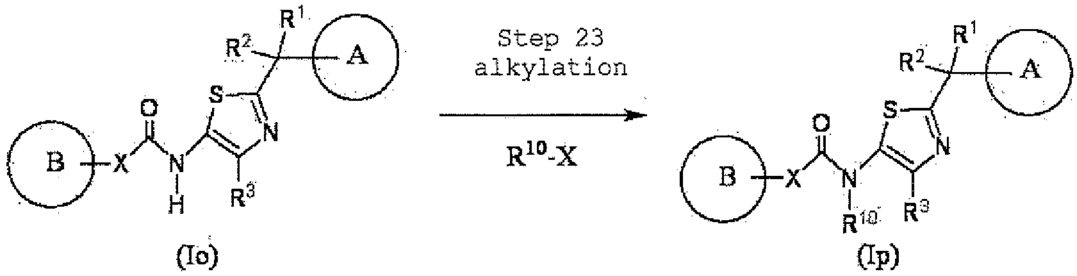


(Io) (Ip)

wherein $R^{10}$ is a $C_{1-6}$ alkyl group optionally having substituent(s), and other symbols are as defined above.

[step 23]

Compound (Ip) can be produced by alkylation of compound (Io). This reaction can be performed, for example, by a treatment with a base according to the method described in J. Heterocyclic. Chem., 30, 939-944 (1993) and Org. Lett, 5, 1265-1268 (2003), in the presence of the corresponding alkyl halide ($R^{10}$-X).
The amount of alkyl halide ($R^{10}$-X) to be used is generally 1 molar equivalent to a large excess, preferably 1 - 10 molar equivalents, relative to compound (Io).
As the "base", those similar to the base shown in production method 1, step 2, can be mentioned. Of these, alkali metal hydrides such as sodium hydride, potassium hydride and the like are preferable. The amount of the base to be used is generally about 0.1 - 10 molar equivalents, preferably 0.5 - 3 molar equivalents, relative to compound (Io).
The reaction time is generally about 0.1 - about 72 hr, preferably about 0.3 - about 24 hr. The reaction temperature is generally about -80°C - about 150°C, preferably about -30°C - about 80°C.

Examples

The present invention is explained in more detail in the following by referring to Examples, Formulation Examples and Experimental Examples, which are not to be construed as limitative.
In the following Examples, the "room temperature" means generally about 10°C - about 35°C. Unless otherwise specified, "%" means weight percent. Other abbreviations used herein mean the following. s is singlet, d is doublet, t is triplet, q is quartet, m is multiplet, br is broad, dd is double doublet, dt is double triplet, td is triple doublet, and J is coupling constant. In the structural formulas of the compounds described in the Examples, "Me" means a methyl group, and "Et" means an ethyl group. In the structural formulas of the compounds, the abbreviation of "Me" may be omitted.
In the Examples and Experimental Examples, the abbreviations mean the following.
LC-MS: liquid chromatography-mass spectrometry spectrum
ESI: electrospray ionization method
TLC: thin layer chromatography
DMF: N,N-dimethylformamide
DMA: N,N-dimethylacetamide
THF: tetrahydrofuran
DMSO: dimethyl sulfoxide
DIEA: N,N-diisopropylethylamine
HATU: N-[(dimethylamino)(3H-[1,2,3]triazolo[4,5-b]naphthal-3-yloxy)methylidene]-N-methylmethanaminium hexafluorophosphate
DPPA: diphenylphosphoryl azide
DBU: 1,8-diazabicyclo[5.4.0]undec-7-ene
HOBt: 1-hydroxybenzotriazole
EDCI·HCl: 1-ethyl-3-(3-dimethylaminopropyl)carbodiimide hydrochloride
LAH: lithium aluminum hydride
M: molarity
N: normality
The nuclear magnetic resonance spectrum ($^1$H-NMR) analysis in the following Examples was measured under the following conditions.
Measurement device: Bruker AVANCE III plus 400 MHz, Bruker AVANCE 300, Varian VNMRS-400
internal standard: trimethylsilane
The MS value in the following Examples was measured under the following conditions.
Measurement device: Waters LC-MS system

HPLC: Agilent HP1100
MS: Waters micromass ZQ
ionization method: ESI
or
HPLC: Agilent 1200
MS: Agilent 6300
ionization method: ESI
or
measurement device: FINNIGAN Thermo LCQ Advantage MAX, Agilent LC 1200 series
ionization method: ESI

Purification by preparative HPLC in the following Examples was performed under the following conditions.

[conditions]

instrument: Waters purification system
column: YMC CombiPrep ODS-A, 5 μm, 50x20 mm
solvent: SOLUTION A; 0.1% trifluoroacetic acid-containing water, SOLUTION B; 0.1% trifluoroacetic acid-containing acetonitrile
gradient cycle: 0.00 min (SOLUTION A/SOLUTION B=90/10), 4.20 min (SOLUTION A/SOLUTION B=0/100), 6.30 min (SOLUTION A/SOLUTION B=0/100), 6.30 min (SOLUTION A/SOLUTION B=90/10), 7.50 min (SOLUTION A/SOLUTION B=90/10)
flow rate: 25 mL/min, detection method: UV 220 nm

Example 1

1-methyl-N-{2-[3-(trifluoromethyl)benzyl]-1,3-thiazol-5-yl}-1H-pyrazole-5-carboxamide

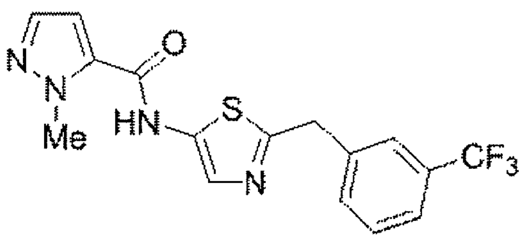

Example 1-A)

1-methyl-1H-pyrazole-5-carboxylic acid

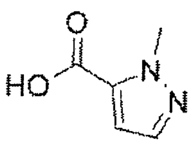

1-Methyl-1H-pyrazole (5.00 g, 60.9 mmol) was dissolved in diethyl ether (anhydrous; 150 mL), n-butyllithium (2.5 M hexane solution, 26.8 mL, 67.0 mmol) was added while cooling to -78°C, and the mixture was stirred for 30 min at -78°C. The reaction mixture was added to finely pulverized dry ice (500 g), the mixture was heated to room temperature, and further stirred at room temperature for 1 hr. To the reaction mixture was added water (200 mL), and the aqueous layer was separated and washed with diethyl ether (3 x 100 mL). The aqueous layer was acidified by adding concentrated hydrochloric acid to pH 3, and the precipitate was collected by filtration, and dried to give the title compound (2.10 g) as a white solid (yield 27%). $^1H$ NMR (400 MHz, DMSO-$d_6$) δ 4.05 (3H, s), 6.79 (1H, d, J = 2.0 Hz), 7.47 (1H, d, J = 2.0 Hz), 13.29 (1H, s).

Example 1-B)

potassium (1E)-2-chloro-3-ethoxy-3-oxoprop-1-en-1-olate

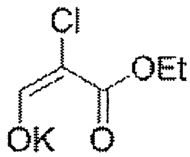

Ethyl chloroacetate (43.7 mL, 408 mmol) and ethyl formate (33.0 mL, 408 mmol) were dissolved in diisopropyl ether (4.08 L), potassium tert-butoxide (45.8 g, 408 mmol) was added while cooling to 0°C, and the mixture was stirred at room temperature for 24 hr. The reaction mixture was concentrated under reduced pressure to give the title compound (35.1 g) as a yellow solid (yield 57%).
$^1$H NMR (400 MHz, DMSO-$d_6$) δ1.12 (3H, t, J = 12 Hz), 3.92 (2H, q, J = 6.9 Hz), 8.95 (1H, s).

Example 1-C)

2-[3-(trifluoromethyl)phenyl]ethanethioamide

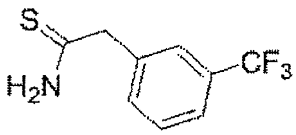

A solution (350 mL) of [3-(trifluoromethyl)phenyl]acetonitrile (25.3 mL, 162 mmol) in ethanol and sodium hydrosulfide (3.89 g, 48.6 mmol) were added in a sealed tube at room temperature. The mixture was cooled to -15°C, and hydrogen sulfide gas was bubbled into the reaction mixture for 10 min. The reaction mixture was tightly sealed, stirred at 80°C for 24 hr. The reaction mixture was concentrated under reduced pressure to give the title compound (23.0 g) as a yellow solid (yield 64%).
$^1$H NMR (400 MHz, $CDCl_3$) δ 4.13 (2H, s), 6.82 (1H, bs), 7.49-7.63 (4H, m), 7.92 (1H, bs)

Example 1-D)

ethyl 2-[3-(trifluoromethyl)benzyl]-1,3-thiazole-5-carboxylate

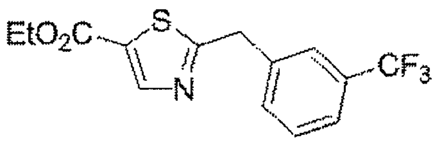

To a solution (100 mL) of 2-[3-(trifluoromethyl)phenyl]ethanethioamide (10.0 g, 45.6 mmol) obtained in Example 1-C) in acetic acid was added potassium (1E)-2-chloro-3-ethoxy-3-oxoprop-1-en-1-olate (25.8 g, 137 mmol) obtained in Example 1-B) at room temperature. The mixture was heated under reflux for 7 hr, and concentrated under reduced pressure. The residue was dissolved in dichloromethane (100 mL), and the solution was washed with saturated aqueous sodium hydrogen carbonate solution (3×30 mL) and saturated brine (30 mL), dried over anhydrous sodium sulfate, and concentrated under reduced pressure. The residue was purified by silica gel column chromatography [eluent: hexane-ethyl acetate (10:1)] to give the title compound (11.0 g) as a yellow oil (yield 76%).
$^1$H NMR (400 MHz, $CDCl_3$) δ1.35 (3H, t, J = 7.2 Hz), 4.34 (2H, q, J = 7.1 Hz), 4.40 (2H, s), 7.46-7.53 (2H, m), 7.56-7.58

(2H, m), 8.31 (1H, s).

Example 1-E)

2-[3-(trifluoromethyl)benzyl]-1,3-thiazole-5-carboxylic acid

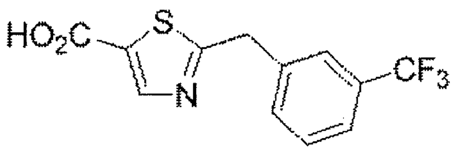

To a solution (70 mL) of ethyl 2-[3-(trifluoromethyl)benzyl]-1,3-thiazole-5-carboxylate (11.0 g, 34.9 mmol) obtained in Example 1-D) in ethanol was added 1M aqueous sodium hydroxide solution (34.9 mL, 34.9 mmol) at 0°C. The mixture was stirred for 30 min at room temperature, ion exchange resin CG50 (10.0 g) was added, and the mixture was stirred for 30 min at room temperature. The reaction mixture was filtered and the filtrate was concentrated under reduced pressure to give the title compound (10.8 g) as a yellow solid (quantitatively).
$^1$H NMR (400 MHz, DMSO-$d_6$) $\delta$ 4.38 (2H, s), 7.52-7.60 (1H, m), 7.63-7.66 (2H, m), 7.71 (1H, s), 7.78 (1H, s).
* The peak of "COOH group" was not observed.

Example 1-F)

tert-butyl {2-[3-(trifluoromethyl)benzyl]-1,3-thiazol-5-yl}carbamate

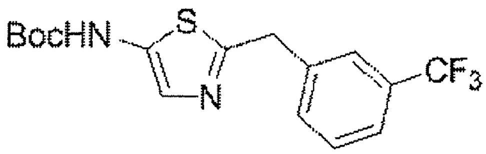

To a solution (70 mL) of 2-[3-(trifluoromethyl)benzyl]-1,3-thiazole-5-carboxylic acid (5.00 g, 26.8 mmol) obtained in Example 1-E) in tert-butanol was added triethylamine (7.28 mL, 52.2 mmol) at room temperature, and the mixture was heated to 80°C, and DPPA (18.76 mL, 87.0 mmol) was added. The mixture was heated under reflux for 2 hr, and concentrated under reduced pressure. The residue was dissolved in ethyl acetate (50 mL), and the solution was washed with water (30 mL) and saturated brine (20 mL), dried over anhydrous sodium sulfate, and concentrated under reduced pressure. The residue was purified by silica gel column chromatography [eluent: hexane-ethyl acetate (3:1)] to give the title compound (6.92 g) as a yellow oil (yield 55%).
MS (ESI+) : $[M+H]^+$ 359.
$^1$H NMR (400 MHz, $CDCl_3$) $\delta$1.47 (9H, s), 4.28 (2H, s), 7.23 (1H, s), 7.41-7.45 (1H, m), 7.48-7.52 (2H, m), 7.55 (1H, s).
* The peak of "NH group" was not observed.

Example 1-G)

2-[3-(trifluoromethyl)benzyl]-1,3-thiazole-5-amine hydrochloride

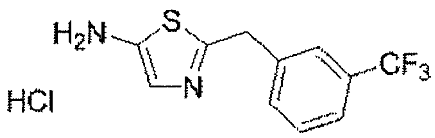

Hydrogen chloride gas was bubbled into a solution (10 mL) of tert-butyl {2-[3-(trifluoromethyl)benzyl]-1,3-thiazol-5-yl}carbamate (1.10 g, 3.07 mmol) obtained in Example 1-F) in ethyl acetate at room temperature for 1 hr. The reaction mixture was concentrated under reduced pressure to give the title compound (880 mg) as a white solid (quantitatively). $^1$H NMR (400 MHz, DMSO-$d_6$) δ 4.42 (2H, s), 7.06 (1H, s), 7.58-7.62 (1H, m), 7.66-7.70 (2H, s), 7.77 (1H, s).
* The peak of "$NH_3$" was not observed.

Example 1-H)

1-methyl-N-{2-[3-(trifluoromethyl)benzyl]-1,3-thiazol-5-yl}-1H-pyrazole-5-carboxamide

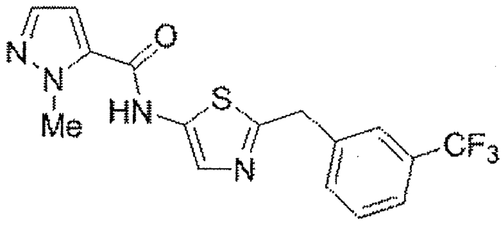

2-[3-(Trifluoromethyl)benzyl]-1,3-thiazole-5-amine hydrochloride (200 mg, 0.74 mmol) obtained in Example 1-G), 1-methyl-1H-pyrazole-5-carboxylic acid (98 mg, 0.77 mmol) obtained in Example 1-A) and HATU (442 mg, 1.16 mmol) were dissolved in DMF (5 mL), triethylamine (0.16 mL, 1.16 mmol) was added at room temperature, and the mixture was stirred at room temperature for 20 hr. The reaction mixture was concentrated under reduced pressure, and the residue was dissolved in ethyl acetate (10 mL). The solution was washed with water (3×5 mL) and saturated brine (5 mL), dried over anhydrous sodium sulfate, and concentrated under reduced pressure. The obtained residue was purified by silica gel column chromatography [eluent: ethyl acetate] to give the title compound (80 mg) as a yellow solid (yield 28%).
MS (ESI+) : $[M+H]^+$ 367.
$^1$H NMR (400 MHz, $CDCl_3$) δ 4.21 (3H, s), 4.32 (2H, s), 6.69 (1H, d, J = 2.0 Hz), 7.42-7.46 (1H, m), 7.48-7.49 (2H, m), 7.50-7.51 (2H, m), 7.55-7.56 (1H, m), 8.67 (1H, s).

Example 2

1-ethyl-5-oxo-N-{2-[3-(trifluoromethyl)benzyl]-1,3-thiazol-5-yl}-pyrrolidine-3-carboxamide

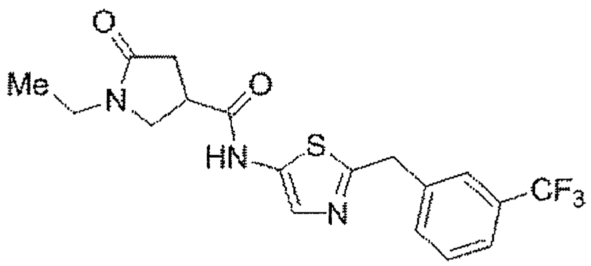

Example 2-A)

methyl 1-ethyl-5-oxopyrrolidine-3-carboxylate

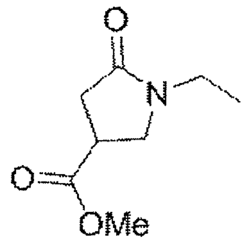

To a solution (80 mL) of dimethyl 2-methylidenebutanedioate (5.00 g, 38.4 mmol) in toluene in a sealed tube was added ethylamine (2M methanol solution, 19.2 mL, 38.4 mmol) at room temperature, and the mixture was stirred at 100°C for 22 hr. After cooling to room temperature, the mixture was concentrated under reduced pressure. The residue was diluted with diethyl ether (100 mL), washed with water (3×50 mL) and saturated brine (50 mL), dried over anhydrous sodium sulfate, and concentrated under reduced pressure to give the title compound (3.50 g) as a yellow oil (yield 53%). $^1H$ NMR (400 MHz, $CDCl_3$) $\delta$ 1.12 (3H, t, J = 7.2 Hz), 2.62-2.74 (2H, m), 3.20-3.40 (3H, m), 3.56-3.65 (2H, m), 3.75 (3H, s).

Example 2-B)

1-ethyl-5-oxopyrrolidine-3-carboxylic acid

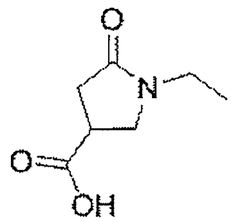

Methyl 1-ethyl-5-oxopyrrolidine-3-carboxylate (1.00 g, 5.84 mmol) obtained in Example 2-A) was dissolved in a mixed solvent of methanol (5 mL), THF (5 mL) and water (5 mL), sodium hydroxide (234 mg, 5.84 mmol) was added at 0°C, and the mixture was heated to room temperature and stirred for 30 min. To the reaction mixture was added ion exchange resin CG50 (5.00 g), and the mixture was further stirred at room temperature for 30 min. The mixture was filtered, and the filtrate was concentrated under reduced pressure to give the title compound (850 mg) as a white solid (yield 93%). $^1H$ NMR (400 MHz, DMSO-$d_6$) $\delta$ 0.96 (3H, t, J = 7.2 Hz), 2.21-2.28 (1H, m), 2.42-2.48 (1H, m), 2.68-2.76 (1H, m), 3.12 (2H, q, J = 7.2 Hz), 3.35-3.46 (2H, m).
* The peak of "COOH group" was not observed.

Example 2-C)

1-ethyl-5-oxo-N-{2-[3-(trifluoromethyl)benzyl]-1,3-thiazol-5-yl}-pyrrolidine-3-carboxamide

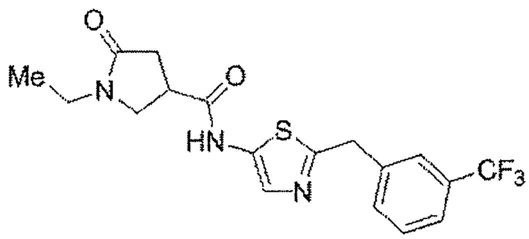

In the same manner as in Example 1-H), 2-[3-(trifluoromethyl)benzyl]-1,3-thiazole-5-amine hydrochloride obtained in Example 1-G) and 1-ethyl-5-oxopyrrolidine-3-carboxylic acid obtained in Example 2-B) were used to give the title compound (80 mg) (yield 26%).

MS (ESI+) : $[M+H]^+$ 398.
$^1$H-NMR (400 MHz, $CDCl_3$) δ 1.13 (3H, t, J = 7.4 Hz), 2.59-2.73 (2H, m), 3.20-3.42 (3H, m), 3.48-3.53 (1H, m), 3.77-3.81 (1H, m), 4.29 (2H, s), 7.33 (1H, s), 7.39-7.43 (1H, m), 7.46-7.47 (1H, m), 7.49-7.52 (2H, m), 10.57 (1H, s).

Example 3

1-methyl-N-{4-methyl-2-[3-(trifluoromethyl)benzyl]-1,3-thiazol-5-yl}-1H-pyrazole-5-carboxamide

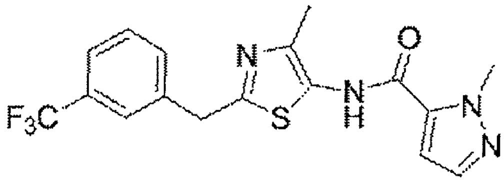

Example 3-A)

ethyl 4-methyl-2-[3-(trifluoromethyl)benzyl]-1,3-thiazole-5-carboxylate

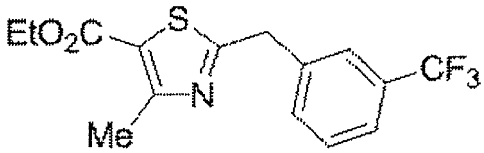

To a solution (100 mL) of 2-[3-(trifluoromethyl)phenyl]ethanethioamide (10.0 g, 45.6 mmol) obtained in Example 1-C) and ethyl 2-chloro-3-oxobutanoate (6.31 mL, 45.6 mmol) in ethanol was added pyridine (4.43 mL, 54.7 mmol) at room temperature. The mixture was stirred for 7 hr at room temperature, and concentrated under reduced pressure. The residue was dissolved in ethyl acetate (100 mL), and the solution was washed with water (3×30 mL) and saturated brine (30 mL), dried over anhydrous sodium sulfate, and concentrated under reduced pressure. The residue was purified by silica gel column chromatography [eluent: hexane-ethyl acetate (10:1)] to give the title compound (14.0 g) as a yellow oil (yield 93%).
$^1$H NMR (400 MHz, $CDCl_3$) δ1.33 (3H, t, J = 7.2 Hz), 2.72 (3H, s), 4.27-4.31 (2H, m), 4.32 (2H, s), 7.46-7.52 (2H, m), 7.55-7.58 (2H, m).

Example 3-B)

4-methyl-2-[3-(trifluoromethyl)benzyl]-1,3-thiazole-5-carboxylic acid

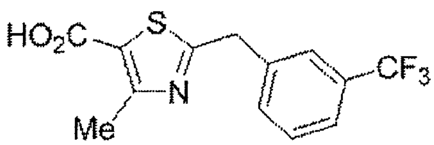

To a solution (100 mL) of ethyl 4-methyl-2-[3-(trifluoromethyl)benzyl]-1,3-thiazole-5-carboxylate (15.0 g, 45.5 mmol) obtained in Example 3-A) in ethanol was added 1M aqueous sodium hydroxide solution (45.5 mL, 45.5 mmol) at 0°C. The mixture was stirred for 30 min at room temperature, ion exchange resin CG50 (15.0 g) was added and the mixture was stirred for 30 min at room temperature. The reaction mixture was filtered and the filtrate was concentrated under reduced pressure to give the title compound (15.1 g) as a white solid (quantitatively).
$^1$H NMR (400 MHz, DMSO-$d_6$) δ 2.51 (3H, s), 4.28 (2H, s), 7.56-7.60 (1H, m), 7.62-7.64 (2H, m), 7.70 (1H, s).
* The peak of "COOH group" was not observed.

Example 3-C)

tert-butyl {4-methyl-2- [3-(trifluoromethyl) benzyl]-1, 3-thiazol-5-yl}carbamate

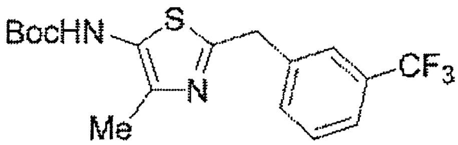

To a solution (100 mL) of 4-methyl-2-[3-(trifluoromethyl)benzyl]-1,3-thiazole-5-carboxylic acid (15.0 g, 49.8 mmol) obtained in Example 3-B) in tert-butanol was added triethylamine (10.35 mL, 74.7 mmol) at room temperature, and the mixture was heated to 80°C and DPPA (26.8 mL, 74.7 mmol) was added. The mixture was heated under reflux for 2 hr, and concentrated under reduced pressure. The residue was dissolved in ethyl acetate (150 mL), and the solution was washed with water (3x50 mL) and saturated brine (50 mL), dried over anhydrous sodium sulfate, and concentrated under reduced pressure. The residue was purified by silica gel column chromatography [eluent: hexane-ethyl acetate (3:1)] to give the title compound (11.7 g) as a yellow oil (yield 63%). MS (ESI+): $[M+H]^+$ 373.
$^1$H NMR (400 MHz, $CDCl_3$) $\delta$ 1.49 (9H, s), 2.29 (3H, s), 4.24 (2H, s), 7.41-7.45 (1H, m), 7.48-7.52 (2H, m), 7.55 (1H, s).
* The peak of "NH group" was not observed.

Example 3-D)

4-methyl-2-[3-(trifluoromethyl)benzyl]-1,3-thiazole-5-amine hydrochloride

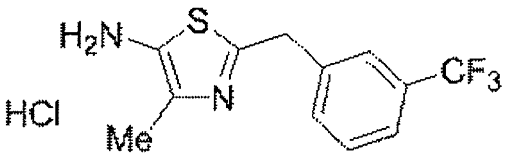

Hydrogen chloride gas was bubbled into a solution (5 mL) of tert-butyl {4-methyl-2-[3-(trifluoromethyl)benzyl]-1,3-thiazol-5-yl}carbamate (900 mg, 2.42 mmol) obtained in Example 3-C) in ethyl acetate at room temperature for 1 hr. The reaction mixture was concentrated under reduced pressure to give the title compound (750 mg) as a white solid (quantitatively).
$^1$H NMR (400 MHz, DMSO-$d_6$) $\delta$ 2.26 (3H, s), 4.39 (2H, s), 7.59-7.63 (1H, m), 7.67-7.72 (2H, m), 7.81 (1H, s).
* The peak of "$NH_3$" was not observed.

Example 3-E)

1-methyl-N-{4-methyl-2-[3-(trifluoromethyl)benzyl]-1,3-thiazol-5-yl}-1H-pyrazole-5-carboxamide

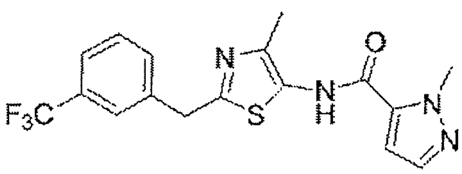

4-Methyl-2-[3-(trifluoromethyl)benzyl]-1,3-thiazole-5-amine hydrochloride (200 mg, 0.74 mmol) obtained in Example 3-D), 1-methyl-1H-pyrazole-5-carboxylic acid (93 mg, 0.74 mmol) obtained in Example 1-A) and HATU (419 mg, 1.10 mmol) were dissolved in DMF (5 mL), triethylamine (0.15 mL, 1.10 mmol) was added at room temperature, and

the mixture was stirred at room temperature for 20 hr. The reaction mixture was concentrated under reduced pressure, and the residue was dissolved in ethyl acetate (10 mL). The solution was washed with water (3×5 mL) and saturated brine (5 mL), dried over anhydrous sodium sulfate, and concentrated under reduced pressure. The obtained residue was purified by silica gel column chromatography [eluent: ethyl acetate] to give the title compound (70 mg) as a yellow solid (yield 25%). MS (ESI+): $[M+H]^+$ 381.
$^1$H NMR (400 MHz, $CDCl_3$) δ 2.36 (3H, s), 4.17 (3H, s), 4.28 (2H, s), 6.69 (1H, d, J = 2.0 Hz), 7.42-7.45 (1H, m), 7.48 (1H, d, J = 2.0 Hz), 7.51-7.56 (3H, m), 8.08 (1H, s).

Example 4

1-ethyl-N-(4-methyl-2- [3-(trifluoromethyl)benzyl]-1,3-thiazol-5-yl}-5-oxopyrrolidine-3-carboxamide

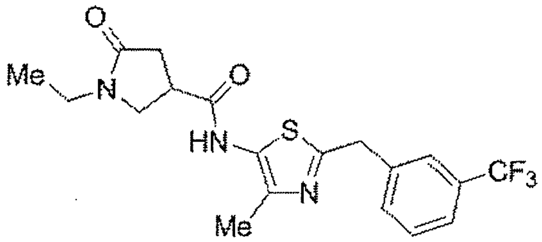

In the same manner as in Example 3-E), 4-methyl-2-[3-(trifluoromethyl)benzyl]-1,3-thiazole-5-amine hydrochloride obtained in Example 3-D) and 1-ethyl-5-oxopyrrolidine-3-carboxylic acid obtained in Example 2-B) were used to give the title compound (178 mg) (yield 58%).
MS (ESI+) : $[M+H]^+$ 412.
$^1$H-NMR ($CDCl_3$) δ 1. 12 (3H, t, J = 8.2 Hz), 2.35 (3H, s), 2.63-2.714 (2H, m), 3.27-3.40 (3H, m), 3.55 (1H, J = 9.4 Hz), 3.72-3.76 (1H, m), 4.26 (2H, s), 7.41-7.45 (1H, m), 7.48-7.54 (3H, m), 8.56 (1H, s).

Example 5

ethyl 5-{[(1-methyl-1H-pyrazol-5-yl)carbonyl]amino}-2-[3-(trifluoromethyl)benzyl]-1,3-thiazole-4-carboxylate

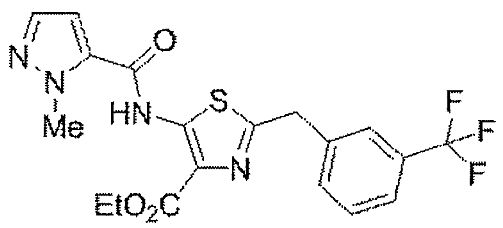

Example 5-A)

ethyl 3-nitrilo-N-{[3-(trifluoromethyl)phenyl]acetyl}alaninate

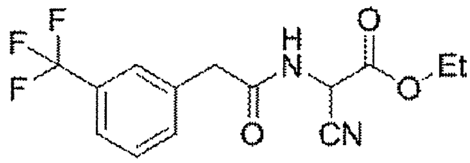

To a mixture of ethyl cyano(hydroxyimino)ethanoate (50 g, 350 mmol), water (500 mL) and saturated aqueous sodium hydrogen carbonate solution (400 mL) was slowly added sodium hydrosulfite (183 g, 1050 mmol). The reaction mixture was stirred at room temperature for 2 hr, saturated with sodium chloride, and extracted with ethyl acetate (500 mL×4). The organic layer was dried over anhydrous sodium sulfate, and concentrated under reduced pressure. From the obtained residue (21.5 g), 12.9 g was dissolved in THF (200 mL), and triethylamine (23 mL) was added. The mixture was cooled to 0°C, and [3-(trifluoromethyl)phenyl]acetyl chloride (15 g, 67.3 mmol) was slowly added dropwise. The reaction mixture was stirred at room temperature overnight, diluted with ethyl acetate and washed with saturated brine. The organic layer was dried over anhydrous sodium sulfate, and concentrated under reduced pressure. The obtained residue was suspended in diisopropyl ether (100 mL), and the resulting solid was collected by filtration to give the title compound (8.5 g) as a pale-brown solid (yield 40%).
$^1H$ NMR (300 MHz, $CDCl_3$) δ 1.34 (3H, t, J = 7.0 Hz), 3.73 (2H, s), 4.34 (2H, q, J = 7.1 Hz), 5.49 (1H, d, J = 8.0 Hz), 6.32 (1H, d, J = 7.2 Hz), 7.43-7.67 (4H, m).

Example 5-B)

ethyl 5-amino-2-[3-(trifluoromethyl)benzyl]-1,3-thiazole-4-carboxylate

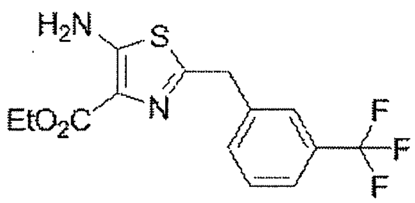

A mixture of ethyl 3-nitrilo-N-{[3-(trifluoromethyl)phenyl]acetyl}alaninate (7.0 g, 22.3 mmol) obtained in Example 5-A), Lawesson reagent (90%, 6.0 g, 13.4 mmol) and toluene (100 mL) was stirred at 80°C for 6 hr. The reaction mixture was extracted with ethyl acetate, and the extract was washed with 1M aqueous sodium hydroxide solution and saturated brine. The organic layer was dried over anhydrous sodium sulfate, and concentrated under reduced pressure. The obtained residue was purified by basic silica gel column chromatography [eluent: hexane-ethyl acetate (90:10-50:50)] to give the title compound (3.4 g) as a yellow solid (yield 46%).
MS (ESI+): $[M+H]^+$ 331.
$^1H$ NMR (300 MHz, $CDCl_3$) 5 1.40 (3H, t, J = 7.0 Hz), 4.22 (2H, s), 4.40 (2H, q, J = 7.0 Hz), 5.97 (2H, brs), 7.36-7.56 (4H, m).

Example 5-C)

1-methyl-1H-pyrazole-5-carbonyl chloride

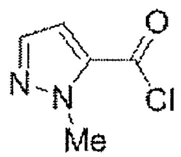

A mixture of 1-methyl-1H-pyrazole-5-carboxylic acid (1.26 g, 10 mmol) obtained in Example 1-A), thionyl chloride (3.65 mL, 50 mmol) and ethyl acetate (10 mL) was stirred at 80°C for 5 hr. The reaction mixture was concentrated under reduced pressure to give the title compound (1.45 g) as an oil (yield 100%) .
$^1H$ NMR (300 MHz, $CDCl_3$) δ 4.14 (3H, s), 7.13 (1H, d, J = 1.9 Hz), 7.52 (1H, d, J = 2.3 Hz).

Example 5-D)

ethyl 5-{[(1-methyl-1H-pyrazol-5-yl)carbonyl]amino}-2-[3-(trifluoromethyl)benzyl]-1,3-thiazole-4-carboxylate

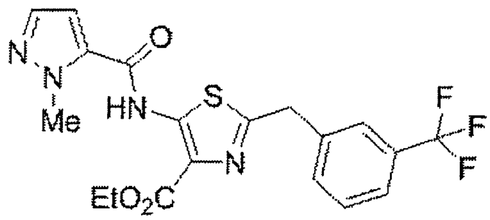

A mixture of ethyl 5-amino-2-[3-(trifluoromethyl)benzyl]-1,3-thiazole-4-carboxylate (1.8 g, 5.45 mmol) obtained in Example 5-B), 1-methyl-1H-pyrazole-5-carbonyl chloride (0.95 g, 6.55 mmol) obtained in Example 5-C) and DMA (5 mL) was stirred at 80°C for 3 hr. The reaction mixture was extracted with ethyl acetate, and the extract was washed with saturated aqueous sodium hydrogen carbonate solution, water and saturated brine. The organic layer was dried over anhydrous sodium sulfate and concentrated under reduced pressure. The obtained residue was recrystallized from ethanol-water to give the title compound (1.8 g) as a pale-brown solid (yield 75%). MS (ESI+): $[M+H]^+$ 439.
$^1H$ NMR (300 MHz, $CDCl_3$) $\delta$ 1.49 (3H, t, J = 7.2 Hz), 4.23 (3H, s), 4.39 (2H, s), 4.53 (2H, q, J = 7.2 Hz), 6.88 (1H, d, J = 2.3 Hz), 7.43-7.61 (5H, m), 11.60 (1H, s).

Example 6

N-{4-(hydroxymethyl)-2-[3-(trifluoromethyl)benzyl]-1,3-thiazol-5-yl}-1-methyl-1H-pyrazole-5-carboxamide

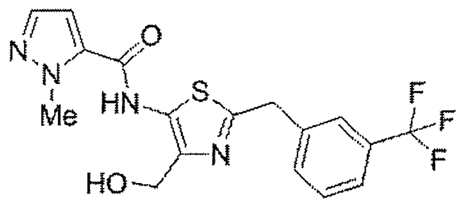

To a solution of ethyl 5-{[(1-methyl-1H-pyrazol-5-yl)carbonyl]amino}-2-[3-(trifluoromethyl)benzyl]-1,3-thiazole-4-carboxylate (1.8 g, 4.1 mmol) obtained in Example 5-D) in THF (33 mL) was added dropwise diisobutylaluminum hydride (DIBAL; 1.5M toluene solution, 11 mL, 16.4 mmol) at 0°C. The reaction mixture was warmed to room temperature and stirred overnight. To the reaction mixture was added aqueous Rochelle salt solution, and the mixture was extracted with ethyl acetate. The organic layer was washed with saturated brine, dried over anhydrous sodium sulfate, and concentrated under reduced pressure. The obtained residue was suspended in hexane-ethyl acetate mixture, and the resulting solid was collected by filtration to give the title compound (1.46 g) as a pale-yellow solid (yield 90%).
MS (ESI+): $[M+H]^+$ 397.
$^1H$ NMR (300 MHz, $CDCl_3$) $\delta$ 2.43 (1H, t, J = 4.7 Hz), 4.22 (3H, s), 4.28 (2H, s), 5.15 (2H, d, J = 4.7 Hz), 6.63 (1H, d, J = 1.9 Hz), 7.40-7.58 (5H, m), 10.04 (1H, s).

Example 7

N-{4-(1-hydroxyethyl)-2-[3-(trifluoromethyl}benzyl]-1,3-thiazol-5-yl}-1-methyl-1H-pyrazole-5-carboxamide

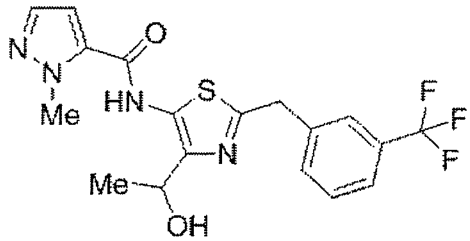

Example 7-A)

N-{4-formyl-2-[3-(trifluoromethyl)benzyl]-1,3-thiazol-5-yl}-1-methyl-1H-pyrazole-5-carboxamide

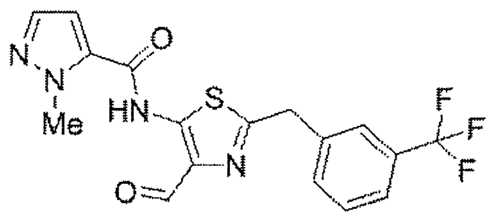

To a mixed solution of N-{4-(hydroxymethyl)-2-[3-(trifluoromethyl)benzyl]-1,3-thiazol-5-yl}-1-methyl-1H-pyrazole-5-carboxamide (1.26 g, 3.18 mmol) obtained in Example 6 in acetonitrile (30 mL)-THF (30 mL) was slowly added Dess-Martin reagent (1.62 g, 3.82 mmol) at 0°C. The reaction mixture was warmed to room temperature, and stirred for 3 hr. The reaction mixture was poured into a mixed aqueous solution of sodium hydrogen carbonate and sodium thiosulfate, and the mixture was extracted with ethyl acetate. The organic layer was washed with water and saturated brine, dried over anhydrous sodium sulfate and concentrated under reduced pressure. The obtained residue was suspended in hexane-ethyl acetate mixture and the resulting solid was collected by filtration to give the title compound (935 mg) as a colorless solid (yield 75%).
MS (ESI+): $[M+H]^+$ 395.
$^1H$ NMR (300 MHz, $CDCl_3$) $\delta$ 4.23 (3H, s), 4.34 (2H, s), 6.92 (1H, d, J = 2.2 Hz), 7.43-7.62 (5H, m), 10.07 (1H, s), 11.78 (1H, brs).

Example 7-B)

N-{4-(1-hydroxyethyl)-2-[3-(trifluoromethyl)benzyl]-1,3-thiazol-5-yl}-1-methyl-1H-pyrazole-5-carboxamide

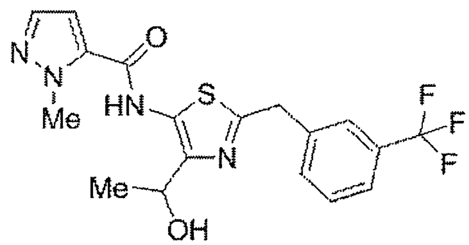

To a solution of N-{4-formyl-2-[3-(trifluoromethyl)benzyl]-1,3-thiazol-5-yl}-1-methyl-1H-pyrazole-5-carboxamide (600 mg, 1.52 mmol) obtained in Example 7-A) in THF (10 mL) was added dropwise methylmagnesium bromide (1.0M ether solution, 4.57 mL, 4.57 mmol) at 0°C, and the mixture was stirred for 1 hr. To the reaction mixture was added saturated aqueous ammonium chloride solution, and the mixture was extracted with ethyl acetate. The organic layer was washed with saturated brine, and dried over anhydrous sodium sulfate, and concentrated under reduced pressure. The obtained residue was purified by silica gel column chromatography [eluent: hexane-ethyl acetate (70:30-30:70)] to give the title compound (575 mg) as an oil (yield 92%). MS (ESI+): $[M+H]^+$ 411.
$^1H$ NMR (300 MHz, $CDCl_3$) $\delta$ 1.64 (3H, d, J = 6.6 Hz), 3.19 (1H, brs), 4.20 (3H, s), 4.26 (2H, s), 5.30-5.42 (1H, m), 6.60 (1H, d, J=2.2 Hz), 7.37-7.60 (5H, m), 10.45 (1H, brs).

Example 7-C)

N-{4-[(1R)-1-hydroxyethyl]-2-[3-(trifluoromethyl)benzyl]-1,3-thiazol-5-yl}-1-methyl-1H-pyrazole-5-carboxamide

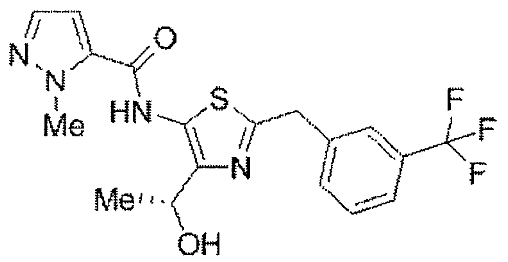

Example 7-D)

N-{4-[(1S)-1-hydroxyethyl]-2-[3-(trifluoromethyl)benzyl]-1,3-thiazol-5-yl}-1-methyl-1H-pyrazole-5-carboxamide

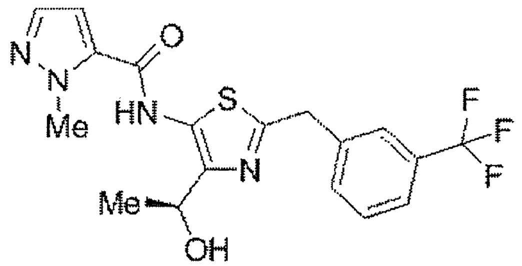

N-{4-(1-hydraxyethyl)-2-[3-(trifluoromethyl)benzyl]-1,3-thiazol-5-yl}-1-methyl-1H-pyrazole-5-carboxamide (racemate; 30 mg) obtained in Example 7-B) was fractionated by high performance liquid chromatography (instrument: K-Prep (manufactured by YMC), column: CHIRALPAK IC (50 mmID×500 mmL manufactured by DAICEL CHEMICAL INDUSTRIES, LTD.), mobile phase: A) hexane 100%, B) 2-propanol=100%, mixing ratio: A/B=90/10, flow rate: 80 mL/min, column temperature: 30°C, sample injection volume: 30 mg (dissolved in mobile phase 5 mL)). A fraction solution containing an optically active form having a shorter retention time, which was obtained by the above-mentioned high performance liquid chromatography conditions, was concentrated to give N-{4-(1-hydroxyethyl)-2-[3-(trifluoromethyl) benzyl]-1,3-thiazol-5-yl}-1-methyl-1H-pyrazole-5-carboxamide (retention time short) (14 mg, 93%, >99.9% ee).
A fraction solution containing an optically active form having a longer retention time was concentrated to give N-{4-(1-hydroxyethyl)-2-[3-(trifluoromethyl)benzyl]-1,3-thiazol-5-yl}-1-methyl-1H-pyrazole-5-carboxamide (retention time long) (12 mg, 80%, 99.1% ee).
The enantiomeric excess (ee) was measured by high performance liquid chromatography (column: CHIRALPAK IC (4.6 mmID×250 mmL manufactured by DAICEL CHEMICAL INDUSTRIES, LTD.), mobile phase: hexane/2-propanol=90/10, flow rate: 1.0 mL/min, column temperature: 30°C, sample concentration: 0.5 mg/mL (mobile phase), injection volume: 10 μL). N-{4-(1-hydroxyethyl)-2-[3-(trifluoromethyl)benzyl]-1,3-thiazol-5-yl}-1-methyl-1H-pyrazole-5-carboxamide (retention time short):
MS (ESI+): $[M+H]^+$ 411.
N-{4-(1-hydroxyethyl)-2-[3-(trifluoromethyl)benzyl]-1,3-thiazol-5-yl}-1-methyl-1H-pyrazole-5-carboxamide (retention time long) :
MS (ESI+): $[M+H]^+$ 411.

Example 8

N-{4-(methoxymethyl)-2-[3-(trifluoromethyl)benzyl]-1,3-thiazol-5-yl}-1-methyl-1H-pyrazole-5-carboxamide

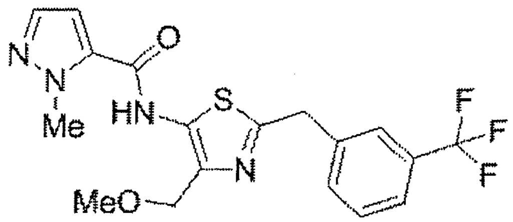

A mixture of N-{4-(hydroxymethyl)-2-[3-(trifluoromethyl)benzyl]-1,3-thiazol-5-yl}-1-methyl-2H-pyrazole-5-carboxamide (20 mg, 0.050 mmol) obtained in Example 6, concentrated sulfuric acid (0.0053 mL) and methanol (0.5 mL) was heated by microwave irradiation (100°C, 10 min). The reaction mixture was diluted with ethyl acetate, and washed with saturated aqueous sodium hydrogen carbonate solution and saturated brine. The organic layer was dried over anhydrous sodium sulfate and concentrated under reduced pressure, and the obtained residue was purified by silica gel column chromatography [eluent: hexane-ethyl acetate (80:20-50:50)] to give the title compound (16 mg) as an oil (yield 78%). MS (ESI+): $[M+H]^+$ 411.
$^1H$ NMR (300 MHz, $CDCl_3$) $\delta$ 3.58 (3H, s), 4.22 (3H, s), 4.28 (2H, s), 4.88 (2H, s), 6.56 (1H, d, J = 2.2 Hz), 7.40-7.58 (5H, m), 9.96 (1H, s).

Example 9-1

N-{4-(1-hydroxy-1-methylethyl)-2-[3-(trifluoromethyl)benzyl]-1,3-thiazol-5-yl}-1-methyl-1H-pyrazole-5-carboxamide

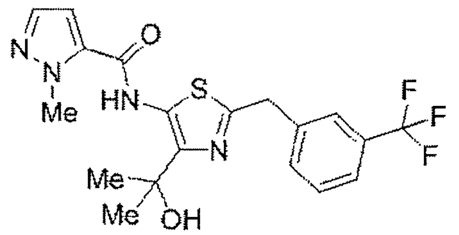

Example 9-2

N-[4-(1-hydroxy-1-methylethyl)-2-{naphthal[3-(trifluoromethyl)phenyl]methyl}-1,3-thiazol-5-yl]-1-methyl-1H-pyrazole-5-carboxamide

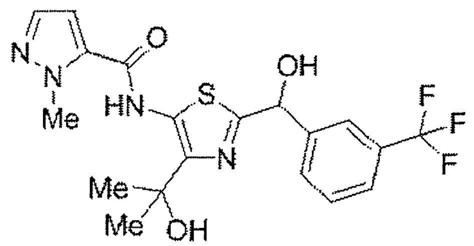

To a solution of ethyl 5-{[(1-methyl-1H-pyrazol-5-yl)carbonyl]amino}-2-[3-(trifluoromethyl)benzyl]-1,3-thiazole-4-carboxylate (130 mg, 0.30 mmol) obtained in Example 5-D) in THF (3 mL) was added dropwise methyllithium (1.0M solution, 1.5 mL) at -78°C, and the mixture was stirred for 1 hr. Water was added to the reaction mixture, and the mixture was extracted with ethyl acetate. The organic layer was washed with saturated brine, dried over anhydrous sodium sulfate, and concentrated under reduced pressure. The obtained residue was purified by silica gel column chromatography [eluent: hexane-ethyl acetate (80:20-0:100)] to give N-{4-(1-hydroxy-1-methylethyl)-2-[3-(trifluoromethyl)benzyl]-1,3-thiazol-5-yl}-1-methyl-1H-pyrazole-5-carboxamide (Example 9-1; 20 mg, yield 16%) as an oil, and N-[4-(1-hydroxy-1-methylethyl)-2-{naphthal[3-(trifluoromethyl)phenyl]methyl}-1,3-thiazol-5-yl]-1-methyl-1H-pyrazole-5-carboxamide

(Example 9-2; 17 mg, yield 13%) as an oil.
N-{4-(1-hydroxy-1-methylethyl)-2-[3-(trifluoromethyl)benzyl]-1,3-thiazol-5-yl}-1-methyl-1H-pyrazole-5-carboxamide:
MS (ESI+): $[M+H]^+$ 425.
$^1$H NMR (300 MHz, $CDCl_3$) δ 1.72 (6H, s), 2.41 (1H, brs), 4.21 (3H, s), 4.27 (2H, s), 6.59 (1H, d, J = 1. 9 Hz), 7. 38-7. 63 (5H, m), 10.74 (1H, s).
N-[4-(1-hydroxy-1-methylethyl)-2-{naphthal[3-(trifluoromethyl)phenyl]methyl}-1,3-thiazol-5-yl]-1-methyl-1H-pyrazole-5-carboxamide:
MS (ESI+): $[M+H]^+$ 441.
$^1$H NMR (300 MHz, $CDCl_3$) δ 1.71 (6H, d, J = 3.8 Hz), 2.45 (1H, s), 3.92 (1H, brs), 4.20 (3H, s), 6.00 (1H, s), 6.59 (1H, d, J = 2.3 Hz), 7.43-7.84 (5H, m), 10.74 (1H, s).

Example 10

1-methyl-N-{4-(1-methylethyl)-2-[3-(trifluoromethyl)benzyl]-1,3-thiazol-5-yl}-1H-pyrazole-5-carboxamide

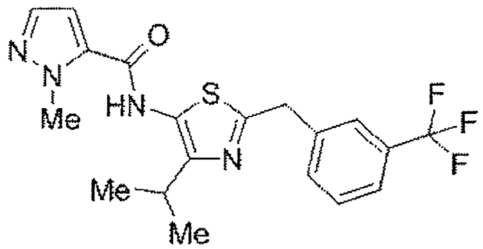

A mixture of N-{4-(1-hydroxy-1-methylethyl)-2-[3-(trifluoromethyl)benzyl]-1,3-thiazol-5-yl}-l-methyl-1H-pyrazole-5-carboxamide (10 mg, 0.024 mmol) obtained in Example 9-1, triethylsilane (0.019 mL, 0.118 mmol) and trifluoroacetic acid (1 mL) was stirred at 50°C overnight. The reaction mixture was diluted with ethyl acetate, and washed with saturated aqueous sodium hydrogen carbonate solution and saturated brine. The organic layer was dried over anhydrous sodium sulfate and concentrated under reduced pressure, and the obtained residue was purified by silica gel column chromatography [eluent: hexane-ethyl acetate (80:20-20:80)] to give the title compound (3 mg) as an oil (yield 30%).
MS (ESI+): $[M+H]^+$ 409.
$^1$H NMR (300 MHz, $CDCl_3$) δ 1.36 (6H, d, J = 6.9 Hz), 2.92-3.04 (1H, m), 4.20 (3H, s), 4.32 (2H, s), 6.65 (1H, d, J = 1.9 Hz), 7.39-7.74 (6H, m).

Example 11

N-{4-ethyl-2-[3-(trifluoromethyl)benzyl]-1,3-thiazol-5-yl}-1-methyl-1H-pyrazole-5-carboxamide

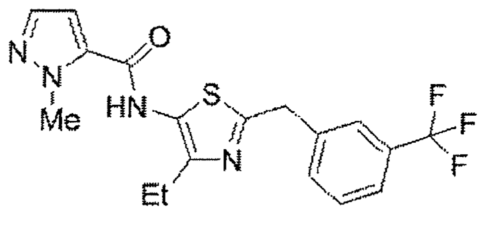

A mixture of N-{4-(1-hydroxyethyl)-2-[3-(trifluoromethyl)benzyl]-1,3-thiazol-5-yl}-1-methyl-1H-pyrazole-5-carboxamide (30 mg, 0.073 mmol) obtained in Example 7-B), triethylsilane (0.035 mL, 0.22 mmol) and trifluoroacetic acid (1 mL) was stirred at 50°C for 6 hr. The reaction mixture was diluted with ethyl acetate, and washed with saturated aqueous sodium hydrogen carbonate solution and saturated brine. The organic layer was dried over anhydrous sodium sulfate and concentrated under reduced pressure, and the obtained residue was purified by silica gel column chromatography [eluent: hexane-ethyl acetate (70:30-0:100)] to give the title compound (23 mg) as an oil (yield 80%).
MS (ESI+): $[M+H]^+$ 395.
$^1$H NMR (300 MHz, $CDCl_3$) δ 1.32 (3H, t, J = 7.6 Hz), 2.71 (2H, q, J = 7.7 Hz), 4.18 (3H, s), 4.30 (2H, s), 6.65 (1H, d, J

= 1.9 Hz), 7.38-7.61 (5H, m), 7.81 (1H, brs).

Example 12

N-{4-ethenyl-2-[3-(trifluorornethyl)benzyl]-1,3-thiazol-5-yl}-1-methyl-1H-pyrazole-5-carboxamide

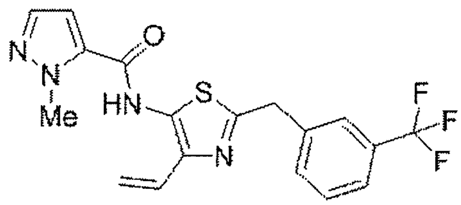

A mixture of N-{4-(1-hydroxyethyl)-2-[3-(trifluaromethyl)benzyl]-1,3-thiazol-5-yl}-1-methyl-1H-pyrazole-5-carboxamide (20 mg, 0.050 mmol) obtained in Example 7-B), methanesulfonyl chloride (0.0058 mL, 0.075 mmol), DBU (0.030 mL, 0.20 mmol) and THF (1 mL) was stirred at room temperature for 5 hr. To the reaction mixture was added saturated aqueous sodium hydrogen carbonate solution, and the mixture was extracted with ethyl acetate. The organic layer was washed with saturated brine, and dried over anhydrous sodium sulfate, and concentrated under reduced pressure. The obtained residue was purified by silica gel column chromatography [eluent: hexane-ethyl acetate (70:30-0:100)] to give the title compound (4.6 mg) as an oil (yield 23%). MS (ESI+): $[M+H]^+$ 393.
$^1H$ NMR (300 MHz, $CDCl_3$) δ 4.20 (3H, s), 4.33 (2H, s), 5.51 (1H, dd, J = 11.1, 1.3 Hz), 6.06 (1H, dd, J = 17.0, 1.5 Hz), 6.61-6.73 (2H, m), 7.42-7.64 (5H, m), 8.05 (1H, brs).

Example 13

N-{4-(1-hydroxypropyl)-2-[3-(trifluoromethyl)benzyl]-1,3-thiazol-5-yl}-1-methyl-1H-pyrazole-5-carboxamide

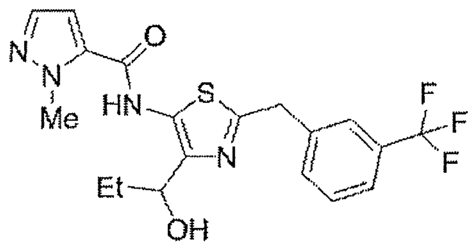

In the same manner as in Example 7-B), N-{4-formyl-2-[3-(trifluoromethyl)benzyl]-1,3-thiazol-5-y1}-1-methyl-1H-pyrazole-5-carboxamide obtained in Example 7-A) and ethylmagnesium bromide were used to give the title compound (156 mg) as an oil (yield 58%) .
MS (ESI+): $[M+H]^+$ 425.
$^1H$ NMR (300 MHz, $CDCl_3$) δ 0.99 (3H, t, J = 7.4 Hz), 1.76-2.07 (2H, m), 3.24-3.76 (1H, m), 4.19 (3H, s), 4.25 (2H, s), 5.11-5.22 (1H, m), 6.58 (1H, d, J = 2.2 Hz), 7.36-7.58 (5H, m), 10.38-10.57 (1H, m).

Example 14

1-methyl-N-{4-propyl-2-[3-(trifluoromethyl)benzyl]-1,3-thiazol-5-yl}-1H-pyrazole-5-carboxamide

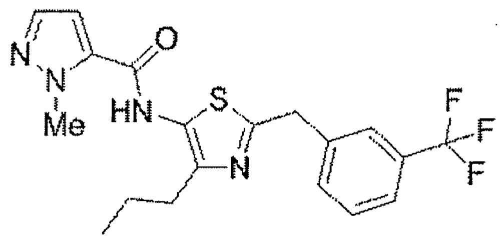

In the same manner as in Example 11, N-{4-(1-hydroxypropyl)-2-[3-(trifluoromethyl)benzyl]-1,3-thiazol-5-yl}-1-methyl-1H-pyrazole-5-carboxamide obtained in Example 13 was used to give the title compound (82 mg) as an oil (yield 84%).
MS (ESI+): $[M+H]^+$ 409.
$^1$H NMR (300 MHz, $CDCl_3$) $\delta$ 0.99 (3H, t, J = 7.4 Hz), 1.78 (2H, sextet, J = 7.3 Hz), 2.60-2.72 (2H, m), 4.20 (3H, s), 4.31 (2H, s), 6.64 (1H, d, J = 1.9 Hz), 7.40-7.62 (5H, m), 7.73 (1H, brs).

Example 15

N-{4-(1-hydroxybutyl)-2-[3-(trifluoromethyl)benzyl]-1,3-thiazol-5-yl}-1-methyl-1H-pyrazole-5-carboxamide

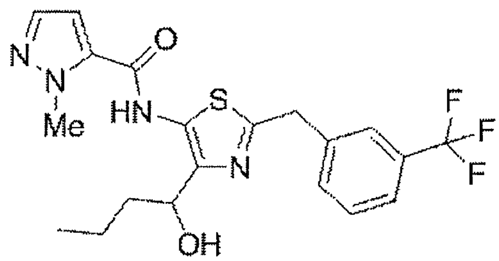

In the same manner as in Example 7-B), N-{4-formyl-2-[3-(trifluoromethyl)benzyl]-1,3-thiazol-5-yl}-1-methyl-1H-pyrazole-5-carboxamide obtained in Example 7-A) and n-propylmagnesium bromide were used to give the title compound (145 mg) as an oil (yield 87%).
MS (ESI+) : $[M+H]^+$ 439.
$^1$H NMR (300 MHz, $CDCl_3$) $\delta$ 0.96 (3H, t, J = 7.3 Hz), 1.36-1.57 (2H, m), 1.77-2.00 (2H, m), 3.11 (1H, d, J = 3.8 Hz), 4.20 (3H, s), 4.26 (2H, s), 5.18-5.27 (1H, m), 6.59 (1H, d, J = 1.9 Hz), 7.37-7.58 (5H, m), 10.40 (1H, s).

Example 16

N-{4-butyl-2-[3-(trifluoromethyl)benzyl]-1,3-thiazol-5-yl}-1-methyl-1H-pyrazole-5-carboxamide

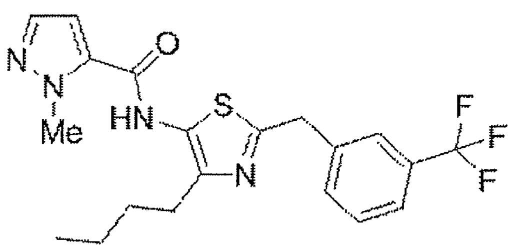

In the same manner as in Example 11, N-{4-(1-hydroxybutyl)-2-[3-(trifluoromethyl)benzyl]-1,3-thiazol-5-yl}-1-methyl-1H-pyrazole-5-carboxamide obtained in Example 15 was used to give the title compound (130 mg) as an oil (yield 93%). MS (ESI+): $[M+H]^+$ 423.
$^1$H NMR (300 MHz, $CDCl_3$) $\delta$ 0.96 (3H, t, J = 7.3 Hz), 1.32-1.48 (2H, m), 1.65-1.80 (2H, m), 2.69 (2H, t, J = 7.5 Hz), 4.20

(3H, s), 4.31 (2H, s), 6.64 (1H, d, J = 2.3 Hz), 7.39-7.62 (5H, m), 7.74 (1H, brs).

Example 17

1-methyl-N-{4-(trifluoromethyl)-2-[3-(trifluoromethyl)benzyl]-1,3-thiazol-5-yl}-1H-pyrazole-5-carboxamide

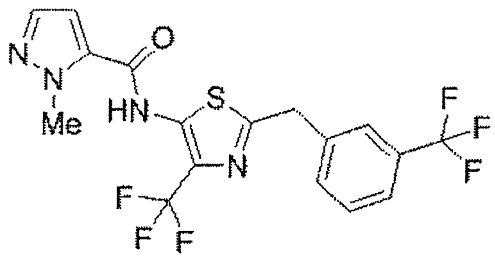

Example 17-A)

2-[3-(trifluoromethyl)phenyl]ethanethioamide

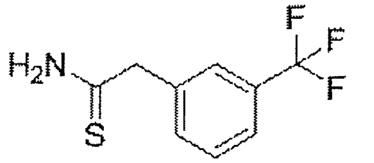

A mixture of [3-(trifluoromethyl)phenyl]acetonitrile (45.2 g, 244 mmol), O,O-diethyl dithiophosphate (50.0 g, 269 mmol) and 4M hydrogen chloride-ethyl acetate solution (500 mL) was stirred at room temperature overnight. The reaction mixture was washed with water, 1M aqueous sodium hydroxide solution and saturated brine. The organic layer was dried over anhydrous sodium sulfate, and concentrated under reduced pressure. The obtained residue was suspended in hexane (500 mL), and the resulting solid was collected by filtration to give the title compound (35 g) as a colorless solid (yield 65%).
MS (ESI+): $[M+H]^+$ 220.
$^1$H NMR (300 MHz, $CDCl_3$) $\delta$ 4.14 (2H, s), 6.66 (1H, brs), 7.37-7.88 (5H, m).

Example 17-B)

ethyl 4-(trifluoromethyl)-2-[3-(trifluoromethyl)benzyl]-1,3-thiazole-5-carboxylate

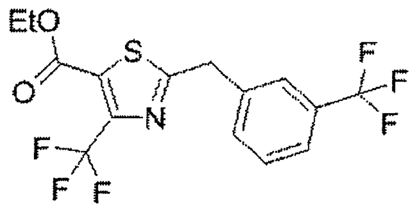

A mixture of 2-[3-(trifluoromethyl)phenyl]ethanethioamide (11.0 g, 50 mmol) obtained in Example 17-A), ethyl 2-chloro-4,4,4-trifluoro-3-oxobutanoate (11.0 g, 50 mmol) and acetic acid (100 mL) was stirred at 100°C for 4 hr. The reaction mixture was concentrated under reduced pressure, and the residue was diluted with ethyl acetate, and washed with saturated aqueous sodium hydrogen carbonate solution and saturated brine. The organic layer was dried over

anhydrous sodium sulfate and concentrated under reduced pressure. The obtained residue was purified by silica gel column chromatography [eluent: hexane-ethyl acetate (97:3-80:20)] to give the title compound (11.4 g) as an oil (yield 60%). MS (ESI+): $[M+H]^+$ 384.
$^1H$ NMR (300 MHz, $CDCl_3$) δ 1.36 (3H, t, J = 7.0 Hz), 4.36 (2H, q, J = 7.1 Hz), 4.40 (2H, s), 7.47-7.66 (4H, m).

Example 17-C)

4-(trifluoromethyl)-2-[3-(trifluoromethyl)benzyl]-1,3-thiazole-5-carboxylic acid

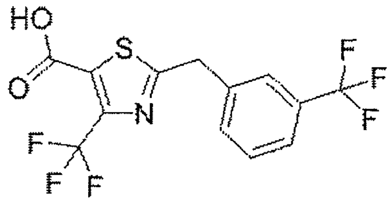

To a mixture of ethyl 4-(trifluoromethyl)-2-[3-(trifluoromethyl)benzyl]-1,3-thiazole-5-carboxylate (5.5 g, 14.4 mmol) obtained in Example 17-B), THF (50 mL) and ethanol (25 mL) was added 1M aqueous sodium hydroxide solution (43 mL, 43 mmol) at 0°C, and the reaction mixture was warmed to room temperature and stirred for 1 hr. To the reaction mixture was added 1M hydrochloric acid (50 mL), and the mixture was extracted with ethyl acetate. The organic layer was washed with saturated brine, and dried over anhydrous sodium sulfate, and concentrated under reduced pressure to give the title compound (5.1 g) as a yellow solid (yield 100%).
MS (ESI+) : $[M+H]^+$ 356.
$^1H$ NMR (300 MHz, $CDCl_3$) δ 4.41 (2H, s), 7.42-7.71 (4H, m), 8.36 (1H, brs).

Example 17-D)

tert-butyl {4-(trifluoromethyl)-2-[3-(trifluoromethyl)benzyl]-1,3-thiazol-5-yl}carbamate

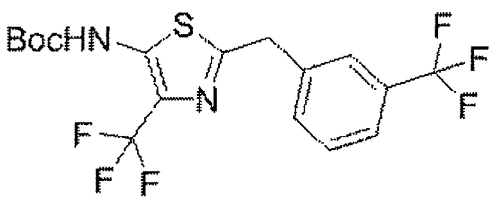

A mixture of 4-(trifluoromethyl)-2-[3-(trifluoromethyl)benzyl]-1,3-thiazole-5-carboxylic acid (4.5 g, 12.7 mmol) obtained in Example 17-C), DPPA (5.5 mL), triethylamine (7.0 mL) and tert-butanol (50 mL) was stirred at 80°C for 3 hr. The reaction mixture was diluted with ethyl acetate, and washed with saturated brine. The organic layer was dried over anhydrous sodium sulfate, and concentrated under reduced pressure. The obtained residue was purified by silica gel column chromatography [eluent: hexane-ethyl acetate (97:3-90:10)] to give the title compound (2.7 g) as an oil (yield 50%).
MS (ESI+) : $[M+H]^+$ 427.
$^1H$ NMR (300 MHz, $CDCl_3$) δ 1.51 (9H, s), 4.27 (2H, s), 7.33-7.59 (5H, m).

Example 17-E)

4-(trifluoromethyl)-2-[3-(trifluoromethyl)benzyl]-1,3-thiazole-5-amine

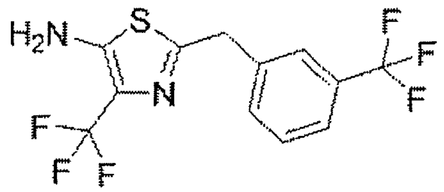

A mixture of tert-butyl {4-(trifluoromethyl)-2-[3-(trifluoromethyl)benzyl]-1,3-thiazol-5-yl}carbamate (1.28 g, 3.0 mmol) obtained in Example 17-D), concentrated hydrochloric acid (5 mL) and ethanol (10 mL) was stirred at 50°C for 3 min. The reaction mixture was neutralized with aqueous sodium hydrogen carbonate solution, and diluted with ethyl acetate. The organic layer was washed with saturated brine, dried over anhydrous sodium sulfate, and concentrated under reduced pressure. The obtained residue was purified by silica gel column chromatography [eluent: hexane-ethyl acetate (90:10-70:30)] to give the title compound (624 mg) as a pale-yellow solid (yield 64%).
MS (ESI+): $[M+H]^+$ 327.
$^1H$ NMR (300 MHz, $CDCl_3$) δ 4.19 (2H, s), 4.36 (2H, brs), 7.39-7.61 (4H, m).

Example 17-F)

1-methyl-N-{4-(trifluoromethyl)-2-[3-(trifluoromethyl)benzyl]-1,3-thiazol-5-yl}-1H-pyrazole-5-carboxamide

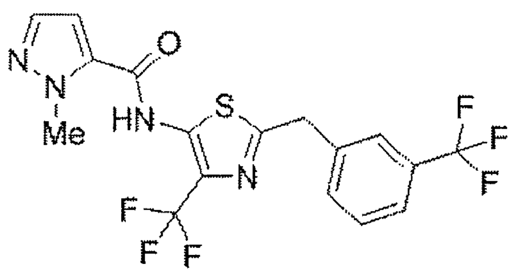

A mixture of 4-(trifluoromethyl)-2-[3-(trifluoromethyl)benzyl]-1,3-thiazole-5-amine (33 mg, 0.10 mmol) obtained in Example 17-E) and 1-methyl-1H-pyrazole-5-carbonyl chloride (15 mg, 0.10 mmol) obtained in Example 5-C) was stirred at 120°C for 1 hr. After cooling to room temperature, the mixture was purified by silica gel column chromatography [eluent: hexane-ethyl acetate (90:10-70:30)] to give the title compound (27 mg) as a pale-yellow solid (yield 62%).
MS (ESI+): $[M+H]^+$ 435.
$^1H$ NMR (300 MHz, $CDCl_3$) δ 4.20 (3H, s), 4.34 (2H, s), 6.67 (1H, d, J = 2.5 Hz), 7.43-7.62 (5H, m), 8.69 (1H, brs).

Example 18

N-{4-acetyl-2-[3-(trifluoromethyl)benzyl]-1,3-thiazol-5-yl}-1-methyl-1H-pyrazole-5-carboxamide

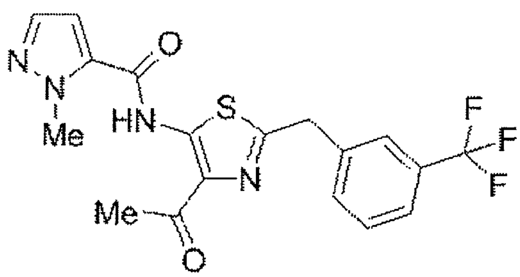

To a solution of N-{4-(1-hydroxyethyl)-2-[3-(trifluoromethyl)benzyl]-1,3-thiazol-5-yl}-1-methyl-1H-pyrazole-5-carboxamide (30 mg, 0.073 mmol) obtained in Example 7-B) in acetonitrile (1 mL) was slowly added Dess-Martin reagent

(37 mg, 0.088 mmol) at 0°C. The reaction mixture was warmed to room temperature, and stirred for 3 hr. The reaction mixture was diluted with ethyl acetate, and washed with water and saturated brine. The organic layer was dried over anhydrous sodium sulfate, and concentrated under reduced pressure to give the title compound (29 mg) as a colorless solid (yield 97%).
MS (ESI+) : $[M+H]^+$ 409.
$^1H$ NMR (300 MHz, $CDCl_3$) δ 2.72 (3H, s), 4.23 (3H, s), 4.33 (2H, s), 6.90 (1H, d, J = 2.2 Hz), 7.42-7.64 (5H, m), 12.29 (1H, s).

Example 19

tert-butyl 3-({4-methyl-2-[3-(trifluoromethyl)benzyl]-1,3-thiazol-5-yl}carbamoyl)pyrrolidine-1-carboxylate

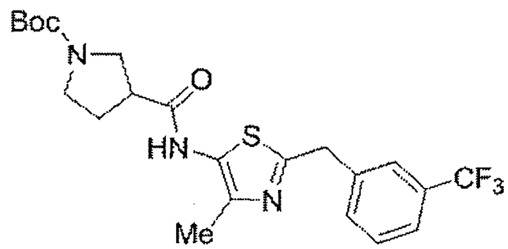

Example 19-A)

2-[3-(trifluoromethyl)phenyl]ethanethioamide

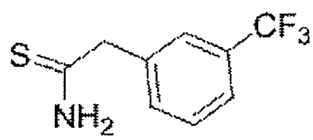

A mixture of [3-(trifluoromethyl)phenyl]acetonitrile (25 g, 135 mmol), O,O-diethyl dithiophosphate (27.5 mL, 148 mmol) and 4M hydrogen chloride-ethyl acetate solution (500 mL) was stirred at room temperature overnight. Water (200 mL) was added thereto in an ice bath, and the organic layer was separated, successively washed twice with water (200 mL), twice with 0.2N aqueous sodium hydroxide solution (200 mL), saturated aqueous sodium hydrogen carbonate solution (200 mL) and saturated brine (200 mL), dried over anhydrous sodium sulfate, and concentrated under reduced pressure. The obtained residue was purified by basic silica gel column chromatography [eluent: hexane-ethyl acetate (2:1)] to give the title compound (27 g) as a pale-yellow oil (yield 92%).
MS (ESI+): $[M+H]^+$ 219.
$^1H$ NMR (300 MHz, $CDCl_3$) δ 4.14 (2H, s), 6.63 (1H, brs), 7.45-7.66 (4H, m).
* The peak of one proton was not observed.

Example 19-B)

ethyl 4-methyl-2-[3-(trifluoromethyl)benzyl]-1,3-thiazole-5-carboxylate

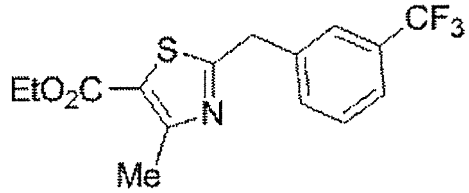

A solution of 2-[3-(trifluoromethyl)phenyl]ethanethioamide (20.6 g, 94 mmol) obtained in Example 19-A) and ethyl 2-chloro-3-oxobutanoate (16.4 mL, 112 mmol) in pyridine (60 mL)-ethanol (60 mL) was stirred at room temperature for 2 days. The reaction mixture was concentrated under reduced pressure, azeotropically distilled twice with toluene, diluted with ethyl acetate, washed successively with 0.2M hydrochloric acid and saturated brine, dried over anhydrous magnesium sulfate, and concentrated under reduced pressure. The obtained residue was purified by silica gel column chromatography [eluent: hexane-ethyl acetate (10:1-2:1)] to give the title compound (15.9 g) as a pale-yellow oil (yield 78%).
MS (ESI+) : $[M+H]^+$ 329.
$^1H$ NMR (300 MHz, $CDCl_3$) δ 1.33 (3H, t, J = 7.2 Hz), 2.71 (3H, s), 4.24-4.37 (4H, m), 7.41-7.63 (4H, m).

Example 19-C)

4-methyl-2-[3-(trifluoromethyl)benzyl]-1,3-thiazole-5-carboxylic acid

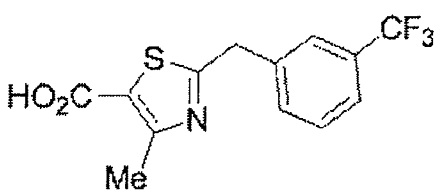

To a solution of ethyl 4-methyl-2-[3-(trifluoromethyl)benzyl]-1,3-thiazole-5-carboxylate (15.9 g, 48 mmol) obtained in Example 19-B) in ethanol (60 mL)-THF (30 mL) was added 4M aqueous sodium hydroxide solution (36 mL), and the mixture was stirred overnight. The reaction mixture was concentrated under reduced pressure, 1M hydrochloric acid (150 mL) was added thereto in an ice bath, and the mixture was extracted with ethyl acetate. The organic layer was washed with saturated brine, dried over anhydrous magnesium sulfate, and concentrated under reduced pressure. The obtained residue was crystallized from hexane-ethyl acetate to give the title compound (12.1 g) as colorless crystals (yield 65%).
MS (ESI+): $[M+H]^+$ 302.
$^1H$ NMR (300 MHz, DMSO-$d_6$) δ 2.57 (3H, s), 4.43 (2H, s), 7.55-7.80 (4H, m), 13.23 (1H, brs).

Example 19-D)

tert-butyl {4-methyl-2-[3-(trifluoromethyl)benzyl]-1,3-thiazol-5-yl}carbamate

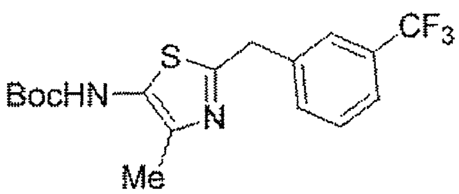

A mixture of 4-methyl-2-[3-(trifluoromethyl)benzyl]-1,3-thiazole-5-carboxylic acid (9.47 g, 31.4 mmol) obtained in Example 19-C), DPPA (16.9 mL, 78.6 mmol), triethylamine (17.4 mL, 126 mmol) and tert-butanol (100 mL) was stirred at 90°C for 2 hr. The reaction mixture was cooled to room temperature, and concentrated under reduced pressure. The obtained residue was purified by silica gel column chromatography [eluent: hexane-ethyl acetate (97:3-1:1)] to give the title compound (8.26 g) as a pale-yellow oil (yield 71%).
MS (ESI+): $[M+H]^+$ 372.
$^1H$ NMR (300 MHz, $CDCl_3$) δ 1.49 (9H, s), 2.29 (3H, s), 4.24 (2H, s), 6.40 (1H, brs), 7.32-7.59 (4H, m).

Example 19-E)

tert-butyl 3-({4-methyl-2-[3-(trifluoromethyl)benzyl]-1,3-thiazol-5-yl}carbamoyl)pyrrolidine-1-carboxylate

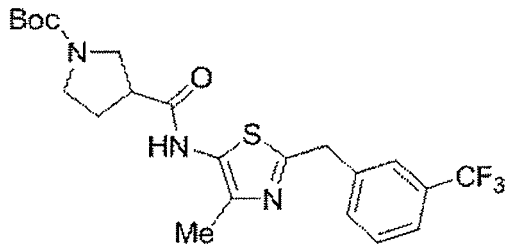

To a solution of tert-butyl {4-methyl-2-[3-(trifluoromethyl)benzyl]-1,3-thiazol-5-yl}carbamate (780 mg, 2.09 mmol) obtained in Example 19-D) in ethanol (1.2 mL) was added dropwise concentrated hydrochloric acid (3 mL), and the mixture was stirred at room temperature for 10 min. The reaction mixture was cooled to 0°C, neutralized with 8M aqueous sodium hydroxide solution (4.5 mL), adjusted to pH 9-10 with saturated aqueous sodium hydrogen carbonate solution, and extracted with ethyl acetate. The organic layer was washed with saturated brine, dried over anhydrous sodium sulfate, and concentrated under reduced pressure. The obtained residue was dissolved in DMF (7 mL), and 1-(tert-butoxycarbonyl)pyrrolidine-3-carboxylic acid (460 mg, 2.09 mmol), N,N,N',N'-tetramethylchloroform amidinium hexafluorophosphate (TCFH) (586 mg, 2.09 mmol) and DIEA (2.62 mL, 15.0 mmol) were added at 0°C. The reaction mixture was stirred at room temperature for 3.5 hr, water was added and the mixture was extracted with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-0:1)] to give the title compound (422 mg) as a yellow oil (yield 43%).
MS (ESI+) : $[M+H]^+$ 469.
$^1$H NMR (300 MHz, $CDCl_3$) $\delta$ 1.46 (9H, s), 2.19 (2H, td, J= 7.8, 4.2 Hz), 2.34 (3H, s), 2.36-2.51 (1H, m), 2.94-3.11 (1H, m), 3.39 (1H, dt, J = 10.9, 7.6 Hz), 3.49-3.76 (3H, m), 4.26 (2H, s), 7.36-7.63 (4H, m).

Example 20

N-{4-methyl-2-[3-(trifluoromethyl)benzyl]-1,3-thiazol-5-yl}pyrrolidine-3-carboxamide hydrochloride

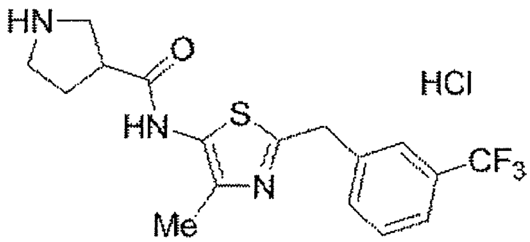

To a solution of tert-butyl 3-({4-methyl-2-[3-(trifluoromethyl)benzyl]-1,3-thiazol-5-yl}carbamoyl)pyrrolidine-1-carboxylate (258 mg, 0.549 mmol) obtained in Example 19-E) in ethyl acetate (0.5 mL) was added 4M hydrogen chloride-ethyl acetate solution (4 mL), and the mixture was stirred at room temperature for 2 hr. The precipitated white solid was collected by filtration, washed with ethyl acetate, dried over anhydrous sodium sulfate and concentrated under reduced pressure to give the title compound (199 mg) as a yellow oil (yield 89%).
MS (ESI+) : $[M+H]^+$ 369.
$^1$H NMR (300 MHz, DMSO-$d_6$) $\delta$ 1.84-2.10 (1H, m), 2.13-2.30 (1H, m), 2.31-2.41 (3H, m), 3.11-3.32 (3H, m), 3.33-3.57 (2H, m), 4.33 (2H, s), 7.45-7.89 (4H, m), 9.13 (1H, brs), 9.45 (1H, brs), 10.91 (1H, s).

Example 21

1-acetyl-N-{4-methyl-2-[3-(trifluoromethyl)benzyl]-1,3-thiazol-5-yl}pyrrolidine-3-carboxamide

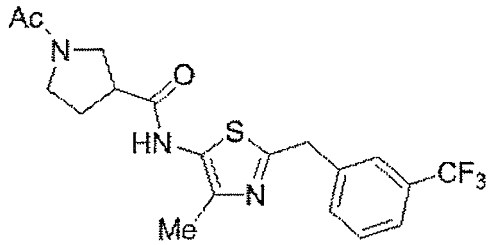

N-{4-methyl-2-[3-(trifluoromethyl)benzyl]-1,3-thiazol-5-yl}pyrrolidine-3-carboxamide hydrochloride (130 mg, 0.32 mmol) obtained in Example 20 was dissolved in water, saturated aqueous sodium hydrogen carbonate solution and ethyl acetate were added and the mixture was stirred for 5 min. The organic layer was separated, washed with saturated brine, dried over anhydrous sodium sulfate, and concentrated under reduced pressure. The obtained residue was dissolved in DMA (3 mL), acetyl chloride (25 μL, 0.35 mmol) was added at 0°C, and the mixture was stirred at the same temperature for 30 min. Water was added to the reaction mixture, and the mixture was extracted with ethyl acetate. The organic layer was washed successively with water and saturated brine, dried over anhydrous sodium sulfate, and concentrated under reduced pressure. The obtained residue was purified by basic silica gel column chromatography [eluent: ethyl acetate-methanol (1:0-7:3)] to give the title compound (95 mg) as a colorless oil (yield 72%).
MS (ESI+) : $[M+H]^+$ 412.
$^1H$ NMR (300 MHz, $CDCl_3$) δ 2.00-2.09 (3H, m), 2.13-2.30 (1H, m), 2.31-2.47 (4H, m), 3.00-3.26 (1H, m), 3.39-3.93 (4H, m), 4.26 (2H, d, J= 3.0 Hz), 7.39-7.59 (4H, m), 7.68-8.10 (1H, m).

Example 22

1-(methylsulfonyl)-N-{4-methyl-2-[3-(trifluoromethyl)benzyl]-1,3-thiazol-5-yl}pyrrolidine-3-carboxamide

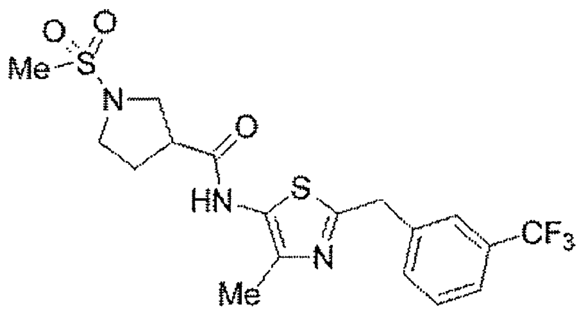

Example 22-A)

1-(methylsulfonyl)-N-{4-methyl-2-[3-(trifluoromethyl)benzyl]-1,3-thiazol-5-yl}pyrrolidine-3-carboxamide

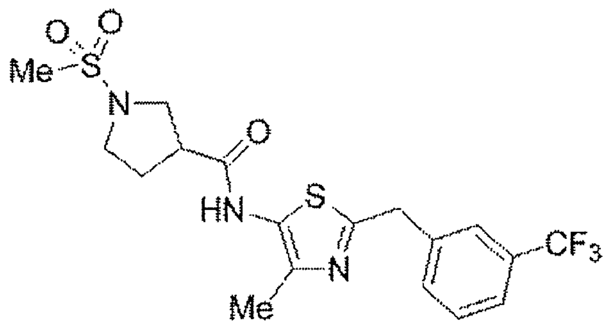

To a mixture of N-{4-methyl-2-[3-(trifluoromethyl)benzyl]-1,3-thiazol-5-yl}pyrrolidine-3-carboxamide hydrochloride (1.52 g, 4.11 mmol) obtained in Example 20 and THF (10 mL) were added triethylamine (855 μL, 6.17 mmol) and methanesulfonyl chloride (478 μL, 6.17 mmol) at 0°C. The reaction mixture was stirred at room temperature overnight. Water was added to the reaction mixture, and the mixture was extracted with ethyl acetate. The organic layer was washed successively with water and saturated brine, dried over anhydrous sodium sulfate, and concentrated under reduced pressure. The obtained residue was purified by basic silica gel column chromatography [eluent: hexane-ethyl acetate (9:1-0:1)], and crystallized from hexane-ethyl acetate to give the title compound (890 mg) as colorless crystals (yield 48%).
MS (ESI+): $[M+H]^+$ 447.
$^1$H NMR (300 MHz, $CDCl_3$) δ 2.13-2.35 (2H, m), 2.37 (3H, s), 2.93 (3H, s), 3.10-3.25 (1H, m), 3.42-3.63 (3H, m), 3.63-3.77 (1H, m), 4.27 (2H, s), 7.41-7.59 (4H, m), 7.64 (1H, s).

Example 22-B)

(3R)-1-(methylsulfonyl)-N-{4-methyl-2-[3-(trifluoromethyl)benzyl]-1,3-thiazol-5-yl}pyrrolidine-3-carboxamide

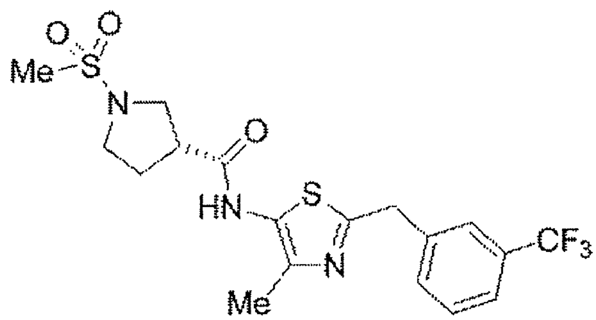

(3S)-1-(methylsulfonyl)-N-{4-methyl-2-[3-(trifluoromethyl)benzyl]-1,3-thiazol-5-yl}pyrrolidine-3-carboxamide

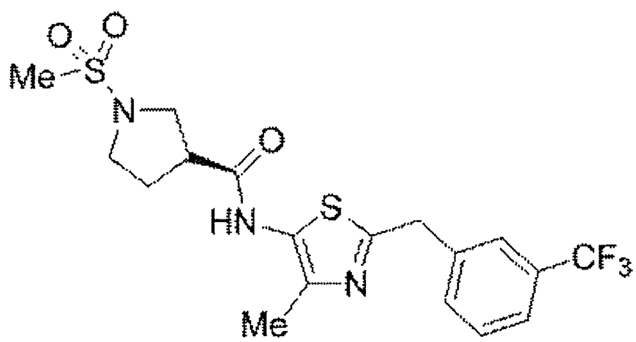

1-(Methylsulfonyl)-N-{4-methyl-2-[3-(trifluoromethyl)benzyl]-1,3-thiazol-5-yl}pyrrolidine-3-carboxamide (racemate; 600 mg) obtained in Example 22-A) was fractionated by high performance liquid chromatography (column: CHIRALCEL OD (50 mmID×500 mmL manufactured by DAICEL CHEMICAL INDUSTRIES, LTD.), mobile phase: hexane/ethanol = 80/20). A fraction solution containing an optically active form having a shorter retention time, which was obtained by the above-mentioned high performance liquid chromatography conditions, was concentrated to give 1-(methylsulfonyl)-N-{4-methyl-2-[3-(trifluoromethyl)benzyl]-1,3-thiazol-5-yl}pyrrolidine-3-carboxamide (retention time short) (292 mg, 97%, >99.9%ee). A fraction solution containing an optically active form having a longer retention time was concentrated to give 1-(methylsulfonyl)-N-{4-methyl-2-[3-(trifluoromethyl)benzyl]-1,3-thiazol-5-yl}pyrrolidine-3-carboxamide (retention time long)(293 mg, 98%, >99.9%ee). 1-(methylsulfonyl)-N-{4-methyl-2-[3-(trifluoromethyl)benzyl]-1,3-thiazol-5-yl}pyrrolidine-3-carboxamide (retention time short):
MS (ESI+) : $[M+H]^+$ 448.
$^1$H NMR (300 MHz, $CDCl_3$) δ 2.18-2.34 (2H, m), 2.37 (3H, s), 2.93 (3H, s), 3.11-3.26 (1H, m), 3.39-3.62 (3H, m), 3.63-3.75 (1H, m), 4.27 (2H, s), 7.31-7.60 (4H, m), 7.66 (1H, s).
1-(methylsulfonyl)-N-{4-methyl-2-[3-(trifluoromethyl)benzyl]-1,3-thiazol-5-yl}pyrrolidine-3-carboxamide (retention time long) :
MS (ESI+): $[M+H]^+$ 448.
$^1$H NMR (300 MHz, $CDCl_3$) δ 2.20-2.35 (2H, m), 2.37 (3H, s), 2.93 (3H, s), 3.10-3.26 (1H, m), 3.40-3.63 (3H, m), 3.63-3.77 (1H, m), 4.27 (2H, s), 7.36-7.61 (4H, m), 7.66 (1H, s).

Example 23

N1-ethyl-N3-{4-methyl-2-[3-(trifluoromethyl)benzyl]-1,3-thiazol-5-yl}pyrrolidine-1,3-dicarboxamide

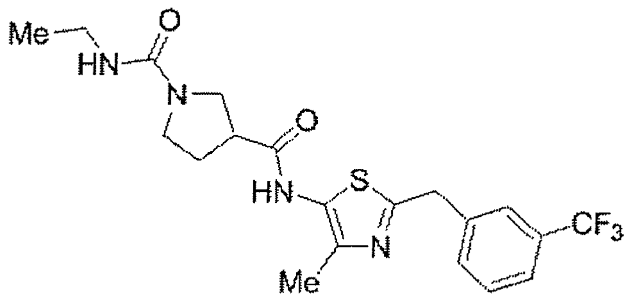

N-{4-methyl-2-[3-(trifluoromethyl)benzyl]-1,3-thiazol-5-yl}pyrrolidine-3-carboxamide hydrochloride (58 mg, 0.16 mmol) obtained in Example 20 was dissolved in water, saturated aqueous sodium hydrogen carbonate solution and ethyl acetate was added, and the mixture was stirred for 5 min. The organic layer was separated, washed with saturated brine, dried over anhydrous sodium sulfate, and concentrated under reduced pressure. The obtained residue was dissolved in THF (3 mL), ethyl isocyanate (14 μL, 0.17 mmol) and DIEA (27 μL, 0.16 mmol) were added, and the mixture was stirred for 2 days. The reaction mixture was concentrated under reduced pressure, and the obtained residue was purified by silica gel column chromatography [eluent: hexane-ethyl acetate (9:1-0:1)] to give the title compound (27 mg) as a yellow oil (yield 39%).
MS (ESI+): $[M+H]^+$ 440.
$^1$H NMR (300 MHz, $CDCl_3$) δ 1.11 (3H, t, J = 7.2 Hz), 2.10-2.24 (1H, m), 2.25-2.40 (4H, m), 3.06-3.39 (4H, m), 3.43-3.62 (2H, m), 3.65-3.79 (1H, m), 4.25 (3H, s), 7.35-7.59 (4H, m), 8.81 (1H, d, J = 9.8 Hz).

Example 24

3-methyl-N-{4-methyl-2-[3-(trifluoromethyl)benzyl]-1,3-thiazol-5-yl}isoxazole-4-carboxamide

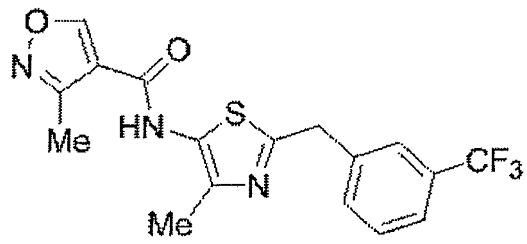

To a solution of tert-butyl {4-methyl-2-[3-(trifluoromethyl)benzyl]-1,3-thiazol-5-yl}carbamate (680 mg, 1.83 mmol) obtained in Example 19-D) in ethanol (2 mL) was added dropwise concentrated hydrochloric acid (1 mL), and the mixture was stirred at room temperature for 10 min. The reaction mixture was cooled to 0°C, neutralized with 8M aqueous sodium hydroxide solution (1.5 mL), adjusted to pH 9-10 with saturated aqueous sodium hydrogen carbonate solution, and extracted with ethyl acetate. The organic layer was washed with saturated brine, dried over anhydrous sodium sulfate, and concentrated under reduced pressure. The obtained residue was dissolved in DMF (5 mL), and 3-methylisoxazole-4-carboxylic acid (232 mg, 1.83 mmol), N,N,N',N'-tetramethylchloroform amidinium hexafluorophosphate (TCFH)(575 mg, 2.01 mmol) and DIEA (1.59 mL, 9.13 mmol) were added at 0°C. The reaction mixture was stirred at room temperature for 3 hr, water was added and the mixture was extracted with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-0:1)] to give the title compound (257 mg) as a yellow oil (yield 37%).
MS (ESI+): $[M+H]^+$ 381.
$^1$H NMR (300 MHz, $CDCl_3$) δ 2.39 (3H, s), 2.56 (3H, s), 4.30 (2H, s), 7.40-7.73 (5H, m), 8.84 (1H, brs).

Example 25

N,1-dimethyl-N-{4-methyl-2-[3-(trifluoromethyl)benzyl]-1,3-thiazol-5-yl}-1H-pyrazole-5-carboxamide

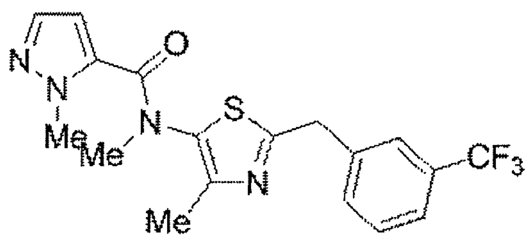

To a solution of 1-methyl-N-{4-methyl-2-[3-(trifluoromethyl)benzyl]-1,3-thiazol-5-yl}-1H-pyrazole-5-carboxamide (54 mg, 0.14 mmol) obtained in Example 3-E) and iodomethane (13 μL, 0.213 mmol) in DMF (2 mL) was added sodium hydride (60%; 10 mg, 0.213 mmol) in an ice bath. The reaction mixture was stirred at room temperature for 1.5 hr. Water was added to the reaction mixture and the mixture was extracted with ethyl acetate. The organic layer was washed successively with water and saturated brine, dried over anhydrous sodium sulfate, and concentrated under reduced pressure. The obtained residue was purified by silica gel column chromatography [eluent: hexane-ethyl acetate (9:1-0:1)] to give the title compound (11.8 mg) as a yellow oil (yield 21%). MS (ESI+): $[M+H]^+$ 394.
$^1$H NMR (300 MHz, $CDCl_3$) δ 2.13 (3H, s), 3.34 (3H, s), 4.08 (3H, s), 4.27 (2H, s), 5.76 (1H, brs), 7.21-7.30 (1H, m), 7.42-7.65 (4H, m).

Example 26

2,5-dimethyl-N-{4-methyl-2-[3-(trifluoromethyl)benzyl]-1,3-thiazol-5-yl}furan-3-carboxamide

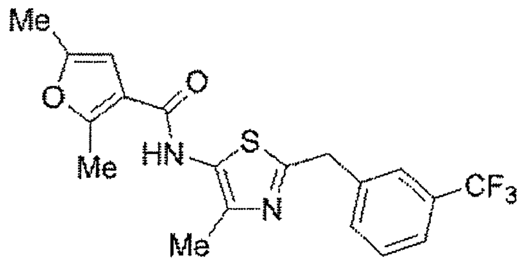

To a solution of tert-butyl {4-methyl-2-[3-(trifluoromethyl)benzyl]-1,3-thiazol-5-yl}carbamate (297 mg, 0.80 mmol) obtained in Example 19-D) in ethanol (1 mL) was added dropwise concentrated hydrochloric acid (2 mL), and the mixture was stirred at room temperature for 10 min. The reaction mixture was cooled to 0°C, basified with 8M aqueous sodium hydroxide solution (4 mL), and diluted with THF (5 mL). A solution of 2,5-dimethylfuran-3-carbonyl chloride (254 mg, 1.60 mmol) in THF (2 mL) was added dropwise at the same temperature, and the mixture was stirred at room temperature for 30 min. The reaction mixture was neutralized with saturated aqueous ammonium chloride solution, and extracted twice with ethyl acetate. The organic layer was washed with saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-0:1)], and crystallized from hexane-ethyl acetate to give the title compound (227 mg) as colorless crystals (yield 72%).
MS (ESI+): $[M+H]^+$ 394.
$^1H$ NMR (300 MHz, $CDCl_3$) $\delta$ 2.29 (3H, s), 2.38 (3H, s), 2.57 (3H, s), 4.28 (2H, s), 6.08 (1H, s), 7.32-7.63 (5H, m).

Example 27

N-{4-methyl-2-[3-(trifluoromethyl)benzyl]-1,3-thiazol-5-yl}thiophene-3-carboxamide

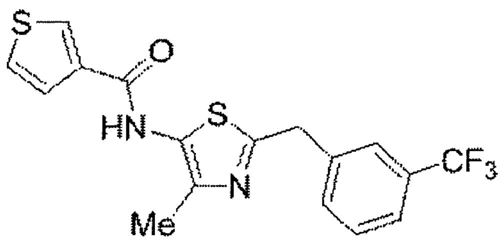

In the same manner as in Example 26, tert-butyl {4-methyl-2-[3-(trifluoromethyl)benzyl]-1,3-thiazol-5-yl}carbamate obtained in Example 19-D) and thiophene-3-carbonyl chloride were used to give the title compound (213 mg) (yield 66%).
MS (ESI+): $[M+H]^+$ 382.
$^1H$ NMR (300 MHz, $CDCl_3$) $\delta$ 2.41 (3H, s), 4.29 (2H, s), 7.39-7.49 (3H, m), 7.48-7.55 (2H, m), 7.58 (1H, s), 7.72 (1H, brs), 7.98-8.05 (1H, m).

Example 28

2-chloro-N-{4-methyl-2-[3-(trifluoromethyl)benzyl]-1,3-thiazol-5-yl}pyridine-3-carboxamide

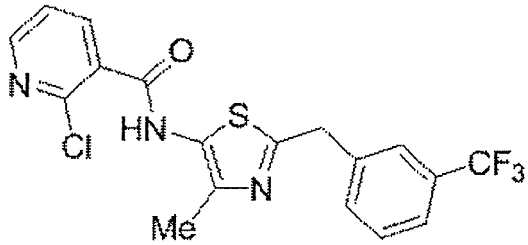

In the same manner as in Example 26, tert-butyl {4-methyl-2-[3-(trifluoromethyl)benzyl]-1,3-thiazol-5-yl}carbamate obtained in Example 19-D) and 2-chloropyridine-3-carbonyl chloride were used to give the title compound (186 mg) (yield 56%).
MS (ESI+) : $[M+H]^+$ 411.
$^1H$ NMR (300 MHz, $CDCl_3$) $\delta$ 2.46 (3H, s), 4.31 (2H, s), 7.39-7.50 (2H, m), 7.50-7.57 (2H, m), 7.59 (1H, s), 8.39 (1H, dd, J = 8.0, 1.9 Hz), 8.55 (1H, dd, J = 4.9, 1.9 Hz), 8.94 (1H, brs).

Example 29

N-{4-methyl-2-[3-(trifluoromethyl)benzyl]-1,3-thiazol-5-yl}piperidine-3-carboxamide hydrochloride

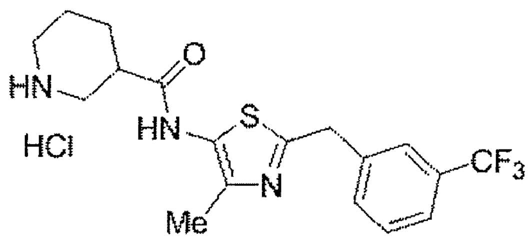

Example 29-A)

tert-butyl 3-({4-methyl-2-[3-(trifluoromethyl)benzyl)-1,3-thiazol-5-yl}carbamoyl)piperidine-1-carboxylate

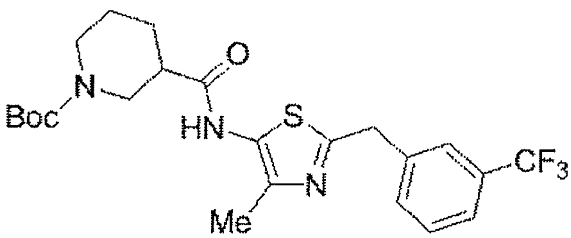

To a solution of tert-butyl {4-methyl-2-[3-(trifluoromethyl)benzyl]-1,3-thiazol-5-yl}carbamate (1.10 g, 2.95 mmol) obtained in Example 19-D) in ethanol (2.0 mL) was added dropwise concentrated hydrochloric acid (4.0 mL), and the mixture was stirred at room temperature for 10 min. The reaction mixture was cooled to 0°C, neutralized with 8M aqueous sodium hydroxide solution (6 mL), adjusted to pH 9-10 with saturated aqueous sodium hydrogen carbonate solution, and extracted with ethyl acetate. The organic layer was washed with saturated brine, dried over anhydrous sodium sulfate, and concentrated under reduced pressure. The obtained residue was dissolved in DMA (9 mL), and 1-(tert-butoxycarbonyl)piperidine-3-carboxylic acid (812 mg, 3.54 mmol), HATU (1.35 g, 3.54 mmol) and DIEA (257 μL, 1.48 mmol) were added at 0°C. The reaction mixture was stirred at 60°C for 2.5 hr, water was added and the mixture was extracted with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-0:1)] to give the title compound (862 mg) as a yellow oil (yield 61%).

MS (ESI+): $[M+H]^+$ 483.
$^1H$ NMR (300 MHz, $CDCl_3$) δ 1.44 (9H, s), 1.47-1.63 (2H, m), 1.78-1.94 (1H, m), 2.18 (1H, d, J = 4.5 Hz), 2.40 (3H, s), 2.50-2.69 (1H, m), 3.46 (2H, brs), 3.67 (2H, d, J = 2.7 Hz), 4.25 (2H, s), 7.33-7.63 (4H, m).
* The peak of "NH group" was not observed.

Example 29-B)

N-{4-methyl-2-[3-(trifluoromethyl)benzyl]-1,3-thiazol-5-yl}piperidine-3-carboxamide hydrochloride

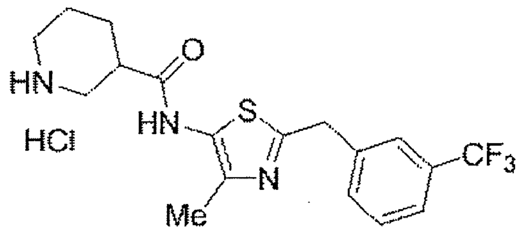

In the same manner as in Example 20, tert-butyl 3-({4-methyl-2-[3-(trifluoromethyl)benzyl]-1,3-thiazol-5-yl}carbamoyl)piperidine-1-carboxylate obtained in Example 29-A) was used to give the title compound (764 mg) (yield 100%).
MS (ESI+): $[M+H]^+$ 383.
$^1H$ NMR (300 MHz, DMSO-$d_6$) δ 1.45-1.64 (1H, m), 1.69-1.85 (2H, m), 1.95-2.09 (1H, m), 2.35 (3H, s), 2.70-3.05 (2H, m), 3.05-3.22 (2H, m), 3.28 (1H, d, J = 11.4 Hz), 4.35 (2H, s), 7.51-7.68 (3H, m), 7.72 (1H, s), 8.99 (1H, d, J = 10.2 Hz), 9.26 (1H, brs), 10.97 (1H, s).

Example 30

1-(ethylsulfonyl)-N-(4-methyl-2-[3-(trifluoromethyl)benzyl]-1,3-thiazol-5-yl}pyrrolidine-3-carboxamide

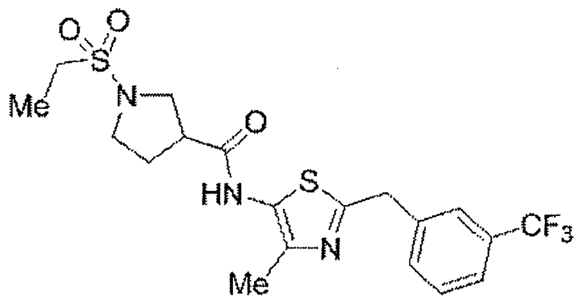

To a mixture of N-{4-methyl-2-[3-(trifluoromethyl)benzyl]-1,3-thiazol-5-yl}pyrrolidine-3-carboxamide hydrochloride (540 mg, 1.46 mmol) obtained in Example 20 and THF (3 mL) were added triethylamine (304 μL, 2.19 mmol) and ethanesulfonyl chloride (90%; 231 μL, 2.19 mmol) at 0°C. The reaction mixture was stirred at room temperature for 3 hr. Water was added to the reaction mixture, and the mixture was extracted with ethyl acetate. The organic layer was washed successively with water and saturated brine, dried over anhydrous sodium sulfate, and concentrated under reduced pressure. The obtained residue was purified by basic silica gel column chromatography [eluent: hexane-ethyl acetate (4:1-0:1)], and crystallized from diethyl ether-hexane to give the title compound (296 mg) as colorless crystals (yield 44%).
MS (ESI+) : $[M+H]^+$ 461.
$^1H$ NMR (300 MHz, $CDCl_3$) δ 1.40 (3H, t, J = 7.3 Hz), 2.28 (2H, quintet, J = 6.7 Hz), 2.36 (3H, s), 3.08 (2H, q, J = 7.5 Hz), 3.12-3.25 (1H, m), 3.46-3.63 (3H, m), 3.68-3.80 (1H, m), 4.26 (2H, s), 7.38-7.64 (4H, m), 7.83 (1H, s).

Example 31

N-{4-methyl-2-[3-(trifluoromethyl)benzyl]-1,3-thiazol-5-yl}-1-[(2,2,2-trifluoroethyl)sulfonyl]pyrrolidine-3-carboxamide

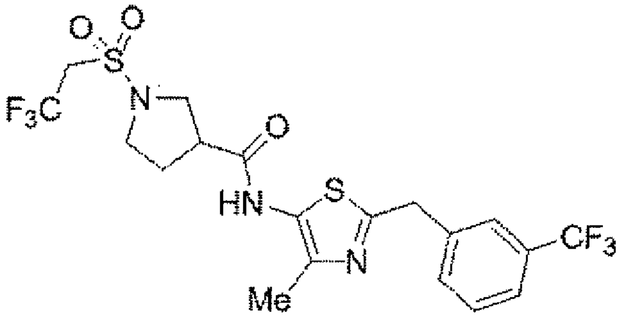

To a mixture of N-{4-methyl-2-[3-(trifluoromethyl)benzyl]-1,3-thiazol-5-yl}pyrrolidine-3-carboxamide hydrochloride (510 mg, 1.38 mmol) obtained in Example 20 and THF (3 mL) were added triethylamine (287 μL, 2.07 mmol) and 2,2,2-trifluoroethanesulfonyl chloride (378 mg, 2.07 mmol) at 0°C. The reaction mixture was stirred at room temperature for 4 hr. Water was added to the reaction mixture, and the mixture was extracted with ethyl acetate. The organic layer was washed successively with water and saturated brine, dried over anhydrous sodium sulfate, and concentrated under reduced pressure. The obtained residue was purified by basic silica gel column chromatography [eluent: hexane-ethyl acetate (4:1-0:1)], and crystallized from hexane-ethyl acetate to give the title compound (158 mg) as colorless crystals (yield 22%).
MS (ESI+): $[M+H]^+$ 515.
$^1H$ NMR (300 MHz, $CDCl_3$) δ 2.24-2.35 (2H, m), 2.36 (3H, s), 3.10-3.22 (1H, m), 3.58 (2H, t, J = 7.0 Hz), 3.64-3.80 (2H, m), 3.86 (2H, q, J = 9.3 Hz), 4.28 (2H, s), 7.39-7.60 (5H, m).

Example 32

N-{4-methyl-2-[3-(trifluoromethyl)benzyl]-1,3-thiazol-5-yl}-1-[(trifluoromethyl)sulfonyl]pyrrolidine-3-carboxamide

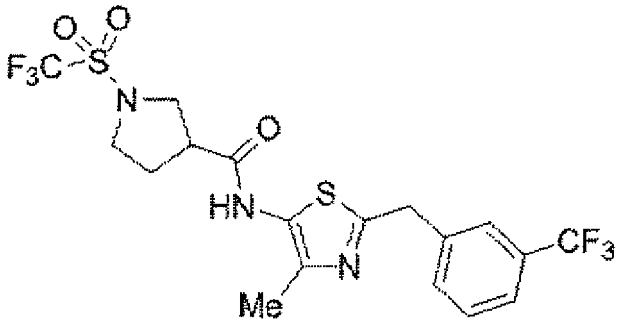

To a mixture of N-{4-methyl-2-[3-(trifluoromethyl)benzyl]-1,3-thiazol-5-yl}pyrrolidine-3-carboxamide hydrochloride (500 mg, 1.35 mmol) obtained in Example 20 and THF (3 mL) were added triethylamine (281 μL, 2.03 mmol) and trifluoromethanesulfonyl chloride (214 μL, 2.03 mmol) at 0°C. The reaction mixture was stirred at room temperature for 2 hr. Water was added to the reaction mixture, and the mixture was extracted with ethyl acetate. The organic layer was washed successively with water and saturated brine, dried over anhydrous sodium sulfate, and concentrated under reduced pressure. The obtained residue was purified by basic silica gel column chromatography [eluent: hexane-ethyl acetate (4:1-0:1)], and crystallized from hexane-diisopropyl ether to give the title compound (200 mg) as colorless crystals (yield 30%).
MS (ESI+): $[M+H]^+$ 501.
$^1H$ NMR (300 MHz, $CDCl_3$) δ 2.26-2.45 (5H, m), 3.19 (1H, quintet, J = 7.4 Hz), 3.60-3.98 (4H, m), 4.28 (2H, s), 7.38

(1H, s), 7.41-7.61 (4H, m).

Example 33

N-{4-methyl-2-[3-(trifluoromethyl)benzyl]-1,3-thiazol-5-yl}piperidine-4-carboxamide hydrochloride

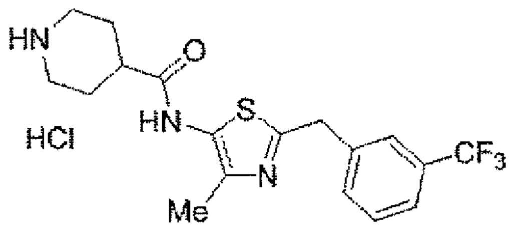

Example 33-A)

tert-butyl 4-({4-methyl-2-[3-(trifluoromethyl)benzyl]-1,3-thiazol-5-yl}carbamoyl)piperidine-1-carboxylate

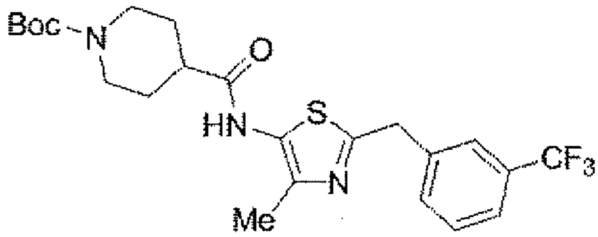

To a solution of tert-butyl {4-methyl-2-[3-(trifluoromethyl)benzyl]-1,3-thiazol-5-yl}carbamate (783 mg, 2.10 mmol) obtained in Example 19-D) in ethanol (3.0 mL) was added dropwise concentrated hydrochloric acid (4.0 mL), and the mixture was stirred at room temperature for 10 min. The reaction mixture was cooled to 0°C, neutralized with 8M aqueous sodium hydroxide solution (6 mL), adjusted to pH 9-10 with saturated aqueous sodium hydrogen carbonate solution, and extracted with ethyl acetate. The organic layer was washed with saturated brine, dried over anhydrous sodium sulfate, and concentrated under reduced pressure. The obtained residue was dissolved in DMA (9 mL), and 1-(tert-butoxycarbonyl)piperidine-4-carboxylic acid (578 mg, 2.52 mmol), HATU (958 mg, 2.52 mmol) and DIEA (183 μL, 1.05 mmol) were added at 0°C. The reaction mixture was stirred at 60°C for 2.5 hr, water was added and the mixture was extracted with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-0:1)] to give the title compound (424 mg) as a yellow oil (yield 42%).
MS ESI+): $[M+H]^+$ 484.
$^1H$ NMR (300 MHz, $CDCl_3$) δ 1.47 (9H, s), 1.65-1.81 (2H, m), 1.81-1.96 (2H, m), 2.34 (3H, s), 2.46 (1H, tt, J = 11.4, 3.8 Hz), 2.70-2.88 (2H, m), 4.14 (2H, t, J = 7.2 Hz), 4.26 (2H, s), 7.33-7.63 (5H, m).

Example 33-B)

N-{4-methyl-2-[3-(trifluoromethyl)benzyl]-1,3-thiazol-5-yl}piperidine-4-carboxamide hydrochloride

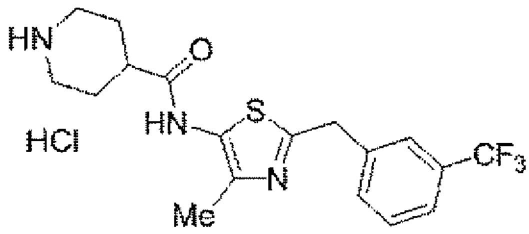

In the same manner as in Example 20, tert-butyl 4-({4-methyl-2-[3-(trifluoromethyl)benzyl]-1,3-thiazol-5-yl}carbamoyl)piperidine-1-carboxylate obtained in Example 33-A) was used to give the title compound (363 mg) (yield 100%). MS (ESI+): $[M+H]^+$ 383.
$^1$H NMR (300 MHz, DMSO-$d_6$) δ 1.67-2.00 (4H, m), 2.32 (3H, s), 2.77-2.97 (3H, m), 3.27 (2H, brs), 4.32 (2H, s), 7.52-7.66 (3H, m), 7.70 (1H, s), 8.65 (1H, brs), 8.97 (1H, brs), 10.64 (1H, s).

Example 34

1-(methylsulfonyl)-N-{4-methyl-2-[3-(trifluoromethyl)benzyl]-1,3-thiazol-5-yl}piperidine-3-carboxamide

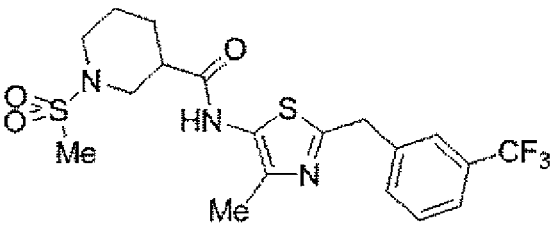

In the same manner as in Example 22, N-{4-methyl-2-[3-(trifluoromethyl)benzyl]-1,3-thiazol-5-yl}piperidine-3-carboxamide hydrochloride obtained in Example 29-B) was used to give the title compound (106 mg) (yield 64%). MS (ESI+): $[M+H]^+$ 461.
$^1$H NMR (300 MHz, $CDCl_3$) δ 1.69-2.13 (4H, m), 2.41 (3H, brs), 2.76 (1H, brs), 2.82 (3H, s), 3.16-3.39 (2H, m), 3.48 (2H, brs), 4.28 (2H, s), 7.39-7.66 (4H, m), 8.22 (1H, brs).

Example 35

1-acetyl-N-{4-methyl-2-[3-(trifluoromethyl)benzyl]-1,3-thiazol-5-yl}piperidine-3-carboxamide

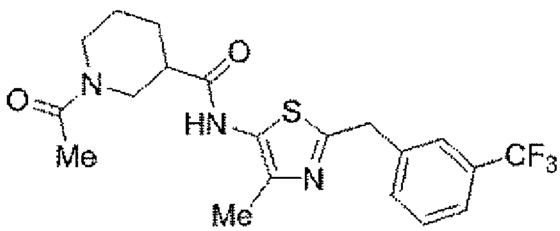

In the same manner as in Example 21, N-{4-methyl-2-[3-(trifluoromethyl)benzyl]-1,3-thiazol-5-yl}piperidine-3-carboxamide hydrochloride obtained in Example 29-B) was used to give the title compound (79 mg) (yield 52%). MS (ESI+): $[M+H]^+$ 426.
$^1$H NMR (300 MHz, $CDCl_3$) δ 1.52-1.61 (2H, m), 1.86 (1H, m), 2.13 (3H, s), 2.33-2.43 (1H, m), 2.45 (3H, s), 2.63-2.75 (1H, m), 3.39 (1H, ddd, J = 13.0, 8.6, 4.2 Hz), 3.48-3.65 (2H, m), 4.22 (1H, d, J = 5.3 Hz), 4.26 (2H, s), 7.39-7.64 (4H, m), 9.21 (1H, brs).

Example 36

N1-ethyl-N3-{4-methyl-2-[3-(trifluoromethyl)benzyl]-1,3-thiazol-5-yl}piperidine-1,3-dicarboxamide

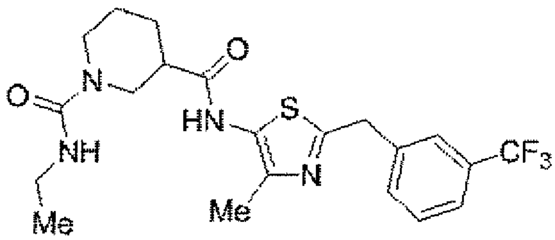

In the same manner as in Example 23, N-{4-methyl-2-[3-(trifluoromethyl)benzyl]-1,3-thiazol-5-yl}piperidine-3-carboxamide hydrochloride obtained in Example 29-B) was used to give the title compound (105 mg) (yield 65%).
MS (ESI+) : $[M+H]^+$ 455.
$^1H$ NMR (300 MHz, $CDCl_3$) δ 1.13 (3H, t, J = 7.2 Hz), 1.54 (2H, dq, J = 9.8, 4.9 Hz), 1.81 (1H, m), 2.26-2.40 (1H, m), 2.45 (3H, s), 2.62-2.73 (1H, m), 3.10-3.47 (5H, m), 4.13-4.30 (3H, m), 4.37-4.53 (1H, m), 7.37-7.45 (1H, m), 7.46-7.54 (2H, m), 7.56 (1H, s), 9.68 (1H, s).

Example 37

1-(methylsulfonyl)-N-{4-methyl-2-[3-(trifluoromethyl)benzyl]-1,3-thiazol-5-yl}piperidine-4-carboxamide

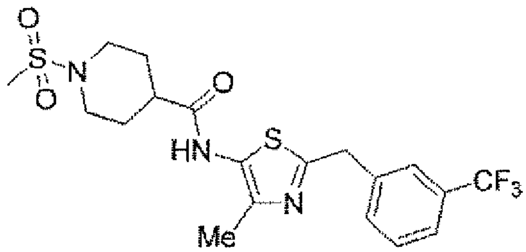

In the same manner as in Example 22, N-4-methyl-2-[3-(trifluoromethyl)benzyl]-1,3-thiazol-5-yl}piperidine-4-carboxamide hydrochloride obtained in Example 33-B) was used to give the title compound (101 mg) (yield 59%).
MS (ESI+): $[M+H]^+$ 461.
$^1H$ NMR (300 MHz, $CDCl_3$) δ 1.86-2.11 (4H, m), 2.35 (3H, s), 2.42-2.57 (1H, m), 2.82 (3H, s), 2.85-2.99 (2H, m), 3.81 (2H, dt, J =12.3, 3.9 Hz), 4.27 (2H, s), 7.31 (1H, s), 7.40-7.60 (4H, m).

Example 38

N1-ethyl-N4-{4-methyl-2-[3-(trifluoromethyl)benzyl]-1,3-thiazol-5-yl}piperidine-1,4-dicarboxamide

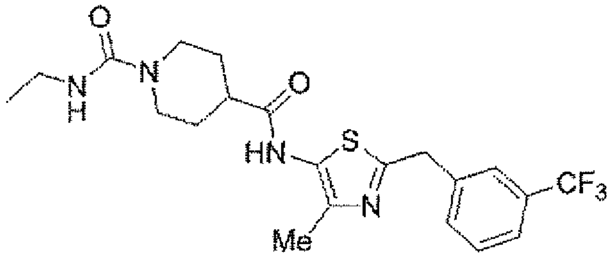

In the same manner as in Example 23, N-{4-methyl-2-[3-(trifluoromethyl)benzyl]-1,3-thiazol-5-yl}piperidine-4-carboxamide hydrochloride obtained in Example 33-B) was used to give the title compound (83 mg) (yield 49%).
MS (ESI+): $[M+H]^+$ 455.
$^1H$ NMR (300 MHz, DMSO-$d_6$) δ 0.99 (3H, t, J = 7.2 Hz), 1.32-1.55 (2H, m), 1.69 (2H, d, J = 10.6 Hz), 2.28 (3H, s), 2.58-2.76 (3H, m), 2.96-3.09 (2H, m), 3.97 (2H, d, J = 13.3 Hz), 4.27 (2H, s), 6.43 (1H, t, J = 5.3 Hz), 7.43-7.73 (4H, m), 10.27 (1H, brs).

Example 39

1-cyano-N-{4-methyl-2-[3-(trifluoromethyl)benzyl]-1,3-thiazol-5-yl}cyclopropanecarboxamide

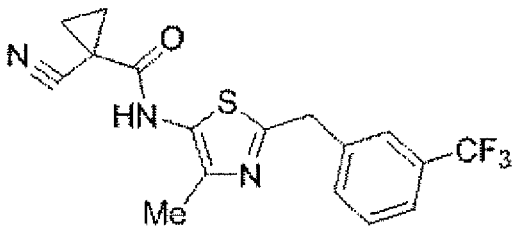

Example 39-A)

4-methyl-2-[3-(trifluoromethyl)benzyl]-1,3-thiazole-5-amine

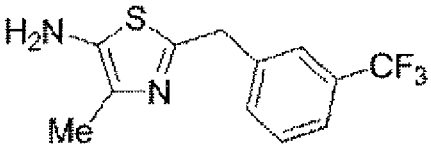

To a solution of tert-butyl {4-methyl-2-[3-(trifluoromethyl)benzyl]-1,3-thiazol-5-yl}carbamate (5.96 g, 16.0 mmol) obtained in Example 19-D) in ethanol (5 mL) was added dropwise concentrated hydrochloric acid (10 mL), and the mixture was stirred at room temperature for 10 min. The reaction mixture was cooled to 0°C, neutralized with 8M aqueous sodium hydroxide solution (15 mL), adjusted to pH 9-10 with saturated aqueous sodium hydrogen carbonate solution, and extracted with ethyl acetate. The organic layer was washed with saturated brine, dried over anhydrous sodium sulfate and concentrated under reduced pressure to give the title compound (4.30 g) as a brown oil (yield 99%).
MS (ESI+): $[M+H]^+$ 272.
$^1H$ NMR (300 MHz, $CDCl_3$) δ 2.25 (3H, s), 3.25 (2H, brs), 4.20 (2H, s), 7.38-7.61 (4H, m).

Example 39-B)

1-cyano-N-{4-methyl-2-[3-(trifluoromethyl)benzyl]-1,3-thiazol-5-yl}cyclopropanecarboxamide

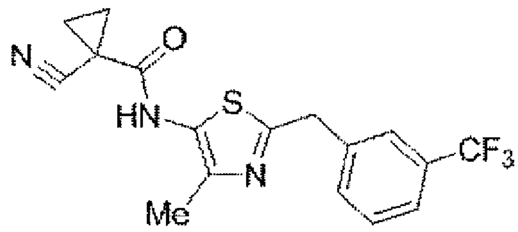

A solution (0.16M; 500 μl, 80 μmol) of 4-methyl-2-[3-(trifluoromethyl)benzyl]-1,3-thiazole-5-amine obtained in Example 39-A) in DMF, a solution (0.192M; 500 μl, 96 μmol) of 1-cyanocyclopropanecarboxylic acid in DMF, a solution (0.192M; 500 μl, 96 μmol) of HATU in DMF, and a solution (0.384M; 500 μl, 192 μmol) of DIEA in DMF were mixed at room temperature, and the mixture was stirred at 60°C for 24 hr. The reaction mixture was cooled to room temperature, ethyl acetate (3.5 mL) and 2% aqueous sodium hydrogen carbonate solution (1.0 mL) were added and the mixture was extracted. The organic layer was separated by upper layer PhaseSeptube (manufactured by Wako Pure Chemical Industries, Ltd.). The solvent was evaporated under reduced pressure, and the residue was dissolved in DMSO-methanol (1:1) (1 mL) and purified by preparative HPLC to give the title compound (13.4 mg). MS(ESI+): $[M+H]^+$ 365.

The Example compounds (Examples 40-144) produced according to the above-mentioned methods are shown in the following Tables. In the Tables, MS shows measured values.

[Table 1-1]

| Ex. | IUPAC name | structure | salt | MS |
|---|---|---|---|---|
| 40 | 2-methyl-N-{4-methyl-2-[3-(trifluoromethyl)-benzyl]-1,3-thiazol-5-yl}furan-3-carboxamide | 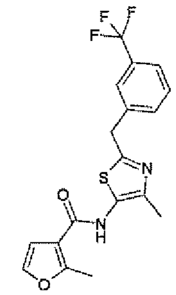 | | 380 |
| 41 | 1-methyl-N-{4-methyl-2-[3-(trifluoromethyl)-benzyl]-1,3-thiazol-5-yl}-1H-imidazole-2-carboxamide | 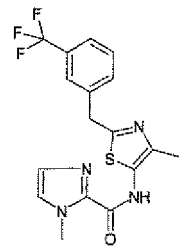 | trifluoroacetate | 380 |
| 42 | 1-methyl-N-{4-methyl-2-[3-(trifluoromethyl)-benzyl]-1,3-thiazol-5-yl}-1H-imidazole-4-carboxamide | 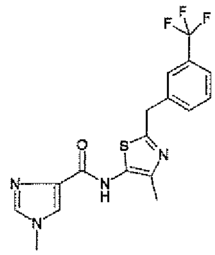 | trifluoroacetate | 380 |

(continued)

| Ex. | IUPAC name | structure | salt | MS |
|---|---|---|---|---|
| 43 | 1-methyl-N-{4-methyl-2-[3-(trifluoromethyl)-benzyl]-1,3-thiazol-5-yl}-1H-imidazole-5-carboxamide | 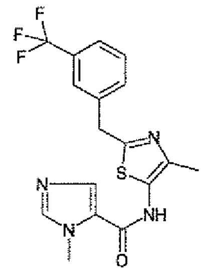 | trifluoroacetate | 380 |
| 44 | 3-methyl-N-{4-methyl-2-[3-(trifluoromethyl)-benzyl]-1,3-thiazol-5-yl}isoxazole-5-carboxamide | 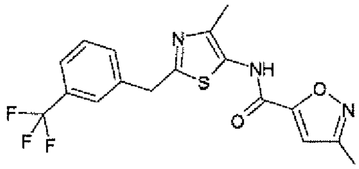 | | 381 |

[Table 1-2]

| Ex. | IUPAC name | structure | salt | MS |
|---|---|---|---|---|
| 45 | 2-fluoro-N-{4-methyl-2-[3-(trifluoromethyl)-benzyl]-1,3-thiazol-5-yl}pyridine-4-carboxamide | 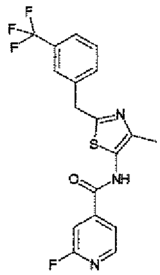 | trifluoroacetate | 395 |
| 46 | N-{4-methyl-2-[3-(trifluoromethyl)-benzyl]-1,3-thiazol-5-yl}-6-oxo-1,4,5,6-tetrahydropyridazine-3-carboxamide | 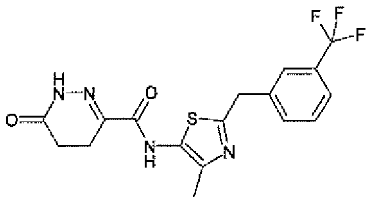 | | 396 |
| 47 | 1,3,5-trimethyl-N-{4-methyl-2-[3-(trifluoromethyl)-benzyl]-1,3-thiazol-5-yl}-1H-pyrazole-4-carboxamide | 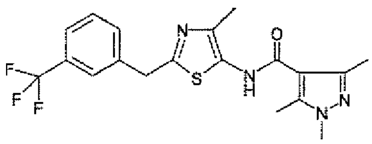 | trifluoroacetate | 408 |
| 48 | 2-(2,5-dimethyl-1,3-thiazol-4-yl)-N-{4-methyl-2-[3-(trifluoromethyl)-benzyl]-1,3-thiazol-5-yl}acetamide | 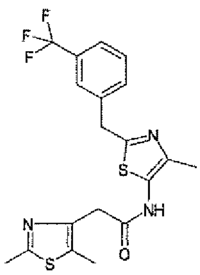 | | 425 |

(continued)

| Ex. | IUPAC name | structure | salt | MS |
|---|---|---|---|---|
| 49 | N-4-{4-methyl-2-[3-(trifluoromethyl)-benzyl]-1,3-thiazol-5-yl}piperidine-1,4-dicarboxamide | 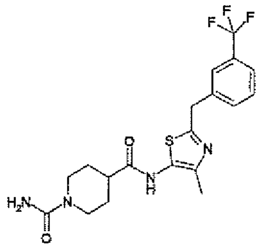 | | 426 |
| 50 | 4-methyl-N-{4-methyl-2-[3-(trifluoromethyl)-benzyl]-1,3-thiazol-5-yl}-3-oxo-3,4-dihydro-2H-1,4-thiazine-5-carboxamide | 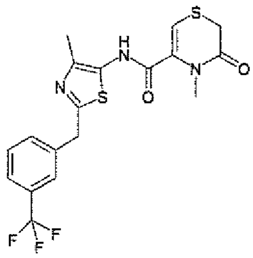 | | 427 |

[Table 1-3]

| Ex. | IUPAC name | structure | salt | MS |
|---|---|---|---|---|
| 51 | 5-chloro-1,3-dimethyl-N-{4-methyl-2-[3-(trifluoromethyl)-benzyl]-1,3-thiazol-5-yl}-1H-pyrazole-4-carboxamide | 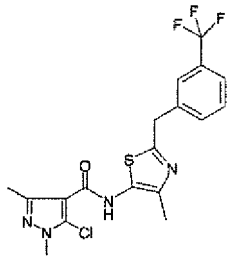 | trifluoroacetate | 428 |
| 52 | 2-methyl-N-{4-methyl-2-[3-(trifluoromethyl)-benzyl]-1,3-thiazol-5-yl}-2H-indazole-3-carboxamide | 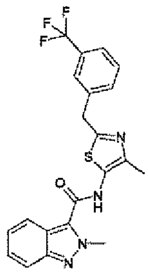 | trifluoroacetate | 430 |
| 53 | 1-cyclopropyl-2,5-dimethyl-N-{4-methyl-2-[3-(trifluoromethyl)-benzyl]-1,3-thiazol-5-yl}-1H-pyrrole-3-carboxamide | 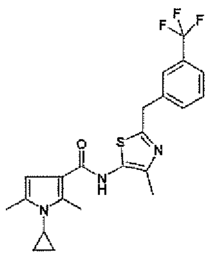 | trifluoroacetate | 433 |

(continued)

| Ex. | IUPAC name | structure | salt | MS |
|---|---|---|---|---|
| 54 | methyl 4-({4-methyl-2-[3-(trifluoromethyl)-benzyl]-1,3-thiazol-5-yl}carbamoyl)-piperidine-1-carboxylate | 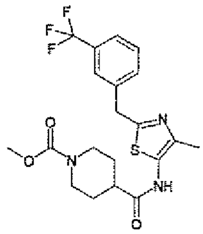 | | 441 |
| 55 | 3-furan-2-yl-1-methyl-N-{4-methyl-2-[3-(trifluoromethyl)-benzyl]-1,3-thiazol-5-yl}-1H-pyrazole-5-carboxamide | 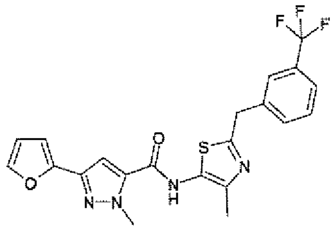 | trifluoroacetate | 446 |
| 56 | N-{4-methyl-2-[3-(trifluoromethyl)-benzyl]-1,3-thiazol-5-yl}cyclopropane-carboxamide | 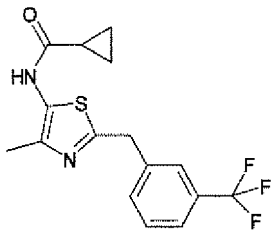 | | 340 |

[Table 1-4]

| Ex. | IUPAC name | structure | salt | MS |
|---|---|---|---|---|
| 57 | N-{4-methyl-2-[3-(trifluoromethyl)-benzyl]-1,3-thiazol-5-yl}cyclopentane-carboxamide | 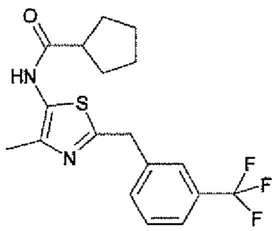 | | 368 |
| 58 | N-{4-methyl-2-[3-(trifluoromethyl)-benzyl]-1,3-thiazol-5-yl}tetrahydro-furan-2-carboxamide | 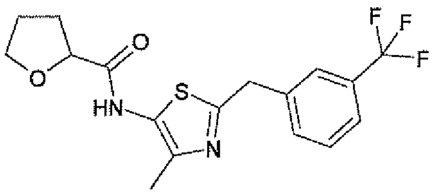 | | 370 |
| 59 | N-{4-methyl-2-[3-(trifluoromethyl)-benzyl]-1,3-thiazol-5-yl}-5-oxo-L-prolinamide | 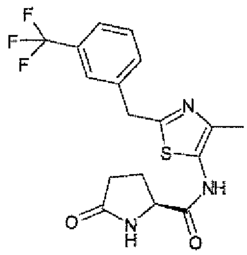 | | 383 |

(continued)

| Ex. | IUPAC name | structure | salt | MS |
|---|---|---|---|---|
| 60 | 2-(2,5-dioxoimidazolidin-4-yl)-N-{4-methyl-2-[3-(trifluoromethyl)-benzyl]-1,3-thiazol-5-yl}acetamide | 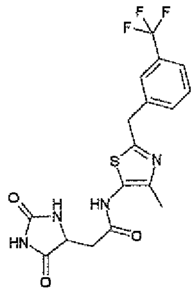 | | 412 |
| 61 | N-{4-methyl-2-[3-(trifluoromethyl)-benzyl]-1,3-thiazol-5-yl}tetrahydrofuran-3-carboxamide | 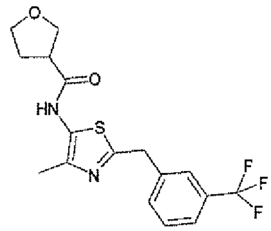 | | 370 |
| 62 | 2-(6-methoxy-3-oxo-2,3-dihydro-1H-inden-1-yl)-N-{4-methyl-2-[3-(trifluoromethyl)-benzyl]-1,3-thiazol-5-yl}acetamide | 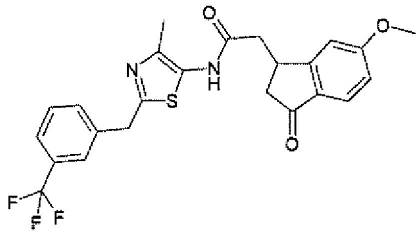 | | 474 |

[Table 1-5]

| Ex. | IUPAC name | structure | salt | MS |
|---|---|---|---|---|
| 63 | 4-(acetylamino)-N-{4-methyl-2-[3-(trifluoromethyl)-benzyl]-1,3-thiazol-5-yl}benzamide | 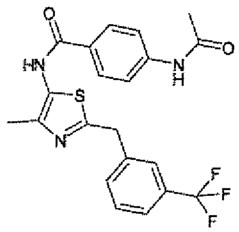 | | 433 |
| 64 | 3-(acetylamino)-N-{4-methyl-2-[3-(trifluoromethyl)-benzyl]-1,3-thiazol-5-yl}benzamide | 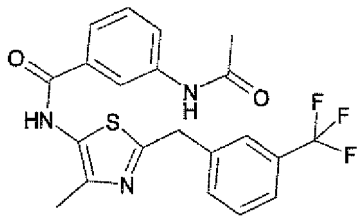 | | 433 |
| 65 | N-{4-methyl-2-[3-(trifluoromethyl)-benzyl]-1,3-thiazol-5-yl}-4-sulfamoylbenzamide | 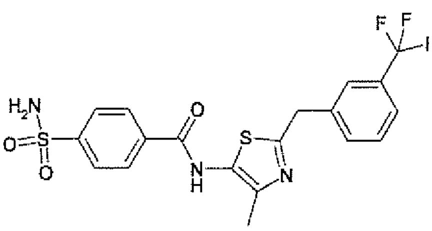 | | 455 |

(continued)

| Ex. | IUPAC name | structure | salt | MS |
|---|---|---|---|---|
| 66 | 4-chloro-N-{4-methyl-2-[3-(trifluoromethyl)-benzyl]-1,3-thiazol-5-yl}-3-sulfamoylbenzamide | 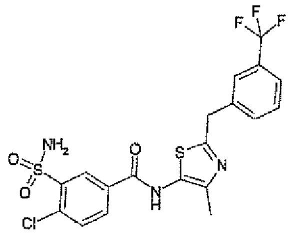 | | 489 |
| 67 | N-{4-methyl-2-[3-(trifluoromethyl)-benzyl]-1,3-thiazol-5-yl}-1,3-benzothiazole-6-carboxamide | 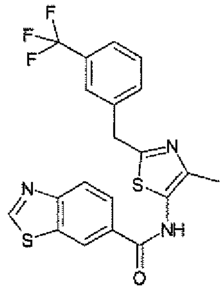 | | 433 |
| 68 | 1-methyl-N-{4-methyl-2-[3-(trifluoromethyl)-benzyl]-1,3-thiazol-5-yl}-1H-indole-2-carboxamide | 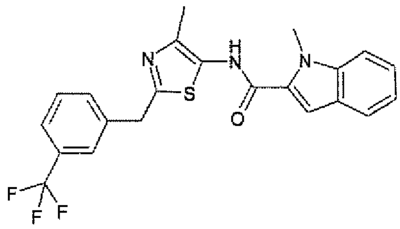 | | 429 |

[Table 1-6]

| Ex. | IUPAC name | structure | salt | MS |
|---|---|---|---|---|
| 69 | N-{4-methyl-2-[3-(trifluoromethyl)-benzyl]-1,3-thiazol-5-yl}-1H-imidazole-4-carboxamide | 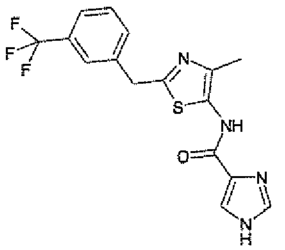 | trifluoroacetate | 366 |
| 70 | N-{4-methyl-2-[3-(trifluoromethyl)-benzyl]-1,3-thiazol-5-yl}pyridine-3-carboxamide | 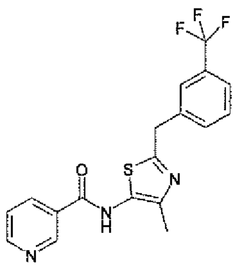 | trifluoroacetate | 377 |

(continued)

| Ex. | IUPAC name | structure | salt | MS |
|---|---|---|---|---|
| 71 | N-{4-methyl-2-[3-(trifluoromethyl)-benzyl]-1,3-thiazol-5-yl}pyridine-4-carboxamide | 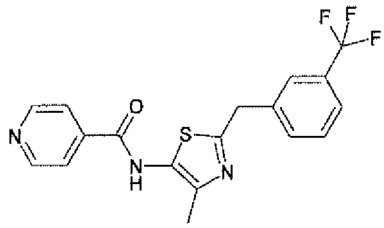 | trifluoroacetate | 377 |
| 72 | N-{4-methyl-2-[3-(trifluoromethyl)-benzyl]-1,3-thiazol-5-yl}pyrazine-2-carboxamide | 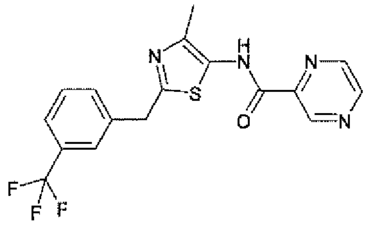 | | 378 |
| 73 | (2E)-3-(1H-imidazol-4-yl)-N-{4-methyl-2-[3-(trifluoromethyl)-benzyl)]-1,3-thiazol-5-yl}prop-2-enamide | 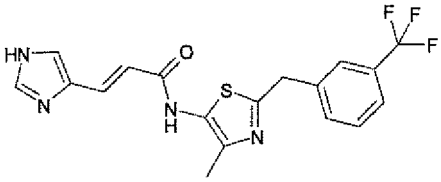 | trifluoroacetate | 392 |
| 74 | N-{4-methyl-2-[3-(trifluoromethyl)-benzyl)-1,3-thiazol-5-yl}-3-pyridin-3-ylpropanamide | 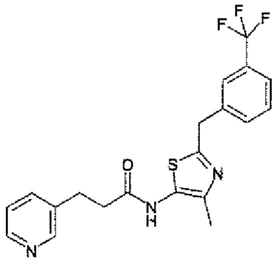 | trifluoroacetate | 405 |

[Table 1-71

| Ex. | IUPAC name | structure | salt | MS |
|---|---|---|---|---|
| 75 | 6-chloro-N-{4-methyl-2-[3-(trifluoromethyl)-benzyl]-1,3-thiazol-5-yl}pyridine-3-carboxamide | 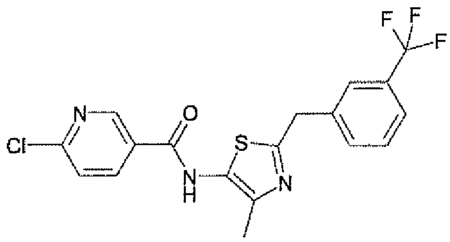 | trifluoroacetate | 411 |
| 76 | 2-(methylsulfanyl)-N-{4-methyl-2-[3-(trifluoromethyl)-benzyl]-1,3-thiazol-5-yl}pyridine-3-carboxamide | 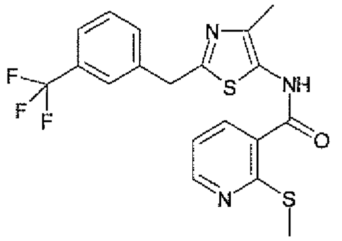 | trifluoroacetate | 423 |

(continued)

| Ex. | IUPAC name | structure | salt | MS |
|---|---|---|---|---|
| 77 | N-{4-methyl-2-[3-(trifluoromethyl)-benzyl]-1,3-thiazol-5-yl}-2-pyridin-3-ylacetamide | 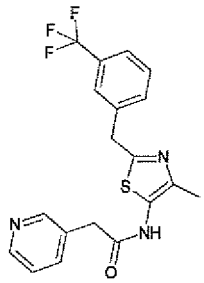 | trifluoroacetate | 391 |
| 78 | 3-methyl-N-{4-methyl-2-[3-(trifluoromethyl)-benzyl]-1,3-thiazol-5-yl)pyridine-2-carboxamide | 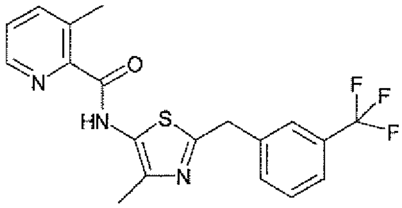 | trifluoroacetate | 391 |
| 79 | 1,3-dimethyl-N-{4-methyl-2-[3-(trifluoromethyl)-benzyl]-1,3-thiazol-5-yl}-1H-thieno[2,3-c]pyrazole-5-carboxamide | 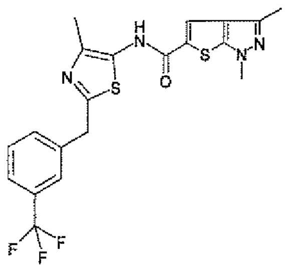 | | 450 |
| 80 | 4-benzyl-N-{4-methyl-2-[3-(trifluoromethyl)-benzyl]-1,3-thiazol-5-yl}morpholine-2-carboxamide | 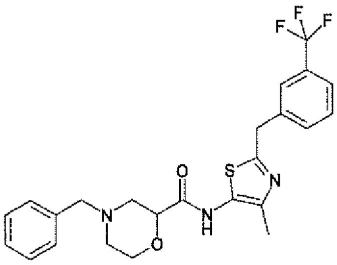 | trifluoroacetate | 475 |

[Table 1-8]

| Ex. | IUPAC name | structure | salt | MS |
|---|---|---|---|---|
| 81 | 2-methyl-N-{4-methyl-2-[3-(trifluoromethyl)-benzyl]-1,3-thiazol-5-yl}-5-(piperidin-1-ylsulfonyl) furan-3-carboxamide | 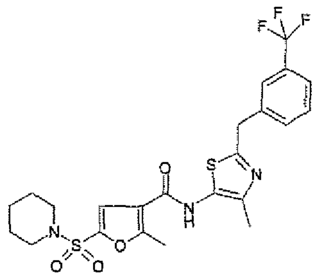 | | 527 |

(continued)

| Ex. | IUPAC name | structure | salt | MS |
|---|---|---|---|---|
| 82 | N-{4-methyl-2-[3-(trifluoromethyl)-benzyl]-1,3-thiazol-5-yl}-5-oxo-1-(thiophen-2-ylmethyl)pyrrolidine-3-carboxamide | 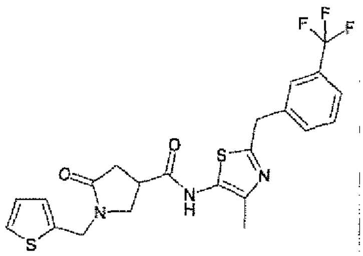 | | 479 |
| 83 | N-{4-methyl-2-[3-(trifluoromethyl)-benzyl]-1,3-thiazol-5-yl}-1-phenyl-5-propyl-1H-pyrazole-4-carboxamide | 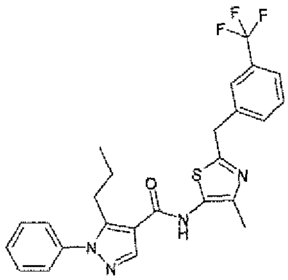 | trifluoroacetate | 484 |
| 84 | N-{4-methyl-2-[3-(trifluoromethyl)-benzyl]-1,3-thiazol-5-yl}-3-(2-oxopyrrolidin-1-yl) benzamide | 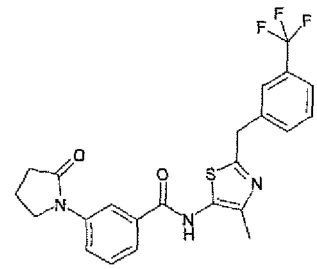 | | 459 |
| 85 | 4-(1,1-dioxido-1,2-thiazinan-2-yl)-N-{4-methyl-2-[3-(trifluoromethyl)-benzyl]-1,3-thiazol-5-yl}benzamide | 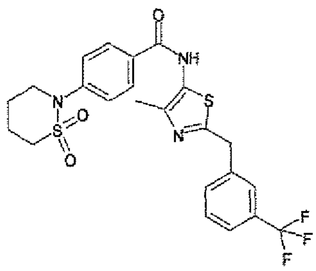 | | 509 |
| 86 | 3-(3,5-dimethylisoxazol-4-yl)-N-{4-methyl-2-[3-(trifluoromethyl)-benzyl]-1,3-thiazol-5-yl}propanamide | 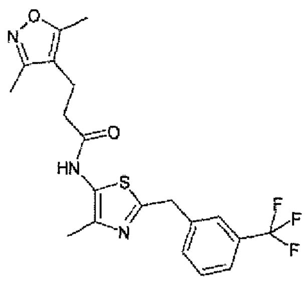 | | 423 |

[Table 1-9]

| Ex. | IUPAC name | structure | salt | MS |
|---|---|---|---|---|
| 87 | 1-[(4-chlorophenyl)-sulfonyl]-N-{4-methyl-2-[3-(trifluoromethyl)-benzyl]-1,3-thiazol-5-yl}cyclopropane-carboxamide | 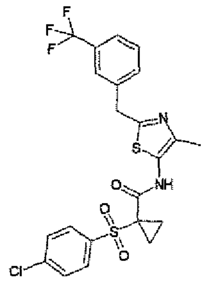 | | 514 |
| 88 | 1-[(3,5-dimethylisoxazol-4-yl) sulfonyl]-N-{4-methyl-2-[3-(trifluoromethyl)-benzyl]-1,3-thiazol-5-yl}piperidine-4-carboxamide | 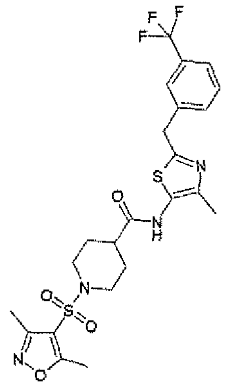 | | 542 |
| 89 | 2-(1,1-dioxidothiomorpholin-4-yl)-3-methyl-N-{4-methyl-2-[3-(trifluoromethyl)-benzyl]-1,3-thiazol-5-yl}butanamide | 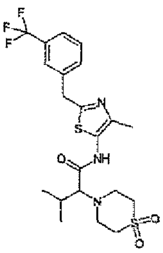 | trifluoroacetate | 489 |
| 90 | 1-[(4-fluorophenyl)-sulfonyl]-N-{4-methyl-2-[3-(trifluoromethyl)-benzyl]-1,3-thiazol-5-yl}piperidine-3-carboxamide | 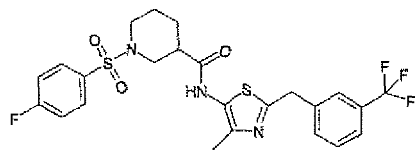 | | 541 |
| 91 | N-{4-methyl-2-[3-(trifluoromethyl)-benzyl]-2,3-thiazol-5-yl}-1,2,3-thiadiazole-4-carboxamide | 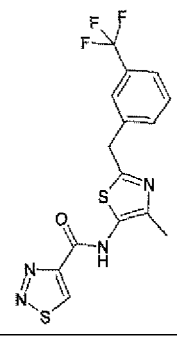 | | 384 |
| 92 | 4-methyl-N-{4-methyl-2-[3-(trifluoromethyl)-benzyl]-1,3-thiazol-5-yl}-1,2,3-thiadiazole-5-carboxamide | 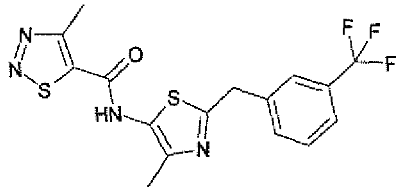 | | 398 |

[Table 1-10]

| Ex. | IUPAC name | structure | salt | MS |
|---|---|---|---|---|
| 93 | 5-chloro-1-methyl-N-{4-methyl-2-[3-(trifluoromethyl)-benzyl]-1,3-thiazol-5-yl}-3-(trifluoromethyl)-1H-pyrazole-4-carboxamide | 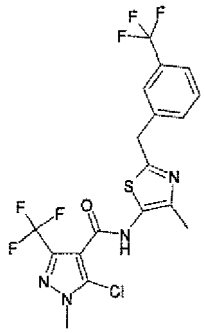 | trifluoroacetate | 482 |
| 94 | 1-benzyl-N-{4-methyl-2-[3-(trifluoromethyl)-benzyl]-1,3-thiazol-5-yl}-6-oxo-1,6-dihydropyridine-3-carboxamide | 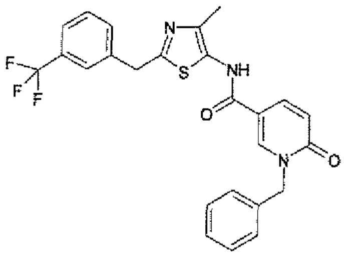 | | 483 |
| 95 | 5-(dimethylsulfamoyl)-2-methyl-N-{4-methyl-2-[3-(trifluoromethyl)-benzyl]-1,3-thiazol-5-yl}furan-3-carboxamide | 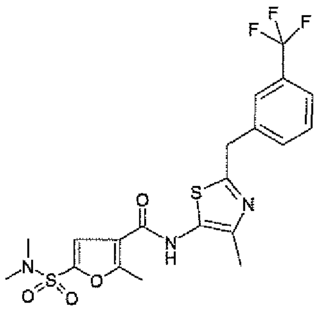 | | 487 |
| 96 | 5-(3-chlorophenoxy)-1,3-dimethyl-N-{4-methyl-2-[3-(trifluoromethyl)-benzyl]-1,3-thiazol-5-yl}-1H-pyrazole-4-carboxamide | 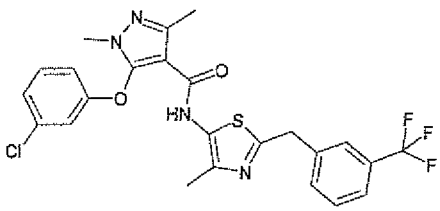 | trifluoroacetate | 520 |
| 97 | N-{4-methyl-2-[3-(trifluoromethyl)-benzyl]-1,3-thiazol-5-yl}-6-(1H-pyrazol-1-yl)pyridine-3-carboxamide | 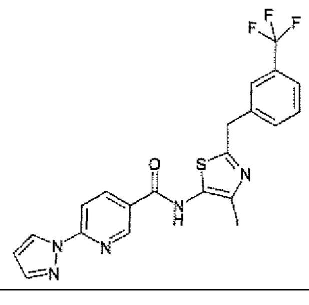 | trifluoroacetate | 443 |
| 98 | 4-methyl-N-{4-methyl-2-[3-(trifluoromethyl)-benzyl]-1,3-thiazol-5-yl}-2-phenyl-1,3-thiazole-5-carboxamide | 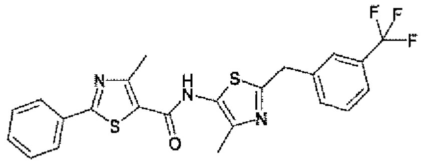 | | 473 |

[Table 1-11]

| Ex. | IUPAC name | structure | salt | MS |
|---|---|---|---|---|
| 99 | N-{4-methyl-2-[3-(trifluoromethyl)-benzyl]-2,3-thiazol-5-yl}-6-oxo-1-[4-(trifluoromethyl)-benzyl]-1,6-dihydropyridine-3-carboxamide | 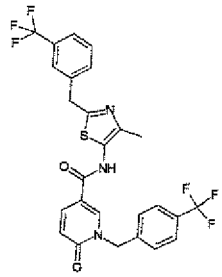 | | 551 |
| 100 | 4-methyl-N-{4-methyl-2-[3-(trifluoromethyl)-benzyl]-1,3-thiazol-5-yl}-1,2,5-oxadiazole-3-carboxamide | 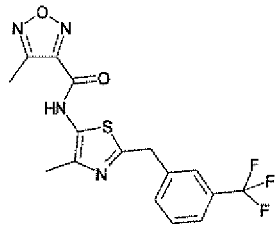 | | 382 |
| 101 | 2-(2,4-dioxo-1,3-thiazolidin-3-yl)-N-{4-methyl-2-[3-(trifluoromethyl)-benzyl]-1,3-thiazol-5-yl}acetamide | 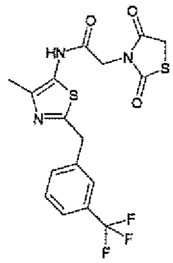 | | 429 |
| 102 | 2-(acetylamino)-N-{4-methyl-2-[3-(trifluoromethyl)-benzyl]-1,3-thiazol-5-yl}-1,3-thiazole-4-carboxamide | 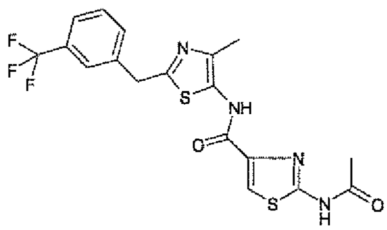 | | 440 |
| 103 | N-{4-methyl-2-[3-(trifluoromethyl)-benzyl]-1,3-thiazol-5-yl}-2-(morpholin-4-ylmethyl)furan-3-carboxamide | 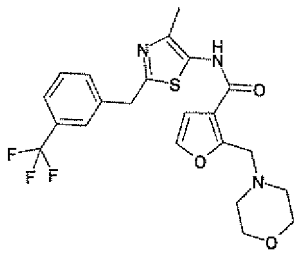 | trifluoroacetate | 465 |
| 104 | 1-[4-(dimethylamino)-phenyl]-N-{4-methyl-2-[3-(trifluoromethyl)-benzyl]-1,3-thiazol-5-yl}-5-oxopyrrolidine-3-carboxamide | 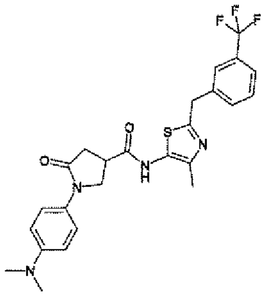 | | 502 |

[Table 1-12]

| Ex. | IUPAC name | structure | salt | MS |
|---|---|---|---|---|
| 105 | N-{4-methyl-2-[3-(trifluoromethyl)-benzyl]-1,3-thiazol-5-yl}-1-(phenylsulfonyl)-prolinamide | 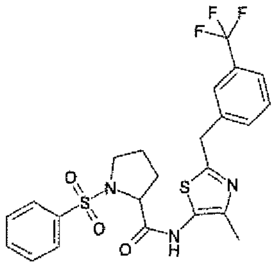 | | 509 |
| 106 | N-{4-methyl-2-[3-(trifluoromethyl)-benzyl]-1,3-thiazol-5-yl}-4-[(tetrahydrofuran-2-ylmethyl)sulfamoyl]-benzamide | 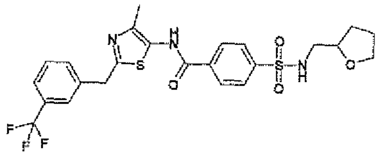 | | 539 |
| 107 | 5-[(3,5-dimethyl-1H-pyrazol-1-yl) methyl]-N-{4-methyl-2-[3-(trifluoromethyl)-benzyl]-1,3-thiazol-5-yl}furan-2-carboxamide | 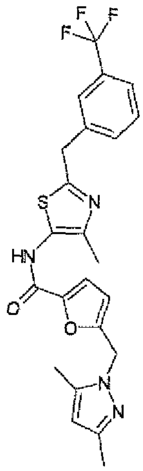 | trifluoroacetate | 474 |
| 108 | 5-[(3-methoxyphenoxy)-methyl]-N-{4-methyl-2-[3-(trifluoromethyl)-benzyl]-1,3-thiazol-5-yl}furan-2-carboxamide | 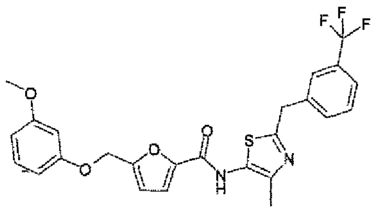 | | 502 |
| 109 | 2-[4-(1-methylethyl)piperazin-1-yl]-N-{4-methyl-2-[3-(trifluoromethyl)-benzyl]-1,3-thiazol-5-yl}acetamide | 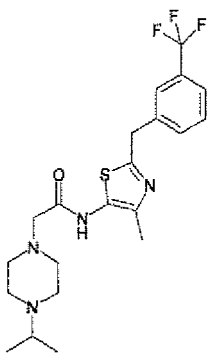 | trifluoroacetate | 440 |

[Table 1-13]

| Ex. | IUPAC name | structure | salt | MS |
|---|---|---|---|---|
| 110 | 1-methyl-N-{4-methyl-2-[3-(trifluoromethyl)-benzyl]-1,3-thiazol-5-yl}-1H-pyrazole-5-carboxamide | 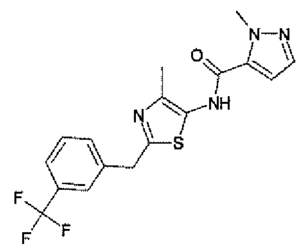 | trifluoroacetate | 380 |
| 111 | 1-methyl-N-{4-methyl-2-[3-(trifluoromethyl)-(trifluoromethyl)-benzyl]-1,3-thiazol-5-yl}-1H-pyrazole-3-carboxamide | 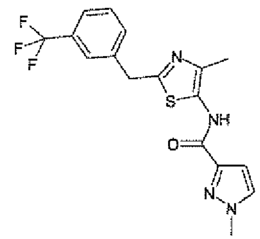 | trifluoroacetate | 380 |
| 112 | N-{4-methyl-2-[3-(trifluoromethyl)-benzyl]-1,3-thiazol-5-yl}-2-oxo-2,3-dihydropyridine-4-carboxamide | 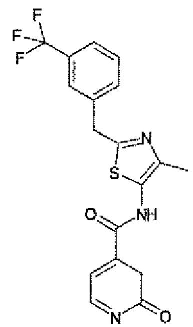 | | 393 |

(continued)

| Ex. | IUPAC name | structure | salt | MS |
|---|---|---|---|---|
| 113 . | 1-methyl-N-{4-methyl-2-[3-(trifluoromethyl)-benzyl]-1,3-thiazol-5- yl}-2-oxo-1,2-dihydropyridine-4-carboxamide | 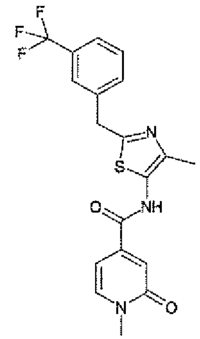 | | 407 |
| 114 | 2-(3-fluorophenyl)-N-{4-methyl-2-[3-(trifluoromethyl)-benzyl]-1,3-thiazol-5-yl}acetamide | 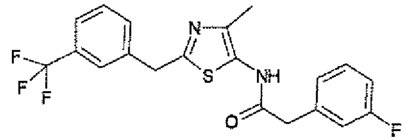 | | 408 |
| 115 | N-{4-methyl-2-[3-(trifluoromethyl)-benzyl]-1,3-thiazol-5-yl}-3,4-dihydro-2H-1,5-benzodioxepine-7-carboxamide | 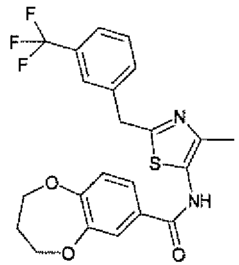 | | 448 |

[Table 1-14]

| Ex. | IUPAC name | structure | salt | MS |
|---|---|---|---|---|
| 16 | 2,5-difluoro-N-{4-methyl-2-[3-(trifluoromethyl)-benzyl]-1,3-thiazol-5yl}benzamide | 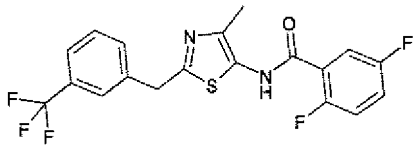 | | 412 |
| 117 | 3-bromo-N-{4-methyl-2-[3-(trifluoromethyl)-benzyl]-1,3-thiazol-5-yl}benzamide | 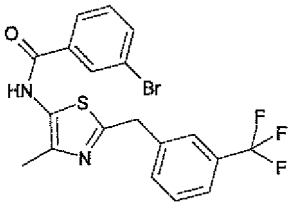 | | 454 |
| 118 | N-{4-methyl-2-[3-(trifluoromethyl)-benzyl]-1,3-thiazol-5-yl}-3-piperidin-1-ylpropanamide | 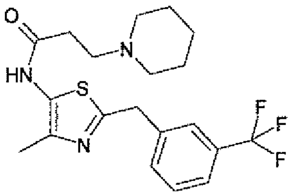 | trifluoroacetate | 411 |
| 119 | 4-(dimethylamino)-N-{4-methyl-2-[3-(trifluoromethyl)-benzyl]-1,3-thiazol-5-yl}benzamide | 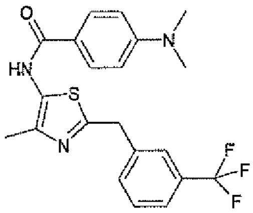 | trifluoroacetate | 419 |
| 120 | N-{4-methyl-2-[3-(trifluoromethyl)-benzyl]-1,3-thiazol-5-yl}-2-pyridin-4-ylacetamide | 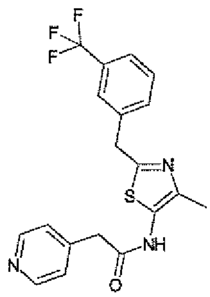 | trifluoroacetate | 391 |
| 121 | N-{4-methyl-2-[3-(trifluoromethyl)-benzyl]-1,3-thiazol-5-yl}-6-piperidin-1-ylpyridine-3-carboxamide | 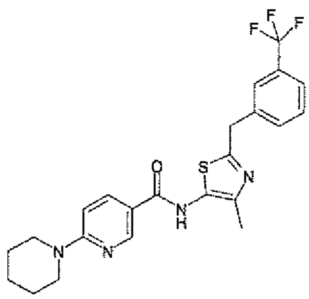 | trifluoroacetate | 460 |

(continued)

| Ex. | IUPAC name | structure | salt | MS |
|---|---|---|---|---|
| 122 | N-{4-methyl-2-[3-(trifluoromethyl)-benzyl]-1,3-thiazol-5-yl}-6-(2-pyrrolidin-1-ylethyl)pyridine-3-carboxamide | 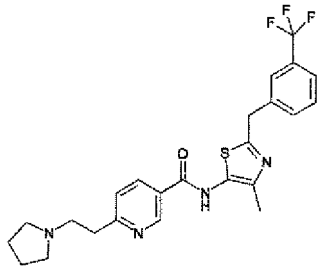 | trifluoroacetate | 474 |

[Table 1-15] Ex. IUPAC name structure salt MS

| | | | | |
|---|---|---|---|---|
| 123 | N-{4-methyl-2-[3-(trifluoromethyl)-benzyl]-1,3-thiazol-5-yl}-5-thiophen-2-ylpyridine-3-carboxamide | 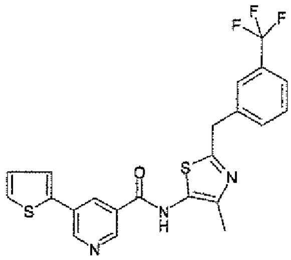 | trifluoroacetate | 459.1 |
| 124 | 2-(1H-indol-3-yl)-N-{4-methyl-2-[3-(trifluoromethyl)-benzyl]-1,3-thiazol-5-yl}acetamide | 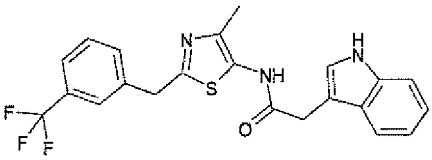 | | 429.1 |
| 125 | 3-(1H-benzimidazol-1-yl)-N-{4-methyl-2-[3-(trifluoromethyl)-benzyl]-1,3-thiazol-5-yl}propanamide | 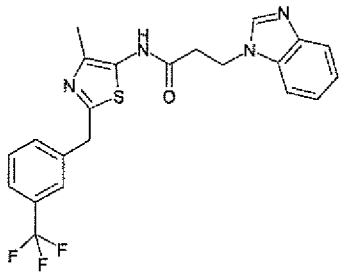 | trifluoroacetate | 444.1 |
| 126 | N-{4-methyl-2-[3-(trifluoromethyl)-benzyl]-1,3-thiazol-5-yl}-1H-benzimidazole-5-carboxamide | 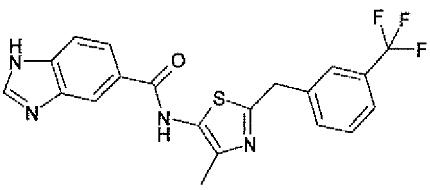 | trifluoroacetate | 416.1 |
| 127 | N-{4-methyl-2-[3-(trifluoromethyl)-benzyl]-1,3-thiazol-5-yl}[1,2,4]triazolo-[1,5-a]pyrimidine-2-carboxamide | 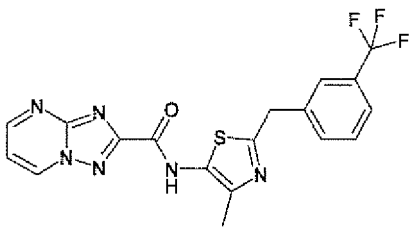 | | 418.1 |

(continued)

| | | | | |
|---|---|---|---|---|
| 128 | (2E)-N-{4-methyl-2-[3-(trifluoromethyl)-benzyl]-1,3-thiazol-5-yl}-3-phenylprop-2-enamide | 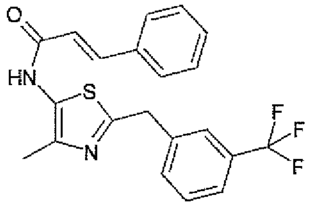 | | 402.1 |
| 129 | (2E)-3-(1,3-benzodioxol-5-yl)-N-{4-methyl-2-[3-(trifluoromethyl)-benzyl]-1,3-thiazol-5-yl}prop-2-enamide | 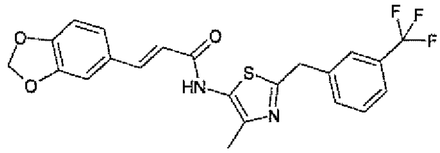 | | 446.1 |

[Table 1-16]

| Ex. | IUPAC name | structure | salt | MS |
|---|---|---|---|---|
| 130 | N-{4-methyl-2-[3-(trifluoromethyl)-benzyl]-1,3-thiazol-5-yl}-2-(3-oxo-3,4-dihydro-2H-1,4-benzothiazin-2-yl)acetamide | 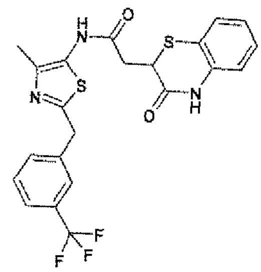 | | 477 |
| 131 | 3-(3,4-dihydroquinolin-1(2H)-yl)-N-{4-methyl-2-[3-(trifluoromethyl)-benzyl]-1,3-thiazol-5-yl}propanamide | 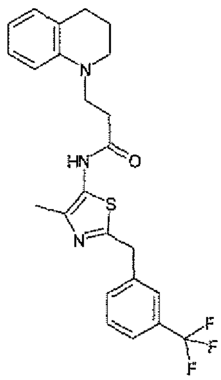 | trifluoroacetate | 459 |
| 132 | N-{4-methyl-2-[3-(trifluoromethyl)-benzyl]-1,3-thiazol-5-yl}furan-2-carboxamide | 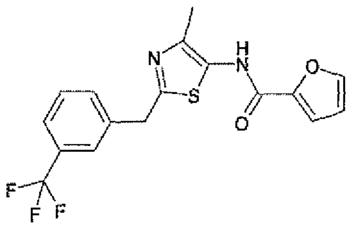 | | 366 |
| 133 | N-{4-methyl-2-[3-(trifluoromethyl)-benzyl]-1,3-thiazol-5-yl}-4-oxo-4H-chromene-2-carboxamide | 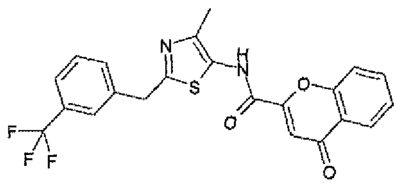 | | 444 |

(continued)

| Ex. | IUPAC name | structure | salt | MS |
|---|---|---|---|---|
| 134 | N-{4-methyl-2-[3-(trifluoromethyl)-benzyl]-1,3-thiazol-5-yl}-5-phenyl-1,3-oxazole-4-carboxamide | 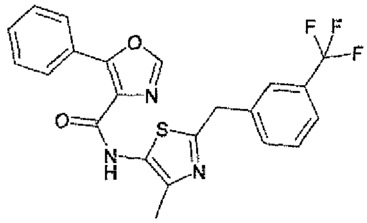 | | 443 |
| 135 | N-{4-methyl-2-[3-(trifluoromethyl)-benzyl]-1,3-thiazol-5-yl}-2-thiophen-2-ylacetamide | 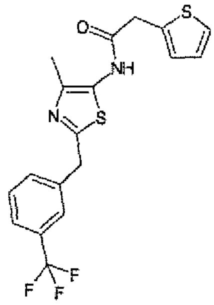 | | 396 |

[Table 1-17]

| Ex. | IUPAC name | structure | salt | MS |
|---|---|---|---|---|
| 136 | 2-(2,3-dihydro-1-benzofuran-5-yl)-N-{4-methyl-2-[3-(trifluoromethyl)-benzyl]-1,3-thiazol-5-yl} acetamide | 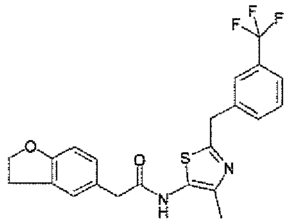 | | 432 |
| 137 | 2-(6-methoxy-1-benzofuran-3-yl)-N-{4-methyl-2-[3-(trifluoromethyl)-benzyl]-1,3-thiazol-5-yl} acetamide | 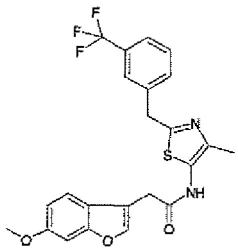 | | 460 |
| 138 | 3-(1H-benzotriazol-1-yl)-N-{4-methyl-2-[3-(trifluoromethyl)-benzyl]-1,3-thiazol-5-yl} propanamide | 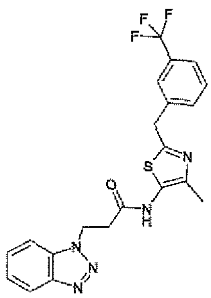 | | 445 |

(continued)

| Ex. | IUPAC name | structure | salt | MS |
|---|---|---|---|---|
| 139 | N-{4-methyl-2-[3-(trifluoromethyl)-benzyl]-1,3-thiazol-5-yl}-2-(1H-tetrazol-1-yl)acetamide | 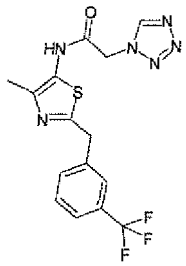 | | 382 |
| 140 | 3-methyl-N-{4-methyl-2-[3-(trifluoromethyl)-benzyl]-1,3-thiazol-5-yl}-4-oxo-3,4-dihydrophthalazine-1-carboxamide | 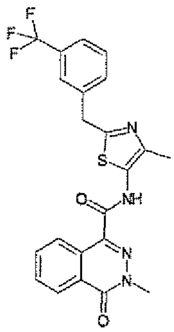 | | 458 |
| 141 | 2-(acetylamino)-N-{4-methyl-2-[3-(trifluoromethyl)-benzyl]-1,3-thiazol-5-yl}-2,3-benzothiazole-6-carboxamide | 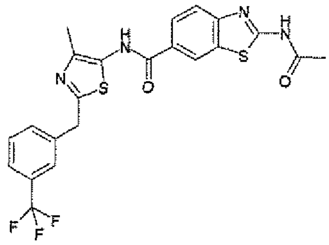 | | 490 |

[Table 1-18]

| Ex. | IUPAC name | structure | salt | MS |
|---|---|---|---|---|
| 142 | 3-(1,3-benzothiazol-2-yl)-N-{4-methyl-2-[3-(trifluoromethyl)-benzyl]-1,3-thiazol-5-yl}propanamide | 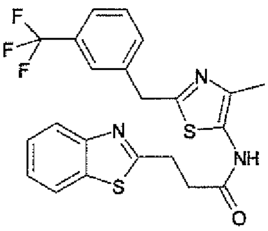 | | 461 |
| 143 | 1-benzyl-N-{4-methyl-2-[3-(trifluoromethyl)-benzyl]-1,3-thiazol-5-yl}-6-oxo-1,6-dihydropyridazine-3-carboxamide | 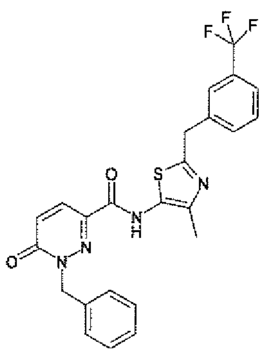 | | 484 |

(continued)

| Ex. | IUPAC name | structure | salt | MS |
|---|---|---|---|---|
| 144 | 2-(5-methoxy-3-oxo-2,3-dihydro-1H-isoindol-1-yl)-N-{4-methyl-2-[3-(trifluoromethyl)-benzyl]-1,3-thiazol-5-yl}acetamide | 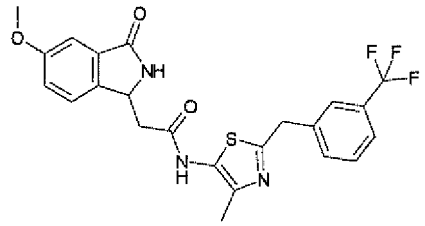 | | 475 |

Example 145

N-(2-(3-chloro-5-fluorobenzyl)-4-methyl-1,3-thiazol-5-yl]-1-methyl-1H-pyrazole-5-carboxamide

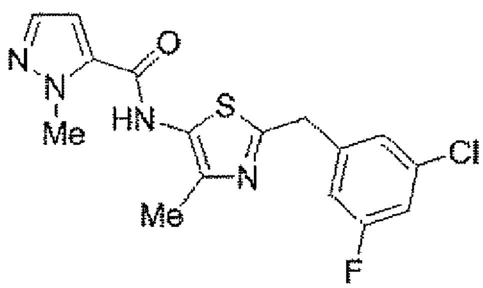

Example 145-A)

(3-chloro-5-fluorophenyl)acetonitrile

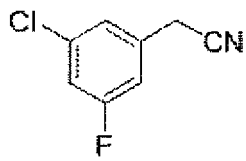

To a solution of 3-chloro-5-fluorobenzyl bromide (58.23 g, 260 mmol) and acetic acid (5.35 mL, 93.6 mmol) in toluene (260 mL) was added dropwise an aqueous solution (195 mL) of sodium cyanide (48.57 g, 990 mmol) and tributylbenzylammonium chloride (5.68 g, 18.2 mmol). The reaction mixture was stirred at room temperature for 4 hr. The organic layer was separated, washed successively with water (200 mL) and saturated brine (200 mL), dried over anhydrous magnesium sulfate and concentrated under reduced pressure. The obtained residue was purified by basic silica gel column chromatography [eluent: hexane-ethyl acetate (4:1)] to give the title compound (44.89 g) as a brown oil (yield 100%).
$^1$H NMR (300 MHz, $CDCl_3$) $\delta$ 3.74 (2H, s), 6.99 (1H, d, J = 8.7 Hz), 7.05-7.13 (1H, m), 7.15 (1H, s).

Example 145-B)

2-(3-chloro-5-fluorophenyl)ethanethioamide

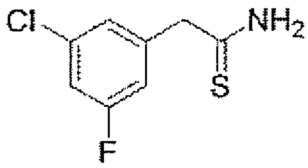

A mixture of (3-chloro-5-fluorophenyl)acetonitrile (46.3 g, 273 mmol) obtained in Example 145-A), 0,0-diethyl dithiophosphate (47.4 mL, 300 mmol) and 4M hydrogen chloride-ethyl acetate solution (546 mL) was stirred at room temperature overnight. Water (300 mL) was added thereto in an ice bath, and the organic layer was separated, successively washed 3 times with water (400 mL), twice with 0.2N aqueous sodium hydroxide solution (200 mL), saturated aqueous sodium hydrogen carbonate solution (200 mL) and saturated brine (200 mL), dried over anhydrous sodium sulfate, and concentrated under reduced pressure. The obtained residue was treated with hexane (400 mL) in an ice bath to allow solidification. The solid was collected by filtration, and washed twice with hexane (100 mL) to give the title compound (36.05 g) as a white solid (yield 67%).
MS (ESI+): $[M+H]^+$ 204.
$^1H$ NMR (300 MHz, $CDCl_3$) 5 4.03 (2H, s), 6.70 (1H, brs), 6.90-7.02 (1H, m), 7.01-7.15 (2H, m), 7.61 (1H brs).

Example 145-C)

ethyl 2-(3-chloro-5-fluorobenzyl)-4-methyl-1,3-thiazole-5-carboxylate

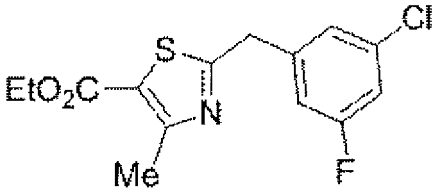

A solution of 2-(3-chloro-5-fluorophenyl)ethanethioamide (51.17 g, 251 mmol) obtained in Example 145-B) and ethyl 2-chloro-3-oxobutanoate (44 mL, 301 mmol) in pyridine (150 mL)-ethanol (150 mL) was stirred at room temperature for 3 days. The reaction mixture was concentrated under reduced pressure, azeotropically distilled twice with toluene, diluted with ethyl acetate, washed successively with 0.2M hydrochloric acid and saturated brine, dried over anhydrous magnesium sulfate, and concentrated under reduced pressure. The obtained residue was purified by basic silica gel column chromatography [eluent: hexane-ethyl acetate (9:1)] to give the title compound (70.26 g) as a pale-yellow solid (yield 89%).
MS (ESI+): $[M+H]^+$ 314.
$^1H$ NMR (300 MHz, $CDCl_3$) 5 1.34 (3H, t, J = 7.0 Hz), 2.71 (3H, s), 4.22 (2H, s), 4.30 (2H, q, J = 7.1 Hz), 6.90-6.97 (1H, m), 7.02 (1H, dt, J = 8.3, 2.1 Hz), 7.10 (1H, s).

Example 145-D)

2-(3-chloro-5-fluorobenzyl)-4-methyl-1,3-thiazole-5-carboxylic acid

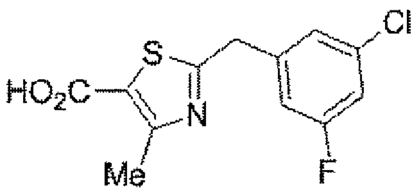

To a solution of ethyl 2-(3-chloro-5-fluorobenzyl)-4-methyl-1,3-thiazole-5-carboxylate (70.3 g, 224 mmol) obtained in Example 145-C) in ethanol (335 mL) was added 8M aqueous sodium hydroxide solution (56 mL), and the mixture was stirred for 3 hr. 6N hydrochloric acid (78 mL) and water (200 mL) were added thereto in an ice bath. The precipitated white solid was collected by filtration, and washed twice with water (150 mL) to give the title compound (61.4 g) as a white solid (yield 65%).
MS (ESI+) : $[M+H]^+$ 285.
$^1H$ NMR (300 MHz, DMSO-$d_6$) $\delta$ 2.58 (3H, s), 4.35 (2H, s), 7.20-7.28 (1H m), 7.30-7.41 (2H, m).
* The peak of "COOH group" was not observed.

Example 145-E)

tert-butyl [2-(3-chloro-5-fluorobenzyl)-4-methyl-1,3-thiazol-5-yl]carbamate

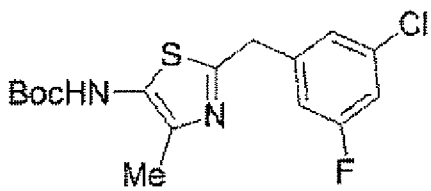

A mixture of 2-(3-chloro-5-fluorobenzyl)-4-methyl-1,3-thiazole-5-carboxylic acid (61.4 g, 215 mmol) obtained in Example 145-D), DPPA (88.9 g, 323 mmol), triethylamine (89 mL, 645 mmol) and tert-butanol (500 mL) was stirred at 90°C for 2.5 hr. The reaction mixture was cooled to room temperature, and concentrated under reduced pressure. The obtained reaction mixture was diluted with ethyl acetate (600 mL), washed twice with 0.2M aqueous sodium hydroxide solution (300 mL), and with saturated brine, dried over anhydrous magnesium sulfate, and concentrated under reduced pressure. The obtained residue was purified by silica gel column chromatography [eluent: hexane-ethyl acetate (9:1-2:1)], and crystallized from hexane to give the title compound (54.63 g) as a pale-yellow solid (yield 71%).
MS (ESI+) : $[M+H]^+$ 357.
$^1H$ NMR (300 MHz, $CDCl_3$) $\delta$ 1.50 (9H, s), 2.29 (3H, s), 4.14 (2H, s), 6.40 (1H, brs), 6.88-6.94 (1H, m), 6.97 (1H, dt, J = 8.3, 2.1 Hz), 7.08 (1H, s).

Example 145-F)

N-[2-(3-chloro-5-fluorobenzyl)-4-methyl-1,3-thiazol-5-yl]-1-methyl-1H-pyrazole-5-carboxamide

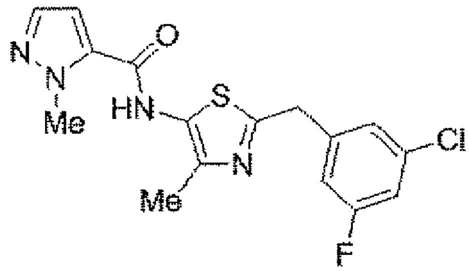

To a solution of tert-butyl [2-(3-chloro-5-fluorobenzyl)-4-methyl-1,3-thiazol-5-yl]carbamate (15.0 g, 42 mmol) obtained in Example 145-E) in ethanol (60 mL) was added dropwise concentrated hydrochloric acid (30 mL) at 0°C, and the mixture was stirred at 50°C for 1.5 hr. The reaction mixture was cooled to 0°C, neutralized with 8M aqueous sodium hydroxide solution (40 mL) , adjusted to pH 10-11 with saturated aqueous sodium hydrogen carbonate solution (150 mL), and extracted twice with ethyl acetate (300 mL). The organic layer was washed with saturated brine (200 mL), dried over anhydrous sodium sulfate, and concentrated under reduced pressure. The obtained residue was dissolved in DMA (84 mL), and 1-methyl-1H-pyrazole-5-carboxylic acid (6.36 g, 50.4 mmol) obtained in Example 1-A), and HATU (19.16 g, 50.4 mmol) and DIEA (3.66 mL, 21 mmol) were added at 0°C. The reaction mixture was stirred at 60°C for 4 hr, water (300 mL) was added and the mixture was extracted twice with ethyl acetate (300 mL). The organic layer was

washed successively with water (300 mL), twice with saturated aqueous sodium hydrogen carbonate solution (300 mL), and saturated brine (300 mL), dried over anhydrous sodium sulfate, and concentrated under reduced pressure. The obtained residue was purified by silica gel column chromatography [eluent: hexane-ethyl acetate (9:1-0:1)] and basic silica gel column chromatography [eluent: hexane-ethyl acetate (9:1-0:1)], and crystallized from ethyl acetate-heptane to give crude crystals (8.02 g) of the title compound as colorless crystals.
To crude crystals (11.8 g) obtained by repeating the aforementioned method was added ethyl acetate (60 mL), and the mixture was heated to 85°C to allow dissolution. The solution was dust removal by filtration, and washed with ethyl acetate (20 mL). The filtrate was heated to 85°C, and heptane (80 mL) was added dropwise. The solution was cooled to 60°C and stirred for 30 min. Further, heptane (60 mL) was added dropwise and the mixture was stirred for 30 min. The mixture was stirred at room temperature for 1 hr, and at 0°C for 1 hr, and the resulting crystals were collected by filtration, and washed with heptane-ethyl acetate mixed solvent (9:1, 50 mLx2) to give the title compound (10.9 g) as colorless crystals.
MS (ESI+): $[M+H]^+$ 365.
$^1H$ NMR (300 MHz, $CDCl_3$) $\delta$ 2.40 (3H, s), 4.20 (2H, s), 4.21 (3H, s), 6.68 (1H, d, J = 2.3 Hz), 6.89-7.04 (2H, m), 7.08-7.14 (1H, m), 7.53 (1H, d, J = 2.3 Hz), 7.72 (1H, brs).
Mp 159-161°C

Example 146

N-[2-(3-chloro-5-fluorobenzyl)-4-methyl-1,3-thiazol-5-yl]-1,3-dimethyl-1H-pyrazole-4-carboxamide

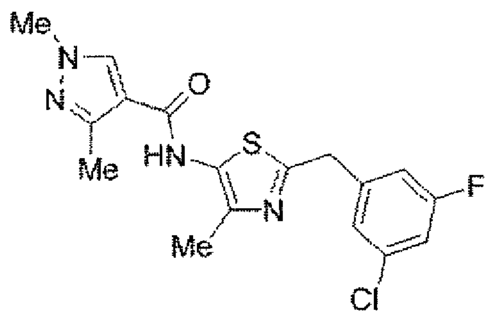

A mixture of tert-butyl [2-(3-chloro-5-fluorobenzyl)-4-methyl-1,3-thiazol-5-yl]carbamate (500 mg, 1.40 mmol) obtained in Example 145-E), concentrated hydrochloric acid (1 mL) and ethanol (2 mL) was stirred at 50°C for 1 hr. The reaction mixture was neutralized with saturated aqueous sodium hydrogen carbonate solution, and extracted with ethyl acetate. The organic layer was washed with saturated brine, dried over anhydrous sodium sulfate, and concentrated under reduced pressure. The residue was dissolved in DMF (3 mL), 1,3-dimethyl-1H-pyrazole-4-carboxylic acid (216 mg, 1.54 mmol), HATU (585 mg, 1.54 mmol) and DIEA (0.12 mL, 0.70 mmol) were added, and the mixture was stirred at 60°C for 4 hr. The reaction mixture was diluted with ethyl acetate, and washed with saturated aqueous sodium hydrogen carbonate solution and saturated brine. The organic layer was dried over anhydrous sodium sulfate, concentrated under reduced pressure and the obtained residue was purified by silica gel column chromatography [eluent: ethyl acetate-methanol (100:0-80:20)]. The obtained solid was crystallized from heptane-ethyl acetate to give the title compound (40 mg) as a pale-brown solid (yield 8%).
MS (ESI+): $[M+H]^+$ 379.
$^1H$ NMR (300 MHz, $CDCl_3$) $\delta$ 2.39 (3H, s), 2.55 (3H, s), 3.88 (3H, s), 4.18 (2H, s), 6.90-7.01 (2H, m), 7.08-7.12 (1H, m), 7.46 (1H, s), 7.81 (1H, s).
mp 125-128°C

Example 147

N-[2-(3-chloro-5-fluorobenzyl)-4-methyl-1,3-thiazol-5-yl]-1,3-dimethyl-1H-pyrazole-5-carboxamide

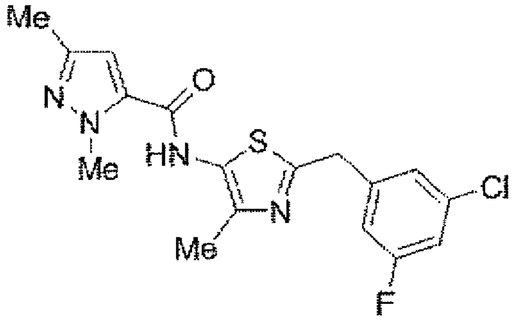

tert-Butyl [2-(3-chloro-5-fluorobenzyl)-4-methyl-1,3-thiazol-5-yl]carbamate (240 mg, 0.67 mmol) obtained in Example 145-E) was dissolved in 12M hydrochloric acid (1 mL) and ethanol (1 mL), and the mixture was stirred at room temperature for 1 hr. The reaction mixture was concentrated under reduced pressure, basified with saturated aqueous sodium hydrogen carbonate solution, and the mixture was extracted with ethyl acetate. The extract was dried over anhydrous sodium sulfate, and concentrated under reduced pressure. The residue was dissolved in DMF (2 mL), 1,3-dimethyl-1H-pyrazole-5-carboxylic acid (94 mg, 0.67mmol), HATU (307 mg, 0.81 mmol) and DIEA (0.058 mL, 0.34 mmol) were added at room temperature, and the mixture was stirred at 60°C for 3 hr. The reaction mixture was diluted with saturated aqueous sodium hydrogen carbonate solution, and extracted with ethyl acetate. The extract was washed with water and saturated brine, dried over anhydrous sodium sulfate, and concentrated under reduced pressure. The obtained residue was purified by silica gel column chromatography [eluent: hexane-ethyl acetate (90:10-50:50)] to give the title compound (102 mg) as a pale-brown powder (yield 40%).
MS (ESI+): $[M+H]^+$ 379.
$^1H$ NMR (300 MHz, $CDCl_3$) $\delta$ 2.30 (3H, s), 2.39 (3H, s), 4.13 (3H, s), 4.19 (2H, s), 6.44 (1H, s), 6.84-7.04 (2H, m), 7.10 (1H s), 7.66 (1H, brs).

Example 148

N-[2-(3-chloro-5-fluorobenzyl)-4-methyl-1,3-thiazol-5-yl]-1-ethyl-1H-pyrazole-5-carboxamide

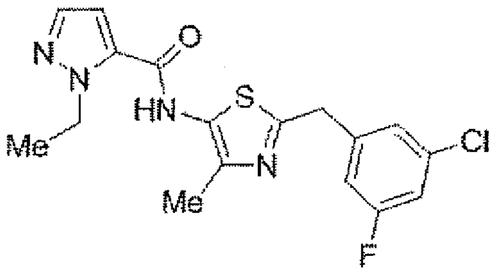

tert-Butyl [2-(3-chloro-5-fluorobenzyl)-4-methyl-1,3-thiazol-5-yl]carbamate (240 mg, 0.67 mmol) obtained in Example 145-E) was dissolved in 12M hydrochloric acid (1 mL) and ethanol (1 mL), and the mixture was stirred at room temperature for 1 hr. The reaction mixture was concentrated under reduced pressure, basified with saturated aqueous sodium hydrogen carbonate solution, and the mixture was extracted with ethyl acetate. The extract was dried over anhydrous sodium sulfate, and concentrated under reduced pressure. The residue was dissolved in DMF (3 mL), 1-ethyl-1H-pyrazole-5-carboxylic acid (94 mg, 0.67mmol), HATU (307 mg, 0.81 mmol) and DIEA (0.058 mL, 0.34 mmol) were added at room temperature, and the mixture was stirred at 60°C for 3 hr. The reaction mixture was diluted with saturated aqueous sodium hydrogen carbonate solution, and extracted with ethyl acetate. The extract was washed with water and saturated brine, dried over anhydrous sodium sulfate, and concentrated under reduced pressure. The obtained residue was purified by silica gel column chromatography [eluent: hexane-ethyl acetate (90:10-50:50)] to give the title compound (93 mg) as a pale-brown powder (yield 37%).
MS (ESI+): $[M+H]^+$ 379.
$^1H$ NMR (300 MHz, $CDCl_3$) $\delta$ 1.46 (3H, t, J = 7.2 Hz), 2.40 (3H, s), 4.20 (2H, s), 4.62 (2H, q, J = 7.2 Hz), 6.66 (1H, d, J = 1.9 Hz), 6.87-7.05 (2H, m), 7.10 (1H, s), 7.54 (1H, d, J = 2.3 Hz), 7.73 (1H, brs).

Example 149

3-chloro-N-[2-(3-chloro-5-fluorobenzyl)-4-methyl-1,3-thiazol-5-yl]-1-methyl-1H-pyrazole-5-carboxamide

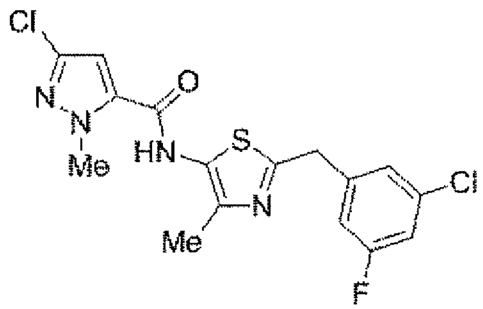

tert-Butyl [2-(3-chloro-5-fluorobenzyl)-4-methyl-1,3-thiazol-5-yl]carbamate (240 mg, 0.67 mmol) obtained in Example 145-E) was dissolved in 12M hydrochloric acid (1 mL) and ethanol (1 mL), and the mixture was stirred at room temperature for 1 hr. The reaction mixture was concentrated under reduced pressure, basified with saturated aqueous sodium hydrogen carbonate solution, and extracted with ethyl acetate. The extract was dried over anhydrous sodium sulfate, and concentrated under reduced pressure. The residue was dissolved in DMF (3 mL), 3-chloro-1-methyl-1H-pyrazole-5-carboxylic acid (108 mg, 0.67mmol), HATU (307 mg, 0.81 mmol) and DIEA (0.058 mL, 0.34 mmol) were added at room temperature, and the mixture was stirred at 60°C for 3 hr. The reaction mixture was diluted with saturated aqueous sodium hydrogen carbonate solution, and extracted with ethyl acetate. The extract was washed with water and saturated brine, dried over anhydrous sodium sulfate, and concentrated under reduced pressure. The obtained residue was purified by silica gel column chromatography [eluent: hexane-ethyl acetate (90:10-50:50)] to give the title compound (58 mg) as a pale-brown powder (yield 22%).
MS (ESI+): $[M]^+$ 399.
$^1H$ NMR (300 MHz, $CDCl_3$) δ 2.45 (3H, s), 4.16-4.29 (5H, m), 6.84-7.06 (2H, m), 7.05-7.17 (1H, m), 7.54 (1H, s), 8.72-8.92 (1H, m).

Example 150

N-[2-(3-chloro-5-fluorobenzyl)-4-methyl-1,3-thiazol-5-yl]-1-(difluoromethyl)-1H-pyrazole-5-carboxamide

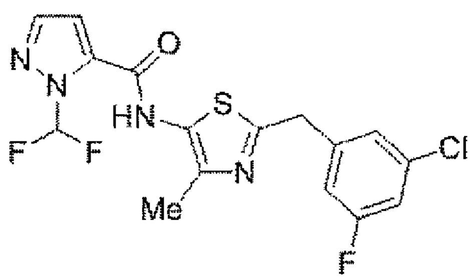

tert-butyl [2-(3-chloro-5-fluorobenzyl)-4-methyl-1,3-thiazol-5-yl]carbamate (240 mg, 0.67 mmol) obtained in Example 145-E) was dissolved in 12M hydrochloric acid (1 mL) and ethanol (1 mL), and the mixture was stirred at room temperature for 1 hr. The reaction mixture was concentrated under reduced pressure, basified with saturated aqueous sodium hydrogen carbonate solution, and the mixture was extracted with ethyl acetate. The extract was dried over anhydrous sodium sulfate, and concentrated under reduced pressure. The residue was dissolved in DMF (2 mL), 1-(difluoromethyl)-1H-pyrazole-5-carboxylic acid (109 mg, 0.67mmol), HATU (307 mg, 0.81 mmol) and DIEA (0.058 mL, 0.34 mmol) were added at room temperature, and the mixture was stirred at 60°C for 3 hr. The reaction mixture was diluted with saturated aqueous sodium hydrogen carbonate solution, and the mixture was extracted with ethyl acetate. The extract was washed with water and saturated brine, dried over anhydrous sodium sulfate, and concentrated under reduced pressure. The obtained residue was purified by silica gel column chromatography [eluent: hexane-ethyl acetate

(90:10-50:50)] to give the title compound (100 mg) as a pale-brown powder (yield 37%).
MS (ESI+): $[M+H]^+$ 401.
$^1$H NMR (300 MHz, $CDCl_3$) δ 2.41 (3H, s), 4.21 (2H, s), 6.86 (1H, s), 6.90-6.97 (1H, m), 6.97-7.06 (1H, m), 7.10 (1H, s), 7.81 (2H, s), 8.07 (1H, s).

Example 151

N-[2-(3-chloro-5-fluorobenzyl)-4-methyl-1,3-thiazol-5-yl]-1-(1-methylethyl)-1H-pyrazole-5-carboxamide

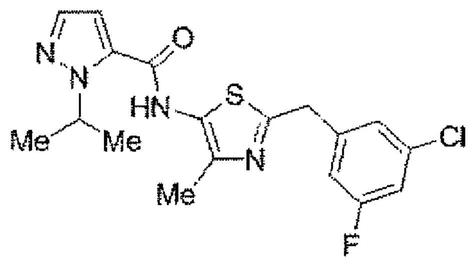

tert-Butyl [2-(3-chloro-5-fluorobenzyl)-4-methyl-1,3-thiazol-5-yl]carbamate (240 mg, 0.67 mmol) obtained in Example 145-E) was dissolved in 12M hydrochloric acid (1 mL) and ethanol (1 mL), and the mixture was stirred at room temperature for 1 hr. The reaction mixture was concentrated under reduced pressure, basified with saturated aqueous sodium hydrogen carbonate solution, and extracted with ethyl acetate. The extract was dried over anhydrous sodium sulfate, and concentrated under reduced pressure. The residue was dissolved in DMF (2 mL), 1-(1-methylethyl)-1H-pyrazole-5-carboxylic acid (104 mg, 0.67mmol)), HATU (307 mg, 0.81 mmol) and DIEA (0.058 mL, 0.34 mmol) were added at room temperature, and the mixture was stirred at 60°C for 3 hr. The reaction mixture was diluted with saturated aqueous sodium hydrogen carbonate solution, and extracted with ethyl acetate. The extract was washed with water and saturated brine, dried over anhydrous sodium sulfate, and concentrated under reduced pressure. The obtained residue was purified by silica gel column chromatography [eluent: hexane-ethyl acetate (90:10-50:50)] to give the title compound (130 mg) as a pale-brown powder (yield 49%).
MS (ESI+): $[M+H]^+$ 393.
$^1$H NMR (300 MHz, $CDCl_3$) δ 1.51 (6H, d, J = 6.8 Hz), 2.40 (3H, s), 4.20 (2H, s), 5.30-5.65 (1H, m), 6.64 (1H, d, J = 2.3 Hz), 6.87-7.05 (2H, m), 7.11 (1H, s), 7.57 (1H d, J = 1.9 Hz), 7.72 (1H, brs).

Example 152

N-[2-(3-chloro-5-fluorobenzyl)-4-methyl-1,3-thiazol-5-yl]-1-methyl-3-(trifluoromethyl)-1H-pyrazole-5-carboxamide

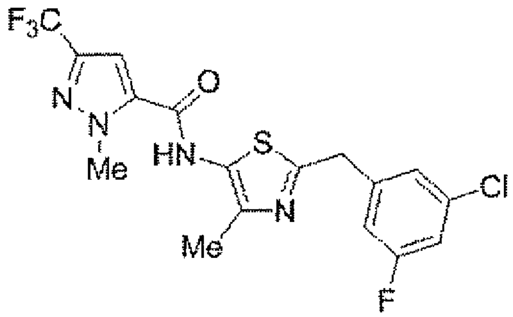

tert-Butyl [2-(3-chloro-5-fluorobenzyl)-4-methyl-1,3-thiazol-5-yl]carbamate (240 mg, 0.67 mmol) obtained in Example 145-E) was dissolved in 12M hydrochloric acid (1 mL) and ethanol (1 mL), and the mixture was stirred at room temperature for 1 hr. The reaction mixture was concentrated under reduced pressure, basified with saturated aqueous sodium hydrogen carbonate solution, and extracted with ethyl acetate. The extract was dried over anhydrous sodium

sulfate, and concentrated under reduced pressure. The residue was dissolved in DMF (2 mL), 1-methyl-3-(trifluoromethyl)-1H-pyrazole-5-carboxylic acid (131 mg, 0.67 mmol), HATU (307 mg, 0.81 mmol) and DIEA (0.058 mL, 0.34 mmol) were added at room temperature, and the mixture was stirred at 60°C was stirred for 3 hr. The reaction mixture was diluted with saturated aqueous sodium hydrogen carbonate solution, and the mixture was extracted with ethyl acetate. The extract was washed with water and saturated brine, dried over anhydrous sodium sulfate, and concentrated under reduced pressure. The obtained residue was purified by silica gel column chromatography [eluent: hexane-ethyl acetate (90:10-50:50)] to give the title compound (43 mg) as a pale-brown powder (yield 15%).
MS (ESI+): $[M+H]^+$ 433.
$^1H$ NMR (300 MHz, $CDCl_3$) $\delta$ 2.41 (3H, s), 4.20 (2H, s), 4.25 (3H, s), 6.89-7.04 (3H, m), 7.11 (1H, s), 7.70 (1H, brs).

Example 153

N-[2-(3-chloro-5-fluoxobenzyl)-4-methyl-1,3-thiazol-5-yl]-1-propyl-1H-pyrazole-5-carboxamide

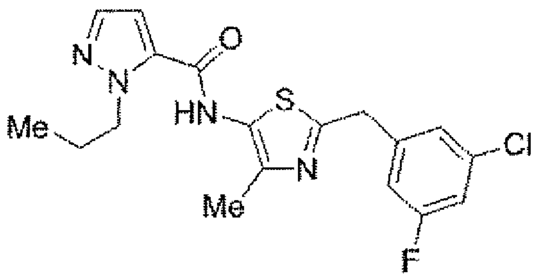

tert-Butyl [2-(3-chloro-5-fluorobenzyl)-4-methyl-1,3-thiazol-5-yl]carbamate (240 mg, 0.67 mmol) obtained in Example 145-E) was dissolved in 12M hydrochloric acid (1 mL) and ethanol (1 mL), and the mixture was stirred at room temperature for 1 hr. The reaction mixture was concentrated under reduced pressure, basified with saturated aqueous sodium hydrogen carbonate solution, and extracted with ethyl acetate. The extract was dried over anhydrous sodium sulfate, and concentrated under reduced pressure. The residue was dissolved in DMF (2 mL), 1-propyl-1H-pyrazole-5-carboxylic acid (104 mg, 0.67 mmol), HATU (307 mg, 0.81 mmol) and DIEA (0.058 mL, 0.34 mmol) were added at room temperature, and the mixture was stirred at 60°C for 3 hr. The reaction mixture was diluted with saturated aqueous sodium hydrogen carbonate solution, and extracted with ethyl acetate. The extract was washed with water and saturated brine, dried over anhydrous sodium sulfate, and concentrated under reduced pressure. The obtained residue was purified by silica gel column chromatography [eluent: hexane-ethyl acetate (90:10-50:50)] to give the title compound (39 mg) as a pale-brown powder (yield 15%).
MS (ESI+): $[M+H]^+$ 393.
$^1H$ NMR (300 MHz, $CDCl_3$) $\delta$ 0.91 (3H, t, J = 7.5 Hz), 1.73-2.06 (2H, m), 2.40 (3H, s), 4.20 (2H, s), 4.40-4.70 (2H, m), 6.66 (1H, d, J = 2.3 Hz), 6.88-7.04 (2H, m), 7.11 (1H, s), 7.54 (1H, d, J = 2.3 Hz), 7.69 (1H, brs).

Example 154

N-[2-(3-chloro-5-fluorobenzyl)-4-metlyl-1,3-thiazol-5-yl]-1-[(4-chlorophenyl)sulfonyl]cyclopropanecarboxamide

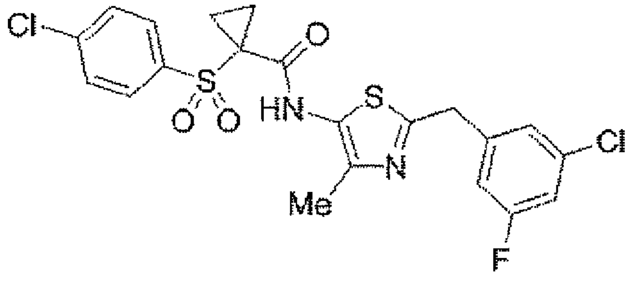

To a solution of tert-butyl [2-(3-chloro-5-fluorobenzyl)-4-methyl-1,3-thiazol-5-yl]carbamate (187 mg, 0.523

mmol) obtained in Example 145-E) in ethanol (0.3 mL) was added dropwise concentrated hydrochloric acid (1 mL) at 0°C, and the mixture was stirred at 50°C for 2 hr. The reaction mixture was cooled to 0°C, neutralized with 8M aqueous sodium hydroxide solution (1.5 mL), adjusted to pH 10-11 with saturated aqueous sodium hydrogen carbonate solution, and extracted twice with ethyl acetate. The organic layer was washed with saturated brine, dried over anhydrous sodium sulfate, and concentrated under reduced pressure. The obtained residue was dissolved in DMA (2 mL), and 1-[(4-chlorophenyl)sulfonyl]cyclopropanecarboxylic acid (150 mg, 0.575 mmol), HATU (219 mg, 0.575 mmol) and DIEA (0.045 mL, 0.261 mmol) were added at 0°C. The reaction mixture was stirred at 60°C for 3 hr, water was added and the mixture was extracted twice with ethyl acetate. The organic layer was washed successively with water, twice with saturated aqueous sodium hydrogen carbonate solution, and saturated brine, dried over anhydrous sodium sulfate, and concentrated under reduced pressure. The obtained residue was purified by silica gel column chromatography [eluent: hexane-ethyl acetate (9:1-0:1)] and basic silica gel column chromatography [eluent: hexane-ethyl acetate (9:1-0:1)], and crystallized from ethyl acetate-hexane to give the title compound (114 mg) as colorless crystals (yield 44%).
MS (ESI+) :$[M+H]^+$ 499.
$^1$H NMR (300 MHz, $CDCl_3$) 5 1.76-1.84 (2H, m), 1.85-1.94 (2H, m), 2.46 (3H, s), 4.16 (2H, s), 6.87-6.94 (1H, m), 6.97 (1H dt, J =8.7, 2.1 Hz), 7.08 (1H, s), 7.51-7.58 (2H, m), 7.73-7.81 (2H, m), 10.08 (1H, s).

Example 155

N-[2-(2,4-dichlorobenzyl)-4-methyl-1,3-thiazol-5-yl]-1-methyl-1H-pyrazole-5-carboxamide

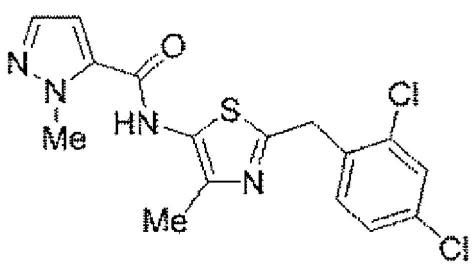

Example 155-A)

2-(2,4-dichlorophenyl)ethanethioamide

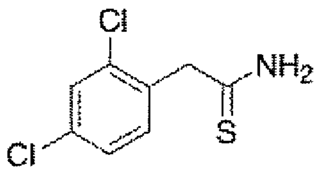

A mixture of (2,4-dichlorophenyl)acetonitrile (10 g, 53.75 mmol)), 0,0-diethyl dithiophosphate (9.33 mL, 59.12 mmol) and 4M hydrogen chloride-ethyl acetate solution (100 mL) was stirred at room temperature overnight. The precipitated white solid was collected by filtration, and washed with ethyl acetate to give the title compound (9.57 g) as a white solid (yield 81%).
MS (ESI+): $[M+H]^+$ 220.
$^1$H NMR (300 MHz, $CDCl_3$) $\delta$ 4.15 (2H, s), 6.67-6.92 (1H, m), 7.27-7.32 (1H, m), 7.34-7.40 (1H m), 7.45 (1H, d, J = 1.9 Hz), 7.55 (1H, brs).

Example 155-B)

ethyl 2-(2,4-dichlorobenzyl)-4-methyl-1,3-thiazole-5-carboxylate

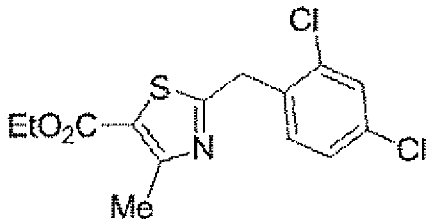

A solution of 2-(2,4-dichlorophenyl)ethanethioamide (9.57 g, 43.47 mmol) obtained in Example 155-A) and ethyl 2-chloro-3-oxobutanoate (7.66 mL, 52.17 mmol) in pyridine (30 mL)-ethanol (30 mL) was stirred at room temperature for 2 days. The reaction mixture was concentrated under reduced pressure, azeotropically distilled twice with toluene, diluted with ethyl acetate, washed successively with 1M hydrochloric acid, saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (9:1-2:1)] to give the title compound (13.87 g) as a white solid (yield 97%).
MS (ESI+): $[M+H]^+$ 330.
$^1H$ NMR (300 MHz, $CDCl_3$) $\delta$ 1.33 (3H, t, J = 7.2 Hz), 2.71 (3H, s), 4.29 (2H, q, J =7.2 Hz), 4.36 (2H, s), 7.14-7.35 (2H, m), 7.43 (1H, d, J = 1.9 Hz).

Example 155-C)

2-(2,4-dichlorobenzyl)-4-methyl-1,3-thiazole-5-carboxylic acid

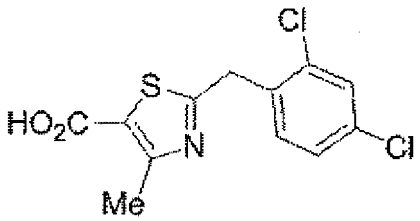

To a solution of ethyl 2-(2,4-dichlorobenzyl)-4-methyl-1,3-thiazole-5-carboxylate (13.87 g, 42.0 mmol) obtained in Example 155-B) in ethanol (60 mL) was added 8M aqueous sodium hydroxide solution (10.5 mL), and the mixture was stirred for 4 hr. 6N Hydrochloric acid (16 mL) and water (50 mL) were added thereto in an ice bath. The precipitated white solid was collected by filtration, and washed twice with water (50 mL) to give the title compound (11.11 g) as a white solid (yield 88%).
MS (ESI+): $[M+H]^+$ 301.
$^1H$ NMR (300 MHz, DMSO-$d_6$) $\delta$ 2.57 (3H, s), 4.41 (2H, s), 7.40-7.51 (1H, m), 7.51-7.61 (1H, m), 7.67 (1H, d, J = 1.9 Hz), 13.24 (1H, brs).

Example 155-D)

tert-butyl [2-(2,4-dichlorobenzyl)-4-methyl-1,3-thiazol-5-yl]carbamate

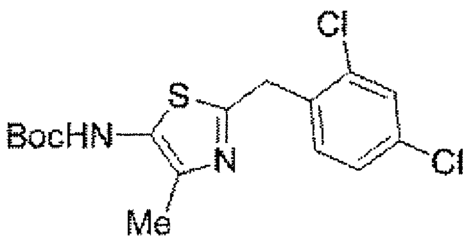

A mixture of 2-(2,4-dichlorobenzyl)-4-methyl-1,3-thiazole-5-carboxylic acid (1.83 g, 6.05 mmol) obtained in Example 155-C), DPPA (1.96 mL, 9.08 mmol), triethylamine (2.52 mL, 18.15 mmol) and tert-butanol (15 mL) was stirred at 90°C for 2 hr. The reaction mixture was cooled to room temperature, and concentrated under reduced pressure. The obtained residue was purified by silica gel column chromatography [eluent: hexane-ethyl acetate (9:1-1:1)] to give the title compound (1.89 g) as a pale-yellow solid (yield 84%).
MS (ESI+): [M+H] + 372.
$^1$H NMR (300 MHz, $CDCl_3$) δ 1.49 (9H, s), 2.28 (3H, s), 4.29 (2H, s), 6.41 (1H, brs), 7.17-7.23 (1H, m), 7.23-7.31 (1H, m), 7.40 (1H, d, J = 1.9 Hz).

Example 155-E)

N-[2-(2,4-dichlorobenzyl)-4-methyl-1,3-thiazol-5-yl]-1-methyl-1H-pyrazole-5-carboxamide

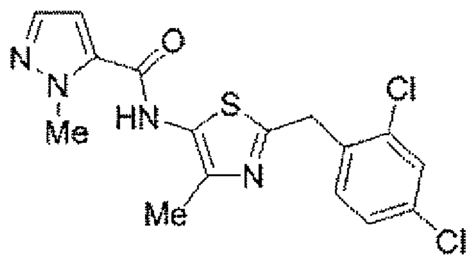

To a solution of tert-butyl [2-(2,4-dichlorobenzyl)-4-methyl-1,3-thiazol-5-yl]carbamate (536 mg, 1.44 mmol) obtained in Example 155-D) in ethanol (0.9 mL) was added dropwise concentrated hydrochloric acid (3 mL) at 0°C, and the mixture was stirred at room temperature for 15 min. The reaction mixture was cooled to 0°C, neutralized with 8M aqueous sodium hydroxide solution (4.5 mL), adjusted to pH 10-11 with saturated aqueous sodium hydrogen carbonate solution, and extracted twice with ethyl acetate. The organic layer was washed with saturated brine, dried over anhydrous sodium sulfate, and concentrated under reduced pressure. The obtained residue was dissolved in DMA (5 mL), and 1-methyl-1H-pyrazole-5-carboxylic acid (218 mg, 1.73 mmol) obtained in Example 1-A), HATU (658 mg, 1.73 mmol) and DIEA (0.126 mL, 0.72 mmol) were added at 0°C. The reaction mixture was stirred at 60°C for 3 hr, water was added, and the mixture was extracted twice with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-0:1)] and basic silica gel column chromatography [eluent: hexane-ethyl acetate (9:1-0:1)], and crystallized from ethyl acetate-hexane to give the title compound (314 mg) as yellow crystals (yield 57%).
MS (ESI+): $[M+H]^+$ 381.
$^1$H NMR (300 MHz, $CDCl_3$) δ 2.39 (3H, s), 4.20 (3H, s), 4.34 (2H, s), 6.67 (1H, d, J = 1.9 Hz), 7.15-7.36 (2H, m), 7.42 (1H, d, J = 2.3 Hz), 7.52 (1H, d, J = 1.9 Hz), 7.70 (1H brs).

Example 156

N-[2-(3-chloro-4-fluorobenzyl)-4-methyl-1,3-thiazol-5-yl]-1-methyl-1H-pyrazole-5-carboxamide

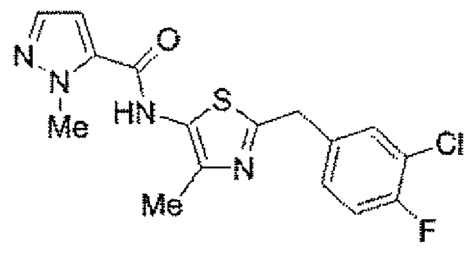

Example 156-A)

2-(3-chloro-4-fluorophenyl)ethanethioamide

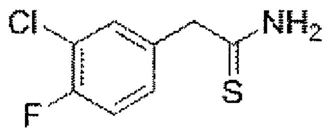

A mixture of (3-chloro-4-fluorophenyl)acetonitrile (10.00 g, 58.96 mmol), 0,0-diethyl dithiophosphate (10.24 mL, 64.86 mmol) and 4M hydrogen chloride-ethyl acetate solution (100 mL) was stirred at room temperature overnight. Water (100 mL) was added thereto in an ice bath, the organic layer was separated, successively washed twice with water (100 mL), twice with 0.2N aqueous sodium hydroxide solution (100 mL), saturated aqueous sodium hydrogen carbonate solution (100 mL) and saturated brine (100 mL), dried over anhydrous sodium sulfate, and concentrated under reduced pressure. The obtained residue was treated with hexane (100 mL) in an ice bath to allow solidification. The solid was collected by filtration, and washed with hexane (50 mL) to give the title compound (8.69 g) as a white solid (yield 73%).
MS (ESI+): $[M+H]^+$ 204 .
$^1$H NMR (300 MHz, $CDCl_3$) δ 4.02 (2H, s), 6.71 (1H, d, J = 5.7 Hz), 7.05-7.22 (2H, m), 7.36 (1H, dd, J = 6.6, 1.7 Hz), 7.46-7.83 (1H, m).

Example 156-B)

ethyl 2-(3-chloro-4-fluorobenzyl)-4-methyl-1,3-thiazole-5-carboxylate

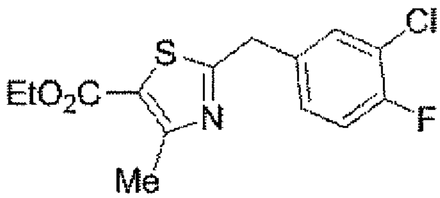

A solution of 2-(3-chloro-4-fluorophenyl)ethanethioamide (8.69 g, 42.67 mmol) obtained in Example 156-A) and ethyl 2-chloro-3-oxobutanoate (7.93 mL, 54.00 mmol) in pyridine (30 mL)-ethanol (30 mL) was stirred at room temperature for 2 days. The reaction mixture was concentrated under reduced pressure, azeotropically distilled twice with toluene, diluted with ethyl acetate, washed successively with 1M hydrochloric acid, saturated aqueous sodium hydrogen carbonate solution and saturated brine, dried over anhydrous magnesium sulfate, and concentrated under reduced pressure. The obtained residue was crystallized from hexane-ethyl acetate to give the title compound (9.86 g) as a white solid (yield 74%).
MS (ESI+): $[M+H]^+$ 313.
$^1$H NMR (300 MHz, $CDCl_3$) δ 1.33 (3H, t, J = 7.2 Hz), 2.71 (3H, s), 4.21 (2H, s), 4.30 (2H, q, J = 7.2 Hz), 7.06-7.14 (1H, m), 7.14-7.24 (1H, m), 7.35 (1H, dd, J = 6.8, 2.3 Hz).

Example 156-C)

2-(3-chloro-4-fluorobenzyl)-4-methyl-1,3-thiazole-5-carboxylic acid

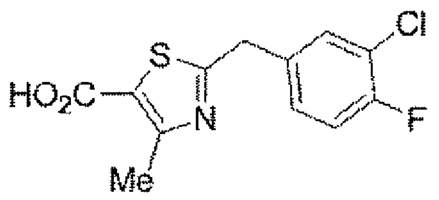

To a solution of ethyl 2-(3-chloro-4-fluorobenzyl)-4-methyl-1,3-thiazole-5-carboxylate (9.86 g, 31.46 mmol) obtained in Example 156-B) in ethanol (50 mL)-THF (25 mL) was added 8M aqueous sodium hydroxide solution (7.87 mL), and the mixture was stirred for 6 hr. 6N Hydrochloric acid (12 mL) and water (20 mL) were added thereto in an ice bath. The precipitated white solid was collected by filtration, and washed twice with water (20 mL) to give the title compound (7.41 g) as a white solid (yield 82%).
MS (ESI+): $[M+H]^+$ 286.
$^1$H NMR (300 MHz, DMSO-$d_6$) δ 2.57 (3H, s), 4.32 (2H, s), 7.31-7.43 (2H, m), 7.55-7.67 (1H, m).
* The peak of "COOH group" was not observed.

Example 156-D)

tert-butyl [2-(3-chloro-4-fluorobenzyl)-4-methyl-1,3-thiazol-5-yl]carbamafe

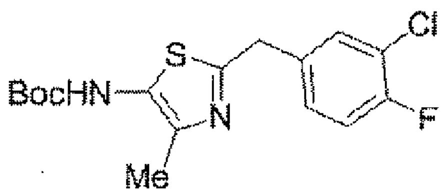

A mixture of 2-(3-chloro-4-fluorobenzyl)-4-methyl-1,3-thiazole-5-carboxylic acid (2.00 g, 7.00 mmol) obtained in Example 156-C), DPPA (2.26 mL, 10.5 mmol), triethylamine (2.91 mL, 21.0 mmol) and tert-butanol (15 mL) was stirred at 90°C for 2 hr. The reaction mixture was cooled to room temperature, and concentrated under reduced pressure. The obtained residue was purified by silica gel column chromatography [eluent: hexane-ethyl acetate (9:1-1:1)] to give the title compound (1.63 g) as a pale-yellow oil (yield 65%).
MS (ESI+): $[M+H]^+$ 356.
$^1$H NMR (300 MHz, $CDCl_3$) δ 1.50 (9H, s), 2.29 (3H, s), 4.14 (2H, s), 6.40 (1H, brs), 7.03-7.12 (1H, m), 7.12-7.22 (1H m), 7.34 (1H, dd, J = 7.2, 2.3 Hz).

Example 156-E)

N-[2-(3-chloro-4-fluorobenzyl)-4-methyl-1,3-thiazol-5-yl]-1-methyl-1H-pyrazole-5-carboxamide

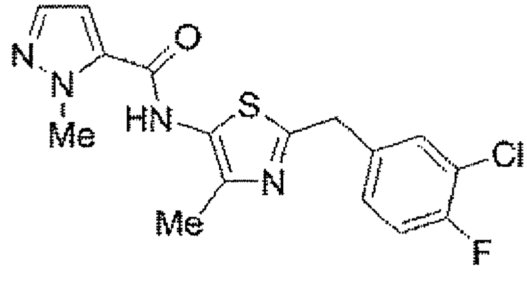

To a solution of tert-butyl [2-(3-chloro-4-fluorobenzyl)-4-methyl-1,3-thiazol-5-yl]carbamate (445 mg, 1.25 mmol) obtained in Example 156-D) in ethanol (0.9 mL) was added dropwise concentrated hydrochloric acid (3 mL) at 0°C, and

the mixture was stirred at room temperature for 15 min. The reaction mixture was cooled to 0°C, neutralized with 8M aqueous sodium hydroxide solution (4.5 mL), adjusted to pH 10-11 with saturated aqueous sodium hydrogen carbonate solution, and extracted twice with ethyl acetate. The organic layer was washed with saturated brine, dried over anhydrous sodium sulfate, and concentrated under reduced pressure. The obtained residue was dissolved in DMA (4 mL), and 1-methyl-1H-pyrazole-5-carboxylic acid (189 mg, 1.50 mmol) obtained in Example 1-A), HATU (570 mg, 1.50 mmol) and DIEA (0.109 mL, 0.63 mmol) were added. The reaction mixture was stirred at 60°C for 3 hr, water was added, and the mixture was extracted twice with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-0:1)] and basic silica gel column chromatography [eluent: hexane-ethyl acetate (9:1-0:1)], and crystallized from ethyl acetate-hexane to give the title compound (207 mg) as colorless crystals (yield 450).
MS (ESI+): $[M+H]^+$ 365.
$^1H$ NMR (300 MHz, $CDCl_3$) 5 2.39 (3H, s), 4.18 (2H, s), 4.20 (3H, s), 6.67 (1H, d, J = 1.9 Hz), 7.03-7.14 (1H, m), 7.14-7.22 (1H, m), 7.35 (1H, dd, J = 7.2, 2.3 Hz), 7.52 (1H, d, J = 1.9 Hz), 7.73 (1H, brs).

Example 157

N-[2-(3-chlorobenzyl)-4-methyl-1,3-thiazol-5-yl]-1-methyl-1H-pyrazole-5-carboxamide

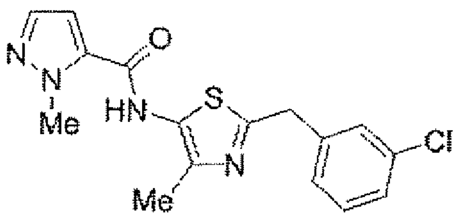

Example 157-A)

2-(3-chlorophenyl)ethanethioamide

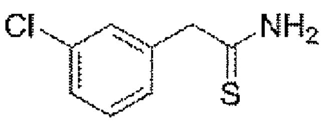

A mixture of (3-chlorophenyl)acetonitrile (10.00 g, 65.97 mmol), 0,0-diethyl dithiophosphate (11.45 mL, 72.56 mmol) and 4M hydrogen chloride-ethyl acetate solution (100 mL) was stirred at room temperature overnight. Water (100 mL) was added thereto in an ice bath, the organic layer was separated, successively washed twice with water (100 mL), twice with 0.2N aqueous sodium hydroxide solution (100 mL), saturated aqueous sodium hydrogen carbonate solution (100 mL) and saturated brine (100 mL), dried over anhydrous sodium sulfate, and concentrated under reduced pressure. The obtained residue was treated with hexane (100 mL) in an ice bath to allow solidification. The solid was collected by filtration, and washed with hexane (50 mL) to give the title compound (8.94 g) as a yellow solid (yield 73%).
MS (ESI+) : $[M+H]^+$ 185.
$^1H$ NMR (300 MHz, $CDCl_3$) $\delta$ 4.07 (2H, s), 6.64 (1H, brs), 7.13-7.23 (1H, m), 7.28-7.37 (3H, m), 7.41-7.61 (1H, m).

Example 157-B)

ethyl 2-(3-chlorobenzyl)-4-methyl-1,3-thiazole-5-carboxylate

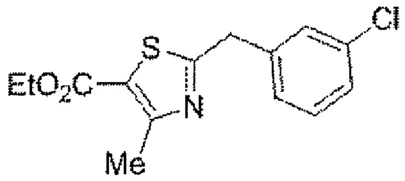

A solution of 2-(3-chlorophenyl)ethanethioamide (8.94 g, 48.15 mmol) obtained in Example 157-A) and ethyl 2-chloro-3-oxobutanoate (8.48 mL, 57.78 mmol) in pyridine (30 mL)-ethanol (30 mL) was stirred at room temperature for 1 day. The reaction mixture was concentrated under reduced pressure, azeotropically distilled twice with toluene, diluted with ethyl acetate, washed successively with 1M hydrochloric acid, saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (97:3-1:1)] to give the title compound (12.58 g) as a yellow solid (yield 88%).
MS (ESI+): $[M+H]^+$ 296.
$^1H$ NMR (300 MHz, $CDCl_3$) $\delta$ 1.33 (3H, t, J = 7.2 Hz), 2.71 (3H, s), 4.23 (2H, s), 4.29 (2H, q, J = 7.2 Hz), 7.16-7.23 (1H, m), 7.24-7.34 (3H, m).

Example 157-C)

2-(3-chlorobenzyl)-4-methyl-1,3-thiazole-5-carboxylic acid

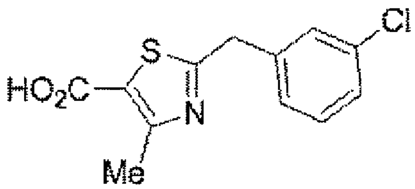

To a solution of ethyl 2-(3-chlorobenzyl)-4-methyl-1,3-thiazole-5-carboxylate (12.58 g, 42.53 mmol) obtained in Example 157-B) in ethanol (60 mL) was added 8M aqueous sodium hydroxide solution (10.63 mL), and the mixture was stirred for 3 hr. 1M Hydrochloric acid (88 mL) was added thereto in an ice bath. The precipitated white solid was collected by filtration, and washed twice with water (20 mL) to give the title compound (11.38 g) as a white solid (yield 93%).
MS (ESI+): $[M+H]^+$ 268.
$^1H$ NMR (300 MHz, DMSO-$d_6$) $\delta$ 2.58 (3H, s), 4.32 (2H, s), 7.12-7.61 (4H, m), 13.22 (1H, brs).

Example 157-D)

tert-butyl [2-(3-chlorobenzyl)-4-methyl-1,3-thiazol-5-yl]carbamate

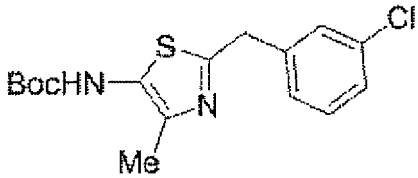

A mixture of 2-(3-chlorobenzyl)-4-methyl-1,3-thiazole-5-carboxylic acid (2.00 g, 7.47 mmol) obtained in Example 157-C), DPPA (2.41 mL, 11.20 mmol), triethylamine (3.11 mL, 22.41 mmol) and tert-butanol (15 mL) was stirred at 90°C for 3 hr. The reaction mixture was cooled to room temperature, and concentrated under reduced pressure. The obtained residue was purified by silica gel column chromatography [eluent: hexane-ethyl acetate (97:3-1:1)] to give the title compound (2.03 g) as a pale-yellow oil (yield 80%).
MS (ESI+): $[M+H]^+$ 339.

$^1$H NMR (300 MHz, $CDCl_3$) δ 1.49 (9H, s), 2.29 (3H, s), 4.16 (2H, s), 6.38 (1H, brs), 7.10-7.41 (4H, m).

Example 157-E)

N-[2-(3-chlorobenzyl)-4-methyl-1,3-thiazol-5-yl]-1-methyl-1H-pyrazole-5-carboxamide

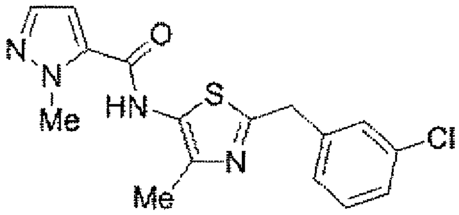

To a solution of tert-butyl [2-(3-chlorobenzyl)-4-methyl-1,3-thiazol-5-yl]carbamate (559 mg, 1.65 mmol) obtained in Example 157-D) in ethanol (0.9 mL) was added dropwise concentrated hydrochloric acid (3 mL) at 0°C, and the mixture was stirred at room temperature for 15 min. The reaction mixture was cooled to 0°C, neutralized with 8M aqueous sodium hydroxide solution (4.5 mL), adjusted to pH 10-11 with saturated aqueous sodium hydrogen carbonate solution, and extracted twice with ethyl acetate. The organic layer was washed with saturated brine, dried over anhydrous sodium sulfate, and concentrated under reduced pressure. The obtained residue was dissolved in DMA (5 mL), and 1-methyl-1H-pyrazole-5-carboxylic acid (250 mg, 1.98 mmol) obtained in Example 1-A), HATU (753 mg, 1.98 mmol) and DIEA (0.144 mL, 0.83 mmol) were added. The reaction mixture was stirred at 60°C overnight, water was added and the mixture was extracted twice with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-0:1)] and basic silica gel column chromatography [eluent: hexane-ethyl acetate (9:1-0:1)], and crystallized from ethyl acetate-hexane to give the title compound (182 mg) as colorless crystals (yield 32%).
MS (ESI+): $[M+H]^+$ 346.
$^1$H NMR (300 MHz, $CDCl_3$) δ 2.39 (3H, s), 4.17-4.23 (5H, m), 6.67 (1H, d, J = 1.9 Hz), 7.14-7.35 (4H, m), 7.52 (1H, d, J = 1.9 Hz), 7.70 (1H, brs).

Example 158

N-[2-(3-chloro-5-fluorobenzyl)-4-methyl-1,3-thiazol-5-yl]-1-methyl-3-nitro-1H-pyrazole-5-carboxamide

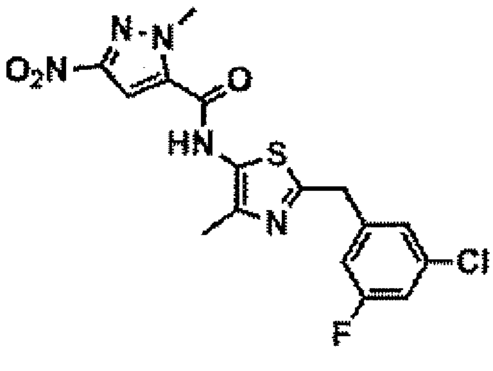

To a solution of tert-butyl [2-(3-chloro-5-fluorobenzyl)-4-methyl-1,3-thiazol-5-yl]carbamate (357 mg, 1.0 mmol) obtained in Example 145-E) in ethanol (1.4 mL) was added dropwise concentrated hydrochloric acid (0.7 mL) in a water bath, and the mixture was stirred at 50°C for 1.5 hr. The reaction mixture was cooled to 0°C, adjusted to pH 10-11 with 8M aqueous sodium hydroxide solution, and extracted twice with ethyl acetate. The organic layer was washed with saturated brine, dried over anhydrous sodium sulfate, and concentrated under reduced pressure. The obtained residue was dissolved in DMA (4 mL), and 1-methyl-3-nitro-1H-pyrazole-5-carboxylic acid (205 mg, 1.2 mmol), HATU (456 mg, 1.2 mmol) and DIEA (0.086 mL, 0.5 mmol) were added. The reaction mixture was stirred at 60°C for 4 hr, water was

added and the mixture was extracted twice with ethyl acetate. The organic layer was successively washed twice with saturated aqueous sodium hydrogen carbonate solution, water and saturated brine, dried over anhydrous sodium sulfate, and concentrated under reduced pressure. The obtained residue was purified by silica gel column chromatography [eluent: hexane-ethyl acetate (90:10-50:50)] and basic silica gel column chromatography [eluent: hexane-ethyl acetate (90:10-65:35)], and crystallized from ethyl acetate-hexane to give the title compound (171 mg) as white crystals (yield 42%).
MS (ESI+): $[M+H]^+$ 410.
$^1$H NMR (300 MHz, $CDCl_3$) δ 2.43 (3H, s), 4.21 (2H, s), 4.30 (3H, s), 6.90-6.97 (1H, m), 7.00 (1H, dt, J = 8.5, 2.1 Hz), 7.07-7.14 (1H, m), 7.31 (1H, s), 7.77 (1H, s).

Example 159

ethyl 5-{[2-(3-chloro-5-fluorobenzyl)-4-methyl-1,3-thiazol-5-yl]carbamoyl}-1-methyl-1H-pyrazole-3-carboxylate

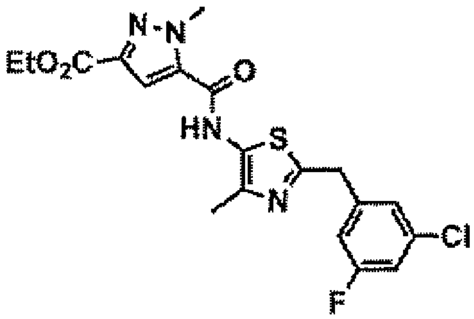

In the same manner as in Example 158, tert-butyl [2-(3-chloro-5-fluorobenzyl)-4-methyl-1,3-thiazol-5-yl]carbamate obtained in Example 145-E) and 3-(ethoxycarbonyl)-1-methyl-1H-pyrazole-5-carboxylic acid were used to give the title compound as white crystals (yield 62%).
MS (ESI+): $[M+H]^+$437.
$^1$H NMR (300 MHz, DMSO-$d_6$) δ 1.31 (3H, t, J = 7.2 Hz), 2.33 (3H, s), 4.13 (3H, s), 4.23-4.38 (4H, m), 7.16-7.27 (1H m), 7.27-7.39 (2H, m), 7.62 (1H, d, J = 1.4 Hz), 10.69 (1H, s).

Example 160

N-[2-(3,4-difluorobenzyl)-4-methyl-1,3-thiazol-5-yl]-1-methyl-1H-pyrazole-5-carboxamide

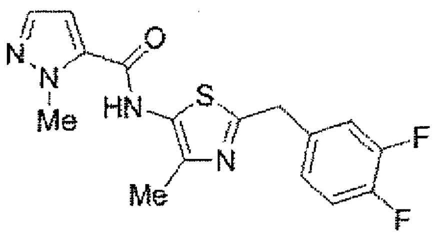

Example 160-A)

2-(3,4-difluorophenyl)ethanethioamide

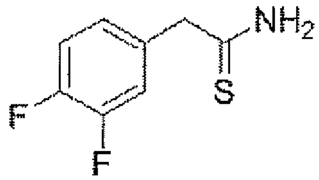

In the same manner as in Example 145-B), 3,4-difluorophenylacetonitrile was used to give the title compound (9.01 g) (yield 74%).
MS (ESI+): $[M+H]^+$188.
$^1$H NMR (300 MHz, $CDCl_3$) δ 4.03 (2H, s), 6.65 (1H, brs), 7.03 (1H, ddd, J = 6.2, 4.2, 2.1 Hz), 7.07-7.24 (2H, m), 7.56 (1H brs).

Example 160-B)

ethyl 2-(3,4-difluorobenzyl)-4-methyl-1,3-thiazole-5-carboxylate

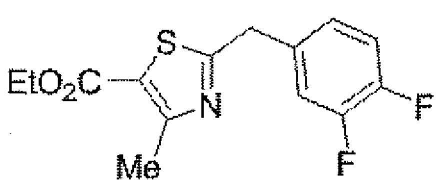

In the same manner as in Example 145-C), 2-(3,4-difluorophenyl)ethanethioamide obtained in Example 160-A) was used to give the title compound (11.93 g) (yield 83%).
MS (ESI+): $[M+H]^+$298.
$^1$H NMR (300 MHz, $CDCl_3$) δ 1.33 (3H, t, J = 7.2 Hz), 2.71 (3H, s), 4.21 (2H, s), 4.30 (2H, q, J = 7.2 Hz), 6.99-7.07 (1H, m), 7.06-7.20 (2H, m).

Example 160-C)

2-(3,4-difluorobenzyl)-4-methyl-1,3-thiazole-5-carboxylic acid

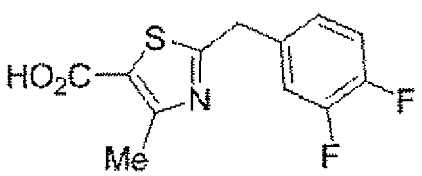

In the same manner as in Example 145-D), ethyl 2-(3,4-difluorobenzyl)-4-methyl-1,3-thiazole-5-carboxylate obtained in Example 160-B) was used to give the title compound (10.14 g) (yield 94%).
MS (ESI+): $[M+H]^+$270.
$^1$H NMR (300 MHz, DMSO-$d_6$) δ 2.58 (3H, s), 4.31 (2H, s), 7.21 (1H, ddd, J = 6.1, 4.1, 2.3 Hz), 7.33-7.53 (2H, m), 13.24 (1H, bars).

Example 160-D)

tert-butyl [2-(3,4-difluorobenzyl)-4-methyl-1,3-thiazol-5-yl]carbamate

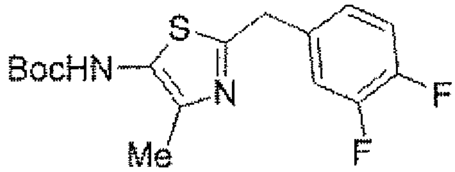

In the same manner as in Example 145-E), 2-(3,4-difluorobenzyl)-4-methyl-1,3-thiazole-5-carboxylic acid obtained in Example 160-C) was used to give the title compound (3.07 g) (yield 81%).
MS (ESI+): $[M+H]^+$341.
$^1$H NMR (300 MHz, $CDCl_3$) $\delta$ 1.49 (9H, s), 2.28 (3H, s), 4.13 (2H, s), 6.49 (1H, brs), 6.96-7.04 (1H, m), 7.04-7.17 (2H, m).

Example 160-E)

N-[2-(3,4-difluorobenzyl)-4-methyl-1,3-thiazol-5-yl]-1-methyl-1H-pyrazole-5-carboxamide

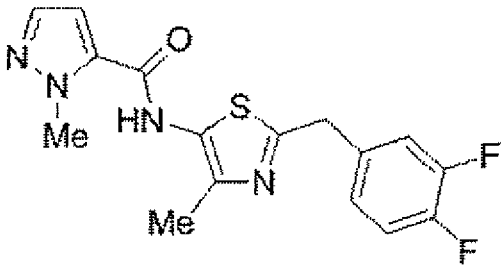

In the same manner as in Example 145-F), tert-butyl [2-(3,4-difluorobenzyl)-4-methyl-1,3-thiazol-5-yl]carbamate obtained in Example 160-D) and 1-methyl-1H-pyrazole-5-carboxylic acid were used to give the title compound (193 mg) (yield 28%) .
MS (ESI+): $[M+H]^+$349.
$^1$H NMR (300 MHz, $CDCl_3$) $\delta$ 2.39 (3H, s), 4.19 (2H, s), 4.20 (3H, s), 6.67 (1H, d, J = 2.3Hz), 7.00-7.07 (1H, m), 7.07-7.20 (2H, m), 7.52 (1H, d, J = 2.3Hz), 7.70 (1H, brs).

Example 161

N-[2-(3-chloro-5-fluorobenzyl)-4-methyl-1,3-thiazol-5-yl]-1-(2-methoxyethyl)-1-pyrazole-5-carboxamide

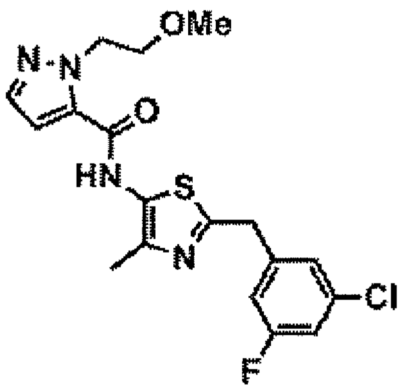

In the same manner as in Example 158, tert-butyl [2-(3-chloro-5-fluorobenzyl)-4-methyl-1,3-thiazol-5-yl]carbamate obtained in Example 145-E) and 1-(2-methoxyethyl)-1H-pyrazole-5-carboxylic acid were used to give the title compound as white crystals (yield 45%).

MS (ESI+) : $[M+H]^+$ 409.
$^1$H NMR (300 MHz, $CDCl_3$) $\delta$ 2.43 (3H, s), 3.40 (3H, s), 3.93 (2H, t, J = 4.9 Hz), 4.19 (2H, s), 4.64 (2H, t, J = 4.9 Hz), 6.76 (1H, d, J = 2.2 Hz), 6.89-7.03 (2H, m), 7.07-7.15 (1H, m), 7.57 (1H, d, J = 2.2 Hz), 9.31 (1H, s).

Example 162

N-[2-(3-chloro-5-fluorobenzyl)-4-methyl-1,3-thiazol-5-yl]-1-(2-methylpropyl)-1H-pyrazole-5-carboxamide

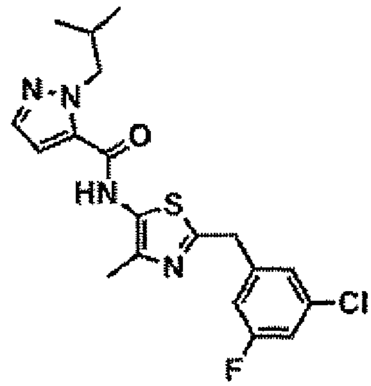

In the same manner as in Example 158, tert-butyl [2-(3-chloro-5-fluorobenzyl)-4-methyl-1,3-thiazol-5-yl]carbamate obtained in Example 145-E) and 1-(2-methylpropyl)-1H-pyrazole-5-carboxylic acid were used to give the title compound as white crystals (yield 44%).
MS (ESI+): $[M+H]^+$ 407.
$^1$H NMR (300 MHz, $COCl_3$) $\delta$ 0.89 (6H, d, J = 6. 9 Hz), 2.17-2.32 (1H, m), 2.40 (3H, s), 4.20 (2H, s), 4.41 (2H, d, J = 7.4 Hz), 6.66 (1H, d, J = 2.2 Hz), 6.89-6.96 (1H, m), 6.99 (1H, dt, J = 8.5, 2.1 Hz), 7.10 (1H, s), 7.54 (1H, d, J = 1.9 Hz), 7.69 (1H, bars).

Example 163

N-[2-(3-chloro-5-fluorobenzyl)-4-methyl-1,3-thiazol-5-yl]-1-(2,2,2-trifluoroethyl)-1H-pyrazole-5-carboxamide

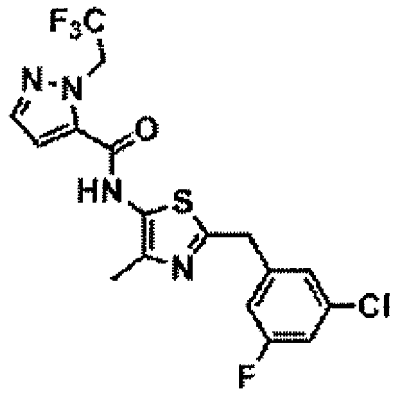

In the same manner as in Example 158, tert-butyl [2-(3-chloro-5-fluorobenzyl)-4-methyl-1,3-thiazol-5-yl]carbamate obtained in Example 145-E) and 1-(2,2,2-trifluoroethyl)-1H-pyrazole-5-carboxylic acid were used to give the title compound as white crystals (yield 45%).
MS (ESI+): $[M+H]^+$ 433.
$^1$H NMR (300 MHz, $CDCl_3$) $\delta$ 2.41 (3H, s), 4.20 (2H, s), 5.34 (2H, q, J = 8.2 Hz), 6.77 (1H, d, J = 1.9 Hz), 6.90-6.97 (1H, m), 6.97-7.03 (1H, m), 7.08-7.13 (1H, m), 7.68 (1H, d, J = 2.2 Hz), 7.71 (1H, s).

Example 164

$N^5$-[2-(3-chloro-5-fluorobenzyl)-4-methyl-1,3-thiazol-5-yl]-1-methyl-1H-pyrazole-3,5-dicarboxamide

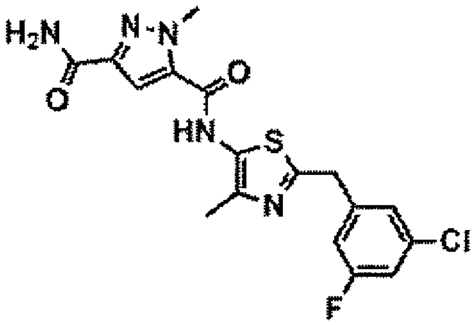

To a solution of ethyl 5-{[2-(3-chloro-5-fluorobenzyl)-4-methyl-1,3-thiazol-5-yl]carbamoyl}-1-methyl-1H-pyrazole-3-carboxylate (475 mg, 1.087 mmol) obtained in Example 159 in methanol (3.2 mL)-THF (6.4 mL) was added 2M aqueous sodium hydroxide solution (1.63 mL, 3.26 mmol) at room temperature, and the mixture was stirred at room temperature overnight. The reaction mixture was neutralized with 1M hydrochloric acid, extracted with ethyl acetate, and washed with saturated brine. The organic layer was dried over anhydrous sodium sulfate and concentrated under reduced pressure to give a crudely purified product of 5-{[2-(3-chloro-5-fluorobenzyl)-4-methyl-1,3-thiazol-5-yl]carbamoyl}-1-methyl-1H-pyrazole-3-carboxylic acid as a pale-yellow solid (467 mg). To a solution of a part (150 mg) of the crudely purified product of the obtained 5-{[2-(3-chloro-5-fluorobenzyl)-4-methyl-1,3-thiazol-5-yl]carbamoyl}-1-methyl-1H-pyrazole-3-carboxylic acid in DMF (3.0 mL) were added EDCI·HCl (84 mg, 0.44 mmol) and HOBt (60 mg, 0.44 mmol), and the mixture was stirred at room temperature for 2 hr. To the reaction mixture was added 28% aqueous ammonia solution (2.0 mL), and the mixture was stirred at room temperature overnight. The reaction mixture was diluted with saturated aqueous sodium hydrogen carbonate solution, and extracted with ethyl acetate. The organic layer was washed with saturated aqueous ammonium chloride solution, water and saturated brine, dried over anhydrous sodium sulfate, and evaporated under reduced pressure. The residue was purified by silica gel column chromatography [eluent: ethyl acetate-methanol (100:0-80:20)], crystallized from ethanol-hexane to give the title compound (15 mg) as white crystals (yield 11%).

MS (ESI): $[M+H]^+$ 408.

$^1H$ NMR (300 MHz, DMSO-$d_6$) 5 2.32 (3H, s), 4.09 (3H, s), 4.25 (2H, s), 7.15-7.25 (1H, m), 7.25-7.40 (3H, m), 7.45 (1H, s), 7.63 (1H, s), 10.66 (1H, s).

Example 165

$N^5$-[2-(3-chloro-5-fluorobenzyl)-4-methyl-1,3-thiazol-5-yl]-$N^3$-(2-hydroxyethyl)-1-methyl-1H-pyrazole-3,5-dicarboxamide

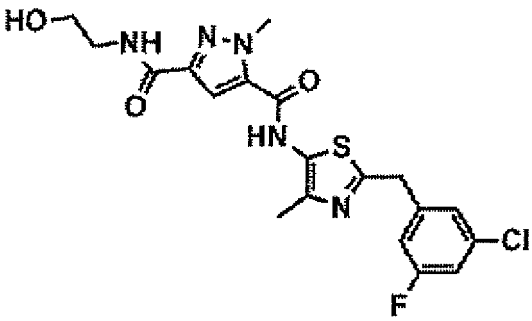

To a solution of ethyl 5-{[2-(3-chloro-5-fluorobenzyl)-4-methyl-1,3-thiazol-5-yl]carbamoyl}-1-methyl-1H-pyrazole-3-carboxylate (475 mg, 1.087 mmol) obtained in Example 159 in methanol (3.2 mL)-THF (6.4 mL) was added 2M aqueous sodium hydroxide solution (1.63 mL, 3.26 mmol) at room temperature, and the mixture was stirred at room temperature overnight. The reaction mixture was neutralized with 1M hydrochloric acid, extracted with ethyl acetate, and washed with saturated brine. The organic layer was dried over anhydrous sodium sulfate and concentrated under reduced pressure to give a crudely purified product of 5-{[2-(3-chloro-5-fluorobenzyl)-4-methyl-1,3-thiazol-5-yl]carbamoyl}-1-methyl-1H-pyrazole-3-carboxylic acid as a pale-yellow solid (467 mg). To a solution of a part (150 mg) of the crudely purified product of the obtained 5-{[2-(3-chloro-5-fluorobenzyl)-4-methyl-1,3-thiazol-5-yl]carbamoyl}-1-methyl-1H-pyrazole-3-carboxylic acid in DMF (3.0 mL) were added EDCI·HCl (84 mg, 0.44 mmol), HOBt (60 mg, 0.44 mmol) and 2-aminoethanol (0.019 mL, 0.44 mmol), and the mixture was stirred at room temperature overnight. The reaction mixture was diluted with saturated aqueous sodium hydrogen carbonate solution, and the mixture was extracted with ethyl acetate. The organic layer was washed with water and saturated brine, dried over anhydrous sodium sulfate, and evaporated under reduced pressure. The obtained residue was purified by silica gel column chromatography [eluent: ethyl acetate-methanol (100:0-95:5)] and basic silica gel column chromatography [eluent: ethyl acetate-methanol (95:5-90:10)], and crystallized from ethyl acetate-hexane to give the title compound (52 mg) as white crystals (yield 33%).
MS (ESI+): $[M+H]^+$ 452.
$^1$H NMR (300 MHz, DMSO-$d_6$) $\delta$ 2.32 (3H, s), 3.23-3.33 (2H, m), 3.47 (2H, q, J = 5.9 Hz), 4.10 (3H, s), 4.25 (2H,s), 4.73 (1H, t, J = 5.5 Hz), 7.16-7.24 (1H, m), 7.28 (1H, s), 7.33 (1H, dt, J = 8.9, 2.0 Hz), 7.49 (1H, s), 8.09 (1H, t, J = 5.8 Hz), 10.65 (1H, s).

Example 166

N-[2-(3-chloro-5-fluorobenzyl)-4-methyl-1,3-thiazol-5-yl]-3-(hydroxymethyl)-1-methyl-1H-pyrazole-5-carboxamide

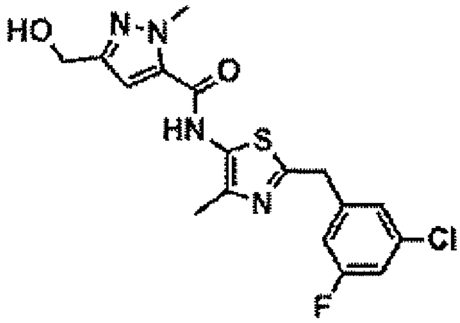

To a solution of ethyl 5-{[2-(3-chloro-5-fluorobenzyl)-4-methyl-1,3-thiazol-5-yl]carbamoyl}-1-methyl-1H-pyrazole-3-carboxylate (475 mg, 1.087 mmol) obtained in Example 159 in methanol (3.2 mL)-THF (6.4 mL) was added 2M aqueous sodium hydroxide solution (1.63 mL, 3.26 mmol) at room temperature, and the mixture was stirred at room temperature overnight. The reaction mixture was neutralized with 1M hydrochloric acid, extracted with ethyl acetate, and washed with saturated brine. The organic layer was dried over anhydrous sodium sulfate and concentrated under reduced pressure to give a crudely purified product of 5-{[2-(3-chloro-5-fluorobenzyl)-4-methyl-1,3-thiazol-5-yl]carbamoyl}-1-methyl-1H-pyrazole-3-carboxylic acid as a pale-yellow solid (467 mg). To a solution of a part (152 mg) of the crudely purified product of the obtained 5-{[2-(3-chloro-5-fluorobenzyl)-4-methyl-1,3-thiazol-5-yl]carbamoyl}-1-methyl-1H-pyrazole-3-carboxylic acid in THF (5.0 mL) were added triethylamine (0.062 mL, 0.446 mmol) and 2-methylpropyl chlorocarbonate (0.056 mL, 0.409 mmol), and the mixture was stirred at room temperature for 15 min. The reaction mixture was filtered, and the residue was washed with THF (5.0 mL). To the combined filtrate was added sodium borohydride (28 mg, 0.744 mmol) while suspending in water (0.5 mL), and the mixture was stirred at room temperature for 30 min. The reaction mixture was diluted with saturated aqueous ammonium chloride solution, and the mixture was extracted with ethyl acetate. The organic layer was washed with saturated brine, dried over anhydrous sodium sulfate, and evaporated under reduced pressure. The obtained residue was purified by silica gel column chromatography [eluent: ethyl acetate-methanol (100:0-95:5)], and crystallized from ethyl acetate-hexane to give the title compound (46 mg) as white crystals (yield 33%).
MS (ESI+): $[M+H]^+$ 395.
$^1$H NMR (300 MHz, DMSO-$d_6$) $\delta$ 2.30 (3H, s), 3.99 (3H, s), 4.25 (2H, s), 4.41 (2H, d, J = 5.5 Hz), 5.14-5.21 (1H, m), 7.02 (1H, s), 7.16-7.23 (1H, m), 7.28 (1H, s), 7.29-7.37 (1H, m), 10.53 (1H, s).

Example 167

N-[2-(3-chloro-5-fluorobenzyl)-4-methyl-1,3-thiazol-5-yl]-1-methyl-6-oxo-1,6-dihydropyridine-3-carboxamide

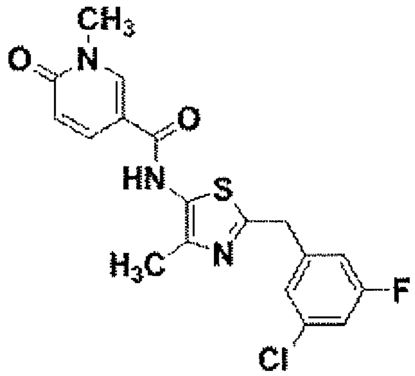

To a solution of tert-butyl [2-(3-chloro-5-fluorobenzyl)-4-methyl-1,3-thiazol-5-yl]carbamate (500 mg, 1.4 mmol) obtained in Example 145-E) in ethanol (2 mL) was added concentrated hydrochloric acid (1 mL), and the mixture was stirred at 50°C for 3 hr. To the reaction mixture was added saturated aqueous sodium hydrogen carbonate solution, and the mixture was extracted with ethyl acetate. The organic layer was washed with saturated brine, dried over anhydrous sodium sulfate, and concentrated under reduced pressure. The obtained residue was dissolved in DMF (3 mL), and 1-methyl-6-oxo-1,6-dihydropyridine-3-carboxylic acid (257 mg, 1.68 mmol), HATU (638 mg, 1.68 mmol) and DIEA (0.144 mL, 0.84 mmol) were added. The reaction mixture was stirred at 60°C for 6 hr, diluted with ethyl acetate, and washed with saturated aqueous sodium hydrogen carbonate solution, water and saturated brine. The organic layer was dried over anhydrous sodium sulfate and concentrated under reduced pressure. The obtained residue was purified by silica gel column chromatography [eluent: ethyl acetate-methanol]. The obtained resultant product was crystallized from ethyl acetate-heptane to give the title compound (240 mg) as a pale-brown solid (yield 44%).
MS (ESI+): $[M+H]^+$ 392.
$^1$H NMR (300MHz, $CDCl_3$) $\delta$2.41 (3H, s), 3.63 (3H, s), 4.19 (2H, s), 6.59 (1H, d, J = 9.5 Hz), 6.87-7.05 (2H, m), 7.10 (1H, s), 7.67 (1H, dd, J = 9.5, 2.7 Hz), 7.79 (1H, s), 8.22 (1H, d, J = 2.7 Hz).

Example 168

N-[2-(3-chloro-5-fluorobenzyl)-4-methyl-1,3-thiazol-5-yl]-1-phenyl-1H-pyrazole-5-carboxamide

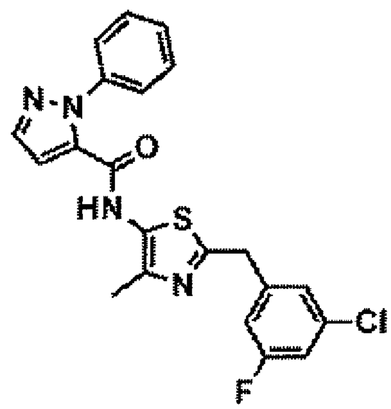

In the same manner as in Example 158, tert-butyl [2-(3-chloro-5-fluorobenzyl)-4-methyl-1,3-thiazol-5-yl]carbamate obtained in Example 145-E) and 1-phenyl-1H-pyrazole-5-carboxylic acid were used to give the title compound as white crystals (yield 33%).
MS (ESI+): $[M+H]^+$ 427.

$^1$H NMR (300 MHz, DMSO-$d_6$) δ 2.23 (3H, s), 4.21 (2H, s), 7.01-7.09 (1H, m), 7.13-7.23 (1H, m), 7.25 (1H, s), 7.27-7.35 (1H, m), 7.34-7.52 (5H, m), 7.80 (1H, d, J = 1.9 Hz), 10.92 (1H, s).

Example 169

N-[2-(3-chloro-5-fluorobenzyl)-4-methyl-1,3-thiazol-5-yl]-1-methyl-4-nitro-1H-imidazole-2-carboxamide

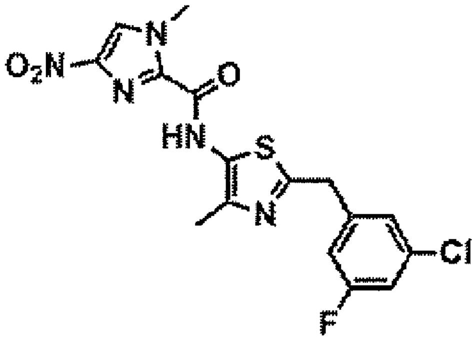

Example 169-A)

1-methyl-4-nitro-1H-imidazole-2-carboxylic acid

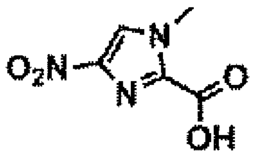

To a solution of ethyl 1-methyl-4-nitro-1H-imidazole-2-carboxylate (500 mg, 2.20 mmol) in methanol (7.5 mL)-THF (15 mL) was added 2M aqueous sodium hydroxide solution (3.8 mL, 7.53 mmol) at room temperature. The mixture was stirred for 3 hr at 40°C, neutralized with 1M hydrochloric acid, extracted with ethyl acetate, and the extract was washed with saturated brine. The organic layer was dried over anhydrous sodium sulfate and concentrated under reduced pressure to give the title compound (200 mg) as a brown solid (yield 46%). $^1$H NMR (300 MHz, DMSO-$d_6$) δ 3.96 (3H, s), 8.58 (1H, s).
* The peak of "COOH group" was not observed.

Example 169-B)

N-[2-(3-chloro-5-fluorobenzyl)-4-methyl-1,3-thiazol-5-yl]-1-methyl-4-nitro-1H-imidazole-2-carboxamide

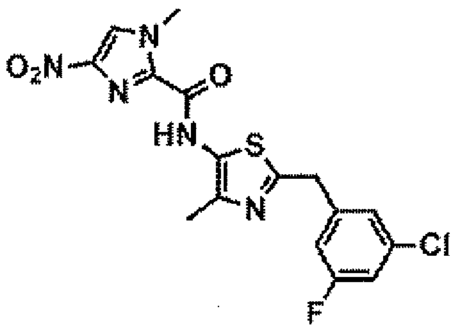

In the same manner as in Example 158, tert-butyl [2-(3-chloro-5-fluorobenzyl)-4-methyl-1,3-thiazol-5-yl]carbamate obtained in Example 145-E) and 1-methyl-4-nitro-1H-imidazole-2-carboxylic acid obtained in Example 169-A) were used to give the title compound as yellow crystals (yield 37%).
MS (ESI+): $[M+H]^+$ 410.
$^1$H NMR (300 MHz, DMSO-$d_6$) δ 2.29 (3H, s), 4.00 (3H, s), 4.26 (2H, s), 7.17-7.27 (1H, s), 7.30 (1H, s), 7.31-7.39 (1H, m), 8.65 (1H, s), 10.98 (1H, s).

Example 170

N-[2-(3-chloro-5-fluorobenzyl)-4-methyl-1,3-thiazol-5-yl]-1,5-dimethyl-1H-pyrazole-4-carboxamide

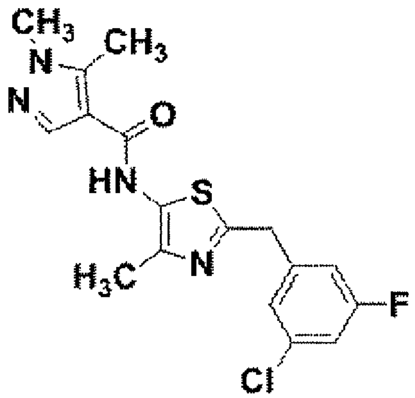

To a solution of tert-butyl [2-(3-chloro-5-fluorobenzyl)-4-methyl-1,3-thiazol-5-yl]carbamate (1.07 g, 3.0 mmol) obtained in Example 145-E) in ethanol (4 mL) was added concentrated hydrochloric acid (2 mL), and the mixture was stirred at 50°C for 3 hr. To the reaction mixture was added saturated aqueous sodium hydrogen carbonate solution, and the mixture was extracted with ethyl acetate. The organic layer was washed with saturated brine, dried over anhydrous sodium sulfate, and concentrated under reduced pressure. The obtained residue was dissolved in DMF (6 mL), and 1,5-dimethyl-1H-pyrazole-4-carboxylic acid (462 mg, 3.3 mmol), HATU (1.25 g, 3.3 mmol) and DIEA (0.258 mL, 1.5 mmol) were added. The reaction mixture was stirred at 60°C overnight, diluted with ethyl acetate, and washed with saturated aqueous sodium hydrogen carbonate solution, water and saturated brine. The organic layer was dried over anhydrous sodium sulfate and concentrated under reduced pressure. The obtained residue was purified by silica gel column chromatography [eluent: ethyl acetate-methanol]. The obtained resultant product was crystallized from ethyl acetate-heptane to give the title compound (295 mg) as a pale-brown solid (yield 26%). $^1$H NMR (300MHz, $CDCl_3$) δ2.40 (3H, s), 2.59 (3H, s), 3.83 (3H, s), 4.18 (2H, s), 6.89-7.01 (2H, m), 7.08-7.13 (1H, m), 7.49 (1H, s), 7.72 (1H, s).
MS (ESI+): $[M+H]^+$ 379.

Example 171

N-[2-(3-chloro-5-fluorobenzyl)-4-methyl-1,3-thiazol-5-yl]-1-methyl-1H-pyrazole-4-carboxamide

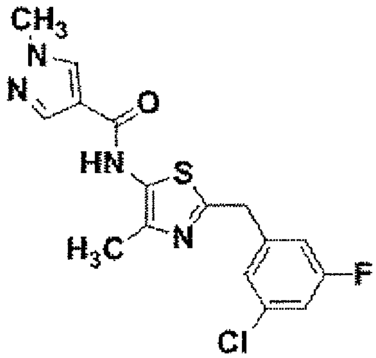

To a solution of tert-butyl [2-(3-chloro-5-fluorobenzyl)-4-methyl-1,3-thiazol-5-yl]carbamate (1.07 g, 3.0 mmol) obtained in Example 145-E) in ethanol (4 mL) was added concentrated hydrochloric acid (2 mL), and the mixture was stirred at 50°C for 3 hr. To the reaction mixture was added saturated aqueous sodium hydrogen carbonate solution, and the mixture was extracted with ethyl acetate. The organic layer was washed with saturated brine, dried over anhydrous sodium sulfate, and concentrated under reduced pressure. The obtained residue was dissolved in DMF (6 mL), and 1-methyl-1H-pyrazole-4-carboxylic acid (416 mg, 3.3 mmol), HATU (1.25 g, 3.3 mmol) and DIEA (0.258 mL, 1.5 mmol) were added. The reaction mixture was stirred at 60°C overnight, diluted with ethyl acetate, and washed with saturated aqueous sodium hydrogen carbonate solution, water and saturated brine. The organic layer was dried over anhydrous sodium sulfate and concentrated under reduced pressure. The obtained residue was purified by silica gel column chromatography [eluent: ethyl acetate-methanol]. The obtained resultant product was crystallized from ethyl acetate-heptane to give the title compound (364 mg) as a pale-brown solid (yield 33%).
MS (ESI+): $[M+H]^+$ 365.
$^1H$ NMR (300MHz, $CDCl_3$) δ2.40 (3H, s), 3.97 (3H, s), 4.18 (2H, s), 6.90-7.02 (2H, m), 7.08-7.12 (1H, m), 7.49 (1H, brs), 7.83 (1H, s), 7.93 (1H, s).

Example 172

N-[2-(3-chloro-5-fluorobenzyl)-4-methyl-1,3-thiazol-5-yl]-1-(methylsulfonyl)cyclopropanecarboxamide

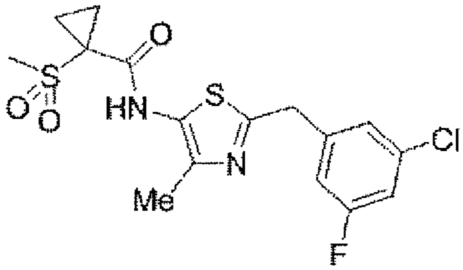

Example 172-A)

ethyl 1-(methylsulfonyl)cyclopropanecarboxylate

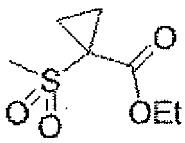

To a solution of ethyl (methylsulfonyl)acetate (3.0 g, 18 mmol), potassium carbonate (4.98 g, 36 mmol), 18-crown 6-ether (478 mg, 1.8 mmol) and tetrabutylammonium bromide (439 mg, 1.8 mmol) in THF (36 mL) was added

1,2-dibromoethane (1.71 mL, 20 mmol), and the mixture was stirred at 65°C for 1 day. The reaction mixture was cooled to room temperature, insoluble materials were filtered off, and the filtrate was concentrated under reduced pressure. The obtained residue was purified by silica gel column chromatography [eluent: hexane-ethyl acetate (97:3-2:3)] to give the title compound (2.65 g) as a colorless oil (yield 76%).
MS (ESI+) : $[M+H]^+$ 193.
$^1H$ NMR (300 MHz, $CDCl_3$) $\delta$1.32 (3H, t, J = 7.2Hz), 1.63-1.70 (2H, m), 1.76-1.83 (2H, m), 3.21 (3H, s), 4.27 (2H, q, J = 7.2Hz).

Example 172-B)

1-(methylsulfonyl)cyclopropanecarboxylic acid

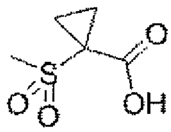

To a solution of ethyl 1-(methylsulfonyl)cyclopropanecarboxylate (0.95 g, 4.94 mmol) obtained in Example 172-A) in methanol (5 mL) was added a solution (28%, 0.60 mL, 5.44 mmol) of sodium methoxide in methanol at 0°C, and the mixture was stirred at room temperature for 30 min. A solution (28%, 1.2 mL, 10.88 mmol) of sodium methoxide in methanol was added, and the mixture was stirred for 2.5 hr. The mixture was neutralized with Dowex 50W-X8 ($H^+$ form), the resin was filtered off, and washed with methanol. The filtrate was concentrated under reduced pressure to give the title compound (0.74 g) as a colorless solid (yield 91%).
$^1H$ NMR (300 MHz, $CDCl_3$) $\delta$ 1.72-1.79 (2H, m), 1.85-1.94 (2H, m), 3.21 (3H, s).
* The peak of "COOH group" was not observed.

Example 172-C)

N-[2-(3-chloro-5-fluorobenzyl)-4-methyl-1,3-thiazol-5-yl]-1-(methylsulfonyl)cyclopropanecarboxamide

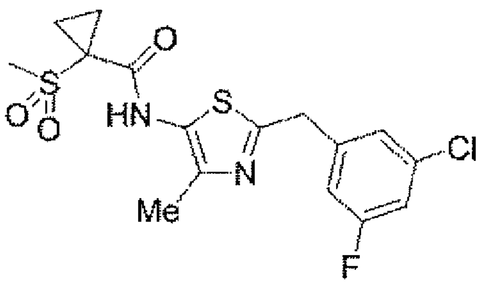

In the same manner as in Example 145-F), tert-butyl [2-(3-chloro-5-fluorobenzyl)-4-methyl-1,3-thiazol-5-yl] carbamate obtained in Example 145-E) and 1-(methylsulfonyl)cyclopropanecarboxylic acid obtained in Example 172-B) were used to give the title compound (704 mg) (yield 58%).
MS (ESI+): $[M+H]^+$ 403.
$^1H$ NMR (300 MHz, $CDCl_3$) $\delta$1.77-1.85 (4H, m), 2.40 (3H, s), 3.09 (3H, s), 4.17 (2H, s), 6.91 (1H, d, J = 9.1Hz), 6.98 (1H, dt, J = 8.4, 2.2Hz), 7.08 (1H, s), 10.08 (1H, s).

Example 173

methyl 1-{[2-(3-chloro-5-fluorobenzyl)-4-methyl-1,3-thiazol-5-yl]carbamoyl}cyclopropanecarboxylate

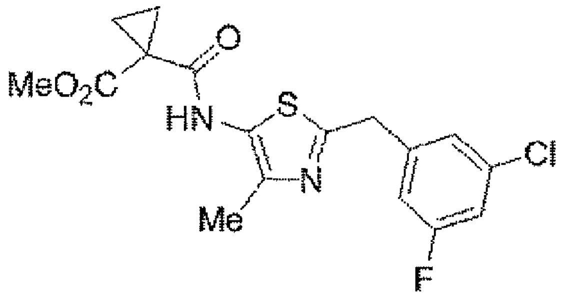

In the same manner as in Example 145-F), tert-butyl [2-(3-chloro-5-fluorobenzyl)-4-methyl-1,3-thiazol-5-yl] carbamate obtained in Example 145-E) and 1-(methoxycarbonyl)cyclopropanecarboxylic acid were used to give the title compound (3.15 g) (yield 57%).
MS (ESI+) : $[M+H]^+$ 383.
$^1H$ NMR (300 MHz, $CDCl_3$) δ1.66-1.76 (2H, m), 1.80-1.88 (2H, m), 2.45 (3H, s), 3.76 (3H, s), 4.16 (2H, s), 6.88-6.99 (2H, m), 7.08 (1H, s), 11.40 (1H, s).

Example 174

1-{[2-(3-chloro-5-fluorobenzyl)-4-methyl-1,3-thiazol-5-yl]carbamoyl}cyclopropanecarboxylic acid

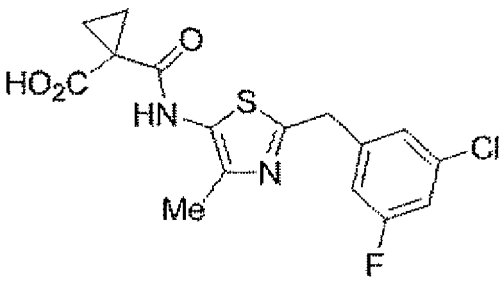

To a solution of methyl 1-{[2-(3-chloro-5-fluorobenzyl)-4-methyl-1,3-thiazol-5-yl]carbamoyl}cyclopropanecarboxylate (2.05 g, 5.35 mmol) obtained in Example 173 in ethanol (8 mL)-THF (13 mL) was added 8M aqueous sodium hydroxide solution (1.34 mL), and the mixture was stirred at room temperature for 3 hr. The reaction mixture was neutralized with 1N hydrochloric acid, and extracted twice with ethyl acetate, the extract was washed with saturated brine, and dried over anhydrous magnesium sulfate. The obtained residue was crystallized from hexane-ethyl acetate to give the title compound (1.91 g) as a white solid (yield 97%).
MS (ESI+): $[M+H]^+$ 369.
$^1H$ NMR (300 MHz, DMSO-$d_6$) δ1.40-1.56 (4H, m), 2.30 (3H, s), 4.22 (2H, s), 7.18 (1H, dt, J = 9.5, 1.7Hz), 7.26 (1H, s), 7.32 (1H, dt, J = 8.9, 2.0Hz), 11.34 (1H, brs).
* The peak of "COOH group" was not observed.

Example 175

N-[2-(3-chloro-5-fluorobenzyl)-4-methyl-1,3-thiazol-5-yl]-N'-(2-methoxyethyl)cyclopropane-1,1-dicarboxamide

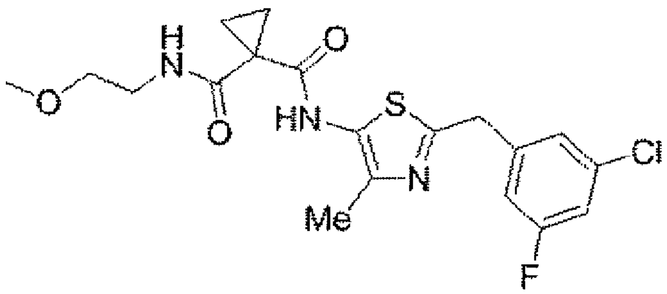

To a solution of 1-{[2-(3-chloro-5-fluorobenzyl)-4-methyl-1,3-thiazol-5-yl]carbamoyl}cyclopropanecarboxylic acid (500 mg, 1.35 mmol) obtained in Example 174 in DMF (3 mL) were added 2-methoxyethylamine (0.142 mL, 1.63 mmol), HATU (620 mg, 1.63 mmol) and DIEA (0.118mL, 0.67 mmol), and the mixture was stirred at room temperature overnight. The reaction mixture was diluted with ethyl acetate, and washed with saturated aqueous sodium hydrogen carbonate solution and saturated brine. The organic layer was dried over anhydrous sodium sulfate, concentrated under reduced pressure and the obtained residue was purified by silica gel column chromatography [eluent: hexane-ethyl acetate (90:10-0:100)] and basic silica gel column chromatography [eluent: eluent: hexane-ethyl acetate (90:10-0:100)]. The obtained solid was recrystallized from ethyl acetate-hexane to give the title compound (229 mg) as a colorless solid (yield 38%).
MS (ESI+): $[M+H]^+$ 426.
$^1H$ NMR (300 MHz, $CDCl_3$) $\delta$1.30-1.39 (2H, m), 1.80-1.89 (2H, m), 2.44 (3H, s), 3.36 (3H, s), 3.46 (4H, d, J = 2.3 Hz), 4.16 (2H, s), 5.77 (1H, brs), 6.87-7.00 (2H, m), 7.08 (1H, s), 12.07 (1H, s).

Example 176

4-(acetylamino)-N-[2-(3-chloro-5-fluorobenzyl)-4-methyl-1,3-thiazol-5-yl]-1-methyl-1H-imidazole-2-carboxamide

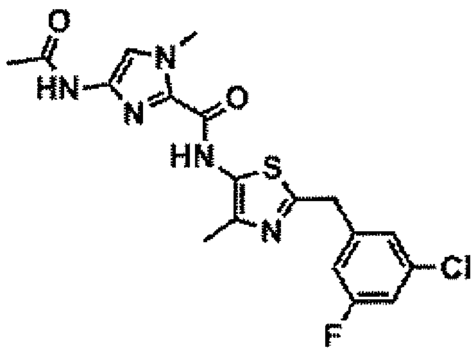

To a solution of N-[2-(3-chloro-5-fluorobenzyl)-4-methyl-1,3-thiazol-5-yl]-1-methyl-4-nitro-1H-imidazole-2-carboxamide (87 mg, 0.212 mmol) obtained in Example 169-B) in ethanol (1.8 mL)-water (0.2 mL) were added iron (118 mg, 2.12 mmol) and ammonium chloride (6 mg, 0.106 mmol), and the mixture was heated under reflux for 6.5 hr. After cooling to room temperature, the reaction mixture was filtered. The filtrate was concentrated under reduced pressure, and ethyl acetate and water were added to the residue. The organic layer was washed with saturated brine, dried over anhydrous sodium sulfate and concentrated under reduced pressure to give a crudely purified product (90 mg) of 4-amino-N-[2-(3-chloro-5-fluorobenzyl)-4-methyl-1,3-thiazol-5-yl]-1-methyl-1H-imidazole-2-carboxamide as a brown oil. To a solution of the crudely purified product (90 mg) of the obtained 4-amino-N-[2-(3-chloro-5-fluorobenzyl)-4-methyl-1,3-thiazol-5-yl]-1-methyl-1H-imidazole-2-carboxamide in THF (2.0 mL) were added acetyl chloride (0.017 mL, 0.233 mmol) and triethylamine (0.036 mL, 0.254 mmol), and the mixture was stirred at room temperature overnight. To the reaction mixture was added saturated aqueous sodium hydrogen carbonate solution, and the mixture was extracted with ethyl acetate. The organic layer was washed with saturated brine, dried over anhydrous sodium sulfate, and concentrated under reduced pressure. The obtained residue was purified by silica gel column chromatography [eluent: ethyl acetate-methanol (100:0-97:3)] and basic silica gel column chromatography [eluent: ethyl acetate-methanol (100:0-97:3)], and crystallized from ethyl acetate-hexane to give the title compound (40 mg) as white crystals (yield 45%).

MS (ESI+) : $[M+H]^+$ 422.
$^1H$ NMR (300 MHz, DMSO-$d_6$) $\delta$ 2.02 (3H, s), 2.30 (3H, s), 3.95 (3H, s), 4.25 (2H, s), 7.14-7.41 (4H, m), 10.59 (2H, d, J = 16.2 Hz).

Example 177

N-[2-(3-chloro-5-fluorobenzyl)-4-methyl-1,3-thiazol-5-yl]-3-ethyl-1-methyl-1H-pyrazole-5-carboxamide

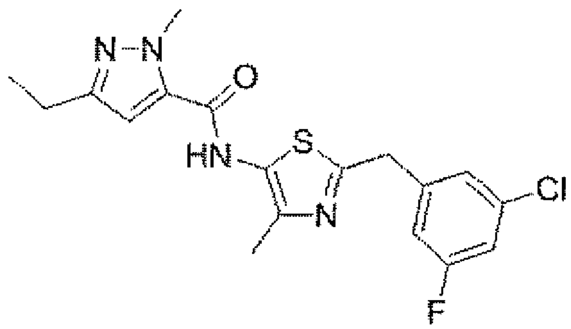

To a solution of tert-butyl [2-(3-chloro-5-fluorobenzyl)-4-methyl-1,3-thiazol-5-yl]carbamate (300 mg, 0.84 mmol) obtained in Example 145-E) in ethanol (1.5 mL) was added dropwise concentrated hydrochloric acid (0.6 mL) at 0°C, and the mixture was stirred at 50°C for 2.5 hr. The reaction mixture was cooled to 0°C, neutralized with 8N aqueous sodium hydroxide solution (0.8 mL), adjusted to pH 10-11 with saturated aqueous sodium hydrogen carbonate solution (150 mL), and extracted with ethyl acetate. The organic layer was washed with saturated brine, dried over anhydrous magnesium sulfate, and concentrated under reduced pressure. The obtained residue was dissolved in DMA (2 mL), and 3-ethyl-1-methyl-1H-pyrazole-5-carboxylic acid (156 mg, 1.01 mmol), HATU (384 mg, 1.01 mmol) and DIEA (0.07 mL, 0.42 mmol) were added. The reaction mixture was stirred at 60°C for 1 hr, water was added and the mixture was extracted with ethyl acetate. The organic layer was washed successively with saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (13:7-1:3)] and basic silica gel column chromatography [eluent: hexane-ethyl acetate (13:7-1:3)], and crystallized from ethyl acetate-hexane to give the title compound (107 mg) as colorless crystals (yield 32%).
MS (ESI+): $[M+H]^+$ 393.
$^1H$ NMR (300 MHz, DMSO-$d_6$) $\delta$ 1.19 (3H, t, J = 7.5 Hz), 2.31 (3H, s), 2.57 (2H, q, J = 7.5 Hz), 3.98 (3H, s), 4.26 (2H, s), 6.88 (1H, s), 7.18-7.25 (1H, m), 7.27-7.31 (1H, m), 7.31-7.38 (1H, m), 10.47 (1H, s).

Example 178

N-[2-(3-chloro-5-fluorobenzyl)-4-methyl-1,3-thiazol-5-yl]-3-methyl-1-(2,2,2-trifluoroethyl)-1H-pyrazole-5-carboxamide

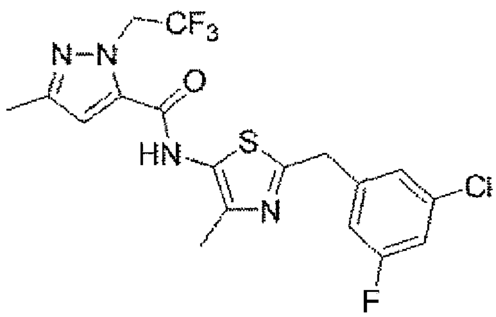

To a solution of tert-butyl [2-(3-chloro-5-fluorobenzyl)-4-methyl-1,3-thiazol-5-yl]carbamate (300 mg, 0.84 mmol)

obtained in Example 145-E) in ethanol (1.5 mL) was added dropwise concentrated hydrochloric acid (0.6 mL) at 0°C, and the mixture was stirred at 50°C for 2.5 hr. The reaction mixture was cooled to 0°C, neutralized with 8N aqueous sodium hydroxide solution, adjusted to pH 10-11 with saturated aqueous sodium hydrogen carbonate solution, and extracted with ethyl acetate. The organic layer was washed with saturated brine, dried over anhydrous magnesium sulfate, and concentrated under reduced pressure. The obtained residue was dissolved in DMA (3 mL), and 3-methyl-1-(2,2,2-trifluoroethyl)-1H-pyrazole-5-carboxylic acid (210 mg, 1.01 mmol), HATU (384 mg, 1.01 mmol) and DIEA (0.07 mL, 0.42 mmol) were added at room temperature. The reaction mixture was stirred at 60°C for 3 hr, water was added and the mixture was extracted with ethyl acetate. The organic layer was washed successively with water, saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (13:7-1:3)] and basic silica gel column chromatography [eluent: hexane-ethyl acetate (13:7-1:3)], and crystallized from ethyl acetate-hexane to give the title compound (70.0 mg) as colorless crystals (yield 19%).
MS (ESI+): $[M+H]^+$ 447.
$^1H$ NMR (300 MHz, DMSO-$d_6$) $\delta$ 2.25 (3H, s), 2.30 (3H, s), 4.26 (2H, s), 5.30-5.45 (2H, m), 7.00 (1H, s), 7.18-7.25 (1H, m), 7.30 (1H, br. S.), 7.31-7.38 (1H, m), 10.67 (1H, s).

Example 179

N-[2-(3-chloro-5-fluorobenzyl)-4-methyl-1,3-thiazol-5-yl]-1,4-dimethyl-1H-imidazole-5-carboxamide

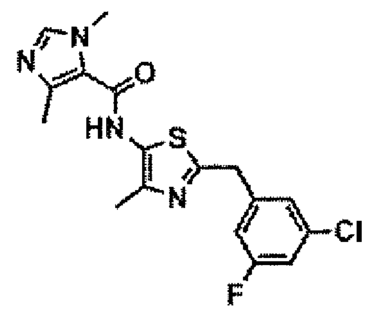

Example 179-A)

1,4-dimethyl-1H-imidazole-5-carboxylic acid

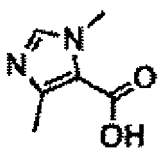

To a solution of ethyl 1,4-dimethyl-1H-imidazole-5-carboxylate (747 mg, 3.81 mmol) in methanol (26.6 mL)-THF (13.3 mL) was added 2M aqueous sodium hydroxide solution (6.7 mL, 13.4 mmol) at room temperature, and the mixture was stirred at room temperature overnight. To the reaction mixture was added ion exchange resin DOWEX 50WX8-100, adjusted to pH 4-5, and the resin was filtered off. The filtrate was concentrated under reduced pressure to give the title compound (397 mg) as a white solid (yield 62%).
$^1H$ NMR (300 MHz, DMSO-$d_6$) $\delta$ 2.33 (3H, s), 3.75 (3H, s), 7.67 (1H, s).
* The peak of "COOH group" was not observed.

Example 179-B)

N-[2-(3-chloro-5-fluorobenzyl)-4-methyl-1,3-thiazol-5-yl]-1,4-dimethyl-1H-imidazole-5-carboxamide

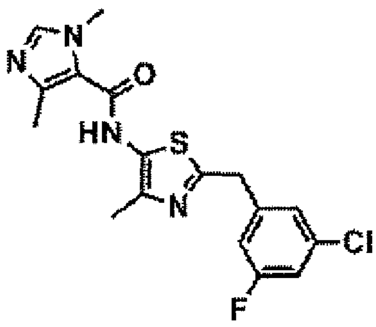

In the same manner as in Example 158, tert-butyl [2-(3-chloro-5-fluorobenzyl)-4-methyl-1,3-thiazol-5-yl]carbamate obtained in Example 145-E) and 1,4-dimethyl-1H-imidazole-5-carboxylic acid obtained in Example 179-A) were used to give the title compound as white crystals (yield 16%). MS (ESI+): $[M+H]^+$ 379.
$^1$H NMR (300 MHz, DMSO-$d_6$) $\delta$ 2.32 (6H, d, J = 6.0 Hz), 3.69 (3H, s), 4.23 (2H, s), 7.13-7.39 (3H, m), 7.68 (1H, s), 10.33 (1H, s).

Example 180

N-[2-(3-chloro-5-fluorobenzyl)-4-methyl-1,3-thiazol-5-yl]-1,5-dimethyl-1H-imidazole-4-carboxamide

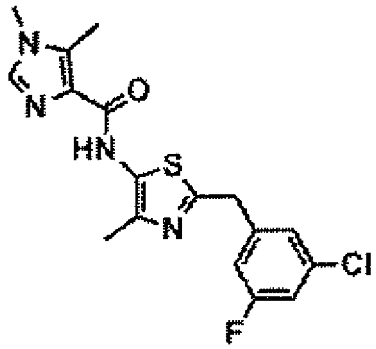

Example 180-A)

1,5-dimethyl-1H-imidazole-4-carboxylic acid

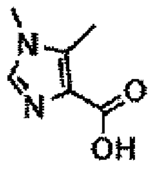

In the same manner as in Example 179-A), ethyl 1,5-dimethyl-1H-imidazole-4-carboxylate and 2M aqueous sodium hydroxide solution were used to give the title compound as a pink solid (yield 13%) .
$^1$H NMR (300 MHz, DMSO-$d_6$) $\delta$ 2.40 (3H, s), 3.53 (3H, s), 7.56 (1H, s).
* The peak of "COOH group" was not observed.

Example 180-B)

N-[2-(3-chloro-5-fluorobenzyl)-4-methyl-1,3-thiazol-5-yl]-1,5-dimethyl-1H-imidazole-4-carboxamide

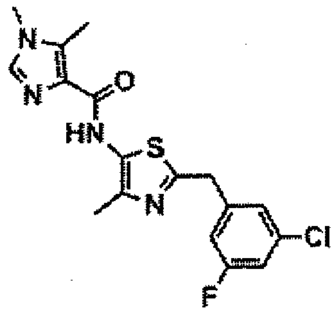

In the same manner as in Example 158, tert-butyl [2-(3-chloro-5-fluorobenzyl)-4-methyl-1,3-thiazol-5-yl]carbamate obtained in Example 145-E) and 1,5-dimethyl-1H-imidazole-4-carboxylic acid obtained in Example 180-A) were used to give the title compound as white crystals (yield 41%). MS (ESI+): $[M+H]^+$ 379.
$^1H$ NMR (300 MHz, DMSO-$d_6$) δ 2.28 (3H, s), 2.47 (3H, s), 3.58 (3H, s), 4.23 (2H, s), 7.16-7.25 (1H, m), 7.28 (1H, s), 7.30-7.38 (1H, m), 7.70 (1H, s), 9.78 (1H, s).

Example 181

N-{2-[3-fluoro-5-(trifluoromethyl)benzyl]-4-methyl-1,3-thiazol-5-yl}-1-methyl-1H-pyrazole-5-carboxamide

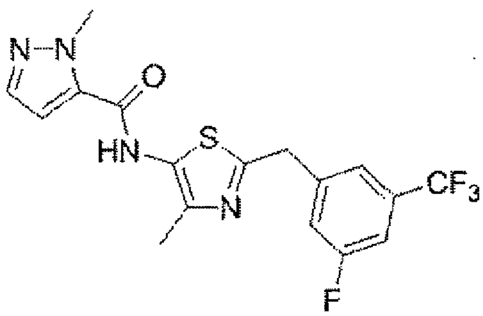

Example 181-A)

2-[3-fluoro-5-(trifluoromethyl)phenyl]ethanethioamide

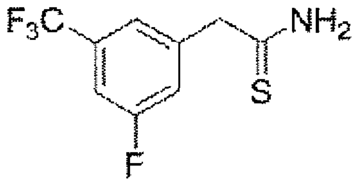

A mixture of [3-fluoro-5-(trifluoromethyl)phenyl]acetonitrile (5.00 g, 24.6 mmol), O,O-diethyl dithiophosphate (4.54 mL, 27.1 mmol) and 4N hydrogen chloride-ethyl acetate solution (60 mL) was stirred at room temperature overnight. In an ice bath, water was added and the organic layer was separated, washed successively with water, 0.1N aqueous sodium hydroxide solution, saturated aqueous sodium hydrogen carbonate solution and saturated brine, dried over

anhydrous magnesium sulfate and concentrated under reduced pressure. The obtained residue was treated with hexane in an ice bath to allow solidification. The solid was collected by filtration, and washed with hexane to give the title compound (4.42 g) as a white solid (yield 76%).
MS (ESI+): $[M+H]^+$ 238.
$^1$H NMR (300 MHz, DMSO-$d_6$) $\delta$ 3.94 (2H, s), 7.46-7.54 (1H, m), 7.54-7.61 (2H, m), 9.37-9.69 (2H, m).

Example 181-B)

ethyl 2-[3-fluoro-5-(trifluoromethyl)benzyl]-4-methyl-1,3-thiazole-5-carboxylate

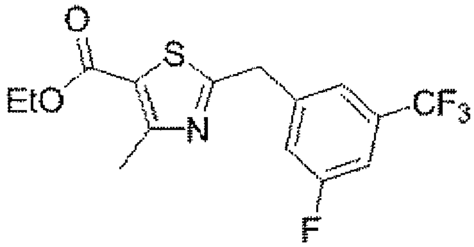

A solution of 2-[3-fluoro-5-(trifluoromethyl)phenyl]ethanethioamide (4.0 g, 16.9 mmol) obtained in Example 181-A) and ethyl 2-chloro-3-oxobutanoate (2.8 mL, 20.2 mmol) in pyridine (8 mL)-ethanol (8 mL) was stirred at room temperature for 2 days. The reaction mixture was concentrated under reduced pressure, azeotropically distilled twice with toluene, diluted with ethyl acetate, washed with 0.1N hydrochloric acid and saturated brine, dried over anhydrous magnesium sulfate, and concentrated under reduced pressure. The obtained residue was purified by basic silica gel column chromatography [eluent: hexane-ethyl acetate (13:7-1:1)] to give the title compound (5.63 g) as a pale-yellow oil (yield 96%).
MS (ESI+): $[M+H]^+$ 348.
$^1$H NMR (300 MHz, DMSO-$d_6$) $\delta$ 1.26 (3H, t, J = 7.2 Hz), 2.60 (3H, s), 4.24 (2H, q, J = 7.2 Hz), 4.48 (2H, s), 7.55-7.71 (3H, m).

Example 181-C)

2-[3-fluoro-5-(trifluoromethyl)benzyl]-4-methyl-1,3-thiazole-5-carboxylic acid

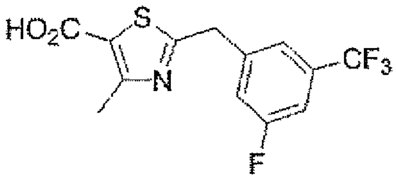

To a solution (25 mL) of ethyl 2-[3-fluoro-5-(trifluoromethyl)benzyl]-4-methyl-1,3-thiazole-5-carboxylate (5.0 g, 14.4 mmol) obtained in Example 181-B) in ethanol was added 8N aqueous sodium hydroxide solution (4.0 mL) at 0°C. The mixture was stirred for 45 min at room temperature, and 6N hydrochloric acid (5.6 mL) and water (14 mL) were added under ice-cooling. The precipitate was collected by filtration, washed with water to give the title compound (4.15 g) as a white solid (yield 90%).
MS (ESI+): $[M+H]^+$ 320.
$^1$H NMR (300MHz, DMSO-$d_6$) $\delta$ 2.58 (3H, s), 4.46 (2H, s), 7.53-7.68 (3H, m), 13.27 (1H, s).

Example 181-D)

tert-butyl {2-[3-fluoro-5-(trifluoromethyl)benzyl]-4-methyl-1,3-thiazol-5-yl}carbamate

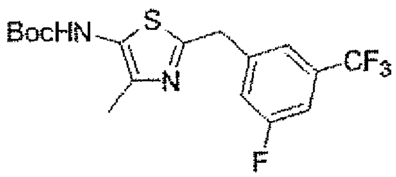

To a solution (35 mL) of 2-[3-fluoro-5-(trifluoromethyl)benzyl]-4-methyl-1,3-thiazole-5-carboxylic acid (4.0 g, 12.5 mmol) obtained in Example 181-C) in tert-butyl alcohol was added triethylamine (5.2 mL, 37.6 mmol) at room temperature, and the mixture was heated to 90°C and DPPA (4.1 mL, 18.8 mmol) was added. The mixture was stirred at the same temperature for 1.5 hr. The reaction product was cooled to room temperature, concentrated under reduced pressure, and the residue was dissolved in ethyl acetate, washed with 0.1N aqueous sodium hydroxide solution and saturated brine, dried over anhydrous magnesium sulfate and concentrated under reduced pressure. The residue was purified by silica gel column chromatography [eluent: hexane-ethyl acetate (3:1-1:1)], and crystallized from ethyl acetate-hexane to give the title compound (1.47 g) as a white solid (yield 30%).
MS (ESI+): $[M+H]^+$ 391.
$^1H$ NMR (300MHz, DMSO-$d_6$) $\delta$1.44 (9H, s), 2.19 (3H, s), 4.30 (2H, s), 7.47-7.63 (3H, m), 9.64 (1H, brs).

Example 181-E)

N-{2-[3-fluoro-5-(trifluoromethyl)benzyl]-4-methyl-1,3-thiazol-5-yl}-1-methyl-1H-pyrazole-5-carboxamide

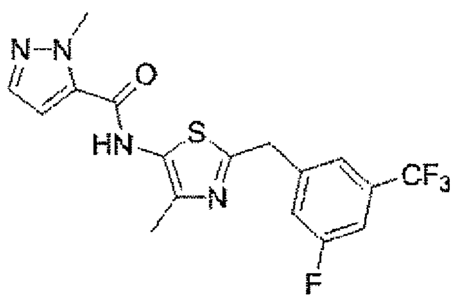

To a solution of tert-butyl {2-[3-fluoro-5-(trifluoromethyl)benzyl]-4-methyl-1,3-thiazol-5-yl}carbamate (400 mg, 1.02 mmol) obtained in Example 181-D) in ethanol (2 mL) was added dropwise concentrated hydrochloric acid (0.8 mL) at 0°C, and the mixture was stirred at 50°C for 2.5 hr. The reaction mixture was cooled to 0°C, neutralized with 8N aqueous sodium hydroxide solution (1.2 mmL), adjusted to pH 10-11 with saturated aqueous sodium hydrogen carbonate solution, and extracted with ethyl acetate. The organic layer was washed with saturated brine, dried over anhydrous magnesium sulfate, and concentrated under reduced pressure. The obtained residue was dissolved in DMA (4 mL), and 1-methyl-1H-pyrazole-5-carboxylic acid (155 mg, 1.23 mmol) obtained in Example 1-A), HATU (468 mg, 1.23 mmol) and DIEA (0.085 mL, 0.512 mmol) were added at room temperature. The reaction mixture was stirred at 60°C for 1.5 hr, water was added and the mixture was extracted with ethyl acetate. The organic layer was washed successively with water, saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (13:7-1:3)] and basic silica gel column chromatography [eluent: hexane-ethyl acetate (13:7-1:3)], and crystallized from ethyl acetate-hexane to give the title compound (139 mg) as colorless crystals (yield 34%).
MS (ESI+): $[M+H]^+$ 399.
$^1H$ NMR (300 MHz, DMSO-$d_6$) $\delta$ 2.74 (3H, s), 4.48 (3H, s), 4.81 (2H, s), 7.50 (1H, d, J = 1.9 Hz), 7.93-8.09 (4H, m), 10.98 (1H, s).

Example 182

N-[2-(3-chloro-5-fluorobenzyl)-4-methyl-1,3-thiazol-5-yl]-2,5-dimethyl-1,3-oxazole-4-carboxamide

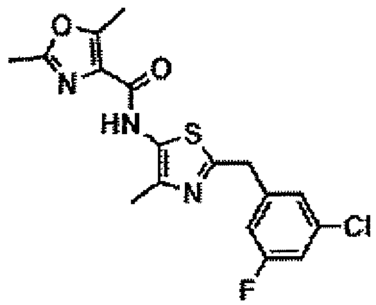

In the same manner as in Example 158, tert-butyl [2-(3-chloro-5-fluorobenzyl)-4-methyl-1,3-thiazol-5-yl]carbamate obtained in Example 145-E) and 2,5-dimethyl-1,3-oxazole-4-carboxylic acid were used to give the title compound as white crystals (yield 54%).
MS (ESI+): $[M+H]^+$ 380.
$^1$H NMR (300 MHz, $CDCl_3$) $\delta$ 2.44 (6H, d, J = 5.2 Hz), 2.63 (3H, s), 4.19 (2H, s), 6.89-7.03 (2H, m), 7.10 (1H, s), 8.85 (1H, s).

Example 183

N-[2-(3-chloro-5-fluorobenzyl)-4-methyl-1,3-thiazol-5-yl]-5-methyl-1,3-thiazole-4-carboxamide

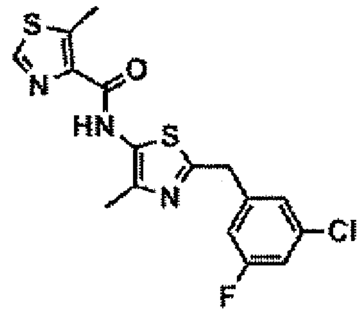

In the same manner as in Example 158, tert-butyl [2-(3-chloro-5-fluorobenzyl)-4-methyl-1,3-thiazol-5-yl]carbamate obtained in Example 145-E) and 5-methyl-1,3-thiazole-4-carboxylic acid were used to give the title compound as white crystals (yield 39%).
MS (ESI+): $[M+H]^+$ 382.
$^1$H NMR (300 MHz, $CDCl_3$) $\delta$ 2.45 (3H, s), 2.89 (3H, s), 4.20 (2H, s), 6.90-7.02 (2H, m), 7.11 (1H, s), 8.54 (1H, s), 9.56 (1H, s) .

Example 184

N-[2-(3-chloro-5-fluorobenzyl)-4-methyl-1,3-thiazol-5-yl]-4-methyl-1,3-thiazole-5-carboxamide

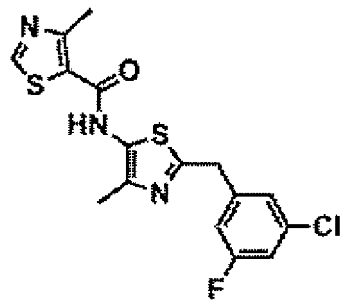

In the same manner as in Example 158, tert-butyl [2-(3-chloro-5-fluorobenzyl)-4-methyl-1,3-thiazol-5-yl]carbamate obtained in Example 145-E) and 4-methyl-1,3-thiazole-5-carboxylic acid were used to give the title compound as white crystals (yield 41%).
MS (ESI+): $[M+H]^+$ 382.
$^1$H NMR (300 MHz, $CDCl_3$) δ 2.41 (3H, s), 2.83 (3H, s), 4.20 (2H, s), 6.88-6.96 (1H, m), 6.99 (1H, dt, J = 8.4, 2.1 Hz), 7.10 (1H, s), 7.56 (1H, s), 8.80 (1H, s).

Example 185

N-[2-(3-chloro-5-fluorobenzyl)-4-methyl-1,3-thiazol-5-yl]-1-methyl-1H-pyrrole-2-carboxamide

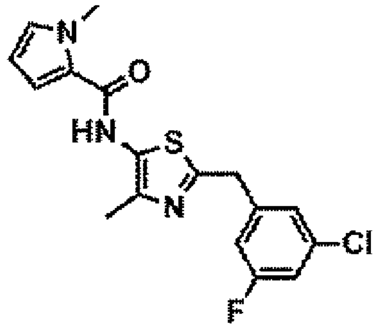

In the same manner as in Example 158, tert-butyl [2-(3-chloro-5-fluorobenzyl)-4-methyl-1,3-thiazol-5-yl]carbamate obtained in Example 145-E) and 1-methyl-1H-pyrrole-2-carboxylic acid were used to give the title compound as white crystals (yield 6%).
MS (ESI+): $[M+H]^+$ 364.
$^1$H NMR (300 MHz, $CDCl_3$) δ 2.40 (3H, s), 3.97 (3H, s), 4.18 (2H, s), 6.17 1H, dd, J=4.1, 2.5 Hz), 6.73 (1H, dd, J=3.8, 1.6 Hz), 6.82 (1H, t, J = 2.1 Hz), 6.89-7.03 (2H, m), 7.10 (1H, s), 7.62 (1H, s).

Example 186

N-[2-(3-chloro-5-fluorobenzyl)-4-methyl-1,3-thiazol-5-yl]-3,5-dimethylisoxazole-4-carboxamide

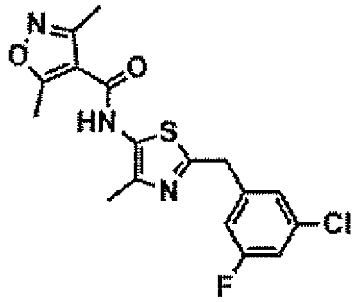

In the same manner as in Example 158, tert-butyl [2-(3-chloro-5-fluorobenzyl)-4-methyl-1,3-thiazol-5-yl]carbamate obtained in Example 145-E) and 3,5-dimethylisoxazole-4-carboxylic acid were used to give the title compound as white crystals (yield 33%).
MS (ESI+): $[M+H]^+$ 380.
$^1$H NMR (300 MHz, $CDCl_3$) 5 2.40 (3H, s), 2.55 (3H, s), 2.71 (3H, s), 4.19 (2H, s), 6.89-6.96 (1H, m), 6.98 (1H, dt, J = 8.5, 2.2 Hz), 7.09 (1H, s), 7.42 (1H, s).

Example 187

ethyl trans-2-{[2-(3-chloro-5-fluorobenzyl)-4-methyl-1,3-thiazol-5-yl]carbamoyl}cyclopropanecarboxylate

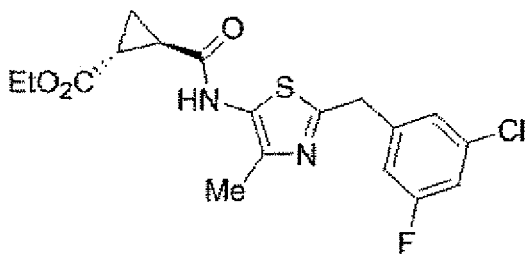

Example 187-A)

trans-2-(ethoxycarbonyl)cyclopropanecarboxylic acid

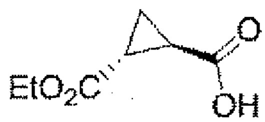

To a solution of diethyl trans-cyclopropane-1,2-dicarboxylate (2.00 g, 10.74 mmol) in THF (20 mL)-water (20 mL) was added 4M aqueous lithium hydroxide solution (2.95 mL), and the mixture was stirred at room temperature for 5 hr. The reaction mixture was washed with diethyl ether. The aqueous layer was neutralized with 1M hydrochloric acid, extracted with ethyl acetate, washed with saturated brine, and dried over anhydrous magnesium sulfate. The obtained residue was crystallized from ethyl acetate-hexane to give the title compound (564 mg) as a colorless solid (yield 33%).
$^1$H NMR (300 MHz, $CDCl_3$) $\delta$1.20-1.35 (3H, m), 1.42-1.59 (2H, m), 2.10-2.30 (2H, m), 4.16 (2H, q, J = 7.2 Hz).
* The peak of "COOH group" was not observed.

Example 187-B)

ethyl trans-2-{[2-(3-chloro-5-fluorobenzyl)-4-methyl-1,3-thiazol-5-yl]carbamoyl}cyclopropanecarboxylate

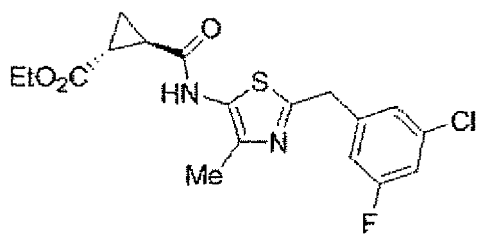

In the same manner as in Example 145-F), tert-butyl [2-(3-chloro-5-fluorobenzyl)-4-methyl-1,3-thiazol-5-yl] carbamate obtained in Example 145-E) and trans-2-(ethoxycarbonyl)cyclopropanecarboxylic acid obtained in Example 187-A) were used to give the title compound (554 mg) (yield 47%).
MS (ESI+): $[M+H]^+$ 397.
$^1$H NMR (300 MHz, $CDCl_3$) $\delta$1.28 (3H, t, J = 7.0 Hz), 1.42-1.53 (1H, m), 1.54-1.62 (1H, m), 2.11-2.20 (1H, m), 2.27 (1H, ddd, J = 9.0, 5.8, 3.8 Hz), 2.37 (3H, s), 4.08-4.27 (4H, m), 6.85-7.02 (2H, m), 7.07 (1H, s), 7.66 (1H, s).

Example 188

trans-2-[2-(3-chloro-5-fluorobenzyl)-4-methyl-1,3-thiazol-5-yl]carbamoyl}cyclopropanecarboxylic acid

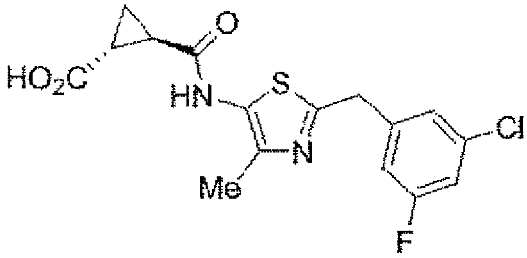

In the same manner as in Example 174, ethyl trans-2-{[2-(3-chloro-5-fluorobenzyl)-4-methyl-1,3-thiazol-5-yl]carbamoyl}cyclopropanecarboxylate obtained in Example 187 was used to give the title compound (412 mg) (yield 89%).
MS (ESI+): $[M+H]^+$ 369.
$^1$H NMR (300 MHz, DMSO-$d_6$) δ1.22-1.37 (2H, m), 1.88 (1H, ddd, J = 8.1, 5.9, 3.8 Hz), 2.32 (3H, s), 2.43-2.57 (1H, m), 4.20 (2H, s), 7.17 (1H, dd, J = 9.1, 1.9 Hz), 7.25 (1H, s), 7.32 (1H, dt, J = 8.7, 2.1 Hz), 10.78 (1H, s), 12.58 (1H, brs).

Example 189

trans-N-[2-(3-chloro-5-fluorobenzyl)-4-methyl-1,3-thiazol-5-yl]-N'-(2-methoxyethyl)cyclopropane-1,2-dicarboxamide

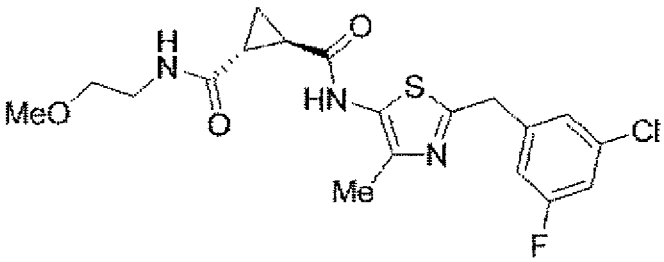

In the same manner as in Example 175, trans-2-{[2-(3-chloro-5-fluorobenzyl)-4-methyl-1,3-thiazol-5-yl]carbamoyl}cyclopropanecarboxylic acid obtained in Example 188 was used to give the title compound (277 mg) (yield 72%).
MS (ESI+): $[M+H]^+$426.
$^1$H NMR (300 MHz, $CDCl_3$) δ 1.47 (2H, t, J = 7.0 Hz), 2.05 (1H, td, J = 7.3, 3.6 Hz), 2.22-2.33 (1H, m), 2.36 (3H, s), 3.35 (3H, s), 3.44 (4H, d, J = 2.3 Hz), 4.14 (2H, s), 6.31 (1H, brs), 6.85-6.93 (1H, m), 6.96 (1H, dt, J = 8.5, 2.0 Hz), 7.07 (1H, s), 8.90 (1H, s).

Example 190

methyl cis-2-{[2-(3-chloro-5-fluorobenzyl)-4-methyl-1,3-thiazol-5-yl]carbamoyl}cyclopropanecarboxylate

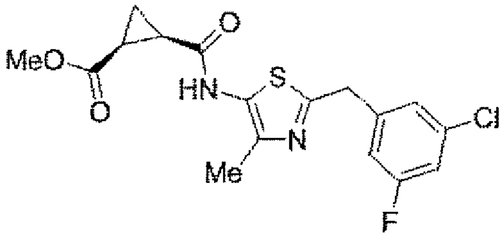

Example 190-A)

cis-2-(methoxycarbonyl)cyclopropanecarboxylic acid

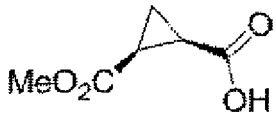

In the same manner as in Example 187-A), dimethyl cis-cyclopropane-1,2-dicarboxylate was used to give the title compound (1.00 g) (yield 55%).
$^1$H NMR (300 MHz, $CDCl_3$) δ1.34 (1H, td, J = 8.5, 5.3 Hz), 1.70 (1H, td, J = 6.8, 4.9 Hz), 2.06-2.20 (2H, m), 3.71 (3H, s).
* The peak of "COOH group" was not observed.

Example 190-B)

methyl cis-2-{[2-(3-chloro-5-fluorobenzyl)-4-methyl-1,3-thiazol-5-yl]carbamoyl}cyclopropanecarboxylate

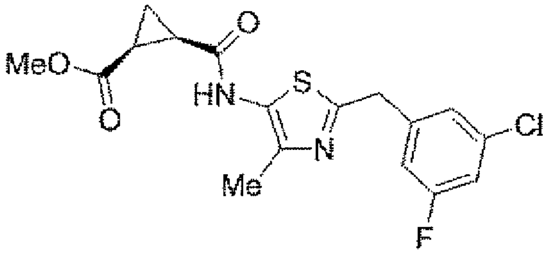

In the same manner as in Example 145-F), tert-butyl [2-(3-chloro-5-fluorobenzyl)-4-methyl-1,3-thiazol-5-yl] carbamate obtained in Example 145-E) and cis-2-(methoxycarbonyl)cyclopropanecarboxylic acid obtained in Example 190-A) were used to give the title compound (497 mg) (yield 36%).
MS (ESI+): $[M+H]^+$383.
$^1$H NMR (300 MHz, $CDCl_3$) δ1.57-1.74 (2H, m), 2.12-2.31 (2H, m), 2.38 (3H, s), 3.79 (3H, s), 4.15 (2H, s), 6.86-7.02 (2H, m), 7.08 (1H, s), 9.17 (1H, brs).

Example 191

cis-2-{[2-(3-chloro-5-fluorobenzyl)-4-methyl-1,3-thiazol-5-yl]carbamoyl}cyclopropanecarboxylic acid

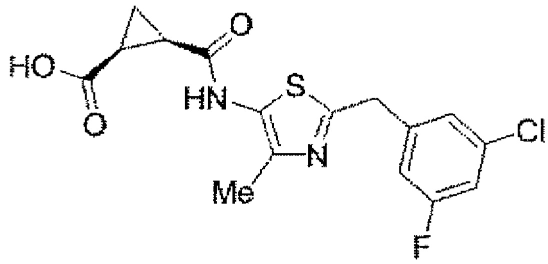

In the same manner as in Example 174, methyl cis-2-{[2-(3-chloro-5-fluorobenzyl)-4-methyl-1,3-thiazol-5-yl]carbamoyl}cyclopropanecarboxylate obtained in Example 190 was used to give the title compound (392 mg) (yield 93%).
MS (ESI+) : $[M+H]^+$369.
$^1$H NMR (300 MHz, DMSO-$d_6$) δ 1.18 (1H, td, J = 8.2, 4.3 Hz), 1.39 (1H, td, J = 6.6, 4.2 Hz), 1.96-2.12 (1H, m), 2.19-2.35 (4H, m), 4.19 (2H, s), 7.17 (1H, d, J = 9.4 Hz), 7.26 (1H, s), 7.27-7.36 (1H, m), 10.58 (1H, brs), 12.11 (1H, brs).

Example 192

1-tert-butyl-N-[2-(3-chloro-5-fluorobenzyl)-4-methyl-1,3-thiazol-5-yl]-3-methyl-1H-pyrazole-5-carboxamide

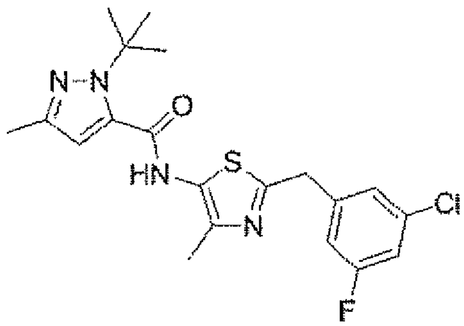

To a solution of tert-butyl [2-(3-chloro-5-fluorobenzyl)-4-methyl-1,3-thiazol-5-yl]carbamate (300 mg, 1.68 mmol) obtained in Example 145-E) in ethanol (2 mL) was added dropwise concentrated hydrochloric acid (0.6 mL) at 0°C, and the mixture was stirred at 50°C for 1.5 hr. The reaction mixture was cooled to 0°C, neutralized with 8M aqueous sodium hydroxide solution, adjusted to pH 10-11 with saturated aqueous sodium hydrogen carbonate solution, and extracted with ethyl acetate. The organic layer was washed with saturated brine, dried over anhydrous magnesium sulfate, and concentrated under reduced pressure. The obtained residue was dissolved in THF (2 mL), and a solution of 1-tert-butyl-3-methyl-1H-pyrazole-5-carbonyl chloride (676 mg, 3.37 mmol) in THF (2 mL), and several drops of 8M aqueous sodium hydroxide solution were added. The reaction mixture was stirred at room temperature for 20 min, water was added and the mixture was extracted with ethyl acetate. The organic layer was washed with saturated brine, dried over anhydrous magnesium sulfate, and concentrated under reduced pressure. The obtained residue was purified by silica gel column chromatography [eluent: hexane-ethyl acetate (2:1-1:3)] and basic silica gel column chromatography [eluent: hexane-ethyl acetate (13:7-1:3)], and crystallized from ethyl acetate-hexane to give the title compound (53.7 mg) as colorless crystals (yield 8%).
MS (ESI+): $[M+H]^+$ 421.
$^1$H NMR (300 MHz, DMSO-$d_6$) δ 1.55 (9H, s), 2.18 (3H, s), 2.31 (3H, s), 4.24 (2H, s), 6.42 (1H, s), 7.18-7.25 (1H, m), 7.27-7.38 (2H, m), 11.05 (1H, s).

Example 193

N-[2-(3-chloro-5-fluorobenzyl)-4-methyl-1,3-thiazol-5-yl]-2-methyl-1,3-oxazole-4-carboxamide

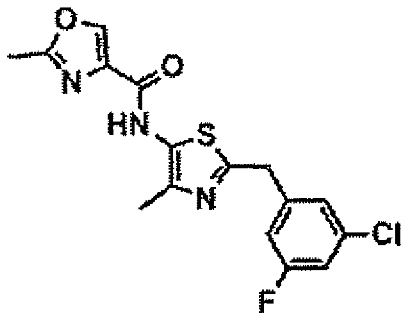

In the same manner as in Example 158, tert-butyl [2-(3-chloro-5-fluorobenzyl)-4-methyl-1,3-thiazol-5-yl]carbamate obtained in Example 145-E) and 2-methyl-1,3-oxazole-4-carboxylic acid were used to give the title compound as white crystals (yield 45%).
MS (ESI+): $[M+H]^+$ 366.
$^1H$ NMR (300 MHz, $CDCl_3$) $\delta$ 2.44 (3H, s), 2.53 (3H, s), 4.19 (2H, s), 6.88-7.02 (2H, m), 7.10 (1H, s), 8.17 (1H, s), 8.81 (1H, s).

Example 194

1-acetyl-N-[2-(3-chloro-5-fluorobenzyl)-4-methyl-1,3-thiazol-5-yl]cyclopropanecarboxamide

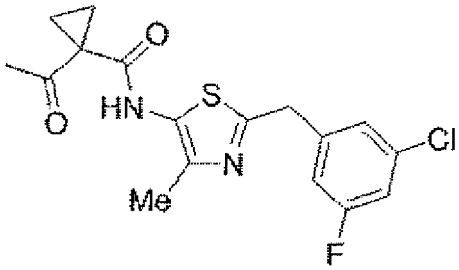

Example 194-A)

methyl 1-acetylcyclopropanecarboxylate

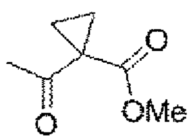

To a mixture of methyl 3-oxobutanoate (10 g, 86 mmol), potassium carbonate (29.8 g, 36 mmol) and acetone (150 mL) was added 1,2-dibromoethane (21 g, 112 mmol) and the mixture was heated under reflux for 1.5 days. The reaction mixture was cooled to room temperature, insoluble material was filtered off, and the filtrate was concentrated under reduced pressure. The obtained residue was purified by silica gel column chromatography [eluent: hexane-ethyl acetate (97:3-3:1)] to give the title compound (6.41 g) as a colorless oil (yield 52%).
$^1H$ NMR (300 MHz, $CDCl_3$) $\delta$ 1.48 (4H, s), 2.47 (3H,s), 3.75 (3H,s).

Example 194-B)

1-acetylcyclopropanecarboxylic acid

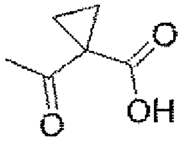

To a solution of methyl 1-acetylcyclopropanecarboxylate (1.66 g, 11.68 mmol) obtained in Example 194-A) in methanol (18 mL) was added 8M aqueous sodium hydroxide solution (2.92 mL, 23.35 mmol), and the mixture was stirred at room temperature for one day. The mixture was neutralized with Dowex 50W-X8 ($H^+$ form), the resin was filtered off, and washed with methanol. The filtrate was concentrated under reduced pressure to give a colorless oil (1.03 g) containing the title compound. This was used in the next step without further purification.

Example 194-C)

1-acetyl-N-[2-(3-chloro-5-fluorobenzyl)-4-methyl-1,3-thiazol-5-yl]cyclopropanecarboxamide

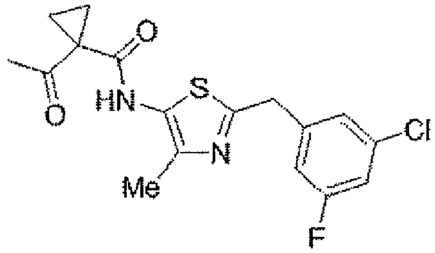

tert-Butyl [2-(3-chloro-5-fluorobenzyl)-4-methyl-1,3-thiazol-5-yl]carbamate (1.06 g, 2.97 mmol) obtained in Example 145-E) was dissolved in 12M hydrochloric acid (3 mL) and ethanol (6 mL), and the mixture was stirred at 60°C for 1 hr. After cooling to room temperature, the reaction mixture was basified with 8M aqueous sodium hydroxide solution and saturated aqueous sodium hydrogen carbonate solution, and extracted with ethyl acetate. The extract was dried over anhydrous sodium sulfate, and concentrated under reduced pressure. The residue was dissolved in DMF (5 mL), and crude 1-acetylcyclopropanecarboxylic acid (457 mg, 3.56 mmol) obtained in Example 194-B), HATU (1.35 g, 3.56 mmol) and DIEA (0.259 mL, 1.49 mmol) were added at room temperature, and the mixture was stirred at 60°C overnight. The reaction mixture was diluted with saturated aqueous sodium hydrogen carbonate solution, and extracted with ethyl acetate. The extract was washed with water and saturated brine, dried over anhydrous sodium sulfate, and concentrated under reduced pressure. The obtained residue was purified by silica gel column chromatography [eluent: hexane-ethyl acetate (90:10-0:100)]. The obtained residue was separated by HPLC (C18, mobile phase: water/acetonitrile (0.1% TFA containing)), saturated aqueous sodium hydrogen carbonate solution was added to the obtained fraction, and the mixture was extracted with ethyl acetate. The extract was dried over anhydrous magnesium sulfate, and concentrated under reduced pressure to give the title compound (115 mg) as a pale-yellow oil (yield 10%).
MS (ESI+): $[M+H]^+$367.
$^1$H NMR (300 MHz, $CDCl_3$) $\delta$1.62-1.76 (2H, m), 1.94-2.17 (5H, m), 2.46 (3H, s), 4.16 (2H, s), 6.84-7.00 (2H, m), 7.08 (1H, s), 11.68 (1H, brs).

Example 195

1-methyl-N-(4-methyl-2-{1-[3-(trifluoromethyl)phenyl]cyclopropyl}-1,3-thiazol-5-yl)-1H-pyrazole-5-carboxamide

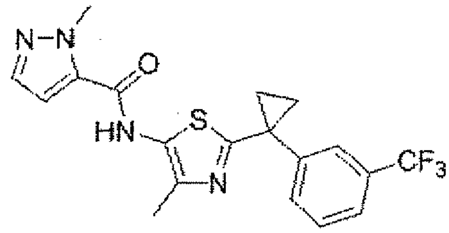

Example 195-A)

1-[3-(trifluoromethyl)phenyl]cyclopropanecarbonitrile

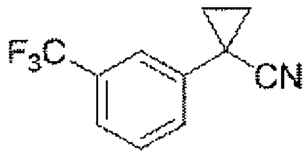

[3-(Trifluoromethyl)phenyl]acetonitrile (15.0 g, 81.0 mmol) was dissolved in DMF (anhydrous; 81 mL), cooled to 0°C, sodium hydride (60 wt%, 7.78 g, 194 mmol) was added, and the mixture was stirred at the same temperature for 10 min. To the reaction mixture was added 1,2-dibromoethane (22.8 g, 122 mmol), and the mixture was stirred at room temperature for 3 hr. Water was added to the reaction mixture and the mixture was extracted with ethyl acetate. The organic layer was washed with saturated brine, dried over anhydrous sodium sulfate, and concentrated under reduced pressure. The obtained residue was purified by silica gel column chromatography [eluent: hexane-ethyl acetate (5:1)] to give the title compound (17.1 g) as yellow crystals (yield quant.).
$^1$H NMR (400 MHz, $CDCl_3$) $\delta$ 1.46 (2H, dd, J = 5.2, 7.6 Hz), 1.81 (2H, dd, J = 5.2, 7.6 Hz), 7.49-7.57 (4H, m).

Example 195-B)

1-[3-(trifluoromethyl)phenyl]cyclopropanethioamide

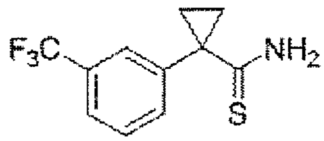

A mixture of 1-[3-(trifluoromethyl)phenyl]cyclopropanecarbonitrile (17.1 g, 81.0 mmol) obtained in Example 195-A), O,O-diethyl dithiophosphate (15.2 mL, 89.0 mmol) and 4N hydrogen chloride-dioxane solution (162 mL) was stirring at room temperature overnight, and concentrated under reduced pressure. The residue was dissolved in ethyl acetate, washed with 1N aqueous sodium hydroxide solution and saturated brine, dried over anhydrous sodium sulfate, and concentrated under reduced pressure. The obtained residue was purified by silica gel column chromatography [eluent: hexane-ethyl acetate (3:1)] to give the title compound (16.9 g) as yellow crystals (yield 85%).
$^1$H NMR (400 MHz, $CDCl_3$) $\delta$ 1.36 (2H, q, J = 3.6 Hz), 2.11 (2H, q, J = 3.6 Hz), 6.41 (1H, brs), 7.42-7.56 (2H, m), 7.60-7.65 (2H, m), 7.68 (1H, s).

Example 195-C)

ethyl 4-methyl-2-{1-[3-(trifluoromethyl)phenyl]cyclopropyl}-1,3-thiazole-5-carboxylate

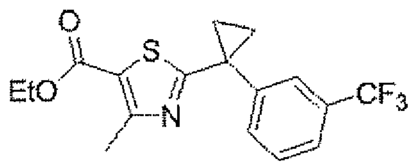

A solution of 1-[3-(trifluoromethyl)phenyl]cyclopropanethioamide (16.9 g, 68.7 mmol) obtained in Example 195-B) and ethyl 2-chloro-3-oxobutanoate (14.7 g, 89.0 mmol) in pyridine (49 mL)-ethanol (49 mL) was stirred at 80°C for 4 hr. The reaction mixture was concentrated under reduced pressure, diluted with ethyl acetate, washed successively with 0.2N hydrochloric acid and saturated brine, dried over anhydrous sodium sulfate, and concentrated under reduced pressure. The obtained residue was purified by silica gel column chromatography [eluent: hexane-ethyl acetate (6:1)] to give the title compound (15.3 g) as white crystals (yield 63%).
$^1$H NMR (400 MHz, $CDCl_3$) $\delta$ 1.29 (3H, t, J = 7.2 Hz), 1.47 (2H, dd, J = 4.4, 6.8 Hz), 1.86 (2H, dd, J = 4.4, 6.8 Hz), 2.66 (3H, s), 4.24 (2H, q, J = 7.2 Hz), 7.49-7.55 (1H, m), 7.60-7.73 (3H, m).

Example 195-D)

4-methyl-2-{1-[3-(trifluoromethyl)phenyl]cyclopropyl}-1,3-thiazole-5-carboxylic acid

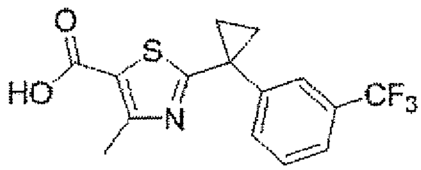

To a solution (450 mL) of ethyl 4-methyl-2-{1-[3-(trifluoromethyl)phenyl]cyclopropyl}-1,3-thiazole-5-carboxylate (15.3 g, 43.1 mmol) obtained in Example 195-C) in ethanol was added 6N aqueous sodium hydroxide solution (14.4 mL, 86.0 mmol) at room temperature. The mixture was stirred at room temperature for 3 hr and concentrated under reduced pressure. The obtained residue was diluted with ethyl acetate, washed successively with 6N hydrochloric acid and saturated brine, dried over anhydrous sodium sulfate and concentrated under reduced pressure to give the title compound (14.1 g) as yellow crystals (yield quant.).
MS (ESI+): $[M+H]^+$ 328.
$^1$H NMR (400 MHz, $CDCl_3$) $\delta$ 1.49 (2H, dd, J = 4.0, 6.8 Hz), 1.88 (2H, dd, J = 4.0, 6.8 Hz), 2.63 (3H, s), 7.52 (1H, t, J = 7.6 Hz), 7.61 (1H, d, J = 7.6 Hz), 7.67 (1H, d, J = 7.6 Hz), 7.72 (1H, s).
* The peak of "COOH group" was not observed.

Example 195-E)

tert-butyl (4-methyl-2-{1-[3-(trifluoromethyl)phenyl]cyclopropyl)-1,3-thiazol-5-yl)carbamate

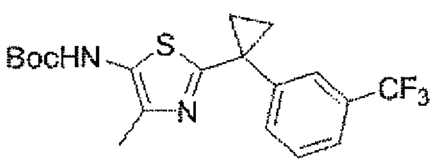

To a solution (98 mL) of 4-methyl-2-{1-[3-(trifluoromethyl)phenyl]cyclopropyl}-1,3-thiazole-5-carboxylic acid (12.0 g, 36.7 mmol) obtained in Example 195-D) in tert-butyl alcohol were added triethylamine (15.3 mL, 110 mmol) and DPPA (15.1 mL, 55.0 mmol) at room temperature. The mixture was stirred at 90°C for 4 hr. The reaction product was cooled to room temperature, and concentrated under reduced pressure. The obtained residue was dissolved in ethyl acetate, washed with 0.2M aqueous sodium hydroxide solution and saturated brine, dried over anhydrous sodium sulfate,

and concentrated under reduced pressure. The residue was purified by silica gel column chromatography [eluent: hexane-ethyl acetate (5:1)], and crystallized from ethyl acetate-hexane to give the title compound (5.80 g) as a colorless oil (yield 40%).
MS (ESI+): $[M+H]^+$ 399.
$^1$H NMR (400 MHz, $CDCl_3$) δ 1.35 (2H, dd, J = 4.4, 6.8 Hz), 1.47 (9H, s), 1.70 (2H, dd, J = 4.4, 6.8 Hz), 2.24 (3H, s), 6.26 (1H, brs), 7.45 (1H, t, J = 7.6 Hz), 7.54 (1H, d, J = 7.6 Hz), 7.64 (1H, d, J = 7.6 Hz), 7.68 (1H, s).

Example 195-F)

1-methyl-N-(4-methyl-2-{1-[3-(trifluoromethyl)phenyl]cyclopropyl}-1,3-thiazol-5-yl)-1H-pyrazole-5-carboxamide

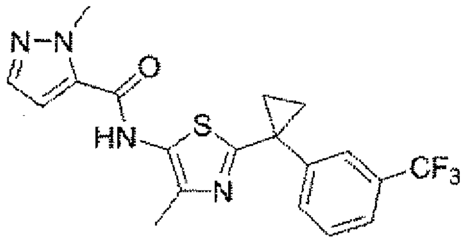

To a solution of tert-butyl (4-methyl-2-{1-[3-(trifluoromethyl)phenyl]cyclopropyl}-1,3-thiazol-5-yl)carbamate (400 mg, 1.00 mmol) obtained in Example 195-E) in ethanol (6 mL) was added dropwise concentrated hydrochloric acid (1.7 mL) at room temperature, and the mixture was stirred at 50°C for 3 hr. The reaction mixture was cooled to room temperature, neutralized with 8M aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (10 mL), and 1-methyl-1H-pyrazole-5-carboxylic acid (183 mg, 1.45 mmol), HATU (551 mg, 1.45 mmol) and DIEA (0.10 mL, 0.61 mmol) were added at room temperature. The reaction mixture was stirred at 60°C for 30 min, saturated aqueous sodium hydrogen carbonate solution was added and the mixture was extracted with ethyl acetate. The organic layer was dried over anhydrous magnesium sulfate and concentrated under reduced pressure. The obtained residue was purified by silica gel column chromatography [eluent: hexane-ethyl acetate (13:7-1:3)] and basic silica gel column chromatography [eluent: hexane-ethyl acetate (13:7-1:3)], and crystallized from ethyl acetate-hexane to give the title compound (199 mg) as colorless crystals (yield 49%).
MS (ESI+): $[M+H]^+$ 407.
$^1$H NMR (300 MHz, DMSO-$d_6$) 5 1.40-1.50 (2H, m), 1.59-1.67 (2H, m), 2.27 (3H, s), 4.01 (3H, s), 7.04 (1H, d, J = 2.3 Hz), 7.52 (1H, d, J = 2.3 Hz), 7.59-7.84 (4H, m), 10.46 (1H, s).

Example 196

1-methyl-N-(4-methyl-2-{1-methyl-1-[3-(trifluoromethyl)phenyl]ethyl}-1,3-thiazol-5-yl)-1H-pyrazole-5-carboxamide

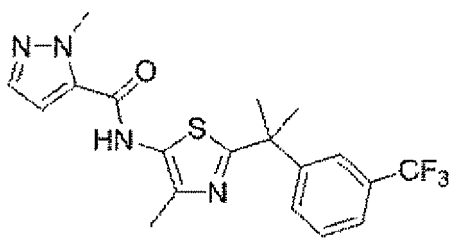

Example 196-A)

ethyl 4-methyl-2-{1-methyl-1-[3-(trifluoromethyl)phenyl]ethyl}-1,3-thiazole-5-carboxylate

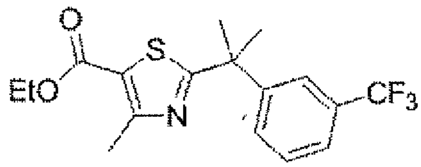

Ethyl 4-methyl-2-[3-(trifluoromethyl)benzyl]-1,3-thiazole-5-carboxylate (15.9 g, 48.4 mmol) obtained in Example 3-A) was dissolved in DMF (anhydrous; 242 mL), and the mixture was cooled to 0°C. Sodium hydride (60 wt%, 5.80 g, 145 mmol) was added at 0°C, and the mixture was stirred at the same temperature for 10 min. To the reaction mixture was added methyl iodide (34.3 g, 242 mmol) at 0°C, and the mixture was stirred for 3 hr at room temperature. Water was added to the reaction mixture and the mixture was extracted with ethyl acetate. The organic layer was washed with saturated brine, dried over anhydrous sodium sulfate, and concentrated under reduced pressure. The obtained residue was purified by silica gel column chromatography [eluent: hexane-ethyl acetate (7:1)] to give the title compound (12.9 g) as a colorless oil (yield 75%).
$^1$H NMR (400 MHz, $CDCl_3$) δ 1.33 (3H, t, J = 7.2 Hz), 1.85 (6H, s), 2.71 (3H, s), 4.29 (2H, q, J = 7.2 Hz), 7.40-7.55 (3H, m), 7.62 (1H, s).

Example 196-B)

4-methyl-2-{1-methyl-1-[3-(trifluoromethyl)phenyl]ethyl}-1,3-thiazole-5-carboxylic acid

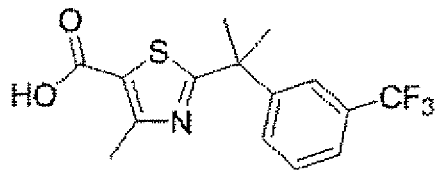

To a solution (298 mL) of ethyl 4-methyl-2-{1-methyl-1-[3-(trifluoromethyl)phenyl]ethyl}-1,3-thiazole-5-carboxylate (10.7 g, 29.8 mmol) obtained in Example 196-A) in ethanol was added 6N aqueous sodium hydroxide solution (9.93 mL, 59.6 mmol) at room temperature. The mixture was stirred at room temperature for 3 hr, and concentrated under reduced pressure. The residue was diluted with was ethyl acetate, washed successively with 6N hydrochloric acid and saturated brine, dried over anhydrous sodium sulfate and concentrated under reduced pressure to give the title compound (9.81 g) as a colorless oil (yield quant.).
MS (ESI+): $[M+H]^+$ 330.
$^1$H NMR (400 MHz, $CDCl_3$) δ 1.85 (6H, s), 2.70 (3H, s), 7.41-7.46 (1H, m), 7.49-7.55 (2H, m), 7.63 (1H, s).
* The peak of "COOH group" was not observed..

Example 196-C)

tert-butyl (4-methyl-2-{1-methyl-1-[3-(trifluoromethyl)phenyl]ethyl]-1,3-thiazol-5-yl)carbamate

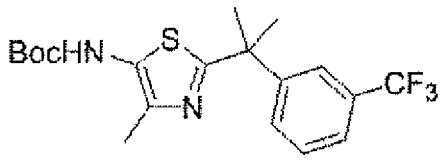

To a solution (75 mL) of 4-methyl-2-{1-methyl-1-[3-(trifluoromethyl)phenyl]ethyl}-1,3-thiazole-5-carboxylic acid (9.30 g, 28.2 mmol) obtained in Example 196-B) in tert-butyl alcohol were added triethylamine (11.8 mL, 85.0 mmol) and DPPA (11.7 g, 42.4 mmol) at room temperature. The mixture was stirred at 90°C for 4 hr. The reaction product was cooled to room temperature, and concentrated under reduced pressure. The obtained residue was dissolved in ethyl acetate, washed with 0.2N aqueous sodium hydroxide solution and saturated brine, dried over anhydrous sodium sulfate, and concentrated under reduced pressure. The residue was purified by silica gel column chromatography [eluent: hexane-ethyl acetate (5:1)] to give the title compound (4.90 g) as a colorless oil (yield 43%).
MS (ESI+): $[M+H]^+$ 401.
$^1H$ NMR (400 MHz, $CDCl_3$) $\delta$ 1.49 (9H, s), 1.81 (6H, s), 2.29 (3H, s), 6.34 (1H, brs), 7.36-7.42 (1H, m), 7.46 (1H, d, J = 8.0 Hz), 7.51 (1H, d, J = 8.0 Hz), 7.61 (1H, s).

Example 196-D)

1-methyl-N-(4-methyl-2-{1-methyl-1-[3-(trifluoromethyl)phenyl]ethyl}-1,3-thiazol-5-yl)-1H-pyrazole-5-carboxamide

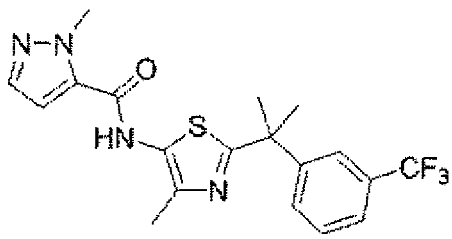

To a solution of tert-butyl (4-methyl-2-{1-methyl-1-[3-(trifluoromethyl)phenyl]ethyl}-1,3-thiazol-5-yl)carbamate (400 mg, 1.00 mmol) obtained in Example 196-C) in ethanol (6 mL) was added dropwise concentrated hydrochloric acid (1.74 mL) at room temperature, and the mixture was stirred at 50°C for 1.5 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (10 mL), and 1-methyl-1H-pyrazole-5-carboxylic acid (151 mg, 1.20 mmol), HATU (456 mg, 1.20 mmol) and DIEA (0.08 mL, 0.50 mmol) were added at room temperature. The reaction mixture was stirred at 60°C for 1.5 hr, saturated aqueous sodium hydrogen carbonate solution was added and the mixture was extracted with ethyl acetate. The organic layer was dried over anhydrous magnesium sulfate and concentrated under reduced pressure. The obtained residue was purified by silica gel column chromatography [eluent: hexane-ethyl acetate (13:7-1:3)] and basic silica gel column chromatography [eluent: hexane-ethyl acetate (4:1-1:3)], and crystallized from ethyl acetate-hexane to give the title compound (191 mg) as colorless crystals (yield 47%).
MS (ESI+): $[M+H]^+$ 409.
$^1H$ NMR (300 MHz, DMSO-$d_6$) $\delta$ 1.79 (6H, s), 2.32 (3H, s), 4.04 (3H, s), 7.06 (1H, d, J = 1.9 Hz), 7.53 (1H, d, J = 1.9 Hz), 7.56-7.72 (4H, m), 10.51 (1H, s).

Example 197

N-[2-(3-chloro-5-fluorobenzyl)-4-methyl-1,3-thiazol-5-yl]-1-methyl-5-oxoprolinamide

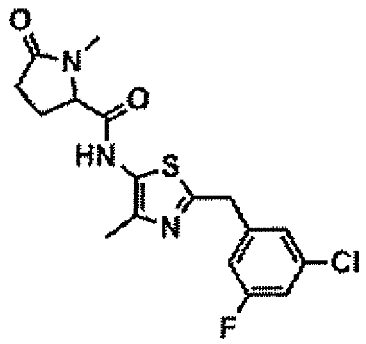

In the same manner as in Example 158, tert-butyl [2-(3-chloro-5-fluorobenzyl)-4-methyl-1,3-thiazol-5-yl]carbamate obtained in Example 145-E) and 1-methyl-5-oxoproline were used to give the title compound as white crystals (yield 31%).
MS (ESI+): $[M+H]^+$ 382.
$^1$H NMR (300 MHz, $CDCl_3$) δ 2.03-2.23 (1H, m), 2.31-2.39 (3H, m), 2.38-2.64 (3H, m), 2.91 (3H, s), 4.12-4.28 (3H, m), 6.91 (1H, dt, J = 9.1, 1.8 Hz), 6.98 (1H, dt, J = 8.5, 2.2 Hz), 7.08 (1H, s).
* The peak of "NH group" was not observed.

Example 198

N-[2-(3-chloro-5-fluorobenzyl)-4-methyl-1,3-thiazol-5-yl]-1,4-dimethyl-1H-pyrazole-5-carboxamide

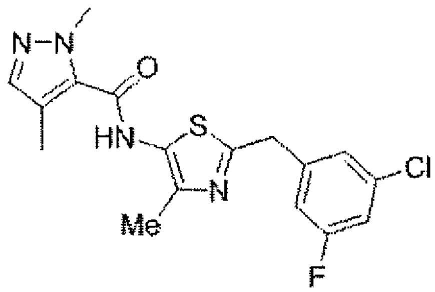

In the same manner as in Example 145-F), tert-butyl [2-(3-chloro-5-fluorobenzyl)-4-methyl-1,3-thiazol-5-yl] carbamate obtained in Example 145-E) and 1,4-dimethyl-1H-pyrazole-5-carboxylic acid were used to give the title compound (241 mg) (yield 21%).
MS (ESI+): $[M+H]^+$ 379.
$^1$H NMR (300 MHz, $CDCl_3$) δ2.41 (6H, s), 4.14 (3H, s), 4.20 (2H, s), 6.91-6.96 (1H, m), 6.99 (1H, dt, J = 8.4, 2.2 Hz), 7.11 (1H, s), 7.36 (1H, s), 7.65 (1H, s).

Example 199

$N^5$-[2-(3-chloro-5-fluorobenzyl)-4-methyl-1,3-thiazol-5-yl]-$N^3$-[2-(dimethylamino)ethyl]-1-methyl-1H-pyrazole-3,5-dicarboxamide

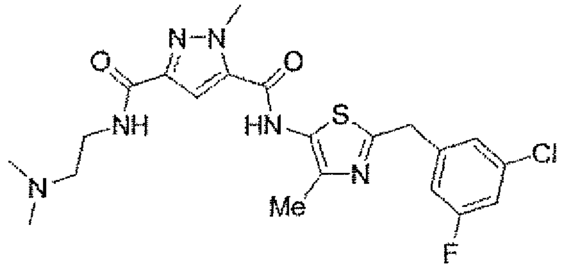

Example 199-A)

ethyl 5-{[2-(3-chloro-5-fluorobenzyl)-4-methyl-1,3-thiazol-5-yl]carbamoyl}-1-methyl-1H-pyrazole-3-carboxylate

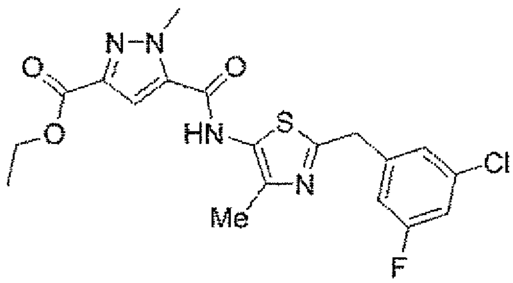

In the same manner as in Example 145-F), tert-butyl [2-(3-chloro-5-fluorobenzyl)-4-methyl-1,3-thiazol-5-yl] carbamate obtained in Example 145-E) and 3-(ethoxycarbonyl)-1-methyl-1H-pyrazole-5-carboxylic acid were used to give the title compound (2.40 g) (yield 77%).
MS (ESI+): $[M+H]^+$ 437.
$^1$H NMR (300 MHz, $CDCl_3$) δ1.40 (3H, t, J = 7.2 Hz), 2.41 (3H, s), 4.20 (2H, s), 4.28 (3H, s), 4.41 (2H, q, J = 7.2 Hz), 6.94 (1H, dd, J = 9.0, 1.5 Hz), 7.00 (1H, dt, J = 8.5, 2.0 Hz), 7.10 (1H, s), 7.26 (1H, s), 7.94 (1H, s).

Example 199-B)

5-{[2-(3-chloro-5-fluorobenzyl)-4-methyl-1,3-thiazol-5-yl]carbamoyl}-1-methyl-1H-pyrazole-3-carboxylic acid

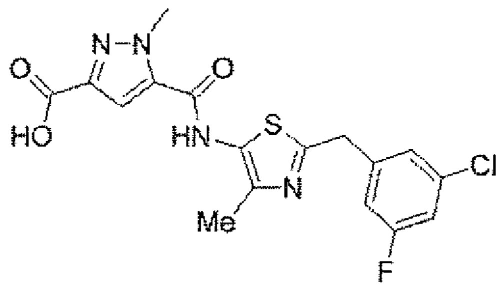

In the same manner as in Example 174, ethyl 5-{[2-(3-chloro-5-fluorobenzyl)-4-methyl-1,3-thiazol-5-yl]carbamoyl}-1-methyl-1H-pyrazole-3-carboxylate obtained in Example 199-A) was used to give the title compound (462 mg) (yield 98%).
MS (ESI+): $[M+H]^+$ 409.
$^1$H NMR (300 MHz, DMSO-$d_6$) δ2.34 (3H, s), 4.12 (3H, s), 4.28 (2H, s), 7.15-7.27 (1H, m), 7.30 (1H, s), 7.32-7.39 (1H, m), 7.59 (1H, s), 10.71 (1H, s).
* The peak of "COOH group" was not observed.

Example 199-C)

$N^5$-[2-(3-chloro-5-fluorobenzyl)-4-methyl-1,3-thiazol-5-yl]-$N^3$-[2-(dimethylamino)ethyl]-1-methyl-1H-pyrazole-3,5-dicarboxamide

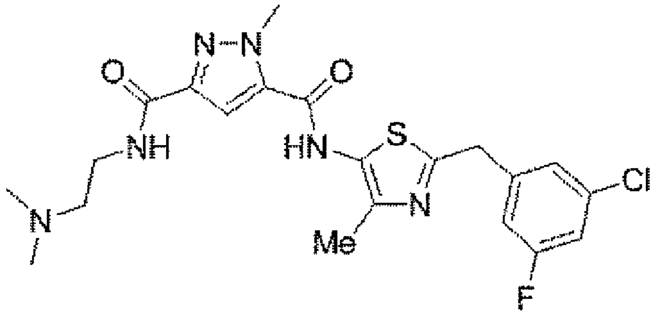

In the same manner as in Example 175, 5-{[2-(3-chloro-5-fluorobenzyl)-4-methyl-1,3-thiazol-5-yl]carbamoyl}-1-methyl-1H-pyrazole-3-carboxylic acid obtained in Example 199-B) and N,N-dimethylethane-1,2-diamine were used to give the title compound (299 mg) (yield 55%).
MS (ESI+): $[M+H]^+$479.
$^1$H NMR (300 MHz, $CDCl_3$) 5 2.19 (6H, s), 2.33 (2H, t, J = 5.9 Hz), 2.42 (3H, s), 3.24 (2H, q, J = 5.7 Hz), 4.17-4.26 (5H, m), 6.88-7.03 (2H, m), 7.12 (1H, s), 7.34 (1H, t, J = 5.1 Hz), 7.73 (1H, s), 9.28 (1H, brs).

Example 200

N-[2-(3-chloro-5-fluorobenzyl)-4-methyl-1,3-thiazol-5-yl]-3-[(dimethylamino)methyl]-1-methyl-1H-pyrazole-5-carboxamide monohydrochloride

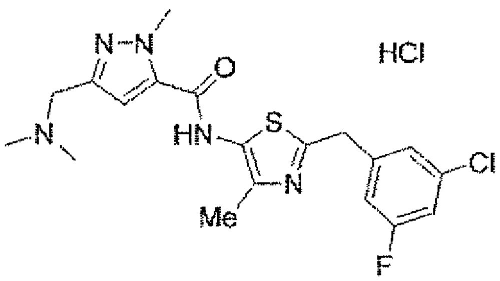

To a solution of ethyl 5-{[2-(3-chloro-5-fluorobenzyl)-4-methyl-1,3-thiazol-5-yl]carbamoyl}-1-methyl-1H-pyrazole-3-carboxylate (1.31 g, 3.00 mmol) obtained in Example 199-A) in THF (30 mL) was added dropwise 1.0M diisobutylaluminum hydride-hexane solution (3.80 mL) under an argon gas atmosphere at -78°C. The reaction mixture was stirred at the same temperature for 30 min. 1.0M Diisobutylaluminum hydride-hexane solution (3.80 mL) was added dropwise, and the mixture was stirred for 30 min, and at 0°C for 1 hr. Methanol was added to terminate the reaction. Saturated aqueous potassium sodium tartarate solution was added, and the mixture was extracted with ethyl acetate, the extract was washed with saturated brine, and dried over anhydrous sodium sulfate. The obtained residue was purified by silica gel column chromatography [eluent: hexane-ethyl acetate (90:10-0:100)] to give a colorless oil (0.22 g). The obtained colorless oil was dissolved in THF (5 mL), and 2M dimethylamine-THF solution (0.28 mL), acetic acid (0.032 mL) and sodium triacetoxyhydroborate (1.06 g, 5.6 mmol) were added. The reaction mixture was stirred at room temperature overnight, basified with saturated aqueous sodium hydrogen carbonate solution and extracted with ethyl acetate. The organic layer was washed with saturated aqueous sodium hydrogen carbonate solution and saturated brine, and dried over anhydrous sodium sulfate. The obtained residue was purified by basic silica gel column chromatography [eluent: hexane-ethyl acetate (4:1-0:1)], the obtained fraction was dissolved in ethyl acetate (3 mL), and 4M hydrogen chloride-ethyl acetate solution (0.2 mL) was added. After stirring for 15 min, the solvent was evaporated under reduced pressure,

and the residue was crystallized from ethyl acetate/hexane to give the title compound (35.6 mg) as a white solid (yield 2.6%).
MS (ESI+): $[M+H]^+$422.
$^1H$ NMR (300 MHz, DMSO-$d_6$) δ2.33 (3H, s), 2.74 (6H, d, J = 4.9 Hz), 4.08 (3H, s), 4.29 (4H, s), 7.13-7.45 (4H, m), 10.69 (1H, brs), 10.90 (1H, s).

Example 201

N-[2-(3-chloro-5-fluorobenzyl)-4-methyl-1,3-thiazol-5-yl]-2-(3,5-dimethyl-1H-pyrazol-1-yl)acetamide

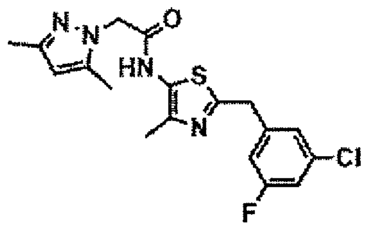

In the same manner as in Example 158, tert-butyl [2-(3-chloro-5-fluorobenzyl)-4-methyl-1,3-thiazol-5-yl]carbamate obtained in Example 145-E) and (3,5-dimethyl-1H-pyrazol-1-yl)acetic acid were used to give the title compound as white crystals (yield 61%).
MS (ESI+): $[M+H]^+$ 393.
$^1H$ NMR (300 MHz, $CDCl_3$) δ 2.25 (3H, s), 2.26 (3H, s), 2.29 (3H, s), 4.15 (2H, s), 4.76 (2H, s), 5.92 (1H, s), 6.85-6.92 (1H, m), 6.96 (1H, dt, J = 8.5, 2.2 Hz), 7.06 (1H, s), 9.72 (1H, s).

Example 202

N-[2-(3-chloro-5-fluorobenzyl)-4-methyl-1,3-thiazol-5-yl]pyrimidine-5-carboxamide

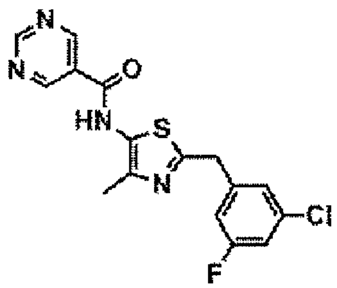

In the same manner as in Example 158, tert-butyl [2-(3-chloro-5-fluorobenzyl)-4-methyl-1,3-thiazol-5-yl]carbamate obtained in Example 145-E) and pyrimidine-5-carboxylic acid were used to give the title compound as white crystals (yield 56%).
MS (ESI+) : $[M+H]^+$ 363.
$^1H$ NMR (300 MHz, $CDCl_3$) δ 2.43 (3H, s), 4.20 (2H, s), 6.88-6.96 (1H, m), 6.98 (1H, dt, J = 8.3, 2.2 Hz), 7.09 (1H, s), 7.88 (1H, br.s.), 9.19 (2H, s), 9.39 (1H, s).

Example 203

2-bromo-N-[2-(3-chloro-5-fluorobenzyl)-4-methyl-1,3-thiazol-5-yl]-4-methyl-1,3-thiazole-5-carboxamide

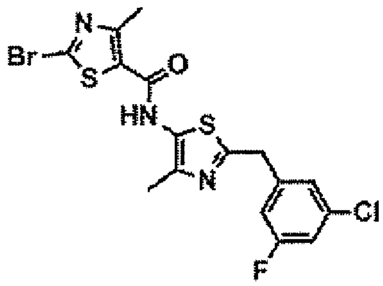

In the same manner as in Example 158, tert-butyl [2-(3-chloro-5-fluorobenzyl)-4-methyl-1,3-thiazol-5-yl]carbamate obtained in Example 145-E) and 2-bromo-4-methyl-1,3-thiazole-5-carboxylic acid were used to give the title compound as white crystals (yield 8%).
MS (ESI+): $[M+H]^+$ 460.
$^1$H NMR (300 MHz, $CDCl_3$) δ 2.39 (3H, s), 2.76 (3H, s), 4.20 (2H, s), 6.89-6.96 (1H, m), 6.95-7.03 (1H, m), 7.09 (1H, s), 7.44 (1H, br.s.).

Example 204

1-methyl-N-{4-methyl-2-[4-(trifluoromethyl)benzyl]-1,3-thiazol-5-yl}-1H-pyrazole-5-carboxamide

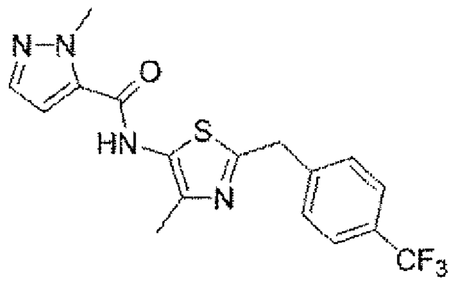

Example 204-A)

2-[4-(trifluoromethyl)phenyl]ethanethioamide

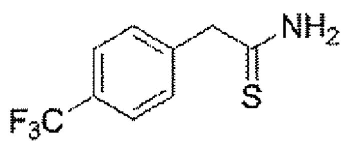

A mixture of [3-(trifluoromethyl)phenyl]acetonitrile (20.0 g, 108 mmol), O,O-diethyl dithiophosphate (18.2 mL, 119 mmol) and 4M hydrogen chloride-dioxane solution (216 mL, 864 mmol) was stirred at room temperature for 18 hr. To the reaction mixture was added water (200 mL), and the mixture was extracted with ethyl acetate (200 mL). The organic layer was washed successively with water (100 mL), 0.2M aqueous sodium hydroxide solution (100 mL) and saturated brine (100 mL), dried over anhydrous magnesium sulfate and concentrated under reduced pressure. The obtained residue was treated with hexane (200 mL) in an ice bath to allow solidification. The solid was collected by filtration and washed with hexane to give the title compound (21.3 g) as a white solid (yield 90%). $^1$H NMR (400 MHz, $CDCl_3$) δ 4.17 (2H, s), 6.61 (1H, brs), 7.46 (2H, d, J = 6.8 Hz), 7.52 (1H, brs), 7.67 (2H, d, J = 7.2 Hz).

Example 204-B)

ethyl 4-methyl-2-[4-(trifluoromethyl)benzyl]-1,3-thiazole-5-carboxylate

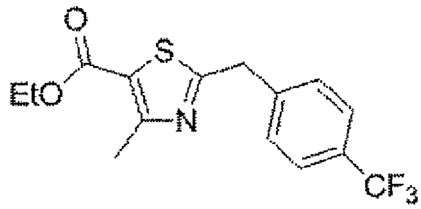

A solution of 2-[4-(trifluoromethyl)phenyl]ethanethioamide (21.3 g, 97.0 mmol) obtained in Example 204-A) and ethyl 2-chloro-3-oxobutanoate (16.1 mL, 117 mmol) in pyridine (240 mL)-ethanol (240 mL) was stirred at 60°C for 18 hr. The reaction mixture was cooled to room temperature, and concentrated under reduced pressure. The obtained residue was diluted with ethyl acetate (1.0 L), washed successively with water (1.0 L), 0.2M hydrochloric acid (500 mL) and saturated brine (500 mL), dried over anhydrous magnesium sulfate, and concentrated under reduced pressure. The obtained residue was purified by silica gel column chromatography [eluent: hexane-ethyl acetate (3:1)] to give the title compound (29.0 g) as yellow crystals (yield 91%). $^1$H NMR (400 MHz, $CDCl_3$) 5 1.32 (3H, t, J = 7.2 Hz), 2.72 (3H, s), 4.28 (2H, q, J = 7.2 Hz), 4.46 (2H, s), 7.40-7.45 (2H, m), 7.52-7.56 (1H, m), 7.70 (1H, d, J = 7.6 Hz).

Example 204-C)

4-methyl-2-[4-(trifluoromethyl)benzyl]-1,3-thiazole-5-carboxylic acid

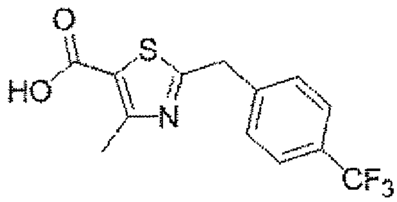

To a solution (147 mL) of ethyl 4-methyl-2-[4-(trifluoromethyl)benzyl]-1,3-thiazole-5-carboxylate (29.0 g, 88.0 mmol) obtained in Example 204-B) in ethanol was added 8N aqueous sodium hydroxide solution (22.0 mL, 176 mmol) at room temperature. The mixture was stirred at room temperature for 3 hr, and 6N hydrochloric acid (26.0 mL) was added thereto in an ice bath. The precipitate was collected by filtration, and washed with water (100 mL) to give the title compound (22.0 g) as a white solid (yield 83%).
MS (ESI+): $[M+H]^+$ 302.
$^1$H NMR (400 MHz, $CDCl_3$) $\delta$ 2.53 (3H, s), 4.38 (2H, s), 7.54 (2H, d, J = 8.4 Hz), 7.68 (2H, d, J = 8.4 Hz), 13.2 (1H, brs).

Example 204-D)

tert-butyl {4-methyl-2-[4-(trifluoromethyl)benzyl]-1,3-thiazol-5-yl}carbamate

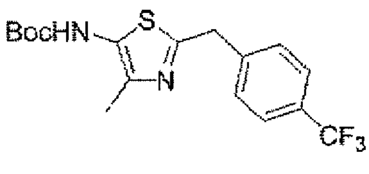

To a solution (177 mL) of 4-methyl-2-[4-(trifluoromethyl)benzyl]-1,3-thiazole-5-carboxylic acid (16.0 g, 53.1

mmol) obtained in Example 204-C) in tert-butyl alcohol were added triethylamine (22.2 mL, 159 mmol) and DPPA (17.2 mL, 80.0 mmol) at room temperature. The mixture was stirred at 95°C for 3 hr. The reaction product was concentrated under reduced pressure, and the residue was dissolved in ethyl acetate (500 mL), washed with water (500 mL) and saturated brine (500 mL), dried over anhydrous magnesium sulfate and concentrated under reduced pressure. The residue was purified by silica gel column chromatography [eluent: hexane-ethyl acetate (3:1)] to give the title compound (7.09 g) as yellow crystals (yield 36%).
MS (ESI+): $[M+H]^+$ 373.
$^1$H NMR (400 MHz, $CDCl_3$) δ 1.49 (9H, s), 2.29 (3H, s), 4.24 (2H, s), 6.41 (1H, brs), 7.41 (2H, d, J = 8.0 Hz), 7.56 (2H, d, J = 8.0 Hz).

Example 204-E)

1-methyl-N-{4-methyl-2-[4-(trifluoromethyl)benzyl]-1,3-thiazol-5-yl}-1H-pyrazole-5-carboxamide

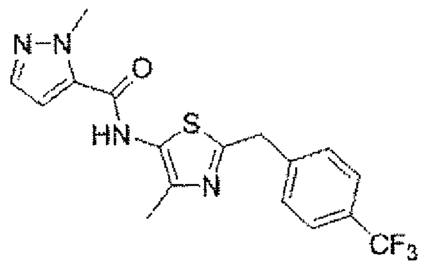

To a solution of tert-butyl {4-methyl-2-[4-(trifluoromethyl)benzyl]-1,3-thiazol-5-yl}carbamate (400 mg, 1.07 mmol) obtained in Example 204-D) in ethanol (10 mL) was added dropwise concentrated hydrochloric acid (3.8 mL) at room temperature, and the mixture was stirred at 50°C for 4 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (10 mL), and 1-methyl-1H-pyrazole-5-carboxylic acid (161 mg, 1.28 mmol), HATU (487 mg, 1.28 mmol) and DIEA (0.088 mL, 0.535 mmol) were added at room temperature. The reaction mixture was stirred at 60°C for 2 hr, saturated aqueous sodium hydrogen carbonate solution was added and the mixture was extracted with ethyl acetate (30 mL). The organic layer was dried over anhydrous magnesium sulfate and concentrated under reduced pressure. The obtained residue was purified by silica gel column chromatography [eluent: hexane-ethyl acetate (4:1-1:2)] and basic silica gel column chromatography [eluent: hexane-ethyl acetate (13:7-1:3)], and crystallized from ethyl acetate-hexane to give the title compound (88.1 mg) as white crystals (yield 22%).
MS (ESI+): $[M+H]^+$ 381.
$^1$H NMR (300 MHz, DMSO-$d_6$) δ 2.30 (3H, s), 4.04 (3H, s), 4.34 (2H, s), 7.06 (1H, d, J = 1.9 Hz), 7.54 (1H, d, J = 1.9 Hz), 7.56 (2H, d, J = 8.0 Hz), 7.72 (2H, d, J = 8.0 Hz), 10.52 (1H, s).

Example 205

N-[2-(3-chloro-5-fluorobenzyl)-4-methyl-1,3-thiazol-5-yl]pyridazine-3-carboxamide

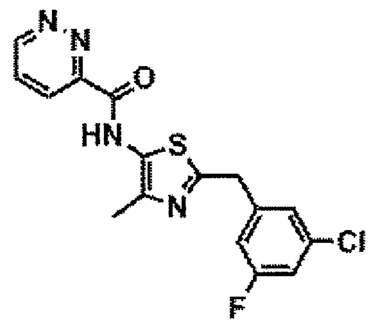

In the same manner as in Example 158, tert-butyl [2-(3-chloro-5-fluorobenzyl)-4-methyl-1,3-thiazol-5-yl]carbamate obtained in Example 145-E) and pyridazine-3-carboxylic acid were used to give the title compound as white crystals (yield 56%).
MS (ESI+): $[M+H]^+$ 363.
$^1H$ NMR (300 MHz, $CDCl_3$) 5 2.55 (3H, s), 4.26 (2H, s), 6.96-7.06 (2H, m), 7.13-7.19 (1H, m), 7.79 (1H, dd, J = 4.8, 8.4 Hz), 8.42 (1H, dd, J = 1.8, 8.4 Hz), 9.40 (1H, dd, J = 1.8, 4.8 Hz), 10.27 (1H, brs).

Example 206

4-chloro-N-[2-(3-chloro-5-fluorobenzyl)-4-methyl-1,3-thiazol-5-yl]-1-methyl-1H-pyrazole-5-carboxamide

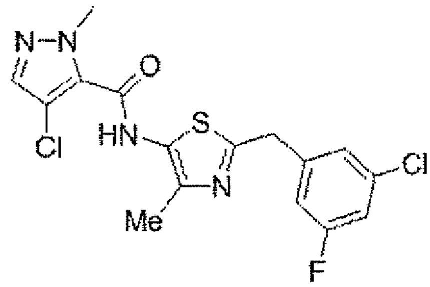

In the same manner as in Example 145-F), tert-butyl [2-(3-chloro-5-fluorobenzyl)-4-methyl-1,3-thiazol-5-yl] carbamate obtained in Example 145-E) and 4-chloro-1-methyl-1H-pyrazole-5-carboxylic acid were used to give the title compound (178 mg) (yield 22%).
MS (ESI+): $[M+H]^+$399.
$^1H$ NMR (300 MHz, DMSO-$d_6$) $\delta$ 2.35 (3H, s), 3.94 (3H, s), 4.27 (2H, s), 7.23 (1H, d, J = 9.8 Hz), 7.28-7.41 (2H, m), 7.72 (1H, s), 11.03 (1H, s).

Example 207

N-[2-(3-chloro-5-fluarobenzyl)-4-methyl-1,3-thiazol-5-yl]-tetrahydro-2H-thiopyran-4-carboxamide

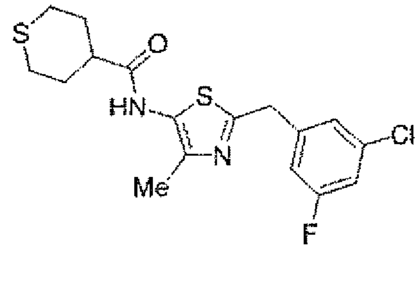

In the same manner as in Example 145-F), tert-butyl [2-(3-chloro-5-fluorobenzyl)-4-methyl-1,3-thiazol-5-yl] carbamate obtained in Example 145-E) and tetrahydro-2H-thiopyran-4-carboxylic acid were used to give the title compound (342 mg) (yield 30%).
MS (ESI+): $[M+H]^+$385.
$^1$H NMR (300 MHz, $CDCl_3$) $\delta$1.88-2.03 (2H, m), 2.17-2.30 (2H, m), 2.34 (3H, s), 2.36-2.44 (1H, m), 2.73 (4H, dd, J = 8.3, 3.4 Hz), 4.16 (2H, s), 6.87-6.94 (1H, m), 6.97 (1H, dt, J = 8.4, 2.2 Hz), 7.08 (1H, s).

Example 208

N-[2-(3-chloro-5-fluorobenzyl)-4-methyl-1,3-thiazol-5-yl]-tetrahydro-2H-thiopyran-4-carboxamide 1,1-dioxide

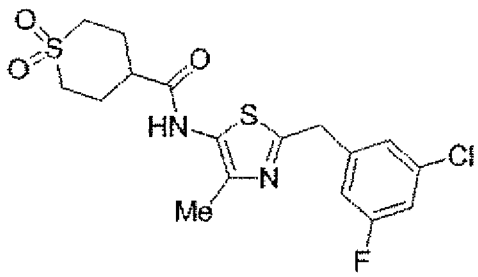

To a solution of N-[2-(3-chloro-5-fluorobenzyl)-4-methyl-1,3-thiazol-5-yl]-tetrahydro-2H-thiopyran-4-carboxamide (317 mg, 0.823 mmol) obtained in Example 207 in ethyl acetate (5 mL)-toluene (5 mL)-DMF (3 mL) was added 3-chloroperbenzoic acid (72%, 395 mg, 1.645 mmol) at 0°C, and the mixture was stirred at room temperature for 2 hr. Saturated aqueous sodium thiosulfate solution was added, and the mixture was stirred for 15 min, and extracted with ethyl acetate. The organic layer was washed successively with saturated aqueous sodium hydrogen carbonate solution and saturated brine, and dried over anhydrous magnesium sulfate. The obtained residue was purified by silica gel column chromatography [eluent: hexane-ethyl acetate (4:1-0:1)], and crystallized from ethyl acetate/hexane to give the title compound (260 mg) as a white solid (yield 76%).
MS (ESI+): $[M+H]^+$417.
$^1$H NMR (300 MHz, DMSO-$d_6$) $\delta$ 2.00-2.23 (4H, m), 2.30 (3H, s), 2.74-2.88 (1H, m), 3.07-3.26 (4H, m), 4.20 (2H, s), 7.18 (1H, dd, J = 9.4, 1.5 Hz), 7.25 (1H, s), 7.32 (1H, dt, J = 9.0, 2.1 Hz), 10.47 (1H, s).

Example 209

1-methyl-N-{4-methyl-2-[2-(trifluoromethyl)benzyl]-1,3-thiazol-5-yl}-1H-pyrazole-5-carboxamide

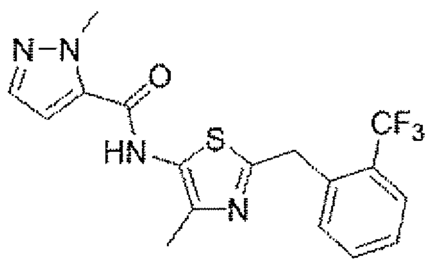

Example 209-A)

2-[2-(trifluoromethyl)phenyl]ethanethioamide

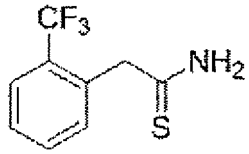

A mixture of [2-(trifluoromethyl)phenyl]acetonitrile (15.0 g, 81.0 mmol), O,O-diethyl dithiophosphate (13.7 mL, 89.0 mmol) and 4M hydrogen chloride-dioxane solution (162 mL, 648 mmol) was stirred at room temperature for 18 hr. To the reaction mixture was added water (200 mL), and the mixture was extracted with ethyl acetate (200 mL). The organic layer was washed successively with water (100 mL), 0.2M aqueous sodium hydroxide solution (100 mL) and saturated brine (100 mL), dried over anhydrous magnesium sulfate and concentrated under reduced pressure. The obtained residue was treated with hexane (200 mL) in an ice bath to allow solidification. The solid was collected by filtration, and washed with hexane to give the title compound (15.7 g) as a white solid (yield 88%). $^1H$ NMR (400 MHz, $CDCl_3$) 5 4.28 (2H, s), 6.58 (1H, brs), 7.44-7.48 (2H, m), 7.58-7.59 (2H, m), 7.72 (1H, d, J = 8.0 Hz).

Example 209-B)

ethyl 4-methyl-2-[2-(trifluoromethyl)benzyl]-1,3-thiazole-5-carboxylate

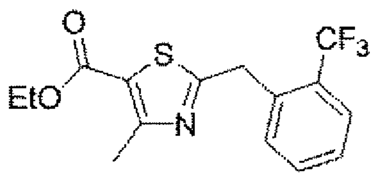

A solution of 2-[2-(trifluoromethyl)phenyl]ethanethioamide (15.7 g, 71.6 mmol) obtained in Example 209-A) and ethyl 2-chloro-3-oxobutanoate (11.9 mL, 86.0 mmol) in pyridine (180 mL)-ethanol (180 mL) was stirred at 60°C for 18 hr. The reaction mixture was cooled to room temperature, and concentrated under reduced pressure. The obtained residue was diluted with ethyl acetate (1.0 L), washed successively with water (1.0 L), 0.2M hydrochloric acid (500 mL) and saturated brine (500 mL), dried over anhydrous magnesium sulfate, and concentrated under reduced pressure. The obtained residue was purified by silica gel column chromatography [eluent: hexane-ethyl acetate (3:1)] to give the title compound (21.4 g) as yellow crystals (yield 91%).
$^1H$ NMR (400 MHz, $CDCl_3$) $\delta$ 1.32 (3H, t, J = 7.2 Hz), 2.72 (3H, s), 4.28 (2H, q, J = 7.2 Hz), 4.46 (2H, s), 7.40-7.45 (2H, m), 7.52-7.56 (1H, m), 7.70 (1H, d, J = 7.6 Hz).

Example 209-C)

4-methyl-2-[2-(trifluoromethyl)benzyl]-1,3-thiazole-5-carboxylic acid

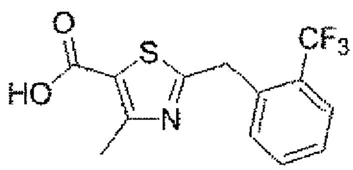

To a solution (110 mL) of ethyl 4-methyl-2-[2-(trifluoromethyl)benzyl]-1,3-thiazole-5-carboxylate (21.4 g, 65.0 mmol) obtained in Example 209-B) in ethanol was added 8M aqueous sodium hydroxide solution (16.2 mL, 130 mmol) at room temperature. The mixture was stirred at room temperature for 3 hr, diluted with ethyl acetate, and 6M hydrochloric acid (22.0 mL) was added to allow solidification in an ice bath. The solid was collected by filtration, and washed with water (100 mL) to give the title compound (17.0 g) as a white solid (yield 87%).
MS (ESI+): $[M+H]^+$ 302.

$^1H$ NMR (400 MHz, $CDCl_3$) δ 2.73 (3H, s), 4.49 (2H, s), 7.41-7.46 (2H, m), 7.53-7.56 (1H, m), 7.71 (1H, d, J = 8.0 Hz).
* The peak of "COOH group" was not observed.

Experimental Example 209-D)

tert-butyl {4-methyl-2-[2-(trifluoromethyl)benzyl]-1,3-thiazol-5-yl}carbamate

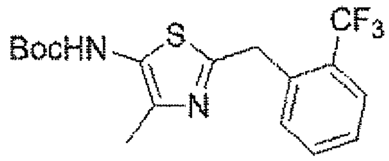

To a solution (177 mL) of 4-methyl-2-[2-(trifluoromethyl)benzyl]-1,3-thiazole-5-carboxylic acid (16.0 g, 53.1 mmol) obtained in Example 209-C) in tert-butyl alcohol were added triethylamine (22.2 mL, 159 mmol) and DPPA (17.2 mL, 80.0 mmol) at room temperature. The mixture was stirred at 95°C for 3 hr. The reaction product was concentrated under reduced pressure, and the residue was dissolved in ethyl acetate (500 mL), washed with water (500 mL) and saturated brine (500 mL), dried over anhydrous magnesium sulfate and concentrated under reduced pressure. The residue was purified by silica gel column chromatography [eluent: hexane-ethyl acetate (3:1)] to give the title compound (13.1 g) as yellow crystals (yield 66%).
MS (ESI+): $[M+H]^+$ 373.
$^1H$ NMR (400 MHz, $CDCl_3$) δ 1.48 (9H, s), 2.30 (3H, s), 4.39 (2H, s), 6.37 (1H, brs), 7.34-7.37 (1H, m), 7.42 (1H, d, J = 7.6 Hz) 7.46-7.50 (1H, m), 7.66 (1H, d, J = 7.6 Hz).

Example 209-E)

1-methyl-N-{4-methyl-2-[2-(trifluoromethyl)benzyl]-1,3-thiazol-5-yl}-1H-pyrazole-5-carboxamide

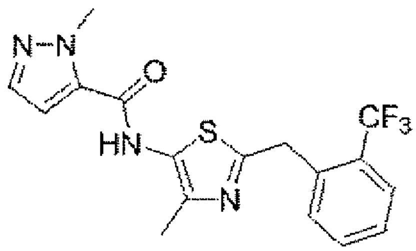

To a solution of tert-butyl {4-methyl-2-[2-(trifluoromethyl)benzyl]-1,3-thiazol-5-yl}carbamate (400 mg, 1.07 mmol) obtained in Example 209-D) in ethanol (10 mL) was added dropwise concentrated hydrochloric acid (4.8 mL) at room temperature, and the mixture was stirred at 50°C for 1.5 hr. The reaction mixture was cooled to room temperature, neutralized with 8M aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (10 mL), and 1-methyl-1H-pyrazole-5-carboxylic acid (161 mg, 1.28 mmol), HATU (487 mg, 1.28 mmol) and DIEA (0.0884 mL, 0.535 mmol) were added at room temperature. The reaction mixture was stirred at 80°C overnight. After cooling to room temperature, saturated aqueous sodium hydrogen carbonate solution was added and the mixture was extracted with ethyl acetate. The organic layer was dried over anhydrous magnesium sulfate and concentrated under reduced pressure. The obtained residue was purified by silica gel column chromatography [eluent: hexane-ethyl acetate (13:7-7:13)] and basic silica gel column chromatography [eluent: hexane-ethyl acetate (7:3-1:1)], and crystallized from ethyl acetate-hexane to give the title compound (107 mg) as white crystals (yield 26%).
MS (ESI+): $[M+H]^+$ 381.
$^1H$ NMR (300 MHz, DMSO-$d_6$) δ 2.30 (3H, s), 4.03 (3H, s), 4.39 (2H, s), 7.05 (1H, d, J = 2.3 Hz), 7.51-7.62 (3H, m), 7.69 (1H, d, J = 7.5 Hz), 7.71-7.83 (1H, m), 10.50 (1H, s).

Example 210

N-(2-{naphthal[3-(trifluoromethyl)phenyl]methyl}-4-methyl-1,3-thiazol-5-yl)-1-methyl-1H-pyrazole-5-carboxamide

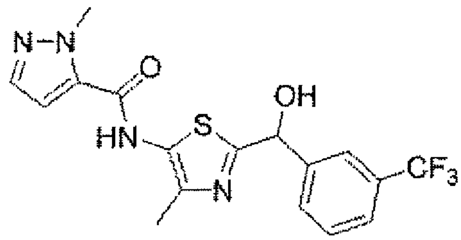

Example 210-A)

tert-butyl (4-methyl-1,3-thiazol-5-yl)carbamate

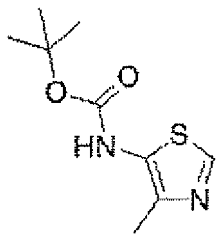

To a solution of 4-methyl-1,3-thiazole-5-carboxylic acid (5.0 g, 34.9 mmol) in tert-butyl alcohol (125 mL) were successively added triethylamine (14.6 mL, 105 mmol) and DPPA (15.1 mL, 69.9 mmol) at room temperature, and the mixture was stirred at 95°C for 1.5 hr. The reaction mixture was cooled to room temperature, saturated aqueous sodium hydrogen carbonate solution was added, and the mixture was extracted with ethyl acetate. The organic layer was washed with saturated brine, dried over anhydrous magnesium sulfate, and concentrated under reduced pressure. The obtained residue was purified by silica gel column chromatography [eluent: hexane-ethyl acetate (2:1-1:3)] to give the title compound (2.34 g) as pale-yellow crystals (yield 78%).
$^1$H NMR (300 MHz, DMSO-$d_6$) $\delta$ 1.47 (9H, s), 2.26 (3H, s), 8.55 (1H, s), 9.73 (1H, brs).

Example 210-B)

1-methyl-N-(4-methyl-1,3-thiazol-5-yl)-1H-pyrazole-5-carboxamide

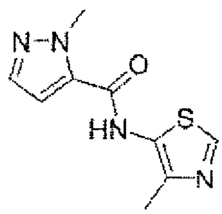

To a solution of tert-butyl (4-methyl-1,3-thiazol-5-yl)carbamate (2.30 g, 10.7 mmol) obtained in Example 210-A) in ethanol (20 mL) was added dropwise concentrated hydrochloric acid (9.3 mL) at room temperature, and the mixture was stirred at 50°C for 1 hr. The reaction mixture was cooled to 0°C, neutralized with 8M aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure.

The obtained residue was dissolved in DMA (35 mL), and 1-methyl-1H-pyrazole-5-carboxylic acid (1.61 g, 12.8 mmol) obtained in Example 1-A), HATU (4.86 g, 12.8 mmol) and DIEA (0.88 mL, 5.35 mmol) were added at room temperature. The reaction mixture was stirred at 60°C for 1.5 hr, water was added and the mixture was extracted with ethyl acetate. The organic layer was washed successively with water, saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (13:7-0:1)] and basic silica gel column chromatography [eluent: hexane-ethyl acetate (13:7-0:1)] to give the title compound (1.41 g) as pale-yellow crystals (yield 59%).
MS (ESI+): $[M+H]^+$ 223.
$^1H$ NMR (300 MHz, DMSO-$d_6$) δ 2.38 (3H, s), 4.08 (3H, s), 7.10 (1H, d, J = 1.9 Hz), 7.56 (1H, d, J = 1.9 Hz), 8.74 (1H, s), 10.59 (1H, s).

Example 210-C)

1-methyl-N-(4-methyl-2-{[3-(trifluoromethyl)phenyl]carbonyl}-1,3-thiazol-5-yl)-1H-pyrazole-5-carboxamide

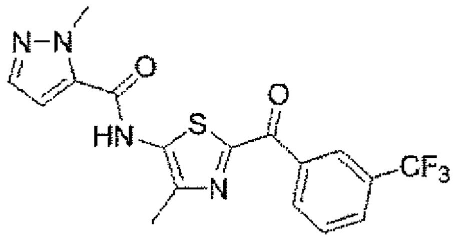

Under an argon atmosphere, 1-methyl-N-(4-methyl-1,3-thiazol-5-yl)-1H-pyrazole-5-carboxamide (300 mg, 1.35 mmol) obtained in Example 210-B) was dissolved in THF (anhydrous; 3 ml), n-butyllithium (1.57M hexane solution, 3.44 mL, 5.40 mmol) was added while cooling at -78°C, and the mixture was stirred at -78°C for 1 hr. To the reaction mixture was added 3-(trifluoromethyl)benzoyl chloride (0.41 mL, 2.70 mmol) at - 78°C, and the mixture was stirred at the same temperature for 30 min. The reaction mixture was warmed to room temperature, saturated aqueous ammonium chloride solution was added and the mixture was extracted with ethyl acetate. The organic layer was washed with saturated brine, dried over anhydrous magnesium sulfate, and concentrated under reduced pressure. The obtained residue was purified by silica gel column chromatography [eluent: hexane-ethyl acetate (13:7-1:3)], and crystallized from ethyl acetate-hexane to give the title compound (21 mg) as pale-yellow crystals (yield 4%).
MS (ESI+): $[M+H]^+$ 395.
$^1H$ NMR (300 MHz, DMSO-$d_6$) δ 2.60 (3H, s), 4.12 (3H, s), 7.20 (1H, d, J = 1.9 Hz), 7.61 (1H, d, J = 1.9 Hz), 7.80-7.90 (1H, m), 8.03-8.12 (1H, m), 8.56-8.72 (2H, m), 11.33 (1H, s).

Example 210-D)

N-(2-{hydroxy[3-(trifluoromethyl)phenyl]methyl}-4-methyl-1,3-thiazol-5-yl)-1-methyl-1H-pyrazole-5-carboxamide

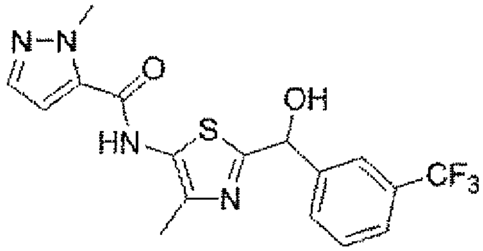

To a solution of 1-methyl-N-(4-methyl-2-{[3-(trifluoromethyl)phenyl]carbonyl}-1,3-thiazol-5-yl)-1H-pyrazole-5-carboxamide (12.6 mg, 0.0320 mmol) obtained in Example 210-C) in methanol (2 mL) was added sodium borohydride

(1.33 mg, 0.0351 mmol) at 0°C, and the mixture was stirred at the same temperature for 10 min. Water was added to the reaction mixture, and the mixture was extracted with ethyl acetate. The organic layer was washed with saturated brine, dried over anhydrous magnesium sulfate, and concentrated under reduced pressure. The obtained residue was purified by silica gel column chromatography [eluent: hexane-ethyl acetate (1:1-0:1)] to give the title compound (13.6 mg) as a colorless oil (yield quant.).
MS (ESI+): $[M+H]^+$ 397.
$^1H$ NMR (300 MHz, DMSO-$d_6$) $\delta$ 2.38 (3H, s), 4.02 (1H, d, J = 3.0 Hz), 4.19 (3H, s), 6.02 (1H, brs), 6.67 (1H, d, J = 1.9 Hz), 7.46-7.83 (6H, m).

Example 211

N-[2-(3-chloro-5-fluorobenzyl)-4-methyl-1,3-thiazol-5-yl]-2-oxoimidazolidine-4-carboxamide

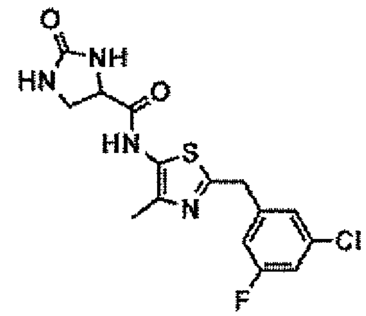

In the same manner as in Example 158, tert-butyl [2-(3-chloro-5-fluorobenzyl)-4-methyl-1,3-thiazol-5-yl]carbamate obtained in Example 145-E) and 2-oxoimidazolidine-4-carboxylic acid were used to give the title compound as white crystals (yield 40%).
MS (ESI+): $[M+H]^+$ 369.
$^1H$ NMR (300 MHz, $CDCl_3$) $\delta$ 2.37 (3H, s), 3.65 (1H, dd, J = 9.3, 6.3 Hz), 4.00 (1H, t, J = 9.8 Hz), 4.17 (2H, s), 4.47-4.58 (1H, m), 4.79 (1H, s), 5.52-5.67 (1H, m), 6.87-6.94 (1H, m), 6.98 (1H, dt, J = 8.3, 2.2 Hz), 7.08 (1H, s), 8.66 (1H, s).

Example 212

N-[2-(3-chloro-5-fluorobenzyl)-4-methyl-1,3-thiazol-5-yl]-2,6-dimethylpyridine-4-carboxamide

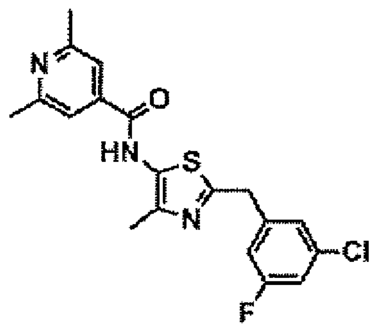

In the same manner as in Example 158, tert-butyl [2-(3-chloro-5-fluorobenzyl)-4-methyl-1,3-thiazol-5-yl]carbamate obtained in Example 145-E) and 2,6-dimethylpyridine-4-carboxylic acid were used to give the title compound as white crystals (yield 61%).
MS (ESI+): $[M+H]^+$ 390.
$^1H$ NMR (300 MHz, $CDCl_3$) $\delta$ 2.44 (3H, s), 2.63 (6H, s), 4.21 (2H, s), 6.90-6.97 (1H, m), 6.99 (1H, dt, J = 8.4, 2.1 Hz), 7.11 (1H, s), 7.33 (2H, s), 7.91 (1H, s).

Example 213

N-[2-(3-chloro-5-fluorobenzyl)-4-methyl-1,3-thiazol-5-yl]-4,6-dimethylpyrimidine-2-carboxamide

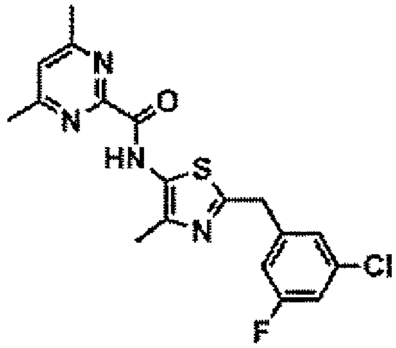

In the same manner as in Example 158, tert-butyl [2-(3-chloro-5-fluorobenzyl)-4-methyl-1,3-thiazol-5-yl]carbamate obtained in Example 145-E) and 4,6-dimethylpyrimidine-2-carboxylic acid were used to give the title compound as white crystals (yield 67%).
MS (ESI+): $[M+H]^+$ 391.
$^1H$ NMR (300 MHz, $CDCl_3$) δ 2.48 (3H, s), 2.62 (6H, s), 4.21 (2H, s), 6.90-7.01 (2H, m), 7.11 (1H, s), 7.20 (1H, s), 10.18 (1H, s).

Example 214

N-[2-(3-chlflro-5-fluorobenzyl)-4-methyl-1,3-thiazol-5-yl]-2-(2,4-dioxo-1,3-diazaspiro[4.4]non-3-yl)acetamide

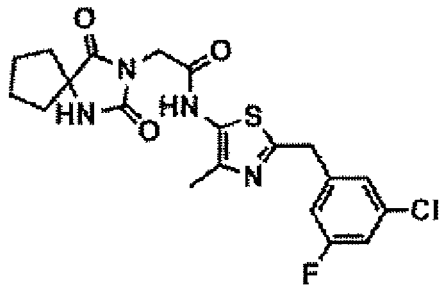

In the same manner as in Example 158, tert-butyl [2-(3-chloro-5-fluorobenzyl)-4-methyl-1,3-thiazol-5-yl]carbamate obtained in Example 145-E) and (2,4-dioxo-1,3-diazaspiro[4.4]non-3-yl)acetic acid were used to give the title compound as white crystals (yield 38%).
MS (ESI+): $[M+H]^+$ 451.
$^1H$ NMR (300 MHz, $CDCl_3$) δ 1.73-2.02 (6H, m), 2.15-2.32 (2H, m), 2.35 (3H, s), 4.16 (2H, s), 4.39 (2H, s), 5.71 (1H, s), 6.87-6.93 (1H, m), 6.97 (1H, dt, J = 8.5, 2.2 Hz), 7.07 (1H, s), 8.68 (1H, s).

Example 215

N-[2-(3-chloro-5-fluorobenzyl)-4-methyl-1,3-thiazol-5-yl]-2-(4-chlorophenyl)-5-methyl-1,3-oxazole-4-carboxamide

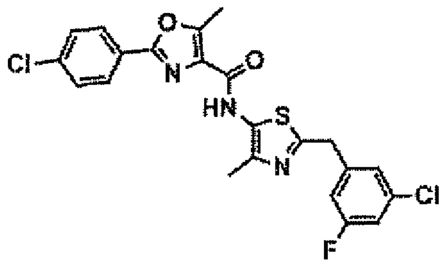

In the same manner as in Example 158, tert-butyl [2-(3-chloro-5-fluorobenzyl)-4-methyl-1,3-thiazol-5-yl]carbamate obtained in Example 145-E) and 2-(4-chlorophenyl)-5-methyl-1,3-oxazole-4-carboxylic acid were used to give the title compound as brown crystals (yield 61%).
MS (ESI+): $[M+H]^+$ 476.
$^1$H NMR (300 MHz, $CDCl_3$) 5 2.48 (3H, s), 2.76 (3H, s), 4.20 (2H, s), 6.89-7.02 (2H, m), 7.11 (1H, s), 7.42-7.52 (2H, m), 7.90-8.05 (2H, m), 8.96 (1H, s).

Example 216

N-[2-(3-chloro-5-fluorobenzyl)-4-methyl-1,3-thiazol-5-yl]-tetrahydrothiophene-3-carboxamide 1,1-dioxide

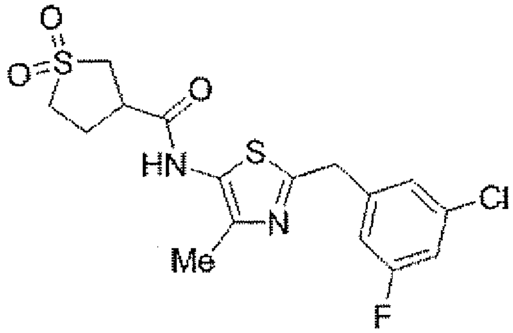

In the same manner as in Example 145-F), tert-butyl [2-(3-chloro-5-fluorobenzyl)-4-methyl-1,3-thiazol-5-yl] carbamate obtained in Example 145-E) and tetrahydrothiophene-3-carboxylic acid 1,1-dioxide were used to give the title compound (452 mg) (yield 56%).
MS (ESI+): $[M+H]^+$403.
$^1$H NMR (300 MHz, DMSO-$d_6$) $\delta$ 2.05-2.26 (1H, m), 2.31 (3H, s), 2.35-2.48 (1H, m), 3.04-3.19 (2H, m), 3.20-3.30 (1H, m), 3.34-3.46 (1H, m), 3.47-3.62 (1H, m), 4.22 (2H, s), 7.17 (1H, d, J = 8.7 Hz), 7.25 (1H, s), 7.32 (1H, dt, J = 8.8, 2.2 Hz), 10.59 (1H, s).

Example 217

1-methyl-N-[4-methyl-2-(3-nitrobenzyl)-1,3-thiazol-5-yl]-1H-pyrazole-5-carboxamide

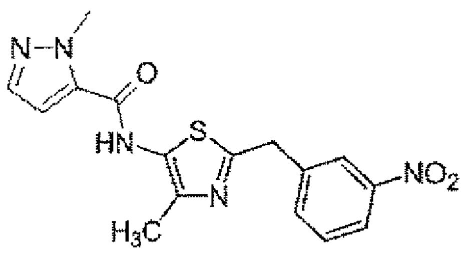

Example 217-A)

2-(3-nitrophenyl)ethanethioamide

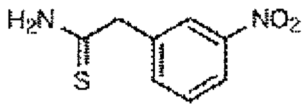

A mixture of (3-nitrophenyl)acetonitrile (5 g, 30.8 mmol), O,O-diethyl dithiophosphate (11.2 g, 60 mmol) and 4N hydrochloric acid-ethyl acetate solution (200 mL) was stirred at room temperature overnight. In an ice bath, water (100 mL) was added and the organic layer was separated, successively washed twice with water (100 mL), twice with 0.2N aqueous sodium hydroxide solution (100 mL), saturated aqueous sodium hydrogen carbonate solution (100 mL) and saturated brine (100 mL), dried over anhydrous sodium sulfate and concentrated under reduced pressure. The obtained residue was recrystallized from ethyl acetate-hexane to give the title compound (4.5 g) as a pale-yellow solid (yield 74%).
$^1$H NMR (300 MHz, $CDCl_3$) $\delta$ 4.14 (2H, s), 6.89 (1H, brs), 7.32-7.99 (3H, m), 7.95-8.54 (2H, m).

Example 217-B)

ethyl 4-methyl-2-(3-nitrobenzyl)-1,3-thiazole-5-carboxylate

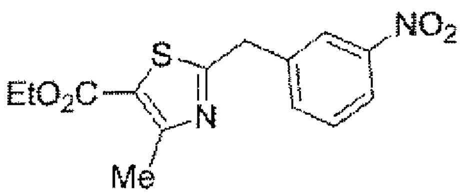

A solution of 2-(3-nitrophenyl)ethanethioamide (1.0 g, 5.1 mmol) obtained in Example 217-A) and ethyl 2-chloro-3-oxobutanoate (1.0 g, 6.12 mmol) in pyridine (3 mL)-ethanol (3 mL) was stirred at room temperature for 2 days. The reaction mixture was concentrated under reduced pressure, azeotropically distilled twice with toluene, diluted with ethyl acetate, washed successively with 0.2N hydrochloric acid and saturated brine, dried over anhydrous magnesium sulfate, and concentrated under reduced pressure. The obtained residue was purified by silica gel column chromatography [eluent: hexane-ethyl acetate (10:1-2:1)] to give the title compound (880 mg) as a solid (yield 56%).
$^1$H NMR (300 MHz, $CDCl_3$) $\delta$ 1.34 (3H, t, J = 7.2 Hz), 2.72 (3H, s), 4.30 (2H, q, J = 6.9 Hz), 4.38 (2H, s), 7.41-7.60 (1H, m), 7.60-7.80 (1H, m), 8.02-8.34 (2H, m).

Example 217-C)

4-methyl-2-(3-nitrobenzyl)-1,3-thiazole-5-carboxylic acid

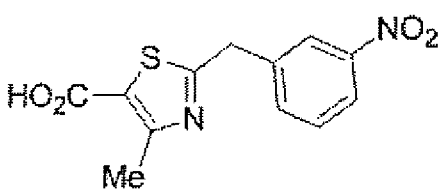

To a solution of ethyl 4-methyl-2-(3-nitrobenzyl)-1,3-thiazole-5-carboxylate (880 mg, 2.9 mmol) obtained in Example 217-B) in methanol (2 mL)-THF (8 mL) was added 2N aqueous sodium hydroxide solution (2 mL), and the

mixture was stirred overnight. The reaction mixture was concentrated under reduced pressure, 1N hydrochloric acid (4 mL) was added in an ice bath, and the mixture was extracted with ethyl acetate. The organic layer was washed with saturated brine, dried over anhydrous magnesium sulfate, and concentrated under reduced pressure. The obtained residue was crystallized from hexane-ethyl acetate to give the title compound (580 mg) as a yellow solid (yield 72%).
$^1$H NMR (300 MHz, DMSO-$d_6$) $\delta$ 2.58 (3H, s), 4.50 (2H, s), 7.59-7.75 (1H, m), 7.84 (1H, d, J = 7.6 Hz), 8.16 (1H, dt, J = 8.3, 1.1 Hz), 8.27 (1H, s).
* The peak of "COOH group" was not observed.

Example 217-D)

tert-butyl [4-methyl-2-(3-nitrobenzyl)-1,3-thiazol-5-yl]carbamate

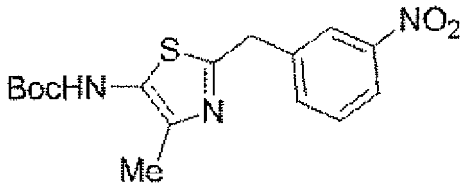

A mixture of 4-methyl-2-(3-nitrobenzyl)-1,3-thiazole-5-carboxylic acid (580 mg, 2.09 mmol) obtained in Example 217-C), DPPA (1.12 mL, 5.23 mmol), triethylamine (0.58 mL, 4.18 mmol) and tert-butyl alcohol (10 mL) was stirred at 90°C for 2 hr. The reaction mixture was cooled to room temperature, and concentrated under reduced pressure. The obtained residue was purified by silica gel column chromatography [eluent: hexane-ethyl acetate (9:1-1:1)] to give the title compound (450 mg) as a pale-yellow oil (yield 62%).
$^1$H NMR (300 MHz, $CDCl_3$) $\delta$ 1.49 (9H, s), 2.30 (3H, s), 4.29 (2H, s), 6.45 (1H, brs), 7.42-7.55 (1H, m), 7.59-7.69 (1H, m), 8.07-8.15 (1H, m), 8.15-8.22 (1H, m).

Example 217-E)

1-methyl-N-[4-methyl-2-(3-nitrobenzyl)-1,3-thiazol-5-yl]-1H-pyrazole-5-carboxamide

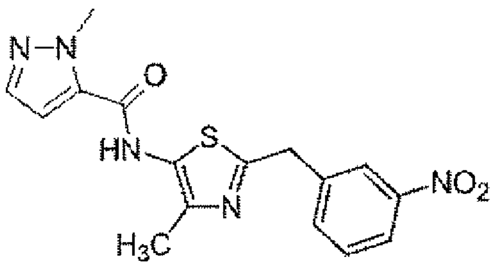

To a solution of tert-butyl [4-methyl-2-(3-nitrobenzyl)-1,3-thiazol-5-yl]carbamate (450 mg, 1.29 mmol) obtained in Example 217-D) in methanol (1 mL) was added dropwise 6N hydrochloric acid (1 mL), and the mixture was stirred at 50°C for 1 hr. The reaction mixture was cooled to 0°C, neutralized with 8M aqueous sodium hydroxide solution (0.75 mL), adjusted to pH 9-10 with saturated aqueous sodium hydrogen carbonate solution, and extracted with ethyl acetate. The organic layer was washed with saturated brine, dried over anhydrous sodium sulfate, and concentrated under reduced pressure. The obtained residue was dissolved in DMA (7 mL), and 1-methyl-1H-pyrazole-5-carboxylic acid (184 mg, 1.46 mmol), HATU (293 mg, 1.68 mmol) and DIEA (0.13 mL, 0.73 mmol) were added at 0°C. The reaction mixture was stirred at 60°C for 2 hr, water was added and the mixture was extracted with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-2:8)] to give the title compound (100 mg) as a solid (yield 33%).

MS ESI+): [M+H]+358.
$^1$H NMR (300 MHz, $CDCl_3$) δ 2.40 (3H, s), 4.20 (3H, s), 4.35 (2H, s), 6.69 (1H, d, J = 2.3 Hz), 7.44-7.58 (2H, m), 7.62-7.72 (1H, m), 7.78 (1H, brs), 8.13 (1H, ddd, J = 8.3, 2.3, 1.1 Hz), 8.16-8.21 (1H, m).

Example 218

N-[2-(3-chloro-5-fluorobenzyl)-4-methyl-1,3-thiazol-5-yl]-2-(4-methoxyphenyl)-1,2-thiazinane-6-carboxamide 1,1-dioxide

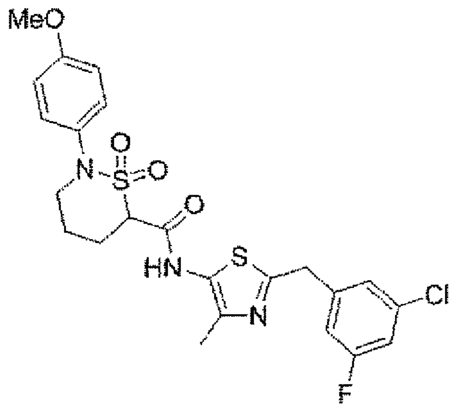

Example 218-A)

ethyl 2-(4-methoxyphenyl)-1,2-thiazinane-6-carboxylate 1,1-dioxide

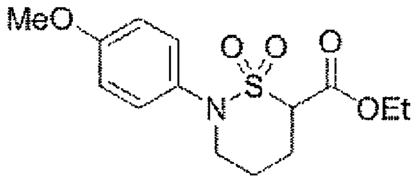

To a solution of ethyl chloroacetate (16.0 mL, 150 mmol) in ethanol (75 mL)-water (75 mL) was added sodium sulfite (12.6 g, 100 mmol), and the mixture was stirred overnight at 85°C. The mixture was cooled to room temperature, azeotropically distilled with toluene, and concentrated under reduced pressure. To a solution of the obtained residue and DMF (0.77 mL, 10 mmol) in toluene (100 mL) was added dropwise oxalyl chloride (19.0 mL, 200 mmol) at room temperature, and the mixture was stirred at 100°C for 4 hr. The reaction mixture was cooled to room temperature, filtered through celite, and the filtrate was concentrated under reduced pressure. To a solution of pyridine (0.239 mL, 2.95 mmol) and p-anisidine (363 mg, 2.95 mmol) in acetonitrile (25 mL) was added dropwise the obtained residue at 0°C. The reaction mixture was stirred at the same temperature for 5 min, and at room temperature for 10 min. Water was added to the reaction mixture, and the mixture was adjusted to pH≤2 with concentrated hydrochloric acid, and extracted with ethyl acetate. The organic layer was dried over anhydrous magnesium sulfate and concentrated under reduced pressure. To a solution of the obtained residue and potassium carbonate (668 mg, 4.83 mmol) in DMF (40 mL) was added dropwise 1-bromo-3-chloropropane (0.191 mL, 1.93 mmol) at 60°C over 1.5 hr. The mixture was stirred at the same temperature for 2.5 hr. The reaction mixture was cooled to room temperature, adjusted to pH≤2 with concentrated hydrochloric acid, and extracted with ethyl acetate. The organic layer was washed with 0.1N hydrochloric acid, dried over anhydrous magnesium sulfate and concentrated under reduced pressure. The obtained residue was purified by silica gel column chromatography [eluent: hexane-ethyl acetate (17:3-3:2)] to give the title compound (134 mg) as colorless crystals (yield 0.43%).
$^1$H NMR (300 MHz, $CDCl_3$) δ 1.30 (3H, t, J = 7.2 Hz), 1.82-2.00 (1H, m), 2.05-2.18 (1H, m), 2.43-2.67 (2H, m), 3.58-3.69 (1H, m), 3.79 (3H, s), 3.85-3.97 (1H, m), 4.00-4.08 (1H, m), 4.18-4.37 (2H, m), 6.83-6.90 (2H, m), 7.22-7.30 (2H, m).

Example 218-B)

2-(4-methoxyphenyl)-1,2-thiazinane-6-carboxylic acid 1,1-dioxide

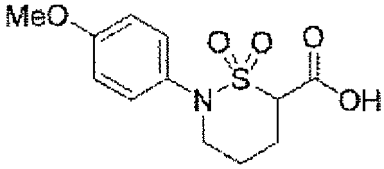

To a solution of ethyl 2-(4-methoxyphenyl)-1,2-thiazinane-6-carboxylate 1,1-dioxide (134 mg, 0.428 mmol) obtained in Example 218-A) in methanol (3 mL)-water (3 mL) was added a solution of potassium hydroxide (28.8 mg, 0.513 mmol) in water (0.259 mL) at room temperature, and the mixture was stirred at the same temperature for 30 min. Water was added to the mixture, and the mixture was concentrated under reduced pressure. The obtained residue was adjusted to pH≤2 with 0.1N hydrochloric acid, and the mixture was extracted with ethyl acetate. The organic layer was dried over anhydrous magnesium sulfate, and concentrated under reduced pressure to give the title compound as a colorless oil (yield 92%). $^1$H NMR (300 MHz, DMSO-$d_6$) 5 1.71-2.00 (2H, m), 2.19-2.39 (2H, m), 3.41-3.52 (1H, m), 3.76 (3H, s), 3.78-3.87 (1H, m), 4.23-4.31 (1H, m), 6.90-6.97 (2H, m), 7.21-7.32 (2H, m), 13.33 (1H, brs).

Example 218-C)

N-[2-(3-chloro-5-fluorobenzyl)-4-methyl-1,3-thiazol-5-yl]-2-(4-methoxyphenyl)-1,2-thiazinane-6-carboxamide 1,1-dioxide

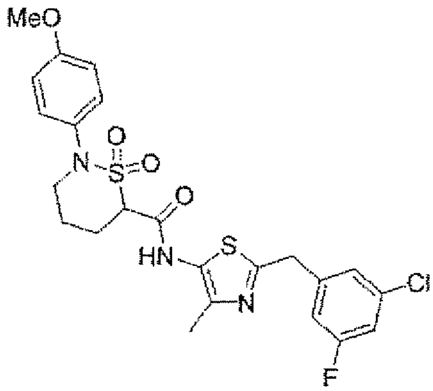

To a solution of tert-butyl [2-(3-chloro-5-fluorobenzyl)-4-methyl-1,3-thiazol-5-yl]carbamate (116 mg, 0.325 mmol) obtained in Example 145-E) in ethanol (2 mL) was added dropwise concentrated hydrochloric acid (0.500 mL) at room temperature, and the mixture was stirred at 50°C for 1.5 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (3 mL), and 2-(4-methoxyphenyl)-1,2-thiazinane-6-carboxylic acid 1,1-dioxide (111 mg, 0.390 mmol) obtained in Example 218-B), HATU (148 mg, 0.390 mmol) and DIEA (0.269 mL, 0.163 mmol) were added at room temperature. The reaction mixture was stirred at 60°C for 1.5 hr, cooled to room temperature, saturated aqueous sodium hydrogen carbonate solution was added and the mixture was extracted with ethyl acetate. The organic layer was dried over anhydrous magnesium sulfate and concentrated under reduced pressure. The obtained residue was purified by silica gel column chromatography [eluent: hexane-ethyl acetate (3:2-7:13)] and basic silica gel column chromatography [eluent: hexane-ethyl acetate (2:3-1:4)], and crystallized from ethyl acetate-hexane to give the title compound (65.1 mg) as colorless crystals (yield 38% . MS (ESI+): $[M+H]^+$ 524.
$^1$H NMR (300 MHz, DMSO-$d_6$) 5 1.85-2.02 (2H, m), 2.28 (3H, s), 2.30-2.37 (1H, m), 2.37-2.47 (1H, m), 3.50-3.63 (1H,

m), 3.76 (3H, s), 3.77-3.89 (1H, m), 4.23 (2H, s), 4.41-4.49 (1H,m), 6.90-6.99 (2H, m), 7.15-7.22 (1H, m), 7.23-7.36 (4H, m), 10.72 (1H, s).

Example 219

N-[2-(3-methoxybenzyl)-4-methyl-l,3-thiazol-5-yl]-1-methyl-1H-pyrazole-5-carboxamide

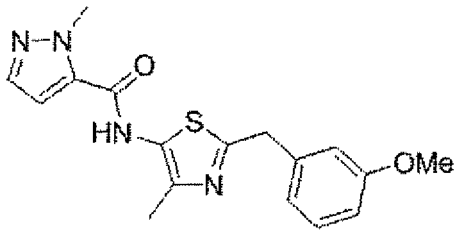

Example 219-A)

2-(3-methoxyphenyl)ethanethioamide

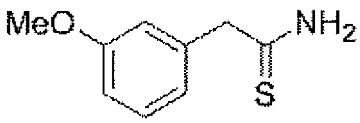

A mixture of (3-methoxyphenyl)acetonitrile (13.0 g, 88.0 mmol), O,O-diethyl dithiophosphate (14.9 mL, 97.0 mmol) and 4M hydrogen chloride-1,4-dioxane (177 mL, 707 mmol) was stirred at room temperature for 18 hr. The reaction mixture was diluted with ethyl acetate (200 mL), water (200 mL) was added, and the organic layer was separated, washed successively with water (100 mL), 0.2N aqueous sodium hydroxide solution (100 mL) and saturated brine (100 mL), dried over anhydrous magnesium sulfate and concentrated under reduced pressure. The obtained residue was treated with hexane (200 mL) in an ice bath to allow solidification. The solid was collected by filtration, and washed with hexane to give the title compound (12.6 g) as a white solid (yield 79%).
$^1$H NMR (400 MHz, $CDCl_3$) 5 3.81 (3H, s), 4.10 (2H, s), 6.49 (1H, brs), 6.80-6.81 (1H, m), 6.84-6.89 (2H, m), 7.31 (1H, t, J = 8.0 Hz), 7.46 (1H, brs).

Example 219-B)

ethyl 2-(3-methoxybenzyl)-4-methyl-1,3-thiazole-5-carboxylate

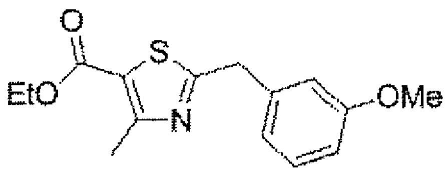

A solution of 2-(3-methoxyphenyl)ethanethioamide (12.6 g, 69.5 mmol) obtained in Example 219-A) and ethyl 2-chloro-3-oxobutanoate (11.5 mL, 83.0 mmol) in pyridine (116 mL)-ethanol (116 mL) was stirred at 60°C for 18 hr. The reaction mixture was cooled to room temperature, and concentrated under reduced pressure. The obtained residue was diluted with ethyl acetate (1.0 L), and water (1.0 L) was added. The organic layer was separated, washed successively with 0.2N hydrochloric acid (500 mL) and saturated brine (500 mL), dried over anhydrous magnesium sulfate, and concentrated under reduced pressure. The obtained residue was purified by silica gel column chromatography [eluent:

hexane-ethyl acetate (3:1)] to give the title compound (20.0 g) as yellow crystals (yield 99%). $^1$H NMR (400 MHz, $CDCl_3$) 5 1.32 (3H, t, J = 7.2 Hz), 2.71 (3H, s), 3.80 (3H, s), 4.23 (2H, s), 4.28 (2H, q, J = 7.2 Hz), 6.82-6.85 (2H, m), 6.90 (1H, d, J = 8.0 Hz), 7.25-7.29 (1H, m).

Example 219-C)

2-(3-methoxybenzyl)-4-methyl-1,3-thiazole-5-carboxylic acid

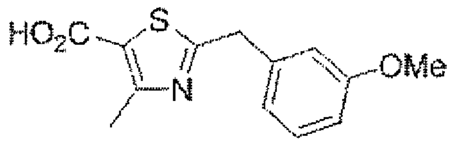

To a solution (114 mL) of ethyl 2-(3-methoxybenzyl)-4-methyl-1,3-thiazole-5-carboxylate (20.0 g, 68.6 mmol) obtained in Example 219-B) in ethanol was added 8N aqueous sodium hydroxide solution (17.2 mL, 137 mmol) at room temperature, and the mixture was stirred at the same temperature for 3 hr. The reaction mixture was ice-cooled, and neutralized with 6N hydrochloric acid. The precipitate was collected by filtration, and washed with water (200 mL) to give the title compound (16.4 g) as a white solid (yield 91%).
MS (ESI+): $[M+H]^+$ 264.
$^1$H NMR (400 MHz, $COCl_3$) $\delta$ 2.58 (3H, s), 3.74 (3H, s), 4.25 (2H, s), 6.86 (1H, dd, J = 8.0, 2.4 Hz), 6.90-6.93 (2H, m), 7.27 (1H, t, J = 8.0 Hz), 13.2 (1H, brs).

Example 219-D)

tert-butyl [2-(3-methoxybenzyl)-4-methyl-1,3-thiazol-5-yl]carbamate

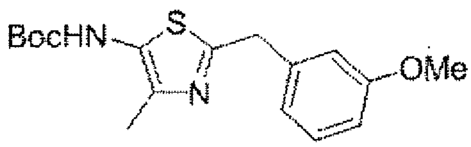

To a solution (192 mL) of 2-(3-methoxybenzyl)-4-methyl-1,3-thiazole-5-carboxylic acid (15.2 g, 57.7 mmol) obtained in Example 219-C) in tert-butyl alcohol was added triethylamine (24.1 mL, 173 mmol) at room temperature, and the mixture was heated to 95°C and DPPA (18.7 mL, 87.0 mmol) was added. The mixture was stirred at the same temperature for 3 hr. The reaction product was concentrated under reduced pressure, and the residue was dissolved in ethyl acetate (500 mL), and water (500 mL) was added. The organic layer was separated, washed with saturated brine (500 mL), dried over anhydrous magnesium sulfate and concentrated under reduced pressure. The residue was purified by silica gel column chromatography [eluent: hexane-ethyl acetate (3:1)] to give the title compound (7.14 g) as yellow crystals (yield 37%).
MS (ESI+): $[M+H]^+$335.
$^1$H NMR (400MHz, $CDCl_3$) $\delta$ 1.48 (9H, s), 2.28 (3H, s), 3.79 (3H, s), 4.16 (2H, s), 6.33 (1H, brs), 6.79 (1H, dd, J = 8.4, 2.4 Hz), 6.84 (1H, s), 6.89 (1H, d, J = 7.2 Hz), 7.22 (1H, t, J = 8.0 Hz).

Example 219-E)

N-[2-(3-methoxybenzyl)-4-methyl-1,3-thiazol-5-yl]-1-methyl-1H-pyrazole-5-carboxamide

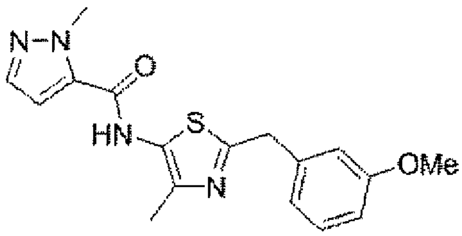

To a solution of tert-butyl [2-(3-methoxybenzyl)-4-methyl-1,3-thiazol-5-yl]carbamate (1.0 g, 2.99 mmol) obtained in Example 219-D) in ethanol (9 mL) was added dropwise concentrated hydrochloric acid (2.3 mL) at room temperature, and the mixture was stirred at 50°C for 1.5 hr. The reaction mixture was cooled to 0°C, neutralized with 8M aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (20 mL), and 1-methyl-1H-pyrazole-5-carboxylic acid (453 mg, 3.59 mmol), HATU (1.37 g, 3.59 mmol) and DIEA (0.247 mL, 1.50 mmol) were added at room temperature. The reaction mixture was stirred at 60°C for 2 hr, water and 0.1N hydrochloric acid were added and the mixture was extracted with ethyl acetate. The organic layer was washed with saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (13:7-1:4)] and basic silica gel column chromatography [eluent: hexane-ethyl acetate (3:2-3:7)], and crystallized from ethyl acetate-hexane to give the title compound (545 mg) as colorless crystals (yield 53%).
MS (ESI+): $[M+H]^+$ 343.
$^1$H NMR (300 MHz, DMSO-$d_6$) $\delta$ 2.30 (3H, s), 3.74 (3H, s), 4.04 (3H, s), 4.17 (2H, s), 6.80-6.94 (3H, m), 7.06 (1H, d, J = 2.3 Hz), 7.22-7.30 (1H, m), 7.53 (1H, d, J = 2.3 Hz), 10.47 (1H, s).

Example 220

N-[2-(3,5-difluorobenzyl)-4-methyl-1,3-thiazol-5-yl]-1-methyl-1H-pyrazole-5-carboxamide

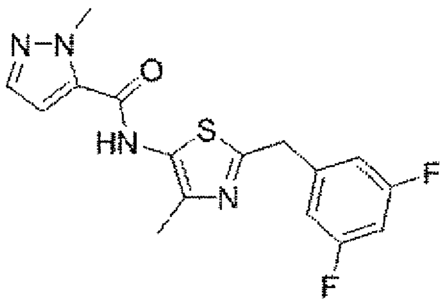

Example 220-A)

2-(3,5-difluorophenyl)ethanethioamide

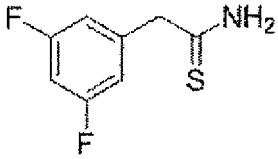

A mixture of (3,5-difluorophenyl)acetonitrile (5.0 g, 32.7 mmol), O,O-diethyl dithiophosphate (6.02 mL, 35.9 mmol) and 4N hydrogen chloride-ethanol (60 mL) was stirred at room temperature overnight. Water was added to the

reaction solution at 0°C, and the organic layer was separated, washed successively with water, 0.1N aqueous sodium hydroxide solution, saturated aqueous sodium hydrogen carbonate solution and saturated brine, dried over anhydrous magnesium sulfate and concentrated under reduced pressure. The obtained residue was treated with hexane in an ice bath to allow solidification. The solid was collected by filtration, and washed with hexane to give the title compound (4.90 g) as a white solid (yield 80%).
MS (ESI+): $[M+H]^+$ 188.
$^1H$ NMR (300 MHz, DMSO-$d_6$) $\delta$ 3.84 (2H, s), 6.99-7.17 (3H, m), 9.25-9.70 (2H, m).

Example 220-B)

ethyl 2-(3,5-difluorobenzyl)-4-methyl-1,3-thiazole-5-carboxylate

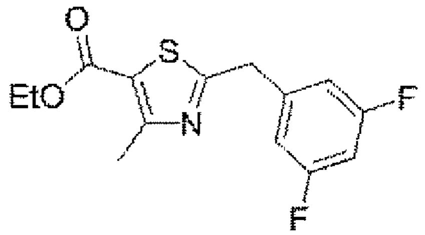

A solution of 2-(3,5-difluorophenyl)ethanethioamide (4.0 g, 21.4 mmol) obtained in Example 220-A) and ethyl 2-chloro-3-oxobutanoate (3.55 mL, 25.6 mmol) in pyridine (10 mL)-ethanol (10 mL) was stirred at room temperature for 3 days. The reaction mixture was concentrated under reduced pressure, and azeotropically distilled with toluene. The obtained residue was diluted with ethyl acetate, and water was added. The organic layer was separated, washed successively with 0.1N hydrochloric acid and saturated brine, dried over anhydrous magnesium sulfate, and concentrated under reduced pressure. The obtained residue was purified by basic silica gel column chromatography [eluent: hexane-ethyl acetate (9:1-4:1)] to give the title compound (5.70 g) as yellow crystals (yield 90%).
$^1H$ NMR (300 MHz, DMSO-$d_6$) $\delta$ 1.26 (3H, t, J = 7.1 Hz), 2.60 (3H, s), 4.24 (2H, q, J = 7.1 Hz), 4.37 (2H, s), 7.06-7.23 (3H, m).

Example 220-C)

2-(3,5-difluorobenzyl)-4-methyl-1,3-thiazole-5-carboxylic acid

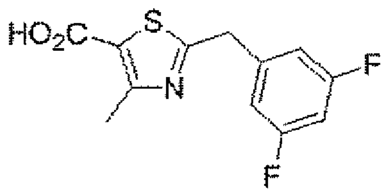

To a solution (30 mL) of ethyl 2-(3,5-difluorobenzyl)-4-methyl-1,3-thiazole-5-carboxylate (5.00 g, 16.8 mmol) obtained in Example 220-B) in ethanol was added dropwise 8N aqueous sodium hydroxide solution (4.70 mL) at room temperature, and the mixture was stirred at the same temperature for 50 min. The reaction mixture was ice-cooled, and 6N hydrochloric acid (6 mL) and water (20 mL) were added. The precipitate was collected by filtration, and washed with water to give the title compound (3.91 g) as a white solid (yield 86%).
MS (ESI+): $[M+H]^+$ 270.
$^1H$ NMR (300 MHz, DMSO-$d_6$) $\delta$ 2.58 (3H, s), 4.35 (2H, s), 7.04-7.24 (3H, m), 13.23 (1H, brs).

Example 220-D)

tert-butyl [2-(3,5-difluorobenzyl)-4-methyl-1,3-thiazol-5-yl]carbamate

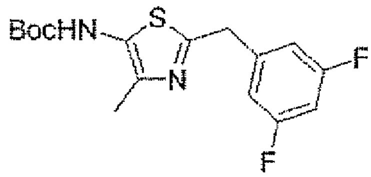

To a solution (40 mL) of 2-(3,5-difluorobenzyl)-4-methyl-1,3-thiazole-5-carboxylic acid (3.80 g, 14.1 mmol) obtained in Example 220-C) in tert-butyl alcohol was added triethylamine (5.90 mL, 42.3 mmol) at room temperature, and the mixture was heated to 90°C and DPPA (4.60 mL, 21.2 mmol) was added. The reaction mixture was stirred at the same temperature for 1 hr. The reaction product was concentrated under reduced pressure, and the residue was dissolved in ethyl acetate, and water was added. The organic layer was separated, and washed with 0.1N aqueous sodium hydroxide solution and saturated brine, dried over anhydrous magnesium sulfate and concentrated under reduced pressure. The residue was purified by silica gel column chromatography [eluent: hexane-ethyl acetate (3:1-1:1)] to give the title compound (1.74 g) as yellow crystals (yield 36%).
$^1$H NMR (300 MHz, DMSO-$d_6$) $\delta$ 1.44 (9H, s), 2.19 (3H, s), 4.19 (2H, s), 6.99-7.19 (3H, m), 9.63 (1H, brs).

Example 220-E)

N-[2-(3,5-difluorobenzyl)-4-methyl-,3-thiazol-5-yl]-1-methyl-1H-pyrazole-5-carboxamide

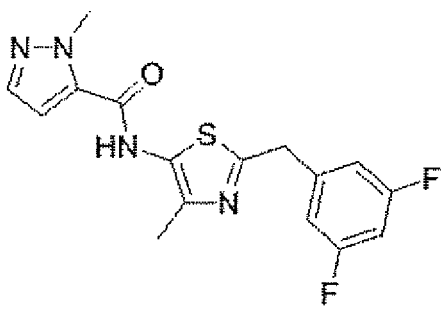

To a solution of tert-butyl [2-(3,5-difluorobenzyl)-4-methyl-1,3-thiazol-5-yl]carbamate (300 mg, 0.881 mmol) obtained in Example 220-D) in ethanol (2 mL) was added dropwise concentrated hydrochloric acid (0.630 mL) at room temperature, and the mixture was stirred at 50°C for 3.5 hr. The reaction mixture was cooled to 0°C, neutralized with 8N aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (8 mL), and 1-methyl-1H-pyrazole-5-carboxylic acid (134 mg, 1.06 mmol), HATU (403 mg, 1.06 mmol) and DIEA (0.0728 mL, 0.441 mmol) were added at room temperature. The reaction mixture was stirred at 60°C for 3.5 hr, cooled to room temperature, water and 0.1N hydrochloric acid were added and the mixture was extracted with ethyl acetate. The organic layer was washed with saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (3:2-3:7)] and basic silica gel column chromatography [eluent: hexane-ethyl acetate (3:2-3:7)], and crystallized from ethyl acetate-hexane to give the title compound (144 mg) as colorless crystals (yield 47%).
MS (ESI+): $[M+H]^+$ 349.
$^1$H NMR (300 MHz, DMSO-$d_6$) $\delta$ 2.31 (3H, s), 4.05 (3H, s), 4.26 (2H, s), 7.04-7.17 (4H, m), 7.54 (1H, d, J = 2.3 Hz), 10.53 (1H, s).

Example 221

1-methyl-N-[4-methyl-2-(3-methylbenzyl)-1,3-thiazol-5-yl]-1H-pyrazole-5-carboxamide

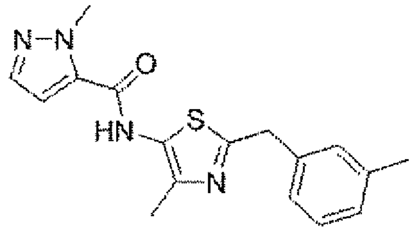

Example 221-A)

2-(3-methylphenyl)ethanethioamide

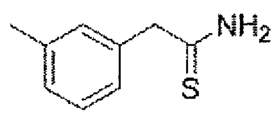

A mixture of (3-methylphenyl)acetonitrile (12.0 g, 91.0 mmol), O,O-diethyl dithiophosphate (15.4 mL, 101 mmol) and 4N hydrogen chloride-1,4-dioxane (183 mL, 732 mmol) was stirred at room temperature for 18 hr. The reaction mixture was diluted with ethyl acetate (200 mL), water (200 mL) was added, and the organic layer was separated, washed successively with water (100 mL), 0.2N aqueous sodium hydroxide solution (100 mL) and saturated brine (100 mL), dried over anhydrous magnesium sulfate and concentrated under reduced pressure. The obtained residue was treated with hexane (200 mL) in an ice bath to allow solidification. The solid was collected by filtration, and washed with hexane to give the title compound (12.0 g) as a white solid (yield 79%).
$^1$H NMR (400 MHz, $CDCl_3$) δ 2.36 (3H, s), 4.08 (2H, s), 6.66 (1H, brs), 7.05-7.08 (2H, m), 7.14 (1H, d, J = 7.6 Hz), 7.28 (1H, t, J = 7.6 Hz), 7.52 (1H, brs).

Example 221-B)

ethyl 4-methyl-2-(3-methylbenzyl)-1,3-thiazole-5-carboxylate

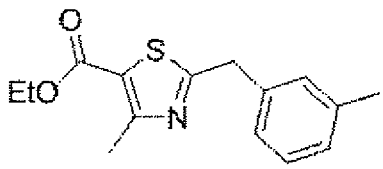

A solution of 2-(3-methylphenyl)ethanethioamide (11.5 g, 69.6 mmol) obtained in Example 221-A) and ethyl 2-chloro-3-oxobutanoate (11.6 mL, 84.0 mmol) in pyridine (116 mL)-ethanol (116 mL) was stirred at 60°C for 18 hr. The reaction mixture was cooled to room temperature, and concentrated under reduced pressure. The obtained residue was diluted with ethyl acetate (1.0 L), and water (1.0 L) was added. The organic layer was separated, washed successively with 0.2N hydrochloric acid (500 mL) and saturated brine (500 mL), dried over anhydrous magnesium sulfate, and concentrated under reduced pressure. The obtained residue was purified by basic silica gel column chromatography [eluent: hexane-ethyl acetate (3:1)] to give the title compound (17.1 g) as yellow crystals (yield 89%). $^1$H NMR (400 MHz, $CDCl_3$) δ 1.32 (3H, t, J = 7.2 Hz), 2.35 (3H, s), 2.71 (3H, s), 4.22 (2H, s), 4.28 (2H, q, J = 7.2 Hz), 7.11 (2H, d, J = 7.6 Hz), 7.12 (1H, s), 7.24 (1H, t, J = 7.6 Hz).

Example 221-C)

4-methyl-2-(3-methylbenzyl)-1,3-thiazole-5-carboxylic acid

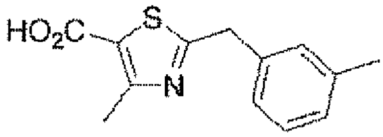

To a solution (103 mL) of ethyl 4-methyl-2-(3-methylbenzyl)-1,3-thiazole-5-carboxylate (17.1 g, 62.1 mmol) obtained in Example 221-B) in ethanol was added 8N aqueous sodium hydroxide solution (15.5 mL, 124 mmol) at room temperature, and the mixture was stirred at the same temperature for 3 hr. The reaction mixture was ice-cooled, and adjusted to neutral with 6N hydrochloric acid. The precipitate was collected by filtration, and washed with water (100 mL) to give the title compound (14.5 g) as a white solid (yield 94%).
MS (ESI+): $[M+H]^+$ 248.
$^1$H NMR (400 MHz, $CDCl_3$) 5 2.29 (3H, s), 2.57 (3H, s), 4.24 (2H, s), 7.09-7.15 (3H, m), 7.24 (1H, t, J = 7.6 Hz), 13.2 (1H, brs).

Example 221-D)

tert-butyl [4-methyl-2-(3-methylbenzyl)-1,3-thiazol-5-yl]carbamate

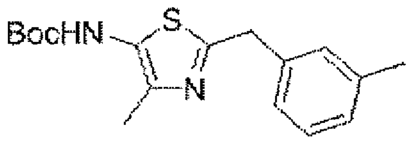

To a solution (179 mL) of 4-methyl-2-(3-methylbenzyl)-1,3-thiazole-5-carboxylic acid (13.3 g, 53.8 mmol) obtained in Example 221-C) in tert-butyl alcohol was added triethylamine (22.5 mL, 161 mmol) at room temperature, and the mixture was heated to 95°C and DPPA (17.4 mL, 81.0 mmol) was added. The mixture was stirred at the same temperature for 3 hr. The reaction product was concentrated under reduced pressure, and the residue was dissolved in ethyl acetate (500 mL), and water (500 mL) was added. The organic layer was separated, washed with saturated brine (500 mL), dried over anhydrous magnesium sulfate and concentrated under reduced pressure. The residue was purified by silica gel column chromatography [eluent: hexane-ethyl acetate (3:1)] to give the title compound (10.3 g) as yellow crystals (yield 60%).
MS (ESI+): $[M+H]^+$319.
$^1$H NMR (400MHz, $CDCl_3$) $\delta$ 1.48 (9H, s), 2.28 (3H, s), 2.32 (3H, s), 4.14 (2H, s), 6.33 (1H, brs), 7.04-7.11 (3H, m), 7.20 (1H, t, J = 7.2 Hz).

Example 221-E)

1-methyl-N-[4-methyl-2-(3-methylbenzyl)-1,3-thiazol-5-yl]-1H-pyrazole-5-carboxamide

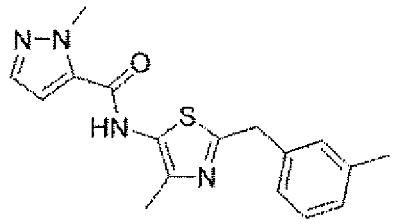

To a solution of tert-butyl [4-methyl-2-(3-methylbenzyl)-1,3-thiazol-5-yl]carbamate (300 g, 0.942 mmol) obtained in Example 221-D) in ethanol (2 mL) was added dropwise concentrated hydrochloric acid (0.670 mL) at room temperature, and the mixture was stirred at 50°C for 1.5 hr. The reaction mixture was cooled to 0°C, neutralized with 8M aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (9 mL), and 1-methyl-1H-pyrazole-5-carboxylic acid (143 mg, 1.13 mmol), HATU (430 mg, 1.13 mmol) and DIEA (0.0778 mL, 0.471 mmol) were added at room temperature. The reaction mixture was stirred at 60°C for 5 hr, water and 0.1N hydrochloric acid were added and the mixture was extracted with ethyl acetate. The organic layer was washed with saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (3:2-3:7)] and basic silica gel column chromatography [eluent: hexane-ethyl acetate (3:2-3:7)], and crystallized from ethyl acetate-hexane to give the title compound (116 mg) as colorless crystals (yield 38%).
MS (ESI+): $[M+H]^+$ 327.
$^1H$ NMR (300 MHz, DMSO-$d_6$) δ 2.29 (6H, s), 4.04 (3H, s), 4.16 (2H, s), 7.03-7.16 (4H, m), 7.18-7.27 (1H, m), 7.53 (1H, d, J = 1.9 Hz), 10.47 (1H, s).

Example 222

N-[2-(3-chloro-5-fluorobenzyl)-4-methyl-1,3-thiazol-5-yl]-1,2-thiazinane-6-carboxamide 1,1-dioxide

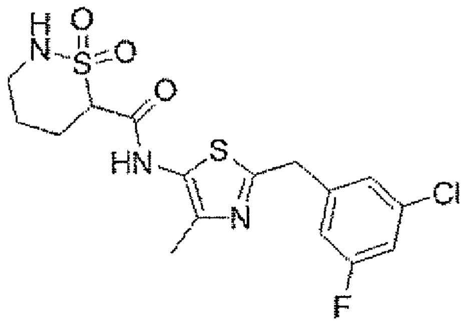

To a solution of N-[2-(3-chloro-5-fluorobenzyl)-4-methyl-1,3-thiazol-5-yl]-2-(4-methoxyphenyl)-1,2-thiazinane-6-carboxamide 1,1-dioxide (55 mg, 0.105 mmol) obtained in Example 218 in acetonitrile (2 mL) was added a solution of ceric ammonium nitrate (184 mg, 0.336 mmol) in water (1.2 mL) at room temperature, and the mixture was stirred at the same temperature for 1 hr. Water was added to the reaction solution, and the mixture was extracted with ethyl acetate. The organic layer was separated, washed with 0.1N hydrochloric acid and saturated brine, dried over anhydrous magnesium sulfate and concentrated under reduced pressure. The obtained residue was purified by silica gel column chromatography [eluent: hexane-ethyl acetate (9:1-3:7)] and reversed-phase silica gel chromatography to give the title compound (17.2 mg) as a colorless oil (yield 39%).
MS (ESI+): $[M+H]^+$ 418.
$^1H$ NMR (300 MHz, DMSO-$d_6$) 5 1.36-1.57 (1H, m), 1.70-1.86 (1H, m), 2.11-2.28 (2H, m), 2.30 (3H, s), 3.20 (2H, m), 4.14-4.21 (1H, m), 4.23 (2H, s), 7.06-7.15 (1H, m), 7.15-7.23 (1H, m), 7.27 (1H, brs), 7.30-7.37 (1H, m), 10.65 (1H, s).

Example 223

1-methyl-N-[4-methyl-2-(naphthalen-2-ylmethyl)-1,3-thiazol-5-yl]-1H-pyrazole-5-carboxamide

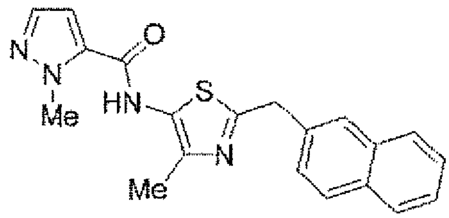

Example 223-A)

2-(naphthalen-2-yl)ethanethioamide

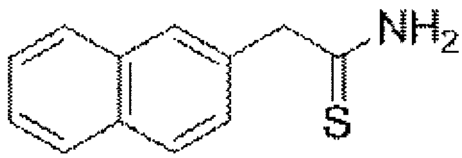

In the same manner as in Example 145-B), 2-naphthylacetonitrile was used to give the title compound (10.0 g) (yield 69%).
$^1$H NMR (400 MHz, $CDCl_3$) δ 4.28 (2H, s), 6.69 (1H, brs), 7.39 (1H, d, J = 8.4 Hz), 7.49-7.55 (2H, m), 7.59 (1H, brs), 7.73 (1H, s), 7.82-7.88 (3H, m).

Example 223-B)

ethyl 4-methyl-2-(naphthalen-2-ylmethyl)-1,3-thiazole-5-carboxylate

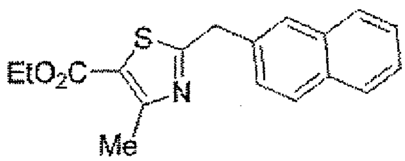

In the same manner as in Example 145-C), 2-(naphthalen-2-yl)ethanethioamide obtained in Example 223-A) was used to give the title compound (12.8 g) (yield 83%).
$^1$H NMR (400 MHz, $CDCl_3$) δ 1.29 (3H, t, J = 7.2 Hz), 2.73 (3H, s), 4.26 (2H, q, J = 7.2 Hz), 4.43 (2H, s), 7.42 (1H, d, J = 8.4 Hz), 7.46-7.52 (2H, m), 7.78 (1H, s), 7.81-7.85 (3H, m).

Example 223-C)

4-methyl-2-(naphthalen-2-ylmethyl)-1,3-thiazole-5-carboxylic acid

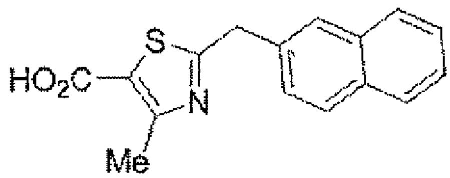

In the same manner as in Example 145-D), ethyl 4-methyl-2-(naphthalen-2-ylmethyl)-1,3-thiazole-5-carboxylate obtained in Example 223-B) was used to give the title compound (9.90 g) (yield 85%).
MS (ESI+): $[M+H]^+$284.
$^1$H NMR (400 MHz, DMSO-$d_6$) $\delta$ 2.59 (3H, s), 4.47 (2H, s), 7.48-7.54 (3H, m), 7.89-7.92 (4H, m), 13.2 (1H, brs).

Example 223-D)

tert-butyl [4-methyl-2-(naphthalen-2-ylmethyl)-1,3-thiazol-5-yl]carbamate

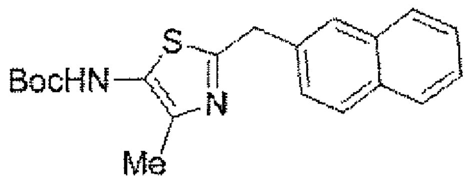

In the same manner as in Example 145-E), 4-methyl-2-(naphthalen-2-ylmethyl)-1,3-thiazole-5-carboxylic acid obtained in Example 223-C) was used to give the title compound (7.24 g) (yield 65%).
MS (ESI+): $[M+H]^+$355.
$^1$H NMR (400 MHz, $CDCl_3$) $\delta$ 1.47 (9H, s), 2.30 (3H, s), 4.35 (2H, s), 6.36 (1H, brs), 7.40-7.49 (3H, m), 7.75 (1H, s), 7.78-7.81 (3H, m).

Example 223-E)

1-methyl-N-[4-methyl-2-(naphthalen-2-ylmethyl)-1,3-thiazol-5-yl]-1H-pyrazole-5-carboxamide

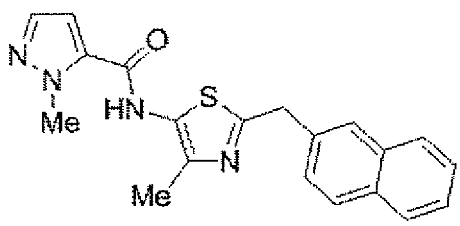

In the same manner as in Example 145-F), tert-butyl [4-methyl-2-(naphthalen-2-ylmethyl)-1,3-thiazol-5-yl]carbamate obtained in Example 223-D) and 1-methyl-1H-pyrazole-5-carboxylic acid were used to give the title compound (180 mg) (yield 44%).
MS (ESI+): $[M+H]^+$363.
$^1$H NMR (300 MHz, DMSO-$d_6$) $\delta$ 2.31 (3H, s), 4.02 (3H, s), 4.38 (2H, s), 7.05 (1H, d, J = 1.9 Hz), 7.44-7.57 (4H, m), 7.83-7.94 (4H, m), 10.49 (1H, s).

Example 224

N-[2-(3-bromobenzyl)-4-methyl-1,3-thiazol-5-yl]-1-methyl-1H-pyrazole-5-carboxamide

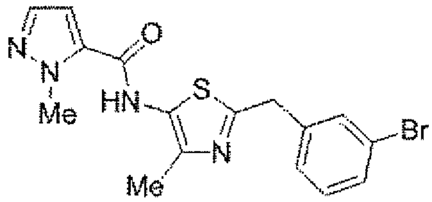

Example 224-A)

2-(3-bromophenyl)ethanethioamide

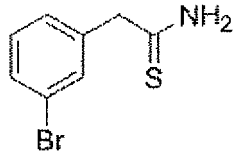

In the same manner as in Example 145-B), 3-bromophenylacetonitrile was used to give the title compound (26.85 g) (yield 92%).
$^1$H NMR (300 MHz, $CDCl_3$) δ 4.06 (2H, s), 7.10-7.22 (1H, m), 7.25 (2H, d, J = 6.8 Hz), 7.36-7.43 (1H, m), 7.43-7.52 (2H, m).

Example 224-B)

ethyl 2-(3-bromobenzyl)-4-methyl-1,3-thiazole-5-carboxylate

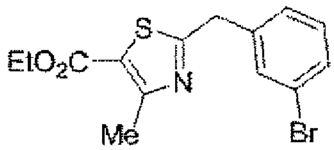

In the same manner as in Example 145-C), 2-(3-bromophenyl)ethanethioamide obtained in Example 224-A) was used to give the title compound (26.62 g) (yield 67%). MS (ESI+): $[M+H]^+$340.
$^1$H NMR (300 MHz, $CDCl_3$) δ 1.33 (3H, t, J = 7.2 Hz), 2.71 (3H, s), 4.23 (2H, s), 4.29 (2H, q, J = 7.2 Hz), 7.08-7.30 (2H, m), 7.39-7.57 (2H, m).

Example 224-C)

2-(3-bromobenzyl)-4-methyl-1,3-thiazole-5-carboxylic acid

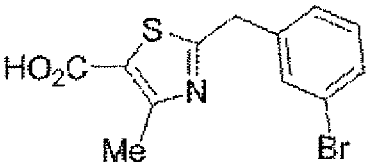

In the same manner as in Example 145-D), ethyl 2-(3-bromobenzyl)-4-methyl-1,3-thiazole-5-carboxylate obtained in Example 224-B) was used to give the title compound (22.17 g) (yield 91%).
MS (ESI+): $[M+H]^+$312.

$^1H$ NMR (300 MHz, DMSO-$d_6$) δ 2.58 (3H, s), 4.32 (2H, s), 7.24-7.42 (2H, m), 7.43-7.54 (1H, m), 7.59 (1H,s), 13.23 (1H, brs).

Example 224-D)

tert-butyl [2-(3-bromobenzyl)-4-methyl-1,3-thiazol-5-yl]carbamate

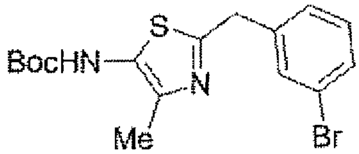

In the same manner as in Example 145-E), 2-(3-bromobenzyl)-4-methyl-1,3-thiazole-5-carboxylic acid obtained in Example 224-C) was used to give the title compound (2.66 g) (yield 69%).
MS (ESI+): $[M+H]^+$383.
$^1H$ NMR (300 MHz, $CDCl_3$) δ 1.49 (9H, s), 2.29 (3H, s), 4.15 (2H, s), 6.38 (1H, brs), 7.09-7.25 (2H, m), 7.38 (1H, d, J = 7.5Hz), 7.44 (1H, s).

Example 224-E)

N-[2-(3-bromobenzyl)-4-methyl-1,3-thiazol-5-yl]-1-methyl-1H-pyrazole-5-carboxamide

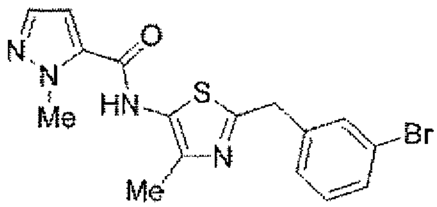

In the same manner as in Example 145-F), tert-butyl [2-(3-bromobenzyl)-4-methyl-1,3-thiazol-5-yl]carbamate obtained in Example 224-D) and 1-methyl-1H-pyrazole-5-carboxylic acid were used to give the title compound (1.47 g) (yield 54%). MS (ESI+): $[M+H]^+$391.
$^1H$ NMR (300 MHz, DMSO-$d_6$) 5 2.30 (3H, s), 4.05 (3H, s), 4.23 (2H, s), 7.06 (1H, d, J = 2.3 Hz), 7.24-7.40 (2H, m), 7.42-7.52 (1H, m), 7.52-7.60 (2H, m), 10.52 (1H, s).

Example 225

N-[2-(3-hydroxybenzyl)-4-methyl-1,3-thiazol-5-yl]-1-methyl-1H-pyrazole-5-carboxamide

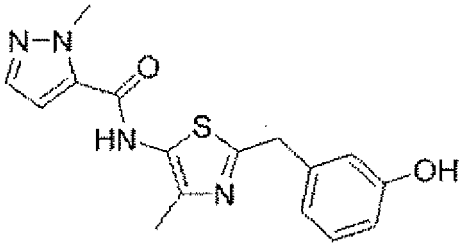

To a solution of N-[2-(3-methoxybenzyl)-4-methyl-1,3-thiazol-5-yl]-1-methyl-1H-pyrazole-5-carboxamide (150 mg, 0.438 mmol) obtained in Example 219 in toluene (5 mL) was added 1M boron tribromide-hexane solution (1.31 mL,

1.31 mmol) at room temperature, and the mixture was stirred at the same temperature for 4 hr. To the reaction mixture was further added 1M boron tribromide-hexane solution (1.31 mL, 1.31 mmol) at room temperature, and the mixture was stirred at 50°C for 2 hr. Water was added to the reaction mixture, and the mixture was extracted with ethyl acetate. The organic layer was dried over anhydrous magnesium sulfate, and concentrated under reduced pressure. The obtained residue was purified by basic silica gel column chromatography [eluent: ethyl acetate-methanol (1:0-19:1)], and crystallized from ethyl acetate-hexane to give the title compound (32.6 mg) as colorless crystals (yield 23%).
MS (ESI+): $[M+H]^+$ 329.
$^1H$ NMR (300 MHz, DMSO-$d_6$) $\delta$ 2.29 (3H, s), 4.04 (3H, s), 4.10 (2H, s), 6.59-6.78 (3H, m), 7.05 (1H, d, J = 2.1 Hz), 7.12 (1H, t, J = 7.6 Hz), 7.53 (1H, d, J = 2.1 Hz), 9.40 (1H, s), 10.46 (1H, brs).

Example 226

N-[2-(3-chloro-5-fluorobenzyl)-4-methyl-1,3-thiazol-5-yl]-2-(4-methoxyphenyl)isothiazolidine-5-carboxamide 1,1-dioxide

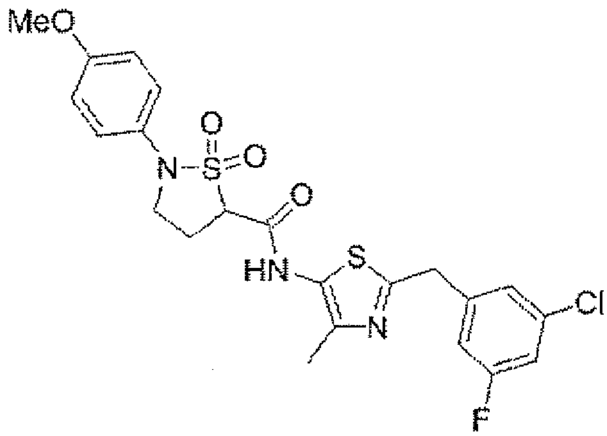

Example 226-A)

ethyl 2-(4-methoxyphenyl)isothiazolidine-5-carboxylate 1,1-dioxide

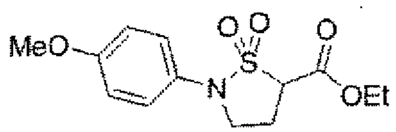

To a solution of pyridine (0.476 ml, 5.89 mmol) and p-anisidine (726 mg, 5.89 mmol) in acetonitrile (20 mL) was added a solution of ethyl (chlorosulfonyl)acetate (1.0 g, 5.36 mmol) in acetonitrile (30 mL) at 0°C. The reaction solution was stirred at the same temperature for 5 min, and at room temperature for 15 min. The reaction mixture was adjusted to pH≤2 by adding water and concentrated hydrochloric acid at 0°C, and the mixture was extracted with ethyl acetate. The organic layer was separated, washed with saturated brine, dried over anhydrous magnesium sulfate and concentrated under reduced pressure. To a mixture of the obtained residue, potassium carbonate (1.49 g, 10.8 mmol) and DMF (40 mL) was added dropwise a solution of 1,2-dibromoethane (0.372 ml, 4.30 mmol) in DMF (40 mL) over 1.5 hr at 60°C and the mixture was stirred at the same temperature for 2.5 hr. The reaction mixture was cooled to room temperature, adjusted to pH≤2 with concentrated hydrochloric acid, and extracted with ethyl acetate. The organic layer was separated, washed with 0.1N hydrochloric acid and saturated brine, dried over anhydrous magnesium sulfate and concentrated under reduced pressure. The obtained residue was purified by silica gel column chromatography [eluent: hexane-ethyl acetate (17:3-3:2)] to give the title compound (438 mg) as a brown oil (yield 27%).
$^1H$ NMR (300 MHz, $CDCl_3$) $\delta$ 1.35 (3H, t, J = 7.0 Hz), 2.52-2.68 (1H, m), 2.77-2.92 (1H, m), 3.61-3.73 (1H, m), 3.73-3.87 (4H, m), 4.18-4.27 (1H, m), 4.27-4.44 (2H, m), 6.84-6.96 (2H, m), 7.20-7.34 (2H, m).

Example 226-B)

2-(4-methoxyphenyl)isothiazolidine-5-carboxylic acid 1,1-dioxide

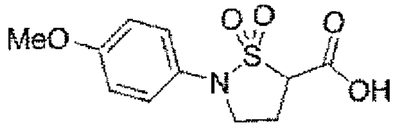

To a solution of ethyl 2-(4-methoxyphenyl)isothiazolidine-5-carboxylate 1,1-dioxide (438 mg, 1.46 mmol) obtained in Example 226-A) in methanol (10 mL)-water (10 mL) was added a solution of potassium hydroxide (82 mg, 1.46 mmol) in water (1 mL) at room temperature, and the mixture was stirred at the same temperature for 30 min. The reaction mixture was acidified with 0.1N hydrochloric acid at room temperature, and extracted with THF. The organic layer was separated, washed with water and saturated brine, dried over anhydrous magnesium sulfate and concentrated under reduced pressure to give the title compound (365 mg) as white crystals (yield 92%).
$^1H$ NMR (300 MHz, DMSO-$d_6$) δ 2.53-2.67 (2H, m), 3.59-3.72 (2H, m), 3.75 (3H, s), 4.54 (1H, t, J = 8.5 Hz), 6.90-7.04 (2H, m), 7.11-7.30 (2H, m), 13.67 (1H, brs).

Example 226-C)

N-[2-(3-chloro-5-fluorobenzyl)-4-methyl-1,3-thiazol-5-yl]-2-(4-methoxyphenyl)isothiazolidine-5-carboxamide 1,1-dioxide

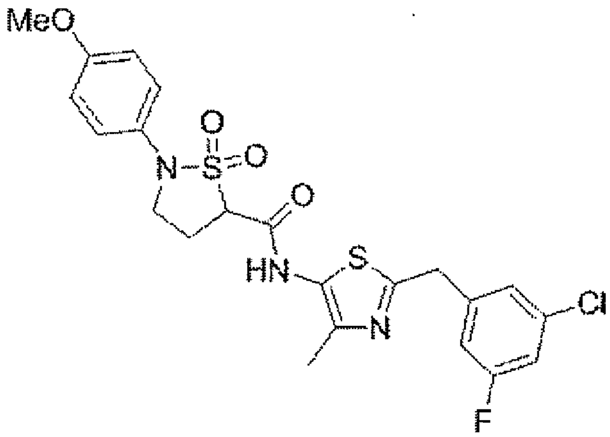

To a solution of tert-butyl [2-(3-chloro-5-fluorobenzyl)-4-methyl-1,3-thiazol-5-yl]carbamate (400 mg, 1.12 mmol) obtained in Example 145-E) in ethanol (2 mL) was added dropwise concentrated hydrochloric acid (1.0 mL) at room temperature, and the mixture was stirred at 50°C for 2 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (10 mL), and 2-(4-methoxyphenyl) isothiazolidine-5-carboxylic acid 1,1-dioxide (365 mg, 1.35 mmol) obtained in Example 226-B), HATU (512 mg, 1.35 mmol) and DIEA (0.098 mL, 0.56 mmol) were added at room temperature. The reaction mixture was stirred at 60°C for 1 hr, cooled to room temperature, saturated aqueous sodium hydrogen carbonate solution was added and the mixture was extracted with ethyl acetate. The organic layer was washed with 0.1N hydrochloric acid and saturated brine, dried over anhydrous magnesium sulfate, and concentrated under reduced pressure. The obtained residue was purified by silica gel column chromatography [eluent: hexane-ethyl acetate (3:2-7:13)] to give the title compound (325 mg) as colorless crystals (yield 57%).
MS (ESI+): $[M+H]^+$ 510.
$^1H$ NMR (300 MHz, DMSO-$d_6$) δ 2.33 (3H, s), 2.53-2.65 (1H, m), 2.65-2.82 (1H, m), 3.61-3.71 (1H, m), 3.75 (3H, s),

3.77-3.88 (1H, m), 4.24 (2H, s), 4.57-4.67 (1H, m), 6.92-7.01 (2H, m), 7.16-7.25 (3H, m), 7.28 (1H, brs), 7.29-7.37 (1H, m), 11.05 (1H, s).

Example 227

N-[2-(3-cyclopropylbenzyl)-4-methyl-1,3-thiazol-5-yl]-1-methyl-1H-pyrazole-5-carboxamide

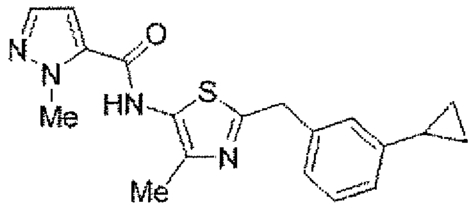

A mixture of N-[2-(3-bromobenzyl)-4-methyl-1,3-thiazol-5-yl]-1-methyl-1H-pyrazole-5-carboxamide (150 mg, 0.38 mmol) obtained in Example 224, cyclopropylboronic acid monohydrate (51.8 mg, 0.50 mmol), 1,1'-bis(diphenylphosphino)ferrocene-palladium (II) dichloride-dichloromethane complex (31.3 mg, 0.04 mmol), cesium carbonate (437 mg, 1.34 mmol), toluene (5 mL) and water (1 mL) was stirred under an argon gas atmosphere at 100°C overnight. After cooling to room temperature, the mixture was diluted with ethyl acetate, washed successively with saturated aqueous sodium hydrogen carbonate solution and saturated brine, and dried over anhydrous magnesium sulfate. The obtained residue was purified by silica gel column chromatography [eluent: hexane-ethyl acetate (9:1-0:1)], and crystallized from ethyl acetate/hexane to give the title compound (48 mg) as a white solid (yield 36%).
MS (ESI+): $[M+H]^+$353.
$^1$H NMR (300 MHz, DMSO-$d_6$) 5 0.60-0.69 (2H, m), 0.89-0.99 (2H, m), 1.82-1.97 (1H, m), 2.29 (3H, s), 4.04 (3H, s), 4.15 (2H, s), 6.94 (1H, d, J 7.9 Hz), 7.02-7.12 (3H, m), 7.16-7.26 (1H, m), 7.53 (1H, d, J = 2.3 Hz), 10.47 (1H, s).

Example 228

N-[2-(3-chloro-5-fluorobenzyl)-4-methyl-1,3-thiazol-5-yl]-3-(methylsulfonyl)benzamide

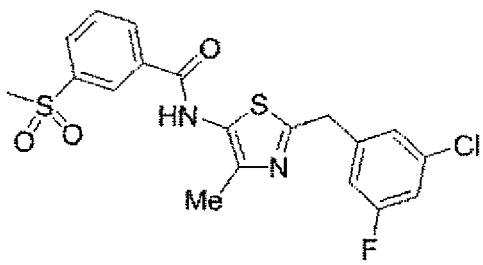

In the same manner as in Example 145-F), tert-butyl [2-(3-chloro-5-fluorobenzyl)-4-methyl-1,3-thiazol-5-yl] carbamate obtained in Example 145-E) and 3-(methylsulfonyl)benzoic acid were used to give the title compound (129 mg) (yield 42%).
MS (ESI+): $[M+H]^+$439.
$^1$H NMR (300 MHz, DMSO-$d_6$) $\delta$ 2.36 (3H, s), 3.29 (3H, s), 4.27 (2H, s), 7.23 (1H, dd, J = 9.4, 1.5 Hz), 7.28-7.39 (2H, m), 7.75-7.87 (1H, m), 8.15 (1H, d, J = 8.3 Hz), 8.26 (1H, d, J= 7.6 Hz), 8.43 (1H, s), 10.89 (1H, s).

Example 229

N-[2-(3-chloro-5-fluorobenzyl)-4-methyl-1,3-thiazol-5-yl]-2-(methylsulfanyl)benzamide

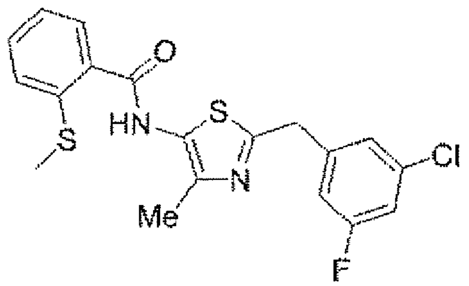

In the same manner as in Example 145-F), tert-butyl [2-(3-chloro-5-fluorobenzyl)-4-methyl-1,3-thiazol-5-yl] carbamate obtained in Example 145-E) and 2-(methylthio)benzoic acid were used to give the title compound (254 mg) (yield 34%). MS (ESI+): $[M+H]^+$407.
$^1$H NMR (300 MHz, $CDCl_3$) δ 2.47 (3H, s), 2.53 (3H, s), 4.20 (2H, s), 6.91-7.04 (2H, m), 7.12 (1H, s), 7.33-7.45 (1H, m), 7.45-7.55 (2H, m), 8.00 (1H, d, J = 7.2 Hz), 9.80 (1H, brs).

Example 230

N-[2-(3-chloro-5-fluorobenzyl)-4-methyl-1,3-thiazol-5-yl]-3-(cyanomethyl)-1-methyl-1H-pyrazole-5-carboxamide

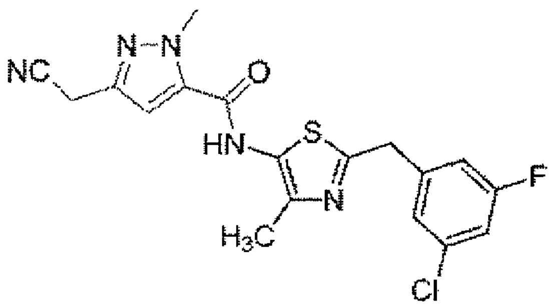

Example 230-A)

ethyl 3-(cyanamethyl)-1-methyl-1H-pyrazole-5-carboxylate

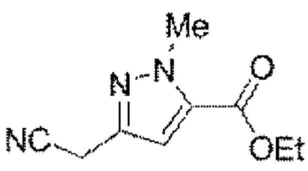

To a solution of ethyl 3-(bromomethyl)-1-methyl-1H-pyrazole-5-carboxylate (1.0 g, 4.05 mmol) in DMSO (10 mL) and water (3 mL) was added potassium cyanide (277 mg, 4.25 mmol) in an ice bath, and the mixture was stirred at room temperature for 2 days. Water was added to the reaction mixture and the mixture was extracted with ethyl acetate. The organic layer was washed twice with water, dried over anhydrous sodium sulfate and concentrated under reduced pressure to give the title compound (370 mg) as a white solid (yield 47%).
MS (ESI+): $[M+H]^+$194.
$^1$H NMR (300 MHz, $CDCl_3$) δ 1.38 (3H, t, J = 7.2 Hz), 3.74 (2H, s), 4.15 (3H, s), 4.35 (2H, q, J = 7.2 Hz), 6.86 (1H, s).

Example 230-B)

3-(cyanomethyl)-1-methyl-1H-pyrazole-5-carboxylic acid

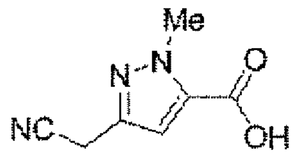

To a solution of ethyl 3-(cyanomethyl)-1-methyl-1H-pyrazole-5-carboxylate (370 mg, 1.92 mmol) obtained in Example 230-A) in THF (4 mL)-methanol (1 mL) was added 1N aqueous sodium hydroxide solution (3 mL), and the mixture was stirred at room temperature for 1 hr. The mixture was concentrated under reduced pressure, and 1N hydrochloric acid (3 mL) was added in an ice bath. The mixture was extracted with ethyl acetate, the extract was washed twice with water, dried over anhydrous sodium sulfate and concentrated under reduced pressure to give the title compound (250 mg) as a white solid (yield 79%).
MS (ESI+): $[M+H]^+$166.
$^1$H NMR (300 MHz, $CDCl_3$) 5 3.77 (2H, s), 4.17 (3H, s), 6.96 (1H, s) .
* The peak of "COOH" was not observed.

Example 230-C)

N-[2-(3-chloro-5-fluorobenzyl)-4-methyl-1,3-thiazol-5-yl]-3-(cyanomethyl)-1-methyl-1H-pyrazole-5-carboxamide

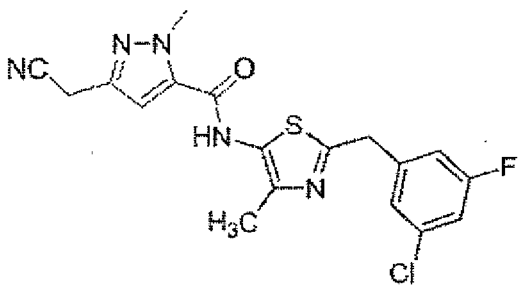

To a solution of tert-butyl [2-(3-chloro-5-fluorobenzyl)-4-methyl-1,3-thiazol-5-yl]carbamate (440 mg, 1.23 mmol) obtained in Example 145-E) in methanol (5 mL) was added dropwise 6N hydrochloric acid (2 mL), and the mixture was stirred at 50°C for 1 hr. The reaction mixture was cooled to 0°C, neutralized with 8M aqueous sodium hydroxide solution (1.5 mL), adjusted to pH 9-10 with saturated aqueous sodium hydrogen carbonate solution, and extracted with ethyl acetate. The organic layer was washed with saturated brine, dried over anhydrous sodium sulfate, and concentrated under reduced pressure. The obtained residue was dissolved in DMA (7 mL), and 3-(cyanomethyl)-1-methyl-1H-pyrazole-5-carboxylic acid (250 mg, 1.5 mmol) obtained in Example 230-B), HATU (469 mg, 1.23 mmol) and DIEA (0.107 mL, 0.62 mmol) were added at 0°C . The reaction mixture was stirred at 60°C for 2 hr, water was added and the mixture was extracted with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-3:7)] to give the title compound (260 mg) as a yellow oil (yield 52%).
MS (ESI+) : $[M+H]^+$404.
$^1$H NMR (300 MHz, $CDCl_3$) $\delta$ 2.42 (3H, s), 3.79 (2H, s), 4.16 (3H, s), 4.20 (2H, s), 6.74 (1H, s), 6.86-7.06 (2H, m), 7.10 (1H, s), 7.86 (1H, s).

Example 231

N-[2-(3-chloro-5-fluorobenzyl)-4-methyl-1,3-thiazol-5-yl]-2-(methylsulfonyl)benzamide

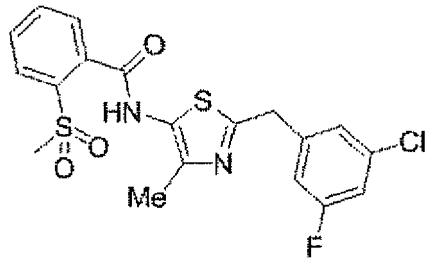

In the same manner as in Example 208, N-[2-(3-chloro-5-flunrobenzyl)-4-methyl-1,3-thiazol-5-yl]-2-(methylsulfanyl)benzamide obtained in Example 229 was used to give the title compound (66 mg) (yield 31%).
MS (ESI+): $[M+H]^+$439.
$^1$H NMR (300 MHz, DMSO-$d_6$) δ 2.32 (3H, s), 3.34 (3H, s), 4.26 (2H, s), 7.23 (1H, d, J = 9.8 Hz), 7.28-7.40 (2H, m), 7.71 (1H, dd, J = 7.2, 1.5 Hz), 7.74-7.89 (2H, m), 7.97-8.06 (1H, m), 11.12 (1H, s).

Example 232

N-[2-(3-chloro-5-fluorobenzyl)-4-methyl-1,3-thiazol-5-yl]-2-(methylsulfinyl)benzamide

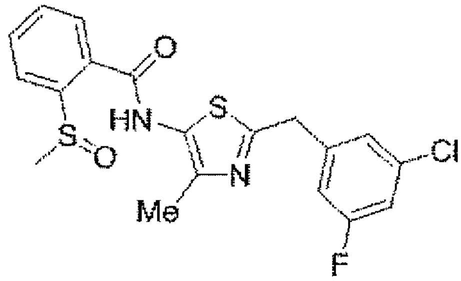

To a solution of N-[2-(3-chloro-5-fluorobenzyl)-4-methyl-1,3-thiazol-5-yl]-2-(methylsulfanyl)benzamide (197 mg, 0.48 mmol) obtained in Example 229 in toluene (2 mL)-acetonitrile (4 mL) was added 3-chloroperbenzoic acid (182 mg, 0.73 mmol) at room temperature, and the mixture was stirred for 1 hr. To the reaction mixture was added saturated aqueous sodium thiosulfate solution and the mixture was stirred for 15 min and extracted with ethyl acetate. The organic layer was washed successively with saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (9:1-0:1), ethyl acetate-methanol (1:0-4:1)] to give the title compound (78 mg) (yield 38%).
MS (ESI+): $[M+H]^+$423.
$^1$H NMR (300 MHz, DMSO-$d_6$) δ 2.35 (3H, s), 2.78 (3H, s), 4.27 (2H, s), 7.17-7.26 (1H, m, J = 11.0, 2.3 Hz), 7.31 (1H, s), 7.32-7.39 (1H, m), 7.65-7.76 (1H, m), 7.83-7.93 (1H, m), 7.97 (1H, d, J = 7.9 Hz), 8.13 (1H, d, J = 6.8 Hz), 11.00 (1H, s).

Example 233

1-methyl-N-{4-methyl-2-[3-(methylsulfonyl)benzyl]-1,3-thiazol-5-yl}-1H-pyrazole-5-carboxamide

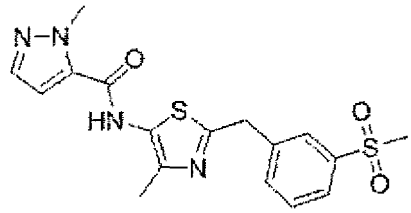

Example 233-A)

3-(methylsulfonyl)benzyl methanesulfonate

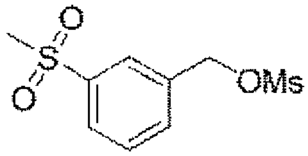

To a solution of 3-(methylsulfonyl)benzoic acid (5.0 g, 25.0 mmol) in THF (25 mL) was added dropwise 1.1M borane-THF solution (34.1 mL, 37.5 mmol) at 0°C, and the mixture was stirred at room temperature for 50 min. To the reaction mixture was further added 1.1M borane-THF solution (10.0 mL) at room temperature, and the mixture was stirred at the same temperature for 2 hr. To the reaction mixture were added water and 1N hydrochloric acid, and the mixture was extracted with ethyl acetate. The organic layer was separated, dried over anhydrous magnesium sulfate and concentrated under reduced pressure. To a solution of the obtained residue and triethylamine (25.1 mL, 180 mmol) in THF (350 mL) was added methanesulfonyl chloride (13.5 ml, 175 mmol) at room temperature, and the mixture was stirred at the same temperature for 30 min. To the reaction mixture was added 1N hydrochloric acid at room temperature, and the mixture was extracted with ethyl acetate. The organic layer was separated, washed with saturated brine, dried over anhydrous magnesium sulfate and concentrated under reduced pressure. The obtained residue was purified by silica gel column chromatography [eluent: hexane-ethyl acetate (1:9-0:1)] to give the title compound (4.91 g) as white crystals (yield 74%).
$^1$H NMR (300 MHz, DMSO-$d_6$) $\delta$ 3.25 (3H, s), 3.29 (3H, s), 5.39 (2H, s), 7.65-8.08 (4H, m) .

Example 233-B)

[3-(methylsulfonyl)phenyl]acetonitrile

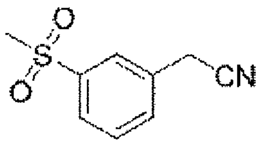

A mixture of 3-(methylsulfonyl)benzyl methanesulfonate (4.10 g, 15.5 mmol) obtained in Example 233-A) and tetrabutylammonium cyanide (5.0 g, 18.6 mmol) was stirred at 60°C for 50 min. The reaction mixture was cooled to room temperature, adjusted to pH 4 with 1N hydrochloric acid, and extracted with ethyl acetate and diethyl ether. The organic layer was separated, washed with saturated brine, dried over anhydrous magnesium sulfate and concentrated under reduced pressure. The obtained residue was purified by silica gel column chromatography [eluent: hexane-ethyl acetate (3:2-3:7)] to give the title compound (2.17 g) as white crystals (yield 72% .
$^1$H NMR (300 MHz, DMSO-$d_6$) $\delta$ 3.24 (3H, s), 4.21 (2H, s), 7.64-7.78 (2H, m), 7.84-8.00 (2H, m).

Example 233-C)

2-[3-(methylsulfonyl)phenyl]ethanethioamide

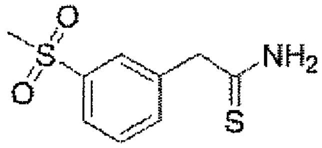

A mixture of [3-(methylsulfonyl)phenyl]acetonitrile (2.14 g, 11.0 mmol) obtained in Example 233-B), 0,0-diethyl dithiophosphate (1.74 mL, 11.0 mmol) and 4N hydrogen chloride-ethanol (27 mL) was stirred at room temperature overnight. Water was added to the reaction mixture at 0°C, and the mixture was extracted with ethyl acetate. The organic layer was separated, washed successively with 0.1N aqueous sodium hydroxide solution, water and saturated brine, dried over anhydrous magnesium sulfate and concentrated under reduced pressure. The obtained residue was treated with hexane in an ice bath to allow solidification. The solid was collected by filtration, and washed with hexane to give the title compound (2.21 g) as a white solid (yield 88%).
MS (ESI+): $[M+H]^+$ 230.
$^1H$ NMR (300 MHz, DMSO-$d_6$) $\delta$ 3.20 (3H, s), 3.93 (2H, s), 7.55-7.65 (1H, m), 7.66-7.73 (1H, m), 7.78-7.85 (1H, m), 7.90-7.93 (1H, m), 9.42-9.63 (2H, m).

Example 233-D)

ethyl 4-methyl-2-[3-(methylsulfonyl)benzyl]-1,3-thiazole-5-carboxylate

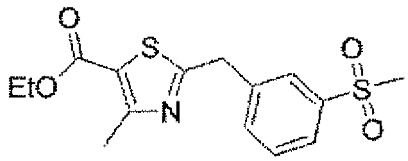

A solution of 2-[3-(methylsulfonyl)phenyl]ethanethioamide (2.0 g, 8.72 mmol) obtained in Example 233-C) and ethyl 2-chloro-3-oxobutanoate (1.45 mL, 10.5 mmol) in pyridine (5 mL)-ethanol (5 mL) was stirred at room temperature overnight. The reaction mixture was diluted with ethyl acetate, washed with water, 0.1N hydrochloric acid and saturated brine, dried over anhydrous magnesium sulfate, and concentrated under reduced pressure. The obtained residue was purified by basic silica gel column chromatography [eluent: hexane-ethyl acetate (4:1-1:1)] to give the title compound (2.39 g) as white crystals (yield 81%).
MS (ESI+) . $[M+H]^+$ 340.
$^1H$ NMR (300 MHz, DMSO-$d_6$) $\delta$ 1.25 (3H, t, J = 7.1 Hz), 2.60 (3H, s), 3.23 (3H, s), 4.24 (2H, q, J = 7.1 Hz), 4.48 (2H, s), 7.58-7.76 (2H, m), 7.86 (1H, dt, J = 7.7, 1.7 Hz), 7.90-7.97 (1H, m).

Example 233-E)

4-methyl-2-[3-(methylsulfonyl)benzyl]-1,3-thiazole-5-carboxylic acid

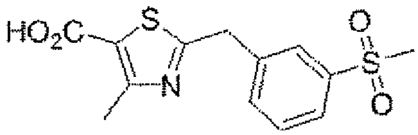

To a solution (15 mL) of ethyl 4-methyl-2-[3-(methylsulfonyl)benzyl]-1,3-thiazole-5-carboxylate (2.3 g, 6.78 mmol) obtained in Example 233-D) in ethanol was added dropwise 8N aqueous sodium hydroxide solution (4.0 mL) at room temperature, and the mixture was stirred at the same temperature for 1 hr. The reaction mixture was ice-cooled, and 6N hydrochloric acid (6 mL) was added. The precipitate was collected by filtration, and washed with water to give the title compound (1.98 g) as a white solid (yield 94%). MS (ESI+): $[M+H]^+$ 312.
$^1$H NMR (300 MHz, DMSO-$d_6$) $\delta$ 2.58 (3H, s), 3.22 (3H, s), 4.46 (2H, s), 7.60-7.75 (2H, m), 7.86 (1H, dt, J = 7.6, 1.5 Hz), 7.91-7.97 (1H, m), 13.24 (1H, s).

Example 233-F)

tert-butyl {4-methyl-2-[3-(methylsulfonyl)benzyl]-1,3-thiazol-5-yl}carbamate
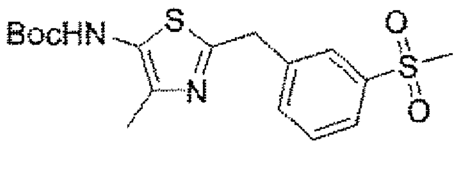

To a solution (18 mL) of 4-methyl-2-[3-(methylsulfonyl)benzyl]-1,3-thiazole-5-carboxylic acid (1.98 g, 6.36 mmol) obtained in Example 233-E) in tert-butyl alcohol was added triethylamine (2.66 mL, 19.1 mmol) at room temperature, and the mixture was heated to 90°C and DPPA (2.06 mL, 9.54 mmol) was added. The mixture was stirred at the same temperature for 30 min. The reaction product was concentrated under reduced pressure, and the residue was diluted with ethyl acetate, washed with 0.1N aqueous sodium hydroxide solution and saturated brine, dried over anhydrous magnesium sulfate and concentrated under reduced pressure. The residue was purified by silica gel column chromatography [eluent: hexane-ethyl acetate (1:1-1:4)] to give the title compound (1.26 g) as a pale-yellow oil (yield 52%).
MS (ESI+) : $[M+H]^+$383.
$^1$H NMR (300 MHz, DMSO-$d_6$) $\delta$ 1.44 (9H, s), 2.19 (3H, s), 3.21 (3H, s), 4.29 (2H, s), 7.57-7.70 (2H, m), 7.83 (1H, dt, J = 7.2, 1.7 Hz, 1 H), 7.88 (1H, m), 9.62 (1H, br s).

Example 233-G)

1-methy1-N-{4-methyl-2-[3-(methylsulfonyl)benzyl]-1,3-thiazol-5-yl}-1H-pyrazole-5-carboxamide

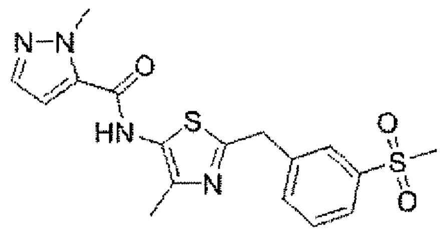

To a solution of tert-butyl {4-methyl-2-[3-(methylsulfonyl)benzyl]-1,3-thiazol-5-yl}carbamate (300 mg, 0.78 mmol) obtained in Example 233-F) in ethanol (2 mL) was added dropwise concentrated hydrochloric acid (0.600 mL) at room temperature, and the mixture was stirred at 50°C for 1 hr. The reaction mixture was cooled to 0°C, neutralized with 8N aqueous sodium hydroxide solution, adjusted to pH 10-11 with saturated aqueous sodium hydrogen carbonate solution, and extracted with ethyl acetate. The organic layer was washed with saturated brine, dried over anhydrous magnesium sulfate, and concentrated under reduced pressure. The obtained residue was dissolved in DMA (3 mL), and 1-methyl-1H-pyrazole-5-carboxylic acid (118 mg, 0.94 mmol), HATU (356 g, 0.94 mmol) and DIEA (0.068 mL, 0.39 mmol) were added at room temperature. The reaction mixture was stirred at 60°C for 2 hr, water was added and the mixture was extracted with ethyl acetate. The organic layer was washed with saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (1:4-0: 1)] and basic silica gel column chromatography [eluent: hexane-ethyl acetate (1:9-0:1)], and crystallized from ethyl acetate-hexane to give the title compound (159 mg) as white crystals (yield 52%).

MS (ESI+) : $[M+H]^+$ 391.
$^1H$ NMR (300 MHz, DMSO-$d_6$) δ 2.31 (3H, s), 3.31 (3H, s), 4.04 (3H, s), 4.37 (2H, s), 7.06 (1H, d, J = 1.9 Hz), 7.53 (1H, d, J = 1.9 Hz), 7.59-7.74 (2H, m), 7.80-7.87 (1H, m), 7.91 (1H, brs), 10.52 (1H, s).

Example 234

1-(carbamoylmethyl)-N-[2-(3-cloloro-5-fluorobenzyl)-4-methyl-1,3-thiazol-5-yl]-1H-pyrazole-5-carboxamide

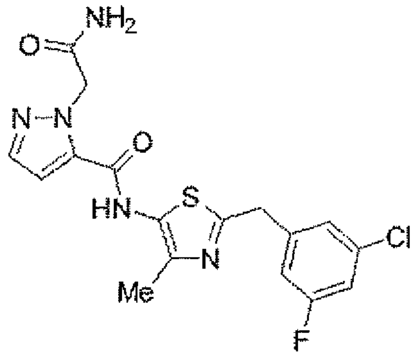

Example 234-A)

ethyl 1-(cyanomethyl)-1H-pyrazole-5-carboxylate

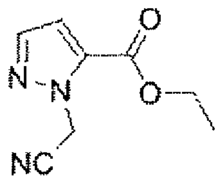

A mixture of ethyl 1H-pyrazole-5-carboxylate (1.0 g, 7.14 mmol), lithium iodide (0.478 g, 3.57 mmol), bromoacetonitrile (0.942 g, 7.85 mmol), potassium carbonate (1.085 g, 7.85 mmol) and N-methylpyrrolidone (6 mL) was stirred at 80°C for 1 day. The reaction mixture was cooled to room temperature, diluted with ethyl acetate and water, and the organic layer was separated. The organic layer was washed with water and saturated brine, and dried over anhydrous sodium sulfate. The obtained residue was purified by silica gel column chromatography [eluent: hexane-ethyl acetate (95:5-1:1)] to give the title compound (269 mg) as a colorless oil (yield 21%) .
MS (ESI+) : $[M+H]^+$180.
NMR (300 MHz, $CDCl_3$) δ 1.17 (3H, t, J = 7.2 Hz), 4.03 (2H, q, J = 7.2 Hz), 6.26 (2H, d, J = 9.4 Hz), 7.83 (2H, d, J = 9.4 Hz).

Example 234-B)

1-(carbamoylmethyl)-1H-pyrazole-5-carboxylic acid

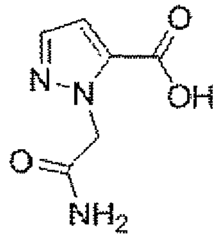

To a solution of ethyl 1-(cyanomethyl)-1H-pyrazole-5-carboxylate (269.4 mg, 1.50 mmol) obtained in Example 234-A) in ethanol (6 mL) was added 1M aqueous sodium hydroxide solution (3.01 ml, 3.01 mmol)). The reaction mixture was stirred at room temperature for 5 hr, and the solvent was evaporated under reduced pressure. The residue was acidified with 1M hydrochloric acid, extracted with ethyl acetate, the extract was washed with saturated brine, and dried over anhydrous magnesium sulfate. The obtained residue was crystallized from ethyl acetate/hexane to give the title compound (59 mg) as a white solid (yield 23%).
MS (ESI+): $[M+H]^+$170.
$^1$H NMR (300 MHz, DMSO-$d_6$) δ 5.09 (2H, s), 6.81 (1H, d, J = 1.9 Hz), 7.15 (1H, brs), 7.47 (1H, brs), 7.52 (1H, d, J = 1.9 Hz), 13.28 (1H, brs).

Example 234-C)

1-(carbamoylmethyl)-N-[2-(3-chloro-5-fluorobenzyl)-4-methyl-1,3-thiazol-5-yl]-1H-pyrazole-5-carboxamide

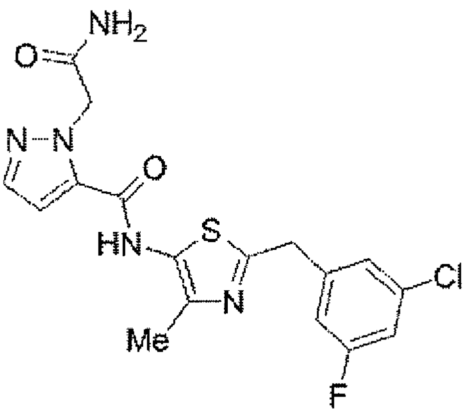

In the same manner as in Example 145-F), tert-butyl [2-(3-chloro-5-fluorobenzyl)-4-methyl-1,3-thiazol-5-yl] carbamate obtained in Example 145-E) and 1-(carbamoylmethyl)-1H-pyrazole-5-carboxylic acid obtained in Example 234-B) were used to give the title compound (15 mg) (yield 10%).
MS (ESI+): $[M+H]^+$408.
$^1$H NMR (300 MHz, $CDCl_3$) δ 2.39 (3H, s), 4.19 (2H, s), 5.23 (2H, s), 5.58 (1H, brs), 6.02 (1H, brs), 6.78 (1H, d, J = 1.9 Hz), 6.93 (1H, d, J = 9.8 Hz), 6.99 (1H, dt, J = 8.7, 2.1 Hz), 7.09 (1H, s), 7.60 (1H, d, J = 2.3 Hz), 8.69 (1H, s).

Example 235

N-[2-(3-chloro-5-fluorobenzyl)-4-methyl-1,3-thiazol-5-yl]-3-methoxy-1-methyl-1H-pyrazole-5-carboxamide

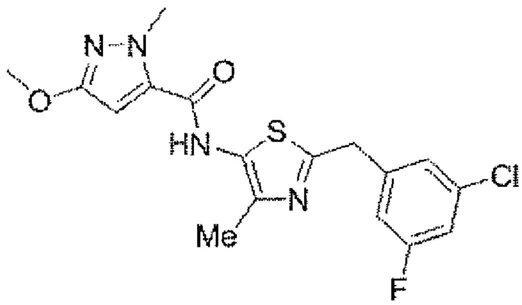

Example 235-A)

methyl 3-hydroxy-1-methyl-1H-pyrazole-5-carboxylate

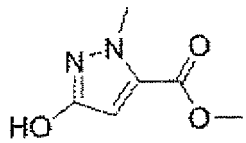

To a solution of dimethyl acetylenedicarboxylate (5 g, 35.18 mmol) in methanol (25 mL)-water (2.5mL) was added dropwise methylhydrazine (1.945 g, 42.22 mmol) at room temperature. The reaction mixture was stirred at 70°C overnight, cooled to room temperature, concentrated under reduced pressure, diluted with water, and extracted with ethyl acetate. The organic layer was washed with saturated brine, and dried over anhydrous sodium sulfate. The obtained residue was purified by silica gel column chromatography [eluent: hexane-ethyl acetate (90:10-3:4)] to give the title compound (1.26 g) as a colorless oil (yield 23%).
MS (ESI+): $[M+H]^+$157.
$^1$H NMR (300 MHz, DMSO-$d_6$) δ 3.79 (3H, s), 3.88 (3H, s), 6.00 (1H, s), 10.06 (1H, s).

Example 235-B)

methyl 3-methoxy-1-methyl-1H-pyrazole-5-carboxylate

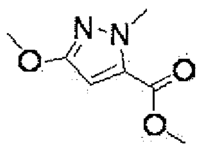

A mixture of methyl 3-hydroxy-1-methyl-1H-pyrazole-5-carboxylate (494 mg, 3.16 mmol) obtained in Example 235-A), iodomethane (1.27 g, 8.95 mmol), potassium carbonate (0.875 g, 6.33 mmol) and DMF (3 mL) was stirred at room temperature overnight. The reaction mixture was diluted with ethyl acetate and water, and the organic layer was separated. The organic layer was washed successively with water and saturated brine, and dried over anhydrous magnesium sulfate. The obtained residue was purified by silica gel column chromatography [eluent: hexane-ethyl acetate (95:5-1:1)] to give the title compound (221 mg) as a white solid (yield 41%).
MS (ESI+) : $[M+H]^+$171.
$^1$H NMR (300 MHz, $CDCl_3$) δ 3.87 (6H, d, J = 3.4 Hz), 4.04 (3H, s), 6.18 (1H, s).

Example 235-C)

3-methoxy-1-methyl-1H-pyrazole-5-carboxylic acid

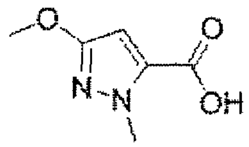

To a solution of methyl 3-methoxy-1-methyl-1H-pyrazole-5-carboxylate (220.7 mg, 1.30 mmol) obtained in Example 235-B) in methanol (5 mL) was added 1M aqueous sodium hydroxide solution, and the mixture was stirred at room temperature for 5 hr. The reaction mixture was concentrated under reduced pressure, acidified with 1M hydrochloric acid, and extracted with ethyl acetate. The organic layer was washed with saturated brine, dried over anhydrous magnesium sulfate to give the title compound (163 mg) as a white solid (yield 80%).
MS (ESI-): $[M-H]^-$155.
$^1$H NMR (300 MHz, DMSO-$d_6$) δ 3.77 (3H, s), 3.92 (3H, s), 6.20 (1H, s), 13.38 (1H, brs).

Example 235-D)

N-[2-(3-chloro-5-fluorobenzyl)-4-methyl-1,3-thiazol-5-yl]-3-methoxy-1-methyl-1H-pyrazole-5-carboxamide

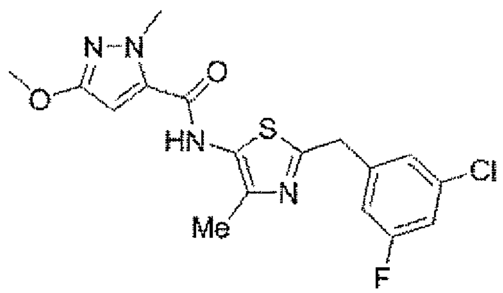

In the same manner as in Example 145-F), tert-butyl [2-(3-chloro-5-fluorobenzyl)-4-methyl-1,3-thiazol-5-yl] carbamate obtained in Example 145-E) and 3-methoxy-1-methyl-1H-pyrazole-5-carboxylic acid obtained in Example 235-C) were used to give the title compound (80 mg) (yield 23%).
MS (ESI+): $[M+H]^+$395.
$^1$H NMR (300 MHz, DMSO-$d_6$) δ 2.30 (3H, s), 3.80 (3H, s), 3.90 (3H, s), 4.26 (2H, s), 6.49 (1H, s), 7.17-7.26 (1H, m), 7.29 (1H, s), 7.31-7.38 (1H, m), 10.48 (1H, s).

Example 236

N-[2-(3-chloro-5-fluorobenzyl)-4-methyl-1,3-thiazol-5-yl]-1-methyl-3-[(2-oxopyrrolidin-1-yl)methyl]-1H-pyrazole-5-carboxamide

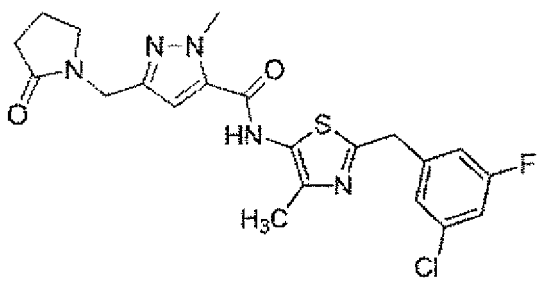

Example 236-A)

1-methyl-3-[(2-oxopyrrolidin-1-yl)methyl]-1H-pyrazole-5-carboxylic acid

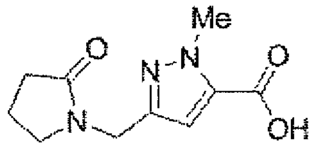

To a solution of pyrrolidin-2-one (227 mg, 2.67 mmol) in DMF (6 mL) was added sodium hydride (60%; 117 mg, 2.91 mmol) in an ice bath. After stirring at room temperature for 30 min, ethyl 3-(bromomethyl)-1-methyl-1H-pyrazole-5-carboxylate (600 mg, 2.43 mmol) was added to the reaction mixture, and the mixture was stirred at room temperature for 1 hr. Water was added to the reaction mixture and the mixture was extracted with ethyl acetate. The organic layer was washed successively with water and saturated brine, dried over anhydrous sodium sulfate, and concentrated under reduced pressure. To a solution of the obtained residue in THF (4 mL)-methanol (1 mL) was added 1N aqueous sodium hydroxide solution (2 mL), and the mixture was stirred at room temperature for 1 hr, concentrated under reduced pressure, and 1N hydrochloric acid (2 mL) was added in an ice bath. The mixture was extracted with ethyl acetate, the extract was washed twice with water, dried over anhydrous sodium sulfate and concentrated under reduced pressure to give the title compound (150 mg) as a white solid (yield 28%).
MS (ESI+): $[M+H]^+$224.
$^1$H NMR (300 MHz, DMSO-$d_6$) $\delta$ 1.80-1.99 (2H, m), 2.16-2.30 (2H, m), 3.21-3.32 (2H, m), 4.01 (3H, s), 4.29 (2H, s), 6.62 (1H, s).
* The peak of "COOH group" was not observed.

Example 236-B)

N-[2-(3-chloro-5-fluorobenzyl)-4-methyl-1,3-thiazol-5-yl]-1-methyl-3-[(2-oxopyrrolidin-1-yl)methyl]-1H-pyrazole-5-carboxamide

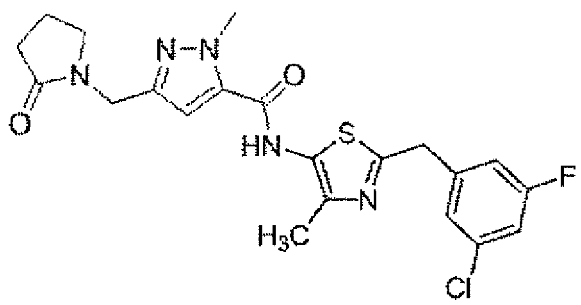

To a solution of tert-butyl [2-(3-chloro-5-fluorobenzyl)-4-methyl-1,3-thiazol-5-yl]carbamate (240 mg, 0.67 mmol) obtained in Example 145-E) in methanol (5 mL) was added dropwise 6N hydrochloric acid (2 mL), and the mixture was stirred at 50°C for 1 hr. The reaction mixture was cooled to 0°C, neutralized with 8M aqueous sodium hydroxide solution (1.5 mL), adjusted to pH 9-10 with saturated aqueous sodium hydrogen carbonate solution, and extracted with ethyl acetate. The organic layer was washed with saturated brine, dried over anhydrous sodium sulfate, and concentrated under reduced pressure. The obtained residue was dissolved in DMA (5 mL), and 1-methyl-3-[(2-oxopyrrolidin-1-yl)methyl]-1H-pyrazole-5-carboxylic acid (150 mg, 0.67 mmol) obtained in Example 236-A), HATU (281 mg, 0.74 mmol) and DIEA (0.059 mL, 0.34 mmol) were added at 0°C. The reaction mixture was stirred at 60°C for 2 hr, water was added and the mixture was extracted with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (8:2-0:1)] to give the title compound (90 mg) as a yellow oil (yield 29%).
MS (ESI+): $[M+H]^+$462.

$^1$H NMR (300 MHz, $CDCl_3$) 5 1.99 (2H, quin, J = 7.6 Hz), 2.26-2.37 (2H, m), 2.40 (3H, s), 3.39 (2H, t, J = 7.2 Hz), 4.16 (3H, s), 4.19 (2H, s), 4.44 (2H, s), 6.81 (1H, s), 6.89-6.96 (1H, m), 6.99 (1H, dt, J = 8.3, 2.1 Hz), 7.10 (1H, s), 8.81 (1H, s).

Example 237

N-[2-(3-chloro-5-fluorobenzyl)-4-methyl-1,3-thiazol-5-yl]-isothiazolidine-5-carboxamide 1,1-dioxide

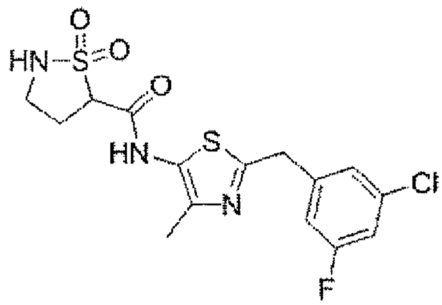

To a solution of N-[2-(3-chloro-5-fluorobenzyl)-4-methyl-1,3-thiazol-5-yl]-2-(4-methoxyphenyl)isothiazolidine-5-carboxamide 1,1-dioxide (100 mg, 0.20 mmol) obtained in Example 226 in acetonitrile (4 mL) was added a solution of ceric ammonium nitrate (344 mg, 0.63 mmol) in water (2.4 mL) at room temperature, and the mixture was stirred at the same temperature for 2.5 hr. Water was added to the reaction solution, and the mixture was extracted with ethyl acetate. The organic layer was separated, washed with saturated brine, dried over anhydrous magnesium sulfate and concentrated under reduced pressure. The obtained residue was purified by silica gel column chromatography [eluent: hexane-ethyl acetate (1:1-1:4)] and basic silica gel column chromatography [eluent: ethyl acetate-methanol (1:0-9:1)] to give the title compound (14.7 mg) as a colorless oil (yield 19%).
MS (ESI+): $[M+H]^+$ 404.
$^1$H NMR (300 MHz, $CDCl_3$) $\delta$ 2.34 (3H, s), 2.54 - 2.71 (1H, m), 2.79-2.94 (1H, m), 3.28-3.42 (1H, m), 3.48-3.68 (1H, m), 4.14-4.21 (3H, m), 6.86-6.94 (1H, m), 6.97 (1H, dt, J = 8.3, 2.1 Hz), 7.07 (1H, brs), 7.26 (1H, s), 8.98 (1H, brs).

Example 238

N-[2-(3-chloro-5-fluorobenzyl)-4-methyl-1,3-thiazol-5-yl]-1-methyl-3-(morpholinomethyl)-1H-pyrazole-5-carboxamide monohydrochloride

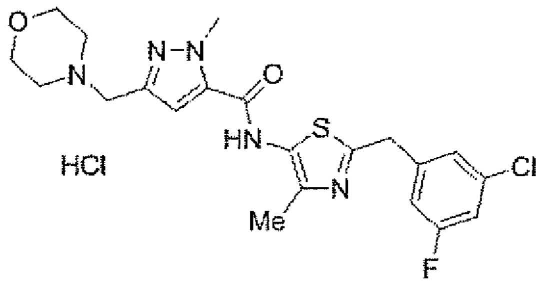

Example 238-A)

ethyl 1-methyl-3-(morpholinomethyl)-1H-pyrazole-5-carboxylate

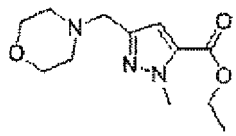

To a solution of ethyl 2-formyl-1-methyl-1H-pyrazole-5-carboxylate (1.00 g, 5.49 mmol), acetic acid (33 mg, 0.55 mmol) and morpholine (0.526 mL, 6.04 mmol) in THF (10 mL) was added sodium triacetoxyborohydride (9.44 g, 42.31 mmol), and the mixture was stirred at room temperature overnight, neutralized with saturated aqueous sodium hydrogen carbonate solution, and extracted with ethyl acetate. The organic layer was washed successively with saturated aqueous sodium hydrogen carbonate solution and saturated brine, and dried over anhydrous sodium sulfate. The obtained residue was purified by NH silica gel column chromatography [eluent: hexane-ethyl acetate (95:5-1:1)] to give the title compound (1.30 g) as a colorless liquid (yield 93%).
MS (ESI+): $[M+H]^+254$.
$^1H$ NMR (300 MHz, $CDCl_3$) δ 1.37 (3H, t, J = 7.2 Hz), 2.43-2.59 (4H, m), 3.52 (2H, s), 3.65-3.77 (4H, m), 4.15 (3H, s), 4.33 (2H, q, J = 7.2 Hz), 6.79 (1H, s).

Example 238-B)

1-methyl-3-(morpholinomethyl)-1H-pyrazole-5-carboxylic acid

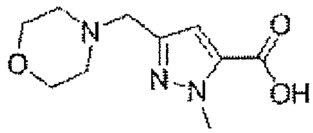

To a solution of ethyl 1-methyl-3-(morpholinomethyl)-1H-pyrazole-5-carboxylate (302 mg, 1.19 mmol) obtained in Example 238-A) in ethanol (5 mL) was added 1M aqueous sodium hydroxide solution (2.38 mL, 2.38 mmol), and the mixture was stirred at room temperature for one day and acidified with Dowex 50W-X8 ($H^+$ form). The resin was filtered off, and washed with ethanol. The filtrate was concentrated under reduced pressure to give the title compound (79 mg) as a white solid (yield 29%). MS (ESI+): $[M+H]^+226$.
$^1H$ NMR (300 MHz, DMSO-$d_6$) δ 2.31-2.43 (4H, m), 3.44 (2H, s), 3.50-3.60 (4H, m), 4.02 (3H, s), 6.67 (1H, s).
* The peak of "COOH group" was not observed.

Example 238-C)

N-[2-(3-chloro-5-fluorobenzyl)-4-methyl-1,3-thiazol-5-yl]-1-methyl-3-(morpholinomethyl)-1H-pyrazole-5-carboxamide monohydrochloride

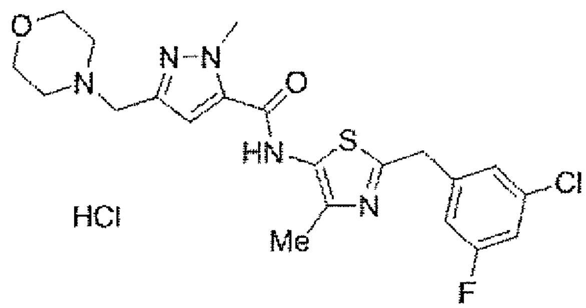

In the same manner as in Example 145-F), tert-butyl [2-(3-chloro-5-fluorobenzyl)-4-methyl-1,3-thiazol-5-yl] carbamate obtained in Example 145-E) and 1-methyl-3-(morpholinomethyl)-1H-pyrazole-5-carboxylic acid obtained in Example 238-B) were used to give the title compound (43 mg) (yield 29%).

MS (ESI+): $[M+H]^+$464.
$^1$H NMR (300 MHz, DMSO-$d_6$) δ 2.33 (3H, s), 3.01-3.20 (2H, m), 3.34 (2H, d, J = 12.1 Hz), 3.75 (2H, t, J = 11. 9 Hz) , 3.91-3.99 (2H, m), 4.06-4.11 (3H, m), 4.29 (2H, s), 4.34 (2H, d, J = 3.0 Hz), 7.23 (1H, d, J = 9.4 Hz), 7.28-7.39 (3H, m), 10.90 (1H, s), 11.23 (1H, brs).

Example 239

N-[2-(3-chloro-5-fluorobenzyl)-4-methyl-1,3-thiazol-5-yl]-3-(4-methoxyphenyl)-2-thia-3-azabicyclo[3.1.0]hexane-1-carboxamide 2,2-dioxide

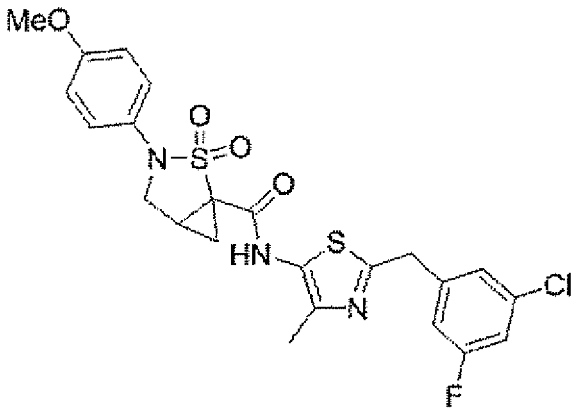

Example 239-A)

4-methoxy-N-(prop-2-en-1-yl)aniline

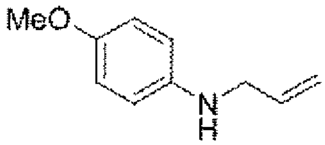

To a mixture of p-anisidine (3.0 g, 24.4 mmol), potassium carbonate (8.08 g, 58.5 mmol) and DMF (55 mL) was added 3-bromoprop-1-ene (2.12 mL, 24.4 mmol) at room temperature, and the mixture was stirred at 80°C overnight. The reaction mixture was cooled to room temperature, filtered, water was added to the filtrate, and the mixture was extracted with ethyl acetate. The organic layer was separated, washed with saturated brine, dried over anhydrous magnesium sulfate and concentrated under reduced pressure. The obtained residue was purified by silica gel column chromatography [eluent: hexane-ethyl acetate (10:1-7:3)] to give the title compound (1.87 g) as a yellow oil (yield 47%).
MS (ESI+): $[M+H]^+$ 164.
$^1$H NMR (300 MHz, DMSO-$d_6$) δ 3.55-3.70 (5H, m), 5.08 (1H, dq, J = 10.2, 1.8 Hz), 5.21 (1H, dq, J = 17.0, 1.8 Hz), 5.34 (1H, t, J = 5.9 Hz), 5.79-5.97 (1H, m), 6.47-6.58 (2H, m), 6.63-6.78 (2H, m).

Example 239-B) ethyl [(4-methoxyphenyl)(prop-2-en-1-yl)sulfamoyl]acetate

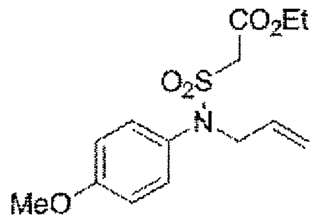

To a solution of ethyl (chlorosulfonyl)acetate (2.06 g, 11.0 mmol) in acetonitrile (35 mL) was added a solution (35 mL) of 4-methoxy-N-(prop-2-en-1-yl)aniline (1.8 g, 11.0 mmol) obtained in Example 239-A) and pyridine (1.07 mL, 13.2 mmol) in acetonitrile at 0°C, and the mixture was stirred at room temperature for 1 hr. Water was added to the reaction mixture at room temperature, and the mixture was extracted with ethyl acetate. The organic layer was separated, washed with water and saturated brine, dried over anhydrous magnesium sulfate and concentrated under reduced pressure. The obtained residue was purified by silica gel column chromatography [eluent: hexane-ethyl acetate (10:1-1:1)] to give the title compound (1.09 g) as a brown oil (yield 32%).
$^1$H NMR (300 MHz, DMSO-$d_6$) $\delta$1.22 (3H, t, J = 6.9 Hz), 3.76 (3H, s), 4.18 (2H, q, J = 6.9 Hz), 4.21-4.25 (2H, m), 4.26 (2H, s), 4.99-5.18 (2H, m), 5.60-5.83 (1H, m), 6.92-7.00 (2H, m), 7.26-7.34 (2H, m).

Example 239-C)

ethyl 3-(4-methoxyphenyl)-2-thia-3-azabicyclo[3.1.0]hexane-1-carboxylate 2,2-dioxide

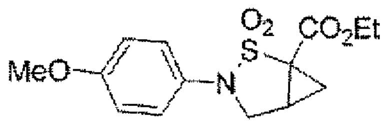

To a solution of ethyl [(4-methoxyphenyl)(prop-2-en-1-yl)sulfamoyl]acetate (1.0 g, 3.19 mmol) obtained in Example 239-B) in ethyl acetate (15 mL) was added dropwise bromine (0.172 mL, 3.35 mmol) at 0°C and the mixture was stirred at the same temperature for 30 min. To the reaction solution was further added dropwise bromine (0.172 mL, 3.35 mmol) at 0°C, and the mixture was stirred at the same temperature for 30 min., and at 30°C for 1 hr. To the reaction mixture was added water at room temperature, and the mixture was extracted with ethyl acetate. The organic layer was separated, washed with aqueous sodium sulfite solution and saturated brine, dried over anhydrous magnesium sulfate and concentrated under reduced pressure. The obtained residue was dissolved in DMF (10 mL), and added dropwise to a mixture of potassium carbonate (1.32 g, 9.57 mmol) and DMF (15 mL) at 50°C over 30 min. The mixture was stirred at the same temperature for 1 hr, cooled to room temperature, and concentrated under reduced pressure. The obtained residue was diluted with ethyl acetate, washed successively with 0.1N hydrochloric acid, water and saturated brine, dried over anhydrous magnesium sulfate and concentrated under reduced pressure. The obtained residue was purified by silica gel column chromatography [eluent: hexane-ethyl acetate (7:3-1:1)] to give the title compound (723 mg) as a brown oil (yield 73%).
MS (ESI+): $[M+H]^+$ 312.
$^1$H NMR (300 MHz, DMSO-$d_6$) $\delta$ 1.23 (3H, t, J = 7.2 Hz), 1.84-1.93 (1H, m), 2.00-2.04 (1H, m), 2.70-2.84 (1H, m), 3.61 (1H, d, J = 10 Hz), 3.75 (3H, s), 3.81-3.91 (1H, m), 4.14-4.33 (2H, m), 6.91-7.03 (2H, m), 7.18-7.33 (2H, m).

Example 239-D)

3-(4-methoxyphenyl)-2-thia-3-azabicyclo[3.1.0]hexane-1-carboxylic acid 2,2-dioxide

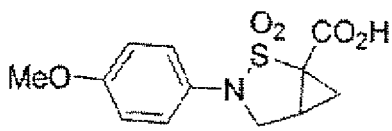

To a mixture of ethyl 3-(4-methoxyphenyl)-2-thia-3-azabicyclo[3.1.0]hexane-1-carboxylate 2,2-dioxide (500 mg, 1.61 mmol) obtained in Example 239-C) and methanol (10 mL)-water (10 mL) was added a solution of potassium hydroxide (90 mg, 1.61 mmol) in water (1 mL) at room temperature, and the mixture was stirred at the same temperature for 30 min. The reaction mixture was acidified with 0.1N hydrochloric acid at room temperature, and extracted with THF. The organic layer was separated, washed with saturated brine, dried over anhydrous magnesium sulfate and concentrated under reduced pressure. The obtained residue was crystallized from ethyl acetate-hexane to give the title compound (268 mg) as white crystals (yield 59%).
MS (ESI+): $[M+H]^+$ 284 .
$^1$H NMR (300 MHz, DMSO-$d_6$) δ 1.77-1.88 (1H, m), 1.89-1.98 (1H, m), 2.62-2.73 (1H, m), 3.58 (1H, d, J = 9.8 Hz), 3.75 (3H, s), 3.79-3.88 (1H, m), 6.88-7.02 (2H, m), 7.19-7.31 (2H, m).
* The peak of "COOH group" was not observed.

Example 239-E)

N-[2-(3-chloro-5-fluorobenzyl)-4-methyl-1,3-thiazol-5-yl]-3-(4-methoxyphenyl)-2-thia-3-azabicyclo[3.1.0]hexane-1-carboxamide 2,2-dioxide

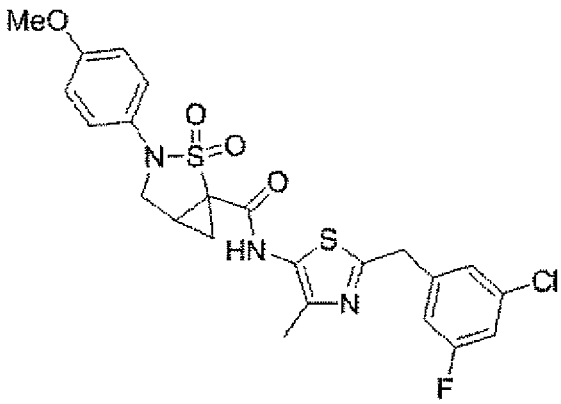

To a solution of tert-butyl [2-(3-chloro-5-fluorobenzyl)-4-methyl-1,3-thiazol-5-yl]carbamate (282 mg, 0.79 mmol) obtained in Example 145-E) in ethanol (1.5 mL) was added dropwise concentrated hydrochloric acid (0.750 mL) at room temperature, and the mixture was stirred at 50°C for 1.5 hr. The reaction mixture was cooled to room temperature, neutralized with 8M aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (8 mL), and 3-(4-methoxyphenyl)-2-thia-3-azabicyclo[3.1.0]hexane-1-carboxylic acid 2,2-dioxide (269 mg, 0.95 mmol) obtained in Example 239-D), HATU (361 mg, 0.95 mmol) and DIEA (0.069 mL, 0.40 mmol) were added at room temperature. The reaction mixture was stirred at 60°C for 1 hr, water was added and the mixture was extracted with ethyl acetate. The organic layer was washed with saturated brine, dried over anhydrous magnesium sulfate, and concentrated under reduced pressure. The obtained residue was purified by silica gel column chromatography [eluent: hexane-ethyl acetate (7:3-1:1)] and basic silica gel column chromatography [eluent: hexane-ethyl acetate (1:1-1:4)], and crystallized from ethyl acetate-hexane to give the title compound (157 mg) as colorless crystals (yield 38%).
MS (ESI+): $[M+H]^+$ 522.
$^1$H NMR (300 MHz, DMSO-$d_6$) δ 1.81-1.96 (2H, m), 2.26 (3H, s), 2.83-2.96 (1H, m), 3.66 (1H, d, J = 9.4 Hz), 3.75 (3H, s), 3.86-4.02 (1H, m), 4.26 (2H, s), 6.93-7.04 (2H, m), 7.15-7.39 (5H, m), 10.29 (1H, brs).
Anal. Calcd for $C_{23}H_{21}ClFN_3O_4S_2$: C, 52.92; H, 4.05; N, 8.05. Found: C, 52.83; H, 4.12; N, 8.06.

Example 240

3-(carbamoylmethyl)-N-[2-(3-chloro-5-fluorobenzyl)-4-methyl-1,3-thiazol-5-yl]-1-methyl-1H-pyrazole-5-carboxamide

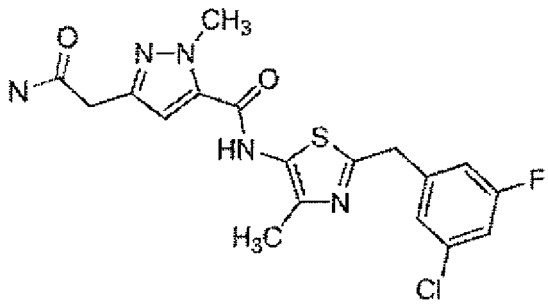

A mixture of N-[2-(3-chloro-5-fluorobenzyl)-4-methyl-1,3-thiazol-5-yl]-3-(cyanomethyl)-1-methyl-1H-pyrazole-5-carboxamide (100 mg, 0.25 mmol) obtained in Example 230 and sulfuric acid (2 mL) was stirred at 100°C for 2 hr. Water was added to the reaction mixture, and the mixture was neutralized with 8N aqueous sodium hydroxide solution, extracted with ethyl acetate (5 mL x 2), the extract was washed with water (3 x 5 mL) and saturated brine (5 mL), dried over anhydrous sodium sulfate, and concentrated under reduced pressure. The obtained residue was purified by silica gel column chromatography [eluent: ethyl acetate-methanol (9:1)] to give the title compound (70 mg) as a yellow solid (yield 48%).
MS (ESI+): $[M+H]^+$423.
$^1$H NMR (300 MHz, DMSO-$d_6$) $\delta$ 2.31 (3H, s), 3.57 (2H, s), 4.00 (3H, s), 4.26 (2H, s), 7.02 (1H, s), 7.22 (1H, dq, J = 9.4, 1.3 Hz), 7.30 (1H, t, J = 1.7 Hz), 7.31-7.39 (1H, m), 10.55 (1H, s), 12.45 (1H, s).
The peak of "NH group" was not observed.

Example 241

N-[2-(3-chloro-5-fluorobenzyl)-4-methyl-1,3-thiazol-5-yl]-2-thia-3-azabicyclo[3.1.0]hexane-1-carboxamide 2,2-dioxide

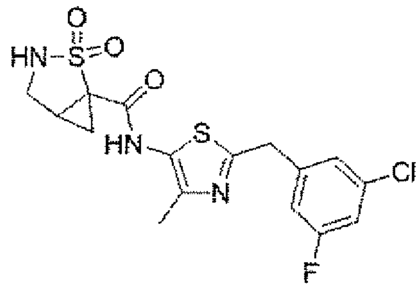

To a solution of N-[2-(3-chloro-5-fluorobenzyl)-4-methyl-1,3-thiazol-5-yl]-3-(4-methoxyphenyl)-2-thia-3-azabicyclo[3.1.0]hexane-1-carboxamide 2,2-dioxide (54.3 mg, 0.10 mmol) obtained in Example 239 in acetonitrile (2 mL) was added a solution of ceric ammonium nitrate (182 mg, 0.33 mmol) in water (1.2 mL) at room temperature, and the mixture was stirred at the same temperature for 1 hr. Water was added to the reaction solution, and the mixture was extracted with ethyl acetate. The organic layer was separated, washed with saturated brine, dried over anhydrous magnesium sulfate and concentrated under reduced pressure. The obtained residue was purified by silica gel column chromatography [eluent: hexane-ethyl acetate (1:1-1:4)] and basic silica gel column chromatography [eluent: ethyl acetate-methanol (1:0-19:1)] to give the title compound (9.9 mg) as a colorless oil (yield 23%).
MS (ESI+): $[M+H]^+$ 416.
$^1$H NMR (300 MHz, $CDCl_3$) $\delta$ 2.32-2.44 (4H, m), 2.46-2.57 (1H, m), 3.38-3.49 (1H, m), 3.56-3.69 (1H, m), 4.17 (2H, s), 4.88 (1H, t, J = 6.8 Hz), 6.70 (1H, s), 6.87-6.94 (1H, m), 6.97 (1H, dt, J = 8.5, 2.2 Hz), 7.07 (1H, brs), 9.76 (1H, s).

Example 242

N-[2-(3-chloro-5-fluorobenzyl)-4-methyl-1,3-thiazol-5-yl]-1-methyl-3-[(methylsulfonyl)methyl]-1H-pyrazole-5-carboxamide

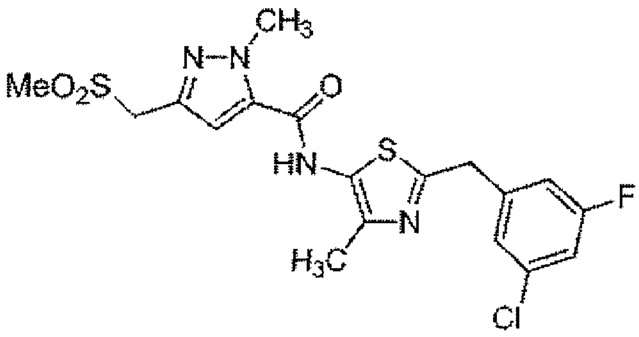

Example 242-A)

1-methyl-3-[(methylsulfonyl)methyl]-1H-pyrazole-5-carboxylic acid

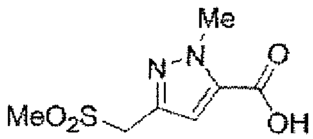

A solution of ethyl 3-(bromomethyl)-1-methyl-1H-pyrazole-5-carboxylate (150 mg, 0.61 mmol) and sodium methanesulfinate (186 mg, 1.82 mmol) in DMF (3 mL) was stirred at room temperature for 30 min. Water was added to the reaction mixture and the mixture was extracted with ethyl acetate. The organic layer was washed successively with water and saturated brine, dried over anhydrous sodium sulfate, and concentrated under reduced pressure. To s solution of the obtained residue in THF (4 mL)-methanol (1 mL) was added 1N aqueous sodium hydroxide solution (1 mL), and the mixture was stirred at room temperature for 1 hr. After concentration under reduced pressure, 1N hydrochloric acid (2 mL) was added thereto in an ice bath. The mixture was extracted with ethyl acetate, the extract was washed twice with water, dried over anhydrous sodium sulfate and concentrated under reduced pressure to give the title compound (118 mg) as a white solid (yield 89%).
MS (ESI+): $[M+H]^+$219.
$^1H$ NMR (300 MHz, DMSQ-$d_6$) $\delta$ 2.97 (3H, s), 4.04-4.14 (3H, m), 4.48 (2H, s), 6.85 (1H, s), 13.39 (1H, brs).

Example 242-B)

N-[2-(3-chloro-5-fluorobenzyl)-4-methyl-1,3-thiazol-5-yl]-1-methyl-3-[(methylsulfonyl)methyl]-1H-pyrazole-5-carboxamide

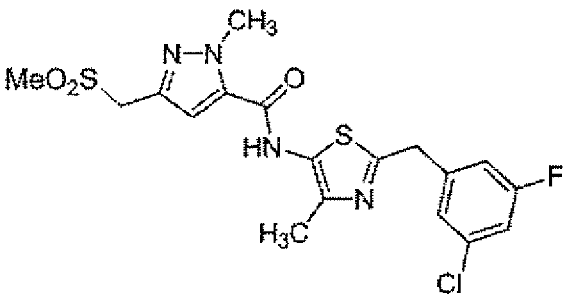

To a solution of tert-butyl [2-(3-chloro-5-fluorobenzyl)-4-methyl-1,3-thiazol-5-yl]carbamate (250 mg, 0.7 mmol) obtained in Example 145-E) in methanol (5 mL) was added dropwise 6N hydrochloric acid (2 mL), and the mixture was stirred at 50°C for 1 hr. The reaction mixture was cooled to 0°C, neutralized with 8M aqueous sodium hydroxide solution (0.75 mL), adjusted to pH 9-10 with saturated aqueous sodium hydrogen carbonate solution, and extracted with ethyl

acetate. The organic layer was washed with saturated brine, dried over anhydrous sodium sulfate, and concentrated under reduced pressure. The obtained residue was dissolved in DMA (7 mL), and 1-methyl-3-[(methylsulfonyl)methyl]-1H-pyrazole-5-carboxylic acid (130 mg, 0.60 mmol) obtained in Example 242-A), HATU (293 mg, 0.77 mmol) and DIEA (0.061 mL, 0.35 mmol) were added at 0°C. The reaction mixture was stirred at 60°C for 2 hr, water was added and the mixture was extracted with ethyl acetate. The organic layer was washed successively with water, saturated aqueous sodium hydrogen carbonate solution and saturated brine, dried over anhydrous sodium sulfate, and concentrated under reduced pressure. The obtained residue was purified by silica gel column chromatography [eluent: hexane-ethyl acetate (5:5-0:1)] to give the title compound (105 mg) as a yellow oil (yield 33%).
MS (ESI+) : $[M+H]^+$457.
$^1$H NMR (300 MHz, $CDCl_3$) δ 2.41 (3H, s), 2.90 (3H, s), 4.19 (3H, s), 4.20 (2H, s), 4.32 (2H, s), 6.89 (1H, s), 6.90-6.97 (1H, m), 7.00 (1H, dt, J = 8.6, 2.1 Hz), 7.06-7.16 (1H, m), 7.90 (1H, brs).

Example 243

N-[2-(3-chloro-5-fluorobenzyl)-4-methyl-1,3-thiazol-5-yl]-3-[(1H-imidazol-1-yl)methyl]-1-methyl-1H-pyrazole-5-carboxamide

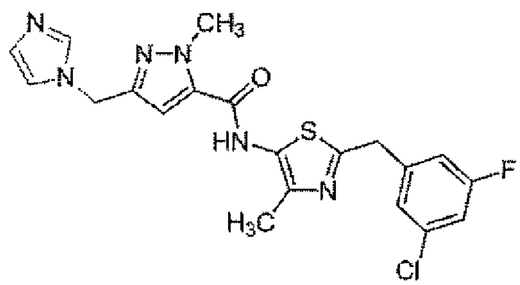

N-[2-(3-chloro-5-fluorobenzyl)-4-methyl-1,3-thiazol-3-yl]-3-(hydroxymethyl)-2-methyl-1H-pyrazole-5-carboxamide (100 mg, 0.25 mmol) obtained in Example 166 was dissolved in THF (3 mL), N,N'-carbonyldiimidazole (53.4 mg, 0.33 mmol) was added at room temperature, and the mixture was stirred at 60°C for 2 hr. Water was added to the reaction mixture, and the mixture was extracted with ethyl acetate (10 mL x 2), and the extract was dried over anhydrous sodium sulfate, and concentrated under reduced pressure. The obtained residue was purified by silica gel column chromatography [eluent: ethyl acetate-methanol (95:5)] to give the title compound (18 mg) as a yellow solid (yield 16%).
MS (ESI+): $[M+H]^+$445.
$^1$H NMR (300 MHz, $CDCl_3$) δ 2.40 (3H, s), 4.18-4.27 (5H, m), 5.44 (2H, s), 6.85 (1H, s), 6.94 (1H, dt, J = 9.1, 1.9 Hz), 6.96-7.03 (1H, m), 7.03-7.08 (1H, m), 7.10 (1H, s), 7.43 (1H, t, J = 1.3 Hz), 7.92 (1H, s), 8.15 (1H, s).

Example 244

N-[2-(3-chloro-5-flucrobenzyl)-4-methyl-1,3-thiazol-5-yl]-3-(4-methoxyphenyl)-2-thia-3-azabicyclo[4.1.0]heptane-1-carboxamide 2,2-dioxide

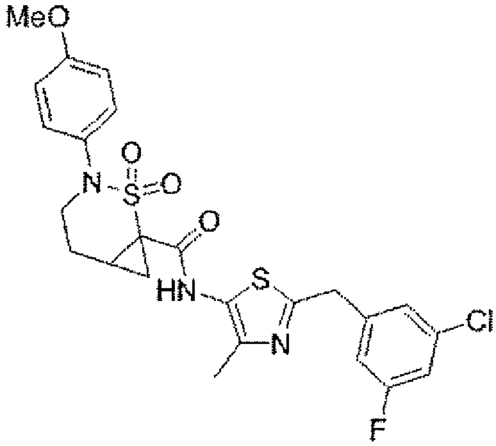

Example 244-A)

ethyl [(but-3-en-1-yl)(4-methoxyphenyl)sulfamoyl]acetate

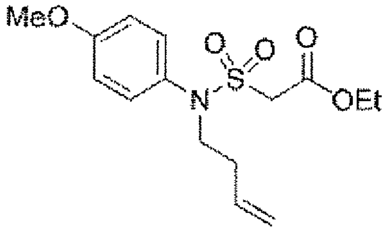

To a solution of pyridine (0.715 mL, 8.84 mmol) and p-anisidine (1.09 g, 8.84 mmol) in acetonitrile (20 mL) was added a solution of ethyl (chlorosulfonyl)acetate (1.5 g, 8.04 mmol) in acetonitrile (30 mL) at 0°C, and the mixture was stirred at the same temperature for 5 min, and at room temperature for 20 min. The reaction solution was adjusted to pH≤2 by adding water and concentrated hydrochloric acid at 0°C, and the mixture was extracted with ethyl acetate. The organic layer was separated, washed with saturated brine, dried over anhydrous magnesium sulfate and concentrated under reduced pressure. To a solution of the obtained residue and but-3-en-1-ol (0.825 mL, 9.65 mmol) in THF (80 mL) were added bis(2-methoxyethyl) (E)-diaaene-1,2-dicarboxylate (2.26 g, 9.65 mmol) and 1,2-bis(diphenylphosphane) ethyl (1.92 g, 4.82 mmol) at room temperature, and the mixture was stirred at the same temperature for 3 hr. The reaction mixture was filtered through celite, water was added to the filtrate, and the mixture was extracted with ethyl acetate. The organic layer was separated, washed with saturated brine, dried over anhydrous magnesium sulfate and concentrated under reduced pressure. The obtained residue was purified by silica gel column chromatography [eluent: hexane-ethyl acetate (1:19-19:1)] to give the title compound (308 mg) as a brown oil (yield 12%).
$^1$H NMR (300 MHz, $CDCl_3$) δ 1.34 (3H, t, J = 7.2 Hz), 2.14-2.28 (2H, m), 3.70-3.81 (2H, m), 3.82 (3H, s), 3.92 (2H, s), 4.28 (2H, q, J = 7.16 Hz), 4.96-5.10 (2H, m), 5.63-5.84 (1H, m), 6.86-6.98 (2H, m), 7.32-7.44 (2H, m).

Example 244-B)

ethyl 3-(4-methoxyphenyl)-2-thia-3-azabicyclo[4.1.0]heptane-1-carboxylate 2,2-dioxide

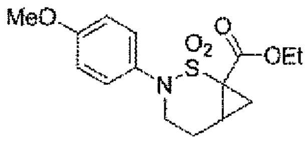

To a solution of ethyl [but-3-en-1-yl(4-methoxyphenyl)sulfamoyl]acetate (308 mg, 0.94 mmol) obtained in Example 244-A) in ethyl acetate (3 mL) was added dropwise bromine (0.072 mL, 1.41 mmol) at 0°C, and the mixture was stirred at the same temperature for 1.5 hr. To the reaction mixture was added water at room temperature, and the mixture was extracted with ethyl acetate. The organic layer was separated, washed with aqueous sodium sulfite solution and saturated brine, dried over anhydrous magnesium sulfate and concentrated under reduced pressure. The obtained residue was dissolved in DMF (4 mL), and added dropwise to a mixture of cesium carbonate (919 mg, 2.82 mmol) and DMF, (5 mL) at 50°C over 20 min. The mixture was stirred at the same temperature for 50 min. To the reaction mixture was added water at room temperature, and the mixture was extracted with ethyl acetate. The organic layer was separated, washed successively with 0.1N hydrochloric acid, water and saturated brine, dried over anhydrous magnesium sulfate and concentrated under reduced pressure. The obtained residue was purified by silica gel column chromatography [eluent: hexane-ethyl acetate (9:1-1:1)] to give the title compound (137 mg) as a pale-brown oil (yield 45%).
MS (ESI+): $[M+H]^+$ 326.
$^1H$ NMR (300 MHz, $CDCl_3$) $\delta$ 1.35 (3H, t, J = 7.1 Hz), 1.72-1.82 (1H, m), 1.90-1.98 (1H, m), 2.15-2.30 (1H, m), 2.31-2.52 (2H, m), 3.39-3.52 (1H, m), 3.79 (3H, s), 3.98-4.10 (1H, m), 4.31 (2H, qd, J = 7.1, 1.0 Hz), 6.82-6.90 (2H, m), 7.17-7.25 (2H, m).

Example 244-C)

3-(4-methoxyphenyl)-2-thia-3-azabicyclo[4.1.0]heptane-1-carboxylic acid 2,2-dioxide

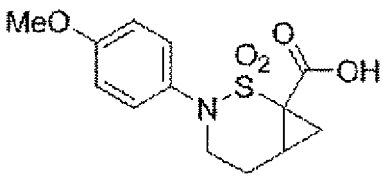

To a solution of ethyl 3-(4-methoxyphenyl)-2-thia-3-azabicyclo[4.1.0]heptane-1-carboxylate 2,2-dioxide (137 mg, 0.42 mmol) obtained in Example 244-B) in methanol (2 mL) was added a solution of potassium hydroxide (23.6 mg, 0.42 mmol) in water (2 mL) at room temperature, and the mixture was stirred at the same temperature for 1 hr. The reaction mixture was acidified with 0.1N hydrochloric acid at room temperature, and extracted with ethyl acetate. The organic layer was separated, dried over anhydrous magnesium sulfate and concentrated under reduced pressure to give the title compound (120 mg) as white crystals (yield 96%).
MS (ESI+): $[M+H]^+$ 298.
$^1H$ NMR (300 MHz, DMSO-$d_6$) $\delta$ 1.64-1.75 (1H, m), 1.79-1.89 (1H, m), 2.08-2.42 (3H, m), 3.37-3.51 (1H, m), 3.75 (3H, s), 3.77-3.91 (1H, m), 6.87-6.97 (2H, m), 7.17-7.26 (2H, m), 13.51 (1H, brs).

Example 244-D)

N-[2-(3-chloro-5-fluorobenzyl)-4-methyl-1,3-thiazol-5-yl]-3-(4-methoxyphenyl)-2-thia-3-azabicyclo[4.1.0]heptane-1-carboxamide 2,2-dioxide

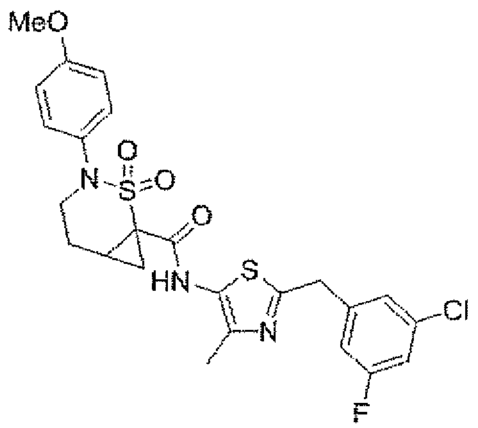

To a solution of tert-butyl [2-(3-chloro-5-fluorobenzyl)-4-methyl-1,3-thiazol-5-yl]carbamate (121 mg, 0.34 mmol) obtained in Example 145-E) in ethanol (2 mL) was added dropwise concentrated hydrochloric acid (1.0 mL) at 50°C, and the mixture was stirred at the same temperature for 1 hr. The reaction mixture was cooled to room temperature, neutralized with 8M aqueous sodium hydroxide solution, adjusted to pH 10-11 with saturated aqueous sodium hydrogen carbonate solution, and extracted with ethyl acetate. The organic layer was washed with saturated brine, dried over anhydrous magnesium sulfate, and concentrated under reduced pressure. The obtained residue was dissolved in DMA (5 mL), and 3-(4-methoxyphenyl)-2-thia-3-azabicyclo[4.1.0]heptane-1-carboxylic acid 2,2-dioxide (121 mg, 0.41 mmol) obtained in Example 244-C), HATU (155 mg, 0.41 mmol) and DIEA (0.029 mL, 0.17 mmol) were added at room temperature. The reaction mixture was stirred at 60°C for 2 hr, cooled to room temperature, water was added and the mixture was extracted with ethyl acetate. The organic layer was washed with 0.1N hydrochloric acid and saturated brine, dried over anhydrous magnesium sulfate, and concentrated under reduced pressure. The obtained residue was purified by basic silica gel column chromatography [eluent: hexane-ethyl acetate (9:1-3:2)] and silica gel column chromatography [eluent: hexane-ethyl acetate (9:1-1:1)] to give the title compound (63.6 mg) as a pale-yellow oil (yield 35%).
MS (ESI+): $[M+H]^+$ 536.
$^1H$ NMR (300 MHz, $CDCl_3$) $\delta$ 1.93 (1H, dd, J = 8.1, 5.9 Hz), 2.10-2.25 (2H, m), 2.26-2.43 (4H, m), 2.43-2.55 (1H, m), 3.61-3.74 (1H, m), 3.75-3.93 (4H, m), 4.16 (2H, s), 6.85-6.94 (3H, m), 6.97 (1H, dt, J = 8.5, 2.0 Hz), 7.08 (1H, brs), 7.17-7.25 (2H, m), 10.27 (1H, s).

Example 245

N-(2-(3-chloro-5-fluorobenzyl)-4-methyl-1,3-thiazol-5-yl]-1-methyl-3-(methylsulfanyl)-1H-pyrazole-5-carboxamide

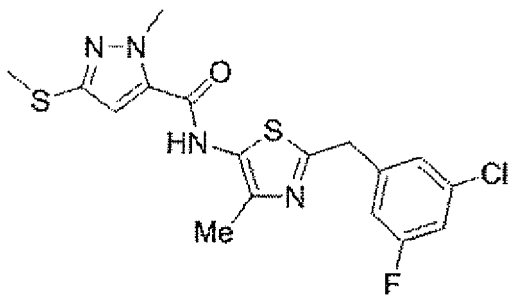

Example 245-A)

methyl 1-methyl-3-(methylsulfanyl)-1H-pyrazole-5-carboxylate

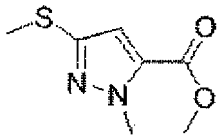

To a solution of methyl 4,4-bis(methylsulfanyl)-2-oxobut-3-enoate (1.00 g, 4.85 mmol) in methanol (10 mL) was added methylhydrazine (0.246 g, 5.33 mmol) at room temperature, and the mixture was stirred at 60°C overnight. The reaction mixture was cooled to room temperature, and concentrated under reduced pressure. The obtained residue was purified by silica gel column chromatography [eluent: hexane-ethyl acetate (95:5-1:1)] to give the title compound (0.613 g) as a colorless liquid (yield 68%).
MS (ESI+): $[M+H]^+$187.
$^1$H NMR (300 MHz, $CDCl_3$) $\delta$2.44 (3H, s), 3.92 (3H, s), 3.94 (3H, s), 6.79 (1H, s).

Example 245-B)

1-methyl-3-(methylsulfanyl)-1H-pyrazole-5-carboxylic acid

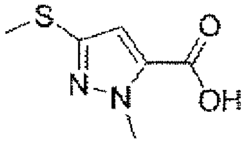

In the same manner as in Example 235-C), methyl 1-methyl-3-(methylsulfanyl)-1H-pyrazole-5-carboxylate obtained in Example 245-A) was used to give the title compound (334 mg) (yield 59%).
$^1$H NMR (300 MHz, DMSO-$d_6$) $\delta$ 2.47 (3H, s), 3.84 (3H, s), 6.77 (1H, s), 12.69 (1H, brs).

Example 245-C)

N-[2-(3-chloro-5-fluorobenzyl)-4-methyl-1,3-thiazol-5-yl]-1-methyl-3-(methylsulfanyl)-1H-pyrazole-5-carboxamide

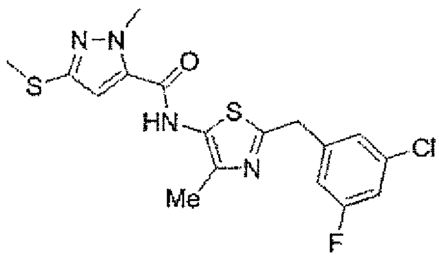

In the same manner as in Example 145-F), tert-butyl [2-(3-chloro-5-fluorobenzyl)-4-methyl-1,3-thiazol-5-yl] carbamate obtained in Example 145-E) and 1-methyl-3-(methylsulfanyl)-1H-pyrazole-5-carboxylic acid obtained in Example 245-B) were used to give the title compound (277 mg) (yield 42%). MS (ESI+): $[M+H]^+$411.
$^1$H NMR (300 MHz, DMSO-$d_6$) $\delta$ 2.27 (3H, s), 3.32 (3H, s), 3.89 (3H, s), 4.25 (2H, s), 6.86 (1H, s), 7.21 (1H, dd, J = 9.4, 1.5 Hz), 7.28-7.31 (1H, m), 7.31-7.37 (1H, m), 10.30 (1H, s).

Example 246

N-[2-(3-chloro-5-fluorobenzyl)-4-methyl-1,3-thiazol-5-yl]-1-methyl-3-(methylsulfonyl)-1H-pyrazole-5-carboxamide

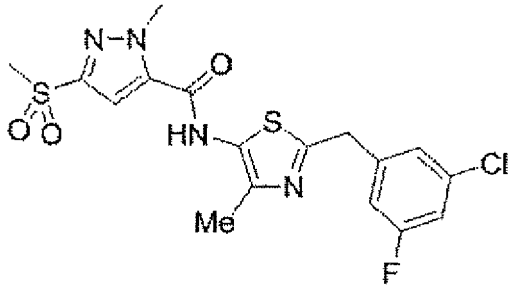

In the same manner as in Example 208, N-[2-(3-chloro-5-fluorobenzyl)-4-methyl-1,3-thiazol-5-yl]-1-methyl-3-(methylsulfanyl)-1H-pyrazole-5-carboxamide obtained in Example 245 was used to give the title compound (74 mg) (yield 30%). MS (ESI+): $[M+H]^+$443.
$^1$H NMR (300 MHz, DMSO-$d_6$) $\delta$ 2.29 (3H, s), 3.49 (3H, s), 4.19 (3H, s), 4.26 (2H, s), 7.22 (1H, d, J = 9.8 Hz), 7.30 (1H, s), 7.31-7.37 (1H, m), 7.44 (1H, s), 10.60 (1H, s).

Example 247

N-[2-(3-chloro-5-fluorobenzyl)-4-methyl-1,3-thiazol-5-yl]-1-methyl-3-(methylsulfinyl)-1H-pyrazole-5-carboxamide

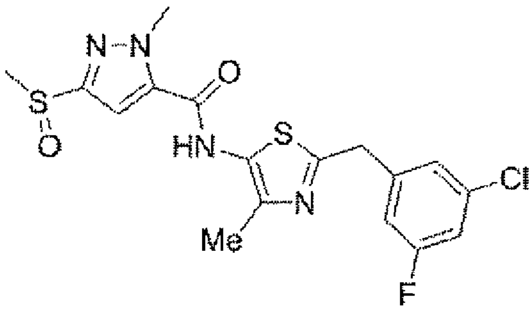

In the same manner as in Example 232, N-[2-(3-chloro-5-fluorobenzyl)-4-methyl-1,3-thiazol-5-yl]-1-methyl-3-(methylsulfanyl)-1H-pyrazole-5-carboxamide obtained in Example 245 was used to give the title compound (103 mg) (yield 43%). MS (ESI+): $[M+H]^+$427.
$^1$H NMR (300 MHz, DMSO-$d_6$) $\delta$ 2.29 (3H, s), 3.07 (3H, s), 4.11 (3H, s), 4.26 (2H, s), 7.17-7.25 (1H, m), 7.30 (1H, s), 7.31-7.38 (2H, m), 10.51 (1H, s).

Example 248

N-[2-(3-chloro-5-fluorobenzyl)-4-methyl-1,3-thiazol-5-yl]-2-thia-3-azabicyclo[4.1.0]heptane-1-carboxamide 2,2-dioxide

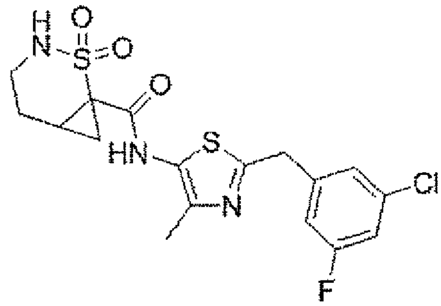

To a solution of N-[2-(3-chloro-5-fluorobenzyl)-4-methyl-1,3-thiazol-5-yl]-3-(4-methoxyphenyl)-2-thia-3-azabicyclo[4.1.0]heptane-1-carboxamide 2,2-dioxide (46.3 mg, 0.09 mmol) obtained in Example 244 in acetonitrile (1 mL) was added a solution of ceric ammonium nitrate (152 mg, 0.28 mmol) in water (0.60 mL) at room temperature, and the mixture was stirred at the same temperature for 30 min. Water was added to the reaction solution, and the mixture was extracted with ethyl acetate. The organic layer was separated, washed with saturated brine, dried over anhydrous magnesium sulfate and concentrated under reduced pressure. The obtained residue was purified by silica gel column chromatography [eluent: hexane-ethyl acetate (1:1-0:1)] and basic silica gel column chromatography [eluent: ethyl acetate-methanol (1:0-19:1)] to give the title compound (8.6 mg) as a colorless oil (yield 23%).
MS (ESI+): $[M+H]^+$ 430.
$^1H$ NMR (300 MHz, $CDCl_3$) $\delta$ 1.60 (1H, dd, J = 8.1, 5.9 Hz), 1.86-2.00 (1H, m), 2.06-2.28 (2H, m), 2.28-2.43 (4H, m), 3.26-3.39 (1H, m), 3.43-3.58 (1H, m), 4.17 (2H, s), 6.85-6.94 (1H, m), 6.97 (1H, dt, J = 8.5, 2.0 Hz), 7.07 (1H, brs), 7.26 (1H, s), 10.27 (1H, brs).

Example 249

N-[2-(3-cyclopropyl-5-fluorobenzyl)-4-methyl-1,3-thiazol-5-yl]-1-methyl-1H-pyrazole-5-carboxamide

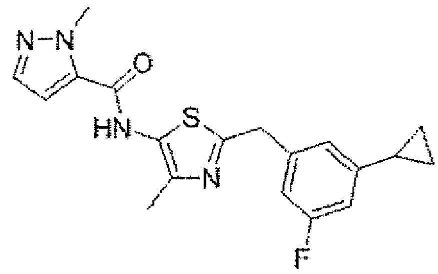

Example 249-A)

3-cyclopropyl-5-fluorobenzoic acid

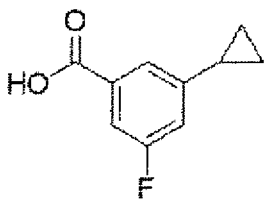

To a mixture of 3-bromo-5-fluorobenzoic acid (5.0 g, 22.8 mmol), cyclopropylboronic acid (5.88 g, 68.5 mmol), potassium phosphate (38.8 g, 183 mmol) and toluene (90 mL)-water (10 mL) was added tetrakis(triphenylphosphine) palladium (0) (5.28 g, 4.57 mmol) at room temperature, and the mixture was stirred at 100°C for 8 hr. The reaction mixture was cooled to room temperature, 1N aqueous sodium hydroxide solution was added, and the mixture was extracted with ethyl acetate. The organic layer was separated, washed with water and saturated brine, dried over anhydrous magnesium sulfate and concentrated under reduced pressure. The obtained residue was purified by silica gel column chromatography [eluent: hexane-ethyl acetate (1:4-0:1)] to give the title compound (1.39 g) as a pale-yellow oil (yield 34%).
$^1H$ NMR (300 MHz, DMSO-$d_6$) $\delta$ 0.70-0.80 (2H, m), 0.96-1.06 (2H, m), 1.96-2.13 (1H, m), 7.13-7.21 (1H, m), 7.36-7.44 (1H, m), 7.49-7.54 (1H, m), 13.23 (1H, brs).

Example 249-B)

3-cyclopropyl-5-fluorobenzyl methanesulfonate

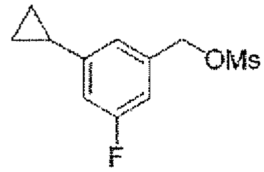

To a solution of 3-cyclopropyl-5-fluorobenzoic acid (1.39 g, 7.71 mmol) obtained in Example 249-A) in THF (7 mL) was added dropwise 1.1M borane-THF solution (14.0 mL, 15.4 mmol) at 0°C, and the mixture was stirred at room temperature for 1 hr. To the reaction mixture were added water and 1N hydrochloric acid at 0°C, and the mixture was extracted with ethyl acetate. The organic layer was separated, washed with water and saturated brine, dried over anhydrous magnesium sulfate and concentrated under reduced pressure. To a solution of the obtained residue and triethylamine (3.22 mL, 23.1 mmol) in THF (80 mL) was added methanesulfonyl chloride (1.19 ml, 15.4 mmol) at 0°C, and the mixture was stirred at the same temperature for 30 min. To the reaction mixture was added water at room temperature, and the mixture was extracted with ethyl acetate. The organic layer was separated, washed with saturated brine, dried over anhydrous magnesium sulfate and concentrated under reduced pressure. The obtained residue was purified by silica gel column chromatography [eluent: hexane-ethyl acetate (4:1-3:2)] to give the title compound (2.04 g) as a colorless oil (yield quant.).
$^1$H NMR (300 MHz, DMSO-$d_6$) $\delta$ 0.67-0.78 (2H, m), 0.94-1.05 (2H, m), 1.90-2.04 (1H, m), 3.25 (3H, s), 5.21 (2H, s), 6.93 (1H, dt, J = 10.3, 2.0 Hz), 6.98-7.11 (2H, m).

Example 249-C)

(3-cyclopropyl-5-fluorophenyl)acetonitrile

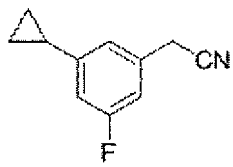

To a solution of 3-cyclopropyl-5-fluorobenzyl methanesulfonate (2.04 g, 8.35 mmol) obtained in Example 249-B) in acetonitrile (80 mL) were added sodium cyanide (819 mg, 16.7 mmol) and 18-crown 6-ether (440 mg, 1.66 mmol) at room temperature, and the mixture was stirred at 65°C for 4.5 hr. The reaction mixture was filtered, and the filtrate was concentrated under reduced pressure. The obtained residue was purified by silica gel column chromatography [eluent: hexane-ethyl acetate9:1-4:1] to give the title compound (930 mg) as a pale-yellow oil (yield 64%).
MS (ESI-): $[M-H]^-$ 174.
$^1$H NMR (400 MHz, $CDCl_3$) $\delta$ 0.66-0.74 (2H, m), 0.97-1.07 (2H, m), 1.83-1.96 (1H, m), 3.70 (2H, s), 6.64-6.74 (1H, m), 6.77-6.88 (2H, m).

Example 249-D)

2-(3-cyclopropyl-5-fluorophenyl)ethanethioamide

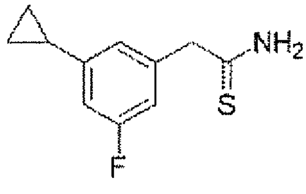

A mixture of (3-cyclopropyl-5-fluorophenyl)acetonitrile (930 mg, 5.31 mmol) obtained in Example 249-C), O,O-diethyl dithiophosphate (0.841 mL, 5.31 mmol) and 4N hydrogen chloride-ethanol (13 mL) was stirred at room temperature

overnight. To the reaction mixture was added water at 0°C, and the mixture was extracted with ethyl acetate. The organic layer was separated, washed successively with 0.1N aqueous sodium hydroxide solution, water and saturated brine, dried over anhydrous magnesium sulfate and concentrated under reduced pressure. The obtained residue was purified by silica gel column chromatography [eluent: hexane-ethyl acetate17:3-1:1] to give the title compound (670 mg) as a pale-yellow oil (yield 60%).
MS (ESI+): $[M+H]^+$ 210.
$^1H$ NMR (300 MHz, DMSO-$d_6$) δ 0.59-0.77 (2H, m), 0.91-1.03 (2H, m), 1.83-1.97 (1H, m), 3.76 (2H, s), 6.67-6.84 (1H, m), 6.84-7.02 (2H, m), 9.01-9.75 (2H, m).

Example 249-E)

ethyl 2-(3-cyclopropyl-5-fluorobenzyl)-4-methyl-1,3-thiazole-5-carboxylate

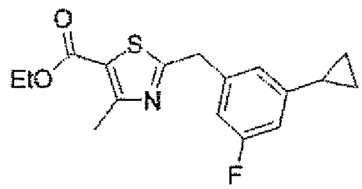

A solution of 2-(3-cyclopropyl-5-fluorophenyl)ethanethioamide (670 mg, 3.20 mmol) obtained in Example 249-D) and ethyl 2-chloro-3-oxobutanoate (0.531 mL, 3.84 mmol) in pyridine (2 mL)-ethanol (2 mL) was stirred at room temperature for 2 days. The reaction mixture was diluted with ethyl acetate, washed with 0.1N hydrochloric acid, water and saturated brine, dried over anhydrous magnesium sulfate, and concentrated under reduced pressure. The obtained residue was purified by silica gel column chromatography [eluent: hexane-ethyl acetate (9:1-7:3)] to give the title compound (897 mg) as a colorless oil (yield 88%).
MS (ESI+): $[M+H]^+$ 320.
$^1H$ NMR (300 MHz, DMSO-$d_6$) δ 0.65-0.74 (2H, m), 0.91-1.02 (2H, m), 1.25 (3H, t, J = 7.0 Hz), 1.87-1.98 (1H, m), 2.60 (3H, s), 4.23 (2H, q, J = 7.0 Hz), 4.27 (2H, s), 6.80 (1H, dt, J = 10, 2.0 Hz), 6.91-7.01 (2H, m).

Example 249-F)

2-(3-cyclopropyl-5-fluorobenzyl)-4-methyl-1,3-thiazole-5-carboxylic acid

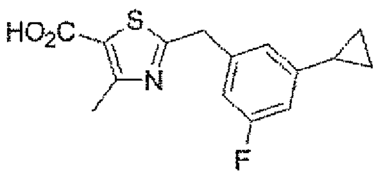

To a solution (7 mL) of ethyl 2-(3-cyclopropyl-5-fluorobenzyl)-4-methyl-1,3-thiazole-5-carboxylate (896 mg, 2.81 mmol) obtained in Example 249-E) in ethanol was added dropwise 8M aqueous sodium hydroxide solution (2.0 mL) at room temperature, and the mixture was stirred at the same temperature for 1 hr. The reaction mixture was ice-cooled, and 6N hydrochloric acid (6 mL) was added. The precipitate was collected by filtration, and washed with water to give the title compound (695 mg) as a white solid (yield 85%). MS (ESI+): $[M+H]^+$ 292.
$^1H$ NMR (300 MHz, DMSO-$d_6$) δ 0.64-0.74 (2H, m), 0.90-1.02 (2H, m), 1.86-2.00 (1H, m), 2.58 (3H, s), 4.25 (2H, s), 6.79 (1H, dt, J = 10, 2.0 Hz), 6.89-6.99 (2H, m), 13.19 (1H, brs).

Example 249-G)

tert-butyl [2-(3-cyclopropyl-5-fluorobenzyl)-4-methyl-1,3-thiazol-5-yl]carbamate

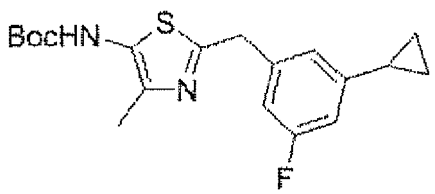

To a solution (6 mL) of 2-(3-cyclopropyl-5-fluorobenzyl)-4-methyl-1,3-thiazole-5-carboxylic acid (695 mg, 2.39 mmol) obtained in Example 249-F) in tert-butyl alcohol was added triethylamine (0.997 mL, 7.16 mmol) at room temperature, and the mixture was heated to 90°C and DPPA (0.774 mL, 3.58 mmol) was added. The mixture was stirred for 30 min at the same temperature. The reaction product was concentrated under reduced pressure, and the residue was diluted with ethyl acetate, washed with 0.1N aqueous sodium hydroxide solution and saturated brine, dried over anhydrous magnesium sulfate and concentrated under reduced pressure. The residue was purified by silica gel column chromatography [eluent: hexane-ethyl acetate (9:1-3:2)] and basic silica gel column chromatography [eluent: hexane-ethyl acetate (4:1-3:2)] to give the title compound (503 mg) as a pale-yellow oil (yield 58%).
MS (ESI+): $[M+H]^+$363.
$^1$H NMR (300 MHz, DMSO-$d_6$) $\delta$ 0.64-0.73 (2H, m), 0.91-1.01 (2H, m), 1.43 (9H, s), 1.86-1.97 (1H, m), 2.18 (3H, s), 4.10 (2H, s), 6.70-6.80 (1H, m), 6.82-6.89 (1H, m), 6.91 (1H, brs), 9.57 (1H, brs).

Example 249-H)

N-[2-(3-cyclopropyl-5-fluorobenzyl)-4-methyl-1,3-thiazol-5-yl]-1-methyl-1H-pyrazole-5-carboxamide

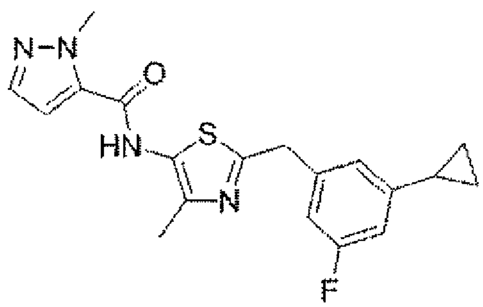

To a solution of tert-butyl [2-(3-cyclopropyl-5-fluorobenzyl)-4-methyl-1,3-thiazol-5-yl]carbamate (490 mg, 1.35 mmol) obtained in Example 249-G) in ethanol (4 mL) was added dropwise concentrated hydrochloric acid (2.0 mL) at 50°C, and the mixture was stirred at the same temperature for 1 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was washed with saturated brine, dried over anhydrous magnesium sulfate, and concentrated under reduced pressure. The obtained residue was dissolved in DMA (6 mL), and 1-methyl-1H-pyrazole-5-carboxylic acid (204 mg, 1.62 mmol), HATU (616 mg, 1.62 mmol) and DIEA (0.118 mL, 0.68 mmol) were added at room temperature. The reaction mixture was stirred at 60°C for 2 hr, water was added and the mixture was extracted with ethyl acetate. The organic layer was washed with saturated aqueous sodium hydrogen carbonate solution and saturated brine, dried over anhydrous magnesium sulfate, and concentrated under reduced pressure. The obtained residue was purified by silica gel column chromatography [eluent: hexane-ethyl acetate (1:1-1:4)] and basic silica gel column chromatography [eluent: hexane-ethyl acetate (1:1-1:4)] to give the title compound (418 mg) as a colorless oil (yield 84%).
MS (ESI+): $[M+H]^+$ 371.
$^1$H NMR (300 MHz, DMSO-$d_6$) $\delta$ 0.64-0.75 (2H, m), 0.91-1.02 (2H, m), 1.88-1.99 (1H, m), 2.30 (3H, s), 4.05 (3H, s),

4.18 (2H, s), 6.77 (1H, dt, J = 10, 2.0 Hz), 6.86-6.99 (2H, m), 7.06 (1H, d, J = 1.8 Hz), 7.54 (1H, d, J = 1.9 Hz), 10.50 (1H, s).

Example 250

N-[2-(3-chloro-5-fluorobenzyl)-4-methyl-1,3-thiazol-5-yl]-tetrahydro-2H-thiopyran-2-carboxamide 1,1-dioxide

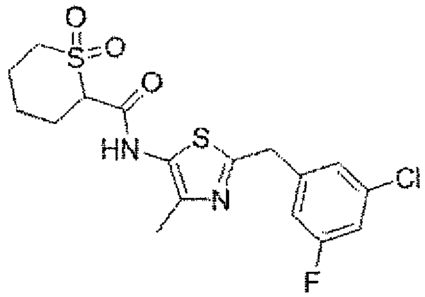

Example 250-A)

tetrahydro-2H-thiopyran-2-carboxylic acid 1,1-dioxide

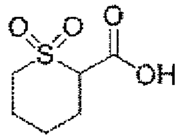

To a solution of tetrahydro-2H-thiopyran-2-carboxylic acid (11.1 g, 76.0 mmol) in methanol (90 mL)-water (90 mL) was added dropwise oxone (93.0 g, 152 mmol) at 50°C, and the mixture was stirred at room temperature for 18 hr. The reaction mixture was extracted with ethyl acetate, and the organic layer was dried over anhydrous sodium sulfate, and concentrated under reduced pressure to give the title compound (5.1 g) as yellow crystals (yield 38%).
$^1$H NMR (400 MHz, DMSO-$d_6$) δ 1.47-1.56 (1H, m), 1.69-1.76 (1H, m), 1.82-2.06 (3H, m), 2.12-2.17 (1H, m), 3.16-3.19 (2H, m), 4.11-4.13 (1H, m).
* The peak of "COOH group" was not observed.

Example 250-B)

N-[2-(3-chloro-5-fluorobenzyl)-4-methyl-1,3-thiazol-5-yl]tetrahydro-2H-thiopyran-2-carboxamide 1,1-dioxide

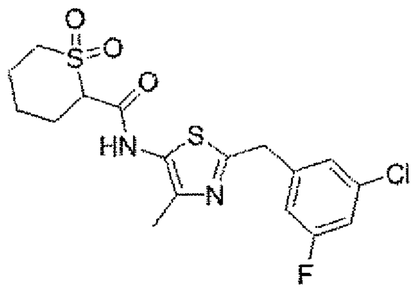

To a solution of tert-butyl [2-(3-chloro-5-fluorobenzyl)-4-methyl-1,3-thiazol-5-yl]carbamate (300 mg, 0.84 mmol) obtained in Example 145-E) in ethanol (4 mL) was added dropwise concentrated hydrochloric acid (2 mL) at 50°C, and

the mixture was stirred at the same temperature for 1 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was washed with saturated brine, dried over anhydrous magnesium sulfate, and concentrated under reduced pressure. The obtained residue was dissolved in DMA (6 mL) and tetrahydro-2H-thiopyran-2-carboxylic acid 1,1-dioxide (180 mg, 1.01 mmol) obtained in Example 250-A), HATU (384 mg, 1.01 mmol) and DIEA (0.073 mL, 0.42 mmol) were added at room temperature. The reaction mixture was stirred at 60°C for 4 hr, saturated aqueous sodium hydrogen carbonate solution was added and the mixture was extracted with ethyl acetate. The organic layer was washed with saturated brine, dried over anhydrous magnesium sulfate, and concentrated under reduced pressure. The obtained residue was purified by silica gel column chromatography [eluent: hexane-ethyl acetate (1:1-0:1)] and basic silica gel column chromatography [eluent: hexane-ethyl acetate (1:1-0:1)], and crystallized from ethyl acetate-hexane to give the title compound (150 mg) as white crystals (yield 43%).
MS (ESI+): $[M+H]^+$ 417.
$^1$H NMR (300 MHz, DMSO-$d_6$) $\delta$ 1.41-1.62 (1H, m), 1.76-2.04 (3H, m), 2.07-2.25 (2H, m), 2.31 (3H, s), 3.02-3.18 (1H, m), 3.26-3.41 (1H, m), 4.16-4.33 (3H, m), 7.15-7.23 (1H, m), 7.25-7.29 (1H, m), 7.33 (1H, dt, J = 8.7, 2.2 Hz), 10.68 (1H, s).

Example 251

N-[2-(3-chloro-5-fluorobenzyl)-4-methyl-1,3-thiazol-5-yl]-1-(4-fluorophenyl)-1H-imidazole-5-carboxamide

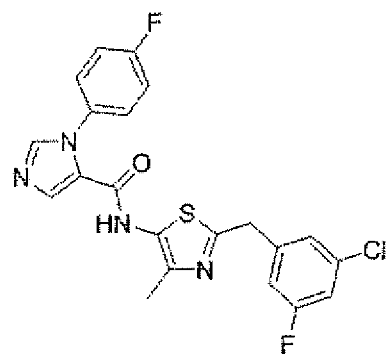

Example 251-A)

ethyl 1-(4-fluorophenyl)-1H-imidazole-5-carboxylate

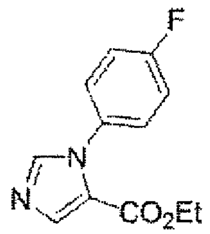

To a solution of ethyl 2-oxoacetate (4.59 g, 45.0 mmol) in toluene (100 mL) were added 4-fluoroaniline (5.00 g, 45.0 mmol) and sodium sulfate (6.39 g, 45.0 mmol) at room temperature, and the mixture was stirred at 110°C for 4 hr. The reaction mixture was cooled to room temperature, the precipitate was filtered off, and the obtained filtrate was concentrated under reduced pressure. The obtained residue was dissolved in THF (100 mL), and toluenesulfonylmethyl isocyanide (10.5 g, 54.0 mmol) and potassium carbonate (12.4 g, 90.0 mmol) were added at room temperature. The reaction mixture was stirred at 65°C for 4 hr, water (100 mL) was added and the mixture was extracted with ethyl acetate (500 mL). The organic layer was washed with water (100 mL) and saturated brine (100 mL), dried over anhydrous magnesium sulfate, and concentrated under reduced pressure. The obtained residue was purified by silica gel column chromatography [eluent: hexane-ethyl acetate (1:1)] to give the title compound (4.7 g) as a brown solid (yield 45%).

MS (ESI+): $[M+H]^+$ 235.
$^1H$ NMR (300 MHz, $CDCl_3$) δ 1.26 (3H, t, J = 7.2 Hz), 4.22 (2H, q, J = 7.2 Hz), 7.12-7.22 (2H, m), 7.29-7.35 (2H, m), 7.65 (1H, s), 7.85 (1H, s).

Example 251-B)

1-(4-fluorophenyl)-1H-imidazole-5-carboxylic acid

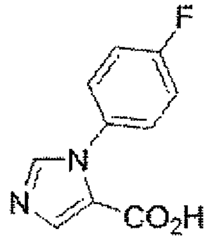

To a solution (15 mL) of ethyl 1-(4-fluorophenyl)-1H-imidazole-5-carboxylate (1.00 g, 4.27 mmol) obtained in Example 251-A) in ethanol was added 8N aqueous sodium hydroxide solution (1.6 mL, 12.8 mmol) at room temperature. The mixture was stirred at 80°C for 3 hr, 6N hydrochloric acid (26.0 mL) was added in an ice bath, and the mixture was extracted with ethyl acetate (100 mL). The organic layer was washed with water (10 mL) and saturated brine (10 mL), dried over anhydrous magnesium sulfate and concentrated under reduced pressure to give the title compound (230 mg) as a pale-yellow solid (yield 26%).
MS (ESI+) :$[M+H]^+$ 207.
$^1H$ NMR (300 MHz, DMSO-$d_6$) δ 7.27-7.38 (2H, m), 7.43-7.55 (2H, m), 7.69-7.75 (1H, m), 8.00-8.05 (1H, m), 12.20 (1H, brs).

Example 251-C)

N-[2-(3-chloro-5-fluorobenzyl)-4-methyl-1,3-thiazol-5-yl]-1-(4-fluorophenyl)-1H-imidazole-5-carboxamide

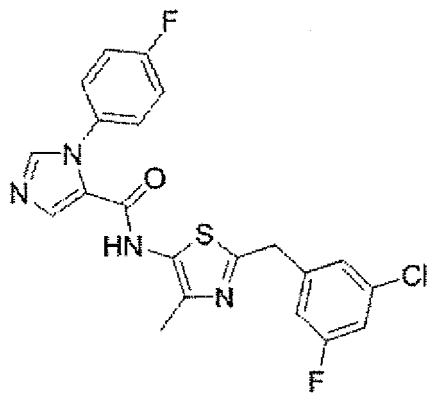

In the same manner as in Example 158, tert-butyl [2-(3-chloro-5-fluorobenzyl)-4-methyl-1,3-thiazol-5-yl]carbamate obtained in Example 145-E) and 1-(4-fluorophenyl)-1H-imidazole-5-carboxylic acid obtained in Example 251-B) were used to give the title compound as white crystals (yield 33%).
MS (ESI+): $[M+H]^+$ 461.
$^1H$ NMR (300 MHz, DMSO-$d_6$) δ 2.28 (3H, s), 4.28 (2H, s), 7.26-7.37 (2H, m), 7.39-7.48 (2H, m), 7.52 (2H, d, J = 7.9 Hz), 7.68 (2H, d, J = 7.9 Hz), 7.88 (1H, s), 8.06 (1H, s), 10.54 (1H, s).

Example 252

N-[2-(3-chloro-5-fluorobenzyl)-4-methyl-1,3-thiazol-5-yl]-2-(1H-tetrazol-1-yl)acetamide

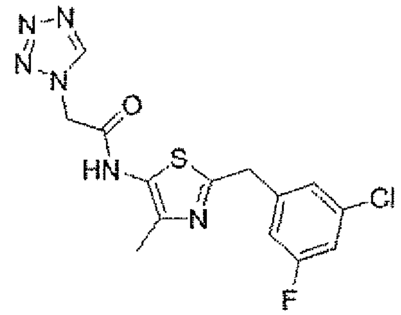

In the same manner as in Example 158, tert-butyl [2-(3-chloro-5-fluorobenzyl)-4-methyl-1,3-thiazol-5-yl]carbamate obtained in Example 145-E) and (1H-tetrazol-1-yl)acetic acid were used to give the title compound as white crystals (yield 46%).
MS (ESI+): $[M+H]^+$ 367.
$^1$H NMR (300 MHz, DMSO-$d_6$) $\delta$ 2.34 (3H, s), 4.22 (2H, s), 5.57 (2H, s), 7.13-7.22 (1H, m), 7.22-7.28 (1H, m), 7.32 (1H, dt, J = 8.8, 2.2 Hz), 9.41 (1H, s), 11.00 (1H, s).

Example 253

N-[2-(4-methoxybenzyl)-4-methyl-1,3-thiazol-5-yl]-1-methyl-1H-pyrazole-5-carboxamide

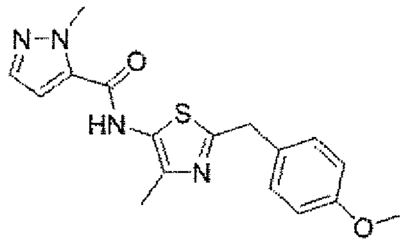

Example 253-A)

2-(4-methoxyphenyl)ethanethioamide

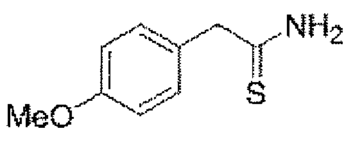

A mixture of (4-methoxyphenyl)acetonitrile (4.00 g, 27.2 mmol), O,O-diethyl dithiophosphate (5.94 mL, 32.6 mmol) and 4M hydrogen chloride-1,4-dioxane (54 mL) was stirred at room temperature for 18 hr. The reaction mixture was diluted with ethyl acetate (100 mL), and water (100 mL) was added. The organic layer was separated, washed successively with 0.2N aqueous sodium hydroxide solution (100 mL) and saturated brine (100 mL), dried over anhydrous sodium sulfate, and concentrated under reduced pressure. The obtained residue was treated with hexane to allow solidification, and the solid was collected by filtration, and washed with hexane to give the title compound (4.37 g) as a

yellow solid (yield 89%).
$^1H$ NMR (400 MHz, $CDCl_3$) δ 3.81 (3H, s), 4.04 (2H, s), 6.71 (1H, brs), 6.89-6.92 (2H, m), 7.16-7.20 (2H, m), 7.70 (1H, brs).

Example 253-B)

ethyl 2-(4-methoxybenzyl)-4-methyl-1,3-thiazole-5-carboxylate

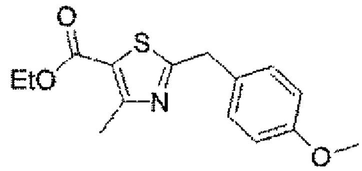

A solution of 2-(4-methoxyphenyl)ethanethioamide (4.37 g, 24.1 mmol) obtained in Example 253-A) and ethyl 2-chloro-3-oxobutanoate (4.00 mL, 28.9 mmol) in pyridine (60 mL)-ethanol (60 mL) was stirred at 90°C for 18 hr. The reaction mixture was cooled to room temperature, and concentrated under reduced pressure. The obtained residue was diluted with ethyl acetate (100 mL), and 2N hydrochloric acid (100 mL) was added. The organic layer was separated, washed with saturated brine (50 mL), dried over anhydrous sodium sulfate, and concentrated under reduced pressure. The obtained residue was purified by silica gel column chromatography [eluent: hexane-ethyl acetate (4:1)] to give the title compound (5.41 g) as a red oil (yield 77%).
$^1H$ NMR (400 MHz, $CDCl_3$) δ 1.31 (3H, t, J = 7.2 Hz), 2.70 (3H, s), 3. 80 (3H, s), 4.19 (2H, s), 4.27 (2H, q, J = 7.2 Hz), 6.87-6.89 (2H, m), 7.21-7.24 (2H, m).

Example 253-C)

2-(4-methoxybenzyl)-4-methyl-1,3-thiazole-5-carboxylic acid

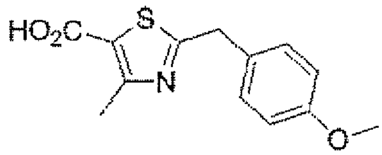

To a solution (37 mL) of ethyl 2-(4-methoxybenzyl)-4-methyl-1,3-thiazole-5-carboxylate (5.41 g, 18.5 mmol) obtained in Example 253-B) in ethanol was added 8N aqueous sodium hydroxide solution (4.64 mL, 37.1 mmol) at room temperature, and the mixture was stirred at the same temperature for 4 hr. The reaction mixture was ice-cooled, and adjusted to neutral with 6N hydrochloric acid. The precipitate was collected by filtration, and washed with water to give the title compound (4.50 g) as a white solid (yield 92%).
$^1H$ NMR (400 MHz, $CDCl_3$) δ 2.57 (3H, s), 3.74 (3H, s), 4.20 (2H, s), 6.90-6.93 (2H, m), 7.25-7.27 (2H, m).
* The peak of "COOH group" was not observed.

Example 253-D)

tert-butyl [2-(4-methoxybenzyl)-4-methyl-1,3-thiazol-5-yl]carbamate

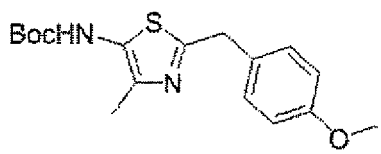

To a solution (25 mL) of 2-(4-methoxybenzyl)-4-methyl-1,3-thiazole-5-carboxylic acid (2.00 g, 7.60 mmol) obtained in Example 253-C) in tert-butyl alcohol was added triethylamine (3.18 mL, 22.8 mmol) at room temperature, the mixture was heated to 95°C and DPPA (2.46 mL, 11.4 mmol) was added. The mixture was stirred at the same temperature for 2 hr. The reaction product was concentrated under reduced pressure, and the residue was dissolved in ethyl acetate, and water was added. The organic layer was separated, washed successively with 2N hydrochloric acid, saturated aqueous sodium hydrogen carbonate solution and saturated brine, dried over anhydrous magnesium sulfate and concentrated under reduced pressure. The residue was purified by silica gel column chromatography [eluent: hexane-ethyl acetate (4:1-2:1)] to give the title compound (1.52 g) as a red brown oil (yield 59%).
MS (ESI+): $[M+H]^+$ 335.
$^1H$ NMR (300 MHz, DMSO-$d_6$) $\delta$ 1.42 (9H, s), 2.17 (3H, s), 3.73 (3H, s), 4.05 (2H, s), 6.82-6.94 (2H, m), 7.09-7.30 (2H, m), 9.51 (1H, brs).

Example 253-E)

N-[2-(4-methoxybenzyl)-4-methyl-1,3-thiazol-5-yl]-1-methyl-1H-pyrazole-5-carboxamide

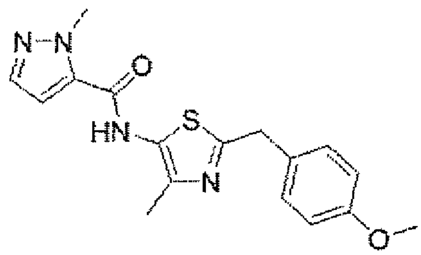

To a solution of tert-butyl [2-(4-methoxybenzyl)-4-methyl-1,3-thiazol-5-yl]carbamate (300 mg, 0.90 mmol) obtained in Example 253-D) in ethanol (3 mL) was added dropwise concentrated hydrochloric acid (1.2 mL) at room temperature, and the mixture was stirred at 50°C for 2 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (5 mL), and 1-methyl-1H-pyrazole-5-carboxylic acid (114 mg, 0.90 mmol), HATU (411 mg, 1.08 mmol) and DIEA (0.079 mL, 0.45 mmol) were added at room temperature. The reaction mixture was stirred at 60°C for 1 hr, saturated aqueous sodium hydrogen carbonate solution was added and the mixture was extracted with ethyl acetate. The organic layer was dried over anhydrous magnesium sulfate and concentrated under reduced pressure. The obtained residue was purified by silica gel column chromatography [eluent: hexane-ethyl acetate (1:1-0:1)] and basic silica gel column chromatography [eluent: hexane-ethyl acetate (1:1-0:1)], and crystallized from ethyl acetate-hexane to give the title compound (84.4 mg) as white crystals (yield 27%).
MS (ESI+): $[M+H]^+$ 343.
$^1H$ NMR (300 MHz, DMSO-$d_6$) $\delta$ 2.29 (4H, s), 3.32 (3H, s), 4.03 (3H, s), 4.13 (2H, br. S.), 6.79-6.98 (2H, m), 7.05 (1H, d, J = 1.1 Hz), 7.17-7.36 (2H, m), 7.46-7.61 (1H, m), 10.46 (1H, br. S.).

Example 254

$N^4$-[2-(3-chloro-5-fluorobenzyl)-4-methyl-1,3-thiazol-5-yl]piperidine-1,4-dicarboxamide

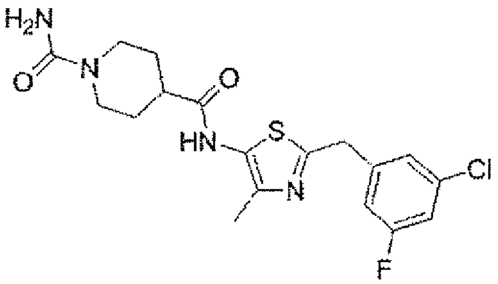

In the same manner as in Example 158, tert-butyl [2-(3-chloro-5-fluorobenzyl)-4-methyl-1,3-thiazol-5-yl]carbamate obtained in Example 145-E) and 1-carbamoylpiperidine-4-carboxylic acid were used to give the title compound as white crystals (yield 25%).
MS (ESI+): $[M+H]^+$ 461.
$^1$H NMR (300 MHz, DMSO-$d_6$) δ 1.31-1.54 (2H, m), 1.60-1.77 (2H, m), 2.29 (3H, s), 2.59-2.77 (3H, m), 3.88-4.06 (2H, m), 4.20 (2H, s), 5.93 (2H, s), 7.10-7.21 (1H, m), 7.21-7.28 (1H, m), 7.32 (1H, dt, J = 8.7, 2.1 Hz), 10.31 (1H, s).

Example 255

N-[2-(4-bromobenzyl)-4-methyl-1,3-thiazol-5-yl]-1-methyl-1H-pyrazole-5-carboxamide

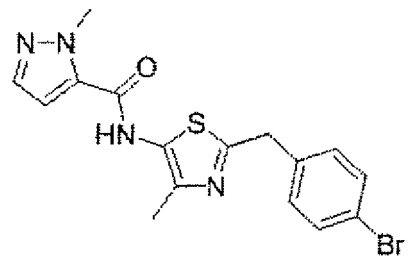

Example 255-A)

2-(4-bromophenyl)ethanethioamide

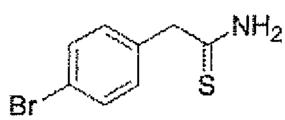

A mixture of (4-bromophenyl)acetonitrile (5.00 g, 25.5 mmol), O,O-diethyl dithiophosphate (5.57 mL, 30.6 mmol) and 4M hydrogen chloride-1,4-dioxane (51 mL) was stirred at room temperature for 18 hr. The reaction mixture was diluted with ethyl acetate (100 mL), water (100 mL) was added, and the organic layer was separated, washed successively with 0.2N aqueous sodium hydroxide solution (100 mL) and saturated brine, dried over anhydrous sodium sulfate, and concentrated under reduced pressure. The obtained residue was treated with hexane to allow solidification, and the solid was collected by filtration, and washed with hexane to give the title compound (4.98 g) as a white solid (yield 85%).
$^1$H NMR (400 MHz, $CDCl_3$) 5 4.04 (2H, s), 6.63 (1H, brs), 7.15-7.18 (2H, m), 7.49-7.53 (2H, m), 7.58 (1H, brs).

Example 255-B)

ethyl 2-(4-bromobenzyl)-4-methyl-1,3-thiazole-5-carboxylate

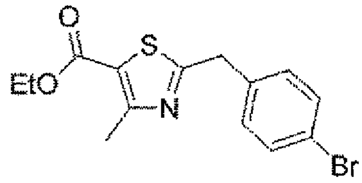

A solution of 2-(4-bromophenyl)ethanethioamide (4.98 g, 21.6 mmol) obtained in Example 255-A) and ethyl 2-chloro-3-oxobutanoate (3.59 mL, 26.0 mmol) in pyridine (60 mL)-ethanol (60 mL) was stirred at 90°C for 18 hr. The reaction mixture was cooled to room temperature, and concentrated under reduced pressure. The obtained residue was diluted with ethyl acetate (100 mL), and 2N hydrochloric acid (100 mL) was added. The organic layer was separated, washed with saturated brine (50 mL), dried over anhydrous sodium sulfate, and concentrated under reduced pressure. The obtained residue was purified by silica gel column chromatography [eluent: hexane-ethyl acetate (4:1)] to give the title compound (5.05 g) as an orange solid (yield 68%).
$^1H$ NMR (400 MHz, $CDCl_3$) $\delta$ 1.32 (3H, t, J = 7.2 Hz) , 2.70 (3H, s), 4.21 (2H, s), 4.28 (2H, q, J = 7.2 Hz), 7.17-7.20 (2H, m), 7.46-7.48 (2H, m).

Example 255-C)

2-(4-bromobenzyl)-4-methyl-1,3-thiazole-5-carboxylic acid

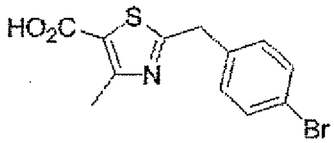

To a solution (37 mL) of ethyl 2-(4-bromobenzyl)-4-methyl-1,3-thiazole-5-carboxylate (4.00 g, 11.7 mmol) obtained in Example 255-B) in ethanol was added 8N aqueous sodium hydroxide solution (4.64 mL, 37.1 mmol) at room temperature, and the mixture was stirred at the same temperature for 4 hr. The reaction mixture was ice-cooled, and adjusted to neutral with 6N hydrochloric acid. The precipitate was collected by filtration, and washed with water to give the title compound (3.40 g) as a pale-yellow solid (yield 93%).
$^1H$ NMR (400 MHz, $CDCl_3$) $\delta$ 2.57 (3H, s), 4.28 (2H, s) , 7.31 (2H, d, J = 8.0 Hz) , 7.54 (2H, d, J = 8.0 Hz)
* The peak of "COOH group" was not observed.

Example 255-D)

tert-butyl [2-(4-bromobenzyl)-4-methyl-1,3-thiazol-5-yl]carbamate

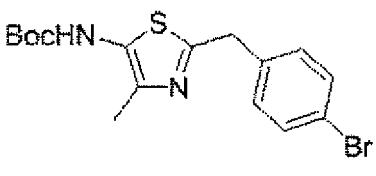

To a solution (36 mL) of 2-(4-bromobenzyl)-4-methyl-1,3-thiazole-5-carboxylic acid (3.40 g, 10.8 mmol) obtained in Example 255-C) in tert-butyl alcohol was added triethylamine (4.55 mL, 32.7 mmol) at room temperature, the mixture was heated to 95°C and DPPA (3.52 mL, 16.3 mmol) was added. The mixture was stirred at the same temperature for 4 hr. The reaction product was concentrated under reduced pressure, and the residue was dissolved in ethyl acetate, and water was added. The organic layer was separated, washed successively with 2N hydrochloric acid, saturated aqueous sodium hydrogen carbonate solution and saturated brine, dried over anhydrous magnesium sulfate and concentrated under reduced pressure. The residue was purified by silica gel column chromatography [eluent: hexane-ethyl acetate (4:1-2:1)] to give the title compound (3.00 g) as a yellow oil (yield 71%).

MS (ESI+): $[M+H]^+$ 383.
$^1$H NMR (300 MHz, DMSO-$d_6$) δ 1.43 (9H, s), 2.18 (3H, s), 4.12 (2H, s), 7.19-7.33 (2H, m), 7.47-7.57 (2H, m), 9.58 (1H, brs).

Example 255-E)

N-[2-(4-bromobenzyl)-4-methyl-1,3-thiazol-5-yl]-1-methyl-1H-pyrazole-5-carboxamide

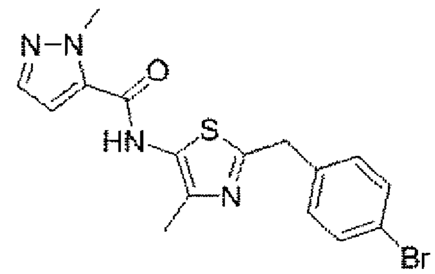

To a solution of tert-butyl [2-(4-bromobenzyl)-4-methyl-1,3-thiazol-5-yl]carbamate (346 mg, 0.90 mmol) obtained in Example 255-D) in ethanol (3 mL) was added dropwise concentrated hydrochloric acid (1.5 mL) at room temperature, and the mixture was stirred at 50°C for 1.5 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (8 mL), and 1-methyl-1H-pyrazole-5-carboxylic acid (136 mg, 1.08 mmol), HATU (411 mg, 1.08 mmol) and DIEA (0.079 mL, 0.45 mmol) were added at room temperature. The reaction mixture was stirred at 60°C for 1 hr, saturated aqueous sodium hydrogen carbonate solution was added and the mixture was extracted with ethyl acetate. The organic layer was dried over anhydrous magnesium sulfate and concentrated under reduced pressure. The obtained residue was purified by silica gel column chromatography [eluent: hexane-ethyl acetate (1:1-1:4)] and basic silica gel column chromatography [eluent: hexane-ethyl acetate (1: 1-1:4)], and crystallized from ethyl acetate-hexane to give the title compound (144 mg) as white crystals (yield 41%).
MS (ESI+): $[M+H]^+$ 391.
$^1$H NMR (300 MHz, DMSO-$d_6$) δ 2.30 (3H, s), 4.04 (3H, s), 4.20 (2H, s), 7.06 (1H, d, J = 2.3 Hz), 7.24-7.35 (2H, m), 7.46-7.59 (3H, m), 10.50 (1H, s).

Example 256

N-{2-[3-fluoro-4-(trifluoromethyl)benzyl]-4-methyl-1,3-thiazol-5-yl}-1-methyl-1H-pyrazole-5-carboxamide

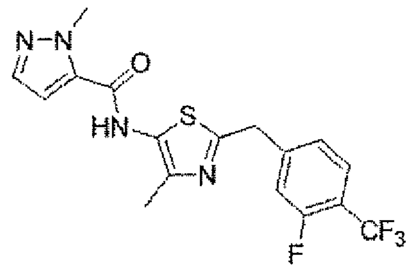

Example 256-A)

[3-fluoro-4-(trifluoromethyl)phenyl]acetonitrile

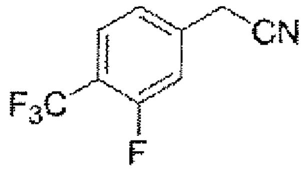

Under a nitrogen atmosphere, to a solution of 3-fluoro-4-(trifluoromethyl)benzoic acid (4.43 g, 21.3 mmol) in THF (77 mL) was added LAH (1.39 mL, 63.9 mmol) at 0°C, and the mixture was stirred at room temperature for 1 hr. The reaction mixture was cooled to 0°C, and saturated aqueous potassium hydrogensulfate solution was added. The mixture was filtered through celite, and washed with methylene chloride. The filtrate was concentrated under reduced pressure, and to a solution of the obtained residue and triethylamine (5.54 mL, 39.7 mmol) in methylene chloride (99 mL) was added methanesulfonyl chloride (1.86 ml, 23.8 mmol) at 0°C, and the mixture was stirred under a nitrogen atmosphere at room temperature for 1 hr. To the reaction mixture was added saturated aqueous ammonium chloride solution at room temperature, and the mixture was extracted with methylene chloride. The organic layer was separated, dried over anhydrous sodium sulfate, and concentrated under reduced pressure. The obtained residue was dissolved in DMSO (124 mL), sodium cyanide (0.73 g, 14.9 mmol) was added at room temperature, and the mixture was stirred at the same temperature for 1 hr. Water was added to the reaction mixture, and the mixture was extracted with ethyl acetate. The organic layer was separated, washed with saturated brine, dried over anhydrous sodium sulfate, and concentrated under reduced pressure. The obtained residue was purified by silica gel column chromatography [eluent: hexane-ethyl acetate (4:1)] to give the title compound (1.89 g) as a yellow oil (yield 62%).
$^1H$ NMR (400 MHz, $CDCl_3$) δ 3.83 (2H, s), 7.20-7.27 (2H, m), 7.61-7.67 (1H, m).

Example 256-B)

2-[3-fluoro-4-(trifluoromethyl)phenyl]ethanethioamide

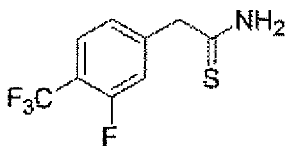

A mixture of [3-fluoro-4-(trifluoromethyl)phenyl]acetonitrile (1.89 g, 9.30 mmol) obtained in Example 256-A), O, O-diethyl dithiophosphate (2.03 mL, 11.2 mmol) and 4N hydrogen chloride-ethanol (18.6 mL) was stirred at room temperature overnight. The reaction mixture was concentrated under reduced pressure, water was added to the obtained residue, and the mixture was extracted with ethyl acetate. The organic layer was separated, washed with 0.2N aqueous sodium hydroxide solution and saturated brine, dried over anhydrous sodium sulfate, and concentrated under reduced pressure. The obtained residue was purified by silica gel column chromatography [eluent: hexane-ethyl acetate (2:1)] to give the title compound (2.02 g) as a pale-yellow solid (yield 92%).
$^1H$ NMR (400 MHz, $CDCl_3$) δ 4.10 (2H, s), 6.72 (1H, brs), 7.18-7.22 (2H, m), 7.58-7.63 (1H, m), 7.68 (1H, brs).

Example 256-C)

ethyl 2-[3-fluoro-4-(trifluoromethyl)benzyl]-4-methyl-1,3-thiazole-5-carboxylate

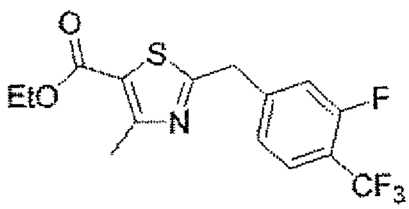

A solution of 2-[3-fluoro-4-(trifluoromethyl)phenyl]ethanethioamide (2.02 g, 8.53 mmol) obtained in Example 256-B) and ethyl 2-chloro-3-oxobutanoate (1.42 mL, 10.2 mmol) in pyridine (21 mL) -ethanol (21 mL) was stirred at 90°C for 16 hr. The reaction mixture was cooled to room temperature, and concentrated under reduced pressure. The obtained residue was diluted with ethyl acetate (40 mL), washed with 2N hydrochloric acid (40 mL) and saturated brine, dried over anhydrous sodium sulfate, and concentrated under reduced pressure. The obtained residue was purified by basic silica gel column chromatography [eluent: hexane-ethyl acetate (4:1)] to give the title compound (1.65 g) as a yellow oil (yield 56%).
$^1$H NMR (400 MHz, $CDCl_3$) δ 1.34 (3H, t, J = 6.8 Hz), 2.72 (3H, s), 4.26-4.34 (4H, m), 7.13-7.21 (2H, m), 7.53-7.60 (1H, m).

Example 256-D)

2-[3-fluoro-4-(trifluoromethyl)benzyl]-4-methyl-1,3-thiazole-5-carboxylic acid

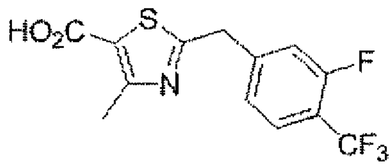

To a solution (24 mL) of ethyl 2-[3-fluoro-4-(trifluoromethyl)benzyl]-4-methyl-1,3-thiazole-5-carboxylate (1.65 g, 4.74 mmol) obtained in Example 256-C) in ethanol was added dropwise 8N aqueous sodium hydroxide solution (1.18 mL, 9.47 mmol) at room temperature, and the mixture was stirred at the same temperature for 4 hr. The reaction mixture was ice-cooled, and 6N hydrochloric acid was added. The precipitate was collected by filtration, and washed with water to give the title compound (1.38 g) as a yellow solid (yield 91%).
$^1$H NMR (400 MHz, $CDCl_3$) δ 2.58 (3H, s), 4.45 (2H, s), 7.41 (1H, d, J = 8.0 Hz), 7.54 (1H, d, J = 12.0 Hz), 7.78 (1H, t, J = 8.0 Hz)
* The peak of "COOH group" was not observed.

Example 256-E)

tert-butyl {2-[3-fluoro-4-(trifluoromethyl)benzyl]-4-methyl-1,3-thiazol-5-yl}carbamate

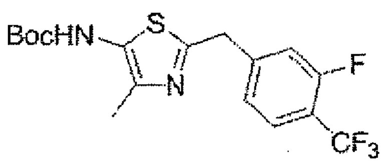

To a solution (14 mL) of 2-[3-fluoro-4-(trifluoromethyl)benzyl]-4-methyl-1,3-thiazole-5-carboxylic acid (1.38 g, 4.32 mmol) obtained in Example 256-D) in tert-butyl alcohol was added triethylamine (1.80 mL, 1.31 mmol) at room temperature, the mixture was heated to 95°C and DPPA (1.40 mL, 6.48 mmol) was added. The mixture was stirred at the same temperature for 1 hr. The reaction product was concentrated under reduced pressure, and the residue was diluted with ethyl acetate, washed with 1N hydrochloric acid, saturated aqueous sodium hydrogen carbonate solution and saturated brine, dried over anhydrous magnesium sulfate and concentrated under reduced pressure. The residue was purified by silica gel column chromatography [eluent: hexane-ethyl acetate (2:1)] to give the title compound (0.74 g) as a yellow oil (yield 44%).
MS (ESI+): $[M+H]^+$ 391.
$^1$H NMR (300 MHz, DMSO-$d_6$) 5 1.44 (5H, s), 2.19 (2H, s), 4.28 (2H, s), 7.26-7.56 (2H, m), 7.66-7.86 (1H, m), 9.64 (1H, brs).

Example 256-F)

N-{2-[3-fluoro-4-(trifluoromethyl)benzyl]-4-methyl-1,3-thiazol-5-yl}-1-methyl-1H-pyrazole-5-carboxamide

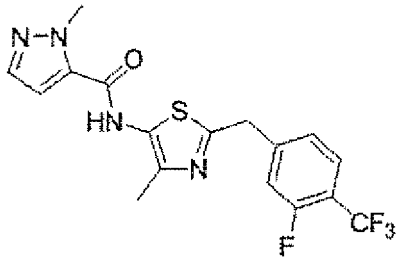

To a solution of tert-butyl {2-[3-fluoro-4-(trifluoromethyl)benzyl]-4-methyl-1,3-thiazol-5-yl}carbamate (535 mg, 1.37 mmol) obtained in Example 256-E) in ethanol (5 mL) was added dropwise concentrated hydrochloric acid (2.5 mL) at room temperature, and the mixture was stirred at 50°C for 2 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (10 mL), and 1-methyl-1H-pyrazole-5-carboxylic acid (207 mg, 1.65 mmol), HATU (626 mg, 1.65 mmol) and DIEA (0.120 mL, 0.69 mmol) were added at room temperature. The reaction mixture was stirred at 60°C for 1 hr, saturated aqueous sodium hydrogen carbonate solution was added and the mixture was extracted with ethyl acetate. The organic layer was dried over anhydrous magnesium sulfate and concentrated under reduced pressure. The obtained residue was purified by silica gel column chromatography [eluent: hexane-ethyl acetate (1:1-1:4)] and basic silica gel column chromatography [eluent: hexane-ethyl acetate (1:1-1:4)], and crystallized from ethyl acetate-hexane to give the title compound (305 mg) as white crystals (yield 56%).
MS (ESI+): $[M+H]^+$ 399.
$^1$H NMR (300 MHz, DMSO-$d_6$) δ 2.31 (3H, s), 4.05 (3H, s), 4.36 (2H, s), 7.07 (1H, d, J = 1.9 Hz), 7.39 (1H, d, J = 8.3 Hz), 7.45-7.56 (2H, m), 7.77 (1H, t, J = 8.0 Hz), 10.55 (1H, s).

Example 257

1-methyl-N-[4-methyl-2-(4-chlorobenzyl)-1,3-thiazol-5-yl]-1H-pyrazole-5-carboxamide

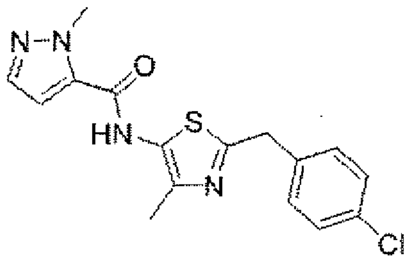

Example 257-A)

2-(4-chlorophenyl)ethanethioamide

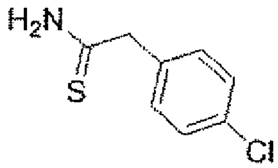

A mixture of 2-(4-chlorophenyl)acetonitrile (15.0 g, 99.0 mmol), O,O-diethyl dithiophosphate (20.3 g, 109 mmol) and 4M hydrogen chloride-ethyl acetate solution (198 mL, 792 mmol) was stirred at room temperature for 18 hr. To the reaction mixture was added water (200 mL), and the mixture was extracted with ethyl acetate (200 mL). The organic layer was washed successively with water (100 mL), 0.2M aqueous sodium hydroxide solution (100 mL) and saturated brine (100 mL), dried over anhydrous magnesium sulfate and concentrated under reduced pressure. The obtained residue was treated with hexane (200 mL) in an ice bath to allow solidification, and the solid was collected by filtration and washed with hexane to give the title compound (14.4 g) as a brown solid (yield 78%).
MS (ESI+): $[M+H]^+$ 186.
$^1H$ NMR (300 MHz, DMSO-$d_6$) $\delta$ 3.79 (2H, s), 7.36 (4H, s), 9.38 (1H, brs), 9.50 (1H, brs).

Example 257-B)

ethyl 4-methyl-2-(4-chlorobenzyl)-1,3-thiazole-5-carboxylate

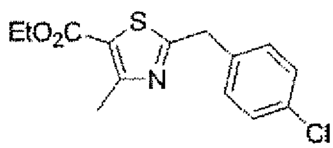

A solution of 2-(4-chlorophenyl)ethanethioamide (14.4 g, 77.5 mmol) obtained in Example 257-A) and ethyl 2-chloro-3-oxobutanoate (15.3 g, 93 mmol) in pyridine (150 mL)-ethanol (150 mL) was stirred at 60°C for 18 hr. The reaction mixture was cooled to room temperature, and concentrated under reduced pressure. The obtained residue was diluted with ethyl acetate (1.0 L), washed successively with water (1.0 L), 0.2M hydrochloric acid (500 mL) and saturated brine (500 mL), dried over anhydrous magnesium sulfate, and concentrated under reduced pressure. The obtained residue was purified by silica gel column chromatography [eluent: hexane-ethyl acetate (1:1)] to give the title compound (18.2 g) as yellow crystals (yield 79%).
MS (ESI+): $[M+H]^+$ 296.
$^1H$ NMR (300 MHz, $CDCl_3$) $\delta$ 1.32 (3H, t, J = 7.2 Hz), 2.70 (3H, s), 4.23 (2H, s), 4.28 (2H, q, J = 7.2 Hz), 7.21-7.35 (4H, m).

Example 257-C)

4-methyl-2-(4-chlorobenzyl)-1,3-thiazole-5-carboxylic acid

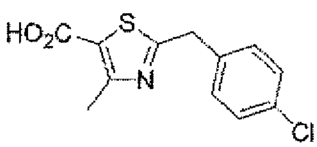

To s solution (150 mL) of ethyl 4-methyl-2-(4-chlorobenzyl)-1,3-thiazole-5-carboxylate (18.2 g, 61.6 mmol) obtained in Example 257-B) in ethanol was added 8N aqueous sodium hydroxide solution (15.4 mL, 123 mmol) at room temperature. The mixture was stirred at room temperature for 3 hr, and 6N hydrochloric acid (26.0 mL) was added thereto in an ice bath. The precipitate was collected by filtration, and washed with water (100 mL) to give the title compound (14.9 g) as a white solid (yield 90%).
MS (ESI+): $[M+H]^+$ 268.
$^1H$ NMR (300 MHz, DMSO-$d_6$) $\delta$ 2.57 (3H, s), 4.30 (2H, s), 7.28-7.51 (4H, m), 13.11 (1H, brs).

Experimental Example 257-D)

tert-butyl [4-methyl-2-(4-chlorobenzyl)-1,3-thiazol-5-yl]carbamate

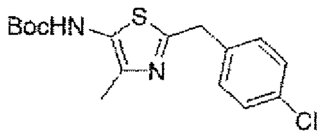

To a solution (15 mL) of 4-methyl-2-(4-chlorobenzyl)-1,3-thiazole-5-carboxylic acid (3.0 g, 11.2 mmol) obtained in Example 257-C) in tert-butyl alcohol were added triethylamine (4.69 mL, 33.6 mmol) and DPPA (3.63 mL, 16.8 mmol) at room temperature. The mixture was stirred at 95°C for 3 hr. The reaction product was concentrated under reduced pressure, and the residue was dissolved in ethyl acetate (100 mL), washed with water (100 mL) and saturated brine (100 mL), dried over anhydrous magnesium sulfate and concentrated under reduced pressure. The residue was purified by silica gel column chromatography [eluent: hexane-ethyl acetate (5:1)] to give the title compound (3.07 g) as yellow crystals (yield 81%).
MS (ESI+): $[M+H]^+$ 339.
$^1H$ NMR (300 MHz, $CDCl_3$) $\delta$ 1.49 (9H, s), 2.28 (3H, s), 4.15 (2H, s), 6.35 (1H, brs), 7.01-7.40 (4H, m).

Example 257-E)

1-methyl-N-[4-methyl-2-(4-chlorobenzyl)-1,3-thiazol-5-yl]-1H-pyrazole-5-carboxamide

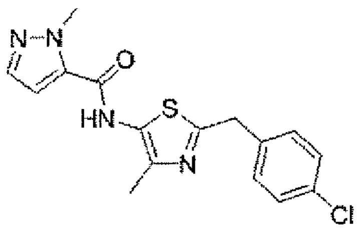

To a solution of tert-butyl [4-methyl-2-(4-chlorobenzyl)-1,3-thiazol-5-yl]carbamate (1.5 g, 4.43 mmol) obtained in Example 257-D) in ethanol (15 mL) was added dropwise concentrated hydrochloric acid (2.0 mL) at room temperature, and the mixture was stirred at 50°C for 4 hr. The reaction mixture was cooled to room temperature, neutralized with 8N aqueous sodium hydroxide solution, adjusted to pH 10-11 with saturated aqueous sodium hydrogen carbonate solution, and extracted with ethyl acetate. The organic layer was dried over anhydrous magnesium sulfate and concentrated under reduced pressure. The obtained residue was dissolved in DMA (5 mL), and 1-methyl-1H-pyrazole-5-carboxylic acid (0.63 g, 5.03 mmol), HATU (1.91 g, 5.03 mmol) and DIEA (0.37 mL, 2.09 mmol) were added at room temperature. The reaction mixture was stirred at 60°C for 2 hr, saturated aqueous sodium hydrogen carbonate solution was added and the mixture was extracted with ethyl acetate (50 mL). The organic layer was dried over anhydrous magnesium sulfate and concentrated under reduced pressure. The obtained residue was purified by silica gel column chromatography [eluent: hexane-ethyl acetate (4:1-1:2)] and basic silica gel column chromatography [eluent: hexane-ethyl acetate (13: 7-1:3)], and crystallized from ethyl acetate-hexane to give the title compound (235 mg) as pale-yellow crystals (yield 16%).
MS (ESI+) : $[M+H]^+$ 347.
$^1H$ NMR (300 MHz, DMSO-$d_6$) $\delta$ 2.30 (3H, s), 4.04 (3H, s), 4.22 (2H, s), 7.05 (1H, d, J = 2.3 Hz), 7.32-7.44 (4H, m), 7.53 (1H, d, J = 1.9 Hz), 10.49 (1H, s).

Experimental Example 1

Increase of intracellular cAMP concentration in human GPR52-expressing CHO cell

Using OptiPlate-384 (PerkinElmer Inc.), $1\times10^4$ human GPR52-expressing CHO(dhfr-) cells were incubated

together with a test compound (10 μm) in an assay buffer (30 μL, HBSS (containing $Ca^{2+}$ and $Mg^{2+}$), 0.5% BSA, 100 μM IBMX, 100 μM Ro20-1724, 5 mM HEPES (pH 7.55)) at 37°C for 30 min. Thereafter, according to the protocol of AlphaScreen cAMP Assay Kit (PerkinElmer Inc.), the intracellular cAMP concentration was measured by EnVision (PerkinElmer Inc.). The GPR52 agonist activity was calculated, assuming the intracellular cAMP concentration in the presence of 1 μM N-(2-hydroxyethyl)-3-[2-[3-(trifluoromethyl)benzyl]-1-benzofuran-4-yl]benzamide to be 100% and assuming the intracellular cAMP concentration when DMSO was added instead of the test compound to be 0%. The results are shown in Table 2.

[Table 2-1]

| Ex. No. | GPR52 agonist activity (%) |
|---|---|
| 1 | 92 |
| 3 | 81 |
| 4 | 72 |
| 6 | 83 |
| 8 | 78 |
| 11 | 92 |
| 12 | 75 |
| 14 | 80 |
| 16 | 72 |
| 17 | 94 |
| 20 | 74 |
| 21 | 69 |
| 23 | 80 |
| 24 | 93 |
| 26 | 78 |
| 29 | 71 |
| 30 | 81 |
| 33 | 70 |
| 39 | 79 |
| 40 | 80 |
| 41 | 72 |
| 43 | 73 |
| 47 | 82 |
| 51 | 77 |
| 76 | 86 |
| 110 | 82 |
| 122 | 96 |
| 145 | 95 |
| 146 | 84 |
| 147 | 78 |
| 148 | 76 |

[Table 2-2]

| Ex. No. | GPR52 agonist activity (%) |
|---|---|
| 149 | 75 |
| 150 | 69 |
| 151 | 69 |
| 153 | 72 |
| 156 | 76 |
| 157 | 77 |
| 158 | 69 |
| 159 | 71 |
| 160 | 74 |
| 161 | 81 |
| 162 | 70 |
| 163 | 78 |
| 164 | 80 |
| 166 | 88 |
| 167 | 70 |
| 169 | 76 |
| 171 | 70 |
| 172 | 89 |
| 175 | 71 |
| 176 | 99 |
| 177 | 86 |
| 179 | 80 |
| 180 | 75 |
| 181 | 91 |
| 184 | 87 |
| 185 | 81 |
| 186 | 94 |
| 189 | 78 |
| 190 | 82 |
| 206 | 74 |
| 208 | 72 |

[Table 2-3]

| Ex. No. | GPR52 agonist activity (%) |
|---|---|
| 210 | 82 |

(continued)

| Ex. No. | GPR52 agonist activity (%) |
|---|---|
| 216 | 77 |
| 219 | 82 |
| 220 | 79 |
| 221 | 90 |
| 224 | 80 |
| 227 | 101 |
| 228 | 69 |
| 230 | 71 |
| 233 | 81 |
| 234 | 78 |
| 235 | 79 |
| 238 | 80 |
| 242 | 76 |
| 243 | 77 |
| 246 | 70 |
| 247 | 69 |
| 249 | 99 |
| 252 | 72 |
| 254 | 77 |

Experimental Example 2

Inhibition test of methamphetamine-induced locomotor hyperactivity

The compound of the present invention can be evaluated by assessing its capacity to inhibit methamphetamine-induced locomotor hyperactivity in mice. The method of Okuyama et al (Life Science, Vol. 65, p. 2109-2125 (1999)) was modified and used for testing the inhibition of methamphetamine-induced locomotor hyperactivity.

Test Method

Locomotor activity was measured using a 32-channel locomotor activity measurement device MDC-LT (Brain-Science Idea Co., Ltd.), which emits infrared beams from the top of a cage (30 cm x 40 cm x 20 cm). 6- to 8-Week-old male ICR mice were acclimated for at least 45 min in the measurement cages, and then either test compound (30 mg/kg body weight) dissolved in vehicle (0.5% aqueous methylcellulose solution) or the vehicle alone was orally administered to the laboratory animals. This oral administration was followed 60 min later by subcutaneous administration of methamphetamine (2 mg/kg body weight).

Activity Calculation

The number of times the laboratory animals passed through the infrared beams emitted inside the measurement cages in 150 min after administration of the test compound or vehicle was counted to quantify locomotor activity. The methamphetamine-induced locomotor hyperactivity inhibition rate (activity inhibition rate (%)) was calculated by the following equation.

Activity inhibition rate (%) = {1-(locomotor activity of test compound treatment group after treatment with methamphetamine ÷ locomotor activity of vehicle treatment group after treatment with methamphetamine)} x 100

The results are shown in Table 3.

Table 3

| Ex. No. | motor activity suppression rate (%) |
|---|---|
| 3 | 32 |
| 145 | 47 |
| 146 | 42 |
| 148 | 67 |
| 153 | 39 |

Formulation Example 1

| | | |
|---|---|---|
| (1) | compound of Example 1 | 10.0 g |
| (2) | Lactose | 70.0 g |
| (3) | Cornstarch | 50.0 g |
| (4) | Soluble starch | 7.0 g |
| (5) | Magnesium stearate | 3.0 g |

The compound of Example 1 (10.0 g) and magnesium stearate (3.0 g) are granulated with an aqueous solution (70 ml) of soluble starch (7.0 g as soluble starch), dried, and the resulting mixture is mixed with lactose (70.0 g) and cornstarch (50.0 g) (lactose, cornstarch, soluble starch and magnesium stearate are all products on the Japanese Pharmacopoeia 14th ed.). The mixture is compressed to give tablets.

Industrial Applicability

Since the compound of the present invention has an agonist action on GPR52, it is useful as a medicament for the prophylaxis or treatment of mental diseases such as schizophrenia and the like, and the like.

This application is based on patent application No. 2010-015648 filed in Japan, the contents of which are encompassed in full herein.

## Claims

**1.** A compound represented by the formula:

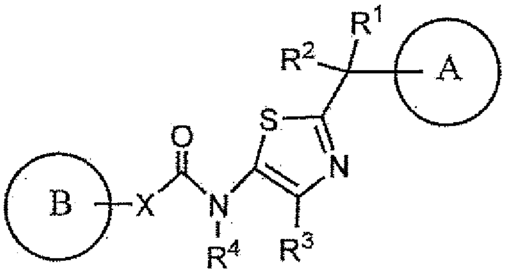

(I)

wherein

$R^1$ and $R^2$ are the same or different and each is a hydrogen atom, a hydroxy group, a $C_{1-6}$ alkyl group optionally having substituent(s), a $C_{3-6}$ cycloalkyl group optionally having substituent(s) or a $C_{1-6}$ alkoxy group optionally having substituent(s), or

$R^1$ and $R^2$ may form, together with the adjacent carbon atom, a 3- to 6-membered saturated hydrocarbon ring;

$R^3$ is a hydrogen atom, a halogen atom, a $C_{1-6}$ alkyl group optionally having substituent(s), a $C_{2-6}$ alkenyl group optionally having substituent(s), a $C_{2-6}$ alkynyl group optionally having substituent(s), a $C_{3-6}$ cycloalkyl group optionally having substituent(s) or an acyl group;

$R^4$ is a hydrogen atom, a $C_{1-6}$ alkyl group optionally having substituent(s) or a $C_{3-10}$ cycloalkyl group optionally having substituent(s);

ring A is a 3- to 10-membered hydrocarbon ring optionally having substituent(s);

ring B is a 3- to 10-membered hydrocarbon ring optionally having substituent(s) or a 3- to 11-membered heterocycle optionally having substituent(s); and

X is a single bond, a $C_{1-6}$ alkylene group optionally having substituent(s) or a $C_{2-6}$ alkenylene group,

[excluding N-{2-[3,5-bis(trifluoromethyl)benzyl]-1,3-thiazol-5-yl}-3-(2-hydroxyethoxy)-4-methoxybenzamide, N-{2-[3,5-bis(trifluoromethyl)benzyl]-1,3-thiazol-5-yl}-4-methoxy-3-[2-(tetrahydro-2H-pyran-2-yloxy)ethoxy]benzamide, 1-(1,3-benzodioxol-5-yl)-N-[2-(2-chlorobenzyl)-1,3-thiazol-5-yl]cyclopropanecarboxamide, N-[2-(2-chlorobenzyl)-1,3-thiazol-5-yl]-1-(4-methoxyphenyl)cyclopropanecarboxamide, N-(3-cyanobenzyl)-N-[2-(cyclopentylmethyl)-4-methyl-1,3-thiazol-5-yl]cyclopropanecarboxamide, N-(3-cyanobenzyl)-N-[2-(cyclopentylmethyl)-4-methyl-1,3-thiazol-5-yl]azetidine-1-carboxamide, N-(3-cyanobenzyl)-N-[2-(cyclopentylmethyl)-4-methyl-1,3-thiazol-5-yl]pyrrolidine-1-carboxamide, methyl 1-{(3-cyanobenzyl)[2-(cyclopentylmethyl)-4-methyl-1,3-thiazol-5-yl]carbamoyl}pyrrolidine-3-carboxylate, N-(3-cyanobenzyl)-N-[2-(cyclopentylmethyl)-4-methyl-1,3-thiazol-5-yl]morpholine-4-carboxamide, and N-[2-(4-chlorobenzyl)-1,3-thiazol-5-yl]-2-phenylacetamide] or a salt thereof.

**2.** The compound according to claim 1, wherein $R^4$ is

(1) a hydrogen atom,
(2) a $C_{3-10}$ cycloalkyl group optionally having substituent(s),
(3) a methyl group optionally having substituent(s) selected from a halogen atom, a cyano group, a hydroxy group, a $C_{1-6}$ alkoxy group optionally having substituent(s), a $C_{6-10}$ aryloxy group optionally having substituent (s), a sulfanyl group optionally having substituent(s), an amino group optionally having substituent(s), an acyl group, a $C_{3-10}$ cycloalkyl group optionally having substituent(s) and a 3- to 11-membered heterocyclic group optionally having substituent(s), or
(4) a $C_{2-6}$ alkyl group optionally having substituent(s), ring B is

(1) benzene having at least one substituent selected from a halogen atom, a cyano group, a $C_{1-6}$ alkyl group optionally having substituent(s), a $C_{2-6}$ alkenyl group optionally having substituent(s), a $C_{2-6}$ alkynyl group optionally having substituent(s), a sulfanyl group optionally having substituent(s), an amino group optionally having substituent(s), an acyl group, a 3- to 10-membered hydrocarbon ring group optionally having substituent(s) and a 3- to 11-membered heterocyclic group optionally having substituent(s),
(2) a 3- to 10-membered saturated hydrocarbon ring optionally having substituent(s) selected from a halogen atom, a cyano group, a hydroxy group, a $C_{1-6}$ alkyl group optionally having substituent(s), a $C_{2-6}$ alkenyl group optionally having substituent(s), a $C_{2-6}$ alkynyl group optionally having substituent(s), a $C_{1-6}$ alkoxy group optionally having substituent(s), a $C_{6-10}$ aryloxy group optionally having substituent(s), a sulfanyl group optionally having substituent(s), an amino group optionally having substituent(s), an acyl group, a $C_{3-10}$ cycloalkyl group optionally having substituent(s) and a 3- to 11-membered nitrogen-containing heterocyclic group optionally having substituent(s), or
(3) a 3- to 11-membered heterocycle optionally having substituent(s),

or a salt thereof.

**3.** The compound according to claim 1, wherein $R^3$ is

(1) a hydrogen atom,
(2) a $C_{1-6}$ alkyl group optionally substituted by 1 to 3 substituents selected from a halogen atom, a hydroxy group and a $C_{1-6}$ alkoxy group,
(3) a $C_{2-6}$ alkenyl group,
(4) a $C_{1-6}$ alkyl-carbonyl group, or
(5) a $C_{1-6}$ alkoxy-carbonyl group,

or a salt thereof.

**4.** The compound according to claim 1, wherein $R^4$ is a hydrogen atom or a $C_{1-6}$ alkyl group, or a salt thereof.

**5.** The compound according to claim 1, wherein ring A is a 6-to 10-membered aromatic hydrocarbon ring optionally substituted by 1 to 3 substituents selected from (i) a halogen atom, (ii) a $C_{1-6}$ alkyl group optionally substituted by 1 to 3 halogen atoms, (iii) a nitro group, (iv) a $C_{1-6}$ alkoxy group, (v) a hydroxy group, (vi) a $C_{3-10}$ cycloalkyl group and (vii) a $C_{1-6}$ alkylsulfonyl group, or a salt thereof.

**6.** The compound according to claim 1, wherein ring B is

(1) a 3- to 10-membered hydrocarbon ring optionally substituted by 1 to 3 substituents selected from

(a) a nonaromatic heterocyclic group optionally substituted by 1 to 3 oxo groups,
(b) an amino group optionally mono- or di-substituted by substituent (s) selected from a $C_{1-6}$ alkyl group and a $C_{1-6}$ alkyl-carbonyl group,
(c) a $C_{1-6}$ alkoxy group,
(d) a $C_{1-6}$ alkylsulfanyl group,
(e) a cyano group,
(f) a halogen atom,
(g) an acyl group and
(h) an oxo group, or

(2) a 3- to 11-membered heterocycle optionally having 1 to 3 substituents selected from

(a) a $C_{3-10}$ cycloalkyl group,
(b) a $C_{6-14}$ aryl group optionally substituted by 1 to 3 substituents selected from

(i) a $C_{1-6}$ alkoxy group,
(ii) a halogen atom and
(iii) an amino group optionally mono- or di-substituted by a $C_{1-6}$ alkyl group,

(c) an aromatic heterocyclic group,
(d) a nonaromatic heterocyclic group,
(e) an amino group optionally mono- or di-substituted by a $C_{1-6}$ alkyl-carbonyl group,
(f) a $C_{1-6}$ alkoxy group,
(g) a $C_{6-10}$ aryloxy group optionally substituted by 1 to 3 halogen atoms,
(h) a $C_{1-6}$ alkylsulfanyl group,
(i) a cyano group,
(j) a nitro group,
(k) a halogen atom,
(l) an acyl group,
(m) a $C_{1-6}$ alkyl group optionally substituted by 1 to 3 substituents selected from

(i) a halogen atom,
(ii) a hydroxy group,
(iii) an amino group optionally mono- or di-substituted by a $C_{1-6}$ alkyl group,
(iv) a $C_{6-10}$ aryloxy group optionally substituted by a $C_{1-6}$ alkoxy group,
(v) a carbamoyl group,
(vi) an aromatic heterocyclic group optionally substituted by 1 to 3 $C_{1-6}$ alkyl groups,
(vii) a nonaromatic heterocyclic group optionally substituted by 1 to 3 oxos
(viii) a $C_{1-6}$ alkoxy group,
(ix) a cyano group and
(x) a $C_{1-6}$ alkylsulfonyl group,

(n) a $C_{7-13}$ aralkyl group optionally substituted by 1 to 3 substituents selected from a $C_{1-6}$ alkyl group optionally substituted by 1 to 3 halogen atoms, and
(o) an oxo group,

or a salt thereof.

**7.** The compound according to claim 1, wherein X is a single bond, a $C_{1-6}$ alkylene group or a $C_{2-6}$ alkenylene group, or a salt thereof.

**8.** The compound according to claim 1, wherein $R^1$ and $R^2$ are the same or different and each is a hydrogen atom, a hydroxy group or a $C_{1-6}$ alkyl group, or $R^1$ and $R^2$ form, together with the adjacent carbon atom, a 3- to 6-membered saturated hydrocarbon ring,
or a salt thereof.

**9.** 1-Methyl-N-{4-methyl-2-[3-(trifluoromethyl)benzyl]-1,3-thiazol-5-yl}-1H-pyrazole-5-carboxamide or a salt thereof.

**10.** N-[2-(3-chloro-5-fluorobenzyl)-4-methyl-1,3-thiazol-5-yl]-1-methyl-1H-pyrazole-5-carboxamide or a salt thereof.

**11.** N-[2-(3-chloro-5-fluorobenzyl)-4-methyl-1,3-thiazol-5-yl]-1,3-dimethyl-1H-pyrazole-4-carboxamide or a salt thereof.

**12.** N-[2-(3-chloro-5-fluorobenzyl)-4-methyl-1,3-thiazol-5-yl]-1-ethyl-1H-pyrazole-5-carboxamide or a salt thereof.

**13.** N-[2-(3-chloro-5-fluorobenzyl)-4-methyl-1,3-thiazol-5-yl]-1-propyl-1H-pyrazole-5-carboxamide or a salt thereof.

**14.** A medicament comprising the compound according to claim 1 or a salt thereof.

**15.** The medicament according to claim 14, which is a GPR52 activating agent.

**16.** The medicament according to claim 14, which is an agent for the prophylaxis or treatment of schizophrenia.

**17.** A method for activation of GPR52 in a mammal, which comprises administering an effective amount of the compound according to claim 1 or a salt thereof to the mammal.

**18.** A method for the prophylaxis or treatment of schizophrenia in a mammal, which comprises administering an effective amount of the compound according to claim 1 or a salt thereof to the mammal.

**19.** Use of the compound according to claim 1 or a salt thereof for the production of a GPR52 activating agent.

**20.** Use of the compound according to claim 1 or a salt thereof for the production of an agent for the prophylaxis or treatment of schizophrenia.

**21.** The compound according to claim 1 or a salt thereof for use in a method of activating GPR52.

**22.** The compound according to claim 1 or a salt thereof for use in the prophylaxis or treatment of schizophrenia.

## INTERNATIONAL SEARCH REPORT

International application No. PCT/JP2011/051530

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D277/20, A61K31/426, A61K31/427, A61K31/428, A61K31/433, A61K31/4439, A61K31/454, A61K31/4709, A61K31/496, A61K31/497, A61K31/501, A61K31/502, A61K31/506, A61K31/519, A61K31/5377, A61K31/541, A61K31/5415, A61P25/18,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2009/150196 A1 (SMITHKLINE BEECHAM CORP.), 17 December 2009 (17.12.2009), Intermediate 55; example 27 & GB 810913 D & GB 810913 D0 | 1-8,14,21-22<br>9-13,15-16, 19-20 |
| X<br>A | WO 2008/139845 A1 (Daiichi Sankyo Co., Ltd.), 20 November 2008 (20.11.2008), table 3 (Family: none) | 1-8,14,21-22<br>9-13,15-16, 19-20 |
| X<br>A | JP 2003-212864 A (Sankyo Co., Ltd.), 30 July 2003 (30.07.2003), tables 4, 5 (Family: none) | 1-8,14,21-22<br>9-13,15-16, 19-20 |

[X] Further documents are listed in the continuation of Box C. [ ] See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 17 February, 2011 (17.02.11) | 01 March, 2011 (01.03.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/051530

C (Continuation). DOCUMENTS CONSIDERED TO BE RELEVANT

| Category* | Citation of document, with indication, where appropriate, of the relevant passages | Relevant to claim No. |
|---|---|---|
| X<br>A | UTO Y, et al., Novel and potent inhibitors of stearoyl-CoA desaturase-1. Part II: Identification of 4-ethylamino-3-(2-hydroxyethoxy)-N-[5-(3-trifluoromethylbenzyl) thiazol-2-yl] benzamide and its biological evaluation, Bioorganic and Medicinal Chemistry Letters, 2009, vol.19, p.4159-4166, Compound 11 | 1-8,14,21-22<br>9-13,15-16, 19-20 |
| A | JP 2009-520782 A (Astra Zeneca AB.), 28 May 2009 (28.05.2009),<br>& US 2008/0312206 A1 & EP 1966174 A<br>& WO 2007/071955 A1 & KR 10-2008-0085183 A<br>& CA 2632924 A & NO 20082690 A<br>& CN 101346361 A & IL 192280 D<br>& ZA 200805248 A | 1-16,19-22 |
| A | JP 2007-519740 A (Vertex Pharmaceuticals Inc.), 19 July 2007 (19.07.2007),<br>& US 2006/0052358 A1 & EP 1716122 A<br>& WO 2005/075435 A & WO 2005/075435 A1<br>& CA 2554796 A & CN 1938279 A | 1-16,19-22 |

Form PCT/ISA/210 (continuation of second sheet) (July 2009)

**INTERNATIONAL SEARCH REPORT**

International application No.
PCT/JP2011/051530

Continuation of A. CLASSIFICATION OF SUBJECT MATTER
(International Patent Classification (IPC))

*C07D277/20*(2006.01)i, *A61K31/426*(2006.01)i, *A61K31/427*(2006.01)i, *A61K31/428*(2006.01)i, *A61K31/433*(2006.01)i, *A61K31/4439*(2006.01)i, *A61K31/454*(2006.01)i, *A61K31/4709*(2006.01)i, *A61K31/496*(2006.01)i, *A61K31/497*(2006.01)i, *A61K31/501*(2006.01)i, *A61K31/502*(2006.01)i, *A61K31/506*(2006.01)i, *A61K31/519*(2006.01)i, *A61K31/5377*(2006.01)i, *A61K31/541*(2006.01)i, *A61K31/5415*(2006.01)i, *A61P25/18*(2006.01)i, *C07D277/44*(2006.01)i, *C07D277/68*(2006.01)i, *C07D417/12*(2006.01)i, *C07D417/14*(2006.01)i, *C07D487/04*(2006.01)i, *C07D495/04*(2006.01)i

(According to International Patent Classification (IPC) or to both national classification and IPC)

Continuation of B. FIELDS SEARCHED
Minimum documentation searched (International Patent Classification (IPC))

C07D277/44, C07D277/68, C07D417/12, C07D417/14, C07D487/04, C07D495/04

Minimum documentation searched (classification system followed by classification symbols)

Form PCT/ISA/210 (extra sheet) (July 2009)

**INTERNATIONAL SEARCH REPORT**

International application No.
PCT/JP2011/051530

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 17-18
because they relate to subject matter not required to be searched by this Authority, namely:
Claims 17 to 18 involve inventions which pertain to methods for treatment of human being by therapy using medicines.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006098520 A
- WO 2009107391 A
- WO 2009157196 A
- WO 2010018874 A
- WO 2008139845 A
- WO 2007071955 A
- WO 2005075435 A
- JP 2003212864 A
- EP 351194 A
- WO 200551954 A
- US 2005176776 A
- US 20016248766 A
- WO 200859368 A
- JP 2010015648 A


### Non-patent literature cited in the description


- *Molecular Brain Research,* 1999, vol. 64, 193-198
- *Bioorg. Med. Chem. Lett.,* 2009, vol. 19 (15), 4159-4166
- IYAKUHIN no KAIHATSU. Design of Molecules. HIROKAWA SHOTEN, 1990, vol. 7, 163-198
- Greene's protective groups in organic synthesis
- *J. Org. Chem.,* 1990, vol. 55, 270
- *J. Org. Chem.,* 1999, vol. 64, 3171-3177
- *J. Org. Chem.,* 1981, vol. 46, 4247-4252
- *Chemische Berichte,* 1910, vol. 43, 3529
- *Bioorg. Med. Chem. Lett.,* 2005, vol. 15, 5211-5217
- *Org. React.,* 1947, vol. 3, 337
- *J. Org. Chem.,* 1987, vol. 52, 4875
- *Org. Lett.,* 2002, vol. 4, 3663
- *J. Med. Chem.,* 1993, vol. 58, 3429-3434
- *J. Med. Chem.,* 1990, vol. 33, 464-479
- *J. Med. Chem.,* 1997, vol. 40, 2196-2210
- *J. Med. Chem.,* 1990, vol. 33, 1496-1504
- *J. Med. Chain.,* 1991, vol. 34, 397-403
- *Chimia,* 2003, vol. 57, 248-254
- *Nucleosides & Nucleotides,* 1995, vol. 14, 313-316
- *J. Med. Chem.,* 1967, vol. 10, 932-936
- *J. Org. Chem.,* 1979, vol. 44, 3938-3945
- *J. Med. Chem.,* 2001, vol. 44, 4207-4215
- *J. Heterocyclic. Chem.,* 1993, vol. 30, 939-944
- *Org. Lett,* 2003, vol. 5, 1265-1268
- **OKUYAMA et al.** *Life Science,* 1999, vol. 65, 2109-2125
- Japanese Pharmacopoeia